(19) Europäisches Patentamt
European Patent Office
Office européen des brevets

(11) EP 3 234 132 B1

(12) **EUROPEAN PATENT SPECIFICATION**

(45) Date of publication and mention of the grant of the patent:
**10.07.2019 Bulletin 2019/28**

(21) Application number: **15817743.6**

(22) Date of filing: **15.12.2015**

(51) Int Cl.:
***C12N 15/11*** (2006.01) ***C12N 15/113*** (2010.01)

(86) International application number:
**PCT/US2015/065906**

(87) International publication number:
**WO 2016/100401 (23.06.2016 Gazette 2016/25)**

# (54) LIGAND-MODIFIED DOUBLE-STRANDED NUCLEIC ACIDS

LIGANDENMODIFIZIERTE DOPPELSTRÄNGIGE NUKLEINSÄUREN

ACIDES NUCLÉIQUES DOUBLE BRIN MODIFIÉS PAR UN LIGAND

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**15.12.2014 US 201462092241 P**
**15.12.2014 US 201462092238 P**
**02.07.2015 US 201562187856 P**
**02.07.2015 US 201562187848 P**

(43) Date of publication of application:
**25.10.2017 Bulletin 2017/43**

(73) Proprietor: **Dicerna Pharmaceuticals, Inc.**
**Cambridge, MA 02140 (US)**

(72) Inventors:
- **BROWN, Bob, Dale**
  **Littleton, MA 01460 (US)**
- **WANG, Weimin**
  **Cambridge, MA 02140 (US)**

(74) Representative: **HGF Limited**
**8th Floor**
**140 London Wall**
**London EC2Y 5DN (GB)**

(56) References cited:
**EP-A1- 2 562 257**
**WO-A1-2011/111874**
**WO-A2-2009/073809**
**WO-A2-2010/033225**
**WO-A2-2010/141726**
**US-A1- 2014 350 074**

- **KUBO T ET AL: "Lipid-Conjugated 27-Nucleotide Double-Stranded RNAs with Dicer-Substrate Potency enhance RNAi-Mediated Gene Silencing", MOLECULAR PHARMACEUTICS, AMERICAN CHEMICAL SOCIETY, US, vol. 9, no. 5, 7 May 2012 (2012-05-07), pages 1374-1382, XP002716978, ISSN: 1543-8384, DOI: 10.1021/MP2006278 [retrieved on 2012-04-11]**
- **JAYAPRAKASH K. NAIR ET AL: "Multivalent N -Acetylgalactosamine-Conjugated siRNA Localizes in Hepatocytes and Elicits Robust RNAi-Mediated Gene Silencing", JOURNAL OF THE AMERICAN CHEMICAL SOCIETY, vol. 136, no. 49, 1 December 2014 (2014-12-01), pages 16958-16961, XP055181463, ISSN: 0002-7863, DOI: 10.1021/ja505986a**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 234 132 B1

## Description

## BACKGROUND

Double-stranded RNA (dsRNA) agents possessing strand lengths of 25 to 35 nucleotides have been described as effective inhibitors of target gene expression in mammalian cells (Rossi et al., U.S. Patent Publication Nos. 2005/0244858 and 2005/0277610). dsRNA agents of such length are believed to be processed by the Dicer enzyme of the RNA interference (RNAi) pathway, leading such agents to be termed "Dicer substrate siRNA" ("DsiRNA") agents. Certain modified structures of DsiRNA agents were previously described (Rossi et al., U.S. Patent Publication No. 2007/0265220). US 2014/0350074 A1 discloses dsNA with a pattern of deoxyribonucleotides that direct the site of Dicer cleavage for reducing target gene expression.

## BRIEF SUMMARY OF THE INVENTION

The present invention provides for a double stranded nucleic acid (dsNA) comprising a sense strand comprising 21 to 83 nucleotides; an antisense strand comprising 15 to 39 nucleotides; a duplex formed by said sense and antisense strand, having a length of 15 to 35 base pairs; wherein the dsNA comprises a discontinuity between the 5' terminus of the sense strand and the 3' terminus of the antisense strand or between the 3' terminus of the sense strand and the 5' terminus of the antisense strand; wherein the sense strand comprises a tetraloop and the tetraloop comprises at least one ligand conjugated nucleotide, wherein the ligand is GalNAc; and wherein said antisense strand is complementary to a target mRNA along at least 15 nucleotides of said antisense strand and said double stranded nucleic acid reduces target gene expression when said double stranded nucleic acid is introduced into a mammal or a mammalian cell, and preferably wherein the tetraloop comprises one, two, three or four ligand conjugated nucleotide and each of the ligands is GalNAc.

In one embodiment, the antisense strand has a length range of: 15-30 nucleotides, 18-25 nucleotides or 19-24 nucleotides.

In one embodiment, the sense strand has a length range of 35 to 55 nucleotides.

In another embodiment, the duplex has a length range of: 15-22 nucleotides or 15-30 nucleotides.

For example, the discontinuity is flanked on either side by a phosphorothioate modified nucleotide.

In another embodiment, the at least one strand of the dsNA comprises a 3' extension.

In another embodiment, the 3' extension has a length of 1-2 or 1-4 nucleotides.

In another embodiment, both sense and antisense strands consist of up to 100% chemically modified nucleotides.

In one embodiment, the antisense strand has a length range of 19-24 nucleotides or 19-30 nucleotides.

In another embodiment, the linker (L) comprises the structure of formula VII:

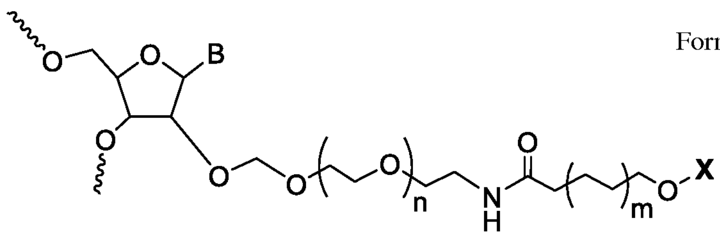

Formula VII

wherein:

B is a nucleobase;
m and n are each independently 1 - 20;
X has the structure

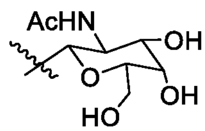

In a further embodiment, the linker is selected from the group consisting of:

a.

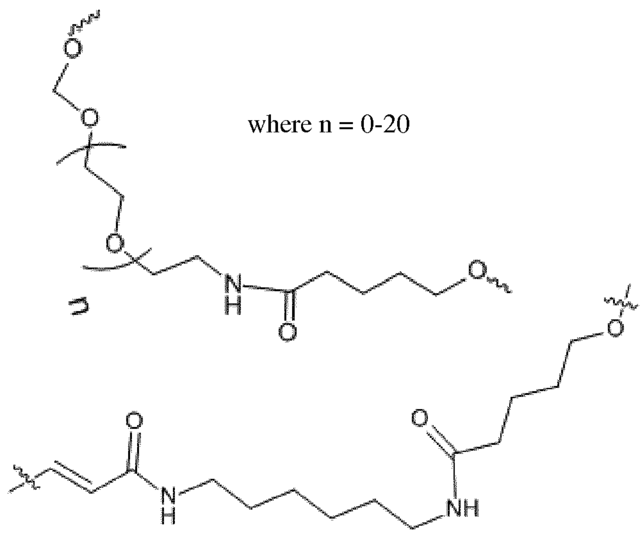

where n = 0-20

b.

c.

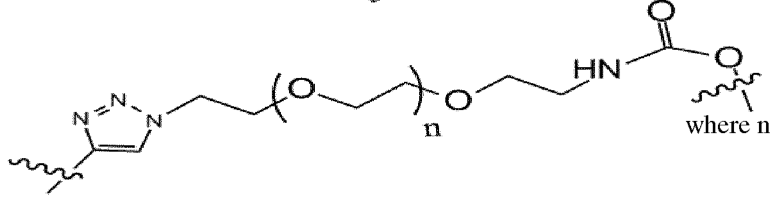

where n = 0-20

and

d.

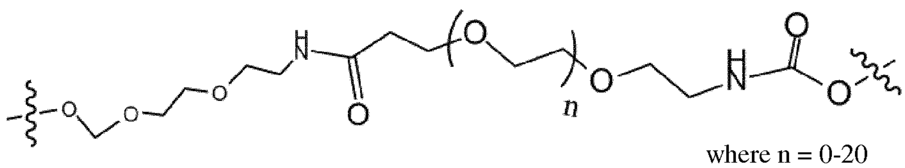

where n = 0-20

wherein said ligand is conjugated to the sugar or base of said nucleotide; and wherein said ligand-conjugated nucleotide is used for synthesizing the dsNA of the invention.

The foregoing features of embodiments will be more readily understood by reference to the following detailed description, taken with reference to the accompanying drawings.

## BRIEF DESCRIPTION OF DRAWINGS

FIGS. 1A and 1B show some embodiment structures and predicted Dicer-mediated processing of exemplary single strand extended Dicer substrates. In FIG. 1A, Panel A depicts a DsiRNA without a single stranded extension. Panel B depicts a "guide strand extended" DsiRNA agent, which has a guide strand 5' overhang 1-30 nucleotides in length (15 nucleotides as shown). Panel C depicts an exemplary "guide strand extended" DsiRNA agent, which has a guide strand 5' overhang 1-30 nucleotides in length (15 nucleotides as shown), with a short oligo complementary to the single-stranded extended region ("discontinuous complement"; discontinuous 3' passenger complement as shown). Panel D depicts an exemplary "passenger strand extended" DsiRNA agent, which has a passenger strand 3' overhang 1-30 nucleotides in length (15 nucleotides as shown). Panel E depicts a "passenger strand extended" DsiRNA agent, which has a passenger strand 5' overhang 1-50 nucleotides in length (15 nucleotides as shown). In each pair of oligonucleotide strands forming a DsiRNA, the upper strand is the passenger strand and the lower strand is the guide strand. White=nucleotide (e.g., a ribonucleotide, deoxyribonucleotide, modified ribonucleotide). FIG. 1B shows nucleotide modifications and patterns of modifications of exemplary single strand extended Dicer substrates. Panel A depicts a DsiRNA without a single stranded extension. Panel B depicts a "guide strand extended" DsiRNA agent, which has a guide strand 5' overhang 1-50 nucleotides in length (15 nucleotides as shown). Panel C depicts an exemplary "guide strand extended" DsiRNA agent, which has a guide strand 5' overhang 1-50 nucleotides in length (15 nucleotides as shown), with a short oligo complementary to the single-stranded extended region ("discontinuous complement"; discontinuous 3' passenger complement as shown). Panel D depicts an exemplary "passenger strand extended" DsiRNA agent, which has a passenger strand 3' overhang 1-50 nucleotides in length (15 nucleotides as shown). In each pair of oligonucleotide strands forming a DsiRNA, the upper strand is the passenger strand and the lower strand is the guide strand. Blue=ribonucleotide or modified ribonucleotide (e.g., 2'-O-methyl ribonucleotide); Gray=deoxyribonucleotide or ribonucleotide; White=ribonucleotide; Dark Yellow=deoxyribonucleotide, ribonucleotide, or modified nucleotide (e.g., 2'-O-methyl ribonucleotide, phosophorothioate deoxyribonucleotide; methylphosphonate deoxyribonucleotide). Small arrow=Dicer cleavage site; large arrow=discontinuity. A=position starting from the nucleotide residue of guide strand that is complementary to the 5' terminal nucleotide residue of passenger strand (position 1A); B=position starting from the 5' terminal nucleotide residue of guide strand (position 1B); C=position starting from the 5' terminal nucleotide of the short oligo complementary to single-stranded extended region (position 1C); D=position starting from the 3' terminal nucleotide residue of passenger strand (position ID); E=position starting from the 3' terminal nucleotide residue of passenger strand (position IE); F=position starting from the 5' terminal nucleotide consecutive to the antisense strand 5' single stranded overhang (position IF). Small arrows indicate predicted Dicer cleavage sites; a large arrow indicates a discontinuity.

FIG. 2 shows the structure and predicted Dicer-mediated processing of exemplary "guide strand extended" DsiRNA agents, which have a guide strand 5' overhang 1-30 nucleotides in length (10-15 nucleotides as shown). Blue=2'-O-methyl ribonucleotide; Gray=deoxyribonucleotide; White=ribonucleotide; Dark Yellow=phosophorothioate deoxyribonucleotide; Green=phosphorothioate 2'-O-methyl ribonucleotide; Pink=phosphorothioate ribonucleotide; Light Yellow=methylphosphonate deoxyribonucleotide. A=position starting from the nucleotide residue of said sense strand that is complementary to the 5' terminal nucleotide residue of passenger strand (position 1A); B=position starting from the 5' terminal nucleotide residue of guide strand (position 1B). Arrows indicate predicted Dicer cleavage sites.

FIG. 3 shows the structure and predicted Dicer-mediated processing of exemplary "passenger strand extended" DsiRNA agents, which have a passenger strand 3' overhang 1-30 nucleotides in length (10-15 nucleotides, as shown). Blue=2'-O-methyl ribonucleotide; Gray=deoxyribonucleotide; White=ribonucleotide; Dark Yellow=phosophorothioate deoxyribonucleotide; Green=phosphorothioate 2'-O-methyl ribonucleotide; Pink=phosphorothioate ribonucleotide; Light Yellow=methylphosphonate deoxyribonucleotide. A=position starting from the nucleotide residue of said sense strand that is complementary to the 5' terminal nucleotide residue of passenger strand (position 1A); D=position starting from the 3' terminal nucleotide residue of passenger strand. Arrows indicate predicted Dicer cleavage sites.

FIG. 4 shows the structure and predicted Dicer-mediated processing of exemplary "guide strand extended" DsiRNA agents, which have a guide strand 5' overhang 1-30 nucleotides in length. Single stranded guide extended DsiRNA agents having a passenger strand with the modification pattern depicted by DP1301P and a guide strand with a modification pattern depicted by DP1337G; DP1339G; DP1371G; and DP 1338G were generated. Additionally, the single stranded extended DsiRNA agents having a passenger strand with the modification pattern depicted in DP1301P, a guide strand with a modification pattern depicted by DP1337G; DP1339G; DP1371G; and DP1338G, and a "discontinuous 3' passenger complement" strand with a modification pattern depicted by DP1372P and DP1373P were generated. DsiRNA agents having a guide strand with the modification depicted DP1370G were used as a reference. Blue=2'-O-methyl ribonucleotide; Gray=deoxyribonucleotide; White=ribonucleotide; Dark Yellow=phosophorothioate deoxyribonucleotide; Green=phosphorothioate 2'-O-methyl ribonucleotide; Pink=phosphorothioate ribonucleotide; Light Yellow=methylphosphonate deoxyribonucleotide. Arrows indicate predicted Dicer cleavage sites.

FIG. 5 shows the structure and predicted Dicer-mediated processing of exemplary "passenger strand extended" DsiRNA agents, which have a passenger strand 3' overhang 1-30 nucleotides in length. Single stranded passenger extended DsiRNA agents having a guide strand with the modification pattern depicted by DP1XXXG and a passenger strand with a modification pattern depicted by DP1YYXP; DP1YxxP; and DP1YxxP were generated. DsiRNA agents having a passenger strand with the modification depicted DP1301P were used as a reference. Blue=2'-O-methyl ribonucleotide; Gray=deoxyribonucleotide; White=ribonucleotide; Dark Yellow=phosophorothioate deoxyribonucleotide; Green=phosphorothioate 2'-O-methyl ribonucleotide; Pink=phosphorothioate ribonucleotide; Light Yellow=methylphosphonate deoxyribonucleotide. Arrows indicate predicted Dicer cleavage sites.

FIG. 6 shows the sequence, structure, and predicted Dicer-mediated processing of exemplary "guide strand extended" DsiRNA agents targeting KRAS-249M, which have a guide strand 5' overhang 1-15 nucleotides in length. Single stranded guide extended DsiRNA agents having a passenger strand depicted by DP 1301 P (SEQ ID NO: 13) and a guide strand depicted by DP1337G (SEQ ID NO: 15); DP1338G (SEQ ID NO: 16); DP1340G (SEQ ID NO: 17); DP1341G (SEQ ID NO: 18); and DP1342G (SEQ ID NO: 18) were generated and tested. DsiRNA agents having a passenger strand depicted by DP1301P and a guide strand depicted by DP 1336G were used as a reference. Descriptions of the modification patterns of the discontinuous complements are labeled to the right. RNA=ribonucleotide; PS=phosphorothioate; DNA=deoxyribonucleotide; 2’OMe=2’-O-methyl; Underline=2’-O-methyl ribonucleotide; Bold=guide strand 5’ overhang; lower=deoxyribonucleotide; UPPER=ribonucleotide. Arrows indicate predicted Dicer cleavage sites. DP1336 G is SEQ ID NO: 14.

FIG. 7 is a histogram showing the normalized fold expression of KRAS-249M using DsiRNA agents having the passenger strands and guide strands depicted in FIG. 5. Hela cells were treated with 0.1 nM of the DsiRNA agents in RNAiMAX, 24 hrs.

FIG. 8 shows the sequence, structure, and predicted Dicer-mediated processing of exemplary "guide strand extended" DsiRNA agents targeting HPRT 1, which have a guide strand 5’ overhang 1-15 nucleotides in length. Single stranded guide extended DsiRNA agents having a passenger strand depicted by DP 1001 P (SEQ ID NO: 19) and a guide strand depicted by DP1350G (SEQ ID NO: 21); DP1351G (SEQ ID NO: 22); DP1352G (SEQ ID NO: 22); DP1353G (SEQ ID NO: 23); DP1354G (SEQ ID NO: 24); and DP1355G (SEQ ID NO: 24) were generated and tested. DsiRNA agents having a passenger strand depicted by DP 1001 P and a guide strand depicted by DP 1002G was used as a reference. Descriptions of the modification patterns of the discontinuous complements are labeled to the right. RNA=ribonucleotide; PS=phosphorothioate; DNA=deoxyribonucleotide; 2’OMe=2’-O-methyl; Underline=2’-O-methyl ribonucleotide; Bold=guide strand 5’ overhang; lower=deoxyribonucleotide; UPPER=ribonucleotide. Arrows indicate predicted Dicer cleavage sites. DP 1002G is SEQ ID NO: 20.

FIG. 9 is a histogram showing the normalized fold expression of HPRT1 using DsiRNA agents having the passenger strands and guide strands depicted in FIG. 7. Hela cells were treated with 0.1 nM of the DsiRNA agents in RNAiMAX, 24 hrs.

FIG. 10 is an image of a gel showing a Dicer activity on single stranded guide extended DsiRNA agents (passenger+guide strands) targeting KRAS-249M or HPRT1. Treatment: 2 hours at 37 C Turbo Dicer (1 U/reaction). Gel: 18% Tris 90’ @ 10 W. Loading: (1 μl 50 μM+50 μl Buffer and load 10 μl) or (5 μl reaction+20 μl Buffer and load 10 μl).

FIG. 11 shows the sequence and structure of exemplary short oligos that complement guide strand extensions ("discontinuous complements"), which are 1-16 nucleotides in length, base paired to 5’ guide strand extensions. Single stranded guide extended DsiRNA agents having a discontinuous complement depicted by DP 1365P; DP 1366P; DP1367P; DP1368P; and DP1369P were generated and tested. Descriptions of the modification patterns of the discontinuous complements are labeled to the right. RNA=ribonucleotide; PS=phosphorothioate; DNA=deoxyribonucleotide; 2’OMe=2’-O-methyl; Underline=2’-O-methyl ribonucleotide; Bold=guide strand 5’ overhang; lower=deoxyribonucleotide; UPPER=ribonucleotide. Arrows indicate predicted Dicer cleavage sites. The lower strand in each oligonucleotide pair is SEQ ID NO: 27.

FIG. 12 is a histogram showing the normalized fold expression of KRAS-249M using DsiRNA agents having the passenger strands and guide strands depicted in FIG. 7 and the discontinuous complements depicted in FIG. 10. The discontinuous complements used is labeled above each set of the three bars corresponding to DsiRNA agents having a 5’ guide single stranded extension (1.-r. DNA, RNA, 2’OMe RNA). Hela cells were treated with 0.1 nM of the DsiRNA agents in RNAiMAX, 24 hours.

FIG. 13 is a histogram showing the normalized fold expression of HPRT1 using DsiRNA agents having the passenger strands and guide strands depicted in FIG. 7 and the discontinuous complements depicted in FIG. 10. The discontinuous complement used is labeled above each set of the three bars corresponding to DsiRNA agents having a 5’ guide single stranded extension (1.-r. DNA, RNA, 2’OMe RNA). Hela cells were treated with 0.1 nM of the DsiRNA agents in RNAiMAX, 24 hours.

FIG. 14 show the structure and predicted Dicer-mediated processing of exemplary single strand extended Dicer substrates in an in vivo experiment (Experimental conditions: Treatment: 10 mg/kg one injection; Target: KRAS; Transfection: in vivo Fectamine; Tissue: Liver). Panel A depicts a modification pattern used in a negative control DsiRNA without a single stranded extension. Panel B depicts a modification pattern used in a positive control DsiRNA without a single stranded extension. Panel C depicts a modification pattern used in a test DsiRNA without a single stranded extension. Panel D depicts a modification pattern used in a test "guide strand extended" DsiRNA agent, which has a guide strand 5’ overhang 1-30 nucleotides in length (10 nucleotides as shown). Panel E depicts a modification pattern used in a test "guide strand extended" DsiRNA agent, which has a guide strand 5’ overhang 1-30 nucleotides in length (10 nucleotides as shown). In each pair of oligonucleotide strands forming a DsiRNA, the upper strand is the passenger strand and the lower strand is the guide strand. Blue=ribonucleotide or modified ribonucleotide (e.g., 2’-O-methyl ribonucleotide); Gray=deoxyribonucleotide or ribonucleotide; White=ribonucleotide; Dark Yellow=deoxyribonucleotide, ribonucleotide, or modified nucleotide (e.g., 2’-O-methyl ribonucleotide, phosophorothioate deoxyribonucleotide; methylphosphonate deoxyribonucleotide). Small arrow=Dicer cleavage site; large arrow=discontinuity. A=position starting from the nucleotide residue of said sense strand that is complementary to the 5’ terminal nucleotide residue of passenger strand (position 1A); B=position starting from the 5’ terminal nucleotide residue of guide strand. Small arrows indicate predicted Dicer cleavage sites; a large arrow indicates a discontinuity.

FIG. 15 shows the sequence, structure, and predicted Dicer-mediated processing of exemplary "guide strand extended" DsiRNA agents targeting KRAS-249M and HPRT 1, which have a guide strand 5' overhang 1-15 nucleotides in length. The sequence and structure of exemplary short oligos that complement guide strand extensions ("discontinuous complements") are shown base paired to 5' guide strand extension sequences. Single stranded guide extended DsiRNA agents having a passenger strand with the modification pattern depicted by DP 1301 P and a guide strand with a modification pattern depicted by DP1337G, DP1338G, DP1339G (SEQ ID NO 16), DP1371G (SEQ ID NO 16), and DP 1352G were generated. Additionally, the single stranded extended DsiRNA agents having a passenger strand with the modification pattern depicted in DP 1301 P(SEQ ID NO 13), a guide strand with a modification pattern depicted by DP1337G (SEQ ID NO 15), DP1338G (SEQ ID NO 13), DP1339G (SEQ ID NO 16), DP 1371G (SEQ ID NO 16), and DP1352G (SEQ ID NO 22); and an "discontinuous complement" strand with a modification pattern depicted by DP1372P (SEQ ID NO 28) and DP1373P (SEQ ID NO 28) were generated. DsiRNA agents having a passenger strand with the modification depicted by DP 1301 P (SEQ ID NO 13) were used as a reference and a guide strand with the modification depicted by DP1370G (SEQ ID NO 13) were used as a reference. Dosage of passenger strands, guide strands, and discontinuous complements are labeled to the right. Descriptions of the modification patterns of the discontinuous complements are also labeled on the right. RNA=ribonucleotide; PS=phosphorothioate; DNA=deoxyribonucleotide; 2'OMe=2'-O-methyl; Underline=2'-O-methyl ribonucleotide; Bold=guide strand 5' overhang; lower=deoxyribonucleotide; UPPER=ribonucleotide. Arrows indicate predicted Dicer cleavage sites. SEQ ID NO: 28 is base paired with SEQ ID NO: 29. DP1370G is SEQ ID NO: 14.

FIG. 16 is a histogram showing the normalized fold expression of mKRAS in liver of individual animals treated with DsiRNA agents having the passenger strands and guide strands depicted in FIGS. 14 and/or 15. Animals were treated with a 10 mg/kg injection of the DsiRNA agents in in vivo Fectamine and liver samples were analyzed.

FIG. 17 is a histogram showing the normalized fold expression of mKRAS in liver of animals treated with DsiRNA agents having the passenger strands and guide strands depicted in FIGS. 14 and/or 15. Animals were treated with a 10 mg/kg injection of the DsiRNA agents in in vivo Fectamine and liver samples were analyzed.

FIG. 18 are graphs showing the normalized fold expression of mKRAS in liver of animals treated with DsiRNA agents having the passenger strands and guide strands depicted in FIGS. 14 and/or 15. Animals were treated with a 10 mg/kg injection of the DsiRNA agents in in vivo Fectamine and liver samples were analyzed.

FIG. 19 is a histogram showing the normalized fold expression of mKRAS in spleen of individual animals treated with DsiRNA agents having the passenger strands and guide strands depicted in FIGS. 14 and/or 15. Animals were treated with a 10 mg/kg injection of the DsiRNA agents in in vivo Fectamine and spleen samples were analyzed.

FIG. 20 is a histogram showing the normalized fold expression of mKRAS in spleen of animals treated with DsiRNA agents having the passenger strands and guide strands depicted in FIGS. 14 and/or 15. Animals were treated with a 10 mg/kg injection of the DsiRNA agents in in vivo Fectamine and spleen samples were analyzed.

FIG. 21 are graphs showing the normalized fold expression of mKRAS in spleen of animals treated with DsiRNA agents having the passenger strands and guide strands depicted in FIGS. 14 and/or 15. Animals were treated with a 10 mg/kg injection of the DsiRNA agents in in vivo Fectamine and spleen samples were analyzed.

FIG. 22 is a histogram showing the normalized fold expression of mKRAS in kidney of individual animals treated with DsiRNA agents having the passenger strands and guide strands depicted in FIGS. 14 and/or 15. Animals were treated with a 10 mg/kg injection of the DsiRNA agents in in vivo Fectamine and kidney samples were analyzed.

FIG. 23 is a histogram showing the normalized fold expression of mKRAS in kidney of animals treated with DsiRNA agents having the passenger strands and guide strands depicted in FIGS. 14 and/or 15. Animals were treated with a 10 mg/kg injection of the DsiRNA agents in in vivo Fectamine and kidney samples were analyzed.

FIG. 24 are graphs showing the normalized fold expression of mKRAS in kidney of animals treated with DsiRNA agents having the passenger strands and guide strands depicted in FIGS. 14 and/or 15. Animals were treated with a 10 mg/kg injection of the DsiRNA agents in *in vivo* Fectamine and kidney samples were analyzed.

FIG. 25 A shows the activity of N-acetylgalactosamine-containing oligomers (DP2421P:DP2460G; DP2421P:DP2461G; DP2422P:DP2462G and DP2286P-GalNac:DP2281G) in primary mouse hepatocyte cultures. Panel 1 shows the activity of DP2421P:DP2460G with respect to concentration, Panel 2 shows the activity of DP2421P:DP2461G with respect to concentration, Panel 3 shows the activity of DP2422P:DP2462G with respect to concentration, and Panel 4 shows the activity of DP2286P-GalNac:DP2281G with respect to concentration. The self-delivery activities exhibited by different classes of oligomer were examined with dsNA oligomers constructed with a 5' extension of the antisense or sense strand, the extension containing 0 to four N-acetylgalactosamine ligands or triantennary ligands as well as dsNA oligonucleotides having a tetraloop, with tetraloop three or four N-acetylgalactosamine ligands or a positioned on the loop, or a triantennary N-acetylgalactosamine ligand on the loop, or three or four N-acetylgalactosamine ligands positioned on the stem.

FIG. 25 B shows the activity of N-acetylgalactosamine-containing oligomers (DP2421P:DP2460G, DP2421P:DP2461G; DP2422P:DP2462G; DP2463P:DP2382G; DP2464P:DP2382G; DP2465P:DP2382G; DP2466P:DP2382G; DP2467:DP2382G; DP2286P-GalNAc:DP2281G) in primary mouse hepatocyte cultures. Panel 1 shows the activity of DP2421P:DP2460G with respect to concentration, Panel 2 shows the activity of

DP2421P:DP2461G with respect to concentration, Panel 3 shows the activity of DP2422P:DP2462G with respect to concentration, Panel 4 shows the activity of DP2463P:DP2382G with respect to concentration, Panel 5 shows the activity of DP2464P:DP2382G with respect to concentration, Panel 6 shows the activity of DP2465P:DP2382G with respect to concentration, Panel 7 shows the activity of DP2466P:DP2382G with respect to concentration, and Panel 8 shows the activity of DP2467:DP2382G with respect to concentration and Panel 9 shows the activity of DP2286P-GalNAc:DP2281G with respect to concentration. The self-delivery activities exhibited by different classes of oligomer were examined with dsNA oligomers constructed with a 5' extension of the antisense or sense strand, the extension containing 0 to four N-acetylgalactosamine ligands or triantennary ligands as well as dsNA oligonucleotides having a a tetraloop, in some embodiments the tetraloop having three or four N-acetylgalactosamine ligands positioned on the loop, or a triantennary N-acetylgalactosamine ligand on the loop, or three or four N-acetylgalactosamine ligands positioned on the stem

FIG. 26 shows the binding affinities of N-acetylgalactosamine containing ligands (DP2421P:DP2460G, DP2421P:DP2461G; DP2422P:DP2462G; DP2463P:DP2382G; DP2464P:DP2382G; DP2465P:DP2382G; DP2466P:DP2382G; DP2467:DP2382G; DP2286P-GalNAc: DP2281G) to ASGPr using fluorescence polarization measurements. Panel 1 shows the binding affinity of DP2421P:DP2460G towards ASGPr with respect to concentration, Panel 2 shows the binding affinity of DP2421P:DP2461G towards ASGPr with respect to concentration, Panel 3 shows the binding affinity of DP2422P:DP2462G towards ASGPr with respect to concentration, Panel 4 shows the binding affinity of DP2463P:DP2382G towards ASGPr with respect to concentration, Panel 5 shows the binding affinity of DP2464P:DP2382G towards ASGPr with respect to concentration, Panel 6 shows the binding affinity of DP2465P:DP2382G towards ASGPr with respect to concentration, Panel 7 shows the binding affinity of DP2466P:DP2382G towards ASGPr with respect to concentration, and Panel 8 shows the binding affinity of DP2467:DP2382G towards ASGPr with respect to concentration and Panel 9 shows the binding affinity of DP2286P-GalNAc:DP2281G towards ASGPr with respect to concentration. The binding affinities exhibited by different groups of oligomers were examined, including dsNA oligomers having a 5'extension of the antisense or sense strand, the extension containing three or four N-acetylgalactosamine ligands or triantennary ligands. Additional oligomers comprising dsNA forming a tetraloop with three or four N-acetylgalactosamine ligands or a triantennary N-acetylgalactosamine ligand on the loop, or having three or four N-acetylgalactosamine ligands on the stem were also tested.

FIG. 27 and FIG. 28 show CTNNB1 mRNA knockdown activity in mice treated with N-acetylgalactosamine DsiRNA conjugates. Each conjugate in the Figures contains a tetraloop but have differing modifications of the nucleotides. The knockdown efficiency of several of the oligomers were examined , including dsNA oligomers with 5' extensions of the antisense or sense strand containing three or four N-acetylgalactosamine ligands, or triantennary ligands. dsNA oligonucleotides having a tetraloop with three or four N-acetylgalactosamine ligands or triantennary N-acetylgalactosamine on the loop, and those having the N-acetylgalactosamine ligands positioned on the stem of the tetraloop.

FIG. 29 shows HAO1 mRNA knockdown activity in mice treated with N-acetylgalactosamine DsiRNA at dosage of 25mpk. The knockdown efficiency of oligomers with 5' extensions or tetraloop configurations were examined, including those with 5'extensions of the sense and antisense strands, and a tetraloop, each having the ligand N-acetylgalactosamine attached to the 5' extension or the tetraloop, where configurations of three or four ligands or triantennary ligands attached to the 5'extension or to the stem or loop of the tetraloop. In figure 29, the first six dsNA molecules after the PBS sample are tetraloop molecules and the the next six dsNA molecules are extension molecules. The six tetraloop molecules differ amongst themselves in modification patterns and likewise the six extension molecules differ amongst themselves in chemical modification patterns. The details of modification patterns are disclosed in Table 3.

FIG. 30 shows results of a dose response study administering a HAO1 DsiRNA N-acetylgalactosamine conjugate to mice. Dose response data are shown for mice administered oligonucleotides constructed with a 5' extension on the sense or antisense strand, and for a tetraloop configuration, where three or four N-acetylgalactosamine ligands were attached to the 5' extension or to the stem or loop of the tetraloop configuration.

FIGS. 31, 31A, 31B, 31C, and 31D show some embodiment structures of the invention. FIG. 31A shows a "nicked antisense" dsNA containing a sense strand with an extended loop (Region E) at the 5' terminus, a duplex region formed by the antisense strand with the single stranded portion of the sense strand (Region B). When the dsNA is duplexed, a discontinuity exists where Dicer cleaves the antisense strand (shown by black arrow). FIG. 31B shows a "nicked antisense" dsNA containing a sense strand with an extended loop (Region E) at the 5' terminus, a duplex region formed by the antisense strand with the single stranded portion of the sense strand (Region B), and a 3' overhang on the antisense strand (Region F, shown by dashed circle). When the dsNA is duplexed, a discontinuity exists where Dicer cleaves the antisense strand (shown by black arrow). FIG. 31C shows a "nicked sense" dsNA containing a sense strand with an extended loop (Region J) at the 3' terminus, and a duplex region formed by the antisense strand with the single stranded portion of the sense strand (Region H). When the dsNA is duplexed, a discontinuity exists where Dicer cleaves the sense strand (shown by black arrow). The nucleotide immediately

proximal to the Dicer cleavage site must be a ribonucleotide (gray). FIG. 31D shows a "nicked sense" dsNA containing a sense strand with an extended loop (Region J) at the 3' terminus, and a duplex region formed by the antisense strand with the single stranded portion of the sense strand (Region H), and a 3' overhang on the antisense strand (Region F shown by dashed circle). When the dsNA is duplexed, a discontinuity exists where Dicer cleaves the sense strand (black arrow). The nucleotide immediately proximal to the Dicer cleavage site must be a ribonucleotide (gray).

FIGS. 32A and 32B depict how positions of nucleotides are calculated from the 5' and 3' termini of the sense and antisense strands of the dsNAs of the invention. FIG. 32A depicts how positions of nucleotides are calculated from the 5' and 3' termini of the sense and antisense strands when the sense strand has an extended loop with a tetraloop (i.e., the discontinuity is on the same side of the dsNA as the antisense strand). The Dicer cleavage site on the sense strand is shown (short, black arrow). FIG. 32B depicts how positions of nucleotides are calculated from the 5' and 3' termini of the sense and antisense strands when the antisense strand has an extended loop with a tetraloop (i.e., the discontinuity is on the same side of the dsRNA as the sense strand). The Dicer cleavage site on the antisense strand is shown (short, black arrow).

FIGS. 33A-33C depicts examples of dsNAs of the invention where a discontinuity exists on the same side of the dsNA molecule as the antisense strand, i.e., "nicked antisense dsNAs". FIG. 33A depicts examples of "nicked antisense" dsRNAs of the invention having a blunt end. FIG. 33B depicts examples of "nicked antisense" dsNAs of the invention having a 3' overhang of 2 nucleotides. FIG. 33C depicts examples of "nicked antisense" dsNAs of the invention having a 3' overhang of 4 nucleotides. When the dsNA is duplexed, a discontinuity exists where Dicer cleaves the antisense strand (black arrow).

FIGS. 34A-34C depicts examples of dsNAs of the invention where a discontinuity exists on the same side of the dsNA molecule as the sense strand, i.e., "nicked sense dsNAs". FIG. 34A depicts examples of "nicked sense" dsRNAs of the invention having a blunt end. FIG. 34B depicts examples of "nicked sense" dsNAs of the invention having a 3' overhang of 2 nucleotides. FIG. 34C depicts examples of "nicked sense" dsNAs of the invention having a 3' overhang of 4 nucleotides. When the dsNA is duplexed, a discontinuity exists where Dicer cleaves the sense strand (black arrow).

FIGS. 35A and 35B depict locations where modifications may be present in the dsNAs of the invention. FIG. 35A depicts a dsNA showing the positions of 2'-O-methyl modifications (shown by o) in Region C of the molecule. FIG. 35B depicts a dsNA in which the sense strand has an extended loop with a tetraloop (i.e., the discontinuity is on the same side of the dsNA as the antisense strand; shown by black arrow). In the dsNA depicted in FIG. 35B the 5' terminus of the sense strand may be phosphorylated (shown by a "p-"); the 5' terminus of the guide strand may be dephosphorylated; the antisense strand may be modified at positions 1, 2, and 3 from the 3' terminus of the antisense strand with 2'-O-methyl (shown by o); and the antisense strand may be modified at odd numbered positions starting at position 5 from the 3' terminus of the antisense strand with 2'-O-methyl (shown by o). In the dsNA depicted in FIG. 35B, the sense strand may contain deoxyribonucleotides (shown by •) at positions 11 and 12 from the 3' terminus of the antisense strand.

FIGS. 36A and 36B depict locations where modifications may be present in the dsNAs of the invention. FIG. 36A depicts a dsNA showing the positions of 2'-O-methyl modifications (shown by o) in Region C of the molecule. FIG. 36B depicts a dsNA in which the antisense strand has an extended loop with a tetraloop (i.e., the discontinuity is on the same side of the dsRNA as the sense strand; shown by black arrow). In the dsNA depicted in FIG.34B the 5' terminus of the sense strand may be phosphorylated (shown by a "p-"); the 5' terminus of the guide strand may be dephosphorylated; the antisense strand may be modified at positions 1, 2, and 3 from the 3' terminus of the antisense strand with 2'-O-methyl (shown by o); and the antisense strand may be modified at odd numbered positions starting at position 5 from the 3' terminus of the antisense strand with 2'-O-methyl (shown by o). In the dsNA depicted in FIG. 36B, the antisense strand may contain deoxyribonucleotides (shown by •) at positions 3 and 4 from the 5' terminus of the antisense strand.

FIG. 37 shows dsRNA constructs for use in experiments comparing the effect of a dsRNA with no tetraloop, the effect of a dsRNA with a tetraloop (i.e., UUCG), the effect of a dsRNA with a tetraloop (i.e., UUCG) in combination with a nick on the same side as the sense strand, the effect of a dsRNA with a tetraloop (i.e., UUCG) in combination with a nick on the same side as the antisense strand, and the effect of another tetraloop (i.e., GAAA) in combination with a nick on the same side as the antisense strand. The sequences in the dsRNAs are all based on human hypoxanthine phosphoribosyltransferase 1 (HPRT-1CC; NCBI database accession nos. NM-000194 and GI: 164518913).

FIG. 38 shows dsRNA constructs for use in experiments modifying the discontinuous antisense strand to determine their effects in the RNAi pathway at the step of Dicer processing and steps downstream of Dicer cleavage (e.g., Ago2 interaction, target recognition, Ago2 cleavage). The sequences in the dsRNAs are all based on human hypoxanthine phosphoribosyltransferase 1 (HPRT-1CC; NCBI database accession nos. NM-000194 and GI: 164518913).

FIG. 39 shows dsRNA constructs for use in experiments modifying the extended sense strand to determine their effects in the RNAi pathway at the step of Dicer processing and steps upstream of Dicer cleavage. The sequences in the dsRNAs are all based on human hypoxanthine phosphoribosyltransferase 1 (HPRT-1CC; NCBI database accession nos. NM-000194 and GI: 164518913).

FIG. 40 shows how the placement of a discontinuity defines cleavage products made by Dicer. The nicked dsNA structure directs a unique cleavage product with the advantage that a defined 21 base antisense strand is produced and loaded into RISC. By moving the nick, productions of other lengths of antisense strands are directed. Chemical modifications enforce the production of non-21 mer products.

FIG. 41 shows that DNA tetraloops and DNA ends control Dicer activity on the dsRNA. Double stranded RNA constructs are use in experiments comparing the effect of a dsRNA with no tetraloop, the effect of a dsRNA with a DNA tetraloop (i.e., d(GTTA)), the effect of a dsRNA with a DNA tetraloop (i.e., d(GTTA)) in combination with a nick on the same side as the sense strand, the effect of a dsRNA with a DNA tetraloop (i.e., d(GTTA)) in combination with a nick on the same side as the antisense strand, and the effect of another DNA tetraloop (i.e., d(TTTT)) in combination with a nick on the same side as the antisense strand. The sequences in the dsRNAs are all based on human hypoxanthine phosphoribosyltransferase 1 (HPRT-1CC; NCBI database accession nos. NM-000194 and GI: 164518913)

FIG. 42 shows a ligand conjugated design wherein the dsNA molecule of the disclosure comprising a nicked tetraloop and the ligand, for example GalNac, cholesterol, folate, mannose-6-phosphate.

FIG. 43 shows a ligand conjugated dsNA molecule of the disclosure comprising a 5'extension on the guide strand and comprising a ligand for example., GalNac , cholesterol, folate and mannose-6-phosphate.

FIG. 44 shows a ligand conjugated dsNA molecule of the disclosure comprising a 5' extension on the passenger strand and a ligand, for example, GalNac, cholesterol, folate and mannose-6-phosphate.

FIG. 45 shows the possible iterative combinations of a single stranded extension on the dsNA molecule, the dsNA molecule is also contemplated with one or two overhangs at the termini devoid of extensions.

FIG. 46 presents dsNAs of the disclosure.

FIG. 47 presents dsNAs of the disclosure.

## DETAILED DESCRIPTION

In an example of the disclosure, the ligand-conjugated nucleotide is on the stem or loop of the dsNA.

In an example of the disclosure, the ligand is selected from the group consisting of: a lipophile, a steroid, a protein, a vitamin, a carbohydrate, and a terpene.

In an example of the disclosure, the ligand is selected from the group consisting of: N-acetyl galactosamine, cholesterol, cholic acid, adamantine acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine), bile acid, PEG, folate, vitamin A, vitamin E, biotin, pyridoxal, a peptide, peptide mimic, mannose-6-phosphate, galactose, fructose, ribose, xylose, arabinose, lyxose, allose, altrose, gulose, iodose, glucose, talose, disaccharide, trisaccharide, tetrasaccharide, oligosaccharide, polysaccharide, an endosomolytic component, uvaol, hecigenin, diosgenin, triterpenesarsasapogenin, Friedelin, epifriedelanol-derivatized lithocholic acid, a cationic lipid, and an antibody.

In an example of the disclosure, the ligand comprises a ASGPr mimic which includes monomeric or monoantennary, biantennary and triantennary GalNACs. Suitable examples of GalNAc and GalNAc mimics are known in the art and can be found in Tables 2, 2a, 3 and 3a on pages 13-25 of WO 2015/006740. The GalNAc ligands disclosed in the tables can be used for conjugating to the dsNA molecules using suitable linkers and shall be considered to be within the scope.

In an example of the disclosure, the ligand comprises cholesterol.

In an example of the disclosure, the ligand comprises mannose-6-phosphate.

In an example of the disclosure, the ligand is a non-nucleic acid moiety.

In an example of the disclosure, the ligand is an organic compound.

In an example of the disclosure, the organic compound is a dye.

In an example of the disclosure, the organic compound is cholesterol.

In an example of the disclosure, the non-nucleic acid moiety comprises a detectable label.

An example of the disclosure also provides for ligand-modified oligonucleotides having strand lengths in the range of 15-85 nucleotides and possessing a single stranded extension positioned at the 5' end of the sense or antisense strand are effective RNA interference agents. Inclusion of one or more modified nucleotides, phosphate backbone modifications, and/or nucleotide analogs within the single stranded region of a single stranded extended dsNA can impart certain advantages to such a modified dsNA molecule, including, for example, enhanced efficacy (including enhanced potency and/or improved duration of effect), display of a recognition domain, and other attributes associated with a single

stranded nucleotide region.

An example of the disclosure provides for a double stranded nucleic acid (dsNA) comprising: an antisense strand comprising 15 to 85 nucleotides; a sense strand comprising 15 to 85 nucleotides; a duplex formed by said sense and antisense strands having a length of 15 to 35 base paired nucleotides; a 5' single stranded extension on at least one strand of said dsNA said extension having a length of 1 to 50 nucleotides; wherein said second strand is sufficiently complementary to a target mRNA along at least 15 nucleotides of said second strand length to reduce target gene expression when said double stranded nucleic acid is introduced into a mammal or a mammalian cell; and wherein said extension comprises a ligand-conjugated nucleotide.

In an example of the disclosure, the sense strand has a length range of 19-30 or 19-36 nucleotides.

It is further disclosed that the 3' extension has a length of 1-6, 1-10, 10-20 or 20-30 nucleotides.

It is further disclosed that the stem comprises a base paired region of at least 15 nucleotides and further comprises one or more mismatches.

It is further disclosed that the stem comprises a base paired region of at least 15 nucleotides and further comprises 1-10 consecutive or nonconsecutive mismatches.

In another embodiment, the number of ligand conjugated nucleotides is three or four.

It is further disclosed that the number of ligand conjugate nucletides is 1-3, 1-6, 1-10 or 1-20 nucleotides

It is further disclosed that the dsNA has at least two ligand-conjugated nucleotides, wherein the dsNA comprises 1-3, 1-6, 1-10 or 1-20 spacer nucleotides.

It is further disclosed that the dsNA has at least two ligand-conjugated nucleotides, wherein each ligand-conjugated nucleotide is separated from a second ligand-conjugated nucleotide by at least one spacer nucleotide.

It is further disclosed that the dsNA has at least two ligand-conjugated nucleotides, wherein the ligand-conjugated nucleotides are adjacent.

An example of the disclosure provides for a dsNA comprising a stem and a loop wherein the ligand conjugated nucleotide is on the stem.

The stem can comprise two, three, four or more ligand conjugated nucleotides. At least one of the ligands or each of the ligands can be conjugated to a nucleotide of the stem through the 2' hydroxyl on the ribose of the nucleotide. The ligand conjugated to a nucleotide of the stem can be GalNAc, mannose-6-phosphate or a combination thereof. The stem can comprise three, four or more ligand conjugated nucleotides, each of the ligands being GalNAc. More than one ligand, for example, two, three, or four can be connected to a single nucleotide of the stem. More than one GalNAc ligand, for example, two, three, or four, can be connected to a single nucleotide of the stem.

In example of the disclosure, the ligand is conjugated to a sugar and/or base of the nucleotide.

In an example of the disclosure, the ligand is attached to said nucleotide via a linker.

In an example of the disclosure, the linker is a releasable linker.

In an example of the disclosure, the linker is 5-90 atoms in length.

In an example of the disclosure, the linker comprises a triazole ring and an amide functional group.

In an example of the disclosure, the linker has at least one bio-labile bond, wherein the bio-labile bond connects the base of a nucleotide of said dsNA to the linker.

In an example of the disclosure, the linker has at least one bio-labile bond, wherein the bio-labile bond connects the sugar of a nucleotide of said dsNA to the linker.

In an example of the disclosure, the linker has at least one bio-labile bond, wherein the bio-labile bond connects the ligand to the linker.

In an example of the disclosure, the linker has at least one bio-labile bond, wherein the bio-labile bond is not at the terminus of the linker.

In an example of the disclosure, the linker has at least three bio-labile bonds, wherein the first bio-labile bond connects the ligand with the linker, the second bio-labile bond is between the first and the third bio-labile bond and wherein the third bio-labile bond connects the ligand and linker.

In an example of the disclosure, the linker has at least one bio-labile bond, wherein the bio-labile bond is a hydrolysable ester bond.

In an example of the disclosure, the 5' single-stranded extension has a length of 1-10, 10-20 or 20-30 nucleotides.

In an example of the disclosure, the dsNA comprises at least one modified nucleotide.

In an example of the disclosure, the nucleotides of said dsNA are selected from the group consisting of: ribonucleotides, deoxyribonucleotides, abasic nucleotides, inverted abasic nucleotides, sugar modified nucleotides, backbone modified nucleotides, nucleotide analogs and non-nucleoside analogs.

In an example of the disclosure, the nucleotide is a locked nucleic acid (LNA) or an unlocked nucleic acid (UNA).

In an example of the disclosure, the at least one strand of said dsNA comprises a 3' extension.

In an example of the disclosure, the the 3' overhang has a length of 1-2, 1-4 or 1-6 nucleotides.

In an example of the disclosure, the at least one nucleotide of said 3' extension comprises a sugar and/or backbone modification.

In an example of the disclosure, the at least one nucleotide comprises a sugar and/or a backbone modification.

In an example of the disclosure, the one or more nucleotide comprises a sugar modification selected from the group consisting of: 2'-*O*-methyl, 2'-methoxyethoxy, 2'-fluoro, 2'- allyl, 2'-*O*-[2-(methylamino)-2-oxoethyl], 4'-thio, 4'-$CH_2$-*O*-2'-bridge, 4'-$(CH_2)_2$-*O*-2'-bridge, 2'-LNA, 2'-amino, and 2'-*O*-(N-methylcarbamate) modification.

In an example of the disclosure, the dsNA comprises a backbone modification selected from the group consisting of: phosphonate, phosphorothioate, phosphotriester, methylphosphonate, unlocked nucleic acid (UNA), locked nucleic acid (LNA), morpholino, SATE (S-acyl-2-thioethyl) modified phosphate, BMEG (Isobutyryl Mercapto Ethyl Glycol) modified phosphate and bicyclic furanose analog modification.

In an example of the disclosure, the dsNA comprises a region containing at least one phosphorothioate linkage.

In an example of the disclosure, the dsNA comprises a region containing 1-5, 1-10 or 1-20 phosphorothioate linkages.

In an example of the disclosure the dsNA comprises a region containing at least two consecutive phosphorothioate linkages.

In an example of the disclosure, the dsNA comprises a nucleic acid analog selected from the group consisting of: hypoxanthine (I), xanthine (X), 3β-D-ribofuranosyl-(2,6-diaminopyrimidine) (K), 3β-D-ribofuranosyl-(1-methyl-pyrazolo[4,3-d]pyrimidine-5,7(4H,6H)-dione) (P), iso-cytosine (iso-C), iso-guanine (iso-G), 1-β-D-ribofuranosyl-(5-nitroindole), 1-β-D-ribofuranosyl-(3-nitropyrrole), 5-bromouracil, 2-aminopurine, 4-thio-dT, 7-(2-thienyl)-imidazo[4,5-b]pyridine (Ds), pyrrole-2-carbaldehyde (Pa), 2-amino-6-(2-thienyl)purine (S), 2-oxopyridine (Y), difluorotolyl, 4-fluoro-6-methylbenzimidazole, 4-methylbenzimidazole, 3-methyl isocarbostyrilyl, 5-methyl isocarbostyrilyl, 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenzyl, tetracenyl and pentacenyl.

In an example of the disclosure, the sense and antisense strands are aligned for maximal complementarity in the duplex region.

In an example of the disclosure, the dsNA is a Dicer cleavage substrate.

In an example of the disclosure, the dsNA is not a Dicer cleavage substrate.

In an example of the disclosure, 1-30 consecutive 3' terminal nucleotides of said antisense strand are unpaired with said sense strand, forming a 3' single stranded extension of 1-30 nucleotides.

In an example of the disclosure, the 3' single stranded extension has a length of 1-10, 10-20 or 20-30 nucleotides.

In an example of the disclosure, the duplex comprises one or more nucleotides selected from the group consisting of: ribonucleotides, deoxyribonucleotides, modified nucleotides, abasic nucleotides, inverted abasic nucleotides, nucleotide analogs and combinations thereof.

In an example of the disclosure, the 3' extension comprises one or more nucleotides selected from the group consisting of: ribonucleotides, deoxyribonucleotides, modified nucleotides, abasic nucleotides, inverted abasic nucleotides, nucleotide analogs and combinations thereof.

In an example of the disclosure, the 5' extension comprises one or more nucleotides selected from the group consisting of: ribonucleotides, deoxyribonucleotides, modified nucleotides, abasic nucleotides, inverted abasic nucleotides, and nucleotide analogs.

In an example of the disclosure, the 5' single-stranded extension comprises at least 10 deoxyribonucleotides.

In an example of the disclosure, the 5' single-stranded extension comprises at least one phosphorothioate modified nucleotide.

In an example of the disclosure, the 5' single-stranded extension comprises 1-5, 1-10 or 1-20 phosphorothioate linkages.

In an example of the disclosure, the 5' single stranded extension comprises at least two consecutive phosphorothioate linkages.

In an example of the disclosure, the ligand-conjugated nucleotide has increased binding affinity to asialoglycoprotein-receptor (ASGPr) as compared to a dsNA lacking a ligand- conjugated nucleotide.

In an example of the disclosure, the ligand-conjugated nucleotide has increased cellular targeting as compared to a dsNA lacking a ligand- conjugated nucleotide.

In an example of the disclosure, the ligand- conjugated nucleotide has increased cellular uptake as compared to a dsNA lacking a ligand- conjugated nucleotide.

In an example of the disclosure, the ligand-conjugated nucleotide has enhanced delivery as compared to a dsNA lacking a ligand-conjugated nucleotide.

In an example of the disclosure, the ligand-conjugated nucleotide has increased stability as compared to a dsNA lacking a ligand-conjugated nucleotide.

In an example of the disclosure, the dsNA comprising a ligand-conjugated nucleotide has increased tracking as compared to a dsNA lacking a ligand-conjugated nucleotide.

In an example of the disclosure, the dsNA comprising a ligand-conjugated nucleotide has increased binding

affinity for a target as compared to a dsNA lacking a ligand-conjugated nucleotide.

In an example of the disclosure, the dsNA comprising a ligand-conjugated nucleotide has decreased immunogenicity as compared to a dsNA lacking a ligand-conjugated nucleotide.

In an example of the disclosure, the dsNA comprising a ligand-conjugated nucleotide has improved pharmacokinetic properties as compared to a dsNA lacking a ligand-conjugated nucleotide.

In an example of the disclosure, the dsNA comprising a ligand-conjugated nucleotide has improved biodistribution properties as compared to a dsNA lacking a ligand-conjugated nucleotide.

In an example of the disclosure, the 5' terminus of the sense and/or antisense strand contains an unmodified phosphate.

In an example of the disclosure, the the 5' terminus of the sense and/or antisense strand contains a chemically modified phosphate.

In an example of the disclosure, the 3'extension comprises a phosphorothioate modification.

In an example of the disclosure, the duplex comprises an abasic nucleotide.

In an example of the disclosure, the sense strand consists of up to 100% chemically modified nucleotides.

In an example of the disclosure, the antisense strand consists of up to 100% chemically modified nucleotides.

In an example of the disclosure, the sense strand is resistant to nuclease degradation in the absence of a modified nucleotide.

In an example of the disclosure, the antisense strand is resistant to nuclease degradation in the absence of a modified nucleotide.

In an example of the disclosure, the dsNA comprises more than one species of ligand.

In an example of the disclosure, the dsNA comprises a GalNac-conjugated nucleotide and a cholesterol-conjugated nucleotide.

In an example of the disclosure, the dsNA comprises a GalNac-conjugated nucleotide and a mannose-6-phosphate-conjugated nucleotide.

In an example of the disclosure, the dsNA comprises a GalNac-conjugated nucleotide, a mannose-6-phosphate-conjugated nucleotide, a folate-conjugated nucleotide and/or a cholesterol conjugated-nucleotide.

In an example of the disclosure, the dsNA molecule comprises a nucleotide conjugated to at least two ligands.

In an example of the disclosure, the dsNA comprises more than one species of linker.

In an example of the disclosure, the linker has at least one chemical bond that is cleavable under physiological conditions.

In an example of the disclosure, the linker has at least one ester bond that is hydrolysable under physiological conditions.

In an example of the disclosure, the linker has at least one amide bond that is hydrolysable under physiological conditions.

In an example of the disclosure, the linker is cleaved to release at least one of the ligands conjugated to a nucleotide of the dsNA molecule.

In an example of the disclosure, the ligand is selected from the group consisting of a lipophile, a steroid, a protein, a vitamin, a carbohydrate, and terpene.

In an example of the disclosure, the ligand is selected from the group consisting of N-acetyl galactosamine, cholesterol, cholic acid, adamantine acetic acid,1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine), bile acid, PEG, folate, vitamin A, vitamin E, biotin, pyridoxal, a peptide, peptide mimic, mannose-6-phosphate, galactose, fructose, ribose, xylose, arabinose, lyxose, allose, altrose, gulose, iodose, glucose, talose, disaccharide, trisaccharide, tetrasaccharide, oligosaccharide, polysaccharide, an endosomolytic component, uvaol, hecigenin, diosgenin, triterpenesarsasapogenin, Friedelin, epifriedelanol-derivatized lithocholic acid, a cationic lipid, and an antibody.

In an example of the disclosure, the number of ligand conjugated nucleotides is 1-3, 1-6, 1-10 or 1-20.

In an example of the disclosure, the dsNA has at least two ligand-conjugated nucleotides; wherein the dsNA comprises 1-3, 1-6, 1-10 or 1-20 spacer nucleotides.

In an example of the disclosure, the dsNA molecules have three ligand conjugated nucleotides on the 5' extension.

In an example of the disclosure, the dsNA has at least two ligand-conjugated nucleotides, wherein each ligand-conjugated nucleotide is separated from a second ligand-conjugated nucleotide by at least one spacer nucleotide.

In an example of the disclosure, the dsNA has at least two ligand-conjugated nucleotides, wherein the ligand-conjugated nucleotides are adjacent.

In an example of the disclosure, the dsNA molecules have four ligand conjugated nucleotides on the 5' extension.

In an example of the disclosure, the dsNA molecules have the ligand conjugated nucleotides on the 5' extension is separated from one another by spacers.

In an example of the disclosure, the dsNA molecules have the ligand conjugated nucleotides on the 5' extension is separated from one another by one spacer.

In an example of the disclosure, the dsNA molecules have the ligand conjugated nucleotides on the 5' extension is separated from one another by two or more spacers.

In an example of the disclosure, the dsNA molecules have triantennary GalNAc conjugated to the nucleotides on the 5' extension.

In In an example of the disclosure, the dsNA molecules have biantennary GalNAc conjugated to the nucleotides on the 5' extension.

In an example of the disclosure, the dsNA molecules have monoantennary GalNAc conjugated to the nucleotides on the 5' extension.

In an example of the disclosure, the the dsNA has at least two ligand-conjugated nucleotides, wherein each ligand-conjugated nucleotide is separated from a second ligand-conjugated nucleotide by at least one spacer nucleotide.

In an example of the disclosure, the ligand is conjugated to a sugar and/or base of said nucleotide.

In an example of the disclosure, the dsNA comprises a structure represented by formula I, wherein:

$$(S_2)_c\text{-}[[X_a\text{-}L\text{-}M]\text{-}(S1)_z]_n\text{-}(S_2)_b\text{-}D$$

$P_n$

26

O

E

(Formula I)

M is a nucleotide;
X is a ligand;
L is an optional linker joining M and X;
S1 and S2 are nucleotide spacers;
P is a unit formed of ligand-modified nucleotide (XLM) and S1 nucleotide spacer;
*a* is independently 1-4 for each P;
*n* is 1-10; each z is independently 0-10 for each P;
each of *b* and *c* is 0-35, wherein one of *b* and *c* is 0;
E is a 5' extension comprising 1 to 50 nucleotides M; and
O is the dsNA molecule

In an example of the disclosure, the dsNA has the structure represented by the following formula II:

Formula II

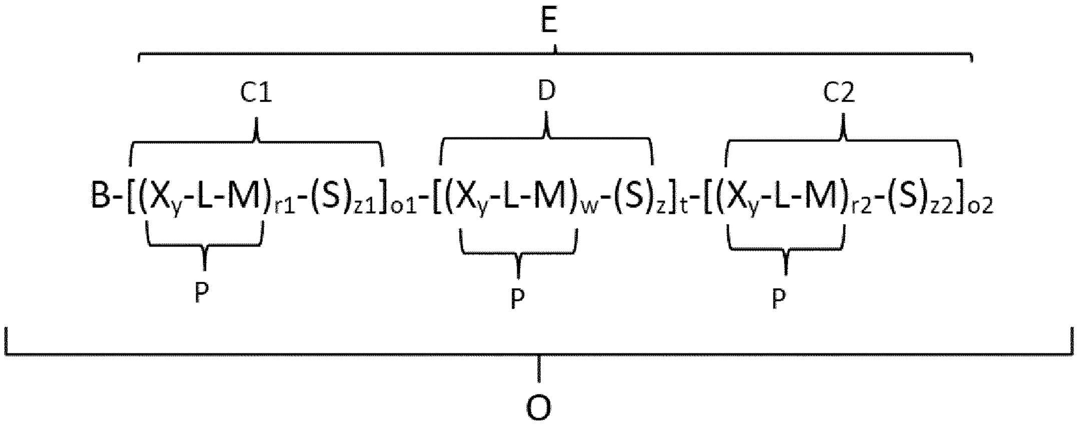

wherein:

B is a duplex formed between the sense and antisense strands;

M is a nucleotide;

X is a ligand, and the value of *y* is 0 to 4;

L is an optional linker;

O is the dsNA comprising B and E

S is an optional spacer nucleotide;

and the value of each of *o1* and *o2* is independently 1-20;

and the value of each of *t* is independently 3-8;

C1 and C2 form a stem duplex and D is the loop;

E is the stem-loop, including each of D, C1, and C2;

each P is collectively X-L-M, representing each ligand-modified nucleotide;

*r1* and *r2* are each independently 0 to 20; and each w is independently 0 to 8; and

*o1* is the sum of *z1* + *r1; o2* is the sum of z2 + *r2; t* is the sum of *z* + *w.*

In an example of the disclosure, *r1, r2* and *w* can each independently be zero, provided that at least one of *r1, o1,r2,o2, t* and w is greater than zero.

In an example of the disclosure, the sense strand comprises a stem-loop structure (E), wherein y = 1-3, o1 = 6-8, r1 = 0, o2 = 6-8, r2 = 0, w = 1-4 and t= 4.

In an example of the disclosure, the sense strand comprises a stem-loop structure (E), wherein y=1-3, o1=6-8, r1=1-4, o2=6-8, r2=0, w=0 and t=4.

In an example of the disclosure, the sense strand comprises a stem-loop structure (E), wherein y=1-3, o1=6-8, r1=0, o2=6-8, r2=1-4, w=0 and t=4.

In an example of the disclosure, the linker (L) comprises a backbone selected from the group consisting of: an alkyl, an alkenyl, an aromatic, a heterocycl, a substituted alkyl, and a substituted alkenyl, and wherein one of more methylenes can be interrupted or terminated by one or more of O, S, S(O), SO2, N, NH, $NH_2$, NH(CO), P, $P(O_4)$, C≡C or C(O), and PEG.

In an example of the disclosure, (O) in comprises a plurality of nucleotides each independently selected from

the group consisting of ribonucleotides, deoxyribonucleotides, modified nucleotides, abasic nucleotides, inverted abasic nucleotides, nucleotide analogs, and non-nucleoside analogs.

In an example of the disclosure, the dsNA comprises a phosphate backbone selected from the group consisting of: phosphonate, phosphorothioates, phosphotriester, methylphosphonate, locked nucleic acid (LNA), unlocked nucleic acid (UNA), a morpholino, SATE (S-acyl-2-thioethyl) modified phosphate and BMEG (Isobutyryl Mercapto Ethyl Glycol) modified phosphate and a bicyclic furanose analog.

In an example of the disclosure, the linker L comprises the formula III:

(Formula III)

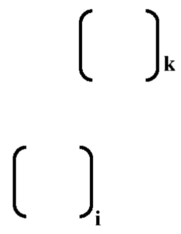

where *i* may be 1-10 and *k* may be 1-10 carbon atoms.

In an example of the disclosure, the linker comprises the structure of formula IV; wherein n is 1-40.

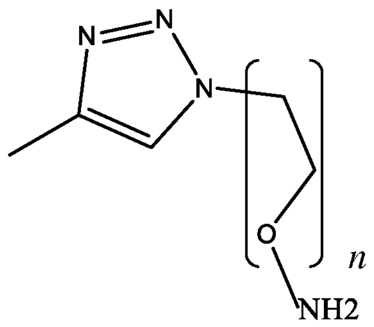

(Formula IV)

In an example of the disclosure, O comprises at least one modified nucleotide comprising a modified base and/or a modified sugar moiety.

In an example of the disclosure, the the modified sugar moiety is selected from the group consisting of: 2'-hydroxyl, 3'-hydroxyl, 2'-O-methyl, 2'-methoxyethoxy, 2'-fluoro, 2'-allyl, 2'-O[2-(methylamino)-2-oxoethyl], 4'-thio, 4'-CH2-O-2'-bridge, 4'-(CH2)2-O-2'-bridge, 2'-LNA, 2'-amino, and 2'-O--(N-methylcarbamate).

In an example of the disclosure, the dsNA comprises a base analog selected from the group consisting of: hypoxanthine (I), xanthine (X), 3β-D-ribofuranosyl-(2,6-diaminopyrimidine) (K), 3β-D-ribofuranosyl-(1-methyl-pyrazolo[4,3-d]pyrimidine-5,7(4H,6H)-dione) (P), iso-cytosine (iso-C), iso-guanine (iso-G), 1-β-D-ribofuranosyl-(5-nitroindole), 1-β-D-ribofuranosyl-(3-nitropyrrole), 5-bromouracil, 2-aminopurine, 4-thio-dT, 7-(2-thienyl)-imidazo[4,5-b]pyridine (Ds), pyrrole-2-carbaldehyde (Pa), 2-amino-6-(2-thienyl)purine (S), 2-oxopyridine (Y), difluorotolyl, 4-fluoro-6-methylbenzimidazole, 4-methylbenzimidazole, 3-methyl isocarbostyrilyl, 5-methyl isocarbostyrilyl, 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenzyl, tetracenyl and pentacenyl.

In an example of the disclosure in formula II, the D is covalently bonded to P at the 5' end of said sense strand or said antisense strand wherein *b* and *c* are each zero; *a* is 1; n is 4, and z is 2, 2, 2, and 1, respectively for each unit of P.

In an example of the disclosure in formula II, the D is covalently bonded to P at the 5' end of said sense strand or said antisense strand wherein a is 1, n is 4, and each of b, c and z are zero.

In an example of the disclosure in formula II, the D is covalently bonded to P at the 5' end of said sense strand or said antisense strand wherein a is 1; n is 3; each of c and z is zero; and b is 1-11.

In an example of the disclosure in formula II, P increases the cellular uptake of the dsNA (O) as compared to a double stranded nucleic acid (dsNA) that does not contain P.

In an example of the disclosure, in formula II the sense and antisense strands form a duplex (D) of at least 21 base pairs.

In an example of the disclosure, the linker (L) comprises a backbone selected from the group consisting of: an alkyl, an alkenyl, an aromatic, a heterocycl, a substituted alkyl, and a substituted alkenyl, wherein one of more methylenes can be interrupted or terminated by one or more of O, S, S(O), SO2, C=C or C(O), and PEG.

The present disclosure also provides for a method for producing a dsNA of the invention, the method comprising: providing a plurality of nucleotides selected from the group consisting of DNA nucleotides, RNA nucleotides, abasic nucleotides, inverted abasic nucleotides, nucleotide analogs or modified nucleotides; wherein one or more nucleotides are conjugated to a ligand; and synthesizing a dsNA comprising nucleotides that are not conjugated to a ligand and nucleotides that are conjugated ligand, wherein the nucleotides of said dsNA are in a predetermined pattern, thereby producing the dsNA.

In an example of the disclosure the linker (L) comprises the structure of formula V:

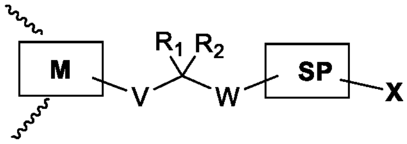

(Formula V)

wherein:

M is a nucleotide or non-nucleotide molecule that is part of the dsNA;

V is attached to M at the 2' or 3' position, at the phosphate, or at the nucleobase of the nucleotide when M is a nucleotide, or V is attached to M through a chemical bond containing either oxygen, nitrogen, carbon or sulphur atoms when M is a non-nucleotide molecule;

V and W are each independently O, S, NH, or NHR' ,

R' is H, C1-C6 alkanyl, C1-C6 alkenyl or aryl;

R1 and R2 are each independently H, C1-C6 alkanyl, C1-C6 alkenyl, C1-C6 alkynyl, or halogenated C1-C6 alkanyl, and where R2 is aryl, heteroaryl, cyclohexane, t-Butane, adamantane or triazole and one of R2 may be substituted with one of

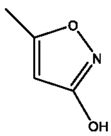 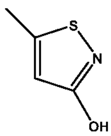 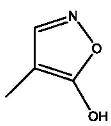 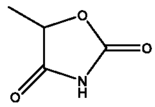 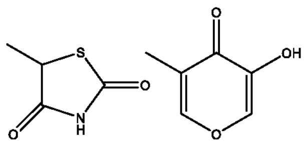

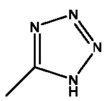 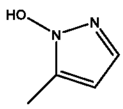 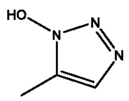 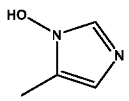 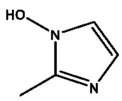 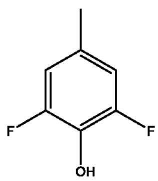

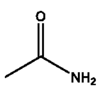 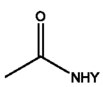 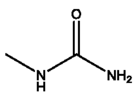 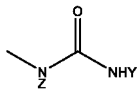 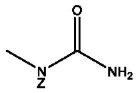 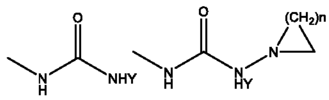

or R1 and R2 are each independently aryl, heteroaryls either of which may be substituted with one or more of H, Me, OMe, halide, -NHX where X=H, C1-C6 or wherein R1 and R2 combine to form an optionally substituted C3-C7 heterocycles including:

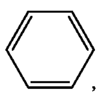, 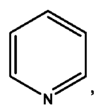, 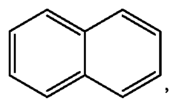,

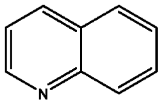, 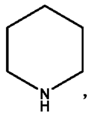, 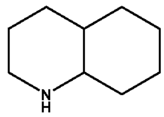,

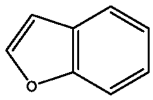, 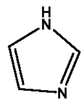, 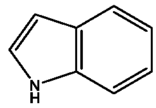

and

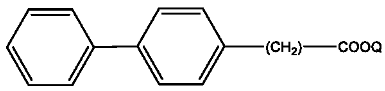

Q is H or a physiologically acceptable salt, C1-C6 alkanyl, C1-C6 alkenyl, C1-C6 alkynyl, aryl, heteroaryl, (CH2)m-aryl or (CH2)m-heteroaryl where m is 1-10, and where n=0-10 and any of the above ring compounds may be substituted with one to three independently selected Cl, F, CF3, C1-C8 alkoxy, NO2, C1-C6 alkanyl, C1-C6 alkenyl, aryl or OY, (C=O)OY, NY2 or C(=O)NHY where Y is H, C1-C6 alkanyl, C1-C6 alkenyl or aryl;

SP is a spacer wherein said spacer comprises an alkyl, an alkenyl, an alkynyl, an aromatic, a heterocycle, a substituted alkyl, a substituted alkenyl, and a substituted alkynyl, wherein one or more methylenes can be interrupted or terminated by one or more of P(O)H, P(O2), $P(O_4)$, polyethylenegylcol (PEG), OY, S, S(OY), SO2(Y), (C=O)OY, NY2, NH, NH-(C=OY) where Y is H, C1-C6 alkanyl, C1-C6 alkenyl or aryl, including

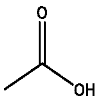 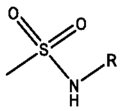 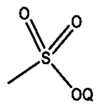 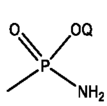 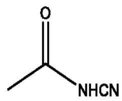 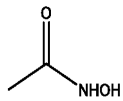 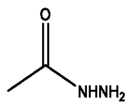

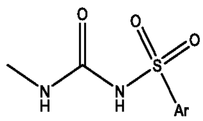 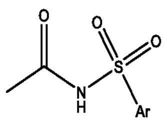 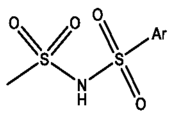 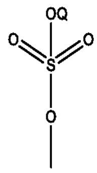

X is a ligand selected from the group consisting of: GalNAc, D-Mannose, L-galactose, D-arabinose, L-fucose, polyols or formula Z shown below

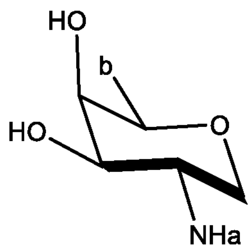

Formula Z

where b is CF3, where a is CF3, alkyl, an alkenyl, an alkynyl, an aromatic, a heterocycle, a substituted alkyl, a substituted alkenyl, and a substituted alkynyl, and

b is one or more methylenes interrupted or terminated by one or more of P(O)H, P(O2), $P(O_4)$, polyethylenegylcol (PEG), Oc', S, S(Oc'), SO2(c'), (C=O)Oc', NY2, NH, NH-(C=Oc') where c' is H, C1-C6 alkanyl, C1-C6 alkenyl or aryl.

In an example of the disclosure, linker (L) comprises the structure of formula VI:

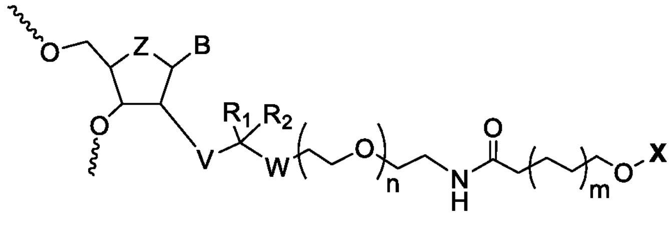

Formula VI

wherein:

B is a nucleobase or H;
Z is O, S, N(R1), or CH2;
V and W are each independently O, S, NH;
R1 and R2 are each independently H, or optionally substituted C1-6 alkyl, or
R1 and R2 combine to form an optionally substituted C3-7 heterocyclyl;
m and n are each independently 1 - 20.

X is a ligand selected from the group consisting of: GalNAc, Folate, Mannose-6-phosphate and Cholesterol.In an example of the disclosure, the linker (L) comprises structure of formula XIII:

Formula XIII

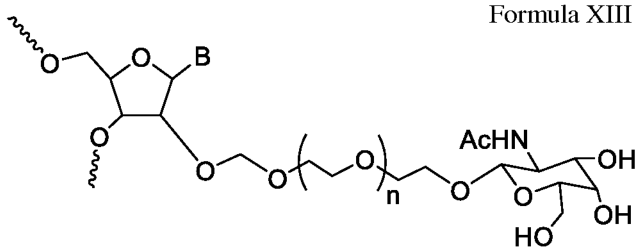

wherein:

B is a nucleobase or H; and
n is 1-20.

In an example of the disclosure, the dsNA molecule comprises at least one ligand modified nucleotide incorporated via solid phase oligonucleotide synthesis using a phosphoramidite synthon having the structure of formula IX:

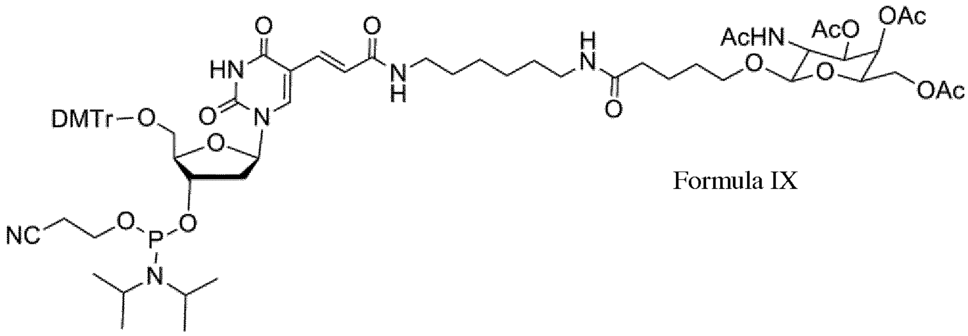

Formula IX

In an example of the disclosure, the dsNA molecule comprises at least one ligand modified nucleotide incorporated via solid phase oligonucleotide synthesis using a phosphoramidite synthon having the structure of formula X:

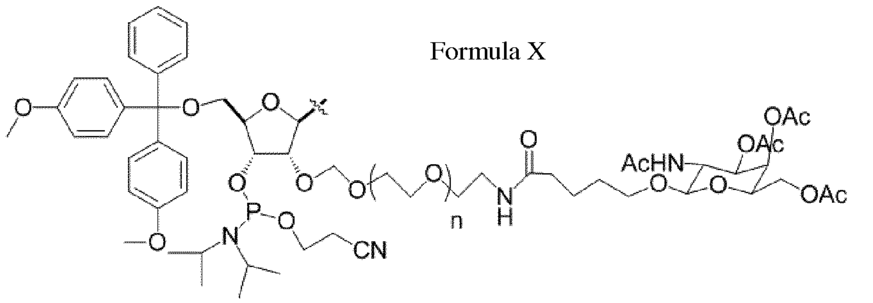

Formula X

There is also disclosed a ligand-conjugated phosphoramidite synthon comprising the structure of formula XI:

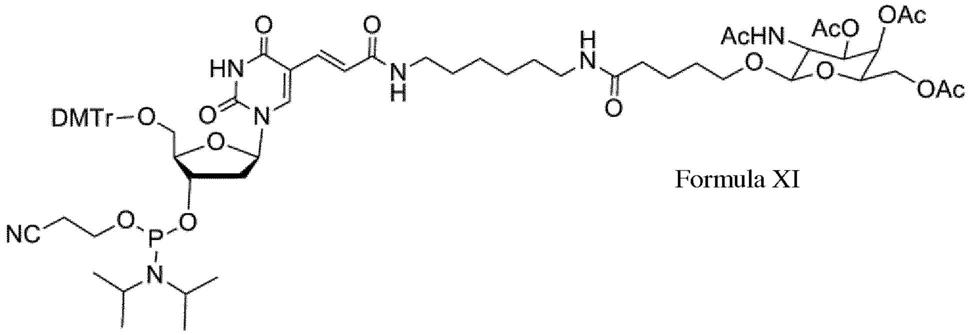

Formula XI

wherein said ligand-conjugated phosphoramidite synthon is used for synthesizing the dsNA of claim 1.

There is also disclosed a ligand-conjugated phosphoramidite synthon comprising the structure of formula XII

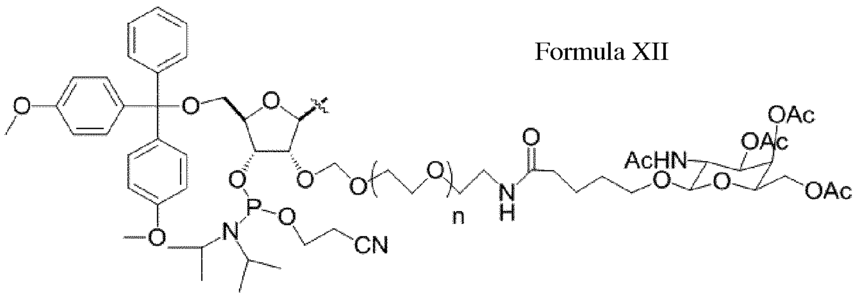

Formula XII

wherein said ligand-conjugated phosphoramidite synthon is used for synthesizing the dsNA of claim 1.

In one example, the dsNA molecule comprises at least one ligand modified nucleotide consisting of a said linker (L) incorporated via solid phase oligonucleotide synthesis.

There is also disclosed a ligand-conjugated phosphoramidite synthon comprising the structure of formula XIII.

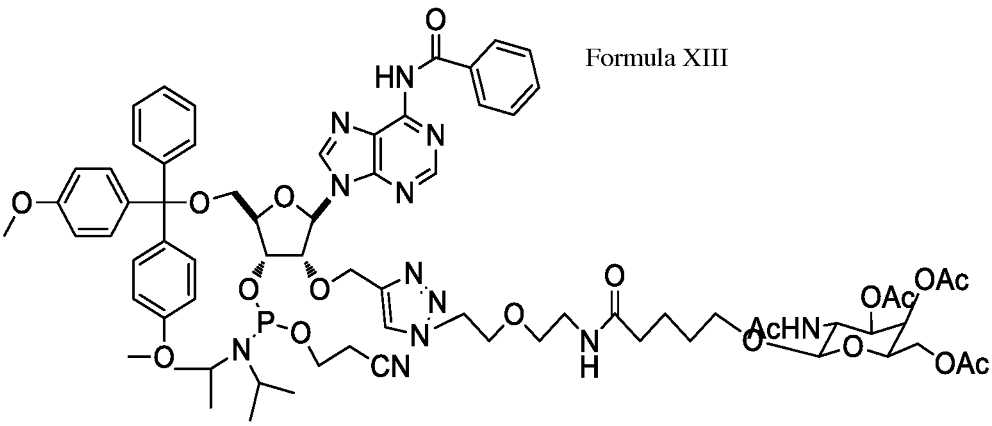

Formula XIII

There is also disclosed a ligand-conjugated phosphoramidite synthon comprising the structure of formula XIV.

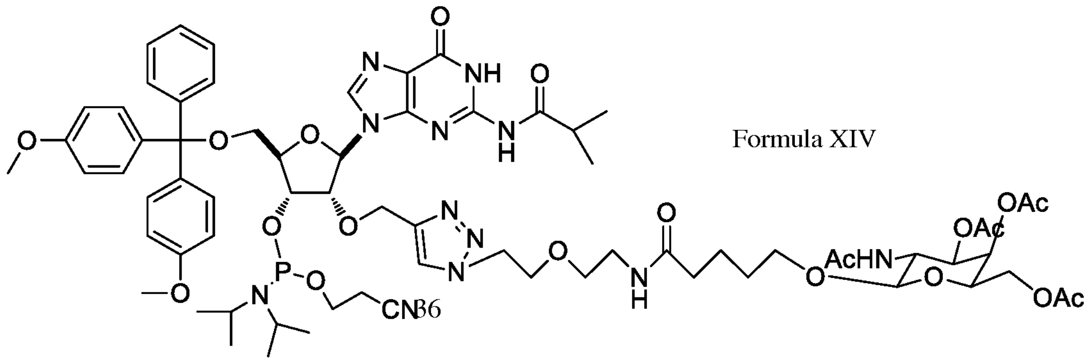

Formula XIV

There is also disclosed a ligand-conjugated phosphoramidite synthon comprising the structure of formula XV.

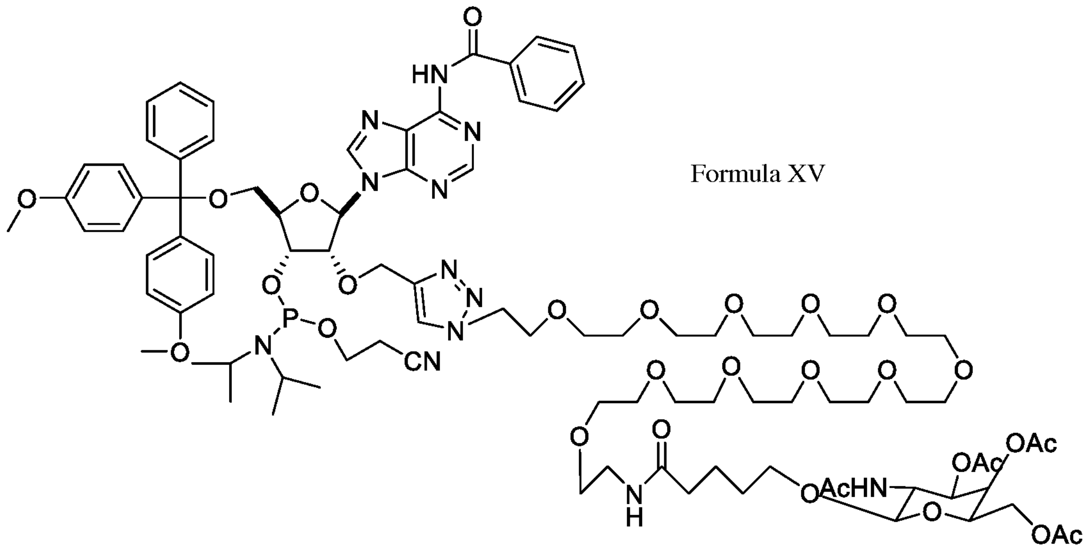

Formula XV

There is also disclosed a ligand-conjugated phosphoramidite synthon comprising the structure of formula XVI

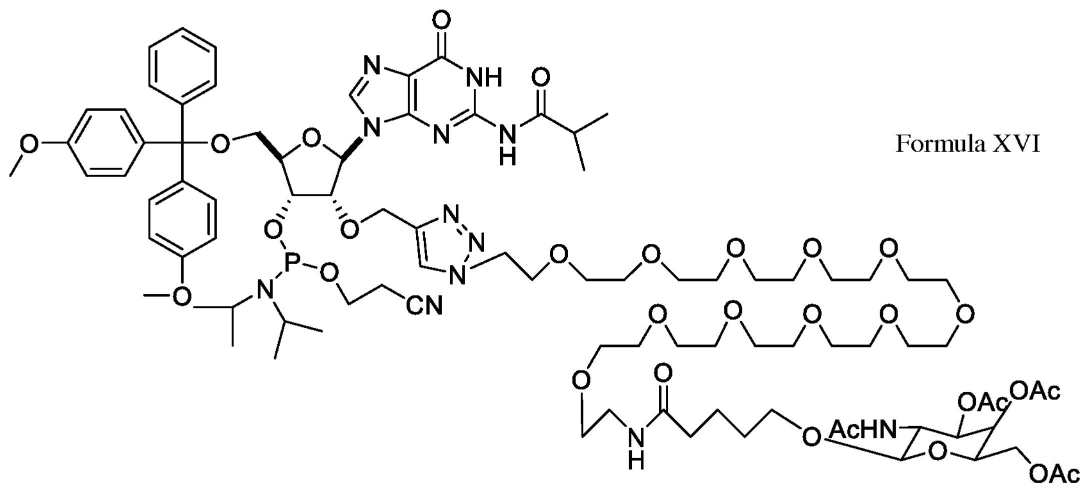

Formula XVI

An example of the disclosure also provides for a method for reducing expression of a target gene in a cell, comprising contacting a cell with a dsNA of the invention in an amount effective to reduce expression of a target gene in the cell.

An example of the disclosure also provides for a method for reducing expression of a target gene in a mammal, comprising administering to said mammal a dsNA of the invention in an amount effective to reduce expression of a target gene in the mammal.

An example of the disclosure also provides for a pharmaceutical composition for reducing expression of a target gene in a cell of a subject, said composition comprising the dsNA of the invention in an amount effective to reduce expression of the target gene in said cell or animal, and a pharmaceutically acceptable carrier.

An example of the disclosure also provides for a kit comprising a dsNA as disclosed herein and instructions for its use.

An example of the disclosure also provides for a method for increasing the potency and half-life of a dsNA by conjugation of a ligand to a nucleotide of said dsNA, wherein said ligand is selected from the group consisting of cholesterol, mannose-6-phophate, GalNAc and folate.

The invention provides compositions and methods for reducing expression of a target gene in a cell, involving contacting a cell with an isolated double stranded nucleic acid in an amount effective to reduce expression of a target gene in a cell. In addition to the tetraloop containing dsNAs of the invention, the application also provides dsNAs that possess a single stranded nucleotide region either at the 5' terminus of the antisense strand, the 3' terminus of the sense strand, the 5' terminus of the sense strand or the 3' end of the antisense strand or combinations thereof wherein the extension is conjugated at least one ligand as defined herein. These structures are effective RNA interference agents (see Figures 25-30 and associated Examples). In most embodiments, the single stranded extension comprises at least one modified nucleotide and/or phosphate back bone modification. Surprisingly, as demonstrated herein, single-stranded extended Dicer-substrate siRNAs (DsiRNAs) or non Dicer-substrate siRNAs either conjugated to a ligand or in the absence of a ligand are effective RNA inhibitory agents when compared to corresponding DsiRNAs or non-Dicer substrate siRNAs.

The ability to conjugate ligands such as GalNAc to the dsNA molecules at the 5' extension or to the tetraloop region of the dsNA molecules enables delivery of the dsNA molecules to the region of interest which for example would be hepatocytes for a ligand such as GalNAc. Other ligands that are contemplated for the conjugation to the dsNA molecules comprise a ASGPr mimic which includes monomeric or monoantennary, biantennary and triantennary GalNAcs. Suitable examples of GalNAc and GalNAc mimics are known in the art and can be found in Tables 2, 2a, 3 and 3a on pages 13-25 of WO 2015/006740. The GalNAc ligands disclosed in the tables can be used for conjugating to the dsNA molecules of the invention using suitable linkers.

The surprising discovery that single stranded extended DsiRNA agents provides motivation to generate functional DsiRNAs that have an extended region that allows for attachment of additional and/or distinct functional groups, inclusion/patterning of stabilizing modifications (e.g., PS-NA moieties) or other forms of modifications capable of adding further functionality and/or enhancing, e.g., pharmacokinetics, pharmacodynamics or bio-distribution of such extended DsiRNAs, as compared to DsiRNAs that do not contain such single stranded DNA or RNA or DNA/RNA hybrid extended domains.

The advantages of a DsiRNA with an extended 5' guide strand, extended 3' passenger strand, extended 5' passenger strand or an extended 3' guide strand that has higher activity of a post-Dicer-processed as compared to siRNA agent of similar length is emphasized by the results presented herein. The ability to extend either the 5' guide strand, the 3' passenger strand, the 5' passenger strand or the 3' guide strand of DsiRNA agents without observing a corresponding reduction in RNA silencing activity allows for certain functional groups to be attached to such agents that could not be added to non-extended molecules without interfering with RNA silencing activity due to tighter configurations.

The surprising discovery that single stranded extended non-Dicer substrates reduce expression of a target gene as well as non-extended dsNAs allows for the generation of functional non-Dicer substrates that have an extended region that allows for attachment of additional and/or distinct functional groups, inclusion/patterning of stabilizing modifications (e.g., PS-NA moieties) or other forms of modifications such modifications or attached moieties adding further functionality and/or enhancing, e.g., pharmacokinetics, pharmacodynamics or bio-distribution of such extended dsNA, as compared to dsRNA agents of corresponding length that do not contain such single DNA or RNA or DNA/RNA hybrid extended domains.

The advantage provided by the newfound ability to lengthen either the 5' guide strand, the 3' passenger strand, or the 5' passenger strand of non-Dicer substrates containing dsNA duplexes while retaining activity of a post-Dicer-processed siRNA agent at levels greater than dsRNA duplexes of similar length is emphasized by the results presented herein. The ability to extend either the 5' guide strand, the 3' passenger strand, or 5' passenger strand of non-Dicer substrates without observing a corresponding reduction in RNA silencing activity can also allow for certain functional groups to be attached to such agents that would otherwise not be possible, because of the ability of such functional groups to interfere with RNA silencing activity when present in tighter configurations

Additionally, single stranded extended DsiRNA agents may include a third short (1-16 nucleotides in length) oligonucleotide which base-pairs with the single stranded region of single stranded extended DsiRNAs, e.g., which base-pairs to a guide 5' single stranded extended region. The third oligo advantageously functions: (a) to stabilize the single

stranded extension and (b) to provide an independent entity to which a targeting molecule (or other active agent) could be attached, and which could then be joined to the single-stranded extended DsiRNA via annealing (versus direct attachment of the targeting molecule to the single stranded extended DsiRNA).

The tetraloop provides enhanced stability to the oligomer including increased nuclease resistance as compared with an oligomer lacking a tetraloop. The presence of a nick in the antisense strand permits the oligomer to function as both a siRNA molecule and as a Dicer substrate; depending on the length of the duplex. The oligomer includes a precut antisense strand due to the presence of the nick, providing a cleavable Dicer substrate and/or a Dicer binding substrate. The tetraloop structure can be covalently linked to a ligand at various locations within the structure, including stem and loop regions. Surprisingly, addition of a ligand to the does not affect the tetraloop structure.. In particular, oligomers containing the tetraloop and the nick where the tetraloop nucleotides were each conjugated to ligand surprisingly maintained their function as siRNA molecules (see, e.g., Example 22-24). The presence of a nick in the oligomer provides for utilization of a variety of chemical modifications on the antisense strand. The tetraloop structure disclosed herein does not require Dicer cleavage for RNAi activity.

The surprising discovery that single stranded non-Dicer substrates comprising a tetraloop do not exhibit decreases in efficacy allows for the generation of non-Dicer substrates that remain effective while providing greater spacing for, e.g., attachment of non-Dicer substrates to additional and/or distinct functional groups, inclusion/patterning of stabilizing modifications (e.g., PS-NA moieties) or other forms of modifications capable of adding further functionality and/or enhancing, e.g., pharmacokinetics, pharmacodynamics or bio-distribution of such agents, as compared to dsRNA agents of corresponding length that do not contain such single stranded DNA-extended domains.

In one embodiment of the current invention, the tetraloop is located at the 5' end of the sense strand. In another embodiment the tetraloop is located at the 3'end of the sense strand. In another embodiment the tetraloop is located at the 5' end of the antisense strand. In another embodiment the tetraloop is located at the 3'end of the antisense strand. The tetraloop, located at any position on the dsNA molecule, 5' or 3' end, sense or antisense strand, can be readily conjugated to one or more ligands such as GalNAc, Mannose-6-phosphate, cholesterol and folate. Provided herein are methods of dsNA synthesis, methods of conjugation, assays for the activity of a dsNA comprising a tetraloop located at the 5' end of the sense strand or at the 5' end of a dsNA to a ligand and the antisense strand. The disclosed methods of conjugation and synthesis can be modified to generate dsNA molecules comprising a tetraloop at the 3' end of the sense strand or at the 3' end of the antisense strand.

## DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale and Marham, The Harper Collins Dictionary of Biology (1991). As used herein, the following terms have the meanings ascribed to them below, unless specified otherwise.

As used herein, the term "nucleic acid" refers to deoxyribonucleotides, ribonucleotides, or modified nucleotides, and polymers thereof in single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which, in certain cases, are metabolized in a manner similar to the reference nucleotides. Examples of such analogs include, without limitation, phosphorothioates, phosphoramidates, methylphosphonates, chiral-methyl phosphonates, 2-O-methyl ribonucleotides, peptidenucleic acids (PNAs).

As used herein, "nucleotide" is used as recognized in the art to include those with natural bases (standard), and modified bases well known in the art. Such bases are generally located at the 1' position of a nucleotide sugar moiety. Nucleotides generally comprise a base; sugar and a phosphate group. Nucleotides also include ribonucleotides, deoxyribonucleotides, abasic nucleotides, inverted abasic nucleotides and nucleotide analogs. The nucleotides can be unmodified or modified at the sugar, phosphate and/or base moiety, (also referred to interchangeably as nucleotide analogs, modified nucleotides, non-natural nucleotides, non-standard nucleotides and other; see, e.g., Usman and McSwiggen, supra; Eckstein, et al., International PCT Publication No. WO 92/07065; Usman et al, International PCT Publication No. WO 93/15187; Uhlman and Peyman, supra). Some of the known examples of modified nucleotides include a base analog selected from the group consisting of: hypoxanthine (I), xanthine (X), 3β-D-ribofuranosyl-(2,6-diaminopyrimidine) (K), 3β-D-ribofuranosyl-(1-methyl-pyrazolo[4,3-d]pyrimidine-5,7(4H,6H)-dione) (P), iso-cytosine (iso-C), iso-guanine (iso-G), 1-β-D-ribofuranosyl-(5-nitroindole), 1-β-D-ribofuranosyl-(3-nitropyrrole), 5-bromouracil, 2-aminopurine, 4-thio-dT, 7-(2-thienyl)-imidazo[4,5-b]pyridine (Ds), pyrrole-2-carbaldehyde (Pa), 2-amino-6-(2-thienyl)purine (S), 2-oxopyridine (Y), difluorotolyl, 4-fluoro-6-methylbenzimidazole, 4-methylbenzimidazole, 3-methyl isocarbostyrilyl, 5-methyl isocarbostyrilyl, 3-methyl-7-propynyl isocarbostyrilyl, 7-azaindolyl, 6-methyl-7-azaindolyl, imidizopyridinyl,

9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenzyl, tetracenyl and pentacenyl. Some of the common modifications to the sugar moiety are selected from the group comprising of 2'-O-methyl, 2'-methoxyethoxy, 2'-fluoro, 2'- allyl, 2'-O-[2-(methylamino)-2-oxoethyl], 4'-thio, 4'-Ch2-O-2'-bridge, 4'-(Ch2)2-O-2'-bridge, 2'-LNA, 2'-amino, or 2'-O-(N-methylcarbamate) modifications Other examples of modified nucleic acid bases are known in the art, for example, as summarized by Limbach, et al, Nucleic Acids Res. 22:2183, 1994. Some of the non-limiting examples of base modifications that can be introduced into nucleic acid molecules include, hypoxanthine, purine, pyridin-4-one, pyridin-2-one, phenyl, pseudouracil, 2,4,6-trimethoxy benzene, 3-methyl uracil, dihydrouridine, naphthyl, aminophenyl, 5-alkylcytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), propyne, and others (Burgin, et al., Biochemistry 35:14090, 1996; Uhlman and Peyman, supra). By "modified bases" in this aspect is meant nucleotide bases other than adenine, guanine, cytosine and uracil at 1' position or their equivalents.

As used herein, the term "monomer" is used interchangeably with "nucleotide".

As used herein, a "double-stranded nucleic acid" or "dsNA" is a molecule comprising two oligonucleotide strands which form a duplex. A dsNA may contain ribonucleotides, deoxyribonucleotides, modified nucleotides, and combinations thereof. The dsNA molecules can be either a Dicer substrate or a Non-Dicer substrate. In embodiments where the dsNA molecule is a dicer substrate, the dsNA molecule is referred to as a dicer substrate NA (dsiNA). In embodiments where the dsiNA comprises a plurality of RNA, it is referred to as a dicer substrate siRNA (DsiRNA). DsiRNA molecules comprise both DNA and RNA molecules. DsiRNA is a subset of DsiNA which is a subset of dsNA. Therefore the term dsNA is inclusive of dsiNA, DNA duplex, DNA/RNA duplex, RNA duplex and DsiRNA The double-stranded NAs of the instant invention include substrates for proteins and protein complexes in the RNA interference pathway, e.g., Dicer and RISC. Exemplary structure of dsNAs of the invention are shown in FIG. 1, B. In certain embodiments the dsNAs comprise an RNA duplex in a region that is capable of functioning as a Dicer substrate siRNA (DsiRNA) and a single stranded region, which is located at a position 5' of the projected Dicer cleavage site of the second strand of the DsiRNA/DNA agent. In another embodiment the dsNA comprises an RNA duplex in a region that is capable of functioning as a Dicer substrate siRNA (DsiRNA) and a single stranded region, which is located at a position 3' of the projected Dicer cleavage site of the first strand of the DsiRNA/DNA agent. In another embodiment, the dsNA comprises an RNA duplex that is a Dicer substrate siRNA (DsiRNA) and a single stranded region comprising at least one modified nucleotide and/or phosphate backbone modification, which is located at a position 3' of the projected Dicer cleavage site of the second strand of the DsiRNA/DNA agent. In alternative embodiments, the instant invention provides a dsNA that comprises an RNA duplex that is a Dicer substrate siRNA (DsiRNA) and a single stranded region comprising at least one modified nucleotide and/or phosphate backbone modification, which is located at a position 5' of the projected Dicer cleavage site of the first strand of the DsiRNA/DNA agent.

As used herein, the term "oligomer" is used interchangeably with "double stranded nucleic acid" (dsNA). An oligomer (dsNA molecule) comprises monomers (nucleotides).

As used herein, the term "plurality" means a number greater than two, for instance, 3, 4, 5, 10, 15, 20, 25, 30, 35, 40, 45, 50 or more.

As used herein, the term "spacer" means a nucleotide that separates the ligand modified nucleotides from one another.

As used herein, the term "releasable linker" refers to a linker that is capable of undergoing cleavage of the bond that connects a ligand with a nucleotide under physiological conditions inside a cell or animal or under in vivo conditions. A releasable linker includes a bio-labile linker and upon cleavage releases the ligand from the dsNA molecule. The linker moieties are also capable of cleavage under various conditions. Conditions suitable for cleavage can include but not limited to pH, UV irradiation, enzymatic activity, temperature, hydrolysis, elimination and substitution reactions, and also thermodynamic properties of the linkage. The releasable linkers can be photolabile in nature wherein linkers are cleaved under particular UV wavelengths, dsNAs of the invention containing invention containing photolabile linkers can be used to deliver compounds to a target cell or tissue of interest and can be subsequently released in the presence of a UV source.

As used herein, the term "biodegradable linker" refers to a nucleic acid or a non nucleic acid molecule that is designed to be biodegradable and serves as a linker to connect one molecule with another molecule, for example a bioactive molecule. The stability of the biodegradable linker can be modulated by using various combinations of nucleotides, deoxyribonucleotides, and chemically modified nucleotides, for example 2'-O-methyl, 2'-O-fluoro, 2'-amino, 2'-O-amino, 2'-C-allyl, 2'-O-allyl and other 2'-modified or base modified nucleotides. The biodegradable linker can be a dimer, trimer, tetramer or longer, for example an oligonucleotide of about 2, 3, 4, 5, 6, 7, 8, 9, 10, 11, 12, 13, 14, 15, 16, 17, 18, 19 or 20 nucleotides in length or can comprise a single nucleotide in length with a phosphorus based linkage. The biodegradable linker can also comprise nucleic acid backbone, nucleic acid sugar, or nucleic acid base modifications.

As used herein, the term "biodegradable" refers to degradation in a biological system, for example enzymatic degradation or chemical degradation

As used herein, the term "detectable label' refers to any suitable label known in the art and include but is not

limited to radiolabels, chemiluminescent, and fluorescent labels. In many instances, the label is of a type which does not substantially alter the cellular uptake of the labeled nucleic acid molecules.

As used herein, the term "dye" refers to a label which can be detected either by chemiluminescence or fluorescence, commonly used examples include but not limited to TAMRA, fluorescein, cy3 and cy5 etc..The signal(s) generated by the label(s) may be measured by any number of ways, including visually (e.g., by microscopy) or fluorescent activated cell sorting (FACS). In any event, measurement of the signal(s) generated label(s) may be used to determine (a) the number or percentage of cells which have taken up the label(s), (b) the amount of one or more label taken up by individual cells or groups of cells, or (c) both (a) and (b).

As used herein, the term "half-life" refers to the time that it takes for a molecule, for an example a dsNA, a metabolite, a pharmaceutical, a radioactive nucleotide, a therapeutic molecule, a dye or a signaling molecule to use half of its pharmacologic, physiologic, or radiologic activity or half of its intensity.

As used herein, the terms "ligand modified nucleotide" or "ligand conjugated nucleotide" or "ligand containing nucleotide" are used interchangeably to denote a nucleotide to which a ligand is attached, attachment being to either the sugar or the base or to the terminal phosphate through the usage of a linker.

As used herein, a "DsiRNA" or Dicer substrate siRNA agent is a molecule comprising two oligonucleotide strands which form a duplex. A DsiRNA agent may contain ribonucleotides, deoxyribonucleotides, modified nucleotides, and combinations thereof. DsiRNA agents are to be processed by the Dicer enzyme of the RNAi interference pathway.

In certain embodiments, the DsiRNAs of the invention can possess deoxyribonucleotide residues at sites immediately adjacent to the projected Dicer enzyme cleavage site(s). For example, in all the DsiRNAs shown in FIG. 2 and in the sixth, seventh, eighth, ninth, tenth, eleventh, and twelfth DsiRNAs shown in FIG. 3, deoxyribonucleotides can be found (starting at the 5' terminal residue of the first strand as position 1) at position 24 and sites 3' of position 24 (e.g., 24, 25, 26, 27, 28, 29, 30, etc.). Deoxyribonucleotides may also be on the second strand commencing at the nucleotide that is complementary to position 20 of the first strand, and also at positions on the second strand that are located in the 5' direction of this nucleotide. Thus, certain effective DsiRNAs of the invention possess only 19 duplexed ribonucleotides prior to commencement of introduction of deoxyribonucleotides within the first strand, second strand, and/or both strands of such DsiRNAs.

As used herein, "duplex" refers to a double helical structure formed by the interaction of two single stranded nucleic acids. According to the present invention, a duplex may contain first and second strands which are sense and antisense, or a target and an antisense strand of the dsNA. A duplex is typically formed by the pairwise hydrogen bonding of bases, i.e., "base pairing", between two single stranded nucleic acids which are oriented antiparallel with respect to each other. As used herein, the term "duplex" refers regions of the first and second strands which align such that if the aligned bases of the strands are complementary, they may Watson-Crick base pair. The term "duplex" does not include one or more single stranded nucleotides which comprise a 5' or 3' terminal single stranded nucleotide(s). In some embodiments the duplex includes a region of aligned first and second strands which may be fully (100%) base paired and a region of aligned first and second strands which contains 1, 2, 3, 4, 5 or more unpaired bases, as long as the first strand 5' terminal nucleotide of the duplex and the first strand 3' terminal nucleotide of the duplex are Watson-Crick base paired with a corresponding nucleotide of the second strand. As used herein, "fully duplexed" refers to all nucleotides in between the paired 5' and 3' terminal nucleotides being base-paired. As used herein, "substantially duplexed" refers to a duplex between the strands wherein there are 1, 2, 3, 4, 5, 6, 7, 8, 9, 10, 11, 12, 13, 14, 15, 16, 17, 18, 19, 20, 21, 22, 23, 24, 25, 30, 40, 50 unpaired base pair(s) (consecutive or nonconsecutive) between the between the 5' terminal and 3' terminal nucleotides of the first strand.

In the context of "substantially duplexed," "consecutive" "consecutive" as it refers to duplexed nucleotides means a stretch of more than two adjacent nucleotides on a sense or an antisense strand that do not form Watson-Crick base pairing with the other strand.

In the context of "substantially duplexed," "nonconsecutive" as it refers to duplexed nucleotides means mismatches on the sense or antisense strand that are separated by more than one nucleotide from each other.

Pairing in duplexes generally occurs by Watson-Crick base pairing, e.g., guanine (G) forms a base pair with cytosine (C) in DNA and RNA (thus, the cognate nucleotide of a guanine deoxyribonucleotide is a cytosine deoxyribonucleotide, and vice versa), adenine (A) forms a base pair with thymine (T) in DNA, and adenine (A) forms a base pair with uracil (U) in RNA. Conditions under which base pairs can form include physiological or biologically relevant conditions (e.g., intracellular: pH 7.2, 140 mM potassium ion; extracellular pH 7.4, 145 mM sodium ion). Furthermore, duplexes are stabilized by stacking interactions between adjacent nucleotides. As used herein, a duplex may be established or maintained by base pairing or by stacking interactions. A duplex is formed by two complementary nucleic acid strands, which may be substantially complementary or fully complementary (see below).

As used herein, "corresponds to" or "corresponding to" refers to first and second strand bases that are aligned in a duplex such that the nucleotide residue of the second strand aligns with the residue of the first strand, when first strand position 1 is base paired with a nucleotide of said second strand such that said second strand comprises a 3' single stranded overhang of 1-6 nucleotides in length. "Corresponds to" does not require pairing via formation of a

Watson-Crick base pair, but rather includes both aligned and unpaired first strand/second strand nucleotides as well as aligned and base paired first strand/second strand nucleotides.

By "complementary" or "complementarity" is meant that a nucleic acid can form hydrogen bond(s) with another nucleic acid sequence by either traditional Watson-Crick or Hoogsteen base pairing. In reference to the nucleic acid molecules of the present disclosure, the binding free energy for a nucleic acid molecule with its complementary sequence is sufficient to allow the relevant function of the nucleic acid to proceed, e.g., RNAi activity. Determination of binding free energies for nucleic acid molecules is well known in the art (see, e.g., Turner, et al., CSH Symp. Quant. Biol. LII, pp. 123-133, 1987; Frier, et al., Proc. Nat. Acad. Sci. USA 83:9373-9377, 1986; Turner, et al., J. Am. Chem. Soc. 109:3783-3785, 1987). A percent complementarity indicates the percentage of contiguous residues in a nucleic acid molecule that can form hydrogen bonds (e.g., Watson-Crick base pairing) with a second nucleic acid sequence (e.g., 5, 6, 7, 8, 9, or 10 nucleotides out of a total of 10 nucleotides in the first oligonucleotide being base paired to a second nucleic acid sequence having 10 nucleotides represents 50%, 60%, 70%, 80%, 90%, and 100% complementary, respectively). To determine that a percent complementarity is of at least a certain percentage, the percentage of contiguous residues in a nucleic acid molecule that can form hydrogen bonds (e.g., Watson-Crick base pairing) with a second nucleic acid sequence is calculated and rounded to the nearest whole number (e.g., 12, 13, 14, 15, 16, or 17 nucleotides out of a total of 23 nucleotides in the first oligonucleotide being based paired to a second nucleic acid sequence having 23 nucleotides represents 52%, 57%, 61%, 65%, 70%, and 74%, respectively; and has at least 50%, 50%, 60%, 60%, 70%, and 70% complementarity, respectively). As used herein, "substantially complementary" refers to complementarity between the strands such that they are capable of hybridizing under biological conditions. Substantially complementary sequences have 60%, 70%, 80%, 90%, 95%, or even 100% complementarity. Additionally, techniques to determine if two strands are capable of hybridizing under biological conditions by examining their nucleotide sequences are well known in the art.

As used herein, the "3' region" with respect to the antisense strand refers to the consecutive nucleotides of the antisense strand that are 3' distal (on the antisense strand) to the nucleotide of the antisense strand that aligns with corresponding positions 1-19, 1-20 or 1-21 of the sense strand. To avoid doubt, the "3' region", when referring to the antisense strand, is meant to encompass antisense nucleotides in a duplex formed between the antisense strand and its cognate target RNA 3' distal to (on the antisense strand which correspond to nucleotides on the target RNA that are 5' distal to) the projected Argonaute 2 (Ago2) cut site.

As used herein, "synthon" means a nucleotide or non-nucleotide moiety that can be used in solid phase synthesis methods to produce a modified DNA, RNA or DNA/RNA molecule having a particular sequence of interest. In certain embodiments, a synthon is a nucleotide or non-nucleotide moiety conjugated to a ligand, for example, GalNac, mannose-6-Phosphate, cholesterol or folate, wherein the conjugated nucleotide or non-nucleotide moiety can be used in solid phase synthesis to produce a nucleic acid molecule of interest. In certain embodiments, a synthon further comprises a linker through which the nucleotide or non-nucleotide moiety is conjugated to the ligand.

The term "alkyl" includes saturated aliphatic groups, including straight-chain alkyl groups (e.g., methyl, ethyl, propyl, butyl, pentyl, hexyl, heptyl, octyl, nonyl, decyl, etc.), branched-chain alkyl groups (isopropyl, tert-butyl, isobutyl, etc.), cycloalkyl (alicyclic) groups (cyclopropyl, cyclopentyl, cyclohexyl, cycloheptyl, cyclooctyl), alkyl substituted cycloalkyl groups, and cycloalkyl substituted alkyl groups. In certain embodiments, a straight chain or branched chain alkyl has 6 or fewer carbon atoms in its backbone (e.g., C1-C6 for straight chain, C3-C6 for branched chain), and more preferably 4 or fewer. Likewise, preferred cycloalkyls have from 3-8 carbon atoms in their ring structure, and more preferably have 5 or 6 carbons in the ring structure. The term C1-C6 includes alkyl groups containing 1 to 6 carbon atoms.

Moreover, unless otherwise specified, the term alkyl includes both "unsubstituted alkyls" and "substituted alkyls," the latter of which refers to alkyl moieties having independently selected substituents replacing a hydrogen on one or more carbons of the hydrocarbon backbone. Such substituents can include, for example, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Cycloalkyls can be further substituted, e.g., with the substituents described above. An "alkylaryl" or an "arylalkyl" moiety is an alkyl substituted with an aryl (e.g., phenylmethyl (benzyl)). The term "alkyl" also includes the side chains of natural and unnatural amino acids. The term "n-alkyl" means a straight chain (i.e., unbranched) unsubstituted alkyl group.

The term "alkenyl" includes unsaturated aliphatic groups analogous in length and possible substitution to the alkyls described above, but that contain at least one double bond. For example, the term "alkenyl" includes straight-chain alkenyl groups (e.g., ethylenyl, propenyl, butenyl, pentenyl, hexenyl, heptenyl, octenyl, nonenyl, decenyl, etc.), branched-chain alkenyl groups, cycloalkenyl (alicyclic) groups (cyclopropenyl, cyclopentenyl, cyclohexenyl, cycloheptenyl, cyclooctenyl), alkyl or alkenyl substituted cycloalkenyl groups, and cycloalkyl or cycloalkenyl substituted alkenyl

groups. In certain embodiments, a straight chain or branched chain alkenyl group has 6 or fewer carbon atoms in its backbone (e.g., C2-C6 for straight chain, C3-C6 for branched chain). Likewise, cycloalkenyl groups may have from 3-8 carbon atoms in their ring structure, and more preferably have 5 or 6 carbons in the ring structure. The term C2-C6 includes alkenyl groups containing 2 to 6 carbon atoms.

Moreover, unless otherwise specified, the term alkenyl includes both "unsubstituted alkenyls" and "substituted alkenyls," the latter of which refers to alkenyl moieties having independently selected substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

The term "alkynyl" includes unsaturated aliphatic groups analogous in length and possible substitution to the alkyls described above, but which contain at least one triple bond. For example, the term "alkynyl" includes straight-chain alkynyl groups (e.g., ethynyl, propynyl, butynyl, pentynyl, hexynyl, heptynyl, octynyl, nonynyl, decynyl, etc.), branched-chain alkynyl groups, and cycloalkyl or cycloalkenyl substituted alkynyl groups. In certain embodiments, a straight chain or branched chain alkynyl group has 6 or fewer carbon atoms in its backbone (e.g., C2-C6 for straight chain, C3-C6 for branched chain). The term C2-C6 includes alkynyl groups containing 2 to 6 carbon atoms.

Moreover, unless otherwise specified, the term alkynyl includes both "unsubstituted alkynyls" and "substituted alkynyls," the latter of which refers to alkynyl moieties having independently selected substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Unless the number of carbons is otherwise specified, "lower alkyl" as used herein means an alkyl group, as defined above, but having from one to five carbon atoms in its backbone structure. "Lower alkenyl" and "lower alkynyl" have chain lengths of, for example, 2-5 carbon atoms.

The term "alkoxy" includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with independently selected groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, etc.

The term "heteroatom" includes atoms of any element other than carbon or hydrogen. Preferred heteroatoms are nitrogen, oxygen, sulfur and phosphorus.

The term "hydroxy" or "hydroxyl" includes groups with an -OH or-O- (with an appropriate counterion).

The term "halogen" includes fluorine, bromine, chlorine, iodine, etc. The term "perhalogenated" generally refers to a moiety wherein all hydrogens are replaced by halogen atoms.

The term "substituted" includes independently selected substituents which can be placed on the moiety and which allow the molecule to perform its intended function. Examples of substituents include alkyl, alkenyl, alkynyl, aryl, (CR'R")0-3NR'R", (CR'R")0-3CN, NO2, halogen, (CR'R")0-3C(halogen)3, (CR'R")0-3CH(halogen)2, (CR'R")0-3CH2(halogen), (CR'R")0-3CONR'R", (CR'R")0-3S(O)1-2NR' R", (CR'R")0-3CHO, (CR'R")0-30(CR'R")0-3H, (CR'R")0-3 S(O)0-2R', (CR'R")0-3O(CR'R")0-3H, (CR'R")0-3COR', (CR'R")0-3CO2R', or (CR'R")0-30R' groups; wherein each R' and R" are each independently hydrogen, a C1-C5 alkyl, C2-C5 alkenyl, C2-C5 alkynyl, or aryl group, or R' and R" taken together are a benzylidene group or a -(CH2)2O(CH2)2- group.

The term "amine" or "amino" includes compounds or moieties in which a nitrogen atom is covalently bonded to at least one carbon or heteroatom. The term "alkyl amino" includes groups and compounds wherein the nitrogen is bound to at least one additional alkyl group. The term "dialkyl amino" includes groups wherein the nitrogen atom is bound to at least two additional alkyl groups.

The term "ether" includes compounds or moieties which contain an oxygen bonded to two different carbon atoms or heteroatoms. For example, the term includes "alkoxyalkyl," which refers to an alkyl, alkenyl, or alkynyl group covalently bonded to an oxygen atom which is covalently bonded to another alkyl group.

The first and second strands of the agents of the invention (sense and antisense oligonucleotides) are not required to be completely complementary in the duplexed region. In one embodiment, the RNA sequence of the antisense strand contains one or more mismatches (1, 2, 3, 4, 5, 6, consecutive or nonconsecutive), i.e., mismatched with respect to the duplexed sense strand of the isolated double stranded nucleic acid according to the invention, contains one or more (1, 2, 3, 4 or 5, 6,7,8,9,10,11,12,13,14,15,16,17,18,19,20 consecutive or nonconsecutive), modified nucleotides (base analog)s. In an exemplary embodiment, such mismatches occur within the 3' region, as defined hereinabove, of the RNA sequence of the antisense strand. In one aspect, two, three, four or five mismatches or modified nucleotides with base analogs are incorporated within the RNA sequence of the antisense strand that is 3' in the antisense strand of the projected Ago2 cleavage site of the target RNA sequence when the target RNA sequence is hybridized.

The use of mismatches or decreased thermodynamic stability (specifically at or near the 3'-terminal residues of sense/5'-terminal residues of the antisense region of siRNAs) has been proposed to facilitate or favor entry of the antisense strand into RISC (Schwarz et al., 2003; Khvorova et al., 2003), presumably by affecting some rate-limiting unwinding steps that occur with entry of the siRNA into RISC. Thus, terminal base composition has been included in design algorithms for selecting active 21mer siRNA duplexes (Ui-Tei et al., 2004; Reynolds et al., 2004).

Inclusion of such mismatches within the DsiRNA agents of the instant invention can allow such agents to exert inhibitory effects that resemble those of naturally-occurring miRNAs, and optionally can be directed against not only naturally-occurring miRNA target RNAs (e.g., 3' UTR regions of target transcripts) but also against RNA sequences for which no naturally-occurring antagonistic miRNA is known to exist. For example, DsiRNAs of the invention possessing mismatched base pairs which are designed to resemble and/or function as miRNAs can be synthesized to target repetitive sequences within genes/transcripts that might not be targeted by naturally-occurring miRNAs (e.g., repeat sequences within the Notch protein can be targeted, where individual repeats within Notch can differ from one another (e.g., be degenerate) at the nucleic acid level, but which can be effectively targeted via a miRNA mechanism that allows for mismatch(es) yet also allows for a more promiscuous inhibitory effect than a corresponding, perfect match siRNA agent). In such embodiments, target RNA cleavage may or may not be necessary for the mismatch-containing DsiRNA agent to exert an inhibitory effect.

A double stranded nucleic acid molecule may comprise or functions as microRNA (miRNA). By "microRNA" or "miRNA" is meant a small double stranded RNA that regulates the expression of target messenger RNAs either by mRNA cleavage, translational repression/inhibition or heterochromatic silencing (see for example Ambros, 2004, Nature, 431, 350-355; Bartel, 2004, Cell, 116, 281-297; Cullen, 2004, Virus Research., 102, 3-9; He et al., 2004, Nat. Rev. Genet., 5, 522-531; and Ying et al., 2004, Gene, 342, 25-28). In one example, the microRNA has partial complementarity (i.e., less than 100% complementarity) between the sense strand (e.g., first strand) or sense region and the antisense strand (e.g., second strand) or antisense region of the miRNA molecule or between the antisense strand or antisense region of the miRNA and a corresponding target nucleic acid molecule (e.g., target mRNA). For example, partial complementarity can include various mismatches or non-base paired nucleotides (e.g., 1, 2, 3, 4, 5 or more mismatches or non-based paired nucleotides, such as nucleotide bulges) within the double stranded nucleic acid molecule structure, which can result in bulges, loops, or overhangs that result between the sense strand or sense region and the antisense strand or antisense region of the miRNA or between the antisense strand or antisense region of the miRNA and a corresponding target nucleic acid molecule.

Single-stranded nucleic acids that base pair over a number of bases are said to "hybridize." Hybridization is typically determined under physiological or biologically relevant conditions (e.g., intracellular: pH 7.2, 140 mM potassium ion; extracellular pH 7.4, 145 mM sodium ion). Hybridization conditions generally contain a monovalent cation and biologically acceptable buffer and may or may not contain a divalent cation, complex anions, e.g. gluconate from potassium gluconate, uncharged species such as sucrose, and inert polymers to reduce the activity of water in the sample, e.g. PEG. Such conditions include conditions under which base pairs can form.

Hybridization is measured by the temperature required to dissociate single stranded nucleic acids forming a duplex, i.e., (the melting temperature; Tm). Hybridization conditions are also conditions under which base pairs can form. Various conditions of stringency can be used to determine hybridization (see, e.g., Wahl, G. M. and S. L. Berger (1987) Methods Enzymol. 152:399; Kimmel, A. R. (1987) Methods Enzymol. 152:507). Stringent temperature conditions will ordinarily include temperatures of at least about 30° C., more preferably of at least about 37° C., and most preferably of at least about 42° C. The hybridization temperature for hybrids anticipated to be less than 50 base pairs in length should be 5-10° C less than the melting temperature (Tm) of the hybrid, where Tm is determined according to the following equations. For hybrids less than 18 base pairs in length, Tm (° C) =2(# of A+T bases)+4(# of G+C bases). For hybrids between 18 and 49 base pairs in length, Tm (° C) =81.5+16.6(log 10[Na+])+0.41 (% G+C)-(600/N), where N is the number of bases in the hybrid, and [Na+] is the concentration of sodium ions in the hybridization buffer ([Na+] for 1xSSC=0.165 M). For example hybridization determination buffer is shown in Table 1

Table 1

| Chemical Name | Final Conc. | Vendor | Cat # | Lot # | m.w/Stock | To make 50ml solution |
|---|---|---|---|---|---|---|
| NaCl | 100mM | Sigma | S-5150 | 41K8934 | 5M | 1 mL |
| KCl | 80mM | Sigma | P-9541 | 70K0002 | 74.55 | 0.298 g |
| MgCl2 | 8mM | Sigma | M-1028 | 120K8933 | 1M | 0.4 mL |
| Sucrose | 2% w/v | Fisher | BP220-212 | 907105 | 342.3 | 1g |
| Tris-Hcl | 16 mM | Fisher | BP1757-500 | 12419 | 1M | 0.8 mL |
| $NaH_2PO_4$ | 1mM | Sigma | S-3193 | 52H-029515 | 120.0 | 0.006 g |
| EDTA | 0.02mM | Sigma | E-7889 | 110K89271 | 0.5 M | 2 μL |
| H20, pH 7.0 at 20 ° C | | Sigma | W-502 | 51K2359 | | Adjust with HCl to 50 ml |

Useful variations on hybridization conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Antisense to Molecular Cloning Techniques, 1987, Academic Press, New York); and Sambrook et al., Molecular Cloning: A Laboratory Manual, Cold Spring Harbor Laboratory Press, New York.

As used herein, "oligonucleotide strand" is a single stranded nucleic acid molecule. An oligonucleotide may comprise ribonucleotides, deoxyribonucleotides, modified nucleotides (e.g., nucleotides with 2' modifications, synthetic base analogs, etc.) or combinations thereof. Such modified oligonucleotides can be preferred over native forms because of properties such as, for example, enhanced cellular uptake and increased stability in the presence of nucleases.

Certain dsNAs for example can be chimeric dsNAs. "Chimeric dsNAs" or "chimeras", in the present context, are dsNAs which contain two or more chemically distinct regions, each made up of at least one nucleotide. The chimeric dsNAs can be Dicer substrates or non-Dicer substrates. In some embodiments these dsNAs typically contain at least one region primarily comprising ribonucleotides (optionally including modified ribonucleotides) that form a Dicer substrate siRNA ("DsiRNA") molecule. This DsiRNA region is covalently attached, e.g., via conventional phosphate bonds or via modified phosphate linkages (e.g., phosphorothioate) to a second region comprising a single stranded nucleotide region ("a single stranded extended region") which confers one or more beneficial properties (such as, for example, increased efficacy, e.g., increased potency and/or duration of DsiRNA activity, function as a recognition domain or means of targeting a chimeric dsNA to a specific location, for example, when administered to cells in culture or to a subject, functioning as an extended region for improved attachment of functional groups, payloads, detection/detectable moieties, functioning as an extended region that allows for more desirable modifications and/or improved spacing of such modifications, etc.). This second region may also include modified or synthetic nucleotides and/or modified or synthetic deoxyribonucleotides.

As used herein, the term "ribonucleotide" encompasses natural and synthetic, unmodified and modified ribonucleotides. Modifications include changes to the sugar moiety, to the base moiety and/or to the linkages between ribonucleotides in the oligonucleotide. As used herein, the term "ribonucleotide" specifically excludes a deoxyribonucleotide, which is a nucleotide possessing a single proton group at the 2' ribose ring position.

As used herein, the term "deoxyribonucleotide" encompasses natural and synthetic, unmodified and modified deoxyribonucleotides. Modifications include changes to the sugar moiety, to the base moiety and/or to the linkages between deoxyribonucleotide in the oligonucleotide. As used herein, the term "deoxyribonucleotide" also includes a modified ribonucleotide that does not permit Dicer cleavage of a dsNA agent, e.g., a 2'-O-methyl ribonucleotide, a phosphorothioate-modified ribonucleotide residue, etc., that does not permit Dicer cleavage to occur at a bond of such a residue.

As used herein, the term "PS-NA" refers to a phosphorothioate-modified nucleotide residue. The term "PS-NA" therefore encompasses both phosphorothioate-modified ribonucleotides ("PS-RNAs") and phosphorothioate-modified deoxyribonucleotides ("PS-DNAs").

In certain examples, a chimeric DsiRNA/DNA agent disclosed herein comprises at least one duplex region of at least 23 nucleotides in length, within which at least 50% of all nucleotides are unmodified ribonucleotides. As used herein, the term "unmodified ribonucleotide" refers to a ribonucleotide possessing a hydroxyl (-OH) group at the 2'

position of the ribose sugar.

In certain examples, a chimeric DsiRNA/DNA agent comprises at least one region, located 3' of the projected Dicer cleavage site on the first strand and 5' of the projected Dicer cleavage site on the second strand, having a length of at least 2 base paired nucleotides in length, wherein at least 50% of all nucleotides within this region of at least 2 base paired nucleotides in length are unmodified deoxyribonucleotides. As used herein, the term "unmodified deoxyribonucleotide" refers to a ribonucleotide possessing a single proton at the 2' position of the ribose sugar.

As used herein, antisense strand, guide strand and second oligonucleotide refer to the same strand of a given dicer substrate molecule according to the invention; while sense strand, passenger strand, and first oligonucleotide refer to the same strand of a given dicer substrate.

As used herein, "antisense strand" refers to a single stranded nucleic acid molecule which has a sequence complementary to that of a target RNA. When the antisense strand contains modified nucleotides with base analogs, it is not necessarily complementary over its entire length, but must at least be sufficiently complementary to hybridize with a target RNA. In certain embodiments the antisense strand is sufficiently complementary to inhibit expression of the target for example, 50%, 60%, 70%, 75%, 80%, 85%, 90%, 95%, or 100% complementary.

As used herein, "sense strand" refers to a single stranded nucleic acid molecule which has a sequence complementary to that of an antisense strand. When the antisense strand contains modified nucleotides with base analogs, the sense strand need not be complementary over the entire length of the antisense strand, but must at least be capable of forming a hybrid with, and thus be able to duplex with the antisense strand.

As used herein, "guide strand" refers to a single stranded nucleic acid molecule of a dsNA or dsNA-containing molecule, which has a sequence sufficiently complementary to that of a target RNA to result in RNA interference. In some embodiments Dicer cleavage is not required for the incorporation of a guide strand into RISC. In some embodiments after cleavage of the dsNA or dsNA-containing molecule by Dicer, a fragment of the guide strand remains associated with RISC, binds a target RNA as a component of the RISC complex, and promotes cleavage of a target RNA by RISC. A guide strand is an antisense strand.

As used herein, "target RNA" refers to an RNA that would be subject to modulation guided by the antisense strand, such as targeted cleavage or steric blockage. The target RNA could be, for example genomic viral RNA, mRNA, a pre-mRNA, or a noncoding RNA. The preferred target is mRNA, such as the mRNA encoding a disease associated protein, such as ApoB, Bc12, Hif-1alpha, Survivin or a p21 ras, such as Ha-ras, K-ras or N-ras.

As used herein, "passenger strand" refers to an oligonucleotide strand of a dsNA or dsNA-containing molecule, which has a sequence that is complementary to that of the guide strand. A passenger strand is a sense strand.

As used herein, "Dicer" refers to an endoribonuclease in the RNase III family that cleaves a dsRNA or dsRNA-containing molecule, e.g., double-stranded RNA (dsRNA) or pre-microRNA (miRNA), into double-stranded nucleic acid fragments about 19-25 nucleotides long, usually with a two-base overhang on the 3' end. With respect to the dsNAs of the invention, the duplex formed by a dsRNA region of a dsNA of the invention is recognized by Dicer and is a Dicer substrate on at least one strand of the duplex. Dicer catalyzes the first step in the RNA interference pathway, which consequently results in the degradation of a target RNA. The protein sequence of human Dicer is provided at the NCBI database under accession number NP-085124.

Dicer "cleavage" is determined as follows (e.g., see Collingwood et al., Oligonucleotides 18:187-200 (2008)). In a Dicer cleavage assay, RNA duplexes (100 pmol) are incubated in 20 $\mu$L of 20 mM Tris pH 8.0, 200 mM NaCl, 2.5 mM MgC12 with or without 1 unit of recombinant human Dicer (Stratagene, La Jolla, Calif.) at 37° C. for 18-24 hours. Samples are desalted using a Performa SR 96-well plate (Edge Biosystems, Gaithersburg, Md.). Electrospray-ionization liquid chromatography mass spectroscopy (ESI-LCMS) of duplex RNAs pre- and post-treatment with Dicer is done using an Oligo HTCS system (Novatia, Princeton, N.J.; Hail et al., 2004), which consists of a ThermoFinnigan TSQ7000, Xcalibur data system, ProMass data processing software and Paradigm MS4 HPLC (Michrom BioResources, Auburn, Calif.). In this assay, Dicer cleavage occurs where at least 5%, 10%, 20%, 30%, 40%, 50%, 60%, 70%, 80%, 90%, 95%, or even 100% of the Dicer substrate dsRNA, (i.e., 25-35 bp dsRNA, preferably 26-30 bp dsRNA, optionally extended as described herein) is cleaved to a shorter dsRNA (e.g., 19-23 bp dsRNA, preferably, 21-23 bp dsRNA).

As used herein, "Dicer cleavage site" refers to the sites at which Dicer cleaves a dsRNA (e.g., the dsRNA region of a dsNA of the invention). Dicer contains two RNase III domains which typically cleave both the sense and antisense strands of a dsRNA. The average distance between the RNase III domains and the PAZ domain determines the length of the short double-stranded nucleic acid fragments it produces and this distance can vary (Macrae I, et al. (2006). "Structural basis for double-stranded RNA processing by Dicer". Science 311 (5758): 195-8.). As shown, e.g., in FIG. 2, Dicer is projected to cleave certain double-stranded nucleic acids of the instant invention that possess an antisense strand having a 2 nucleotide 3' overhang at a site between the 21st and 22nd nucleotides removed from the 3' terminus of the antisense strand, and at a corresponding site between the 21st and 22nd nucleotides removed from the 5' terminus of the sense strand. The projected and/or prevalent Dicer cleavage site(s) for dsNA molecules distinct from those depicted in FIG. 2 may be similarly identified via art-recognized methods, including those described in Macrae et al. While the Dicer cleavage event depicted in FIG. 2 generates a 21 nucleotide siRNA, it is noted that Dicer cleavage of a dsNA (e.g.,

DsiRNA) can result in generation of Dicer-processed siRNA lengths of 19 to 23 nucleotides in length. Indeed, in one aspect of the invention that is described in greater detail below, a double stranded DNA region is included within a dsNA for purpose of directing prevalent Dicer excision of a typically non-preferred 19mer siRNA.

As used herein, "overhang" refers to unpaired nucleotides, in the context of a duplex having two or four free ends at either the 5' terminus or 3' terminus of a dsNA. In certain embodiments, the overhang is a 3' or 5' overhang on the antisense strand or sense strand. "Overhang" and "extension" are used synonymously throughout.

As used herein, "target" refers to any nucleic acid sequence whose expression or activity is to be modulated. In particular embodiments, the target refer to RNA which duplexes to a single stranded nucleic acid that is an antisense strand in a RISC complex. Hybridization of the target RNA to the antisense strand results in processing by the RISC complex. Consequently, expression of the RNA or proteins encoded by the RNA, e.g., mRNA, is reduced.

As used herein, the term "RNA processing" refers to processing activities performed by components of the siRNA, miRNA or RNase H pathways (e.g., Drosha, Dicer, Argonaute2 or other RISC endoribonucleases, and RNaseH), which are described in greater detail below (see "RNA Processing" section below). The term is explicitly distinguished from the post-transcriptional processes of 5' capping of RNA and degradation of RNA via non-RISC- or non-RNase H-mediated processes. Such "degradation" of an RNA can take several forms, e.g. deadenylation (removal of a 3' poly (A) tail), and/or nuclease digestion of part or all of the body of the RNA by any of several endo- or exo- nucleases (e.g., RNase III, RNase P, RNase T1, RNase A (1, 2, 3, 4/5), oligonucleotidase, etc.).

As used herein, "reference" is meant a standard or control. As is apparent to one skilled in the art, an appropriate reference is where only one element is changed in order to determine the effect of the one element.

As used herein, "modified nucleotide" refers to a nucleotide that has one or more modifications to the nucleoside, the nucleobase, furanose ring, or phosphate group. For example, modified nucleotides exclude ribonucleotides containing adenosine monophosphate, guanosine monophosphate, uridine monophosphate, and cytidine monophosphate and deoxyribonucleotides containing deoxyadenosine monophosphate, deoxyguanosine monophosphate, deoxythymidine monophosphate, and deoxycytidine monophosphate. Modifications include those naturally occurring that result from modification by enzymes that modify nucleotides, such as methyltransferases. Modified nucleotides also include synthetic or non-naturally occurring nucleotides. Synthetic or non-naturally occurring modifications in nucleotides include those with 2' modifications, e.g., 2'-methoxyethoxy, 2'-fluoro, 2'-allyl, 2'-O-[2-(methylamino)-2-oxoethyl], 4'-thio, 4'-CH2-O-2'-bridge, 4'-(CH2)2-O-2'-bridge, 2'-LNA, and 2'-O-(N-methylcarbamate) or those comprising base analogs. In connection with 2'-modified nucleotides as described for the present disclosure, by "amino" is meant 2'-NH2 or 2'-O-NH2, which can be modified or unmodified. Such modified groups are described, e.g., in Eckstein et al., U.S. Pat. No. 5,672,695 and Matulic-Adamic et al., U.S. Pat. No. 6,248,878.

The term "in vitro" has its art recognized meaning, e.g., involving purified reagents or extracts, e.g., cell extracts. The term "in vivo" also has its art recognized meaning, e.g., involving living cells, e.g., immortalized cells, primary cells, cell lines, and/or cells in an organism.

In reference to the nucleic acid molecules of the present disclosure, nucleotides in certain positions on either strand of the dsNA may be specified. With reference to FIGS. 1-3, the conventions for denoting positions of the DsiRNAs of the invention are shown in Table 2.

In reference to the nucleic acid molecules of the present disclosure, the modifications may exist in patterns on a strand of the dsNA. As used herein, "alternating positions" refers to a pattern where every other nucleotide is a modified nucleotide or there is an unmodified nucleotide (e.g., an unmodified ribonucleotide) between every modified nucleotide over a defined length of a strand of the dsNA (e.g., 5'-MNMNMN-3'; 3'-MNMNMN-5'; where M is a modified nucleotide and N is an unmodified nucleotide). The modification pattern starts from the first nucleotide position at either the 5' or 3' terminus according to any of the position numbering conventions described herein (in certain embodiments, position 1 is designated in reference to the terminal residue of a strand following a projected Dicer cleavage event of a DsiRNA agent of the invention; thus, position 1 does not always constitute a 3' terminal or 5' terminal residue of a pre-processed agent of the invention).

**Table 2: Description of Numbering Convention as to Strand Positions**

**Position 1:** A position located on the passenger strand is denoted by a number without a superscript label. (e.g., position 1). Position 1 of the passenger strand is the 5'- terminal nucleotide, except for the 5' extended passenger strands, where the 5' terminal nucleotide occurs in the extended region and is accorded the highest number with a superscript E (see below and FIG. 1A).

**Position A:** A position located on the guide strand is designated with a superscript A (e.g., position 1A. The guide strand is numbered such that the first base paired nucleotide at its 3' terminus is referred to as (e.g., position 1A). Where the guide strand contains a 3' terminal single stranded overhang of 1-6 nucleotides, those nucleotides are

simply referred to as 3' terminal guide strand unpaired or single stranded residues.

**Position A1:** A position located on the guide strand in the extended 5' region is labeled with a superscript B (e.g., position 1B represents the 5' terminal nucleotide of an extended guide strand (see FIG. 1A)).

**Position C:** A position located on the third oligonucleotide. The third oligonucleotide is complementary to the extended region of the guide strand and is discontinuous with the passenger strand. Position 1C (see FIG. 1A) represents the 5' terminal nucleotide of the third oligoncleotide.

**Position D:** A position located on a 3' extended passenger strand, such that position 1D references the 3' terminal nucleotide residue of the extended passenger strand.

**Position E:** A position located on the extended region of a 5' extended position 1E of the passenger strand, which is an unpaired nucleotide of the strand 5' single stranded extension.(see FIG. 1A).

**Position IE:** is the unpaired nucleotide consecutive (i.e., adjacent) to the first base paired nucleotide of the passenger strand (see FIG. 1A).

**Position F:** A position located in the duplex region of a 5' extended passenger strand, such that position IF references the first base paired nucleotide on the 5' passenger strand (starting from the 5' end).

The pattern of modified nucleotides at alternating positions may run the full length of the strand, but in certain embodiments includes at least 4, 6, 8, 10, 12, 14 nucleotides containing at least 2, 3, 4, 5, 6 or 7 modified nucleotides, respectively. As used herein, "alternating pairs of positions" refers to a pattern where two consecutive modified nucleotides are separated by two consecutive unmodified nucleotides over a defined length of a strand of the dsNA (e.g., 5'-MMNNMMNNMMNN-3'; 3'-MMNNMMNNMMNN-5'; where M is a modified nucleotide and N is an unmodified nucleotide). The modification pattern starts from the first nucleotide position at either the 5' or 3' terminus according to any of the position numbering conventions described herein. The pattern of modified nucleotides at alternating positions may run the full length of the strand, but preferably includes at least 8, 12, 16, 20, 24, 28 nucleotides containing at least 4, 6, 8, 10, 12 or 14 modified nucleotides, respectively. It is emphasized that the above modification patterns are exemplary and are not intended as limitations on the scope of the invention.

As used herein, "base analog" refers to a heterocyclic moiety which is located at the 1' position of a nucleotide sugar moiety in a modified nucleotide that can be incorporated into a nucleic acid duplex (or the equivalent position in a nucleotide sugar moiety substitution that can be incorporated into a nucleic acid duplex). In the dsNAs of the invention, a base analog is generally either a purine or pyrimidine base excluding the common bases guanine (G), cytosine (C), adenine (A), thymine (T), or uracil (U). Base analogs can duplex with other bases or base analogs in dsRNAs. Base analogs include those useful in the compounds and methods of the invention, e.g., those disclosed in U.S. Pat. Nos. 5,432,272 and 6,001,983 to Benner and US Patent Publication No. 20080213891 to Manoharan. Non-limiting examples of bases include hypoxanthine (I), xanthine (X), 3β-D-ribofuranosyl-(2,6-diaminopyrimidine) (K), 3-β-D-ribofuranosyl-(1-methyl-pyrazolo[4,3-d]pyrimidine-5,7(4H,6H)-dione) (P), iso-cytosine (iso-C), iso-guanine (iso-G), 1-β-D-ribofuranosyl-(5-nitroindole), 1-β-D-ribofuranosyl-(3-nitropyrrole), 5-bromouracil, 2-aminopurine, 4-thio-dT, 7-(2-thienyl)-imidazo[4,5-b]pyridine (Ds) and pyrrole-2-carbaldehyde (Pa), 2-amino-6-(2-thienyl)purine (S), 2-oxopyridine (Y), difluorotolyl, 4-fluoro-6-methylbenzimidazole, 4-methylbenzimidazole, 3-methyl isocarbostyrilyl, 5-methyl isocarbostyrilyl, and 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenzyl, tetracenyl, pentacenyl, and structural derivates thereof (Schweitzer et al., J. Org. Chem., 59:7238-7242 (1994); Berger et al., Nucleic Acids Research, 28(15):2911-2914 (2000); Moran et al., J. Am. Chem. Soc., 119:2056-2057 (1997); Morales et al., J. Am. Chem. Soc., 121:2323-2324 (1999); Guckian et al., J. Am. Chem. Soc., 118:8182-8183 (1996); Morales et al., J. Am. Chem. Soc., 122(6):1001-1007 (2000); McMinn et al., J. Am. Chem. Soc., 121:11585-11586 (1999); Guckian et al., J. Org. Chem., 63:9652-9656 (1998); Moran et al., Proc. Natl. Acad. Sci., 94:10506-10511 (1997); Das et al., J. Chem. Soc., Perkin Trans., 1:197-206 (2002); Shibata et al., J. Chem. Soc., Perkin Trans., 1: 1605-1611 (2001); Wu et al., J. Am. Chem. Soc., 122(32):7621-7632 (2000); O'Neill et al., J. Org. Chem., 67:5869-5875 (2002); Chaudhuri et al., J. Am. Chem. Soc., 117:10434-10442 (1995); and U.S. Pat. No. 6,218,108.). Base analogs may also be a universal base.

As used herein, "universal base" refers to a heterocyclic moiety located at the 1' position of a nucleotide sugar moiety in a modified nucleotide, or the equivalent position in a nucleotide sugar moiety substitution, that, when present in a nucleic acid duplex, can be positioned opposite more than one type of base without altering the double helical structure (e.g., the structure of the phosphate backbone). Additionally, the universal base does not destroy the ability of the single stranded nucleic acid in which it resides to duplex to a target nucleic acid. The ability of a single stranded nucleic acid containing a universal base to duplex a target nucleic can be assayed by methods apparent to one in the art (e.g., UV absorbance, circular dichroism, gel shift, single stranded nuclease sensitivity, etc.). Additionally, conditions under which duplex formation is observed may be varied to determine duplex stability or formation, e.g., temperature, as melting temperature (Tm) correlates with the stability of nucleic acid duplexes. Compared to a reference single stranded nucleic acid that is exactly complementary to a target nucleic acid, the single stranded nucleic acid containing

a universal base forms a duplex with the target nucleic acid that has a lower Tm than a duplex formed with the complementary nucleic acid. However, compared to a reference single stranded nucleic acid in which the universal base has been replaced with a base to generate a single mismatch, the single stranded nucleic acid containing the universal base forms a duplex with the target nucleic acid that has a higher Tm than a duplex formed with the nucleic acid having the mismatched base.

Some universal bases are capable of base pairing by forming hydrogen bonds between the universal base and all of the bases guanine (G), cytosine (C), adenine (A), thymine (T), and uracil (U) under base pair forming conditions. A universal base is not a base that forms a base pair with only one single complementary base. In a duplex, a universal base may form no hydrogen bonds, one hydrogen bond, or more than one hydrogen bond with each of G, C, A, T, and U opposite to it on the opposite strand of a duplex. Preferably, the universal bases do not interact with the base opposite to it on the opposite strand of a duplex. In a duplex, base pairing between a universal base occurs without altering the double helical structure of the phosphate backbone. A universal base may also interact with bases in adjacent nucleotides on the same nucleic acid strand by stacking interactions. Such stacking interactions stabilize the duplex, especially in situations where the universal base does not form any hydrogen bonds with the base positioned opposite to it on the opposite strand of the duplex. Non-limiting examples of universal-binding nucleotides include inosine, 1-β-D-ribo furanosyl-5-nitroindole, and/or 1-β-D-ribofuranosyl-3-nitropyrrole (US Pat. Appl. Publ. No. 20070254362 to Quay et al.; Van Aerschot et al., An acyclic 5-nitroindazole nucleoside analogue as ambiguous nucleoside. Nucleic Acids Res. 1995 Nov. 11; 23(21):4363-70; Loakes et al., 3-Nitropyrrole and 5-nitroindole as universal bases in primers for DNA sequencing and PCR. Nucleic Acids Res. 1995 Jul. 11; 23(13):2361-6; Loakes and Brown, 5-Nitroindole as a universal base analogue. Nucleic Acids Res. 1994 Oct. 11; 22(20):4039-43).

As used herein, "loop" refers to a structure formed by a single strand of a nucleic acid, in which complementary regions that flank a particular single stranded nucleotide region hybridize in a way that the single stranded nucleotide region between the complementary regions is excluded from duplex formation or Watson-Crick base pairing. A loop is a single stranded nucleotide region of any length, for example 1, 2, 3, 4, 5, 6, 7, 8, 9, 10 or more. Examples of loops include the unpaired nucleotides present in such structures as hairpins, stem loops, or extended loops.

As used herein, "extended loop" in the context of a dsRNA refers to a single stranded loop and in addition 1, 2, 3, 4, 5, 6 or up to 55 base pairs or duplexes flanking the loop. In an extended loop, nucleotides that flank the loop on the 5' side form a duplex with nucleotides that flank the loop on the 3' side. An extended loop may form a hairpin or stem loop.

As used herein, "tetraloop" in the context of a dsRNA refers to a loop (a single stranded region) consisting of four nucleotides that forms a stable secondary structure that contributes to the stability of an adjacent Watson-Crick hybridized nucleotides. Without being limited to theory, a tetraloop may stabilize an adjacent Watson-Crick base pair by stacking interactions. In addition, interactions among the four nucleotides in a tetraloop include but are not limited to non-Watson-Crick base pairing, stacking interactions, hydrogen bonding, and contact interactions (Cheong et al., Nature 1990 Aug. 16; 346(6285):680-2; Heus and Pardi, Science 1991 Jul. 12; 253(5016):191-4). A tetraloop confers an increase in the melting temperature (Tm) of an adjacent duplex that is higher than expected from a simple model loop sequence consisting of four random bases. For example, a tetraloop can confer a melting temperature of at least 50° C, at least 55° C., at least 56° C, at least 58° C, at least 60° C, at least 65° C or at least 75° C in 10 mM $NaHPO_4$ to a hairpin comprising a duplex of at least 2 base pairs in length. A tetraloop may contain ribonucleotides, deoxyribonucleotides, modified nucleotides, and combinations thereof. Examples of RNA tetraloops include the UNCG family of tetraloops (e.g., UUCG), the GNRA family of tetraloops (e.g., GAAA), and the CUUG tetraloop. (Woese et al., Proc Natl Acad Sci USA. 1990 November; 87(21):8467-71; Antao et al., Nucleic Acids Res. 1991 Nov. 11; 19(21):5901-5). Examples of DNA tetraloops include the d(GNNA) family of tetraloops (e.g., d(GTTA), the d(GNRA)) family of tetraloops, the d(GNAB) family of tetraloops, the d(CNNG) family of tetraloops, the d(TNCG) family of tetraloops (e.g., d(TTCG)). (Nakano et al. Biochemistry, 41 (48), 14281-14292, 2002. SHINJI et al. Nippon Kagakkai Koen Yokoshu VOL. 78th; NO. 2; PAGE. 731 (2000).)

As used herein, "increase" or "enhance" is meant to alter positively by at least 5% compared to a reference in an assay. An alteration may be by two fold or three fold or four fold or five fold or tenfold or 100 fold or, 5%, 10%, 25%, 30%, 50%, 75%, or even by 100% compared to a reference in an assay. By "enhance Dicer cleavage," it is meant that the processing of a quantity of a dsRNA or dsRNA-containing molecule by Dicer results in more Dicer cleaved dsRNA products, that Dicer cleavage reaction occurs more quickly compared to the processing of the same quantity of a reference dsRNA or dsRNA-containing molecule in an in vivo or in vitro assay of this disclosure, or that Dicer cleavage is directed to cleave at a specific, preferred site within a dsNA and/or generate higher prevalence of a preferred population of cleavage products (e.g., by inclusion of DNA residues as described herein). In one embodiment, enhanced or increased Dicer cleavage of a dsNA molecule is above the level of that observed with an appropriate reference dsNA molecule. In another embodiment, enhanced or increased Dicer cleavage of a dsNA molecule is above the level of that observed with an inactive or attenuated molecule. Increase as it refers to binding affinity of a ligand conjugated dsNA of the invention means, for example the ligand conjugated dsNA binds to its target with an affinity that is 2-fold, 3-fold, 4-fold,

5-fold, 10-fold or more than a dsNA that is not conjugated to a ligand but is directed to the same target. Increase as it refers to cellular uptake means for example, the amount of the ligand conjugated dsNA that is taken up by cells, for example by receptor mediated endocytosis is 2-fold, 3-fold, 4-fold, 5-fold, 10-fold or more than a dsNA that is not conjugated to a ligand. Increased as it refers to tracking means that the progression or movement of a dsRNA can be more easily determined.

As used herein "reduce" is meant to alter negatively by at least 5% compared to a reference in an assay. An alteration may be by two fold or three fold or four fold or five fold or tenfold or 100 fold or more or 5%, 10%, 25%, 30%, 50%, 75%, or even by 100% compared to a reference in an assay. By "reduce expression," it is meant that the expression of the gene, or level of RNA molecules or equivalent RNA molecules encoding one or more proteins or protein subunits, or level or activity of one or more proteins or protein subunits encoded by a target gene, is reduced below that observed in the absence of the nucleic acid molecules (e.g., dsRNA molecule or dsRNA-containing molecule) in an in vivo or in vitro assay of this disclosure. In one embodiment, inhibition, down-regulation or reduction with a dsNA molecule is below that level observed in the presence of an inactive or attenuated molecule. In another embodiment, inhibition, down-regulation, or reduction with dsNA molecules is below that level observed in the presence of, e.g., a dsNA molecule with scrambled sequence or with mismatches. In another embodiment, inhibition, down-regulation, or reduction of gene expression with a nucleic acid molecule of the instant disclosure is greater in the presence of the nucleic acid molecule than in its absence.

As used herein, "cell" is meant to include both prokaryotic (e.g., bacterial) and eukaryotic (e.g., mammalian or plant) cells. Cells may be of somatic or germ line origin, may be totipotent or pluripotent, and may be dividing or non-dividing. Cells can also be derived from or can comprise a gamete or an embryo, a stem cell, or a fully differentiated cell. Thus, the term "cell" is meant to retain its usual biological meaning and can be present in any organism such as, for example, a bird, a plant, and a mammal, including, for example, a human, a cow, a sheep, an ape, a monkey, a pig, a dog, and a cat. Within certain aspects, the term "cell" refers specifically to mammalian cells, such as human cells, that contain one or more isolated dsNA molecules of the present disclosure. In particular aspects, a cell processes dsRNAs or dsRNA-containing molecules resulting in RNA interference of target nucleic acids, and contains proteins and protein complexes required for RNAi, e.g., Dicer and RISC.

As used herein, "animal" is meant a multicellular, eukaryotic organism, including a mammal, pig, monkey, dog, cat, mouse, horse, cow, rat or more preferably a human. The example methods of the disclosure in general comprise administration of an effective amount of the agents herein, such as an agent of the structures of formulae herein, to a subject (e.g., animal, human) in need thereof, including a mammal, for example, pig, monkey, dog, cat, mouse, horse, cow, rat or more preferably a human. Such treatment will be suitably administered to subjects, particularly humans, suffering from, having, susceptible to, or at risk for a disease, or a symptom thereof.

By "pharmaceutically acceptable carrier" is meant, a composition or formulation that allows for the effective distribution of the nucleic acid molecules of the instant disclosure in the physical location most suitable for their desired activity.

The present disclosure is directed to compositions that comprise both a double stranded NA ("dsNA") duplex and a single stranded extended region-in most embodiments, a dsDNA duplex-within the same agent, and methods for preparing them, that are capable of reducing the expression of target genes in eukaryotic cells. One of the strands of the dsNA region contains a region of nucleotide sequence that has a length that ranges from about 15 to about 22 nucleotides that can direct the destruction of the RNA transcribed from the target gene. The dsDNA duplex region of such an agent is not necessarily complementary to the target RNA, and, therefore, in such instances does not enhance target RNA hybridization of the region of nucleotide sequence capable of directing destruction of a target RNA. Double stranded NAs of the invention can possess strands that are chemically linked, and/or can also possess an extended loop, optionally comprising a tetraloop that links the first and second strands. In some embodiments, the extended loop containing the tetraloop is at the 3' terminus of the sense strand, at the 5' terminus of the antisense strand, or both. In some embodiments, the extended loop containing the tetraloop is at the 5' terminus of the sense strand, at the 3' terminus of the sense strand, or both. In some embodiments the dsNA agents are Dicer substrates and are cleaved by Dicer. In other embodiments the dsNA agents are not dicer substrates and are not cleaved by Dicer. In some examples the sense and antisense strand of length 15-85 nucleotides form a duplex of length 15-35 base pairs. In some examples the duplex contains an extension of 1-50 nucleotides which is located either on the sense strand or on the antisense strand or both. The extension in the example could be at the 5' terminus of the sense strand or the 5' terminus of antisense strand or both. The extension in another example could be at the 3' terminus of the sense strand or the 3' terminus of antisense strand or both. In another example, the extension is at the 5' terminus of the sense strand or the 3' terminus of antisense strand or both The dsNA agents of aforesaid examples also comprise of at least one ligand modified nucleotide

In one example of the disclosure, the dsNA comprises a double stranded RNA duplex region comprising 15-85 nts (for example, 15, 16, 17, 18, 19, 20, 21, 22, 23, 24, 25, 26, 27, 28, 29 30, 31, 32, 33, 34, 37, 39, 40, 41, 42, 43, 45, 50, 55, 60, 65, 70, 75, 80, 81, 82, 83, 84 nts) in length wherein there is at least one ligand modified nucleotide.

"Extended" dsNA agents according to the invention can be "guide extended" (the nucleotide region at the 5'

terminus of the antisense strand that is present on the molecule in addition to the 15-35 base antisense sequence required for participation of the antisense strand in a dicer substrate) or "passenger extended" (the nucleotide region at the 3' terminus of the sense strand that is optionally present on the molecule in addition to the 15-35 base sense sequence required for sense strand participation in a dicer substrate; or the nucleotide region at the 5' terminus of the sense strand that is optionally present on the sense strand in addition to the 15-35 base sequence required for sense strand participation in a dicer substrate). Therefore, as used herein, the term "extended" is not meant to refer to the antisense (or second strand, or guide strand) 3' overhang of 1-6 single stranded nucleotides; rather "extended" as used herein refers to the opposite end of the dicer substrate molecule, that is, a 5' extended sense or antisense strand, where the extended region is 1-50, preferably 10-15 nucleotides in length or a 3' extended sense strand, where the extended region is 1-30, preferably 10-15 nucleotides in length. The 5' extended antisense strand may be single stranded, and optionally may be duplexed with a third nucleic acid molecule which is complementary, preferably fully (100%) complementary, to the 5' extended single stranded region of the antisense strand. Therefore, in some embodiments, i.e., when the third nucleic acid molecule is present, the 5' extended region of the antisense strand is not single stranded, but rather is a duplex, or double stranded region. Preferably, according to the invention, the third nucleic acid molecule, i.e., the sense region that is complementary to the 5' extended antisense region is not present unless a cognate 5' extended antisense region is present.

The extended dsNA agents of the instant invention can enhance the following attributes of such agents relative to dsNAs lacking an extension as defined herein: in vitro efficacy (e.g., potency and duration of effect), in vivo efficacy (e.g., potency, duration of effect, pharmacokinetics, pharmacodynamics, intracellular uptake, reduced toxicity). In certain embodiments, the 5' extended region of the first or second strand can optionally provide an additional agent, such as an aptamer or fragment thereof; or a binding site (e.g., a "decoy" binding site) for a native or exogenously introduced moiety capable. The moiety can therefore bind to the extension of either a non-sequence-selective or sequence-specific manner via the binding site. For example, the extended region of a dsNA can be designed to comprise one or more transcription factor recognition sequences and/or a sequence-specific recognition domain for a probe, marker, etc.).

The double-stranded nucleic acid agent is conjugated to a ligand providing additional functionality to the agent. For example, the molecule may be conjugated at the 5'extension with a ligand such as N-acetylgalactosamine, which provides specific targeting to hepatic cells and tissue. The ligand can be conjugated directly to the base structure of the 5' extension or via a linker. The linker may be a releasable linker, such as, for example, Bicin.

As used herein, the term "pharmacokinetics" refers to the process by which a drug is absorbed, distributed, metabolized, and eliminated by the body. In certain embodiments of the instant invention, enhanced pharmacokinetics of a 5' extended second strand or 3' extended first strand .dsNA agent relative to an appropriate control dsNA refers to increased absorption and/or distribution of such an agent, and/or slowed metabolism and/or elimination of such a 5' second strand extended dsNA agent or 3' first strand extended dsNA agent from a subject administered such an agent.

As used herein, the term "pharmacodynamics" refers to the action or effect of a drug on a living organism. In certain embodiments of the instant invention, enhanced pharmacodynamics of a 5' second strand extended dsNA agent or 3' first strand extended dsNA agent relative to an appropriate control dsNA refers to an increased (e.g., more potent or more prolonged) action or effect of a 5' second strand extended dsNA agent or 3' first strand extended dsNA agent, respectively, upon a subject administered such agent, relative to an appropriate control dsNA

As used herein, the term "stabilization" refers to a state of enhanced persistence of an agent in a selected environment (e.g., in a cell or organism). In certain embodiments, the 5' second strand extended dsNA or 3' first strand extended dsNA agents of the instant invention exhibit enhanced stability relative to appropriate control dsNAs. Such enhanced stability can be achieved via enhanced resistance of such agents to degrading enzymes (e.g., nucleases) or other agents.

In addition to the attributes described above for the 5' antisense extended dsNAs according to the invention, the optional third nucleic acid sense molecule of 10-30, and preferably 10-15 nucleotides, when present, stabilizes the dsNA, and/or increases potency, prolongs action or effect, enhances pharmacodynamic or pharmacological effects, and/or provides an additional agent (or portion thereof), such as an aptamer or fragment thereof; or a binding site (e.g., a "decoy" binding site) for a native or exogenously introduced moiety (e.g., a label).

## DSiRNA DESIGN/SYNTHESIS

It was previously shown that longer dsRNA species of from 25 to about 30 nucleotides (DsiRNAs) yield unexpectedly effective RNA inhibitory results in terms of potency and duration of action, as compared to 19-23mer siRNA agents. Without wishing to be bound by the underlying theory of the dsRNA processing mechanism, it is thought that the longer dsRNA species serve as a substrate for the Dicer enzyme in the cytoplasm of a cell. In addition to cleaving the dsNA of the invention into shorter segments, Dicer is thought to facilitate the incorporation of a single-stranded cleavage product derived from the cleaved dsNA into the RISC complex that is responsible for the destruction of the cytoplasmic RNA of or derived from the target gene. Prior studies (Rossi et al., published U.S. Patent Application No. 2007/0265220) have shown that the cleavability of a dsRNA species (specifically, a DsiRNA agent) by Dicer corresponds

with increased potency and duration of action of the dsRNA species. The instant invention, at least in part, provides for design of RNA inhibitory agents that direct the site of Dicer cleavage, such that preferred species of Dicer cleavage products are thereby generated.

In a model of DsiRNA processing, Dicer enzyme binds to a DsiRNA agent, resulting in cleavage of the DsiRNA at a position 19-23 nucleotides removed from a Dicer PAZ domain-associated 3' overhang sequence of the antisense strand of the DsiRNA agent. This Dicer cleavage event results in excision of those duplexed nucleic acids previously located at the 3' end of the passenger (sense) strand and 5' end of the guide (antisense) strand. Cleavage of a DsiRNA typically yields a 19mer duplex with 2-base overhangs at each end. As presently modeled in FIG. 2, this Dicer cleavage event generates a 21-23 nucleotide guide (antisense) strand (or, in certain instances where a longer guide strand 3' overhang is present, 24-27 nucleotide guide strands could result from Dicer cleavage) capable of directing sequence-specific inhibition of target mRNA as a RISC component.

The first and second oligonucleotides of the DsiRNA agents of the instant invention are not required to be completely complementary in the duplexed region. In one embodiment, the 3'-terminus of the sense strand contains one or more mismatches. In one aspect, about two mismatches are incorporated at the 3' terminus of the sense strand. For example, the DsiRNA is a double stranded RNA molecule containing two RNA oligonucleotides in the range of 25-66 nucleotides in length and, when annealed to each other, have a two nucleotide mismatch on the 3'-terminus of the sense strand (the 5'-terminus of the antisense strand). The use of mismatches or decreased thermodynamic stability (specifically at the 3'-sense/5'-antisense position) has been proposed to facilitate or favor entry of the antisense strand into RISC (Schwarz et al., 2003; Khvorova et al., 2003), presumably by affecting some rate-limiting unwinding steps that occur with entry of the siRNA into RISC. Thus, terminal base composition has been included in design algorithms for selecting active 21mer siRNA duplexes (Ui-Tei et al., 2004; Reynolds et al., 2004). With Dicer cleavage of the dsRNA region of this embodiment, the small end-terminal sequence which contains the mismatches will either be left unpaired with the antisense strand (become part of a 3'-overhang) or be cleaved entirely off the final 21-mer siRNA. These specific forms of "mismatches", therefore, do not persist as mismatches in the final RNA component of RISC. The finding that base mismatches or destabilization of segments at the 3'-end of the sense strand of Dicer substrate improved the potency of synthetic duplexes in RNAi, presumably by facilitating processing by Dicer, was a surprising finding of past works describing the design and use of 25-30mer dsRNAs (also termed "DsiRNAs" herein; Rossi et al., published U.S. Patent Application Nos. 2005/0277610, 2005/0244858 and 2007/0265220). Exemplary mismatched or wobble base pairs of agents possessing mismatches are G:A, C:A, C:U, G:G, A:A, C:C, U:U, I:A, I:U and I:C. Base pair strength of such agents can also be lessened via modification of the nucleotides of such agents, including, e.g., 2-amino- or 2,6-diamino modifications of guanine and adenine nucleotides. The Dicer substrates and non-Dicer substrate molecules contemplated herein can have any of the structures contemplated below. Figures 43-46 presents dsNA molecules of the invention comprising extensions. Figure 47 presents a dsNA molecules comprising a tetraloop located at the 5' or 3' end or both ends of the antisense strand.

## EXEMPLARY STRUCTURES OF dsNA AGENTS

The compositions of the invention comprise a dsNA which is a precursor molecule, i.e., the dsNA of the present invention is processed in vivo to produce an active small interfering nucleic acid (siRNA). The dsNA is processed by Dicer to an active siRNA which is incorporated into RISC. In some embodiments the dsNA molecule of the invention is not cleaved by Dicer but is still incorporated into RISC. dsNAs can have any of the structures described herein below for dsiRNAs.

In one example, the present disclosure provides compositions for RNA interference (RNAi) having a first or second strand that has at least 8 consecutive ribonucleotides. In certain examples, a dsNA agent described herein has 9, 10, 11, 12, 13, 14, 15, 16, 17, 18, 19, 20, 21, 22, 23 or more (e.g., 24, 25, 26, 27, 28, 29, 30, 31, 32, 33, 34, 35, 26, or more, up to the full length of the strand) ribonucleotides, modified ribonucleotides (2'-O-methyl ribonucleotides, phosphorothioate linkages). In certain examples, the ribonucleotides or modified ribonucleotides are consecutive.

In one example, the present disclosure provides compositions for RNA interference (RNAi) that possess one or more deoxyribonucleotides within a region of a double stranded nucleic acid that is positioned 3' of a projected sense strand Dicer cleavage site and correspondingly 5' of a projected antisense strand Dicer cleavage site. In one example, at least one nucleotide of the guide strand between and including the guide strand nucleotides corresponding to and thus base paired with passenger strand positions 24 to the 3' terminal nucleotide residue of the passenger strand is a deoxyribonucleotide. In some examples, the double stranded nucleic acid possesses one or more base paired deoxyribonucleotides within a region of the double stranded nucleic acid that is positioned 3' of a projected sense strand Dicer cleavage site and correspondingly 5' of a projected antisense strand Dicer cleavage site.

In certain examples, the dsNA agents can have any of the following exemplary structures:

In one such example, the dsNA comprises:

5'-XXXXXXXXXXXXXXXXXXXXXXXXXN*-3'

wherein "X"=RNA, "Y" is an optional overhang domain comprised of 0-10 RNA monomers that are optionally 2'-O-methyl RNA monomers-in certain examples, "Y" is an overhang domain comprised of 0-4 RNA monomers that are optionally 2'-O-methyl RNA monomers, "Z"=DNA, RNA, or modified nucleotide, and "N"=1 to 50 or more, but is optionally 1-30 or, optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In one such example, the dsNA comprises:

5'-Zm$(Z)_2$Zm$(Z)_2$Zm$(Z)_2$Zm(Z)25N-3'

3'-Y(Z)25N-5' wherein "Y" is an optional overhang domain comprised of 0-10 RNA monomers that are optionally 2'-O-methyl RNA monomers and or 2'-Fluoro monomers-in certain examples, "Y" is an overhang domain comprised of 0-4 RNA monomers that are optionally 2'-O-methyl RNA monomers or 2'-Fluoro monomers, "Z"=DNA, RNA, or modified nucleotide that are optionally 2'-O-methyl RNA monomers and or 2'-Fluoro monomers , Zm is a ligand modified nucleotide that are optionally thymines or adenosines conjugated with GalNac through sugar or base of the nucleotide, and "N"=1 to 50 or more, but is optionally 1-30 or, optionally 1-15 or, optionally, 1-10. In an example, N comprises at least 9 consecutive phosphorothioate linkages. In one example, the top strand is the sense strand, and the bottom strand is the antisense strand. Alternatively, the bottom strand is the sense strand and the top strand is the antisense strand.

In one such example, the dsNA comprises:

5'-Zm(Z)Zm(Z)Zm(Z)Zm$(Z)_{25}$N-3'

3'-Y$(Z)_{25}$N-5'

wherein "Y" is an optional overhang domain comprised of 0-10 RNA monomers that are optionally 2'-O-methyl RNA monomers and or 2'-Fluoro monomers-in certain examples, "Y" is an overhang domain comprised of 0-4 RNA monomers that are optionally 2'-O-methyl RNA monomers or 2'-Fluoro monomers, "Z"=DNA, RNA, or modified nucleotide that are optionally 2'-O-methyl RNA monomers and or 2'-Fluoro monomers , Zm is a ligand modified nucleotide that are optionally thymines or adenosines conjugated with GalNac through sugar or base of the nucleotide, and "N"=1 to 50 or more, but is optionally 1-30 nucleotides or, optionally 1-15 nucleotides or, optionally, 1-10 nucleotides. In an example, N comprises at least 9 consecutive phosphorothioate linkages. In one example, the top strand is the sense strand, and the bottom strand is the antisense strand. Alternatively, the bottom strand is the sense strand and the top strand is the antisense strand.

In one such example, the dsNA comprises

5'-$(Zm)_4(Z)_{25}$N-3'

3'-Y$(Z)_{25}$N-5' wherein "Y" is an optional overhang domain comprised of 0-10 RNA monomers that are optionally 2'-O-methyl RNA monomers and or 2'-Fluoro monomers-in certain examples, "Y" is an overhang domain comprised of 0-4 RNA monomers that are optionally 2'-O-methyl RNA monomers or 2'-Fluoro monomers, "Z"=DNA, RNA, or modified nucleotide that are optionally 2'-O-methyl RNA monomers and or 2'-Fluoro monomers , Zm is a ligand modified nucleotide that are optionally thymines or adenosines conjugated with GalNac through sugar or base of the nucleotide, and "N"=1 to 50 or more, but is optionally 1-30 nucleotides or, optionally 1-15 nucleotides or, optionally, 1-10 nucleotides. In an example, N comprises at least 9 consecutive phosphorothioate linkages. In one example, the top strand is the sense strand, and the bottom strand is the antisense strand. Alternatively, the bottom strand is the sense strand and the top strand is the antisense strand.

In one such example, the dsNA comprises:

5'-$(Zm)_4(Z)_8$N$(Z)_{25}$NDD-3'

3'-DDY$(Z)_{25}$N-5' wherein "Y" is an optional overhang domain comprised of 0-10 RNA monomers that are optionally 2'-O-methyl RNA monomers and or 2'-Fluoro monomers-in certain examples, "Y" is an overhang domain comprised of 0-4 RNA monomers that are optionally 2'-O-methyl RNA monomers or 2'-Fluoro monomers, "Z"=DNA, RNA, or modified nucleotide that are optionally 2'-O-methyl RNA monomers and or 2'-Fluoro monomers , Zm is a ligand modified nucleotide that are optionally thymines or adenosines conjugated with GalNac through sugar or base of the nucleotide, "D"=DNA" and "N"=1 to 50 nucleotides or more, but is optionally 1-30 nucleotides or, optionally 1-15 nucleotides or, optionally, 1-10. In an example, N comprises at least 9 consecutive phosphorothioate linkages. In one example, the top strand is the sense strand, and the bottom strand is the antisense strand. Alternatively, the bottom strand is the sense strand and the top strand is the antisense strand.

In a related example, the dsNA comprises:

5′-XXXXXXXXXXXXXXXXXXXXXXXN*DD-3′

3′-YXXXXXXXXXXXXXXXXXXXXXXXN*XXZN-5′

wherein "X"=RNA, "Y" is an optional overhang domain comprised of 0-10 RNA monomers that are optionally 2'-O-methyl

RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In any of the above-depicted structures, the 5' end of either the sense strand or antisense strand optionally comprises a phosphate group. In another such example, the DsiRNA comprises:

5′-XXXXXXXXXXXXXXXXXXXXXXXXXN*|EN-3′

3′-YXXXXXXXXXXXXXXXXXXXXXXXXXN*ZN-5′

wherein "X"=RNA, "X"=2'-O-methyl RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "Z"=DNA, RNA, or modified nucleotide, and "N"=1 to 50 nucleotides or more, but is optionally 1-30 or, optionally 1-15 or, optionally, 1-10. "E"=DNA, RNA, or modified nucleotide, "|"=a discontinuity, and "N"=1 to 50 or more, but is optionally 1-15 or, optionally, 1-10. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand. In one example, the top strand is the sense strand, and the bottom strand is the antisense strand. Alternatively, the bottom strand is the sense strand and the top strand is the antisense strand, with 2'-O-methyl RNA monomers located at alternating residues along the top strand, rather than the bottom strand presently depicted in the above schematic.

In another such example, the dsNA comprises:

5′-XXXXXXXXXXXXXXXXXXXXXXXXXN*|EN-3′

3′-YXXXXXXXXXXXXXXXXXXXXXXXXXN*ZN-5′

wherein "X"=RNA, "Y" is an optional overhang domain comprised of 0-10 RNA monomers that are optionally 2'-O-methyl RNA monomers-in certain examples, "Y" is an overhang domain comprised of 0-4 RNA monomers that are optionally 2'-O-methyl RNA monomers, "Z"=DNA, RNA, or modified nucleotide, and "N"=1 to 50 or more, but is optionally 1-30 or, optionally 1-15 or, optionally, 1-10. "E"=DNA, RNA, or modified nucleotide, "1"=a discontinuity, and "N"=1 to 50 or more, but is optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In one example, the top strand is the sense strand, and the bottom strand is the antisense strand. Alternatively, the bottom strand is the sense strand and the top strand is the antisense strand, with 2'-O-methyl RNA monomers located at alternating residues along the top strand, rather than the bottom strand presently depicted in the above schematic.

In a related example, the dsNA comprises:

5′-XXXXXXXXXXXXXXXXXXXXXXXN*DD|EN-3′

3′-YXXXXXXXXXXXXXXXXXXXXXXXN*XXZN-5′

wherein "X"=RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In another such example, the dsNA comprises:

5′-XXXXXXXXXXXXXXXXXXXXXXXXN*ZN-3′

3′-YXXXXXXXXXXXXXXXXXXXXXXXXXN*-5′

wherein "X"=RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "Z"=DNA, RNA, or modified nucleotide, and "N"=1 to 50 or more, but is optionally 1-30 or, optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In a related example, the dsNA comprises:

5′-XXXXXXXXXXXXXXXXXXXXXXXN*DDZN-3′

3′-YXXXXXXXXXXXXXXXXXXXXXXXN*XX-5′

wherein "X"=RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In an additional example, the dsNA comprises:

5′-XXXXXXXXXXXXXXXXXXXXXXXN*DDZN-3′

3′-YXXXXXXXXXXXXXXXXXXXXXXXN*XX-5′

wherein "X"=RNA, "X"=2'-O-methyl RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In another such example, the dsNA comprises:

5′-XXXXXXXXXXXXXXXXXXXXXXXXXN*-3′

3′-YXXXXXXXXXXXXXXXXXXXXXXXXXN*ZN-5′

wherein "X"=RNA, "X"=2'-O-methyl RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "Z"=DNA, RNA, or modified nucleotide, and "N"=1 to 50 or more, but is optionally 1-30 or, optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand. In one example, the top strand is the sense strand, and the bottom strand is the antisense strand. Alternatively, the bottom strand is the sense strand and the top strand is the antisense strand, with 2'-O-methyl RNA monomers located at alternating residues along the top strand, rather than the bottom strand presently depicted in the above schematic.

In another such example, the dsNA comprises:

5′-XXXXXXXXXXXXXXXXXXXXXXXN*DD-3′

3′-YXXXXXXXXXXXXXXXXXXXXXXXN* XXZN-5′

wherein "X"=RNA, "X"=2'-O-methyl RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1

to 50 or more, but is optionally 1-30 or, optionally 1-15 or, optionally, 1-10. In an example, N comprises at least 9 consecutive phosphorothioate linkages. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In one example, the top strand is the sense strand, and the bottom strand is the antisense strand. Alternatively, the bottom strand is the sense strand and the top strand is the antisense strand, with 2'-O-methyl RNA monomers located at alternating residues along the top strand, rather than the bottom strand presently depicted in the above schematic.

In any of the above-depicted structures, the 5' end of either the sense strand or antisense strand optionally comprises a phosphate group.

In another such example, the DsiRNA comprises:

5′-XXXXXXXXXXXXXXXXXXXXXXXXXN*|EN-3′

3′-YXXXXXXXXXXXXXXXXXXXXXXXXXN*ZN-5′

wherein "X"=RNA, "X"=2'-O-methyl RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "Z"=DNA, RNA, or modified nucleotide, and "N"=1 to 50 or more, but is optionally 1-30 or, optionally 1-15 or, optionally, 1-10. "E"=DNA, RNA, or modified nucleotide, "|"=a discontinuity, and "N"=1 to 50 or more, but is optionally 1-15 or, optionally, 1-10. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand. In one example, the top strand is the sense strand, and the bottom strand is the antisense strand. Alternatively, the bottom strand is the sense strand and the top strand is the antisense strand, with 2'-O-methyl RNA monomers located at alternating residues along the top strand, rather than the bottom strand presently depicted in the above schematic.

In a related example, the DsiRNA comprises:

5′-XXXXXXXXXXXXXXXXXXXXXXXN*DD|EN-3′

3′-YXXXXXXXXXXXXXXXXXXXXXXXN*XXZN-5′

wherein "X"=RNA, "X"=2'-O-methyl RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand. In one example, the top strand is the sense strand, and the bottom strand is the antisense strand. Alternatively, the bottom strand is the sense strand and the top strand is the antisense strand, with 2'-O-methyl RNA monomers located at alternating residues along the top strand, rather than the bottom strand presently depicted in the above schematic.

In another such example, the DsiRNA comprises:

5′-XXXXXXXXXXXXXXXXXXXXXXXXXN*ZN-3′

3′-YXXXXXXXXXXXXXXXXXXXXXXXXXN*-5

wherein "X"=RNA, "X"=2'-O-methyl RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "Z"=DNA, RNA, or modified nucleotide, and "N"=1 to 50 or more, but is optionally 1-30 or, optionally 1-15 or, optionally, 1-10. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand. In one example, the top strand is the sense strand, and the bottom strand is the antisense strand. Alternatively, the bottom strand is the sense strand and the top strand is the antisense strand, with 2'-O-methyl RNA monomers located at alternating residues along the top strand, rather than the bottom strand presently depicted in the above schematic.

In a related example, the DsiRNA comprises:

5′-XXXXXXXXXXXXXXXXXXXXXXXN*DDZN-3′

3′-YXXXXXXXXXXXXXXXXXXXXXXXN*XX-5′

wherein "X"=RNA, "X"=2'-O-methyl RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand. In one example, the top strand is the sense strand, and the bottom strand is the antisense strand. Alternatively, the bottom strand is the sense strand and the top strand is the antisense strand, with 2'-O-methyl RNA monomers located at alternating residues along the top strand, rather than the bottom strand presently depicted in the above schematic.

In one example, an extended dsNA agent is provided that comprises deoxyribonucleotides positioned at sites modeled to function via specific direction of Dicer cleavage. An exemplary structure for such a molecule is shown:

5′-XXXXXXXXXXXXXXXXXXXXXN*XXDD-3′

3′-YXXXXXXXXXXXXXXXXXXXXXN*DDXXZN-5′

wherein "X"=RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

The above structure is modeled to force Dicer to cleave a maximum of a 21mer duplex as its primary post-processing form. In examples where the bottom strand of the above structure is the antisense strand, the positioning of two deoxyribonucleotide residues at the ultimate and penultimate residues of the 5' end of the antisense strand is likely to reduce off-target effects (as prior studies have shown a 2'-O-methyl modification of at least the penultimate position from the 5' terminus of the antisense strand to reduce off-target effects; see, e.g., US 2007/0223427).

In a related example, the dsNA comprises:

5′-XXXXXXXXXXXXXXXXXXXXXN*XXDDEN-3′

3′-YXXXXXXXXXXXXXXXXXXXXXN*DDXXZN-5′

wherein "X"=RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In a related example, the dsNA comprises:

5′-XXXXXXXXXXXXXXXXXXXXXN*XXDDZN-3′

3′-YXXXXXXXXXXXXXXXXXXXXXN*DDXX-5′

wherein "X"=RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "D"=DNA, "Z"=DNA, RNA, or modified nucleotide, and "N"=1 to 50 or more, but is optionally 1-30 or, optionally 1-15 or, optionally, 1-10. "N*"=0 to 15 or more, but is optionally 0, 1, 2, 3, 4, or 5. In one example, the top strand is the sense strand, and the bottom strand is the antisense strand. Alternatively, the bottom strand is the sense strand and the top strand is the antisense strand.

In any of the above-depicted structures, the 5' end of either the sense strand or antisense strand optionally comprises a phosphate group.

In one example, the present invention provides a double stranded nucleic acid having a substantially duplexed

region between the first and second strands comprising a fully duplexed region having no unpaired bases between the 5' terminal and 3' terminal nucleotides of the first strand that are paired with corresponding nucleotides of the second strand. In another example, the present invention provides a double stranded nucleic acid having a substantially duplexed region comprising, between the 5' terminal and 3' terminal nucleotides of the first strand that are paired with corresponding nucleotides of the second strand, 1 , 2, 3, or 5 unpaired bases. In some examples, the unpaired bases are consecutive. In other examples, the unpaired bases are non-consecutive.

As used herein "DsiRNAmm" refers to a DsiRNA having a "mismatch tolerant region" containing one, two, three or four mismatched or five or six or seven or eight or nine or ten or eleven or twelve or thirteen or fourteen or fifteen or twenty or thirty mismatched base pairs of the duplex formed by the sense and antisense strands of the DsiRNA, where such mismatches are positioned within the DsiRNA at a location(s) lying between (and thus not including) the two terminal base pairs of either end of the double stranded region of the DsiRNA. The mismatched base pairs are located within a "mismatch-tolerant region" which is defined herein with respect to the location of the projected Ago2 cut site of the corresponding target nucleic acid. The mismatch tolerant region is located "upstream of' the projected Ago2 cut site of the target strand. "Upstream" in this context will be understood as the 5'-most portion of the DsiRNAmm duplex, where 5' refers to the orientation of the sense strand of the DsiRNA duplex. Therefore, the mismatch tolerant region is upstream of the base on the sense (passenger) strand that corresponds to the projected Ago2 cut site of the target nucleic acid; alternatively, when referring to the antisense (guide) strand of the DsiRNAmm, the mismatch tolerant region can also be described as positioned downstream of the base that is complementary to the projected Ago2 cut site of the target nucleic acid, that is, the 3'-most portion of the antisense strand of the DsiRNAmm (where position 1 of the antisense strand is the 5' terminal nucleotide of the antisense strand).

For example, the mismatch tolerant region is positioned between and including base pairs 3-9 when numbered from the nucleotide starting at the 5' end of the sense strand of the duplex. Therefore, a DsiRNAmm possesses a single mismatched base pair at any one of positions 3, 4, 5, 6, 7, 8 or 9 of the sense strand of a right-hand extended DsiRNA (where position 1 is the 5' terminal nucleotide of the sense strand and position 9 is the nucleotide residue of the sense strand that is immediately 5' of the projected Ago2 cut site of the target RNA sequence corresponding to the sense strand sequence). In certain examples, for a DsiRNAmm that possesses a mismatched base pair nucleotide at any of positions 3, 4, 5, 6, 7, 8 or 9 of the sense strand, the corresponding mismatched base pair nucleotide of the antisense strand not only forms a mismatched base pair with the DsiRNAmm sense strand sequence, but also forms a mismatched base pair with a DsiRNAmm target RNA sequence (thus, complementarity between the antisense strand sequence and the sense strand sequence is disrupted at the mismatched base pair within the DsiRNAmm, and complementarity is similarly disrupted between the antisense strand sequence of the DsiRNAmm and the target RNA sequence). In alternative examples, the mismatch base pair nucleotide of the antisense strand of a DsiRNAmm only form a mismatched base pair with a corresponding nucleotide of the sense strand sequence of the DsiRNAmm, yet base pairs with its corresponding target RNA sequence nucleotide (thus, complementarity between the antisense strand sequence and the sense strand sequence is disrupted at the mismatched base pair within the DsiRNAmm, yet complementarity is maintained between the antisense strand sequence of the DsiRNAmm and the target RNA sequence).

A DsiRNAmm that possesses a single mismatched base pair within the mismatch-tolerant region (mismatch region) as described above (e.g., a DsiRNAmm harboring a mismatched nucleotide residue at any one of positions 3, 4, 5, 6, 7, 8 or 9 of the sense strand) can further include one, two or even three additional mismatched base pairs. In preferred examples, these one, two or three additional mismatched base pairs of the DsiRNAmm occur at position(s) 3, 4, 5, 6, 7, 8 and/or 9 of the sense strand (and at corresponding residues of the antisense strand). In one example where one additional mismatched base pair is present within a DsiRNAmm, the two mismatched base pairs of the sense strand can occur, e.g., at nucleotides of both position 4 and position 6 of the sense strand (with mismatch also occurring at corresponding nucleotide residues of the antisense strand).

In DsiRNAmm agents possessing two mismatched base pairs, mismatches can occur consecutively (e.g., at consecutive positions along the sense strand nucleotide sequence). Alternatively, nucleotides of the sense strand that form mismatched base pairs with the antisense strand sequence can be interspersed by nucleotides that base pair with the antisense strand sequence (e.g., for a DsiRNAmm possessing mismatched nucleotides at positions 3 and 6, but not at positions 4 and 5, the mismatched residues of sense strand positions 3 and 6 are interspersed by two nucleotides that form matched base pairs with corresponding residues of the antisense strand). For example, two residues of the sense strand (located within the mismatch-tolerant region of the sense strand) that form mismatched base pairs with the corresponding antisense strand sequence can occur with zero, one, two, three, four or five matched base pairs located between these mismatched base pairs.

For certain DsiRNAmm agents possessing three mismatched base pairs, mismatches can occur consecutively (e.g., in a triplet along the sense strand nucleotide sequence). Alternatively, nucleotides of the sense strand that form mismatched base pairs with the antisense strand sequence can be interspersed by nucleotides that form matched base pairs with the antisense strand sequence (e.g., for a DsiRNAmm possessing mismatched nucleotides at positions 3, 4 and 8, but not at positions 5, 6 and 7, the mismatched residues of sense strand positions 3 and 4 are adjacent to one

another, while the mismatched residues of sense strand positions 4 and 8 are interspersed by three nucleotides that form matched base pairs with corresponding residues of the antisense strand). For example, three residues of the sense strand (located within the mismatch-tolerant region of the sense strand) that form mismatched base pairs with the corresponding antisense strand sequence can occur with zero, one, two, three or four matched base pairs located between any two of these mismatched base pairs.

For certain DsiRNAmm agents possessing four mismatched base pairs, mismatches can occur consecutively (e.g., in a quadruplet along the sense strand nucleotide sequence). Alternatively, nucleotides of the sense strand that form mismatched base pairs with the antisense strand sequence can be interspersed by nucleotides that form matched base pairs with the antisense strand sequence (e.g., for a DsiRNAmm possessing mismatched nucleotides at positions 3, 5, 7 and 8, but not at positions 4 and 6, the mismatched residues of sense strand positions 7 and 8 are adjacent to one another, while the mismatched residues of sense strand positions 3 and 5 are interspersed by one nucleotide that forms a matched base pair with the corresponding residue of the antisense strand-similarly, the mismatched residues of sense strand positions 5 and 7 are also interspersed by one nucleotide that forms a matched base pair with the corresponding residue of the antisense strand). For example, four residues of the sense strand (located within the mismatch-tolerant region of the sense strand) that form mismatched base pairs with the corresponding antisense strand sequence can occur with zero, one, two or three matched base pairs located between any two of these mismatched base pairs.

In another example, a DsiRNAmm comprises a mismatch tolerant region which possesses a single mismatched base pair nucleotide at any one of positions 13, 14, 15, 16, 17, 18, 19, 20 or 21 of the antisense strand of a left-hand extended DsiRNA (where position 1 is the 5' terminal nucleotide of the antisense strand and position 13 is the nucleotide residue of the antisense strand that is immediately 3' (downstream) in the antisense strand of the projected Ago2 cut site of the target RNA sequence sufficiently complementary to the antisense strand sequence). In certain examples, for a DsiRNAmm that possesses a mismatched base pair nucleotide at any of positions 13, 14, 15, 16, 17, 18, 19, 20 or 21 of the antisense strand with respect to the sense strand of the DsiRNAmm, the mismatched base pair nucleotide of the antisense strand not only forms a mismatched base pair with the DsiRNAmm sense strand sequence, but also forms a mismatched base pair with a DsiRNAmm target RNA sequence (thus, complementarity between the antisense strand sequence and the sense strand sequence is disrupted at the mismatched base pair within the DsiRNAmm, and complementarity is similarly disrupted between the antisense strand sequence of the DsiRNAmm and the target RNA sequence). In alternative examples, the mismatch base pair nucleotide of the antisense strand of a DsiRNAmm only forms a mismatched base pair with a corresponding nucleotide of the sense strand sequence of the DsiRNAmm, yet base pairs with its corresponding target RNA sequence nucleotide (thus, complementarity between the antisense strand sequence and the sense strand sequence is disrupted at the mismatched base pair within the DsiRNAmm, yet complementarity is maintained between the antisense strand sequence of the DsiRNAmm and the target RNA sequence).

A DsiRNAmm of the invention that possesses a single mismatched base pair within the mismatch-tolerant region as described above (e.g., a DsiRNAmm harboring a mismatched nucleotide residue at positions 13, 14, 15, 16, 17, 18, 19, 20 or 21 of the antisense strand) can further include one, two or even three additional mismatched base pairs. In preferred examples, these one, two or three additional mismatched base pairs of the DsiRNAmm occur at position(s) 13, 14, 15, 16, 17, 18, 19, 20 and/or 21 of the antisense strand (and at corresponding residues of the sense strand). In one example where one additional mismatched base pair is present within a DsiRNAmm, the two mismatched base pairs of the antisense strand can occur, e.g., at nucleotides of both position 14 and position 18 of the antisense strand (with mismatch also occurring at corresponding nucleotide residues of the sense strand).

In DsiRNAmm agents possessing two mismatched base pairs, mismatches can occur consecutively (e.g., at consecutive positions along the antisense strand nucleotide sequence). Alternatively, nucleotides of the antisense strand that form mismatched base pairs with the sense strand sequence can be interspersed by nucleotides that base pair with the sense strand sequence (e.g., for a DsiRNAmm possessing mismatched nucleotides at positions 13 and 16, but not at positions 14 and 15, the mismatched residues of antisense strand positions 13 and 16 are interspersed by two nucleotides that form matched base pairs with corresponding residues of the sense strand). For example, two residues of the antisense strand (located within the mismatch-tolerant region of the sense strand) that form mismatched base pairs with the corresponding sense strand sequence can occur with zero, one, two, three, four, five, six or seven matched base pairs located between these mismatched base pairs.

For certain DsiRNAmm agents possessing three mismatched base pairs, mismatches can occur consecutively (e.g., in a triplet along the antisense strand nucleotide sequence). Alternatively, nucleotides of the antisense strand that form mismatched base pairs with the sense strand sequence can be interspersed by nucleotides that form matched base pairs with the sense strand sequence (e.g., for a DsiRNAmm possessing mismatched nucleotides at positions 13, 14 and 18, but not at positions 15, 16 and 17, the mismatched residues of antisense strand positions 13 and 14 are adjacent to one another, while the mismatched residues of antisense strand positions 14 and 18 are interspersed by three nucleotides that form matched base pairs with corresponding residues of the sense strand). For example, three residues of the antisense strand (located within the mismatch-tolerant region of the antisense strand) that form mismatched base pairs with the corresponding sense strand sequence can occur with zero, one, two, three, four, five or six matched base pairs located between any two of these mismatched base pairs.

For certain DsiRNAmm agents possessing four mismatched base pairs, mismatches can occur consecutively (e.g., in a quadruplet along the antisense strand nucleotide sequence). Alternatively, nucleotides of the antisense strand that form mismatched base pairs with the sense strand sequence can be interspersed by nucleotides that form matched base pairs with the sense strand sequence (e.g., for a DsiRNAmm possessing mismatched nucleotides at positions 13, 15, 17 and 18, but not at positions 14 and 16, the mismatched residues of antisense strand positions 17 and 18 are adjacent to one another, while the mismatched residues of antisense strand positions 13 and 15 are interspersed by one nucleotide that forms a matched base pair with the corresponding residue of the sense strand-similarly, the mismatched residues of antisense strand positions 15 and 17 are also interspersed by one nucleotide that forms a matched base pair with the corresponding residue of the sense strand). For example, four residues of the antisense strand (located within the mismatch-tolerant region of the antisense strand) that form mismatched base pairs with the corresponding sense strand sequence can occur with zero, one, two, three, four or five matched base pairs located between any two of these mismatched base pairs.

In a further example, a DsiRNAmm possesses a single mismatched base pair nucleotide at any one of positions 11, 12, 13, 14, 15, 16, 17, 18 or 19 of the antisense strand of a left-hand extended DsiRNA (where position 1 is the 5' terminal nucleotide of the antisense strand and position 11 is the nucleotide residue of the antisense strand that is immediately 3' (downstream) in the antisense strand of the projected Ago2 cut site of the target RNA sequence sufficiently complementary to the antisense strand sequence). In certain examples, for a DsiRNAmm that possesses a mismatched base pair nucleotide at any of positions 11, 12, 13, 14, 15, 16, 17, 18 or 19 of the antisense strand with respect to the sense strand of the DsiRNAmm, the mismatched base pair nucleotide of the antisense strand not only forms a mismatched base pair with the DsiRNAmm sense strand sequence, but also forms a mismatched base pair with a DsiRNAmm target RNA sequence (thus, complementarity between the antisense strand sequence and the sense strand sequence is disrupted at the mismatched base pair within the DsiRNAmm, and complementarity is similarly disrupted between the antisense strand sequence of the DsiRNAmm and the target RNA sequence). In alternative examples, the mismatch base pair nucleotide of the antisense strand of a DsiRNAmm only forms a mismatched base pair with a corresponding nucleotide of the sense strand sequence of the DsiRNAmm, yet this same antisense strand nucleotide base pairs with its corresponding target RNA sequence nucleotide (thus, complementarity between the antisense strand sequence and the sense strand sequence is disrupted at the mismatched base pair within the DsiRNAmm, yet complementarity is maintained between the antisense strand sequence of the DsiRNAmm and the target RNA sequence).

A DsiRNAmm that possesses a single mismatched base pair within the mismatch-tolerant region as described above (e.g., a DsiRNAmm harboring a mismatched nucleotide residue at positions 11, 12, 13, 14, 15, 16, 17, 18 or 19 of the antisense strand) can further include one, two or even three additional mismatched base pairs. In preferred examples, these one, two or three additional mismatched base pairs of the DsiRNAmm occur at position(s) 11, 12, 13, 14, 15, 16, 17, 18 and/or 19 of the antisense strand (and at corresponding residues of the sense strand). In one example where one additional mismatched base pair is present within a DsiRNAmm, the two mismatched base pairs of the antisense strand can occur, e.g., at nucleotides of both position 14 and position 18 of the antisense strand (with mismatch also occurring at corresponding nucleotide residues of the sense strand).

In DsiRNAmm agents possessing two mismatched base pairs, mismatches can occur consecutively (e.g., at consecutive positions along the antisense strand nucleotide sequence). Alternatively, nucleotides of the antisense strand that form mismatched base pairs with the sense strand sequence can be interspersed by nucleotides that base pair with the sense strand sequence (e.g., for a DsiRNAmm possessing mismatched nucleotides at positions 12 and 15, but not at positions 13 and 14, the mismatched residues of antisense strand positions 12 and 15 are interspersed by two nucleotides that form matched base pairs with corresponding residues of the sense strand). For example, two residues of the antisense strand (located within the mismatch-tolerant region of the sense strand) that form mismatched base pairs with the corresponding sense strand sequence can occur with zero, one, two, three, four, five, six or seven matched base pairs located between these mismatched base pairs.

For certain DsiRNAmm agents possessing three mismatched base pairs, mismatches can occur consecutively (e.g., in a triplet along the antisense strand nucleotide sequence). Alternatively, nucleotides of the antisense strand that form mismatched base pairs with the sense strand sequence can be interspersed by nucleotides that form matched base pairs with the sense strand sequence (e.g., for a DsiRNAmm possessing mismatched nucleotides at positions 13, 14 and 18, but not at positions 15, 16 and 17, the mismatched residues of antisense strand positions 13 and 14 are adjacent to one another, while the mismatched residues of antisense strand positions 14 and 18 are interspersed by three nucleotides that form matched base pairs with corresponding residues of the sense strand). For example, three residues of the antisense strand (located within the mismatch-tolerant region of the antisense strand) that form mismatched base pairs with the corresponding sense strand sequence can occur with zero, one, two, three, four, five or six matched base pairs located between any two of these mismatched base pairs.

For certain DsiRNAmm agents possessing four mismatched base pairs, mismatches can occur consecutively

(e.g., in a quadruplet along the antisense strand nucleotide sequence). Alternatively, nucleotides of the antisense strand that form mismatched base pairs with the sense strand sequence can be interspersed by nucleotides that form matched base pairs with the sense strand sequence (e.g., for a DsiRNAmm possessing mismatched nucleotides at positions 13, 15, 17 and 18, but not at positions 14 and 16, the mismatched residues of antisense strand positions 17 and 18 are adjacent to one another, while the mismatched residues of antisense strand positions 13 and 15 are interspersed by one nucleotide that forms a matched base pair with the corresponding residue of the sense strand-similarly, the mismatched residues of antisense strand positions 15 and 17 are also interspersed by one nucleotide that forms a matched base pair with the corresponding residue of the sense strand). For example, four residues of the antisense strand (located within the mismatch-tolerant region of the antisense strand) that form mismatched base pairs with the corresponding sense strand sequence can occur with zero, one, two, three, four or five matched base pairs located between any two of these mismatched base pairs.

In an additional example, a DsiRNAmm possesses a single mismatched base pair nucleotide at any one of positions 15, 16, 17, 18, 19, 20, 21, 22 or 23 of the antisense strand of a left-hand extended DsiRNA (where position 1 is the 5' terminal nucleotide of the antisense strand and position 15 is the nucleotide residue of the antisense strand that is immediately 3' (downstream) in the antisense strand of the projected Ago2 cut site of the target RNA sequence sufficiently complementary to the antisense strand sequence). In certain examples, for a DsiRNAmm that possesses a mismatched base pair nucleotide at any of positions 15, 16, 17, 18, 19, 20, 21, 22 or 23 of the antisense strand with respect to the sense strand of the DsiRNAmm, the mismatched base pair nucleotide of the antisense strand not only forms a mismatched base pair with the DsiRNAmm sense strand sequence, but also forms a mismatched base pair with a DsiRNAmm target RNA sequence (thus, complementarity between the antisense strand sequence and the sense strand sequence is disrupted at the mismatched base pair within the DsiRNAmm, and complementarity is similarly disrupted between the antisense strand sequence of the DsiRNAmm and the target RNA sequence). In alternative examples, the mismatch base pair nucleotide of the antisense strand of a DsiRNAmm only forms a mismatched base pair with a corresponding nucleotide of the sense strand sequence of the DsiRNAmm, yet this same antisense strand nucleotide base pairs with its corresponding target RNA sequence nucleotide (thus, complementarity between the antisense strand sequence and the sense strand sequence is disrupted at the mismatched base pair within the DsiRNAmm, yet complementarity is maintained between the antisense strand sequence of the DsiRNAmm and the target RNA sequence).

A DsiRNAmm that possesses a single mismatched base pair within the mismatch-tolerant region as described above (e.g., a DsiRNAmm harboring a mismatched nucleotide residue at positions 15, 16, 17, 18, 19, 20, 21, 22 or 23 of the antisense strand) can further include one, two or even three additional mismatched base pairs. In preferred examples, these one, two or three additional mismatched base pairs of the DsiRNAmm occur at position(s) 15, 16, 17, 18, 19, 20, 21, 22 and/or 23 of the antisense strand (and at corresponding residues of the sense strand). In one example where one additional mismatched base pair is present within a DsiRNAmm, the two mismatched base pairs of the antisense strand can occur, e.g., at nucleotides of both position 16 and position 20 of the antisense strand (with mismatch also occurring at corresponding nucleotide residues of the sense strand).

In DsiRNAmm agents possessing two mismatched base pairs, mismatches can occur consecutively (e.g., at consecutive positions along the antisense strand nucleotide sequence). Alternatively, nucleotides of the antisense strand that form mismatched base pairs with the sense strand sequence can be interspersed by nucleotides that base pair with the sense strand sequence (e.g., for a DsiRNAmm possessing mismatched nucleotides at positions 16 and 20, but not at positions 17, 18 and 19, the mismatched residues of antisense strand positions 16 and 20 are interspersed by three nucleotides that form matched base pairs with corresponding residues of the sense strand). For example, two residues of the antisense strand (located within the mismatch-tolerant region of the sense strand) that form mismatched base pairs with the corresponding sense strand sequence can occur with zero, one, two, three, four, five, six or seven matched base pairs located between these mismatched base pairs.

For certain DsiRNAmm agents possessing three mismatched base pairs, mismatches can occur consecutively (e.g., in a triplet along the antisense strand nucleotide sequence). Alternatively, nucleotides of the antisense strand that form mismatched base pairs with the sense strand sequence can be interspersed by nucleotides that form matched base pairs with the sense strand sequence (e.g., for a DsiRNAmm possessing mismatched nucleotides at positions 16, 17 and 21, but not at positions 18, 19 and 20, the mismatched residues of antisense strand positions 16 and 17 are adjacent to one another, while the mismatched residues of antisense strand positions 17 and 21 are interspersed by three nucleotides that form matched base pairs with corresponding residues of the sense strand). For example, three residues of the antisense strand (located within the mismatch-tolerant region of the antisense strand) that form mismatched base pairs with the corresponding sense strand sequence can occur with zero, one, two, three, four, five or six matched base pairs located between any two of these mismatched base pairs.

For certain DsiRNAmm agents possessing four mismatched base pairs, mismatches can occur consecutively (e.g., in a quadruplet along the antisense strand nucleotide sequence). Alternatively, nucleotides of the antisense strand that form mismatched base pairs with the sense strand sequence can be interspersed by nucleotides that form matched

base pairs with the sense strand sequence (e.g., for a DsiRNAmm possessing mismatched nucleotides at positions 17, 19, 21 and 22, but not at positions 18 and 20, the mismatched residues of antisense strand positions 21 and 22 are adjacent to one another, while the mismatched residues of antisense strand positions 17 and 19 are interspersed by one nucleotide that forms a matched base pair with the corresponding residue of the sense strand-similarly, the mismatched residues of antisense strand positions 19 and 21 are also interspersed by one nucleotide that forms a matched base pair with the corresponding residue of the sense strand). For example, four residues of the antisense strand (located within the mismatch-tolerant region of the antisense strand) that form mismatched base pairs with the corresponding sense strand sequence can occur with zero, one, two, three, four or five matched base pairs located between any two of these mismatched base pairs.

For reasons of clarity, the location(s) of mismatched nucleotide residues within the above DsiRNAmm agents are numbered in reference to the 5' terminal residue of either sense or antisense strands of the DsiRNAmm. The numbering of positions located within the mismatch-tolerant region (mismatch region) of the antisense strand can shift with variations in the proximity of the 5' terminus of the antisense strand to the projected Ago2 cleavage site. Thus, the location(s) of preferred mismatch sites within either antisense strand or sense strand can also be identified as the permissible proximity of such mismatches to the projected Ago2 cut site. Accordingly, in one preferred example, the position of a mismatch nucleotide of the sense strand of a DsiRNAmm is the nucleotide residue of the sense strand that is located immediately 5' (upstream) of the projected Ago2 cleavage site of the corresponding target RNA sequence. In other preferred examples, a mismatch nucleotide of the sense strand of a DsiRNAmm is positioned at the nucleotide residue of the sense strand that is located two nucleotides 5' (upstream) of the projected Ago2 cleavage site, three nucleotides 5' (upstream) of the projected Ago2 cleavage site, four nucleotides 5' (upstream) of the projected Ago2 cleavage site, five nucleotides 5' (upstream) of the projected Ago2 cleavage site, six nucleotides 5' (upstream) of the projected Ago2 cleavage site, seven nucleotides 5' (upstream) of the projected Ago2 cleavage site, eight nucleotides 5' (upstream) of the projected Ago2 cleavage site, or nine nucleotides 5' (upstream) of the projected Ago2 cleavage site.

Exemplary single mismatch-containing, 5' guide single strand extended DsiRNAs (DsiRNAmm) include the following structures (such mismatch-containing structures may also be incorporated into other exemplary DsiRNA structures shown herein).

5′-XXMXXXXXXXXXXXXXXXXXXXN*DD-3′
3′-YXXMXXXXXXXXXXXXXXXXXXXN*XXZN-5′

5′-XXXMXXXXXXXXXXXXXXXXXXN*DD-3′
3′-YXXXMXXXXXXXXXXXXXXXXXXN*XXZN-5′

5′-XXXXMXXXXXXXXXXXXXXXXXN*DD-3′
3′-YXXXXMXXXXXXXXXXXXXXXXXN*XXZN-5′

5′-XXXXXMXXXXXXXXXXXXXXXXN*DD-3′
3′-YXXXXXMXXXXXXXXXXXXXXXXN*XXZN-5′

5′-XXXXXXMXXXXXXXXXXXXXXXN*DD-3′
3′-YXXXXXXMXXXXXXXXXXXXXXXN*XXZN-5′

5′-XXXXXXXMXXXXXXXXXXXXXXN*DD-3′
3′-YXXXXXXXMXXXXXXXXXXXXXXN*XXZN-5′

5′-XXXXXXXXMXXXXXXXXXXXXXN*DD-3′
3′-YXXXXXXXXMXXXXXXXXXXXXXN*XXZN-5′

Wherein "X"=RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "Z"=DNA, RNA, or modified nucleotide, "N"=1 to 50 or more, but is optionally

1-30 or, optionally 1-15 or, optionally, 1-10. "N*"=0 to 15 or more, but is optionally 0, 1, 2, 3, 4, or 5, and "D"=DNA, "M"=Nucleic acid residues (RNA, DNA or non-natural or modified nucleic acids) that do not base pair (hydrogen bond) with corresponding "M" residues of otherwise complementary strand when strands are annealed. Any of the residues of such agents can optionally be 2'-O-methyl RNA monomers-alternating positioning of 2'-O-methyl RNA monomers that commences from the 3'-terminal residue of the bottom (second) strand, as shown above, can also be used in the above DsiRNAmm agents. For the above mismatch structures, the top strand is the sense strand, and the bottom strand is the antisense strand.

In certain examples, a DsiRNA can contain mismatches that exist in reference to the target RNA sequence yet do not necessarily exist as mismatched base pairs within the two strands of the DsiRNA-thus, a DsiRNA can possess perfect complementarity between first and second strands of a DsiRNA, yet still possess mismatched residues in reference to a target RNA (which, in certain examples, may be advantageous in promoting efficacy and/or potency and/or duration of effect). In certain examples, where mismatches occur between antisense strand and target RNA sequence, the position of a mismatch is located within the antisense strand at a position(s) that corresponds to a sequence of the sense strand located 5' of the projected Ago2 cut site of the target region-e.g., antisense strand residue(s) positioned within the antisense strand to the 3' of the antisense residue which is complementary to the projected Ago2 cut site of the target sequence.

Exemplary 25/27mer DsiRNAs that harbor a single mismatched residue in reference to target sequences include the following preferred structures;

Target RNA Sequence: 5'-. AXXXXXXXXXXXXXXXXXXXX.-3'
DsiRNAmm Sense Strand: 5'-XXXXXXXXXXXXXXXXXXXXXXXN*DD-3'
DsiRNAmm Antisense Strand: 3'-EXXXXXXXXXXXXXXXXXXXXXXXXN*XXZN-5'
Target RNA Sequence: 5'-.. XAXXXXXXXXXXXXXXXXXXX -3'
DsiRNAmm Sense Strand: 5'-XXXXXXXXXXXXXXXXXXXXXXXN*DD-3'
DsiRNAmm Antisense Strand: 3'-XEXXXXXXXXXXXXXXXXXXXXXXXN*XXZN-5'
Target RNA Sequence: 5'- ... AXXXXXXXXXXXXXXXXXXX-3'
DsiRNAmm Sense Strand: 5'-BXXXXXXXXXXXXXXXXXXXXXXN*DD-3'
DsiRNAmm Antisense Strand: 3'-XXEXXXXXXXXXXXXXXXXXXXXXXN*XXZN-5'
Target RNA Sequence: 5'- ... XAXXXXXXXXXXXXXXXXX .-3'
DsiRNAmm Sense Strand: 5'-XBXXXXXXXXXXXXXXXXXXXXXN*DD-3'
DsiRNAmm Antisense Strand: 3'-XXXEXXXXXXXXXXXXXXXXXXXXXN*XXZN-5'
Target RNA Sequence: 5'- ... XXAXXXXXXXXXXXXXXXX .-3'
DsiRNAmm Sense Strand: 5'-XXBXXXXXXXXXXXXXXXXXXXXN*DD-3'
DsiRNAmm Antisense Strand: 3'-XXXXEXXXXXXXXXXXXXXXXXXXXN*XXZN-5'
Target RNA Sequence: 5'- ... XXXAXXXXXXXXXXXXXXXX.-3'
DsiRNAmm Sense Strand: 5'-XXXBXXXXXXXXXXXXXXXXXXXN*DD-3'
DsiRNAmm Antisense Strand: 3'-XXXXXEXXXXXXXXXXXXXXXXXXXN*XXZN-5'
Target RNA Sequence: 5'- ... XXXXAXXXXXXXXXXXXXXX.-3'
DsiRNAmm Sense Strand: 5'-XXXXBXXXXXXXXXXXXXXXXXXN*DD-3'
DsiRNAmm Antisense Strand: 3'-XXXXXXEXXXXXXXXXXXXXXXXXXN*XXZN-5'
Target RNA Sequence: 5'- ... XXXXXAXXXXXXXXXXXXXX.-3'
DsiRNAmm Sense Strand: 5'-XXXXXBXXXXXXXXXXXXXXXXXN*DD-3'
DsiRNAmm Antisense Strand: 3'-XXXXXXXEXXXXXXXXXXXXXXXXXN*XXZN-5'
Target RNA Sequence: 5'- ... XXXXXXAXXXXXXXXXXXXX.-3'
DsiRNAmm Sense Strand: 5'-XXXXXXBXXXXXXXXXXXXXXXXN*DD-3'
DsiRNAmm Antisense Strand: 3'-XXXXXXXXEXXXXXXXXXXXXXXXXN*XXZN-5'
Target RNA Sequence: 5'- ... XXXXXXXAXXXXXXXXXXXX.-3'
DsiRNAmm Sense Strand: 5'-XXXXXXXBXXXXXXXXXXXXXXXN*DD-3'
DsiRNAmm Antisense Strand: 3'-XXXXXXXXXEXXXXXXXXXXXXXXXN*XXZN-5'
Target RNA Sequence: 5'- ... XXXXXXXXAXXXXXXXXXXX.-3'
DsiRNAmm Sense Strand: 5'-XXXXXXXXBXXXXXXXXXXXXXXN*DD-3'
DsiRNAmm Antisense Strand: 3'-XXXXXXXXXXEXXXXXXXXXXXXXXN*XXZN-5'

Wherein "X"=RNA, "Y" is an optional overhang domain comprised of 0-10 RNA monomers that are optionally 2'-O-methyl RNA monomers-in certain examples, "Y" is an overhang domain comprised of 1-4 RNA monomers that are optionally 2'-O-methyl RNA monomers, "Z"=DNA, RNA, or modified nucleotide, "N"=1 to 50 or more, but is optionally 1-30 or, optionally 1-15 or, optionally, 1-10. "N*"=0 to 15 or more, but is optionally 0, 1, 2, 3, 4, or 5, "D"=DNA, "p"=a phosphate group, "E"=Nucleic acid residues (RNA, DNA or non-natural or modified nucleic acids) that do not base pair

(hydrogen bond) with corresponding "A" RNA residues of otherwise complementary (target) strand when strands are annealed, yet optionally do base pair with corresponding "B" residues ("B" residues are also RNA, DNA or non-natural or modified nucleic acids). Any of the residues of such agents can optionally be 2'-O-methyl RNA monomers-e.g., alternating positioning of 2'-O-methyl RNA monomers that commences from the 3'-terminal residue of the bottom (second) strand, as shown above, or other patterns of 2'-O-methyl and/or other modifications as described herein can also be used in the above DsiRNA agents.

In addition to the above-exemplified structures, DsiRNAs can also possess one, two or three additional residues that form further mismatches with the target RNA sequence. Such mismatches can be consecutive, or can be interspersed by nucleotides that form matched base pairs with the target RNA sequence. Where interspersed by nucleotides that form matched base pairs, mismatched residues can be spaced apart from each other within a single strand at an interval of one, two, three, four, five, six, seven or even eight base paired nucleotides between such mismatch-forming residues.

As for the above-described DsiRNAmm agents, a preferred location within DsiRNAs for antisense strand nucleotides that form mismatched base pairs with target RNA sequence (yet may or may not form mismatches with corresponding sense strand nucleotides) is within the antisense strand region that is located 3' (downstream) of the antisense strand sequence which is complementary to the projected Ago2 cut site of the DsiRNA. Thus, in one preferred example, the position of a mismatch nucleotide (in relation to the target RNA sequence) of the antisense strand of a DsiRNAmm is the nucleotide residue of the antisense strand that is located immediately 3' (downstream) within the antisense strand sequence of the projected Ago2 cleavage site of the corresponding target RNA sequence. In other preferred examples, a mismatch nucleotide of the antisense strand of a DsiRNAmm (in relation to the target RNA sequence) is positioned at the nucleotide residue of the antisense strand that is located two nucleotides 3' (downstream) of the corresponding projected Ago2 cleavage site, three nucleotides 3' (downstream) of the corresponding projected Ago2 cleavage site, four nucleotides 3' (downstream) of the corresponding projected Ago2 cleavage site, five nucleotides 3' (downstream) of the corresponding projected Ago2 cleavage site, six nucleotides 3' (downstream) of the projected Ago2 cleavage site, seven nucleotides 3' (downstream) of the projected Ago2 cleavage site, eight nucleotides 3' (downstream) of the projected Ago2 cleavage site, or nine nucleotides 3' (downstream) of the projected Ago2 cleavage site.

In DsiRNA agents possessing two mismatch-forming nucleotides of the antisense strand (where mismatch-forming nucleotides are mismatch forming in relation to target RNA sequence), mismatches can occur consecutively (e.g., at consecutive positions along the antisense strand nucleotide sequence). Alternatively, nucleotides of the antisense strand that form mismatched base pairs with the target RNA sequence can be interspersed by nucleotides that base pair with the target RNA sequence (e.g., for a DsiRNA possessing mismatch-forming nucleotides at positions 13 and 16 (starting from the 5' terminus (position 1) of the antisense strand), but not at positions 14 and 15, the mismatched residues of sense strand positions 13 and 16 are interspersed by two nucleotides that form matched base pairs with corresponding residues of the target RNA sequence). For example, two residues of the antisense strand (located within the mismatch-tolerant region of the antisense strand) that form mismatched base pairs with the corresponding target RNA sequence can occur with zero, one, two, three, four or five matched base pairs (with respect to target RNA sequence) located between these mismatch-forming base pairs.

For certain DsiRNAs possessing three mismatch-forming base pairs (mismatch-forming with respect to target RNA sequence), mismatch-forming nucleotides can occur consecutively (e.g., in a triplet along the antisense strand nucleotide sequence). Alternatively, nucleotides of the antisense strand that form mismatched base pairs with the target RNA sequence can be interspersed by nucleotides that form matched base pairs with the target RNA sequence (e.g., for a DsiRNA possessing mismatched nucleotides at positions 13, 14 and 18, but not at positions 15, 16 and 17, the mismatch-forming residues of antisense strand positions 13 and 14 are adjacent to one another, while the mismatch-forming residues of antisense strand positions 14 and 18 are interspersed by three nucleotides that form matched base pairs with corresponding residues of the target RNA). For example, three residues of the antisense strand (located within the mismatch-tolerant region of the antisense strand) that form mismatched base pairs with the corresponding target RNA sequence can occur with zero, one, two, three or four matched base pairs located between any two of these mismatch-forming base pairs.

For certain DsiRNAs possessing four mismatch-forming base pairs (mismatch-forming with respect to target RNA sequence), mismatch-forming nucleotides can occur consecutively (e.g., in a quadruplet along the sense strand nucleotide sequence). Alternatively, nucleotides of the antisense strand that form mismatched base pairs with the target RNA sequence can be interspersed by nucleotides that form matched base pairs with the target RNA sequence (e.g., for a DsiRNA possessing mismatch-forming nucleotides at positions 13, 15, 17 and 18, but not at positions 14 and 16, the mismatch-forming residues of antisense strand positions 17 and 18 are adjacent to one another, while the mismatch-forming residues of antisense strand positions 13 and 15 are interspersed by one nucleotide that forms a matched base pair with the corresponding residue of the target RNA sequence-similarly, the mismatch-forming residues of antisense strand positions 15 and 17 are also interspersed by one nucleotide that forms a matched base pair with the corresponding residue of the target RNA sequence). For example, four residues of the antisense strand (located within the mismatch-tolerant region of the antisense strand) that form mismatched base pairs with the corresponding target RNA sequence

can occur with zero, one, two or three matched base pairs located between any two of these mismatch-forming base pairs.

The above DsiRNAmm and other DsiRNA structures are described in order to exemplify certain structures of DsiRNAmm and DsiRNA agents. Design of the above DsiRNAmm and DsiRNA structures can be adapted to generate, e.g., DsiRNAmm forms of an extended DsiRNA agent shown infra (including, e.g., design of mismatch-containing DsiRNAmm agents). As exemplified above, DsiRNAs can also be designed that possess single mismatches (or two, three or four mismatches) between the antisense strand of the DsiRNA and a target sequence, yet optionally can retain perfect complementarity between sense and antisense strand sequences of a DsiRNA.

It is further noted that the DsiRNA agents exemplified infra can also possess insertion/deletion (in/del) structures within their double-stranded and/or target RNA-aligned structures. Accordingly, the DsiRNAs can be designed to possess in/del variations in, e.g., antisense strand sequence as compared to target RNA sequence and/or antisense strand sequence as compared to sense strand sequence, with preferred location(s) for placement of such in/del nucleotides corresponding to those locations described above for positioning of mismatched and/or mismatch-forming base pairs.

In certain examples, the "D" residues of any of the above structures include at least one PS-DNA or PS-RNA. Optionally, the "D" residues of any of the above structures include at least one modified nucleotide that inhibits Dicer cleavage.

In one example, the DsiRNA agent has an asymmetric structure, with the sense strand having a 25-base pair length, the antisense strand having a 42-nucleotide length with a 2 base 3'-overhang (and, therefore, the DsiRNA agent possesses a 5' overhang 15 nucleotides in length at the 3' end of the sense strand/5' end of the antisense strand), and with deoxyribonucleotides located at positions 24 and 25 of the sense strand (numbering from position 1 at the 5' of the sense strand) and each base paired with a cognate nucleotide of the antisense strand. The 5' overhang comprises a modified nucleotide, preferably a 2'-O-methyl ribonucleotide, and/or a phosphate backbone modification, preferably phosphorothioate.

In another example, the DsiRNA agent has a structure, with the sense strand having a 40-nucleotide length, the antisense strand having a 27-nucleotide length with a 2 base 3'-overhang (and, therefore, the DsiRNA agent possesses a 3' overhang 15 nucleotides in length at the 3' end of the sense strand/5' end of the antisense strand), and with deoxyribonucleotides located at positions 24 and 25 of the sense strand (numbering from position 1 at the 5' of the sense strand) and each base paired with a cognate nucleotide of the antisense strand. The 3' overhang comprises a deoxyribonucleotide and/or a phosphate backbone modification, preferably methylphosphonate.

## Modification of dsNAs

One major factor that inhibits the effect of double stranded RNAs ("dsRNAs") is the degradation of dsRNAs (e.g., siRNAs and DsiRNAs) by nucleases. A 3'-exonuclease is the primary nuclease activity present in serum and modification of the 3'-ends of antisense DNA oligonucleotides is crucial to prevent degradation (Eder et al., 1991). An RNase-T family nuclease has been identified called ERI-1 which has 3' to 5' exonuclease activity that is involved in regulation and degradation of siRNAs (Kennedy et al., 2004; Hong et al., 2005). This gene is also known as Thex1 (NM-02067) in mice or THEX1 (NM-153332) in humans and is involved in degradation of histone mRNA; it also mediates degradation of 3'-overhangs in siRNAs, but does not degrade duplex RNA (Yang et al., 2006). It is therefore reasonable to expect that 3'-end-stabilization of dsRNAs, including the dsNAs of the instant invention, will improve stability.

XRN1 (NM-019001) is a 5' to 3' exonuclease that resides in P-bodies and has been implicated in degradation of mRNA targeted by miRNA (Rehwinkel et al., 2005) and may also be responsible for completing degradation initiated by internal cleavage as directed by a siRNA. XRN2 (NM-012255) is a distinct 5' to 3' exonuclease that is involved in nuclear RNA processing. Although not currently implicated in degradation or processing of siRNAs and miRNAs, these both are known nucleases that can degrade RNAs and may also be important to consider.

RNase A is a major endonuclease activity in mammals that degrades RNAs. It is specific for ssRNA and cleaves at the 3'-end of pyrimidine bases. SiRNA degradation products consistent with RNase A cleavage can be detected by mass spectrometry after incubation in serum (Turner et al., 2007). The 3'-overhangs enhance the susceptibility of siRNAs to RNase degradation. Depletion of RNase A from serum reduces degradation of siRNAs; this degradation does show some sequence preference and is worse for sequences having poly A/U sequence on the ends (Haupenthal et al., 2006). This suggests the possibility that lower stability regions of the duplex may "breathe" and offer transient single-stranded species available for degradation by RNase A. RNase A inhibitors can be added to serum and improve siRNA longevity and potency (Haupenthal et al., 2007).

In 21mers, phosphorothioate or boranophosphate modifications directly stabilize the internucleoside phosphate linkage. Boranophosphate modified RNAs are highly nuclease resistant, potent as silencing agents, and are relatively non-toxic. Boranophosphate modified RNAs cannot be manufactured using standard chemical synthesis methods and instead are made by in vitro transcription (IVT) (Hall et al., 2004 and Hall et al., 2006). Phosphorothioate (PS) modifications can be readily placed in an RNA duplex at any desired position and can be made using standard chemical synthesis methods, though the ability to use such modifications within an RNA duplex that retains RNA silencing activity can be

limited.

In certain examples, the 5' single strand extended region of the guide strand or 3' single strand extended region of the passenger strand has at least one phosphorothioate backbone modification. In some examples, every linkage of the 5' single strand extended region of the guide strand or 3' single strand extended region of the passenger strand has a phosphorothioate backbone modification. In some examples, every linkage of the 5' single strand extended region of the guide strand has a phosphorothioate backbone modification except the linkage of the terminal 5' nucleotide of the guide strand. In certain examples, the 5' single strand extended region of the guide strand or 3' single strand extended region of the passenger strand has at least one methylphosphonate backbone modification. In some examples, every linkage of the 5' single strand extended region of the guide strand or 3' single strand extended region of the passenger strand has a methylphosphonate backbone modification. In some examples, every linkage of the 3' single strand extended region of the passenger strand has a phosphorothioate backbone modification except the terminal 5' nucleotide of the guide strand.

It is noted, however, that the PS modification shows dose-dependent toxicity, so most investigators have recommended limited incorporation in siRNAs, historically favoring the 3'-ends where protection from nucleases is most important (Harborth et al., 2003; Chiu and Rana, 2003; Braasch et al., 2003; Amarzguioui et al., 2003). More extensive PS modification can be compatible with potent RNAi activity; however, use of sugar modifications (such as 2'-O-methyl RNA) may be superior (Choung et al., 2006).

A variety of substitutions can be placed at the 2'-position of the ribose which generally increases duplex stability (Tm) and can greatly improve nuclease resistance. 2'-O-methyl RNA is a naturally occurring modification found in mammalian ribosomal RNAs and transfer RNAs. 2'-O-methyl modification in siRNAs is known, but the precise position of modified bases within the duplex is important to retain potency and complete substitution of 2'-O-methyl RNA for RNA will inactivate the siRNA. For example, a pattern that employs alternating 2'-O-methyl bases can have potency equivalent to unmodified RNA and is quite stable in serum (Choung et al., 2006; Czauderna et al., 2003).

The 2'-fluoro (2'-F) modification is also compatible with dsRNA (e.g., siRNA and dsNA) function; it is most commonly placed at pyrimidine sites (due to reagent cost and availability) and can be combined with 2'-O-methyl modification at purine positions; 2'-F purines are available and can also be used. Heavily modified duplexes of this kind can be potent triggers of RNAi in vitro (Allerson et al., 2005; Prakash et al., 2005; Kraynack and Baker, 2006) and can improve performance and extend duration of action when used in vivo (Morrissey et al., 2005a; Morrissey et al., 2005b). A highly potent, nuclease stable, blunt 19mer duplex containing alternative 2'-F and 2'-O-Me bases is taught by Allerson. In this design, alternating 2'-O-Me residues are positioned in an identical pattern to that employed by Czauderna, however the remaining RNA residues are converted to 2'-F modified bases. A highly potent, nuclease resistant siRNA employed by Morrissey employed a highly potent, nuclease resistant siRNA in vivo. In addition to 2'-O-Me RNA and 2'-F RNA, this duplex includes DNA, RNA, inverted abasic residues, and a 3'-terminal PS internucleoside linkage. While extensive modification has certain benefits, more limited modification of the duplex can also improve in vivo performance and is both simpler and less costly to manufacture. Soutschek et al. (2004) employed a duplex in vivo and was mostly RNA with two 2'-O-Me RNA bases and limited 3'-terminal PS internucleoside linkages.

Locked nucleic acids (LNAs) and unlocked nucleic acids (UNA) are a different class of 2'-modification that can be used to stabilize dsRNA (e.g., siRNA and dsNAs). Patterns of LNA incorporation that retain potency are more restricted than 2'-O-methyl or 2'-F bases, so limited modification is preferred (Braasch et al., 2003; Grunweller et al., 2003; Elmen et al., 2005). Even with limited incorporation, the use of LNA modifications can improve dsRNA performance in vivo and may also alter or improve off target effect profiles (Mook et al., 2007).

Synthetic nucleic acids introduced into cells or live animals can be recognized as "foreign" and trigger an immune response. Immune stimulation constitutes a major class of off-target effects which can dramatically change experimental results and even lead to cell death. The innate immune system includes a collection of receptor molecules that specifically interact with DNA and RNA that mediate these responses, some of which are located in the cytoplasm and some of which reside in endosomes (Marques and Williams, 2005; Schlee et al., 2006). Delivery of siRNAs by cationic lipids or liposomes exposes the siRNA to both cytoplasmic and endosomal compartments, maximizing the risk for triggering a type 1 interferon (IFN) response both in vitro and in vivo (Morrissey et al., 2005b; Sioud and Sorensen, 2003; Sioud, 2005; Ma et al., 2005). RNAs transcribed within the cell are less immunogenic (Robbins et al., 2006) and synthetic RNAs that are immunogenic when delivered using lipid-based methods can evade immune stimulation when introduced unto cells by mechanical means, even in vivo (Heidel et al., 2004). However, lipid based delivery methods are convenient, effective, and widely used. Some general strategy to prevent immune responses is needed, especially for in vivo application where all cell types are present and the risk of generating an immune response is highest. Use of chemically modified RNAs may solve most or even all of these problems.

Although certain sequence motifs are clearly more immunogenic than others, it appears that the receptors of the innate immune system in general distinguish the presence or absence of certain base modifications which are more commonly found in mammalian RNAs than in prokaryotic RNAs. For example, pseudouridine, N6-methyl-A, and 2'-O-methyl modified bases are recognized as "self' and inclusion of these residues in a synthetic RNA can help evade

immune detection (Kariko et al., 2005). Extensive 2'-modification of a sequence that is strongly immunostimulatory as unmodified RNA can block an immune response when administered to mice intravenously (Morrissey et al., 2005b). However, extensive modification is not needed to escape immune detection and substitution of as few as two 2'-O-methyl bases in a single strand of a siRNA duplex can be sufficient to block a type 1 IFN response both in vitro and in vivo; modified U and G bases are most effective (Judge et al., 2006). As an added benefit, selective incorporation of 2'-O-methyl bases can reduce the magnitude of off-target effects (Jackson et al., 2006). Use of 2'-O-methyl bases should therefore be considered for all dsRNAs intended for in vivo applications as a means of blocking immune responses and has the added benefit of improving nuclease stability and reducing the likelihood of off-target effects.

Although cell death can result from immune stimulation, assessing cell viability is not an adequate method to monitor induction of IFN responses. IFN responses can be present without cell death, and cell death can result from target knockdown in the absence of IFN triggering (for example, if the targeted gene is essential for cell viability). Relevant cytokines can be directly measured in culture medium and a variety of commercial kits exist which make performing such assays routine. While a large number of different immune effector molecules can be measured, testing levels of IFN-$\alpha$, TNF-$\alpha$, and IL-6 at 4 and 24 hours post transfection is usually sufficient for screening purposes. It is important to include a "transfection reagent only control" as cationic lipids can trigger immune responses in certain cells in the absence of any nucleic acid cargo. Including controls for IFN pathway induction should be considered for cell culture work. It is essential to test for immune stimulation whenever administering nucleic acids in vivo, where the risk of triggering IFN responses is highest.

dsNAs of the invention can have any of the modification patterns described herein below for DsiRNAs. Modifications can be included in the dsNA agents of the present invention so long as the modification does not prevent the dsNA agent from serving as a substrate for Dicer. Indeed, one surprising finding of the instant invention is that a 5' extended single stranded nucleotide region of the antisense strand or 3' extended single stranded nucleotide region of the sense strand can be attached to previously described dsNA molecules, resulting in enhanced RNAi efficacy and duration, provided that such extension is performed in a region of the extended molecule that does not interfere with Dicer processing (e.g., 3' of the Dicer cleavage site of the sense strand/5' of the Dicer cleavage site of the antisense strand). In one example, one or more modifications are made that enhance Dicer processing of the dsiNA agent. In a second example, one or more modifications are made that result in more effective RNAi generation. In a third example, one or more modifications are made that support a greater RNAi effect. In a fourth example, one or more modifications are made that result in greater potency per each dsNA agent molecule to be delivered to the cell. Modifications can be incorporated in the 3'-terminal region, the 5'-terminal region, in both the 3'-terminal and 5'-terminal region or in some instances in various positions within the sequence. With the restrictions noted above in mind, any number and combination of modifications can be incorporated into the dsNA agent. Where multiple modifications are present, they may be the same or different. Modifications to bases, sugar moieties, the phosphate backbone, and their combinations are contemplated. Either 5'-terminus can be phosphorylated.

Examples of modifications contemplated for the phosphate backbone include phosphonates, including methylphosphonate, phosphorothioate, and phosphotriester modifications such as alkylphosphotriesters, locked nucleic acids (LNA), unlocked nucleic acid, morpholino, bicyclic furanose analogs and the like. Examples of modifications contemplated for the sugar moiety include 2'-alkyl pyrimidine, such as 2'-O-methyl, 2'-fluoro, amino, and deoxy modifications and the like (see, e.g., Amarzguioui et al., 2003). Examples of modifications contemplated for the base groups include abasic sugars, 2-O-alkyl modified pyrimidines, 4-thiouracil, 5-bromouracil, 5-iodouracil, and 5-(3-aminoallyl)-uracil and the like. Locked nucleic acids, or LNA's, Unlocked nucleic acids, or UNA's could also be incorporated. Many other modifications are known and can be used so long as the above criteria are satisfied. Examples of modifications are also disclosed in U.S. Pat. Nos. 5,684,143, 5,858,988 and 6,291,438 and in published U.S. published patent application No. 2004/0203145 A1. Other modifications are disclosed in Herdewijn (2000), Eckstein (2000), Rusckowski et al. (2000), Stein et al. (2001); Vorobjev et al. (2001).

One or more modifications contemplated can be incorporated into either strand. The placement of the modifications in the dsNA agent can greatly affect the characteristics of the dsNA agent, including conferring greater potency and stability, reducing toxicity, enhance Dicer processing, and minimizing an immune response. In one example, the antisense strand or the sense strand or both strands have one or more 2'-O-methyl modified nucleotides. In another example, the antisense strand contains 2'-O-methyl modified nucleotides. In another example, the antisense stand contains a 3' overhang that comprises 2'-O-methyl modified nucleotides. The antisense strand could also include additional 2'-O-methyl modified nucleotides.

In certain examples, the 5' single strand extended region of the guide strand, 3' single strand extended region of the passenger strand, or 5' single strand extended region of the passenger strand has at least one modified nucleotide, optionally a 2'-O-methyl ribonucleotide. In some examples, every nucleotide of the 5' single strand extended region of the guide strand or 3' single strand extended region of the passenger strand is a modified ribonucleotide, optionally a 2'-O-methyl ribonucleotide. In certain examples, an oligonucleotide complementary to the 5' single strand extended region of the guide strand has at least one modified nucleotide, optionally a 2'-O-methyl ribonucleotide. In some examples,

every nucleotide of an oligonucleotide complementary to the 5' single strand extended region of the guide strand is a modified nucleotide, optionally a 2'-O-methyl ribonucleotide.

In certain examples, the dsiNA agent has one or more properties which enhance its processing by Dicer. According to these examples, the dsiNA agent has a length sufficient such that it is processed by Dicer to produce an active siRNA and at least one of the following properties: (i) the dsiNA agent is asymmetric, e.g., has a 3' overhang on the antisense strand and (ii) the dsiNA agent has a modified 3' end on the sense strand to direct orientation of Dicer binding and processing of the dsRNA region to an active siRNA. In certain such examples, the presence of one or more base paired deoxyribonucleotides in a region of the sense strand that is 3' to the projected site of Dicer enzyme cleavage and corresponding region of the antisense strand that is 5' of the projected site of Dicer enzyme cleavage can also serve to orient such a molecule for appropriate directionality of Dicer enzyme cleavage.

In certain examples, the length of the single stranded antisense extended region is 1-30 nucleotides, 1-15 nucleotides, 10-15 nucleotides, 11-15 nucleotides. Thus, a single stranded extended dsNA of the instant invention may possess a single strand extended region at the 5' terminus of an antisense/guide strand or at the 3' terminus of a sense/passenger strand that is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more (e.g., 31, 32, 33, 34, 35, 36, 37, 38, 39, 40 or more) nucleotides in length.

In some examples, the longest strand in the double stranded nucleic acid comprises 36-66 nucleotides. In one example, the dsNA agent has a structure such that the 5' end of the antisense strand overhangs the 3' end of the sense strand, or the 3' end of the antisense strand overhangs the 5' end of the sense strand. In certain examples, the 5' extension of the antisense strand is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more (e.g., 31, 32, 33, 34, 35, 36, 37, 38, 39, 40 or more) nucleotides in length, and optionally is 10-30 nucleotides, for example 15 nucleotides. In another example, the dsNA agent has a structure such that the 3' end of the sense strand overhangs the 5' end of the antisense strand, and the 3' end of the antisense strand overhangs the 5' end of the sense strand. In certain examples, the 3' extension of the sense strand is 1-30 nucleotides, and optionally is 10-30 nucleotides, for example 15 nucleotides. In certain examples, the 3' extension of the antisense strand is 1-10 nucleotides, and optionally is 1-6 nucleotides, preferably 1-4 nucleotides, for example 2 nucleotides. In another example, the DsNA agent has a structure such that the 5' end of the sense strand overhangs the 3' end of the antisense strand. In certain examples, the 5' overhang of the sense strand is 4-30 nucleotides, and optionally is 10-30 nucleotides, for example 15 nucleotides. Both the sense and the antisense strand may also have a 5' phosphate An iterative combination the extension features at different 5' and 3' terminus of sense and anti sense strands as shown in figures 34, 35 and 36 is also contemplated in the current invention.

In certain embodiments, the sense strand of a dsNA of the invention has a total length of at least 25 nucleotides (e.g., the sense strand possesses a length of 25, 26, 27, 28, 29, 30 or more (e.g., 31, 32, 33, 34, 35, 36, 37, 38, 39, 40 or more) nucleotides). In certain embodiments, the length of the sense strand is between 25 nucleotides and 30 nucleotides, for example between 26 and 30 nucleotides, or, between 27 and 30 nucleotides in length. In related examples, the antisense strand has a length of at least 36 nucleotides (e.g., the sense strand possesses a length of 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66 or more (e.g., 67, 28, 69, 70, 71, 72, 73, 74, 75, 76, 77, 78, 79, 80 or more) nucleotides). In certain such examples, the antisense strand has a length of between 37 and 57 nucleotides in length, or between 37 and 52 nucleotides in length, or between 37 and 47 nucleotides in length, or between 42 and 62 nucleotides in length, or between 42 and 57 nucleotides in length, or between 42 and 47 nucleotides in length.

In certain embodiments, the sense strand of a DsNA of the invention has a total length of 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or more (e.g., 61, 62, 63, 64, 65, 66, 67, 68, 69, 70 or more) nucleotides). In certain embodiments, the length of the sense strand is between 25 nucleotides and 30 nucleotides, optionally between 35 and 55 nucleotides, between 40 and 55 nucleotides in length, between 40 and 60 nucleotides in length, or, between 45 and 60 nucleotides in length. In related embodiments, the antisense strand has a length of 25, 27, 28, 29, 30, 31, 32, 33, 34, 35, 36, or more (e.g., 37, 38, 39, 40, 41, 42, 43, 44, 45, 46, 47, 48, 49, 50 nucleotides). In certain such embodiments, the antisense strand has a length of between 27 and 32 nucleotides in length.

In certain embodiments, the presence of one or more base paired deoxyribonucleotides in a region of the sense strand that is 3' of the projected site of Dicer enzyme cleavage and corresponding region of the antisense strand that is 5' of the projected site of Dicer enzyme cleavage can serve to direct Dicer enzyme cleavage of such a molecule. While certain exemplified agents of the invention possess a sense strand deoxyribonucleotide that is located at position 24 or more 3' when counting from position 1 at the 5' end of the sense strand, and having this position 24 or more 3' deoxyribonucleotide of the sense strand base pairing with a cognate deoxyribonucleotide of the antisense strand, in some embodiments, it is also possible to direct Dicer to cleave a shorter product, e.g., a 19mer or a 20mer via inclusion of deoxyribonucleotide residues at, e.g., position 20 of the sense strand. Such a position 20 deoxyribonucleotide base pairs with a corresponding deoxyribonucleotide of the antisense strand, thereby directing Dicer-mediated excision of a 19mer as the most prevalent Dicer product (it is noted that the antisense strand can also comprise one or two deoxyribonucleotide residues immediately 3’ of the antisense residue that base pairs with the position 20 deoxyribonucleotide residue of the sense strand in such embodiments, to further direct Dicer cleavage of the antisense strand). In such embodiments, the double-stranded DNA region (which is inclusive of modified nucleic acids that block Dicer cleavage) will generally possess a length of greater than 1 or 2 base pairs (e.g., 3 to 5 base pairs or more), in order to direct Dicer cleavage to generate what is normally a non-preferred length of Dicer cleavage product. A parallel approach can also be taken to direct Dicer excision of 20mer siRNAs, with the positioning of the first deoxyribonucleotide residue of the sense strand (when surveying the sense strand from position 1 at the 5’ terminus of the sense strand) occurring at position 21.

In certain embodiments the DsNA agent of the invention can generally comprise modified nucleotides from about 5 to about 100% of the nucleotide positions (e.g., 5%, 10%, 15%, 20%, 25%, 30%, 35%, 40%, 45%, 50%, 55%, 60%, 65%, 70%, 75%, 80%, 85%, 90%, 95% or 100% of the nucleotide positions). The actual percentage of modified nucleotides present in a given siNA molecule depends on the total number of nucleotides present in the siNA. If the siNA molecule is single stranded, the percent modification can be based upon the total number of nucleotides present in the single stranded siNA molecules. Likewise, if the siNA molecule is double stranded; the percent modification can be based upon the total number of nucleotides present in the sense strand, antisense strand, or both the sense and antisense strands. In addition, the actual percentage of modified nucleotides present in a given siNA molecule can also depend on the total number of purine and pyrimidine nucleotides present in the siNA, for example, wherein all pyrimidine nucleotides and/or all purine nucleotides present in the siNA molecule are modified. In some cases even 100% of the sense strand and or antisense strand can be completely modified.

In certain examples, the sense strand of the DsiNA agent is modified for Dicer processing by suitable modifiers located at the 3’ end of the sense strand, i.e., the DsiNA agent is designed to direct orientation of Dicer binding and processing via sense strand modification. Suitable modifiers include nucleotides such as deoxyribonucleotides, dideoxyribonucleotides, acyclonucleotides and the like and sterically hindered molecules, such as fluorescent molecules and the like. Acyclonucleotides substitute a 2-hydroxyethoxymethyl group for the 2’-deoxyribofuranosyl sugar normally present in dNMPs. Other nucleotide modifiers could include 3’-deoxyadenosine (cordycepin), 3’-azido-3’-deoxythymidine (AZT), 2’,3’-dideoxyinosine (ddl), 2’,3’-dideoxy-3’-thiacytidine (3TC), 2’,3’-didehydro-2’,3’-dideoxythymidine (d4T) and the monophosphate nucleotides of 3’-azido-3’-deoxythymidine (AZT), 2’,3’-dideoxy-3’-thiacytidine (3TC) and 2’,3’-didehydro-2’,3’-dideoxythymidine (d4T). In one example, deoxyribonucleotides are used as the modifiers. When nucleotide modifiers are utilized, 1-3 nucleotide modifiers, or 2 nucleotide modifiers are substituted for the ribonucleotides on the 3’ end of the sense strand. When sterically hindered molecules are utilized, they are attached to the ribonucleotide at the 3’ end of the antisense strand. Thus, the length of the strand does not change with the incorporation of the modifiers. In another example, the invention contemplates substituting two DNA bases in the DsiNA agent to direct the orientation of Dicer processing of the antisense strand. In a further example of the present invention, two terminal DNA bases are substituted for two ribonucleotides on the 3’-end of the sense strand forming a blunt end of the duplex on the 3’ end of the sense strand and the 5’ end of the antisense strand, and a two-nucleotide RNA overhang is located on the 3’-end of the antisense strand. This is an asymmetric composition with DNA on the blunt end and RNA bases on the overhanging end. In certain examples of the instant invention, the modified nucleotides (e.g., deoxyribonucleotides) of the penultimate and ultimate positions of the 3’ terminus of the sense strand base pair with corresponding modified nucleotides (e.g., deoxyribonucleotides) of the antisense strand (optionally, the penultimate and ultimate residues of the 5’ end of the antisense strand in those DsiNA agents of the instant invention possessing a blunt end at the 3’ terminus of the sense strand/5’ terminus of the antisense strand).

The sense and antisense strands of a DsiNA agent of the instant invention anneal under biological conditions, such as the conditions found in the cytoplasm of a cell. In addition, a region of one of the sequences, particularly of the antisense strand, of the DsiNA agent has a sequence length of at least 19 nucleotides, wherein these nucleotides are in the 21-nucleotide region adjacent to the 3’ end of the antisense strand and are sufficiently complementary to a nucleotide sequence of the RNA produced from the target gene to anneal with and/or decrease levels of such a target RNA.

The DsiNA agent of the instant invention may possess one or more deoxyribonucleotide base pairs located at any positions of sense and antisense strands that are located 3’ of the projected Dicer cleavage site of the sense strand and 5’ of the projected Dicer cleavage site of the antisense strand. In certain examples, one, two, three or all four of positions 24-27 of the sense strand (starting from position 1 at the 5’ terminus of the sense strand) are deoxyribonucleotides, each deoxyribonucleotide of which base pairs with a corresponding deoxyribonucleotide of the antisense strand. In certain examples, the deoxyribonucleotides of the 5’ region of the antisense strand (e.g., the region of the antisense strand located 5’ of the projected Dicer cleavage site for a given DsiNA molecule) are not complementary to the target RNA to which the DsiNA agent is directed. In related examples, the entire region of the antisense strand located 5’ of the projected Dicer cleavage site of a DsiNA agent is not complementary to the target RNA to which the DsiNA agent is directed. In certain examples, the deoxyribonucleotides of the antisense strand or the entire region of the antisense strand that is located 5’ of the projected Dicer cleavage site of the DsiNA agent is not sufficiently complementary to the target RNA to enhance annealing of the antisense strand of the DsiNA to the target RNA when the antisense strand is

annealed to the target RNA under conditions sufficient to allow for annealing between the antisense strand and the target RNA (e.g., a "core" antisense strand sequence lacking the DNA-extended region anneals equally well to the target RNA as the same "core" antisense strand sequence also extended with sequence of the DNA-extended region).

The DsNA agent may also have one or more of the following additional properties: (a) the antisense strand has a right or left shift from the typical 21mer, (b) the strands may not be completely complementary, i.e., the strands may contain simple mismatch pairings and (c) base modifications such as locked nucleic acid(s) and unlocked nucleic acids (UNA) may be included in the 5' end of the sense strand. A "typical" 21mer siRNA is designed using conventional techniques. In one technique, a variety of sites are commonly tested in parallel or pools containing several distinct siRNA duplexes specific to the same target with the hope that one of the reagents will be effective (Ji et al., 2003). Other techniques use design rules and algorithms to increase the likelihood of obtaining active RNAi effector molecules (Schwarz et al., 2003; Khvorova et al., 2003; Ui-Tei et al., 2004; Reynolds et al., 2004; Krol et al., 2004; Yuan et al., 2004; Boese et al., 2005). High throughput selection of siRNA has also been developed (U.S. published patent application No. 2005/0042641 A1). Potential target sites can also be analyzed by secondary structure predictions (Heale et al., 2005). This 21mer is then used to design a right shift to include 3-9 additional nucleotides on the 5' end of the 21mer. The sequence of these additional nucleotides may have any sequence. In one example, the added ribonucleotides are based on the sequence of the target gene. Even in this example, full complementarity between the target sequence and the antisense siRNA is not required.

The first and second oligonucleotides of a DsNA agent of the instant invention are not required to be completely complementary. They only need to be substantially complementary to anneal under biological conditions and to provide a substrate for Dicer that produces a siRNA sufficiently complementary to the target sequence. Locked nucleic acids, or LNA's, unlocked nucleic acids (UNA), are well known to a skilled artisan (Elman et al., 2005; Kurreck et al., 2002; Crinelli et al., 2002; Braasch and Corey, 2001; Bondensgaard et al., 2000; Wahlestedt et al., 2000). In one example, an LNA is incorporated at the 5' terminus of the sense strand. In another example, an LNA is incorporated at the 5' terminus of the sense strand in duplexes designed to include a 3' overhang on the antisense strand.

In certain examples, the DsNA agent of the instant invention has an asymmetric structure, with the sense strand having a 27-base pair length, and the antisense strand having a 29-base pair length and a 2 base 3'-overhang. Such agents may possess between one and four deoxyribonucleotides at the 3' terminal region (specifically, the region 3' of the projected Dicer cleavage site) of the sense strand, at least one of which deoxyribonucleotide base pairs with a cognate deoxyribonucleotide of the 5' terminal region (specifically, the region 5' of the projected Dicer cleavage site) of the antisense strand. In other examples, the sense strand has a 28-base pair length, and the antisense strand has a 30-base pair length and a 2 base 3'-overhang. Such agents may possess between one and five deoxyribonucleotides at the 3' terminal region (specifically, the region 3' of the projected Dicer cleavage site) of the sense strand, at least one of which base deoxyribonucleotide pairs with a cognate deoxyribonucleotide of the 5' terminal region (specifically, the region 5' of the projected Dicer cleavage site) of the antisense strand. In additional examples, the sense strand has a 29-base pair length, and the antisense strand has a 31-base pair length and a 2 base 3'-overhang. Such agents may possess between one and six deoxyribonucleotides at the 3' terminal region (specifically, the region 3' of the projected Dicer cleavage site) of the sense strand, at least one of which deoxyribonucleotide base pairs with a cognate deoxyribonucleotide of the 5' terminal region (specifically, the region 5' of the projected Dicer cleavage site) of the antisense strand. In further examples, the sense strand has a 30-base pair length, and the antisense strand has a 32-base pair length and a 2 base 3'-overhang. Such agents optionally possess between one and seven deoxyribonucleotides at the 3' terminal region (specifically, the region 3' of the projected Dicer cleavage site) of the sense strand, at least one of which deoxyribonucleotide base pairs with a cognate deoxyribonucleotide of the 5' terminal region (specifically, the region 5' of the projected Dicer cleavage site) of the antisense strand. In other examples, the sense strand has a 31-base pair length, and the antisense strand has a 33-base pair length and a 2 base 3'-overhang. Such agents may possess between one and eight deoxyribonucleotides at the 3' terminal region (specifically, the region 3' of the projected Dicer cleavage site) of the sense strand, at least one of which deoxyribonucleotide base pairs with a cognate deoxyribonucleotide of the 5' terminal region (specifically, the region 5' of the projected Dicer cleavage site) of the antisense strand. In additional examples, the sense strand has a 32-base pair length, and the antisense strand has a 34-base pair length and a 2 base 3'-overhang. Such agents optionally possess between one and nine deoxyribonucleotides at the 3' terminal region (specifically, the region 3' of the projected Dicer cleavage site) of the sense strand, at least one of which deoxyribonucleotide base pairs with a cognate deoxyribonucleotide of the 5' terminal region (specifically, the region 5' of the projected Dicer cleavage site) of the antisense strand. In certain further examples, the sense strand has a 33-base pair length, and the antisense strand has a 35-base pair length with a 2 base 3'-overhang. Such agents optionally possess between one and ten deoxyribonucleotides of the 3' terminal region (specifically, the region 3' of the projected Dicer cleavage site) of the sense strand, at least one of which base pairs with a cognate deoxyribonucleotide of the 5' terminal region (specifically, the region 5' of the projected Dicer cleavage site) of the antisense strand. In still other examples, any of these DsNA agents have an asymmetric structure that further contains 2 deoxyribonucleotides at the 3' end of the sense strand that may, base pair with cognate deoxyribonucleotides of the antisense strand.

Certain DsNA agent compositions containing two separate oligonucleotides can be linked by a third structure. The third structure will not block Dicer activity on the DsNA agent and will not interfere with the directed destruction of the RNA transcribed from the target gene. In one example, the third structure may be a chemical linking group. Many suitable chemical linking groups are known in the art and can be used. Alternatively, the third structure may be an oligonucleotide that links the two oligonucleotides of the DsNA agent in a manner such that a hairpin structure is produced upon annealing of the two oligonucleotides making up the dsNA composition. The hairpin structure will not block Dicer activity on the DsNA agent and will not interfere with the directed destruction of the target RNA.

In certain examples, the DsNA agent has several properties which enhance its processing by Dicer. According to such examples, the DsNA agent has a length sufficient such that it is processed by Dicer to produce an siRNA and at least one of the following properties: (i) the DsNA agent is asymmetric, e.g., has a 3' overhang on the sense strand and (ii) the DsNA agent has a modified 3' end on the antisense strand to direct orientation of Dicer binding and processing of the dsRNA region to an active siRNA. According to these examples, the longest strand in the DsNA agent comprises 25-43 nucleotides. In one example, the sense strand comprises 25-39 nucleotides and the antisense strand comprises 26-43 nucleotides. The resulting dsNA can have an overhang on the 3' end of the sense strand. The overhang is 1-4 nucleotides, such as 2 nucleotides. The antisense or sense strand may also have a 5' phosphate.

In certain examples, the DsNA agent has several properties which enhance its processing by Dicer. According to such examples, the DsNA agent has a length sufficient such that it is processed by Dicer to produce an siRNA and at least one of the following properties: (i) the DsNA agent is asymmetric, e.g., has a 5' overhang on the sense strand and (ii) the DsNA agent has a modified 3' end on the antisense strand to direct orientation of Dicer binding and processing of the dsRNA region to an active siRNA. According to these examples, the longest strand in the DsNA agent comprises 25-43 nucleotides. In one example, the sense strand comprises 25-43 nucleotides and the antisense strand comprises 25-39 nucleotides. The resulting dsNA can have an overhang on the 3' end of the sense strand. The overhang is 1-4 nucleotides, such as 2 nucleotides. The antisense or sense strand may also have a 5' phosphate

In certain examples, the sense strand of a DsiNA agent is modified for Dicer processing by suitable modifiers located at the 3' end of the sense strand, i.e., the DsiNA agent is designed to direct orientation of Dicer binding and processing. Suitable modifiers include nucleotides such as deoxyribonucleotides, dideoxyribonucleotides, acyclonucleotides and the like and sterically hindered molecules, such as fluorescent molecules and the like. Acyclonucleotides substitute a 2-hydroxyethoxymethyl group for the 2'-deoxyribofuranosyl sugar normally present in dNMPs. Other nucleotide modifiers could include 3'-deoxyadenosine (cordycepin), 3'-azido-3'-deoxythymidine (AZT), 2',3'-dideoxyinosine (ddI), 2',3'-dideoxy-3'-thiacytidine (3TC), 2',3'-didehydro-2',3'-dideoxythymidine (d4T) and the monophosphate nucleotides of 3'-azido-3'-deoxythymidine (AZT), 2',3'-dideoxy-3'-thiacytidine (3TC) and 2',3'-didehydro-2',3'-dideoxythymidine (d4T). In one example, deoxyribonucleotides are used as the modifiers. When nucleotide modifiers are utilized, 1-3 nucleotide modifiers, or 2 nucleotide modifiers are substituted for the ribonucleotides on the 3' end of the sense strand. When sterically hindered molecules are utilized, they are attached to the ribonucleotide at the 3' end of the antisense strand. Thus, the length of the strand does not change with the incorporation of the modifiers. In another example, the invention contemplates substituting two DNA bases in the dsNA to direct the orientation of Dicer processing. In a further example, two terminal DNA bases are located on the 3' end of the sense strand in place of two ribonucleotides forming a blunt end of the duplex on the 5' end of the antisense strand and the 3' end of the sense strand, and a two-nucleotide RNA overhang is located on the 3'-end of the antisense strand. This is an asymmetric composition with DNA on the blunt end and RNA bases on the overhanging end.

In certain other examples, the antisense strand of a DsiNA agent is modified for Dicer processing by suitable modifiers located at the 3' end of the antisense strand, i.e., the DsiNA agent is designed to direct orientation of Dicer binding and processing. Suitable modifiers include nucleotides such as deoxyribonucleotides, dideoxyribonucleotides, acyclonucleotides and the like and sterically hindered molecules, such as fluorescent molecules and the like. Acyclonucleotides substitute a 2-hydroxyethoxymethyl group for the 2'-deoxyribofuranosyl sugar normally present in dNMPs. Other nucleotide modifiers could include 3'-deoxyadenosine (cordycepin), 3'-azido-3'-deoxythymidine (AZT), 2',3'-dideoxyinosine (ddI), 2',3'-dideoxy-3'-thiacytidine (3TC), 2',3'-didehydro-2',3'-dideoxythymidine (d4T) and the monophosphate nucleotides of 3'-azido-3'-deoxythymidine (AZT), 2',3'-dideoxy-3'-thiacytidine (3TC) and 2',3'-didehydro-2',3'-dideoxythymidine (d4T). In one example, deoxyribonucleotides are used as the modifiers. When nucleotide modifiers are utilized, 1-3 nucleotide modifiers, or 2 nucleotide modifiers are substituted for the ribonucleotides on the 3' end of the antisense strand. When sterically hindered molecules are utilized, they are attached to the ribonucleotide at the 3' end of the antisense strand. Thus, the length of the strand does not change with the incorporation of the modifiers. In another example, the invention contemplates substituting two DNA bases in the dsNA to direct the orientation of Dicer processing. In a further invention, two terminal DNA bases are located on the 3' end of the antisense strand in place of two ribonucleotides forming a blunt end of the duplex on the 5' end of the sense strand and the 3' end of the antisense strand, and a two-nucleotide RNA overhang is located on the 3'-end of the sense strand. This is also an asymmetric composition with DNA on the blunt end and RNA bases on the overhanging end.

The sense and antisense strands anneal under biological conditions, such as the conditions found in the

cytoplasm of a cell. In addition, a region of one of the sequences, particularly of the antisense strand, of the dsNA has a sequence length of at least 19 nucleotides, wherein these nucleotides are adjacent to the 3' end of antisense strand and are sufficiently complementary to a nucleotide sequence of the target RNA to direct RNA interference.

Additionally, the DsiNA agent structure can be optimized to ensure that the oligonucleotide generated by Dicer cleavage is the most effective in inhibiting gene expression. For example, in one embodiment of the invention, a 27-35-bp oligonucleotide of the DsiNA agent structure is synthesized wherein the anticipated 21 to 22-bp segment that will inhibit gene expression is located on the 3'-end of the antisense strand. The remaining bases located on the 5'-end of the antisense strand will be cleaved by Dicer and will be discarded. This cleaved portion can be homologous (i.e., based on the sequence of the target sequence) or non-homologous and added to extend the nucleic acid strand. As surprisingly identified in the instant invention, such extension can be performed with base paired DNA residues (double stranded DNA:DNA extensions), resulting in extended DsiNA agents having improved efficacy or duration of effect than corresponding double stranded RNA:RNA-extended DsiNA agents.

US 2007/0265220 disclose that 27mer DsNAs show improved stability in serum over comparable 21mer siRNA compositions, even absent chemical modification. Modifications of DsNA agents, such as inclusion of 2'-O-methyl RNA in the antisense strand, in patterns such as detailed in US 2007/0265220 and in the instant Examples, when coupled with addition of a 5' Phosphate, can improve stability of DsNA agents. Addition of 5'-phosphate to all strands in synthetic RNA duplexes may be an inexpensive and physiological method to confer some limited degree of nuclease stability.

The chemical modification patterns of the DsNA agents of the instant invention are designed to enhance the efficacy of such agents. Accordingly, such modifications are designed to avoid reducing potency of DsNA agents; to avoid interfering with Dicer processing of DsNA agents; to improve stability in biological fluids (reduce nuclease sensitivity) of DsNA agents; or to block or evade detection by the innate immune system. Such modifications are also designed to avoid being toxic and to avoid increasing the cost or impact the ease of manufacturing the instant DsNA agents of the invention.

## RNA Processing siRNA

The process of siRNA-mediated RNAi is triggered by the presence of long, dsRNA molecules in a cell. During the initiation step of RNAi, these dsRNA molecules are cleaved into 21-23 nucleotide (nt) small-interfering RNA duplexes (siRNAs) by Dicer, a conserved family of enzymes containing two RNase III-like domains (Bernstein et al. 2001; Elbashir et al. 2001). The siRNAs are characterized by a 19-21 base pair duplex region and 2 nucleotide 3' overhangs on each strand. During the effector step of RNAi, the siRNAs become incorporated into a multimeric protein complex called RNA-induced silencing complex (RISC), where they serve as guides to select fully complementary mRNA substrates for degradation. Degradation is initiated by endonucleolytic cleavage of the mRNA within the region complementary to the siRNA. More precisely, the mRNA is cleaved at a position 10 nucleotides from the 5' end of the guiding siRNA (Elbashir et al. 2001 Genes and Dev. 15: 188-200; Nykanen et al. 2001 Cell 107: 309-321; Martinez et al. 2002 Cell 110: 563-574). An endonuclease responsible for this cleavage was identified as Argonaute2 (Ago2; Liu et al. Science, 305: 1437-41).

## miRNA

The majority of human miRNAs (70%)-and presumably the majority of miRNAs of other mammals-are transcribed from introns and/or exons, and approximately 30% are located in intergenic regions (Rodriguez et al., Genome Res. 2004, 14(10A), 1902-1910). In human and animal, miRNAs are usually transcribed by RNA polymerase II (Farh et al. Science 2005, 310(5755), 1817-1821), and in some cases by pol III (Borchert et al. Nat. Struct. Mol. Biol. 2006, 13(12), 1097-1101). Certain viral encoded miRNAs are transcribed by RNA polymerase III (Pfeffer et al. Nat. Methods 2005, 2(4), 269-276; Andersson et al. J. Virol. 2005, 79(15), 9556-9565), and some are located in the open reading frame of viral gene (Pfeffer et al. Nat. Methods 2005, 2(4), 269-276; Samols et al. J. Virol. 2005, 79(14), 9301-9305). miRNA transcription results in the production of large monocistronic, bicistronic or polycistronic primary transcripts (pri-miRNAs). A single pri-miRNA may range from approximately 200 nucleotides (nt) to several kilobases (kb) in length and have both a 5' 7-methylguanosine (m7) caps and a 3' poly (A) tail. Characteristically, the mature miRNA sequences are localized to regions of imperfect stem-loop sequences within the pri-miRNAs (Cullen, Mol. Cell. 2004, 16(6), 861-865).

The first step of miRNA maturation in the nucleus is the recognition and cleavage of the pri-miRNAs by the RNase III Drosha-DGCR8 nuclear microprocessor complex, which releases a ~70 nt hairpin-containing precursor molecule called pre-miRNAs, with a monophosphate at the 5' terminus and a 2-nt overhang with a hydroxyl group at the 3' terminus (Cai et al. RNA 2004, 10(12), 1957-1966; Lee et al. Nature 2003, 425(6956), 415-419; Kim Nat. Rev. Mol. Cell. Biol. 2005, 6(5), 376-385). The next step is the nuclear transport of the pre-miRNAs out of the nucleus into the cytoplasm by Exportin-5, a carrier protein (Yi et al. Genes. Dev. 2003, 17(24), 3011-3016, Bohnsack et al. RNA 2004, 10(2), 185-191). Exportin-5 and the GTP-bound form of its cofactor Ran together recognize and bind the 2 nucleotide 3' overhang and the adjacent stem that are characteristics of pre-miRNA (Basyuk et al. Nucl. Acids Res. 2003, 31(22),

6593-6597, Zamore Mol. Cell. 2001, 8(6), 1158-1160). In the cytoplasm, GTP hydrolysis results in release of the pre-miRNA, which is then processed by a cellular endonuclease III enzyme Dicer (Bohnsack et al.). Dicer was first recognized for its role in generating siRNAs that mediate RNA interference (RNAi). Dicer acts in concert with its cofactors TRBP (Trans activating region binding protein; Chendrimata et al. Nature 2005, 436(7051), 740-744) and PACT (interferon-inducible double strand-RNA-dependent protein kinase activator; Lee et al. EMBO J. 2006, 25(3), 522-532). These enzymes bind at the 3' 2 nucleotide overhang at the base of the pre-miRNA hairpin and remove the terminal loop, yielding an approximately 21-nt miRNA duplex intermediate with both termini having 5' monophosphates, 3' 2 nucleotide overhangs and 3' hydroxyl groups. The miRNA guide strand, the 5' terminus of which is energetically less stable, is then selected for incorporation into the RISC(RNA-induced silencing complex), while the 'passenger' strand is released and degraded (Maniataki et al. Genes. Dev. 2005, 19(24), 2979-2990; Hammond et al. Nature 2000, 404(6775), 293-296). The composition of RISC remains incompletely defined, but a key component is a member of the Argonaute (Ago) protein family (Maniataki et al.; Meister et al. Mol. Cell. 2004, 15(2), 185-197).

The mature miRNA then directs RISC to complementary mRNA species. If the target mRNA has perfect complementarity to the miRNA-armed RISC, the mRNA will be cleaved and degraded (Zeng et al. Proc. Natl. Acad. Sci. USA 2003, 100(17), 9779-9784; Hutvagner et al. Science 2002, 297(55 89), 2056-2060). But as the most common situation in mammalian cells, the miRNAs targets mRNAs with imperfect complementarity and suppress their translation, resulting in reduced expression of the corresponding proteins (Yekta et al. Science 2004, 304(5670), 594-596; Olsen et al. Dev. Biol. 1999, 216(2), 671-680). The 5' region of the miRNA, especially the match between miRNA and target sequence at nucleotides 2-7 or 8 of miRNA (starting from position 1 at the 5' terminus), which is called the seed region, is essentially important for miRNA targeting, and this seed match has also become a key principle widely used in computer prediction of the miRNA targeting (Lewis et al. Cell 2005, 120(1), 15-20; Brennecke et al. PLoS Biol. 2005, 3(3), e85). miRNA regulation of the miRNA-mRNA duplexes is mediated mainly through multiple complementary sites in the 3' UTRs, but there are many exceptions. miRNAs may also bind the 5' UTR and/or the coding region of mRNAs, resulting in a similar outcome (Lytle et al. Proc. Natl. Acad. Sci. USA 2007, 104(23), 9667-9672).

## RNase H

RNase H is a ribonuclease that cleaves the 3'-O-P bond of RNA in a DNA/RNA duplex to produce 3'-hydroxyl and 5'-phosphate terminated products. RNase H is a non-specific endonuclease and catalyzes cleavage of RNA via a hydrolytic mechanism, aided by an enzyme-bound divalent metal ion. Members of the RNase H family are found in nearly all organisms, from archaea and prokaryotes to eukaryotes. During DNA replication, RNase H is believed to cut the RNA primers responsible for priming generation of Okazaki fragments; however, the RNase H enzyme may be more generally employed to cleave any DNA:RNA hybrid sequence of sufficient length (e.g., typically DNA:RNA hybrid sequences of 4 or more base pairs in length in mammals).

## MicroRNA and MicroRNA-Like Therapeutics

MicroRNAs (miRNAs) have been described to act by binding to the 3' UTR of a template transcript, thereby inhibiting expression of a protein encoded by the template transcript by a mechanism related to but distinct from classic RNA interference. Specifically, miRNAs are believed to act by reducing translation of the target transcript, rather than by decreasing its stability. Naturally-occurring miRNAs are typically approximately 22 nt in length. It is believed that they are derived from larger precursors known as small temporal RNAs (stRNAs) approximately 70 nt long.

Interference agents such as siRNAs, and more specifically such as miRNAs, that bind within the 3' UTR (or elsewhere in a target transcript, e.g., in repeated elements of, e.g., Notch and/or transcripts of the Notch family) and inhibit translation may tolerate a larger number of mismatches in the siRNA/template (miRNA/template) duplex, and particularly may tolerate mismatches within the central region of the duplex. In fact, there is evidence that some mismatches may be desirable or required, as naturally occurring stRNAs frequently exhibit such mismatches, as do miRNAs that have been shown to inhibit translation in vitro (Zeng et al., Molecular Cell, 9: 1-20). For example, when hybridized with the target transcript, such miRNAs frequently include two stretches of perfect complementarity separated by a region of mismatch. Such a hybridized complex commonly includes two regions of perfect complementarily (duplex portions) comprising nucleotide pairs, and at least a single mismatched base pair, which may be, e.g., G:A, G:U, G:G, A:A, A:C, U:U, U:C, C:C, G:-, A:-, U:-, C:-, etc. Such mismatched nucleotides, especially if present in tandem (e.g., a two, three or four nucleotide area of mismatch) can form a bulge that separates duplex portions which are located on either flank of such a bulge. A variety of structures are possible. For example, the miRNA may include multiple areas of nonidentity (mismatch). The areas of nonidentity (mismatch) need not be symmetrical in the sense that both the target and the miRNA include nonpaired nucleotides. For example, structures have been described in which only one strand includes nonpaired nucleotides (Zeng et al.). Typically the stretches of perfect complementarily within a miRNA agent are at least 5 nucleotides in length, e.g., 6, 7, or more nucleotides in length, while the regions of mismatch may be, for

example, 1, 2, 3, or 4 nucleotides in length.

In general, any particular siRNA could function to inhibit gene expression both via (i) the "classical" siRNA pathway, in which stability of a target transcript is reduced and in which perfect complementarily between the siRNA and the target is frequently preferred, and also by (ii) the "alternative" pathway (generally characterized as the miRNA pathway in animals), in which translation of a target transcript is inhibited. Generally, the transcripts targeted by a particular siRNA via mechanism (i) would be distinct from the transcript targeted via mechanism (ii), although it is possible that a single transcript could contain regions that could serve as targets for both the classical and alternative pathways. (Note that the terms "classical" and "alternative" are used merely for convenience and generally are believed to reflect historical timing of discovery of such mechanisms in animal cells, but do not reflect the importance, effectiveness, or other features of either mechanism.) One common goal of siRNA design has been to target a single transcript with great specificity, via mechanism (i), while minimizing off-target effects, including those effects potentially elicited via mechanism (ii). However, it is among the goals of the instant invention to provide RNA interference agents that possess mismatch residues by design, either for purpose of mimicking the activities of naturally-occurring miRNAs, or to create agents directed against target RNAs for which no corresponding miRNA is presently known, with the inhibitory and/or therapeutic efficacies/potencies of such mismatch-containing DsiRNA agents (e.g., DsiRNAmm agents) tolerant of, and indeed possibly enhanced by, such mismatches.

The tolerance of miRNA agents for mismatched nucleotides (and, indeed the existence and natural use of mechanism (ii) above in the cell) suggests the use of miRNAs in manners that are advantageous to and/or expand upon the "classical" use of perfectly complementary siRNAs that act via mechanism (i). Because miRNAs are naturally occurring molecules, there are likely to be distinct advantages in applying miRNAs as therapeutic agents. miRNAs benefit from hundreds of millions of years of evolutionary "fine tuning" of their function. Thus, sequence-specific "off target" effects should not be an issue with naturally occurring miRNAs, nor, by extension, with certain synthetic DsiRNAs of the invention (e.g., DsiRNAmm agents) designed to mimic naturally occurring miRNAs. In addition, miRNAs have evolved to modulate the expression of groups of genes, driving both up and down regulation (in certain instances, performing both functions concurrently within a cell with a single miRNA acting promiscuously upon multiple target RNAs), with the result that complex cell functions can be precisely modulated. Such replacement of naturally occurring miRNAs can involve introducing synthetic miRNAs or miRNA mimetics (e.g., certain DsiRNAmms) into diseased tissues in an effort to restore normal proliferation, apoptosis, cell cycle, and other cellular functions that have been affected by down-regulation of one or more miRNAs. In certain instances, reactivation of these miRNA-regulated pathways has produced a significant therapeutic response (e.g., In one study on cardiac hypertrophy, overexpression of miR-133 by adenovirus-mediated delivery of a miRNA expression cassette protected animals from agonist-induced cardiac hypertrophy, whereas reciprocally reduction of miR-133 in wild-type mice by antagomirs caused an increase in hypertrophic markers (Care et al. Nat. Med. 13: 613-618)).

To date, more than 600 miRNAs have been identified as encoded within the human genome, with such miRNAs expressed and processed by a combination of proteins in the nucleus and cytoplasm. miRNAs are highly conserved among vertebrates and comprise approximately 2% of all mammalian genes. Since each miRNA appears to regulate the expression of multiple, e.g., two, three, four, five, six, seven, eight, nine or even tens to hundreds of different genes, miRNAs can function as "master-switches", efficiently regulating and coordinating multiple cellular pathways and processes. By coordinating the expression of multiple genes, miRNAs play key roles in embryonic development, immunity, inflammation, as well as cellular growth and proliferation.

Expression and functional studies suggest that the altered expression of specific miRNAs is critical to a variety of human diseases. Mounting evidence indicates that the introduction of specific miRNAs into disease cells and tissues can induce favorable therapeutic responses (Pappas et al., Expert Opin Ther Targets. 12: 115-27). The promise of miRNA therapy is perhaps greatest in cancer due to the apparent role of certain miRNAs as tumor suppressors. The rationale for miRNA-based therapeutics for, e.g., cancer is supported, at least in part, by the following observations:

(1). miRNAs are frequently mis-regulated and expressed at altered levels in diseased tissues when compared to normal tissues. A number of studies have shown altered levels of miRNAs in cancerous tissues relative to their corresponding normal tissues. Often, altered expression is the consequence of genetic mutations that lead to increased or reduced expression of particular miRNAs. Diseases that possess unique miRNA expression signatures can be exploited as diagnostic and prognostic markers, and can be targeted with the DsiRNA (e.g., DsiRNAmm) agents of the invention.

(2) Mis-regulated miRNAs contribute to cancer development by functioning as oncogenes or tumor suppressors. Oncogenes are defined as genes whose over-expression or inappropriate activation leads to oncogenesis. Tumor suppressors are genes that are required to keep cells from being cancerous; the down-regulation or inactivation of tumor suppressors is a common inducer of cancer. Both types of genes represent preferred drug targets, as such targeting can specifically act upon the molecular basis for a particular cancer. Examples of oncogenic miRNAs are miR-155 and miR-17-92; let-7 is an example of a tumor suppressive miRNA.

(3) Administration of miRNA induces a therapeutic response by blocking or reducing tumor growth in pre-clinical animal studies. The scientific literature provides proof-of-concept studies demonstrating that restoring miRNA function can prevent or reduce the growth of cancer cells in vitro and also in animal models. A well-characterized example is the anti-tumor activity of let-7 in models for breast and lung cancer. DsiRNAs (e.g., DsiRNAmms) of the invention which are designed to mimic let-7 can be used to target such cancers, and it is also possible to use the DsiRNA design parameters described herein to generate new DsiRNA (e.g., DsiRNAmm) agents directed against target RNAs for which no counterpart naturally occurring miRNA is known (e.g., repeats within Notch or other transcripts), to screen for therapeutic lead compounds, e.g., agents that are capable of reducing tumor burden in pre-clinical animal models.

(4) A given miRNA controls multiple cellular pathways and therefore may have superior therapeutic activity. Based on their biology, miRNAs can function as "master switches" of the genome, regulating multiple gene products and coordinating multiple pathways. Genes regulated by miRNAs include genes that encode conventional oncogenes and tumor suppressors, many of which are individually pursued as drug targets by the pharmaceutical industry. Thus, miRNA therapeutics could possess activity superior to siRNAs and other forms of lead compounds by targeting multiple disease and/or cancer-associated genes. Given the observation that mis-regulation of miRNAs is frequently an early event in the process of tumorigenesis, miRNA therapeutics, which replaces missing miRNAs, may be the most appropriate therapy.

(5) miRNAs are natural molecules and are therefore less prone to induce non-specific side-effects. Millions of years of evolution helped to develop the regulatory network of miRNAs, fine-tuning the interaction of miRNA with target messenger RNAs. Therefore, miRNAs and miRNA derivatives (e.g., DsiRNAs designed to mimic naturally occurring miRNAs) will have few if any sequence-specific "off-target" effects when applied in the proper context.

The physical characteristics of siRNAs and miRNAs are similar. Accordingly, technologies that are effective in delivering siRNAs (e.g., DsiRNAs of the invention) are likewise effective in delivering synthetic miRNAs (e.g., certain DsiRNAmms of the invention).

**Conjugation and Delivery of dsNA Agents**

An example of the disclosure relates to a method for treating a subject having or at risk of developing a disease or disorder. In such examples, the dsNA can act as a novel therapeutic agent for controlling the disease or disorder. The method comprises administering a pharmaceutical composition comprising a dsNA of the invention to the patient (e.g., human), such that the expression, level and/or activity a target RNA is reduced. The expression, level and/or activity of a polypeptide encoded by the target RNA might also be reduced by a DsNA of the instant invention.

In the treatment of a disease or disorder, the DsiRNA can be brought into contact with the cells or tissue exhibiting or associated with a disease or disorder. For example, DsNA substantially identical to all or part of a target RNA sequence may be brought into contact with or introduced into a diseased, disease-associated or infected cell, either in vivo or in vitro. Similarly, DsNA substantially identical to all or part of a target RNA sequence may administered directly to a subject having or at risk of developing a disease or disorder.

Therapeutic use of the DsNA agents of the instant invention can involve use of formulations of DsNA agents comprising multiple different DsNA agent sequences. For example, two or more, three or more, four or more, five or more, etc. of the presently described agents can be combined to produce a formulation that, e.g., targets multiple different regions of one or more target RNA(s). A DsNA agent of the instant invention may also be constructed such that either strand of the DsNA agent independently targets two or more regions of a target RNA. Use of multifunctional DsNA molecules that target more than one region of a target nucleic acid molecule is expected to provide potent inhibition of RNA levels and expression. For example, a single multifunctional DsNA construct of the invention can target both conserved and variable regions of a target nucleic acid molecule, thereby allowing down regulation or inhibition of, e.g., different strain variants of a virus, or splice variants encoded by a single target gene.

A DsNA agent of the invention can be conjugated (e.g., at its 5' or 3' terminus of its sense or antisense strand) or unconjugated to another moiety (e.g. a non-nucleic acid moiety such as a peptide), an organic compound (e.g., a dye, cholesterol, or the like). Modifying DsNA agents in this way may improve cellular uptake or enhance cellular targeting activities of the resulting DsNA agent derivative as compared to the corresponding unconjugated DsNA agent, are useful for tracing the DsNA agent derivative in the cell, or improve the stability of the DsNA agent derivative compared to the corresponding unconjugated DsNA agent. Exemplary dsNAs are conjugated to GalNAc, folate, cholesterol, mannose-6-phosphate. In one example a single dsNA comprises more than one GalNAc molecule and more than one folate molecule and more than one cholesterol molecule and more than one mannose-6-phosphate molecule or any combination of all.

The GalNAc ligand not only can be attached to the nucleobase of the nucleoside monomer (i.e. C5 position of Thymidine as shown in Example 6), but also can be attached to the ribose sugar via 2'-OH (or 3'-OH) with a linker. 2'-OH as a conjugation site is particularly useful in the case of tetraloop DsiRNA because the 2'-OHs of the four nucleotide in the loop are exposed to the solvent and are not involved in hydrogen bonding and base stacking which is needed for forming the stable loop based on their crystal structure.

Various linkers may be used to conjugate ligands to the dsNA agents of the invention. Commonly used linkers in art can be used to conjugate the ligands to the dsNA molecules, a few of those linkers are exemplified in tables 4 and 5. Any of these linkers can be used for conjugation of ligands to the dsNA molecules.

In particular, dsNA agents using an acetal linker demonstrated in vitro cell and in vivo targeting and knockdown potency comparable to a click linker. The acetal chemistry used to install the linker to 2'-OH is much milder as compared to the traditional alkylation conditions; therefore, allow selective conjugation to 2' position. This will avoid the tedious separation of the mixture of 2' and 3' isomers and improve the yield significantly.

**RNAi *In Vitro* Assay to Assess DsiRNA Activity**

An in vitro assay that recapitulates RNAi in a cell-free system can optionally be used to evaluate dsNA constructs. For example, such an assay comprises a system described by Tuschl et al., 1999, Genes and Development, 13, 3191-3197 and Zamore et al., 2000, Cell, 101, 25-33, adapted for use with dsNA agents directed against target RNA, and commercially available kits, including Turbo Dicer (Genlantis). A Drosophila extract derived from syncytial blastoderm is used to reconstitute RNAi activity in vitro. Target RNA is generated via in vitro transcription from an appropriate plasmid using T7 RNA polymerase or via chemical synthesis. Sense and antisense dsNA strands (for example 20 uM each) are annealed by incubation in buffer (such as 100 mM potassium acetate, 30 mM HEPES-KOH, pH 7.4, 2 mM magnesium acetate) for 1 minute at 90° C. followed by 1 hour at 37° C., then diluted in lysis buffer (for example 100 mM potassium acetate, 30 mM HEPES-KOH at pH 7.4, 2 mM magnesium acetate). Annealing can be monitored by gel electrophoresis on an agarose gel in TBE buffer and stained with ethidium bromide. The Drosophila lysate is prepared using zero to two-hour-old embryos from Oregon R flies collected on yeasted molasses agar that are dechorionated and lysed. The lysate is centrifuged and the supernatant isolated. The assay comprises a reaction mixture containing 50% lysate [vol/vol], RNA (10-50 pM final concentration), and 10% [vol/vol] lysis buffer containing dsNA (10 nM final concentration). The reaction mixture also contains 10 mM creatine phosphate, 10 ug/ml creatine phosphokinase, 100 um GTP, 100 uM UTP, 100 uM CTP, 500 uM ATP, 5 mM DTT, 0.1 U/uL RNasin (Promega), and 100 uM of each amino acid. The final concentration of potassium acetate is adjusted to 100 mM. The reactions are pre-assembled on ice and preincubated at 25° C. for 10 minutes before adding RNA, then incubated at 25° C. for an additional 60 minutes. Reactions are quenched with 4 volumes of 1.25×Passive Lysis Buffer (Promega). Target RNA cleavage is assayed by RT-PCR analysis or other methods known in the art and are compared to control reactions in which DsiRNA is omitted from the reaction.

Alternately, internally-labeled target RNA for the assay is prepared by in vitro transcription in the presence of [alpha-32P] CTP, passed over a G50 Sephadex column by spin chromatography and used as target RNA without further purification. Optionally, target RNA is 5'-32P-end labeled using T4 polynucleotide kinase enzyme. Assays are performed as described above and target RNA and the specific RNA cleavage products generated by RNAi are visualized on an autoradiograph of a gel. The percentage of cleavage is determined by PHOSPHOR IMAGER® (autoradiography) quantitation of bands representing intact control RNA or RNA from control reactions without dsNA and the cleavage products generated by the assay.

The ability of a double stranded nucleic acid molecule (dsNA) composition of the invention to inhibit protein synthesis can be measured using techniques which are known in the art, for example, by detecting an inhibition in gene transcription or protein synthesis. The level or activity of a target RNA can be determined by any suitable method now known in the art or that is later developed. It can be appreciated that the method used to measure a target RNA and/or the expression of a target RNA can depend upon the nature of the target RNA. For example, if the target RNA encodes a protein, the term "expression" can refer to a protein or the RNA/transcript derived from the target RNA. In such instances, the expression of a target RNA can be determined by measuring the amount of RNA corresponding to the target RNA or by measuring the amount of that protein. Protein can be measured in protein assays such as by staining or immunoblotting or, if the protein catalyzes a reaction that can be measured, by measuring reaction rates. All such methods are known in the art and can be used. Where target RNA levels are to be measured, any art-recognized methods for detecting RNA levels can be used (e.g., RT-PCR, Northern Blotting, etc.). In targeting viral RNAs with the dsNA agents of the instant invention, it is also anticipated that measurement of the efficacy of a dsNA agent in reducing levels of a target virus in a subject, tissue, in cells, either in vitro or in vivo, or in cell extracts can also be used to determine the extent of reduction of target viral RNA level(s). Any of the above measurements can be made on cells, cell extracts, tissues, tissue extracts or any other suitable source material.

Nuclease Resistance Assay

Nuclease resistance of the dsNA molecules is assayed at 0.1 $\mu$M oligonucleotide using 5 × 10-3 units/ml snake

venom phosphodiesterase (U.S. Biochemical Corp.) in a buffer of 50 mM Tris-HCl, pH 8.5, 72 mM CaCl, and 14 mM MgCl2 in a final volume of 50 μl. For Bal31 nuclease assays, nuclease stabilities of the dsNA molecules are assayed at 0.1 μM oligonucleotide using 2 × 10-3 units/ml Bal31 nuclease (Boehringer Mannheim) in a buffer of 20 mM Tris-HCl, pH 7.5, 10 mM NaCl, 5 mM CaCl2, 5 mM $MgCl_2$, 5 mM EDTA (final volume = 100 μl). For both nuclease assays, 5-μl reaction aliquots are removed at the indicated times, added to an equal volume of 80% formamide containing bromphenol blue and xylene cyanol gel tracking dyes, and then heated for 2 min at 95°C. Aliquots are then stored at -20°C until analysis by denaturing polyacrylamide electrophoresis. Quantitation is performed on a Molecular Dynamics PhosphorImager (Molecular Dynamics, Sunnyvale, CA). The same protocol can be repeated with lysosomal extracts and endosomal extracts to mimic the nuclease resistance of dsNA molecules against lysosomal environments.

Cellular Uptake Assay

Cellular uptake experiments were carried out by following the protocols described in Ts'o et al., Cell-Type Specific and Ligand Specific Enhancement of Cellular Uptake of Oligodeoxynucleoside-Methylphosphonates Covalently Linked with a Neoglycopeptide, YEE(ah-GalNAc)s , Bioconjugate Chem. 1995, 6, 695-701.

**Methods of Introducing Nucleic Acids, Vectors, and Host Cells**

DsNA agents of the invention may be directly introduced into a cell (i.e., intracellularly); or introduced extracellularly into a cavity, interstitial space, into the circulation of an organism, introduced orally, or may be introduced by bathing a cell or organism in a solution containing the nucleic acid. Vascular or extravascular circulation, the blood or lymph system, and the cerebrospinal fluid are sites where the nucleic acid may be introduced.

The DsNA agents of the invention can be introduced using nucleic acid delivery methods known in art including injection of a solution containing the nucleic acid, bombardment by particles covered by the nucleic acid, soaking the cell or organism in a solution of the nucleic acid, or electroporation of cell membranes in the presence of the nucleic acid. Other methods known in the art for introducing nucleic acids to cells may be used, such as lipid-mediated carrier transport, chemical-mediated transport, and cationic liposome transfection such as calcium phosphate, and the like. The nucleic acid may be introduced along with other components that perform one or more of the following activities: enhance nucleic acid uptake by the cell or other-wise increase inhibition of the target RNA.

A cell having a target RNA may be from the germ line or somatic, totipotent or pluripotent, dividing or non-dividing, parenchyma or epithelium, immortalized or transformed, or the like. The cell may be a stem cell or a differentiated cell. Cell types that are differentiated include adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes, and cells of the endocrine or exocrine glands.

Depending on the particular target RNA sequence and the dose of DsNA agent material delivered, this process may provide partial or complete loss of function for the target RNA. A reduction or loss of RNA levels or expression (either RNA expression or encoded polypeptide expression) in at least 50%, 60%, 70%, 80%, 90%, 95% or 99% or more of targeted cells is exemplary. Inhibition of target RNA levels or expression refers to the absence (or observable decrease) in the level of RNA or RNA-encoded protein. Specificity refers to the ability to inhibit the target RNA without manifest effects on other genes of the cell. The consequences of inhibition can be confirmed by examination of the outward properties of the cell or organism (as presented below in the examples) or by biochemical techniques such as RNA solution hybridization, nuclease protection, Northern hybridization, reverse transcription, gene expression monitoring with a microarray, antibody binding, enzyme linked immunosorbent assay (ELISA), Western blotting, radioimmunoassay (RIA), other immunoassays, and fluorescence activated cell analysis (FACS). Inhibition of target RNA sequence(s) by the DsNA agents of the invention also can be measured based upon the effect of administration of such DsNA agents upon measurable phenotypes such as tumor size for cancer treatment, viral load/titer for viral infectious diseases, etc. either in vivo or in vitro. For viral infectious diseases, reductions in viral load or titer can include reductions of, e.g., 50%, 60%, 70%, 80%, 90%, 95% or 99% or more, and are often measured in logarithmic terms, e.g., 10-fold, 100-fold, 1000-fold, 105-fold, 106-fold, 107-fold reduction in viral load or titer can be achieved via administration of the DsNA agents of the invention to cells, a tissue, or a subject.

For RNA-mediated inhibition in a cell line or whole organism, expression a reporter or drug resistance gene whose protein product is easily assayed can be measured. Such reporter genes include acetohydroxyacid synthase (AHAS), alkaline phosphatase (AP), beta galactosidase (LacZ), beta glucoronidase (GUS), chloramphenicol acetyltransferase (CAT), green fluorescent protein (GFP), horseradish peroxidase (HRP), luciferase (Luc), nopaline synthase (NOS), octopine synthase (OCS), and derivatives thereof. Multiple selectable markers are available that confer resistance to ampicillin, bleomycin, chloramphenicol, gentamycin, hygromycin, kanamycin, lincomycin, methotrexate, phosphinothricin, puromycin, and tetracyclin. Depending on the assay, quantitation of the amount of gene expression allows

one to determine a degree of inhibition which is greater than 10%, 33%, 50%, 90%, 95% or 99% as compared to a cell not treated according to the present invention.

Lower doses of injected material and longer times after administration of RNA silencing agent may result in inhibition in a smaller fraction of cells (e.g., at least 10%, 20%, 50%, 75%, 90%, or 95% of targeted cells). Quantitation of gene expression in a cell may show similar amounts of inhibition at the level of accumulation of target RNA or translation of target protein. As an example, the efficiency of inhibition may be determined by assessing the amount of gene product in the cell; RNA may be detected with a hybridization probe having a nucleotide sequence outside the region used for the inhibitory DsNA, or translated polypeptide may be detected with an antibody raised against the polypeptide sequence of that region.

The DsNA agent may be introduced in an amount which allows delivery of at least one copy per cell. Higher doses (e.g., at least 5, 10, 100, 500 or 1000 copies per cell) of material may yield more effective inhibition; lower doses may also be useful for specific applications.

## RNA Interference Based Therapy

As is known, RNAi methods are applicable to a wide variety of genes in a wide variety of organisms and the disclosed compositions and methods can be utilized in each of these contexts. Examples of genes which can be targeted by the disclosed compositions and methods include endogenous genes which are genes that are native to the cell or to genes that are not normally native to the cell. Without limitation, these genes include oncogenes, cytokine genes, idiotype (Id) protein genes, prion genes, genes that expresses molecules that induce angiogenesis, genes for adhesion molecules, cell surface receptors, proteins involved in metastasis, proteases, apoptosis genes, cell cycle control genes, genes that express EGF and the EGF receptor, multi-drug resistance genes, such as the MDR1 gene.

More specifically, a target mRNA of the invention can specify the amino acid sequence of a cellular protein (e.g., a nuclear, cytoplasmic, transmembrane, or membrane-associated protein). In another embodiment, the target mRNA of the invention can specify the amino acid sequence of an extracellular protein (e.g., an extracellular matrix protein or secreted protein). As used herein, the phrase "specifies the amino acid sequence" of a protein means that the mRNA sequence is translated into the amino acid sequence according to the rules of the genetic code. The following classes of proteins are listed for illustrative purposes: developmental proteins (e.g., adhesion molecules, cyclin kinase inhibitors, Wnt family members, Pax family members, Winged helix family members, Hox family members, cytokines/lymphokines and their receptors, growth/differentiation factors and their receptors, neurotransmitters and their receptors); oncogene-encoded proteins (e.g., ABLI, BCLI, BCL2, BCL6, CBFA2, CBL, CSFIR, ERBA, ERBB, EBRB2, ETSI, ETSI, ETV6, FGR, FOS, FYN, HCR, HRAS, JUN, KRAS, LCK, LYN, MDM2, MLL, MYB, MYC, MYCLI, MYCN, NRAS, PIM I, PML, RET, SRC, TALI, TCL3, and YES); tumor suppressor proteins (e.g., APC, BRCA1, BRCA2, MADH4, MCC, NF I, NF2, RB I, TP53, and WTI); and enzymes (e.g., ACC synthases and oxidases, ACP desaturases and hydroxylases, ADP-glucose pyrophorylases, ATPases, alcohol dehydrogenases, amylases, amyloglucosidases, catalases, cellulases, chalcone synthases, chitinases, cyclooxygenases, decarboxylases, dextriinases, DNA and RNA polymerases, galactosidases, glucanases, glucose oxidases, granule-bound starch synthases, GTPases, helicases, hernicellulases, integrases, inulinases, invertases, isomerases, kinases, lactases, lipases, lipoxygenases, lysozymes, nopaline synthases, octopine synthases, pectinesterases, peroxidases, phosphatases, phospholipases, phosphorylases, phytases, plant growth regulator synthases, polygalacturonases, proteinases and peptidases, pullanases, recombinases, reverse transcriptases, RUBISCOs, topoisomerases, and xylanases).

In one aspect, the target mRNA molecule of the invention specifies the amino acid sequence of a protein associated with a pathological condition. For example, the protein may be a pathogen-associated protein (e.g., a viral protein involved in immunosuppression of the host, replication of the pathogen, transmission of the pathogen, or maintenance of the infection), or a host protein which facilitates entry of the pathogen into the host, drug metabolism by the pathogen or host, replication or integration of the pathogen's genome, establishment or spread of infection in the host, or assembly of the next generation of pathogen. Pathogens include RNA viruses such as flaviviruses, picornaviruses, rhabdoviruses, filoviruses, retroviruses, including lentiviruses, or DNA viruses such as adenoviruses, poxviruses, herpes viruses, cytomegaloviruses, hepadnaviruses or others. Additional pathogens include bacteria, fungi, helminths, schistosomes and trypanosomes. Other kinds of pathogens can include mammalian transposable elements. Alternatively, the protein may be a tumor-associated protein or an autoimmune disease-associated protein.

The target gene may be derived from or contained in any organism. The organism may be a plant, animal, protozoa, bacterium, virus or fungus. See e.g., U.S. Pat. No. 6,506,559.

Stability profile, pharamacokinetics, cellular targeting ability and biodistribution characteristics of dsNA molecules are evaluated by suitable methods known in the art including but not limited to methods based on the protocols taught by Rice et al. (In vivo targeting function of N-linked oligosaccharides with terminating galactose and N-acetylgalactosamine residues, Chiu MH, Tamura T, Wadhwa MS, Rice KG, J Biol Chem. 1994 Jun 10; 269 (23):16195-202). $^{125}$I (Sodium Iodide), Sephadex G-10, Chloramine T, Sodium meta bisulphite, heparin, BSA, HEPES, Collagenase,

ketamine hydrochloride, Xylazine hydrocholoride and ICR mice are obtained from commercial vendors.

Radiolabeling dsNA molecules

dsNA molecules conjugated to ligands such as GalNac, Cholesterol, Folate or Mannose -6-phosphate are labeled with $^{125}I$ using procedures described by Rice et al. Radioiodinated dsNAs are chromatographed on a SephadexG-10 column (0.8 × 25 cm) and eluted with 0.15 M sodium chloride (pH 7.0) and collected in 0.5 ml fractions. The purity of each iodinated dsNA comprising, for example, a GalNac ligand is analyzed by spotting 1 μl (2 nCi) at the origin of a Thin layer chromatography (TLC) plate developed with ethyl acetate: acetic acid: pyridine: water at a ratio optimized for each dsNA. Quantitative densitometry is performed on a Phosphor Imager (Molecular Dynamics, Sunnyvale, CA) following 12 hour auto radiographic exposure at room temperature. ImageQuant software (Molecular Dynamics, Sunnyvale, CA) is used to integrate the densitometry trace and establish > 95% purity for each iodinated oligonucleotide.

In vitro Stability Assay for dsNA molecules

Each radioiodinated oligosaccharide (1.5 μl, 75 nCi) is added to 100 μl of heparinized whole mouse blood and incubated at 37°C. Time points (10 μl) are removed at 10, 20, and 30 min and 1, 2, 3, 4, 5, and 6 h, and analyzed using TLC and quantitative autoradiography as described above.

Pharmacokinetic Analysis

The pharmacokinetics of dsNAs is performed in three to four mice. Mice are anesthetized and a dual jugular vein cannulation is performed. dsNAs with GalNAc ligands are dosed in the left vein while blood time points are taken at 1, 3, 6, 10, 15, 20, 30, 40, and 60 min from the right vein. Serial blood time points are analyzed by direct γ-counting, after which, the dsNA is extracted from blood by adding 60 μl of water and 200 μl of acetonitrile. Proteins are precipitated by centrifugation for 10 min (13,000 × g)

$$\text{Eq. 1 } Cb = Ae{-}\alpha t + Be{-}\beta t$$

and the pellet is washed twice with 50 μl of 80 v/v% acetonitrile, resulting in recovery of 80% of the radioactivity. Extracts are combined and evaporated to dryness on a Centra-Vap under reduced pressure and reconstituted in 3 μl of water. Each time point is analyzed by spotting 1 μl onto a TLC plate which is then developed and auto radiographed as described above. Pharmacokinetic parameters are derived from direct blood counts versus time for triplicate data sets of each oligosaccharide then averaged to obtain the mean and standard deviation. Iterative non-linear least-squares fits for individual data sets are obtained with PCNONLIN (SCI Software, Lexington, KY) using a two-compartment open model described by the integrated equation 1:

Cb is the concentration of oligosaccharide in blood. A and B are constants, and α and β are hybrid first-order rate constants that characterize the slopes of the fast and slow phases of decline in a plasma concentration versus time profile. The mean residence time (MRT) is calculated according to equation 2:

$$\text{Eq. 2} \qquad MRT = \frac{\left(\frac{A}{\alpha^2} + \frac{B}{\beta^2}\right)}{\left(\frac{A}{\alpha} + \frac{B}{\beta}\right)}$$

The Mean residence time (MRT) is the average time that the oligosaccharide was in the mouse (Riegelman and Collier, J.Pharmacokinet. BioPharm. 8,509-534,1980). The total body clearance (Cltb) is calculated using equation 3 and the volume of distribution at steady-state (Vdss) is calculated according to equation 4 (Benet and Galeazzi, J.Pharm.Sci, 68, 1071-1074, 1979).

$$\text{Eq. 3} \qquad Cl_{tb} = \frac{dose}{\left(\frac{A}{\alpha} + \frac{B}{\beta}\right)}$$

$$\text{Eq. 4 Vdss} = CL_{tb} * MRT$$

Whole-body autoradiography

Targeting and/or biodistribution of a dsNA of the invention is determined by suitable methods known in the art, including the methods described below.

Mice are anesthetized by intraperitoneal. injection of ketamine hydrochloride (100 mg/kg) and xylazine hydrochloride (10 mg/kg). A single silastic catheter is inserted into the right jugular vein and an intravenous bolus dose of radiolabeled dsNA with GalNAc ligand (0 μl, 7 μCi, in saline) is administered after which the catheter is removed and the vein is ligated. After 30 min, mice are euthanized by lethal injection of phenobarbital (100 mg/kg). Immediately after sacrifice, the mice are immersed in a hexane dry ice bath (-70°C) for 5 min and mounted in a 4% (w/v) carboxymethylcellulose block which is then cooled to -20°C. Longitudinal sections of 25 μm are cut near the midline of the mice at a temperature of -15°C on a cryo-microtome (LKB 2250, Sweden). The sections are collected on adhesive tape (Scotch 810, 3M Co., Minneapolis,MN), dehydrated at -15°C for 24 h, and then autoradiographed for 48 h using a Phosphor Imager.

Biodistribution Assay of dsNA

Mice are anesthetized, as described above, followed by insertion of a single cannula into the right jugular vein. dsNA (15 μl, 2 μCi, in saline) are dosed intravenously and allowed to bio-distribute 30 min after which mice are sacrificed by cervical dislocation. The major organs (liver, lungs, spleen, stomach, kidneys, heart, large intestine, and small intestine) were harvested, rinsed with saline, and measured by direct γ-counting for total radioactivity. The level of radioactivity indicates the areas of distribution and allows one to determine which tissues are being enriched for the dsNA molecule.

Cellular Targeting and Delivery

An optimal biodistribution time based on the assay described above is selected to analyze the injection of dsNA molecules. Whole-body auto radiographic analysis allows screening of all tissues to identify the major biodistribution sites. Direct γ-counting of dissected tissues is used to confirm the results observed by whole-body-autoradiography. Quantitative biodistribution analysis is used to determine liver targeting efficiencies of dsNA molecules with GalNac ligands. The cellular targeting of dsNAs is established from the ratio of the radioactive dose in liver nonparenchymal and parenchymal cells.

Binding Affinity Assays:

Binding Affinity is measured through several methods well established in the art. One of the methods of measuring binding affinity is by Fluorescence polarization as shown in Example 20. Another method that is also used is taught by Seth et al (Targeted delivery of antisense oligonucleotides to hepatocytes using triantennary N-acetyl galactosamine improves potency 10-fold in mice, Nucleic Acids Res. 2014 Jul;42(13):8796-807.).

Desialylation and 1251-Labeling of α1-acid glycoprotein (AGP) for Competition Assay in Mouse Primary Hepatocytes (100 nmol AGP) are incubated in 50-mM sodium acetate buffer (pH 5) with 1 U neuraminidase-agarose for 16 h at 37° C. Desialylation is confirmed by either sialic acid assay or size exclusion chromatography (SEC). Iodination using iodine monochloride is achieved as described by Atsma et al (Atsma D.E., Kempen H.J., Nieuwenhuizen W., van 't Hooft, F.M., Pauwels E.K., Partial characterization of low density lipoprotein preparations isolated from fresh and frozen plasma after radiolabeling by seven different methods. J. Lipid Res. 1991; 32:173-181). One milliliter of desialylated α1-acid glycoprotein (de-AGP, 1 mg/ml) and 0.2 ml 1-M glycine in 0.25 M NaOH (pH 10) is added to a mixture of 10-mM iodine chloride solution (7 μl/mg protein), Na125I (2.5 μl/mg protein) and 1M glycine in 0.25 M NaOH (25 μl/mg protein). After incubation for 10 min at room temperature, 1251 -labeled de-AGP is separated from free 125I by concentrating the mixture twice utilizing a 3 kDa MWCO spin column. The protein is tested for labeling efficiency and purity on a HPLC system equipped with an Agilent SEC-3 column (7.8 × 300 mm) and a β-RAM counter.

Competition experiments utilizing 125I-labeled de-AGP and dsNA with GalNAc ligands are performed as follows-freshly isolated mouse hepatocytes (106 cells/ml) are plated on six-well plates in 2 ml of appropriate growth media. The primary hepatocytes are cultured in William's media containing 10% fetal bovine serum (FBS), 1× non-essential amino acids and 1× sodium pyruvate. Cells are incubated 16-20 h at 37° C with 5 and 10% CO2, respectively. Cells are then washed with media without FBS prior to the experiment. Cells are incubated for 30 min at 37° C with 1 ml competition mix containing appropriate growth media with 2% FBS, 10-8 M 1251 -labeled de-AGP and GalNAc- containing dsNAs at concentrations ranging from 10-11 to 10-5 M. Non-specific binding is determined in the presence of 10-2 M GalNAc sugar. Cells are washed twice with media without FBS to remove unbound 1251 -labeled de-AGP and competitor GalNAc

-dsNA. Cells are lysed using Qiagen's RLT buffer containing 1% β-mercaptoethanol. Lysates are transferred to round bottom assay tubes after a brief 10-min freeze/thaw cycle and assayed on a γ-counter. Non-specific binding is subtracted before dividing 1251 protein counts by the value of the lowest GalNAc-dsNA concentration counts. The inhibition curves are fitted according to a single site competition binding equation using a non-linear regression algorithm.

Immunogenicity Assay

Synthetic siRNA duplexes can induce high levels of inflammatory cytokines and type I interferons, in particular interferon-a, after systemic administration in mammals and in primary human blood cell cultures. The levels of immunogenicity are determined by measuring the amount of interferon-a before and after the dsNA molecules of interest at varying concentrations are administered as described above. Synthetic Poly(I:C) (Sigma-Aldrich) is used to induce an immune response in PBMCs as a positive control. After 24 hours of incubation, the culture supernatants are collected and assayed for INF-α by sandwich ELISA (Invitrogen) according to the supplier's instructions.

## Pharmaceutical Compositions

An example of the disclosure provides for a pharmaceutical composition comprising the DsNA agent of the present invention. The dsNA agent sample can be suitably formulated and introduced into the environment of the cell by any means that allows for a sufficient portion of the sample to enter the cell to induce gene silencing, if it is to occur. Many formulations for dsNA are known in the art and can be used so long as the dsNA gains entry to the target cells so that it can act. See, e.g., U.S. published patent application Nos. 2004/0203145 A1 and 2005/0054598 A1. For example, the DsNA agent of the instant invention can be formulated in buffer solutions such as phosphate buffered saline solutions, liposomes, micellar structures, and capsids. Formulations of DsNA agent with cationic lipids can be used to facilitate transfection of the DsNA agent into cells. For example, cationic lipids, such as lipofectin (U.S. Pat. No. 5,705,188), cationic glycerol derivatives, and polycationic molecules, such as polylysine (published PCT International Application WO 97/30731), can be used. Suitable lipids include Oligofectamine, Lipofectamine (Life Technologies), NC388 (Ribozyme Pharmaceuticals, Inc., Boulder, Colo.), or FuGene 6 (Roche) all of which can be used according to the manufacturer's instructions.

Such compositions typically include the nucleic acid molecule and a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered

sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, (e.g., a gas such as carbon dioxide, or a nebulizer). Such methods include those described in U.S. Pat. No. 6,468,798.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

The compounds can also be administered by transfection or infection using methods known in the art, including but not limited to the methods described in McCaffrey et al. (2002), Nature, 418(6893), 38-9 (hydrodynamic transfection); Xia et al. (2002), Nature Biotechnol., 20(10), 1006-10 (viral-mediated delivery); or Putnam (1996), Am. J. Health Syst. Pharm. 53(2), 151-160, erratum at Am. J. Health Syst. Pharm. 53(3), 325 (1996).

The compounds can also be administered by any method suitable for administration of nucleic acid agents, such as a DNA vaccine. These methods include gene guns, bio injectors, and skin patches as well as needle-free methods such as the microparticle DNA vaccine technology disclosed in U.S. Pat. No. 6,194,389, and the mammalian transdermal needle-free vaccination with powder-form vaccine as disclosed in U.S. Pat. No. 6,168,587. Additionally, intranasal delivery is possible, as described in, inter alia, Hamajima et al. (1998), Clin. Immunol. Immunopathol., 88(2), 205-10. Liposomes (e.g., as described in U.S. Pat. No. 6,472,375) and microencapsulation can also be used. Biodegradable targetable microparticle delivery systems can also be used (e.g., as described in U.S. Pat. No. 6,471,996).

In one example, the active compounds are prepared with carriers that will protect the compound against rapid elimination from the body, such as a controlled release formulation, including implants and microencapsulated delivery systems. Biodegradable, bio compatible polymers can be used, such as ethylene vinyl acetate, polyanhydrides, polyglycolic acid, collagen, polyorthoesters, and polylactic acid. Such formulations can be prepared using standard techniques. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the disclosure, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to

more accurately determine useful doses in humans. Levels in plasma may be measured, for example, by high performance liquid chromatography.

As defined herein, a therapeutically effective amount of a nucleic acid molecule (i.e., an effective dosage) depends on the nucleic acid selected. For instance, if a plasmid encoding a DsNA agent is selected, single dose amounts in the range of approximately 1 pg to 1000 mg may be administered; in some examples, 10, 30, 100, or 1000 pg, or 10, 30, 100, or 1000 ng, or 10, 30, 100, or 1000 μg, or 10, 30, 100, or 1000 mg may be administered. In some examples, 1-5 g of the compositions can be administered. The compositions can be administered from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

It can be appreciated that the method of introducing DsNA agents into the environment of the cell will depend on the type of cell and the makeup of its environment. For example, when the cells are found within a liquid, one preferable formulation is with a lipid formulation such as in lipofectamine and the DsNA agents can be added directly to the liquid environment of the cells. Lipid formulations can also be administered to animals such as by intravenous, intramuscular, or intraperitoneal injection, or orally or by inhalation or other methods as are known in the art. When the formulation is suitable for administration into animals such as mammals and more specifically humans, the formulation is also pharmaceutically acceptable. Pharmaceutically acceptable formulations for administering oligonucleotides are known and can be used. In some instances, it may be preferable to formulate DsNA agents in a buffer or saline solution and directly inject the formulated DsNA agents into cells, as in studies with oocytes. The direct injection of DsNA agents duplexes may also be done. For suitable methods of introducing dsNA (e.g., DsNA agents), see U.S. published patent application No. 2004/0203145 A1.

Suitable amounts of a DsNA agent must be introduced and these amounts can be empirically determined using standard methods. Typically, effective concentrations of individual DsNA agent species in the environment of a cell will be about 50 nanomolar or less, 10 nanomolar or less, or compositions in which concentrations of about 1 nanomolar or less can be used. In another example, methods utilizing a concentration of about 200 picomolar or less, and even a concentration of about 50 picomolar or less, about 20 picomolar or less, about 10 picomolar or less, or about 5 picomolar or less can be used in many circumstances.

The method can be carried out by addition of the DsNA agent compositions to any extracellular matrix in which cells can live provided that the DsNA agent composition is formulated so that a sufficient amount of the DsNA agent can enter the cell to exert its effect. For example, the method is amenable for use with cells present in a liquid such as a liquid culture or cell growth media, in tissue explants, or in whole organisms, including animals, such as mammals and especially humans.

The level or activity of a target RNA can be determined by any suitable method now known in the art or that is later developed. It can be appreciated that the method used to measure a target RNA and/or the expression of a target RNA can depend upon the nature of the target RNA. For example, if the target RNA encodes a protein, the term "expression" can refer to a protein or the RNA/transcript derived from the target RNA. In such instances, the expression of a target RNA can be determined by measuring the amount of RNA corresponding to the target RNA or by measuring the amount of that protein. Protein can be measured in protein assays such as by staining or immunoblotting or, if the protein catalyzes a reaction that can be measured, by measuring reaction rates. All such methods are known in the art and can be used. Where target RNA levels are to be measured, any art-recognized methods for detecting RNA levels can be used (e.g., RT-PCR, Northern Blotting, etc.). In targeting viral RNAs with the DsNA agents of the instant invention, it is also anticipated that measurement of the efficacy of a DsNA agent in reducing levels of a target virus in a subject, tissue, in cells, either in vitro or in vivo, or in cell extracts can also be used to determine the extent of reduction of target viral RNA level(s). Any of the above measurements can be made on cells, cell extracts, tissues, tissue extracts or any other suitable source material.

The determination of whether the expression of a target RNA has been reduced can be by any suitable method that can reliably detect changes in RNA levels. Typically, the determination is made by introducing into the environment of a cell undigested DsNA such that at least a portion of that DsNA agent enters the cytoplasm, and then measuring the level of the target RNA. The same measurement is made on identical untreated cells and the results obtained from each measurement are compared.

The DsNA agent can be formulated as a pharmaceutical composition which comprises a pharmacologically effective amount of a DsNA agent and pharmaceutically acceptable carrier. A pharmacologically or therapeutically effective amount refers to that amount of a DsNA agent effective to produce the intended pharmacological, therapeutic or preventive result. The phrases "pharmacologically effective amount" and "therapeutically effective amount" or simply "effective amount" refer to that amount of RNA effective to produce the intended pharmacological, therapeutic or preventive result. For example, if a given clinical treatment is considered effective when there is at least a 20% reduction

in a measurable parameter associated with a disease or disorder, a therapeutically effective amount of a drug for the treatment of that disease or disorder is the amount necessary to effect at least a 20% reduction in that parameter.

Suitably formulated pharmaceutical compositions of this disclosure can be administered by any means known in the art such as by parenteral routes, including intravenous, intramuscular, intraperitoneal, subcutaneous, transdermal, airway (aerosol), rectal, vaginal and topical (including buccal and sublingual) administration. In some examples, the pharmaceutical compositions are administered by intravenous or intraparenteral infusion or injection.

In general, a suitable dosage unit of dsNA will be in the range of 0.001 to 0.25 milligrams per kilogram body weight of the recipient per day, or in the range of 0.01 to 20 micrograms per kilogram body weight per day, or in the range of 0.01 to 10 micrograms per kilogram body weight per day, or in the range of 0.10 to 5 micrograms per kilogram body weight per day, or in the range of 0.1 to 2.5 micrograms per kilogram body weight per day. Pharmaceutical composition comprising the dsNA can be administered once daily. However, the therapeutic agent may also be dosed in dosage units containing two, three, four, five, six or more sub-doses administered at appropriate intervals throughout the day. In that case, the dsNA contained in each sub-dose must be correspondingly smaller in order to achieve the total daily dosage unit. The dosage unit can also be compounded for a single dose over several days, e.g., using a conventional sustained release formulation which provides sustained and consistent release of the dsNA over a several day period. Sustained release formulations are well known in the art. In this example, the dosage unit contains a corresponding multiple of the daily dose. Regardless of the formulation, the pharmaceutical composition must contain dsNA in a quantity sufficient to inhibit expression of the target gene in the animal or human being treated. The composition can be compounded in such a way that the sums of the multiple units of dsNA together contain a sufficient dose.

Data can be obtained from cell culture assays and animal studies to formulate a suitable dosage range for humans. The dosage of compositions of the disclosure lies within a range of circulating concentrations that include the ED50 (as determined by known methods) with little or no toxicity. The dosage may vary within this range depending upon the dosage form employed and the route of administration utilized. For any compound used in the method of the disclosure, the therapeutically effective dose can be estimated initially from cell culture assays. A dose may be formulated in animal models to achieve a circulating plasma concentration range of the compound that includes the IC50 (i.e., the concentration of the test compound which achieves a half-maximal inhibition of symptoms) as determined in cell culture. Such information can be used to more accurately determine useful doses in humans. Levels of dsNA in plasma may be measured by standard methods, for example, by high performance liquid chromatography.

The pharmaceutical compositions can be included in a kit, container, pack, or dispenser together with instructions for administration.

## Methods of Treatment

The present disclosure provides for both prophylactic and therapeutic methods of treating a subject at risk of (or susceptible to) a disease or disorder caused, in whole or in part, by the expression of a target RNA and/or the presence of such target RNA (e.g., in the context of a viral infection, the presence of a target RNA of the viral genome, capsid, host cell component, etc.).

"Treatment", or "treating" as used herein, is defined as the application or administration of a therapeutic agent (e.g., a DsNA agent or vector or transgene encoding same) to a patient, or application or administration of a therapeutic agent to an isolated tissue or cell line from a patient, who has the disease or disorder, a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease.

In one example, the disclosure provides a method for preventing in a subject, a disease or disorder as described above, by administering to the subject a therapeutic agent (e.g., a DsNA agent or vector or transgene encoding same). Subjects at risk for the disease can be identified by, for example, any or a combination of diagnostic or prognostic assays as described herein. Administration of a prophylactic agent can occur prior to the detection of, e.g., viral particles in a subject, or the manifestation of symptoms characteristic of the disease or disorder, such that the disease or disorder is prevented or, alternatively, delayed in its progression.

Another example of the disclosure pertains to methods of treating subjects therapeutically, i.e., alter onset of symptoms of the disease or disorder. These methods can be performed in vitro (e.g., by culturing the cell with the DsNA agent) or, alternatively, in vivo (e.g., by administering the DsNA agent to a subject).

With regards to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype"). Thus, another example of the disclosure provides methods for tailoring an individual's prophylactic or therapeutic treatment with either the target RNA molecules of the present invention or target RNA modulators according

to that individual's drug response genotype. Pharmacogenomics allows a clinician or physician to target prophylactic or therapeutic treatments to patients who will most benefit from the treatment and to avoid treatment of patients who will experience toxic drug-related side effects.

Therapeutic agents can be tested in an appropriate animal model. For example, a DsNA agent (or expression vector or transgene encoding same) as described herein can be used in an animal model to determine the efficacy, toxicity, or side effects of treatment with said agent. Alternatively, a therapeutic agent can be used in an animal model to determine the mechanism of action of such an agent. For example, an agent can be used in an animal model to determine the efficacy, toxicity, or side effects of treatment with such an agent. Alternatively, an agent can be used in an animal model to determine the mechanism of action of such an agent.

The practice of the present invention employs, unless otherwise indicated, conventional techniques of chemistry, molecular biology, microbiology, recombinant DNA, genetics, immunology, cell biology, cell culture and transgenic biology, which are within the skill of the art. See, e.g., Maniatis et al., 1982, Molecular Cloning (Cold Spring Harbor Laboratory Press, Cold Spring Harbor, N.Y.); Sambrook et al., 1989, Molecular Cloning, 2nd Ed. (Cold Spring Harbor Laboratory Press, Cold Spring Harbor, N.Y.); Sambrook and Russell, 2001, Molecular Cloning, 3rd Ed. (Cold Spring Harbor Laboratory Press, Cold Spring Harbor, N.Y.); Ausubel et al., 1992), Current Protocols in Molecular Biology (John Wiley and Sons, including periodic updates); Glover, 1985, DNA Cloning (IRL Press, Oxford); Anand, 1992; Guthrie and Fink, 1991; Harlow and Lane, 1988, Antibodies, (Cold Spring Harbor Laboratory Press, Cold Spring Harbor, N.Y.); Jakoby and Pastan, 1979; Nucleic Acid Hybridization (B. D. Hames and S. J. Higgins eds. 1984); Transcription And Translation (B. D. Hames and S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Riott, Essential Immunology, 6th Edition, Blackwell Scientific Publications, Oxford, 1988; Hogan et al., Manipulating the Mouse Embryo, (Cold Spring Harbor Laboratory Press, Cold Spring Harbor, N.Y., 1986); Westerfield, M., The zebrafish book. A guide for the laboratory use of zebrafish (Danio rerio), (4th Ed., Univ. of Oregon Press, Eugene, 2000).

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

## EXAMPLES

The present invention is described by reference to the following Examples, which are offered by way of illustration and are not intended to limit the invention in any manner. Standard techniques well known in the art or the techniques specifically described below were utilized.

## Example 1 Methods of Synthesis

### Oligonucleotide Synthesis, *In Vitro and In vivo* Use

Individual RNA strands were synthesized and HPLC purified according to standard methods (Integrated DNA Technologies, Coralville, Iowa). All oligonucleotides were quality control released on the basis of chemical purity by HPLC analysis and full length strand purity by mass spectrometry analysis. Duplex RNA DsNAs were prepared before use by mixing equal quantities of each strand, briefly heating to 100° C. in RNA buffer (IDT) and then allowing the mixtures to cool to room temperature.

### Cell Culture and RNA Transfection

HeLa cells were obtained from ATCC and maintained in Dulbecco's modified Eagle medium (HyClone) supplemented with 10% fetal bovine serum (HyClone) at 37° C. under 5% CO2. For RNA transfections of FIGS. 7, 9, 12, and 13, HeLa cells were transfected with DsNAs as indicated at a final concentration of 0.1 nM using Lipofectamine™ RNAiMAX (Invitrogen) and following manufacturer's instructions. Briefly, 2.54, of a 0.0204 stock solution of each DsNA were mix with 46.54, of Opti-MEM I (Invitrogen) and 14, of Lipofectamine™ RNAiMAX. The resulting 504, mix was added into individual wells of 12 well plates and incubated for 20 min at RT to allow DsNA: Lipofectamine™ RNAiMAX complexes to form. Meanwhile, HeLa cells were trypsinized and resuspended in medium at a final concentration of 367 cells/μL.

Finally, 4504, of the cell suspension were added to each well (final volume 500 μL) and plates were placed into the incubator for 24 hours.

**RNA Isolation and Analysis, *In Vitro***

Cells were washed once with 2 mL of PBS, and total RNA was extracted using RNeasy Mini Kit™ (Qiagen) and eluted in a final volume of 30 μL. 1 μg of total RNA was reverse-transcribed using Transcriptor 1st Strand cDNA Kit™ (Roche) and random hexamers following manufacturer's instructions. One-thirtieth (0.66 μL) of the resulting cDNA was mixed with 54, of iQ™ Multiplex Powermix (Bio-Rad) together with 3.334, of H2O and 14, of a 304 mix containing 2 sets of primers and probes specific for human genes HPRT-1 (accession number NM-000194) and SFRS9 (accession number NM-003769) genes:

(SEQ ID NO:1) Hu HPRT forward primer F517 GACTTTGCTTTCCTTGGTCAG
(SEQ ID NO:2) Hu HPRT reverse primer R591 GGCTTATATCCAACACTTCGTGGG
(SEQ ID NO:3) Hu HPRT probe P554 Cy5-ATGGTCAAGGTCGCAAGCTTGCTGGT-IBFQ
(SEQ ID NO:4) Hu SFRS9 forward primer F569 TGTGCAGAAGGATGGAGT
(SEQ ID NO:5) Hu SFRS9 reverse primer R712 CTGGTGCTTCTCTCAGGATA
(SEQ ID NO:6) Hu SFRS9 probe P644 HEX-TGGAATATGCCCTGCGTAAACTGGA-IBFQ

***In Vivo* Sample Preparation and Injection**

DsNA was formulated in Invivofectamine™ according to manufacturer's protocol (Invitrogen, Carlsbad, Calif.). Briefly, the N/group of mice and body weight of the mice used were determined, then amount of DsNA needed for each group of mice treated was calculated. One ml IVF-oligo was enough for 4 mice of 25 g/mouse at 10 mg/kg dosage. One mg DsNA was added to one ml Invivofectamine™, and mixed at RT for 30 min on a rotator. 14 ml of 5% glucose was used to dilute formulated IVF-DsNA and was applied to 50 kDa molecular weight cutoff spin concentrators (Amicon). The spin concentrators were spun at 4000 rpm for ~2 hours at 4° C until the volume of IVF-DsNA was brought down to less than 1 ml. Recovered IVF-DsNA was diluted to one ml with 5% glucose and readied for animal injection.

**Animal Injection and Tissue Harvesting**

Animals were subjected to surgical anesthesia by i.p. injection with Ketamine/Xylazine. Each mouse was weighed before injection. Formulated IVF-DsNA was injected i.v. at 100 ul/10 g of body weight. After 24 hours, mice were sacrificed by $CO_2$ inhalation. Tissues for analysis were collected and placed in tubes containing 2 ml RNAlater™ (Qiagen) and rotated at RT for 30 min before incubation at 4° C overnight. The tissues were stored subsequently at -80° C until use.

**Tissue RNA Preparation and Quantitation**

About 50-100 mg of tissue pieces were homogenized in 1 ml QIAzol™ (Qiagen) on Tissue Lyser™ (Qiagen). Then total RNA were isolated according to the manufacturer's protocol. Briefly, 0.2 ml Chloroform (Sigma-Aldrich) was added to the QIAzol™ lysates and mixed vigorously by vortexing. After spinning at 14,000 rpm for 15 min at 4° C., aqueous phase was collected and mixed with 0.5 ml of isopropanol. After another centrifugation at 14,000 rpm for 10 min, the RNA pellet was washed once with 75% ethanol and briefly dried. The isolated RNA was resuspended in 100 μl RNase-Free water, and subjected to clean up with RNeasy™ total RNA preparation kit (Qiagen) or SV 96 total RNA Isolation System (Promega) according to manufacturer's protocol.

**First Strand cDNA Synthesis, In Vivo**

1 μg of total RNA was reverse-transcribed using Transcriptor 1st Strand cDNA Kit™ (Roche) and oligo-dT following manufacturer's instructions. One-fortieth (0.66 μL) of the resulting cDNA was mixed with 54, of IQ Multiplex Powermix (Bio-Rad) together with 3.33 μL of H2O and 1 μL of a 3 μM mix containing 2 sets of primers and probes specific for mouse genes HPRT-1 (accession number NM-013556) and KRAS (accession number NM-021284) genes:

(SEQ ID NO:7) Mm HPRT forward primer F576 CAAACTTTGCTTTCCCTGGT
(SEQ ID NO:8) Mm HPRT reverse primer R664 CAACAAAGTCTGGCCTGTATC
(SEQ ID NO:9) Mm HPR obe P616 Cy5-TGGTTAAGGTTGCAAGCTTGCTGGTG-IBFQ
(SEQ ID NO:10) Mm KRAS forward primer F275 CTTTGTGGATGAGTACGACC

(SEQ ID NO:11) Mm KRAS reverse primer R390 CACTGTACTCCTCTTGACCT
(SEQ ID NO:12) Mm KRAS probe P297 FAM-CGATAGAGGACTCCTACAGGAAACAAGT - IBFQ

**Quantitative RT-PCR**

A CFX96 Real-time System with a CI000 Thermal cycler (Bio-Rad) was used for the amplification reactions. PCR conditions were: 95° C for 3 min; and then cycling at 95° C, 10 sec; 55° C, 1 min for 40 cycles. Each sample was tested in triplicate. For HPRT experiment, relative HPRT mRNA levels were normalized to SFRS9 mRNA levels and compared with mRNA levels obtained in control samples treated with the transfection reagent plus a control mismatch duplex, or untreated. For KRAS examples, relative KRAS mRNA levels were normalized to HPRT-1 mRNA levels and compared with mRNA levels obtained in control samples from mice treated with 5% glucose. Data were analyzed using Bio-Rad CFX Manager version 1.0 (in vitro Examples) or 1.5 (in vivo Example) software.

**Example 2 Efficacy of DsiRNA Agents Possessing Single Stranded Extensions**

DsiRNA agents possessing single stranded extensions were examined for efficacy of sequence-specific target mRNA inhibition. Specifically, KRAS-249M and HPRT-targeting DsiRNA duplexes possessing 5' single stranded guide extensions were transfected into HeLa cells at a fixed concentration of 20 nM and HPRT expression levels were measured 24 hours later (FIGS. 7 and 9). Transfections were performed in duplicate, and each duplicate was assayed in triplicate for KRAS-249M and HPRT expression, respectively, by qPCR.

Under these conditions (0.1 nM duplexes, Lipofectamine™ RNAiMAX transfection), KRAS-249 gene expression was reduced by about 60-85% by duplexes DNA10PS, RNA10PS, RNA10PS-2'-OME, DNA15PS, RNA15PS, and RNA15PS-2'OME (FIG. 7). By comparison, a duplex without the single stranded guide extensions reduced KRAS-249 gene expression by about 90%. Thus, the duplexes having single stranded guide extensions were as effective in silencing KRAS-249 as a duplex without the single stranded guide extensions. All single stranded extended duplexes contained phosphorothioate backbone modifications in the single stranded extension region. For duplexes DNA10PS, RNA10PS, RNA10PS-2'-OME, having 10 nucleotide single stranded guide extensions, KRAS-249 gene expression was reduced about 75-85%. For duplexes DNA10PS, RNA10PS, RNA10PS-2'-OME, having 15 nucleotide single stranded guide extensions, KRAS-249 gene expression was reduced 60-70%. Generally, the duplexes having the 10 nucleotide guide extensions reduced KRAS target gene expression more than the duplexes having the 15 nucleotide guide extensions, regardless of the nucleotides present in the 5' guide extensions. In particular, the silencing activity of duplexes having guide extensions containing deoxyribonucleotides, was more sensitive to the increased length of 15 nucleotides, compared to the duplexes containing ribonucleotides and 2'-O-methyl ribonucleotides. Processing of 5' guide strand extended duplexes by Dicer, which were used in the experiments targeting gene expression of KRAS-249, was also shown by in vitro assay (FIG. 10).

Similarly, under the same conditions (0.1 nM duplexes, Lipofectamine™ RNAiMAX transfection), HPRT1 gene expression was reduced by about 65-85% by duplexes DNA10PS, RNA10PS, RNA10PS-2'-OME, DNA15PS, RNA15PS, and RNA15PS-2'OME (FIG. 9). By comparison, a duplex without the single stranded guide extensions reduced HPRT1 gene expression by about 90%. Thus, the duplexes having single stranded guide extensions were as effective in silencing HPRT1 as a duplex without the single stranded guide extensions. All single stranded extended duplexes contained phosphorothioate backbone modifications in the single stranded extension region. For duplexes DNA10PS, RNA10PS, RNA10PS-2'-OME, having 10 nucleotide single stranded guide extensions, KRAS-249 gene expression was reduced about 80-85%. For duplexes DNA10PS, RNA10PS, RNA10PS-2'-OME, having 15 nucleotide single stranded guide extensions, KRAS-249 gene expression was reduced 60-80%. Generally, the duplexes having the 10 nucleotide guide extensions reduced KRAS target gene expression more than the duplexes having the 15 nucleotide guide extensions, regardless of the nucleotides present in the 5' guide extensions. In particular, the silencing activity of duplexes having guide extensions containing deoxyribonucleotides or 2'-O-methyl ribonucleotides, was more sensitive to the increased length of 15 nucleotides, compared to the duplexes containing ribonucleotides. Processing of 5' guide strand extended duplexes by Dicer, which were used in the experiments targeting gene expression of HPRT1, was also shown by in vitro assay (FIG. 10).

Because the duplex having the single stranded guide extensions were as effective in silencing KRAS-249 and HPRT1, respectively, as a duplex without the single stranded guide extensions, this discovery allows for the modification of DsiRNA agents with single stranded guide extensions without loss of efficacy.

**Example 3 Efficacy of DsiRNA Agents Possessing Single Stranded Extensions in Combination with a Short Oligonucleotide Complementary to the Single Stranded Extension**

DsiRNA agents possessing single stranded extensions were examined for efficacy of sequence-specific target

mRNA inhibition in combination with a short oligo complementary to the single stranded extension. Specifically, KRAS-249M and HPRT-targeting DsiRNA duplexes possessing 15 nucleotide long 5' single stranded guide extensions including a 15 nucleotide discontinuous complement were transfected into HeLa cells at a fixed concentration of 20 nM and HPRT expression levels were measured 24 hours later (FIGS. 12 and 13). Transfections were performed in duplicate, and each duplicate was assayed in triplicate for KRAS-249M and HPRT expression, respectively, by qPCR.

Under these conditions (0.1 nM duplexes, Lipofectamine™ RNAiMAX transfection), KRAS-249 gene expression was reduced by about 15-60% by duplexes DNA15PS (1301+1340), RNA15PS (1301+1341), RNA15PS-2'-OME (1301+1342) in the presence of discontinuous complements RNA15, PS-RNA15, PS-DNA15, PS-2'OMe-RNA15, and 2'OMe-RNA15 (FIG. 12). A duplex without the single stranded guide extensions reduced KRAS-249 gene expression by about 85%. All single stranded extended duplexes contained phosphorothioate backbone modifications in the single stranded extension region. Generally, the duplexes having ribonucleotide or 2'-O-methyl ribonucleotide guide extensions reduced KRAS target gene expression more than the duplexes having deoxyribonucleotide guide extensions, regardless of the discontinuous complement present. For duplexes DNA15PS (1301+1340), RNA15PS (1301+1341), RNA15PS-2'-OME (1301+1342), the reductions in gene expression were comparable with or without the 2'OMe-RNA15 discontinuous complement.

Similarly, under the same conditions (0.1 nM duplexes, Lipofectamine™ RNAiMAX transfection), HPRT1 gene expression was reduced by about 30-85% by duplexes DNA15PS (1001+1353), RNA15PS (1001+1354), and RNA15PS-2'OME (1001+1355) in the presence of discontinuous complements RNA15, PS-RNA15, PS-DNA15, PS-2'OMe-RNA15, and 2'OMe-RNA15 (FIG. 13). A duplex without the single stranded guide extensions reduced HPRT1 gene expression by about 90%. All single stranded extended duplexes contained phosphorothioate backbone modifications in the single stranded extension region. Generally, the duplexes having ribonucleotide or 2'-O-methyl ribonucleotide guide extensions reduced KRAS target gene expression more than the duplexes having deoxyribonucleotide guide extensions, regardless of the discontinuous complement present. Duplexes RNA15PS (1301+1341) and RNA15PS-2'-OME (1301+1342), showed enhanced reduction in gene expression in the presence of discontinuous complements RNA15, PS-RNA15, PS-2'OMe-RNA15, 2'OMe-RNA15, compared to the same duplexes RNA15PS (1301+1341) and RNA15PS-2'-OME (1301+1342) without any discontinuous complement.

## Example 4 *In Vivo* Efficacy of DsiRNA Agents

DsiRNA agents possessing DNA duplex extensions were examined for in vivo efficacy of sequence-specific target mRNA inhibition either in a single dose protocol or in a repeated dose protocol (e.g., single 10 mg/kg injection in invivoFectamine). Expression of KRAS in liver, kidney, spleen and lymph node tissues was measured 24 hours post-injection, with real-time PCR (RT-PCR) performed in triplicate to assess KRAS expression. Under these conditions, single stranded guide extended DsiRNA agents exhibited statistically significant levels of KRAS target gene inhibition in all tissues examined. KRAS percent inhibition levels in such single stranded guide extension DsiRNA treated tissues were: liver (10%, 20%, 30%, 40%, 50%, 60%, 70%, 80%, 90%, 95%, 96%, 97%, 98%, 99%, or 100%), spleen (10%, 20%, 30%, 40%, 50%, 60%, 70%, 80%, 90%, 95%, 96%, 97%, 98%, 99%, or 100%),), kidney (1910%, 20%, 30%, 40%, 50%, 60%, 70%, 80%, 90%, 95%, 96%, 97%, 98%, 99%, or 100%),) and lymph nodes (10%, 20%, 30%, 40%, 50%, 60%, 70%, 80%, 90%, 95%, 96%, 97%, 98%, 99%, or 100%)). Thus, the in vivo efficacy of the extended DsiRNAs of the instant invention was demonstrated across many tissue types.

Further demonstration of the capability of the extended Dicer substrate agents of the invention to reduce gene expression of specific target genes in vivo was performed via administration of the DsiRNAs of the invention to mice or other mammalian subjects, either systemically (e.g., by i.v. or i.p. injection) or via direct injection of a tissue (e.g., injection of the eye, spinal cord/brain/CNS, etc.). Measurement of additional target RNA levels were performed upon target cells (e.g., RNA levels in liver and/or kidney cells were assayed following injection of mice; eye cells were assayed following ophthalmic injection of subjects; or spinal cord/brain/CNS cells were assayed following direct injection of same of subjects) by standard methods (e.g., Trizol® preparation (guanidinium thiocyanate-phenol-chloroform) followed by qRT-PCR).

In any such further in vivo experiments, an extended Dicer substrate agent of the invention (e.g., a guide 5' extended or passenger 3' extended DsiRNA) can be deemed to be an effective in vivo agent if a statistically significant reduction in RNA levels was observed when administering an extended Dicer substrate agent of the invention, as compared to an appropriate control (e.g., a vehicle alone control, a randomized duplex control, a duplex directed to a different target RNA control, etc.). Generally, if the p-value (e.g., generated via 1 tailed, unpaired T-test) assigned to such comparison was less than 0.05, an extended Dicer substrate agent (e.g., guide 5' extended or passenger 3' extended DsiRNA agent) of the invention was deemed to be an effective RNA interference agent. Alternatively, the p-value threshold below which to classify an extended Dicer substrate agent of the invention as an effective RNA interference agent can be set, e.g. at 0.01, 0.001, etc., in order to provide more stringent filtering, identify more robust differences, and/or adjust for multiple hypothesis testing, etc. Absolute activity level limits can also be set to distinguish between effective and non-effective extended Dicer substrate agents. For example, in certain embodiments, an effective extended

Dicer substrate agent of the invention was one that not only shows a statistically significant reduction of target RNA levels in vivo but also exerts, e.g., at least an approximately 10% reduction, approximately 15% reduction, at least approximately 20% reduction, approximately 25% reduction, approximately 30% reduction, etc. in target RNA levels in the tissue or cell that was examined, as compared to an appropriate control. Further in vivo efficacy testing of the extended Dicer substrate agents (e.g., guide 5' extended and passenger 3' extended DsiRNA agents) of the invention was thereby performed.

DsiRNA agents possessing single stranded extensions (FIGS. 14 and 15) effectively inhibited the sequence-specific target KRAS mRNA expression in vivo in liver, spleen, and kidney. In liver, the 5' passenger extended DsiRNA agents 1371 (PS 3M) and 1339 (PS10M) showed inhibition of KRAS mRNA expression as compared to DsiRNA agents without the 5' passenger extensions K249M and 1370 (3M), when normalized to glucose only control (FIGS. 16-18). The inhibition of KRAS mRNA expression by the DsiRNA agents was at least 75-90% in liver of animals injected with the 5' passenger extended DsiRNA agents 1371 (PS 3M) and 1339 (PS10M). The amount of inhibition of the 5' passenger extended DsiRNA agents 1371 (PS 3M) and 1339 (PS10M) in liver was comparable to that of DsiRNA agents without the 5' passenger extensions K249M and 1370 (3M), which was significant compared to the negative glucose control.

In spleen, the 5' passenger extended DsiRNA agents 1371 (PS 3M) and 1339 (PS 10M) also showed inhibition of KRAS mRNA expression as compared to DsiRNA agents without the 5' passenger extensions K249M and 1370 (3M), when normalized to glucose only control (FIGS. 19-21). The inhibition of KRAS mRNA expression by the DsiRNA agents was at least 90-95% in spleen of animals injected with the 5' passenger extended DsiRNA agents 1371 (PS 3M) and 1339 (PS10M). The amount of inhibition of the 5' passenger extended DsiRNA agents 1371 (PS 3M) and 1339 (PS10M) in spleen was comparable to that of DsiRNA agents without the 5' passenger extensions K249M and 1370 (3M), which was significant compared to the negative glucose control.

In kidney, the 5' passenger extended DsiRNA agents 1371 (PS 3M) and 1339 (PS 10M) showed inhibition of KRAS mRNA expression as compared to DsiRNA agents without the 5' passenger extensions K249M and 1370 (3M), when normalized to glucose only control (FIGS. 22-24). The inhibition of KRAS mRNA expression by the DsiRNA agents was at least 20-40% in kidney of animals injected with the 5' passenger extended DsiRNA agents 1371 (PS 3M) and 1339 (PS10M). Nevertheless, the amount of inhibition of the 5' passenger extended DsiRNA agents 1371 (PS 3M) and 1339 (PS 10M) was comparable to that of DsiRNA agents without the 5' passenger extensions K249M and 1370 (3M). In these experiments, a DsiRNA agent without the 5' passenger extension M97M and not sequence specific to KRAS was used as a positive control.

Because the DsiRNA agents having a single stranded guide extension were as effective in silencing KRAS in vivo, as DsiRNA agents without the single stranded guide extension, this discovery allows for the modification of DsiRNA agents with single stranded guide extensions without loss of efficacy in vivo.

All patents and publications mentioned in the specification are indicative of the levels of skill of those skilled in the art to which the invention pertains..

One skilled in the art would readily appreciate that the present invention is well adapted to carry out the objects and obtain the ends and advantages mentioned, as well as those inherent therein. The methods and compositions described herein as presently representative of preferred embodiments are exemplary and are not intended as limitations on the scope of the invention. Changes therein and other uses are defined by the scope of the claims.

It will be readily apparent to one skilled in the art that varying substitutions and modifications can be made to the invention disclosed herein within the scope of the following claims. The present invention teaches one skilled in the art to test various combinations and/or substitutions of chemical modifications described herein toward generating nucleic acid constructs with improved activity for mediating RNAi activity. Such improved activity can comprise improved stability, improved bioavailability, and/or improved activation of cellular responses mediating RNAi. Therefore, the specific embodiments described herein are not limiting and one skilled in the art can readily appreciate that specific combinations of the modifications described herein can be tested without undue experimentation toward identifying DsiRNA molecules with improved RNAi activity.

The invention illustratively described herein suitably can be practiced in the absence of any element or elements, limitation or limitations that are not specifically disclosed herein. Thus, for example, in each instance herein any of the terms "comprising", "consisting essentially of', and "consisting of' may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments, optional features, modification and variation of the concepts herein disclosed may be resorted to by those skilled in the art, and that such modifications and variations are considered to be within the scope of this invention as defined by the appended claims.

In addition, where features or aspects of the invention are described in terms of Markush groups or other grouping of alternatives, those skilled in the art will recognize that the invention is also thereby described in terms of any

individual member or subgroup of members of the Markush group or other group.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Embodiments of this invention are described herein, including the best mode known to the inventors for carrying out the invention. Variations of those embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Example 5. In Vitro Cell Culture Assay to Assess Nucleic Acid Inhibition of Target RNA

The dsRNAs of the invention are administered to human hepatoma (Huh7) cells and subsequently levels of targeted mRNAs are measured in the human hepatoma (Huh7) cells, to assess in vitro efficacy for reduction of target expression of the dsRNAs of the invention against the targeted transcripts.

Double stranded RNAs specific for the human target gene Hypoxanthine-Guanine Phosphoribosyl Transferase (HPRT1; GenBank Accession No. NM-000194 and GI: 164518913) are tested for efficacy in human hepatoma (Huh7) cells. The preceding target gene is an art-recognized "housekeeping" gene. Housekeeping genes are selected as target genes for the dual purpose of ensuring that the target genes possess strong and homogenous expression in human liver cells and minimizing inter-species expression level variability. Few embodiments of the invention are shown in FIGS. 37, 38, 39 and 41, including control dsRNAs to be used as a reference for comparison. Specific dsRNAs for targeting HPRT1 are shown, for example, in FIGS. 37, 38, 39, and 41. Specific sequences of dsRNAs targeting GAPDH, LMNA, HNRPA1 and ATP1B3 may be similarly constructed for targeting their respective transcript in human liver cells.

dsNA molecules targeted to RNA are designed and synthesized as described herein. These nucleic acid molecules can be tested in vivo for the ability to reduce gene expression and for Dicer cleavage activity, for example, using the following procedure. Two formats are used to test the efficacy of dsNA. The reagents are tested in cell culture using, for example, human hepatoma (Huh7) cells, to determine the extent of RNA and protein inhibition. dsNA reagents are directed to a specific target as described herein. RNA inhibition is measured after delivery of these reagents by a suitable transfection agent to, for example, cultured epidermal keratinocytes. Relative amounts of target RNA are measured and compared to versus actin using real-time PCR monitoring of amplification (eg., ABI 7700 TAQMAN). A comparison is made to a mixture of oligonucleotide sequences made to unrelated targets or to a randomized dsNA control with the same overall length and chemistry, but randomly substituted at each position. Primary and secondary lead reagents directed to a the target are optimized. After an optimal transfection agent concentration is chosen, a RNA time-course of inhibition is performed with the lead dsNA molecule. In addition, a cell-plating format can be used to determine RNA inhibition.

dsNA constructs are tested for efficacy in reducing target RNA expression, for example using the following protocol. Cells are plated approximately 24 hours before transfection in 96-well plates at 5,000-7,500 cells/well, 100 ul/well, such that at the time of transfection cells are 70-90% confluent. For transfection, annealed dsNA are mixed with the transfection reagent (Lipofectamine 2000, Invitrogen) in a volume of 50 $\mu$l/well and incubated for 20 minutes at room temperature. The dsNA transfection mixtures are added to cells to give a final dsNA concentration of 50 pM, 200 pM, or 1 nM in a volume of 150 ul. Each dsNAtransfection mixture is tested in triplicate. Cells are incubated at 37° C. for 24 hours in the continued presence of the DsiRNA transfection mixture. At 24 hours, RNA is prepared from each well of treated cells. Following centrifugation of the cell samples, the supernatants with the transfection mixtures are removed and discarded, the cells are lysed, and RNA is prepared from each well. Target RNA level or expression following treatment is evaluated by a quantitative method (e.g., RT-PCR, Northern blot) for the target gene and for a control gene (e.g., actin or 36B4, an RNA polymerase subunit) to all on for normalization. Alternatively, the cells are lysed and total protein is prepared from each well. Target protein level or expression following treatment is evaluated by Western blot and the signal is quantified. Triplicate data is averaged and the standard deviations determined for each treatment.

Normalized data are graphed and the percent reduction of target mRNA by dsRNAs of the invention in comparison to appropriate control dsRNAs (e.g., inverted control dsRNAs) is determined.

Thus it can be shown that the nicked dsRNAs of the invention for example, nicked tetraloop structures reduce gene expression of specific target in cells, in comparison to a reference dsRNA. It is expected that the nicked dsRNAs with a tetraloop have enhanced cleavage by Dicer. Thus dsRNAs of the invention having a nicked tetraloop structure reduce expression of a target gene and enhance cleavage by Dicer in comparison to a reference dsRNA. dsNAs possessing structures encompassed by the nicked tetraloop structure of the invention, are robustly effective sequence-specific inhibitors of in vitro expression of target genes in human hepatoma (Huh7) cells.

## Example 6. In Vitro Assay to Assess Serum Stability

Serum stability of dsNA agents is assessed via incubation of dsNA agents in 50% fetal bovine serum for various periods of time (up to 24 h) at 37° C. Serum is extracted and the nucleic acids are separated on a 20% non-denaturing gel using PAGE and visualized with Gelstar stain. Relative levels of protection from nuclease degradation are assessed for dsNA (optionally with and without modifications).

## Example 7. In Vivo Assay of Nicked dsRNA with Tetraloop

The invention provides compositions for reducing expression of a target gene in a cell, involving contacting a cell with nicked dsRNA having a tetraloop in an amount effective to reduce expression of a target gene in a subject in need thereof. The dsRNAs of the invention are systemically administered to mice and subsequently levels of targeted mRNAs are measured in liver samples of treated mice. The study assesses in vivo efficacy of the dsRNAs of the invention against the targeted transcripts.

Double stranded RNA agents specific for the following mouse housekeeping target genes are tested for efficacy in mouse liver: Hypoxanthine-Guanine Phosphoribosyl Transferase (HPRT1; GenBank Accession No. NM-013556); Glyceraldehyde 3-Phosphate Dehydrogenase (GAPDH; GenBank Accession No. NM-008084); Lamin A (LMNA; GenBank Accession No. NM-019390); Heterogeneous Nuclear Ribonucleoprotein A1 (HNRPA1; GenBank Accession No. NM-010447) and ATPase, Na+/K+ Transporting, Beta 3 Polypeptide (ATP1B3; GenBank Accession No. NM-007502; two distinct locations were targeted within the ATP1B3 mRNA).

Specific sequences of dsRNAs targeting HPRT1, GAPDH, LMNA, HNRPA1 and ATP1B3 having a structure of the dsRNAs of the invention, for example a nicked, tetraloop structure containing any one of the following sequences on the antisense strand for targeting their respective transcripts are constructed:

HPRT1 antisense sequence: 3'-UUCGGUCUGAAACAACCUAAACUUUAA-5'

GAPDH antisense sequence: 3'-ACUCGUAGAGGGAGUGUUAAAGGUAGG-5'

LMNA antisense sequence:
3'-CUCGAACUGAAGGUCUUCUUGUAAAUG-5'

HNRPA1 antisense sequence:
3'-GUCCUGACAUAAACACUGAUUAACAUA-5'

ATP1B3 antisense sequence:
3'-AUCCCUAUGUUACCAUGGAACGGUUGU-5'

ATP1B3 antisense sequence:
3'-GGUCUGCCUAUAGGUGUUUAUAGCACA-5'

Legend: Upper Case = RNA residues

Mice (CD-1 females) weighing approximately 25 grams are purchased, housed, treated and sacrificed.

An initial dose-ranging and time point selection study performed to establish in vivo efficacy of the nicked dsRNAs for reducing expression of targeted transcripts while also establishing the optimal nicked dsRNA dose and sample collection time. This is done, for example, for two independent, active sequences targeting (HPRT1). Different doses (50 and 200 $\mu$g) of the dsRNAs to be tested are dissolved in phosphate-buffered saline (PBS; 2.5 mL total volume per dose) and administered to mice as single hydrodynamic injections through the tail vein. Liver samples are collected from dosed mice at the following time points: 24, 48 and 72 hours, and 7 days after administration. A total of four animals

per group are treated with the dsRNAs in order to assure that at least 3 animals can be evaluated at each dosage/time point.

The study is also performed using the following conditions. A dose (200 μg) of the dsRNA to be tested is dissolved in phosphate-buffered saline (PBS; 2.5 mL total volume per dose) and administered to mice as single hydrodynamic injections through the tail vein. Liver samples are collected from dosed mice at 24 hours after administration. A total of seven animals per group are treated with each dsRNA agent.

Target mRNA levels are assessed using quantitative reverse transcriptase-polymerase chain reaction ("qRT-PCR"). cDNAs are synthesized using a mix of oligo-dT and random hexamer priming. qPCR reactions are run in triplicate. Absolute quantification is performed by extrapolation against a standard curve run derived from a cloned linearized amplicon target. Data are normalized using the control as 100%. Data are normalized by setting the control gene expression level to be the measured target mRNA expression value for all mice not administered target mRNA-specific DsiRNA agents, which were averaged to obtain a 100% control value (e.g., for mice injected with GAPDH DsiRNAs, the set of HPRT1, LMNA, HNRPA1, ATP1B3-1 and ATP1B3-3 mice are all used as negative controls to yield normalized, basal GAPDH levels. Thus, there are seven study mice and 35 control mice for each arm of the study). In evaluating the significance of the results, P values are calculated using a 1 tailed, unpaired T-Test. Values below 0.05 are deemed to be statistically significant.

Reduced levels of targeted mRNAs are observed in liver samples of treated mice due to treatment with the nicked tetraloop dsRNAs of the invention. Results of the studies show that dsRNA agents directed against HPRT1 target sequences reduce target mRNA levels in vivo when administered at 50 microgram and 200 microgram or other concentrations, for example between 1μg and 500μg, for example 1, 2, 3, 4, 5, 10, 20, 30, 40, 50, 60, 70, 80, 90, 100, 200, 300, 400, 500 μg with reductions in target gene transcript expression levels of at least 10, 20, 30, 40, 50, 60, 70, 80, 90, or 100% observed at 24 hours post-administration when compared to a control that is not expected to reduce gene transcript levels.

## Example 8.Synthesis of Ligand Derivatives

Derivatives of the ligand N-acetyl galactosamine, referred to as GalNac in this example was synthesized according to scheme I: (Refer page 210)

**Scheme I**

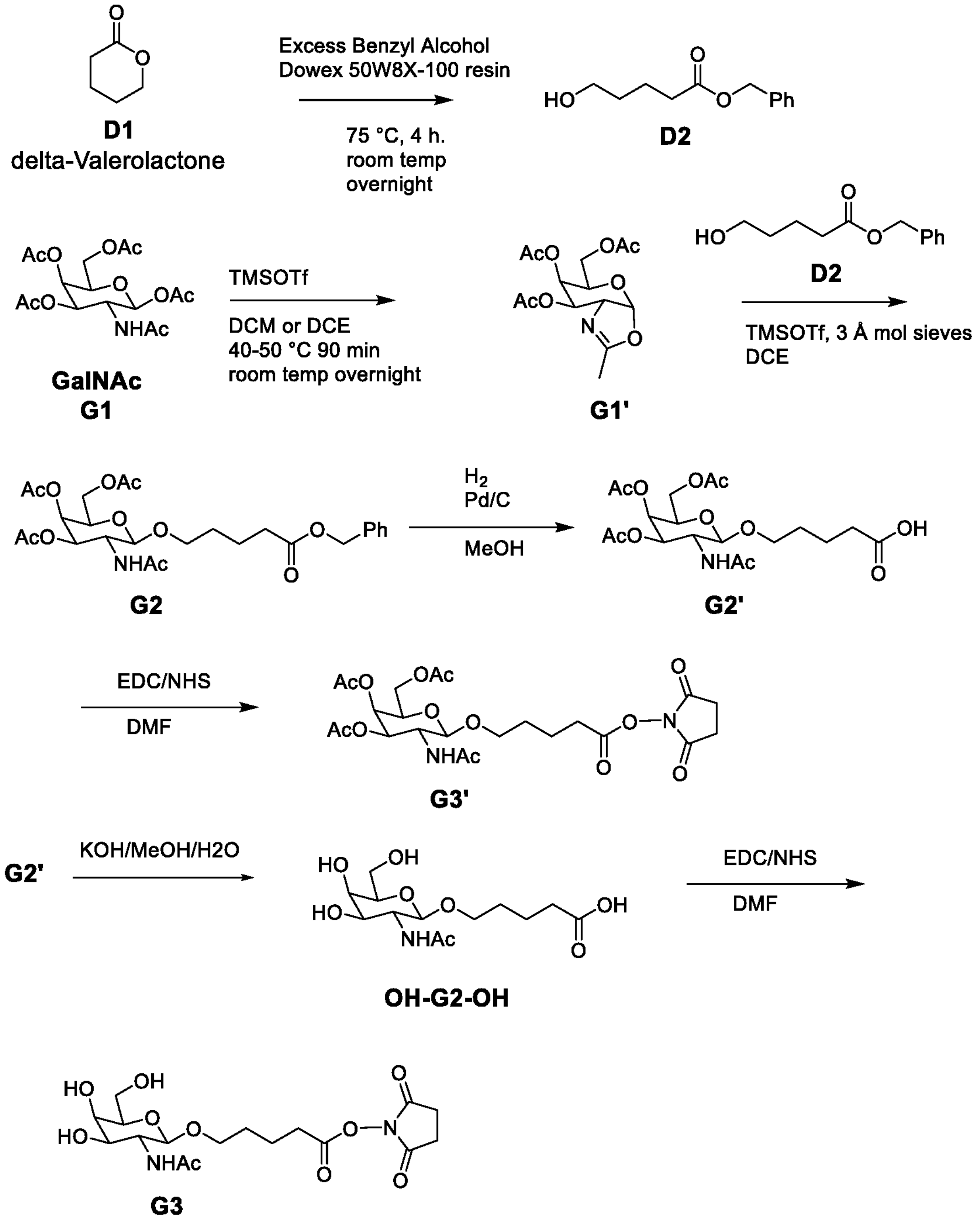


## a. Synthesis of D2

A flask was charged with a stir-bar, benzyl alcohol (90 mL,94 g, 0.87 mol, 8.0 equivalents, Aldrich anhydrous), delta-valerolactone (10.0 mL, 10.8 g, 0.108 mol,1.0 equivalents, Alfa, used as received), and Dowex 50W8X-100 resin (207 mg, Aldrich). The flask was fitted with a rubber septum with a $N_2$ (Nitrogen) inlet needle, and the flask was placed in an oil bath pre-heated to 75 °C. The mixture was stirred vigorously for 4 h, and then the power to the oil bath was turned off, and the solution was allowed to stir overnight, cooling to room temperature under N2 (Nitrogen). The clear, colorless solution was filtered to remove the DOWEX beads, and the beads and filter were rinsed with DCM . The filtrate was concentrated on the rotavap to remove the (Dichloromethane) DCM. The mixture was purified by flash chromatography (FC) on silica gel (ISCO) in 3 batches, each on a 330 g silica gel column (the same column was re-used for all 3 batches). 330-g column, equilibrated with 5% Ethyl Acetate (EtOAc)/Hexanes. 33-35 mL of the reaction solution was injected onto the column for each run and eluted with EtOAc/Hexanes 5%-100%. UV 254 nm, 280 nm. All of the desired fractions from the 3 columns were combined and evaporated to give a D2 as a colorless liquid: 15.82 g (0.076 mol,

70%). This was repurified by FC on silica gel: 220-g column, elute with EtOAc/Hexanes 5%-70%, 254 nm, 280 nm. The desired fractions were combined and evaporated to give a colorless liquid which was dried under full vacuum for 24 hours to give D2 12.60 g (56%) as a colorless liquid pure enough to use in the next step.

**b. Synthesis of G1'**

4-g Scale:

An oven-dried flask was charged with the GalNAc (G1) (4.360 g, 11.20 mmol, 1.00 equivalents) and a stir bar. 1,2-Dichloroethane (Aldrich . anhydrous, 26 mL) was added to give a milky suspension. To this mixture was added Trimethylsilyl trifluoromethanesulfonate (TMSOTf) (Alfa, 2.8 mL over 1 minute) via syringe at room temperature under $N_2$. This mixture was stirred at room temperature for 30 minutes, but remained heterogeneous. The flask was placed in an oil-bath pre-heated to 50 °C. Within a few minutes the reaction mixture became homogeneous. After heating for 95 minutes, the power to the oil bath was turned off, and the flask was allowed to cool to room temperature overnight. After 21 hours an aliquot was removed, diluted with $CH_3CN$, and checked by Mass spectroscopy (MS) CIPOS: 362.1 (10), 330.1 (100), 210.1 (20), 168.1 (12), 150.1 (28).

The reaction solution (amber color) was poured into a 500-mL separatory funnel containing 130 mL of ice-cold saturated aqueous $NaHCO_3$, and the reaction flask was rinsed with DCM (100 mL). After gas evolution had subsided, the phases were separated (the lower organic phase was cloudy yellow, and the upper aqueous phase was milky with a bit of color). The aqueous phase was extracted with DCM (1 x 50 mL). The combined DCM phases were washed with $H_2O$ (120 mL) and saturated aqueous NaCl (120 mL). The DCM phase (clear, light yellow) was dried over $Na_2SO_4$, filtered ($Na_2SO_4$ rinsed with about 100 mL of DCM), and concentrated by rotavap to give an amber oil. This was dried at room temperature under full vacuum overnight (about 3.7 g), and then stored in the freezer. This material was used without further purification in the next step.

20-g Scale:

To a mixture of Galactosamine pentaacetate (G1) (20.0 g, 51.0 mmol, 1.0 equivalents, LC Scientific) in dichloromethane (120 mL) was added TMSOTf (14.0 mL, 77.0 mmol, 1.5 equivalents, Alfa) at room temperature, and the reaction was heated to reflux and stirred for 90 minutes at reflux. Subsequently the mixture was stirred at room temperature overnight. The reaction mixture was poured onto an ice cold sodium bicarbonate solution, extracted with dichloromethane, washed with water, and dried over sodium sulfate and filtered. After concentration on the rotavap, the expected product G1' was obtained as dark orange gum (about 16 g). The crude product was used without further purification.

**c. Synthesis of G2**

The 5-hydroxpentanoic acid benzyl ester D2 (12.44 g, 59.73 mmol, 1.75 equivalents) was dissolved in 1,2-dichloroethane (80 mL, Aldrich anhydrous), and this solution was added to the crude GalNAc derivative (G1') (11.24 g, 34.13 mmol, 1.00 equivalents). Once this solution was homogeneous, to it was added dried 3Å molecular sieves (10.5 g, beads). The mixture was stirred at room temperature under N2 for 30 minutes. TMSOTf (3.2 mL, 18 mmol, 0.52 equivalents) was added via syringe. The mixture was stirred at room temperature under N2 overnight. The reaction was monitored by MS (CI POS), and TLC (silica gel, 100% EtOAc, stained with PMA or $H_2SO_4$/MeOH). MS POS: 638.3 (15), 538.2 (100), 438.2 (10), 330.1 (40). After 24 hours at room temperature, the reaction was worked up. The amber solution was poured into a separatory funnel containing 100 mL of saturated aqueous $NaHCO_3$, and the flask and sieves were rinsed with 80 mL of DCM, which was also added to the separatory funnel. The phases were separated, and the aqueous phase was extracted with1 x 80 mL DCM. The combined organic phases were washed with $H_2O$ (100 mL) and saturated aqueous NaCl (100 mL). The amber organic phase was dried ($Na_2SO_4$), filtered (paper), and evaporated to give a viscous, amber liquid: 26.54 g (145%).The crude sample was purified directly by FC on silica gel (ISCO): 330-g column, compound injected onto the equilibrated column and rinsed with 8 mL DCM; eluted with 0% - 100% EtOAc/Hexanes, UV 254 nm, 280 nm. The desired fractions were combined and evaporated to give an almost colorless, viscous liquid: 13.52 g (74%).This material was contaminated by a small amount of GalNAc-OBn (M+1 = 438) and minor amounts of "dimer" and "trimer" (homologs of pentanoic acid) derivatives (M+1 = 738 and 638).

**d. Synthesis of G2'**

The starting material G2 (2.42 g, 4.50 mmol, 1.0 equivalents) was dissolved in MeOH (60 mL), and the solution placed in a Parr jar. 5% Pd/C (592 mg, Aldrich, Degussa E1002 U/W, wet) was added. The jar was placed on the Parr hydrogenator, and the jar was evacuated and back-filled with H2 four times. The jar was then pressurized to 48 psi

(Hydrogen) $H_2$, and shaken at room temperature overnight. After 18 hours, the reaction was stopped. The jar was evacuated and back-filled with N2 four times. An aliquot of the reaction mixture was removed, filtered, diluted with MeOH, and checked by MS. MS POS: 448.2 (100), 330.1 (65), 210.1 (7), 150.1 (8); MS NEG: 560.2 (5), 446.2 (100).The reaction was complete and clean. The reaction mixture was filtered through Celite and rinsed with MeOH (100 mL). The clear, colorless filtrate was evaporated and dried under full vacuum at room temperature to give a colorless foam/glass solid: 1.70 g (84 %) of G2'.

**e. Synthesis of G3**

To a mixture of G2' (2 g, 4.47 mmol) in dichloromethane (100 mL) was added EDC (3.08 g, 16.06 mmol) and NHS (0.77 g, 6.71 mmol), and the reaction mixture was stirred at room temperature overnight. The reaction mixture was concentrated on the rotavap (40 °C), and the residue was washed with saturated. Aqueous sodium bicarbonate (200 mL) and extracted with EtOAc (2 X 500 mL). The combined organic solution was dried over anhydrous. $Na_2SO_4$ and evaporated. The crude G3 was used in the next step without purification (1.4 g, 57%).

**$^1$H NMR** (300 MHz, DMSO-$d_6$)) δ 7.83 (d, J = 9.36 Hz, 1H), 5.21 (d, J = 3.27 Hz, 1H), 4.95 (dd, J = 11.28, 3.3 Hz, 1H), 4.5 (d, J = 8.49 Hz, 1H), 3.99 - 4.06 (m, 3H), 3.84 - 3.94 (m, 1H), 3.70 - 3.77 (m, 1H), 3.43 - 3.49 (m, 1H), 2.80 (s, 4H), 2.67 (t, J = 7.41 Hz, 2H), 2.1 (s, 3H), 1.99 (s, 3H), 1.89 (s, 3H), 1.76 (s, 3H), 1.57 - 1.62 (m, 4H).

**$^{13}$C NMR** (75 MHz, DMSO-$d_6$)

δ 170.81, 170.58, 170.50, 170.21, 169.86, 169.49, 101.41, 71.04, 70.41, 68.54, 67.26, 62.01, 49.8 1, 30.31, 28.34, 25.99, 25.78, 23.28, 21.44, 21.08, 21.02, 21.01

**MS:** (APCI$^+$) M +1 = 545.2

**Example 9. Synthesis of Multivalent Ligand derivatives**

An example of such is the triantennary GalNac and the synthesis of these branched GalNac derivatives is shown in Scheme II. (Refer pages 211-213).

**a. Synthesis of T5 (3-[2-(benzyloxycarbonylamino)-3-(2-carboxyethoxy)-2-(2-carboxyethoxymethyl)-propoxy]propanoic acid)**

A flask was charged with 3,3'-((2-(((benzyloxy)carbonyl)amino)-2-((2-carboxyethoxy)methyl)propane-1,3-diyl)bis(oxy))dipropionic acid (T4) (4.39 g, 9.31 mmol, 1.0 equivalent) (CAS Registry Number: 200133-16-0) and DMF (44 mL) to give a homogeneous solution. HOBt·H2O (1.59 g, 9.30 mmol, 1.0 equivalents) was added at room temperature under N2, and the mixture was stirred about 1 minute until homogeneous. DIEA (9.5 mL, 54.5 mmol, 5.9 equivalents) was added via syringe. HBTU (11.70 g, 30.9 mmol, 3.3 equivalents) was added all at once. The reaction mixture was stirred 1-2 minutes until homogeneous. The flask was immersed in an ice-water bath, and BOC-1,3-diaminopropane (5.80 mL, 33.2 mmol, 3.6 equivalents) was added slowly via syringe over about 5 minutes. The bath was left in place, and the reaction solution (clear, faintly yellow-amber) was stirred under $N_2$ for 24 h, the reaction solution was diluted with CH2Cl2 (350 mL) and washed with H2O (150 mL), saturated aqueous $NaHCO_3$ (2 x 150 mL), H2O (150 mL), and saturated aqueous NaCl (150 mL), dried over $Na_2SO_4$, filtered, and evaporated to give a viscous, amber liquid. The crude material was purified by FC on SiO2 (ISCO). Eluted with MeOH/EtOAc 0% to 20%, then 10% MeOH/$CH_2Cl_2$ to provide an almost colorless, sticky foam/oil: 7.17 g (82%) T5.

**$^1$H NMR** (300 MHz, $CDCl_3$): δ 7.34-7.28 (m, 5H), 6.85 (m, 3H), 5.55 (s, 1H), 5.18 (m, 3H), 5.01 (s, 2H), 3.68-3.63 (m, 12H), 3.28-3.21 (m, 6H), 3.13-3.07 (m, 6H), 2.39 (t, J = 5.5 Hz, 6H), 1.58 (br q, J = 6.0 Hz, 6H), 1.41 (s, 27H)

MS (APCI$^+$) M +1= 940.5

CAS Registry Number: 1162069-31-9

**b. Synthesis of T8 di-tert-Butyl (10-amino-10-(13,13-dimethyl-5,11-dioxo-2,12-dioxa-6,10-diazatetradecyl)-5,15-dioxo-8,12-dioxa-4,16-diazanonadecane-1,19-diyl)dicarbamate**

T5 (2.84 g, 3.02 mmol) was dissolved in MeOH (50 mL) at room temperature, and the solution was placed in a Parr jar. 5% Pd/C (0.6670 g, Degussa E1002 U/W, wet, Aldrich) was added. The jar was placed on a Parr hydrogenator, and evacuated and back-filled with H2 four times. The jar was pressurized to 47 psi H2 and shaken at room temperature for 6 h. The jar was evacuated and back-filled with N2 four times. The reaction mixture was filtered through Celite, and the Celite was rinsed with about 200 mL MeOH. The slightly cloudy filtrate was filtered through filter paper with some $CH_2Cl_2$ to rinse, and the resulting clear filtrate was evaporated and dried under full vacuum to give a foam solid: 2.38 g (98%) T8.

**$^1$H** NMR (300 MHz, DMSO-d6): δ 7.85 (t, J = 5.5 Hz, 3H), 6.76 (t, J = 5.7 Hz, 3H), 3.55 (t, J = 6.4 Hz, 6H), 3.16 (s, 6H),

3.02 (dt, J = 6.5 Hz, 6H), 2.90 (q, J = 6.5 Hz, 6H), 2.26 (t, J = 6.1 Hz, 6H), 1.48 (quint, J = 6.9 Hz, 6H), 1.36 (s, 27 H). **MS** ($APCI^+$) M +1= 806.5

**c. Synthesis of T9 Methyl 15,15-bis(13,13-dimethyl-5,11-dioxo-2,12-dioxa-6,10-diazatetradecyl)-2,2-dimethyl-4,10,17-trioxo-3,13,20,23,26,29,32-heptaoxa-5,9,16-triazapentatriacontan-35-oate (T9):**

A flask was charged with 3-oxo-2, 6, 9, 12, 15, 18-hexaoxahenicosan-21-oic acid (532.0 mg, 4.06 mmol, 1.0 equivalents, BroadPharm), T8 (3.385 g, 4.12 mmol. 1.0 equivalents), HBTU (1.888 g, 4.98 mmol, 1.2 equivalents), HOBt·$H_2O$ (710.3 mg, 4.15 mmol, 1.0 equivalents), and DMF (15 mL). The mixture was stirred a few minutes under N2 until clear and homogeneous. To this was added DIEA (1.6 mL, 9.19 mmol, 2.3 equivalents) via syringe over 1 minute. The reaction solution was stirred under N2 at room temperature for 41 h. The solution was diluted with $CH_2Cl_2$ (180 mL) and washed with 1:1 $H_2O$/saturated aqueous NaHCO3 (2 x 70 mL), saturated aqueous $NaHCO_3$ (70 mL), and saturated aqueous NaCl (80 mL). The combined aqueous phases were extracted with $CH_2Cl_2$ (80 mL), and this $CH_2Cl_2$ phase was washed with $H_2O$ (50 mL) and saturated aqueous NaCl (50 mL). The combined $CH_2Cl_2$ phases were dried over $Na_2SO_4$, filtered, and evaporated to give an oil. The crude oil was purified by FC on $SiO_2$ (ISCO), eluting with (0.7M NH3 in MeOH)/$CH_2Cl_2$, 0%-10%, to give 1.604 g (35%) T9 as an oil.
**$^1$H NMR** (300 MHz, DMSO-d6): δ 7.81 (br t, J = 5.7 Hz, 3H), 7.11 (s, 1H), 6.76 (br t, J = 5.5 Hz, 3H), 3.61 (t, J = 6.4 Hz, 2H), 3.59 (s, 3H), 3.56-3.47 (m, 32H), 3.02 (br q, J = 6.5 Hz, 6H), 2.90 (br q, J = 6.5 Hz, 6H), 2.53 (t, J = 6.3 Hz, 2H), 2.26 (br t, J = 6.3 Hz, 6H), 1.48 (br quint, J = 6.9 Hz, 6H), 1.36 (s, 27 H).
MS ($APCI^+$) M +1= 1140.7

**d. Synthesis of T10 (Methyl 30-amino-21,21-bis((3-((3-aminopropyl)amino)-3-oxopropoxy)methyl)-19,26-dioxo-4,7,10,13,16,23-hexaoxa-20,27-diazatriacontanoate tris(2,2,2-trifluoroacetic acid) salt (T10):**

In a 100-mL round bottom flask, a mixture of T9 (1.5 g, 1.315 mmol), TFA (6 mL) and dichloromethane (24 mL) was stirred at room temperature for 1 h. The reaction mixture was concentrated by rotavap, and the crude product was dried under vacuum overnight. There was no purification needed, and crude T10 (1.55 g, yield: quant.) was used in the next step directly.
**$^1$H NMR** (300 MHz, DMSO-$d_6$) δ 8.08 (t, J = 5.79 Hz, 3H), 7.72 (br, 9H), 7.14 (s, 1H), 3.61 (t, J = 6.03 Hz, 2H), 3.58 (m, 3H), 3.46 - 3.56 (m, 30H), 3.07 - 3.15 (m, 6H), 2.74 - 2.80 (m, 6H), 2.53 (t, J = 6.33 Hz, 2H), 2.28 - 2.34 (m, 8H), 1.62 - 1.71 (m, 6H).
**$^{13}$C NMR** (75 MHz, DMSO-$d_6$)
δ 172.15, 171.31, 171.00, 70.28, 70.19, 70.01, 68.8, 67.84, 67.37, 66.49, 60.19, 51.83, 37.26, 37.1 1, 36.46, 36.09, 34.95, 27.95
**MS:** ($APCI^+$) M +1 = 840.5

**e. Synthesis of 3GT2-Methyl ester:**

To a solution of T10 (1.55 g, 1.315 mmol) in dichloromethane (45 mL) at 0 °C (ice-water bath) was added DIPEA (2.29 mL, 13.15 mmol), and the mixture was stirred for 5 minutes. G3 was added, and the reaction mixture was stirred at room temperature overnight. The reaction mixture was washed with saturated aqueous sodium bicarbonate (200 mL), and the aqueous phase was extracted with dichloromethane (500 mL). The combined organic phases were washed with brine, dried over anhydrous Na2SO4 and evaporated. The residue was purified on flash silica gel column chromatography (gradient elution from 0% to 40% MeOH/CH2Cl2). 3GT2-Methyl ester was obtained as white foam (1.4 g, 51%)
**$^1$H NMR** (300 MHz, DMSO-$d_6$) δ 7.81 - 7.85 (m, 6H), 7.74 (t, J = 5.19 Hz, 3H), 7.13 (s, 1H), 5.21 (d, J = 3.3 Hz, 3H), 4.95 (dd, J = 11.25, 3.57 Hz, 3H), 4.47 (d, J = 8.25 Hz, 3H), 4.02 - 4.05 (m, 12H), 3.81- 3.91 (m, 6H), 3.38 - 3.73 (m, 83H), 2.53 (t, J = 6.33 Hz, 2H), 2.32 (t, J = 6.87 Hz, 2H), 2.27 (t, J = 6.33 Hz, 6H), 2.1 (s, 9H), 2.04 (t, J = 7.17 Hz, 6H), 1.99 (s, 9H), 1.88 (s, 9H), 1.76 (s, 9H), 1.45 - 1.54 (m, 18H).
**$^{13}$C NMR** (75 MHz, DMSO-$d_6$)
δ 172.51, 170.65, 170.58, 170.5, 170.21, 169.94, 101.53, 71.02, 70.32, 70.21, 70.03, 69.22, 68.78, 67.90, 67.24, 66.52, 61.98, 51.87, 49.90, 36.92, 36.83, 36.57, 35.59, 34.98, 29.89, 29.13, 23.31, 2 2.39,21.01

**f. Synthesis of 3GT3:**

3GT2-Methyl ester (1.4 g, 0.66 mmol) was dissolved in a solution of MeOH (30 mL) and H2O (10 mL), LiOH monohydate (1.1 g, 26.22 mmol) was added, and the reaction mixture was stirred at room temperature for 4 h. The reaction mixture was concentrated on the rotavap, then diluted with H2O (10 mL). The mixture was acidified with 1 N

HCl, til pH = 3 ~ 4. The mixture was loaded onto a C18 (80 g) column and purified by reverse phase chromatography (ISCO) (gradient elution from 0% to 60% CH3CN/H2O). After lyophilization, 3GT3 was obtained as colorless solid (634 mg, 55%).

**$^1$H NMR** (300 MHz, DMSO-$d_6$) δ 7.86 (t, J = 5.76 Hz, 3H), 7.74 (t, J = 5.76 Hz, 3H), 7.62 (d, J = 8.79 Hz, 3H), 7.15 (s, 1H), 4.48 - 4.63 (m, 9H, OH), 4.20 (d, J = 8.25 Hz, 3H), 3.47 - 3.73 (m, 52H), 3.26 - 3.37 (m, 4H), 2.95 - 3.10 (m, 12H), 2.42 (t, J = 6.33 Hz, 2H), 2.32 (t, J = 6.33 Hz, 2H), 2.27 (t, J = 6.33 Hz, 6H), 2.04 (t, J = 6.87 Hz, 6H), 1.79 (s, 9H), 1.40 - 1.51 (m, 18H).

**$^{13}$C NMR** (75 MHz, DMSO-$d_6$)

δ 173.33, 172.62, 170.91, 170.67, 170.09, 101.94, 75.82, 72.08, 70.31, 70.03, 68.77, 68.45, 68.08, 67.90, 67.41, 66.93, 61.03, 60.17, 52.61, 37.11, 36.93, 36.83, 36.57, 35.65, 35.52, 29.88, 29.19, 2 3.60, 22.52

**g. Synthesis of Tri-antennary GalNAc Ligand (3GT5S):**

To a mixture of 3GT3 (36 mg, 0.021 mmol) in DMF (3 mL) was added EDC (5.96 mg, 0.031 mmol) and NHS (6.99 mg, 0.061 mmol), and the reaction mixture was stirred at room temperature overnight. The reaction mixture was concentrated on the rotavap (40 °C), and the residue was loaded onto a C18 (13 g) column and purified by reverse phase chromatography (ISCO) (gradient elution from H2O (0.1%TFA) to 60% CH3CN/H2O (0.1% TFA)). After lyophilization, 3GT5S was obtained as an off-white solid (21 mg, 55%).

**$^1$H NMR** (300 MHz, DMSO-$d_6$) δ 7.85 (t, J = 5.76 Hz, 3H), 7.74 (t, J = 5.76 Hz, 3H), 7.62 (d, J = 8.79 Hz, 3H), 7.14 (s, 1H), 4.55 - 4.63 (m, 6H, OH), 4.48 (d, J = 3.84 Hz, 3H, OH), 4.20 (d, J = 7.95 Hz, 3H), 3.47 - 3.73 (m, 52H), 3.26 - 3.37 (m, 4H), 2.95 - 3.10 (m, 12H), 2.91 (t, J = 6.03 Hz, 2H), 2.80 (s, 4H), 2.32 (t, J = 6.33 Hz, 2H), 2.27 (t, J = 6.57 Hz, 6H), 2.03 (t, J = 7.29 Hz, 6H), 1.78 (s, 9H), 1.40 - 1.51 (m, 18H).

**Example** 10. **Synthesis of N-Acetyl Galactosamine (GalNAc) conjugated phosphoroamidite monomer with the GalNAc linked through nucleobase**

The scheme III below shows the steps involved in the synthesis of GalNac conjugated monomers (D6). D3 was obtained commercially and is a modified thymidine having the linker attached at to the methyl group at C5.

**Scheme III**

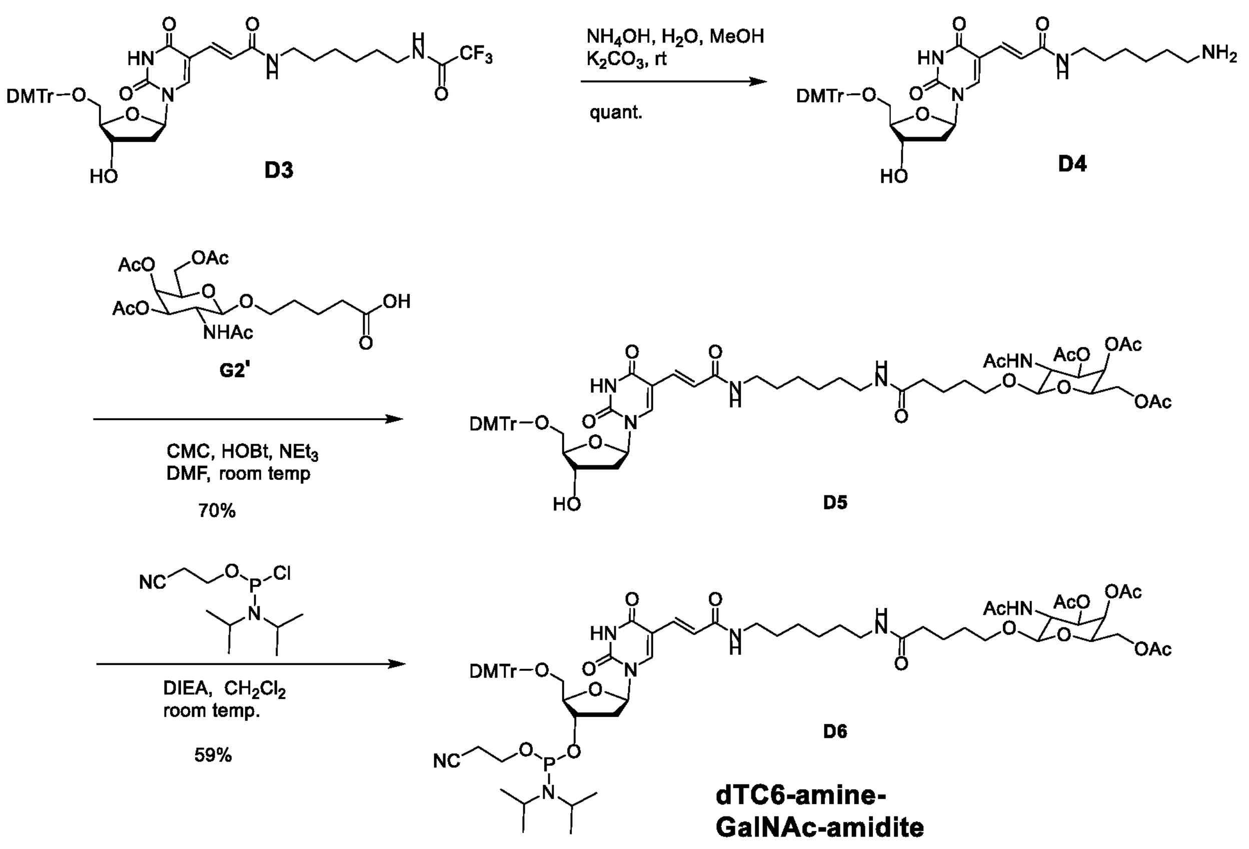


D3

D4

G2'

D5

D6

**dTC6-amine-GalNAc-amidite**

**a. Synthesis of** D4

To a solution of (E)-3-(1-((2R,4S,5R)-5-((bis(4-methoxyphenyl)(phenyl)methoxy)methyl)-4-hydroxytetrahydrofuran-2-yl)-2,4-dioxo-1,2,3,4-tetrahydropyrimidin-5-yl)-N-(6-(2,2,2-trifluoroacetamido)hexyl)acrylamide (D3) (4.55 g, 5.73 mmol, 1 equivalents, Glen Research) in methanol (24 mL) was added 36 mL of concentrated ammonium hydroxide solution (Fisher) over 5-10 minutes. The solution was stirred at room temperature and monitored by MS and TLC (silica gel, elute with either 100% EtOAc or 10% (7M NH3 in MeOH)/DCM, and visualize with UV or stain with either Phosphomolybdic acid (PMA) or H2SO4/MeOH). After 24 hours there was still a small amount of SM remaining. K2CO3 (222 mg, 1.61 mmol, 0.28 equivalents) was added. The reaction mixture was stirred another 15 hours at which time more K2CO3 (103 mg, 0.748 mmol, 0.13 equivalents) was added. The solution was stirred for 3 hours at room temperature. The volatiles were evaporated on a rotavap (bath temperature <33 °C). MS CI POS:795 (1), 753.3 (5), 699.3 (100), 303.1 (20) . The residue was dissolved in about 10% MeOH/DCM (about 7 mL) with sonication, and this solution was deposited on an equilibrated ISCO column: 80-g column (ISCO Gold), eluted with (7M NH3 in MeOH)/DCM 0.75% to 15%. UV 280 nm, 254 nm. The desired fractions were combined and evaporated to give a foam solid that was crushed to an off-white powder which was dried under full vacuum at room temperature for several days to give D4: 4.17 g (quantitatively.)

**b .Synthesis of D5**

5-(((2R, 3R, 4R, 5R, 6R)-3-acetamido-4, 5-diacetoxy-6-(acetoxymethyl)tetrahydro-2H-pyran-2-yl)oxy)pentanoic acid (G2') (1.60 g, 3.58 mmol, 1.20 equivalents) was dissolved in 12 mL of dry DMF at room temperature (with brief sonication). To this solution was added, in this order: triethylamine (0.60 mL, 1.44 equivalents, Aldrich anhydrous), Hydroxybenzotriazole hydrate (HOBt hydrate), (0.459 g, 1.00 equivalents, CHEM-IMPEX), CMC (1.65 g, 1.30 equivalents, Aldrich), and the amine SM (D4) (2.09 g, 2.99 mmol, 1.0 equivalents). 12 mL of dry DMF was added to rinse down the flask. The solution was sonicated briefly to dissolve the last bit of SMamine (D4). The clear solution was stirred under N2 at room temperature overnight, and monitored by TLC (silica gel, elute with either 100% EtOAc or 10% (7M NH3 in MeOH)/DCM, and visualize with UV or stain with either PMA or H2SO4/MeOH). After 19 hours, the reaction solution was concentrated on the rotavap to remove almost all the DMF (water bath temperature < 39 °C) to give a slurry. This was dissolved in MeOH (few mL) and DCM (6 mL) and deposited on an equilibrated ISCO column and purified by flash chromatography: 120 g silica gel, 0.5%-14% (2.3M NH3 in MeOH) in DCM. UV 214 nm, 254 nm. The desired fractions were combined and evaporated to give D5: 2.36 g (70%).

**c .Synthesis of D6**

D5 (2.24 g, 1.99 mmol, 1 equivalents) was dissolved in dichloromethane (30 mL, Aldrich. anhydrous) at room temperature under N2. Diisopropylethylamine (1.40 mL, 8.18 mmol, 4.12 equivalents, Aldrich. anhydrous) was added. The solution was stirred a few minutes, then 2-cyanoethyl N,N-diisopropylchlorophosphoramidite (0.90 mL, 4.0 mmol, 2.0 equivalents, Toronto Research) was added via syringe, dropwise over about 1 minute. The resulting clear, almost colorless solution was stirred at room temperature and monitored by TLC: 10% (7M NH3 in MeOH)/DCM or 10% MeOH/DCM; UV, PMA or H2SO4/MeOH. Very little D5 remained after 3 hours. (D6 has higher Rf than D5.) After 4.5 hours, the reaction solution was quenched with 1 mL MeOH and stirred for 5 minutes. It was then concentrated on to about 5 mL volume and deposited on an equilibrated column, and purified by FC on silica gel (ISCO): 120 g column, elute with 2% to 60% [20% (2.33M NH3 in MeOH)]/DCM. The desired fractions were combined and evaporated to give an almost-colorless, glassy foam-solid which was dried under full vacuum at room temperature to give 1.56 g (59%) of D6.

**Example 11.Synthesis of dsNAs comprising a stem-loop with four N-acetyl-galactosamine (GalNac) ligands in the loop**

Stem-loop oligomers wherein the GalNac ligand is conjugated to a monomer in the loop were produced according to Scheme A. (Refer pages 225-226)

**a .Synthesis of Sense Strand A2**

In a 15mL Falcon tube, Compound A1 (30mg, 0.00252mmol) was dissolved in degassed 3:1 dimethylacetamide/deionized water (750μL). In 2mL scintillation vial Azido-Peg11-amine (Quanta BioDesign, order #10524, 46mg, (0.00806 mmol) was dissolved in degassed 3:1 dimethylacetamide/deionized water (150μl). In a 2mL scintillation vial, copper(I) bromide dimethyl sulfide complex 16.6mg, 0.0806mmol) in was dissolved in degassed acetonitrile (300μL).

The peg-azide solution was added to the RNA solution followed by the addition of the CuBr as a solution/slurry.

The reaction solution turned green-blue in color upon addition of the CuBr reagent. The reaction mixture was heated at 40°C for 1 hour in a shaker and the reaction was monitored by LC-MS (2-40% Water/ACN with 100mM HFIP and 8.3mM triethylaminegradient over 2 minutes, BEH C18 2.1x50mm).

The desired product was observed by LC-MS. The reaction was quenched with 0.5M Ethylenediaminetetraacetic acid, pH 8 (5 ml).This reaction was placed in the shaker for 15 minutes and then dialyzed against water (1x) using a 15mL Millipore 10K membrane (4200rpm, 15minutes, 4°C).

The reaction mixture was purified by ion-pairing chromatography (5-25% Water/Acetonitrile containing 100mM triethylammonium acetate over 35 minutes, XBridge C18 19x150mm). The product fractions were pooled and dialyzed against water 3x using a 15mL Millipore 10K membrane (4200rpm, 15minutes, 4°C) and lyophilized in 15mL Falcon tube to afford an amorphous white solid, Compound A2 (18mg).

**b .Synthesis of Sense Strand A3**

In a 1.5mL Eppendorf vial, Compound Organix 1 (27.6mg, 0.066mmol) was dissolved in anhydrous DMSO (200uL). In a separate 15 ml falcon tube, Compound A2 (50mg, 0.005mmol) was dissolved in water (2000uL) and diluted with DMSO (200uL).The solution containing G3 was added to the solution containing Compound A2 followed by the addition of triethylamine (30.67ul).

The resulting solution was placed in a shaker and monitored by UPLC-MS for desired product formation. The reaction mixture was purified by ion-pairing chromatography (5-40% Water/Acetonitrile containing 100mM triethylammonium acetate over 35 minutes, XBridge C18 19x150mm). The product fractions were pooled and dialyzed against water 3x using a 15mL Millipore 10K membrane (4200rpm, 15minutes, and 4°C) and lyophilized in 15mL Falcon tube to afford an amorphous white solid, sense strand A3.

**c .Synthesis of Duplex A5**

Sense strand A3 (13.08 mg) was dissolved in DI water (5 ml) and added to a vial containing antisense strand A4 (complimentary to A3, commercially obtained) (0.812mg) in water (0.812 ml). The resulting solution was mixed and heated to 90°C for 3 minutes and let cool down to room temperature for 5 minutes. The solution was lyophilized to afford the duplex as an amorphous white solid, duplex A5.

**Example 12. Synthesis of Stem-Loop dsNAs with three GalNac ligands at the loop**

A stem-loop oligomer was produced with the GalNac conjugated monomers in the loop .The following scheme illustrates the synthesis of GalNac conjugated stem-loop oligomer wherein the GalNac monomers are confined to the loop and are three in number (Scheme B) as shown in pages 233-234.

The sense strand B1 and the complimentary strand to sense strand B3 (Referred to as antisense B4) were obtained by synthesis from commercial manufacturers using standard solid phase nucleic acid synthesis procedures. The sense strand B2 was synthesized in a manner similar to the synthesis of sense strand A2 as described above. The sense strand B3 was synthesized in a manner similar to the synthesis of sense strand A3 as described above. The duplex B5 was synthesized in a manner similar to the synthesis of compound A5 as described above.

**Example 13. Synthesis of Stem- Loop dsNAs with branched GalNac ligands at the loop**

A stem-loop oligomer was produced with the GalNac conjugated monomers in the loop .The Scheme C illustrates the synthesis of GalNac conjugated stem-loop oligomer where the GalNac monomer is confined to the loop and contains a triantennary GalNac ligand as shown in Scheme C (Refer pages 235-236) as 3GT3.

The sense strand C1 and the complimentary strand to C3 (Referred to as antisense strand C4) were obtained by synthesis from commercial manufacturers using standard solid phase nucleic acid synthesis procedures. The sense strand C2 was synthesized in a manner similar to the synthesis of compound A2 as described above.

In a 15 ml falcon tube, sense strand C2 (24mg, 0.002mmol) was dissolved in water (400uL) and then diluted with DMSO (1000 ul). In a separate 1.5 ml Eppendorf vial, Compound 3GTS (40.79mg, 0.023mmol) was dissolved in anhydrous DMSO (150uL). To the solution containing 3GTS, HATU ((1-[Bis(dimethylamino)methylene]-1H-1,2,3-triazolo[4,5-b]pyridinium 3-oxidi hexafluorophosphate, 8.93mg, 0.023mmol) in DMSO (50ul) and N, N -Diisopropylethylamine (8.2ul, 0.047 mmol) were added. After 5 minutes, the solution containing sense strand C2 was added to the reaction mixture.

The reaction mixture was placed in a shaker and monitored by UPLC-MS for desired product formation. The reaction mixture was purified by ion-pairing chromatography (5-40% Water/Acetonitrile containing 100mM triethylammonium acetate over 35 minutes, XBridge C18 19x150mm). The product fractions were pooled and dialyzed against

water 3x using a 15mL Millipore 10K membrane (4200rpm, 15minutes, and 4°C) and lyophilized in 15mL Falcon tube to afford an amorphous white solid, sense strand C3. The duplex C5 was synthesized in a manner similar to the synthesis of duplex A5 as described above.

### Example 14. Synthesis of Stem- Loop dsNAs with four GalNac ligands at the stem

A stem-loop oligomer was produced with the GalNac conjugated monomers in the stem .The following scheme illustrates the synthesis of GalNac conjugated stem-loop oligomer where the GalNac monomers are confined to the stem and are four in number (Scheme D) as shown in pages 237-238.

The sense strand D1 and the complimentary strand to D3 (Referred to as antisense strand D4) were obtained by synthesis from commercial manufacturers using standard solid phase nucleic acid synthesis procedures. The sense strand D2 was synthesized in a manner similar to the synthesis of sense strand A2 as described above. The sense strand D3 was synthesized in a manner similar to the synthesis of sense strand A3 as described above. The duplex D5 was synthesized in a manner similar to the synthesis of compound A5 as described above.

### Example 15 Synthesis of dsNA Oligomers with multiple GalNac ligands at the 5' extension of Antisense strand

A dsNA oligomer was produced with GalNac conjugated monomers in the 5' extension region of the antisense strand. The following scheme illustrates the synthesis of a dsNA oligomer comprising GalNac ligands where the ligands are confined to the extension at the 5' region of the antisense strand and are four in number (Scheme E, Refer pages 239-240). However the number of GalNac conjugated monomers could vary and the position of GalNac ligands can also vary anywhere along the antisense strand.

The antisense strand E1 and the complimentary strand to E2 (Referred to as sense strand E3) were obtained by synthesis from commercial manufacturers using standard solid phase nucleic acid synthesis procedures. The antisense strand E2 was synthesized in a manner similar to that in schemes as described above. The duplex E4 was synthesized in a manner similar to the synthesis of duplex A5 as described above.

### Example 16. Synthesis of dsNA with multiple GalNac ligands at the 5' extension of sense strand

A dsNA oligomer was produced with GalNac conjugated monomers in the 5' extension region of the sense strand. The following scheme illustrates the synthesis of a dsNA oligomer comprising GalNac ligands where the ligands are confined to the extension at the 5' region of the sense strand and are four in number (Scheme F, Refer pages 241-242). However the number of GalNac conjugated monomers could vary and the position of GalNac ligands can also vary anywhere along the sense strand.

The sense strand F1 and the complimentary strand to F2 (Referred to as antisense strand F3) were obtained by synthesis from commercial manufacturers using standard solid phase nucleic acid synthesis procedures. The sense strand F2 was synthesized in a manner similar to the synthesis of sense strand A3 as described above. The duplex F4 was synthesized in a manner similar to the synthesis of duplex A5 as described above.

### Example 17. Synthesis of dsNAs with multiple N-acetyl-galactosamine ligands separated by one spacer on the 5' extension of the sense strand separated by a spacer

A dsNA oligomer was produced with GalNac conjugated monomers in the 5' extension region of the sense strand separated by a spacer. The following scheme illustrates the synthesis of a dsNA oligomer comprising GalNac ligands where the ligands are confined to the extension at the 5' region of the sense strand and are four in number (Scheme G, Refer pages 243-244). However the number of GalNac conjugated monomers could vary and the position of GalNac ligands can also vary anywhere along the sense strand. The extension comprising GalNac ligands separated by a spacer can also be placed on the 5' region of the anti-sense strand.

The sense strand G1 and the complimentary strand to G2 (Referred to as antisense strand G3) were obtained by synthesis from commercial manufacturers using standard solid phase nucleic acid synthesis procedures. The sense strand G2 was synthesized in a manner similar to the synthesis of sense strand A3 as described above. The duplex G4 was synthesized in a manner similar to the synthesis of duplex A5 as described above.

### Example 18. Synthesis of dsNAs with multiple N-acetyl-galactosamine ligands separated by two spacers on the 5' extension of the sense strand

A dsNA oligomer was produced with GalNac conjugated monomers in the 5' extension region of the sense strand separated by two spacers. The following scheme illustrates the synthesis of a dsNA oligomer comprising GalNac

ligands where the ligands are confined to the extension at the 5' region of the sense strand and are four in number (Scheme H, Refer pages 245-246). However the number of GalNac conjugated monomers, number of spacers could vary and the position of GalNac ligands can also vary anywhere along the sense strand. The extension comprising GalNac ligands separated by spacers can also be placed on the 5' region of the anti-sense strand.

The sense strand H1 and the complimentary strand to H2 (Referred to as antisense strand H3) were obtained by synthesis from commercial manufacturers using standard solid phase nucleic acid synthesis procedures. The sense strand H2 was synthesized in a manner similar to the synthesis of sense strand A3 as described above. The duplex H4 was synthesized in a manner similar to the synthesis of duplex A5 as described above.

**Example 19. Synthesis of dsNA Oligomers with multiple GalNac ligands at the 5' extension of sense strand separated from duplex by multiple spacers**

A dsNA oligomer was produced with GalNac conjugated monomers in the 5' extension region of the sense strand separated from the duplex region by multiple spacers. The following scheme illustrates the synthesis of a dsNA oligomer comprising GalNac ligands where the ligands are confined to the extension at the 5' region of the sense strand and are four in number and are separated from the duplex by multiple spacers. (Scheme I, Refer page 247-248). However the number of GalNac conjugated monomers, number of spacers could vary and the position of GalNac ligands can also vary anywhere along the sense strand. The extension comprising GalNac ligands separated by spacers from the duplex can also be placed on the 5' region of the anti-sense strand.

The sense strand I1 and the complimentary strand to I2 (Referred to as antisense strand 13) were obtained by synthesis from commercial manufacturers using standard solid phase nucleic acid synthesis procedures. The sense strand 12 was synthesized in a manner similar to the synthesis of sense strand A3 as described above. The duplex 14 was synthesized in a manner similar to the synthesis of duplex A5 as described above.

**Example 20. Synthesis of dsNA Oligomers with multiple GalNac ligands at the 5' extension of antisense strand separated by two spacers**

A dsNA oligomer was produced with GalNac conjugated monomers in the 5' extension region of the antisense strand separated by two spacers. The following scheme illustrates the synthesis of a dsNA oligomer comprising GalNac ligands where the ligands are confined to the extension at the 5' region of the antisense strand and are four in number (Scheme J, Refer page 249-250). However the number of GalNac conjugated monomers, number of spacers could vary and the position of GalNac ligands can also vary anywhere along the antisense strand. The extension comprising GalNac ligands separated by spacers can also be placed on the 5' region of the sense strand.

The antisense strand J1 and the complimentary strand to J2 (Referred to as antisense strand J3) were obtained by synthesis from commercial manufacturers using standard solid phase nucleic acid synthesis procedures. The anti-sense strand J1, J2 were synthesized in a manner similar to the synthesis of sense strand A3 as described above. The duplex J4 was synthesized in a manner similar to the synthesis of duplex A5 as described above.

**Example 21 Synthesis of dsNA Oligomers with multiple GalNac ligands at the 5' extension of antisense strand separated from duplex by multiple spacers**

A dsNA oligomer was produced with GalNac conjugated monomers in the 5' extension region of the antisense strand separated from the duplex region by multiple spacers. The following scheme illustrates the synthesis of a dsNA oligomer comprising GalNac ligands where the ligands are confined to the extension at the 5' region of the antisense strand and are four in number and are separated from the duplex by multiple spacers. (Scheme K1, Refer pages 252-253). However the number of GalNac conjugated monomers, number of spacers could vary and the position of GalNac ligands can also vary anywhere along the antisense strand. The extension comprising GalNac ligands separated by spacers from the duplex can also be placed on the 5' region of the sense strand.

The antisense strand K1 and the complimentary strand to K2 (Referred to as antisense strand K3) were obtained by synthesis from commercial manufacturers using standard solid phase nucleic acid synthesis procedures. The anti-sense strand K1 and K2 were synthesized in a manner similar to the synthesis of sense strand A3 as described above. The duplex K4 was synthesized in a manner similar to the synthesis of duplex A5 as described above.

**Example 22 .In vitro Self-delivery of GalNAc conjugated DsiRNAs to mouse hepatocytes in culture.**

8 to 10 week old CD-1 or C57-BL/6 female mice were used for primary hepatocyte preparation. The mice were anesthetized using isofluorane. Once anesthetized, the abdominal cavity was opened and the vena cava cannulated with an 18 gauge catheter and the portal vein was cut to drain blood from the liver. The liver was perfused with perfusion

buffer [HBSS, 1 mM EDTA (Boston Bioproducts)] for 5-10 minutes, at a flow rate of 5 ml/min till the perfusate was clear. The liver was then perfused with collagenase buffer II [DMEM (Gibco), 1% BSA (Fisher), 0.8 mg/ml Collagenase I (Worthington laboratories)] at 37°C for 5-10 minutes. The digested liver was transferred to cold solution III [DMEM (Gibco), 1% BSA (Fisher)] and minced. The cell suspension was passed through a 70 μm mesh (Corning) and centrifuged at 50 x g for 3 minutes. The supernatant was discarded and the pellet was washed in cold solution III twice more, followed by one wash in William's buffer with thawing and plating supplements (Life Technologies). The viability and concentration of the hepatocytes was estimated by Trypan blue exclusion and counting in a hemacytometer. The cells were plated in collagen I coated 96 well plates (Corning) at a concentration of 5 x 104 cells per well and incubated at 37°C, 5% CO2 in a humidified atmosphere for 4-5 hours. The medium was changed to William's medium with maintenance supplements (Life Technologies) and varying concentrations of GalNAc conjugated oligonucleotides were added to the medium and the cells were incubated for 24 hours, after which the medium was renewed and the cells grown for another 24 hours. The cells were then lysed and RNA was purified using the SV96 kit (Promega), the RNA was reverse transcribed using the Transcriptor first strand synthesis kit (Roche).

The relative quantity of the target gene was measured by multiplexed qPCR on a Bio-Rad CFX96 system, normalized to a multiplexed housekeeping gene. The percent remaining of a selected transcript relative to an untreated control was calculated and IC50 values were estimated by non-linear regression. Examples of in vitro self-delivery (without the usage of transfecting agents) data of selected compounds/conjugates are shown in Figure 25 A and B. The Figure 25 A shows the activity of dsNA molecules with extension and the figure 25B shows the activity of dsNA molecules with tetraloop. The extension molecules differ from each other in the pattern and nature of modifications as illustrated by the digramatic keys in the figure. Likewise the tetraloop molecules in Figure 25B differ from each other in the pattern and the nature of modifications. The sequence information for the dsNA molecules are shown in Table 3. These data demonstrated that GalNAc dsNA conjugates including 5'-extension and nicked tetraloop self-deliver into primary hepatocytes in culture with low nM IC50s.

## Example 23. In vitro Cell Free binding to CD301

The binding of monovalent and triantennary N-acetyl-galactosamine constructs were estimated using a fluorescence polarization competition binding assay. The tracer ligand was a custom synthesized FITC-triantennary N-acetyl-galactosamine construct - 3GT1'-FITC. A constant concentration of 4 μg/ml of macrophage asialoglycoprotein receptor - CD301 (R & D systems) and 50 nM 3GT1 '-FITC were incubated at 25°C in binding buffer (20 mM HEPES pH 7.4, 150 mM NaCl, 1 mM CaCl2, 0.5 mM MgCl2) along with varying concentrations of dsNAs of invention. Fluorescence polarization measurements were made after 30 minutes on a Spectramax M5 microplate reader (Molecular Devices). Binding of the dsNA conjugates was estimated as a decrease in polarization units through the displacement of 3GT1 '-FITC from CD301. The relative binding of each dsNA conjugate is estimated through non-linear regression and calculation of IC50 values. Examples of in vitro binding data of selected compounds/conjugates are shown in Figure 13. These data demonstrated that dsNA conjugates based on 5'-extension and nicked tetraloop bind ASGPr effectively and in many cases showed better binding affinity as compared to the Tris-linker based tri-antennary GalNAc (DP2465P:DP2382G).

The cell free binding to CD301 of a dsNA conjugate linked to GalNAc ligands through an acetal linker (see Example 25 for synthesis) and a dsNA conjugate linked to GalNAc ligands through a 2'-triazole linker was comparable (Figure 48). These two dsNA agents contained the same nucleic acid sequence and GalNAc ligand such that only the linker differed between the two agents. The cell free binding of the dsNA conjugate with the 2'-triazole linker from Figure 48 and a dsNA conjugate with the same triazole linker but with same PEG linker length as the acetal linker of Figure 48 were also comparable (Refer Figure 49). This shows that the use of an acetal linker in place of the 2'-triazole linker did not impair the binding of the dsNA conjugate. It is interesting to note that the activity of the dsNA molecules in terms of knockdown of target or binding to the receptor was not greatly affected due to the increase or decrease in the length of the linker. It was surprising to learn that the acetal linker was able to survive the rigors of nucleic acid synthesis and was still functional under in vivo/in vitro conditions to deliver the conjugate to the receptor. (Refer Figures 48-50).

The in vitro knockdown potency of the two dsNA GalNAc described above with triazole linkers but with different length PEG segments of the linker, was tested. Figure 50 shows that the two dsNA conjugates had comparable intrinsic knockdown potency in mice, demonstrating that changing the PEG length in the linker did not change the in vivo function of the dsNA agent.

Preparations of GalNAc ligands, GalNAc containing monomers, and GalNAc oligonucleotide conjugates are described below in the examples and synthetic schemes. Specific oligonucleotide sequences used for the compounds and/or conjugates described below are shown in Table 3.

## Example 24. In vivo Evaluation of RNAi Stability, Immunogenicity, and Activity

Wild type or diseased genetic engineered mice were dosed by subcutaneous or intravenous injection in less

than 200ul of volume. Animals were observed for behavioral or physiological changes. For pharmacokinetic and pharmacodynamic analysis, animals were sacrificed 24 to 168 hours post last dose as indicated by CO2 asphyxiation. Blood and tissue samples were collected following standard procedures. For mRNA analysis, tissues were stored in freezer later until RT-qPCR was performed following standard protocols. Frozen tissues were also collected for western and immunohistochemistry analysis. Urine samples were collected and assayed for metabolites during study course using enzymatic or LC/MS method. Cytokine, blood cell count and blood chemistry analysis were conducted following standard procedures. Examples of in vivo data of selected compounds/conjugates are shown in Figure 27, 28, 29 and 30. These data demonstrated that GalNAc dsNA conjugates including 5'-extension at the sense strand and nicked tetraloop constructs are active in knocking down target mRNA in vivo in mice when dosed subcutaneously and intravenously.

**Example 25. Synthesis of N-Acetyl Galactosamine (GalNAc) conjugated phosphoroamidite monomer with the GalNAc linked through 2'-acetal linker**

The GalNAc ligand not only can be attached to the nucleobase of the nucleoside monomer (i.e. C5 position of Thymidine as shown in Example 8, 9), but also can be attached to the ribose sugar via 2'-OH (or 3'-OH) with a linker. 2'-OH as a conjugation site is particularly useful in the case of tetraloop DsiRNA because the 2'-OHs of the four nucleotides in the loop are exposed to the solvent and are not involved in hydrogen bonding and base stacking which is needed for forming the stable loop based on their crystal structure. The acetal chemistry used to install the linker to 2'-OH (shown in the following schemes IV-VI, Refer pages 215-217) is much milder as compared to the traditional alkylation conditions; therefore, allow selective conjugation to 2' position. This will avoid the tedious separation of the mixture of 2' and 3' isomers and improve the yield significantly. The synthesis of compounds 2-4 is depicted in scheme IV, the synthesis of compounds 5-9 is depicted in scheme V and the synthesis of compounds 10 and 11 are depicted in scheme VI. (Refer Page 215-217).

**a. Synthesis of Compound 2**

2-(2-Aminoethoxy) ethanol (105.0 g), triethylamine (101.0 g) and ethyl trifluoroacetate were added to a flask and the mixture was stirred for 24 h. Dichloromethane (1 L) was added and the solution was washed with water (500 ml). The organic layer was dried (MgSO4) and evaporated to give a liquid (161.0 g).

**b. Synthesis of Compound 3**

The mixture of compound 2 (161.0 g) and para-formaldehyde (24.0) in methylene chloride (200 ml) was cooled in an ice bath under stirring. Anhydrous hydrogen chloride was bubbled through the mixture for 3 h. The reaction mixture was allowed up to room temperature and two clear homogeneous layers were separated. The organic layer was dried over anhydrous calcium chloride and concentrated on a rotary evaporator to liquid (201 g).

**c. Synthesis of Compound 4**

The obtained compound 3 was dissolved in acetonitrile (200 ml) and sodium acetate (100 g) was added. The mixture was stirred at room temperature for 14 h. The precipitate was filtered off; the filtrate was concentrated on a rotary evaporator to liquid. The residue was dissolved in ethyl acetate (100 ml) and washed with 10% aqueous solution of sodium hydrogen carbonate (50 ml) and water (2x50 ml). The organic layer was dried over anhydrous sodium sulfate and concentrated on a rotary evaporator to liquid (176.0 g) (MS M-1: 272.0).

**d. Synthesis of Compound 69**

To a cool solution (-15 °C) under nitrogen of nucleoside 5 (61.3 g) and compound 4 (55 g) in 1,2-dichloromethane (500 ml) tin tetrachloride (18 ml) was added and the solution was kept at -12 °C for 20 min. A saturated aqueous solution of sodium hydrogen carbonate (500 ml) and methylene chloride (1 L) were added and the suspension was stirred at 0 DC for 20 min. The suspension was filtered, organic layer was separated, washed with water (200 ml), dried over anhydrous sodium sulfate, and evaporated to dryness. The residue was purified by column chromatography on silica gel. The column was eluted with methylene chloride-methanol (100:0-95:5), and then evaporation of appropriate fractions to give compound 6 as a foam (55.2 g) (MS M-1: 825.4).

**e. Synthesis of Compound 7**

Nucleoside 6 (55 g) was dissolved in 0.5 M tetrabutylammonium fluoride trihydrate in tetrahydrofuran (1 L),

kept for 10 min at 20 °C, evaporated to dryness, evaporated with chloroform (10 ml), and applied on a column with silica gel. The column was eluted with methylene chloride-methanol (100:0 - 90:10), and then evaporation of appropriate fractions to give compound 7 as a foam (30.5 g) (MS M-1: 583.2).

### f. Synthesis of Compound 8

Nucleoside 7 (30.0 g) was dried by evaporation with pyridine (2x20 ml). The residue was dissolved in dry pyridine (300 ml), dimethoxytrityl chloride (18.2 g) was added, and the resulted solution was kept for 16 h at 20 °C. MeOH (10 ml) was added and after 30 min the mixture was concentrated in vacuum to near dryness. The residue was dissolved in methylene chloride (500 ml), and washed with 10% aqueous solution of sodium bicarbonate (200 ml) and water (2x200 ml). The organic layer was dried over anhydrous sodium sulfate, evaporated in vacuum, evaporated with toluene (2x100 ml), and purified by column chromatography on silica gel. The column was eluted with methylene chloride-methanol (100:0 - 90:5), and then evaporation of appropriate fractions to give compound 8 as a foam (40.5 g) (MS M-1: 885.3).

### g. Synthesis of Phosphoramidite [Compound 9]

The dimethoxytritylated derivative 8 (40.0 g) was dissolved in 600 ml dichloromethane under argon and ethyl-(di-isopropyl)amine (10 ml) and 2-cyanoethyl diisopropylphosphoramidochloridite (16.0 g) were added. After stirring the solution for 3 h, TLC indicated complete reaction. A 10% aqueous solution of sodium hydrogen carbonate (2 ml) was added, the solution was stirred for 10 min, and partitioned between methylene chloride (500 ml) and aqueous sodium carbonate (300 ml). The organic phase was washed with aqueous sodium chloride (2x300 ml) and the aqueous phases were back extracted with methylene chloride (200 ml). Evaporation of the organics left an oil, which was flash purified on silica gel (hexane-ethyl acetate, 20:80) to afford the product as a foam after coevaporation (36.5 g) (MS M-1: 1085.4). Compound 11 was made from nucleoside 10 in the same way as described above. Similarly Schemes VII (Refer Pages 218-219) and VIII (Refer Page 220) depict the production of compound 17, the procedures describe above can be adopted to make compound 17.

### Example 26. Synthesis of Cholesterol conjugated Nicked Tetraloop dsNA with cholesterol ligands on the loop

Cholesterol conjugated dsNA molecules can be produced by following the protocols established for the synthesis of GalNAc conjugated dsNA molecules. The cholesterol conjugated dsNA molecules are synthesized by using post solid phase conjugation through standard click chemistry or amide chemistry methods. In one example the Cholesterol conjugated dsNA molecules is made by solid phase synthesis using Cholesterol phosphoramidites.

A stem-loop dsNA having cholesterol conjugated nucleotides in the loop is produced using click chemistry, for instance, as shown in scheme L (Refer Page 256-257). Compound K2 required for the synthesis is produced by following the protocol illustrated in scheme Kb (Refer Pages 254-255). In one example, a stem-loop dsNA having cholesterol conjugated nucleotides in the loop is produced using amide chemistry, for instance as shown in scheme L1 (Refer Page 258-259). The schemes L and L1 illustrate the synthesis of cholesterol conjugated stem-loop dsNA wherein the cholesterol monomers are confined to the loop and are singular in number. Similarly scheme M (Refer Pages 260-261) illustrates the synthesis of cholesterol conjugated stem-loop dsNA molecules wherein the cholesterol monomers are confined to the loop but are multiple in numbers.

The sense strand and its complementary antisense strand, as shown in the aforesaid schemes are obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

### Example 27. Synthesis of Cholesterol conjugated Nicked Tetraloop dsNA with cholesterol ligands at the stem

A stem-loop dsNA having cholesterol conjugated nucleotides in the stem is produced using click chemistry, for instance, as shown in scheme N (Refer Pages 262-263). Compound K2 required for the synthesis is produced by following the protocol illustrated in scheme Kb. The scheme N (Refer Pages 262-263) illustrates the synthesis of cholesterol conjugated stem-loop dsNA wherein the cholesterol monomers are confined to the stem and are singular in number. Similarly scheme O (Refer Pages 264-265) illustrates the synthesis of cholesterol conjugated stem-loop dsNA molecules wherein the cholesterol monomers are confined to the stem but are multiple in numbers.

The sense strand and the complementary antisense strand, as shown in the aforesaid schemes is obtained by synthesis from commercial manufacturers using standard solid phase nucleic acid synthesis procedures. The sense strand is synthesized in a manner similar to the synthesis of sense strand A3 as described previously. The duplex shown

in aforesaid schemes is synthesized in a manner similar to the synthesis of duplex A5 as described previously.

**Example 28 Synthesis of dsNAs with cholesterol ligands at the 5' extension of the Antisense strand**

A dsNA molecule having cholesterol conjugated nucleotides in the 5' extension of the antisense strand is produced by using post solid phase conjugation through click chemistry, for instance, as shown in scheme P (Refer Pages 266-267). Compound K2 required for the synthesis can be produced by following the protocol illustrated in scheme Kb.(Refer Pages 254-255)

The scheme P1(Refer Pages 268-269) illustrates the solid phase synthesis of cholesterol conjugated dsNA molecules using cholesterol phosphoramidites, wherein the cholesterol monomers are confined to the 5' extension of the antisense strand, and are singular in number. However the number of cholesterol conjugated monomers can vary, for example a ligand can be conjugated at the 1st, 2nd, 3rd, 4th, 5th position from the 5' end or 3' end of antisense strand or at a position beyond the 5$^{th}$ position. The scheme Q (Refer Page 270-271) illustrates the post solid phase conjugation of cholesterol to dsNA molecules using click chemistry wherein the cholesterol monomers are confined to the 5' extension of the antisense strand but are multiple in numbers. The scheme Q1 (Refer Page 272-273) illustrates the solid phase synthesis of cholesterol conjugated dsNA molecules using cholesterol phosphoramidites, wherein the cholesterol monomers are confined to the 5' extension at the antisense strand but are multiple in numbers.

The sense strand and its complementary antisense strand, as shown in the aforesaid schemes is be obtained by synthesis from commercial manufacturers using standard solid phase nucleic acid synthesis procedures. The sense strand is synthesized in a manner similar to the synthesis of sense strand A3 as described previously. The duplex shown in aforesaid schemes shall be synthesized in a manner similar to the synthesis of duplex A5 as described previously.

**Example 29 Synthesis of dsNAs with cholesterol ligands at the 5' extension of the sense strand**

A dsNA molecule having cholesterol conjugated nucleotides in the 5' extension of the sense strand is produced by using post solid phase conjugation through click chemistry, for instance, as shown in scheme R (Refer Pages 274-275). Compound K2 required for the synthesis is produced by following the protocol illustrated in scheme Kb.

The scheme R1 (Refer Pages 276-277) illustrates the solid phase synthesis of cholesterol conjugated dsNA molecule using cholesterol phosphoramidites, wherein the cholesterol monomers are confined to the 5' extension of the sense strand, and are singular in number. However the number of cholesterol conjugated monomers can vary, for example a ligand is conjugated at the 1st, 2nd, 3rd, 4th, 5th position from the 5' end or 3' end of sense strand or at a position beyond the 5$^{th}$ position. Using the synthetic schemes discussed previously , the number of cholesterol conjugated nucleotides is varied, as well as the position of the cholesterol ligands, for example a ligand can also be anywhere along the sense strand.

The sense strand and its complementary antisense strand, as shown in the aforesaid schemes are obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

**Example 30. Synthesis of dsNAs with multiple cholesterol ligands at the 5' extension of the sense strand separated by one or more spacers**

A dsNA with cholesterol conjugated nucleotides in the 5' extended region of the sense strand, the nucleotides being separated by a spacer according to the invention are produced by following the protocols developed for the synthesis of similar dsNA molecules comprising a GalNAc ligand. The schemes G, H, I, J and K (Refer Pages 243-254) describe the synthesis of GalNAc conjugated dsNA molecules wherein the ligand conjugated nucleotides are separated by one or more spacers. The cholesterol conjugated nucleotides can be in either stem region, or loop region, on the 5' extension of the sense strand, or the 5' extension of the antisense strand, the 3 'extension of the sense strand, or the 3' extension of the antisense strand or a combination of any of the aforesaid options. The schemes G, H, I, J and K produce specific to GalNAc ligand conjugation, are easily adapted and modified to produce with Cholesterol ligand conjugates. The mode of conjugation of ligand to nucleotide is similar and can occur through post synthetic conjugation via click chemistry or amide chemistry or solid phase synthesis using ligand phosphoramidites.

**Example 31. Synthesis of Folate conjugated Nicked Tetraloop dsNA with Folate on the loop**

Folate conjugated dsNA molecules are produced by following the protocols established for the synthesis of GalNAc conjugated dsNA molecules. The Folate conjugated dsNA molecules are synthesized by using post solid phase conjugation, through standard click chemistry or amide chemistry methods. In one embodiment the Folate conjugated

dsNA molecules are made by solid phase synthesis using Folate phosphoramidites.

A stem-loop dsNA having Folate conjugated nucleotides in the loop is produced using click chemistry, for instance, as shown in scheme U (Refer Pages 281-282). Compound S6 (click chemistry) or S13 (amide chemistry) required for the synthesis is produced by following the protocol illustrated in schemes S (Refer Pages 278-279) and T(Refer Page 280) respectively. The production of compounds T7 and S13 are depicted in schemes T and S respectively following the same protocols used for making GalNAc conjugates as described earlier. In one embodiment a stem-loop dsNA having Folate conjugated nucleotides in the loop is produced using amide chemistry, for instance as shown in scheme U1. The schemes U (Refer Pages 281-282) and U1 (Refer Page 283) illustrate the synthesis of Folate conjugated stem-loop dsNA wherein the Folate monomers are confined to the loop and are singular in number. Similarly schemes V (Refer Pages 284-285) and V1 (Refer Pages 286-287) illustrate the synthesis of Folate conjugated stem-loop dsNA molecules wherein the Folate monomers are confined to the loop but are multiple in numbers.

The sense strand and its complementary antisense strand, as shown in the aforesaid schemes are obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

## Example 32. Synthesis of Folate conjugated Nicked Tetraloop dsNA with Folate at the stem

A stem-loop dsNA having Folate conjugated nucleotides in the stem is produced using click chemistry, for instance, as shown in scheme W (Refer Pages 288-289). Compound S6 (click chemistry) or S13 (amide chemistry) required for the synthesis is produced by following the protocol illustrated in schemes S (Refer Pages 278-279) and T (Refer Page 280) respectively. The scheme W1 (Refer Pages 290-291) illustrates the synthesis of Folate conjugated stem-loop dsNA using amide chemistry, wherein the Folate monomers are confined to the stem and are singular in number. Similarly schemes X (Refer Pages 292-293) and X1 (Refer Pages 294-295) illustrate the synthesis of Folate conjugated stem-loop dsNA molecules wherein the Folate monomers are confined to the stem but are multiple in numbers.

The sense strand and its complementary antisense strand, as shown in the aforesaid schemes are obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

## Example 33 Synthesis of dsNAs with Folate ligands at the 5' extension of the Antisense strand

A dsNA molecule having Folate conjugated nucleotides in the 5' extension of the antisense strand can be produced by using post solid phase conjugation through click chemistry, for instance, as shown in scheme Y (Refer Pages 296-297). Compound S6 (click chemistry) or S13 (amide chemistry) required for the synthesis can be produced by following the protocol illustrated in schemes S (Refer Pages 278-279) and T (Refer Page 280) respectively.

The scheme Y1 (Refer Pages 298-299) illustrates the solid phase synthesis of Folate conjugated dsNA molecule using Folate phosphoramidites, wherein the Folate monomers are confined to the 5' extension of the antisense strand, and are singular in number. However the number of Folate conjugated monomers can vary, for example a ligand can be conjugated at 1st, 2nd, 3rd, 4th, or 5th position from the 5' end or 3' end of antisense strand or at a position beyond the 5$^{th}$ position. The scheme Z (Refer Pages 300-301) illustrates the post solid phase conjugation of Folate to dsNA molecules using click chemistry wherein the Folate monomers are confined to the 5' extension of the antisense strand but are multiple in numbers. The scheme Z1 (Refer Pages 302-303) illustrates the solid phase synthesis of Folate conjugated dsNA molecules using Folate phosphoramidites, wherein the Folate monomers are confined to the 5' extension at the antisense strand but are multiple in numbers.

The sense strand and its complementary antisense strand, as shown in the aforesaid schemes are obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

## Example 34 Synthesis of dsNAs with Folate ligands at the 5' extension of the sense strand

Folate conjugated dsNA molecules are produced by following the protocols established for the synthesis of GalNAc conjugated dsNA molecules. The Folate conjugated dsNA molecules are synthesized by using post solid phase conjugation through standard click chemistry or amide chemistry methods. In one embodiment the Folate conjugated dsNA molecules are made by solid phase synthesis using Folate phosphoramidites.

A dsNA molecule having Folate conjugated nucleotides in the 5' extension of the sense strand is produced by using post solid phase conjugation through click chemistry, for instance, as shown in scheme AA (Refer Pages 304-305).

Compound S6 (click chemistry) or S13 (amide chemistry) for the synthesis is produced by following the protocol illustrated in schemes S (Refer Pages 278-279) and T (Refer Page 280) respectively.

The scheme AA1 (Refer Pages 306-307) illustrates the solid phase synthesis of Folate conjugated dsNA molecule using Folate phosphoramidites, wherein the Folate monomers are confined to the 5' extension of the sense strand, and are singular in number. However the number of Folate conjugated monomers can vary, for example a ligand can be conjugated at the 1st, 2nd, 3rd, $4^{th}$ or 5th position from the 5' end or 3' end of sense strand or at a position beyond the $5^{th}$ position. Using the synthetic schemes discussed previously, the number of Folate conjugated nucleotides can be varied, as well as the position of the Folate ligands, for example a ligand can also be anywhere along the sense strand.

The sense strand and its complementary antisense strand, as shown in the aforesaid schemes are obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

## Example 35. Synthesis of dsNAs with multiple Folate ligands at the 5' extension of the sense strand separated by one or more spacers

A dsNA with Folate conjugated nucleotides in the 5' extended region of the sense strand, the nucleotides being separated by a spacer is produced by following the protocols developed for the synthesis of similar dsNA molecules wherein the ligand is GalNAc. The schemes G, H, I, J and K (Refer Pages 243-254) describe the synthesis of GalNAc conjugated dsNA molecules wherein the ligand conjugated nucleotides are separated by one or more spacers. The Folate conjugated nucleotides can be on the 5' extension of the sense strand, the 5' extension of the antisense strand, the 3' extension of the sense strand, or the 3' extension of the antisense strand or a combination of any of aforesaid options. The schemes G, H, I, J and K specific to GalNAc ligand conjugation, are easily adapted and modified to produce Folate ligand conjugates. The mode of conjugation of ligand to nucleotide is similar and often occurs through the post synthetic conjugation via click chemistry or amide chemistry or solid phase synthesis using ligand phosphoramidites. The scheme V(Refer Pages 284-285) and V1(Refer Pages 286-287) show the synthesis of Folate Nicked Tetraloop dsNA conjugates with multiple folates on the loop using post solid phase conjugation method utilizing click chemistry as described earlier for GalNAc conjugates.

## Example 36. Synthesis of Mannose-6-phophate conjugated Nicked Tetraloop dsNA with Mannose-6-phophate ligand on the loop

Mannose-6-phophate conjugated dsNA molecules are produced by following the protocols established for the synthesis of GalNAc conjugated dsNA molecules. The Mannose-6-phophate conjugated dsNA molecules are synthesized by using post solid phase conjugation through standard click chemistry or amide chemistry methods.

A stem-loop dsNA having Mannose-6-phophate conjugated nucleotides in the loop is produced using click chemistry, for instance, as shown in scheme AB (Refer Pages 308-309). Compound 114 (click chemistry) or compounds 108 (amide chemistry) required for the synthesis is produced by following the protocol illustrated in schemes IX (Refer Page 221) and X (Refer Pages 222-223) respectively. In one embodiment, a stem-loop dsNA having Mannose-6-phophate conjugated nucleotides in the loop is produced using amide chemistry, for instance as shown in scheme AC (Refer Pages 310-311). The schemes AB (Refer Pages 308-309) and AC (Refer Pages 310-311) illustrate the synthesis of Mannose-6-phophate conjugated stem-loop dsNA molecules wherein the Mannose-6-phophate monomers are confined to the loop and are produced by post synthetic methods. Similarly schemes AD (Refer Pages 312-313) and AE (Refer Pages 314-315) illustrate the solid phase synthesis of Mannose-6-phophate conjugated stem-loop dsNA molecules using Mannose-6-phophate nucleoside phosphoroamidites, wherein the Mannose-6-phophate monomers are confined to the loop. In scheme AD (Refer Pages 312-313) the ligand Mannose-6-phosphate is linked to the nucleotide using 2'- Triazole linkers and in scheme AE the ligand Mannose -6-phosphate is linked to the nucleotide using 2'-Acetal linkers.

The sense strand and its complementary antisense strand, as shown in the aforesaid schemes is obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

## Example 37. Synthesis of Mannose-6-phophate conjugated Nicked Tetraloop dsNA with Mannose-6-phophate ligand at the stem

A stem-loop dsNA having Mannose-6-phophate conjugated nucleotides in the stem is produced using click chemistry, for instance, as shown in scheme AF (Refer Pages 316-317). Compound 114 (click chemistry) or compound

108 (amide chemistry) required for the synthesis is produced by following the protocol illustrated in schemes IX (Refer Page 221) and X (Refer Page 222-223) respectively. The scheme AF (Refer Pages 316-317) illustrates the synthesis of Mannose-6-phophate conjugated stem-loop dsNA using click chemistry, wherein the Mannose-6-phophate monomers are confined to the stem and are in multiples.

The sense strand and its complementary antisense strand, as shown in the aforesaid schemes are obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

**Example 38 Synthesis of dsNAs with Mannose-6-phophate ligands at the 5' extension of the Antisense strand**

A dsNA molecule having Mannose-6-phophate conjugated nucleotides in the 5' extension of the antisense strand is produced by using post solid phase conjugation through click chemistry, for instance, as shown in scheme AG (Refer Pages 318-319). Compound 114 (click chemistry) or compound 108 (amide chemistry) required for the synthesis is produced by following the protocol illustrated in schemes IX (Refer Page 221) and X (Refer Page 222-223) respectively.

The scheme AG (Refer Pages 318-319) illustrates the synthesis of Mannose-6-phophate conjugated dsNA molecule using postsyntheic conjugation, wherein the Mannose-6-phophate monomers are confined to the 5' extension of the antisense strand. However the number of Mannose-6-phophate conjugated monomers can vary, for example a ligand can be conjugated at the 1st, 2nd, 3rd, 4$^{th}$ or 5th position from the 5' end or 3' end of antisense strand or at a position beyond the 5$^{th}$ position.

The sense strand and its complementary antisense strand, as shown in the aforesaid schemes is obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously. Similar variations of dsNA molecules containing Mannose-6-phophate comjugates are shown in schemes AI, AJ, AK, AL and AM (Refer Pages 322-331). These are synthesized following the same protocols designed for GalNac and cholesterol conjugates as described earlier.

**Example 39 Synthesis of dsNAs with Mannose-6-phophate ligands at the 5' extension of the sense strand**

A dsNA molecule having Mannose-6-phophate conjugated nucleotides in the 5' extension of the sense strand can be produced by using post solid phase conjugation through amide chemistry, for instance, as shown in scheme AH. Compound 114 (click chemistry) or compound 108 (amide chemistry) for the synthesis can be produced by following the protocol illustrated in schemes IX and X respectively.

The scheme AH (Refer Pages 320-321) illustrates the synthesis of Mannose-6-phophate conjugated dsNA molecule using amide chemistry, wherein the Mannose-6-phophate monomers are confined to the 5' extension of the sense strand. The scheme AN (Refer Page 332) illustrates the solid phase synthesis of Mannose-6-phosphate conjugated dsNA molecule using Mannose-6-phosphate-amidite, wherein the Mannose-6-phosphate monomers are confined to the 5' extension of the sense strand. However the number of Mannose-6-phophate conjugated monomers can vary, for example a ligand can be conjugated at 1st, 2nd, 3rd, 4th, 5th or farther position from the 5' end or 3' end of sense strand or at a position beyond the 5$^{th}$ position. Using the synthetic schemes discussed previously, the number of Mannose-6-phophate conjugated nucleotides can be varied, as well as the position of the Mannose-6-phophate ligands, for example a ligand can also be anywhere along the sense strand.

The sense strand and its complementary antisense strand, as shown in the aforesaid schemes are obtained by synthesis from commercial manufacturers using standard solid phase nucleic acid synthesis procedures. The sense strand is synthesized in a manner similar to the synthesis of sense strand A3 as described previously. The duplex shown in aforesaid schemes is synthesized in a manner similar to the synthesis of duplex A5 as described previously.

**Example 40. Synthesis of dsNAs with multiple Mannose-6-phophate ligands at the 5' extension of the sense strand separated by one or more spacers**

A dsNA with Mannose-6-phophate conjugated nucleotides in the 5' extended region of the sense strand, the nucleotides being separated by spacers is produced by following the protocols developed for the synthesis of similar dsNA molecules wherein the ligand is GalNAc. The schemes G, H, I, J and K (Refer Pages 243-254) describe the synthesis of GalNAc conjugated dsNA molecules wherein the ligand conjugated nucleotides are separated by one or more spacers.

The Mannose-6-phophate conjugated nucleotides can be in the 5' extension of the sense strand, or the 5' extension of the antisense strand, or the 3 'extension of the sense strand, or the 3' extension of the antisense strand or

a combination of any of aforesaid options. The schemes G, H, I, J and K produce specific to GalNAc ligand conjugation, are easily adapted and modified to produce with Cholesterol ligand conjugates. The mode of conjugation of ligand to nucleotide is similar and can occur through post synthetic conjugation via click chemistry or amide chemistry or solid phase synthesis using ligand phosphoramidites.

For instance, the schemes AI, AJ and AK (Refer Pages 322-327) illustrate the synthesis of a dsNA comprising Mannose-6-phosphate ligands where the ligands are confined to at the 5' extended region of the sense strand and are separated by one or two or more spacers respectively. However the number of Mannose-6-phosphate conjugated nucleotides can vary and the position of Mannose-6-phosphate ligands can also vary, for example, a ligand can be anywhere along the sense strand. The Mannose-6-phosphate ligand conjugated nucleotides separated by one or more spacers can also be located on the 5' region of the antisense strand and/or the sense strand. The schemes AL and AM (Refer Pages 328-331) illustrate the synthesis of a dsNA comprising Mannose-6-phosphate ligands, where the ligands are confined to at the 5' extended region of the antisense strand and are separated by two or more spacers respectively.

The sense strand and its complementary antisense strand, as shown in the aforesaid schemes can be obtained by synthesis from commercial manufacturers using standard solid phase nucleic acid synthesis procedures. The sense strand is synthesized in a manner similar to the synthesis of sense strand A3 as described previously. The duplex shown in aforesaid schemes shall be synthesized in a manner similar to the synthesis of duplex A5 as described previously.

## Example 41. Synthesis of GalNAc amidite synthons

2-(2-Chloroethoxy)ethanol (124.6 g) and sodium azide (130 g) in 5 l H2O were heated under reflux overnight. Upon cooling the reaction mixture was extracted with CH2Cl2 (3 x 2000 ml), the organic layers were combined, washed with brine, anhydrous $Na_2SO_4$, filtered, and concentrated under reduced pressure to give 2-(2-azidoethoxy)ethanol a liquid 105 g. Yield: 80%. (Refer Scheme XI, Page 224). A solution of 105 g of 2-(2-azidoethoxy)ethanol and 200 mL of Et3N in 2 L of dry CH2Cl2 was cooled to 0 °C under a nitrogen atmosphere. A solution of methanesulfonyl chloride (116 g) in CH2Cl2 (500 mL) was added dropwise to this mixture over a 30-min period, and the solution was warmed to room temperature and stirred for 1.5 h. After the precipitate was filtered off, the solvent was evaporated and the crude product was purified by column chromatography eluting with a 2:1 mixture of n-hexane and ethyl acetate to give 2-(2-azidoethoxy)ethyl methanesulfonate as a pale yellow oil (126 g). (Refer Scheme XI, Page 224).

A solution of 126 g of compound 3 and 116.5 g of potassium phthalimide in 4 L of dry DME was heat at reflux for 18 h under a nitrogen atmosphere. After cooling to room temperature and concentration in vacuo, the resulting residue was diluted with ethyl acetate (10 L) and the solids were filtered. Concentration of the filtrate in vacuo followed by purification of the crude product by column chromatography eluting with a 4:1 mixture of n-hexane and ethyl acetate to give 4 as a colorless solid (103g). (Refer Scheme XI, page 224)

A solution of 100 g of compound 4 and 40 mL of 80% hydrazine hydrate in 2000 mL of absolute ethanol was heated at 55 °C for 2 h, during which time a white precipitate formed. The mixture was cooled to room temperature and concentrated in vacuo, after which the crude residue was diluted with 2000 mL of dry CH2Cl2. After the precipitate was filtered off, the solvent was dried and concentrated in vacuo to afford pale yellow oil, which was used in the next step without further purification (45.7 g). MS M+1: 131.0.

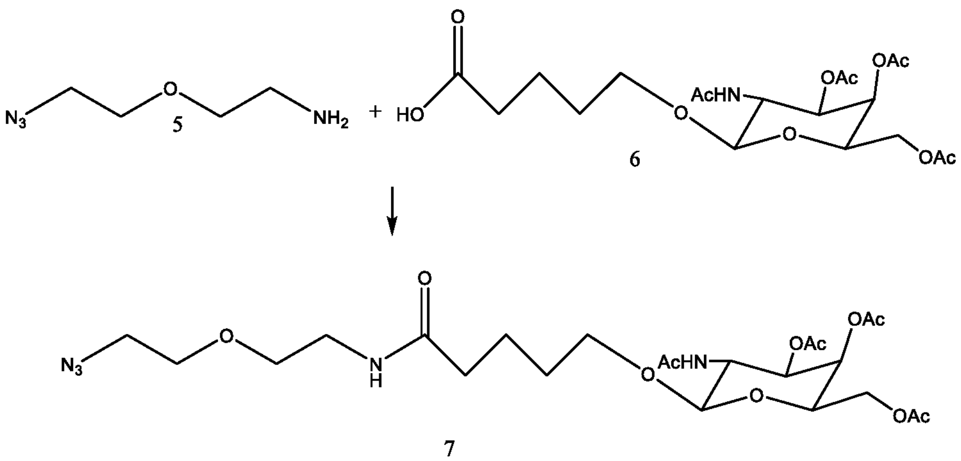

Compound 6 (157 g) was dissolved in dry dichloromethane (2 L). Triethylamine (100 ml) and dic (75.7 g) were added and the mixture was stirred for 4 h at the room temperature. Compound 5 (45.7) in dichloromethane (500 mL) was added and the reaction mixture was stirred for 2 h. The mixture was washed with water (1 L) and concentrated.

The residue was purified by column chromatography. The desired product was eluted MeOH-CH2Cl2 (0:100-10:90). Evaporation of appropriate fractions gave the compound 7 as a solid (138.0 g).MS M-1: 558.2.

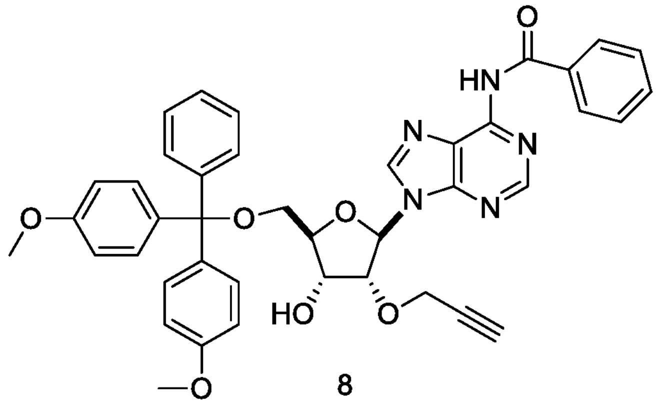

8

A mixture of 5'-DMT-N6-bz-Adenosine (551.7 g; 80 mmol), tetrabutylammonium bromide (283.2 g). dibutyltin oxide (DBTO) (238.4 g), and propargyl bromide (338 ml), and dry DMF (2.5), was stirred for 24 hrs at 50° C. The mixture was then poured onto crushed ice. The liquid was decanted. The gummy mass was dissolved in dichloromethane (5 L) and the organic layer was washed with distilled water three times. The organic layer was dried with sodium sulfate and then concentrated. The crude reaction mixture was purified on silica gel column using a gradient system of chloroform:hexane:acetone (50:40:10 to 50:30:20). The yield of pure 5'-DM T-2'-0-propargyl-N- bzA was 60.5 g as a solid MS M-1 710.2.

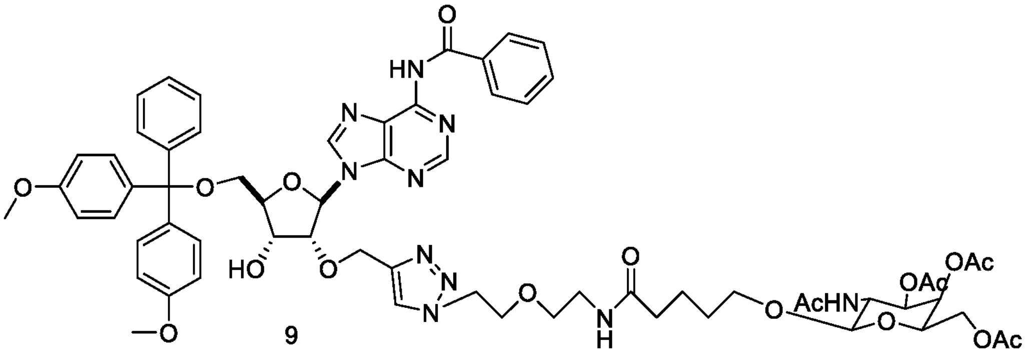

9

Compound 7 (71 g) and compound 8 (60.5 g) were dissolved in THF (1 L). CuSOS4.5H2O (2.1) and sodium ascorbate (2.0) were added under nitrogen. The mixture was stirred overnight. EDTA (5 g) in water (1 L) was added and stirred for 30 min. Toluene (1 L) was added and the two layers were separated. The organic layer was evaporated and residue was purified by silica gel chromatography. The desired product was eluted MeOH-CH2Cl2 (0:100-10:90). Evaporation of appropriate fractions gave the compound 9 as a solid (91.0 g).MS M-1: 1269.6.

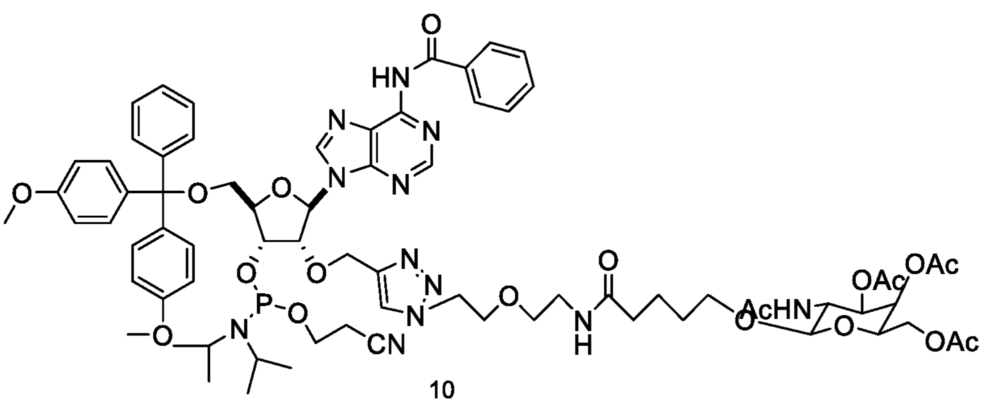

10

The dimethoxytritylated derivative 9 (40.0 g) was dissolved in 600 ml dichloromethane under argon and ethyl¬(di-isopropyl)amine (10 ml) and 2-cyanoethyl diisopropylphosphoramidochloridite (15.0 g) were added. After stirring the solution for 3 h, TLC indicated complete re¬action. A 10% aqueous solution of sodium hydrogen carbonate (2 ml) was added, the solution was stirred for 10 min, and parti¬tioned between methylene chloride (500 ml) and aqueous sodium carbonate (300 ml). The organic phase was washed with aqueous sodium chloride (2x300 ml) and the aqueous phases were back extracted with methylene chloride (200 ml). Evaporation of the organics left an oil, which was flash purified on silica gel (ethyl acetate) to afford the product as a foam (34.5 g) (MS M-1: 1469.6).

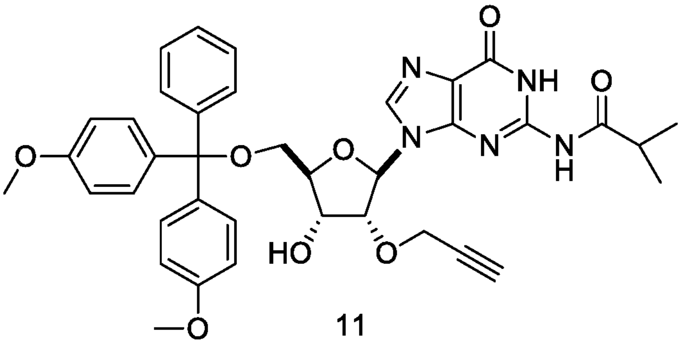

11

A mixture of 5'-DMT-N2-ibuG (328 g), tetrabutylammonium bromide (177 g), DBTO (150 g), propargyl bromide (420 ml) and dry DMF (1.3 L) was stirred for 24 hrs at 50c C. The mixture was then poured onto crushed ice. The liquid was decanted. The gummy mass was dissolved in dichloromethane (5 L) and the organic layer was washed with distilled water three times. The organic layer was dried with sodium sulfate and then concentrated. The residue was purified on silica gel column using a gradient system of chloroform:hexane:acetone:methanol (50:30:20:0 to 50:30:20:2). The yield of pure 5'-DMT-2'-0-propargyl-N2- ibu-G was 45.0 g as a solid MS M-1 692.2.

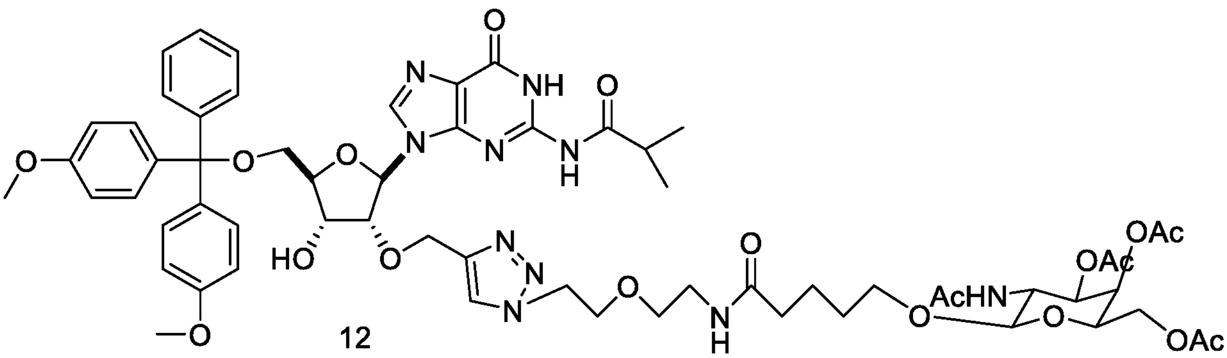

12

Compound 7 (18.0 g) and compound 11 (15.0 g) were dissolved in THF (300 ml). CuSOS4.5H2O (0.6 g) and sodium ascorbate (0.5 g) were added under nitrogen. The mixture was stirred overnight. EDTA (2.0 g) in water (0.5 L) was added and stirred for 30 min. Toluene (0.5 L) was added and the two layers were separated. The organic layer was evaporated and residue was purified by silica gel chromatography. The desired product was eluted MeOH-CH2C12 (0:100-10:90). Evaporation of appropriate fractions gave the compound 9 as a solid (24.0 g).MS M-1: 1251.6.

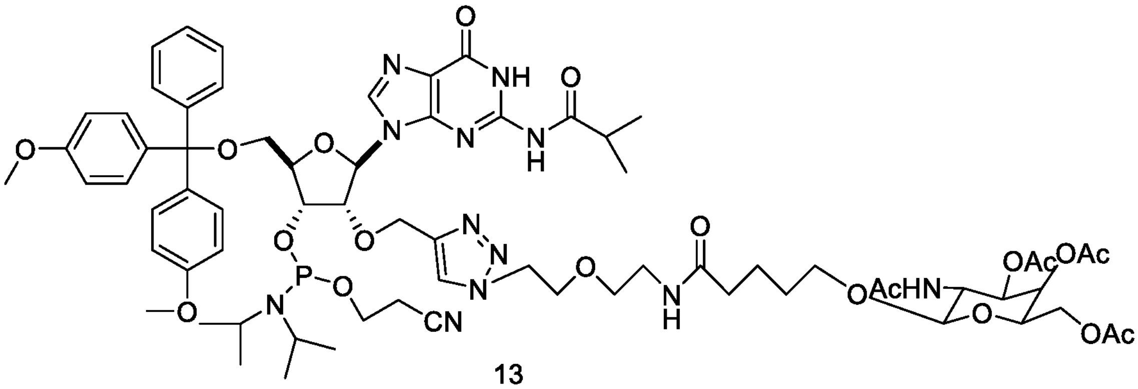

13

The compound 12 (24 g) was dissolved in 500 ml dichloromethane under argon and ethyl-(diisopropyl)amine (8 ml) and 2-cyanoethyl diisopropylphosphoramidochloridite (10.0 g) were added. After stirring the solution for 3 h, TLC indicated complete re¬action. A 10% aqueous solution of sodium hydrogen carbonate (2 ml) was added, the solution was stirred for 10 min, and part¬tioned between methylene chloride (500 ml) and aqueous sodium carbonate (300 ml). The organic phase was washed with aqueous sodium chloride (2x300 ml) and the aqueous phases were back extracted with methylene chloride (200 ml). Evaporation of the or¬ganics left an oil, which was flash purified on silica gel (ethyl acetate) to afford the product as a foam (19.5 g) (MS M-1: 1451.6).

The following compounds are made using the same protocols for the synthesis of compound 13 with slight variations.

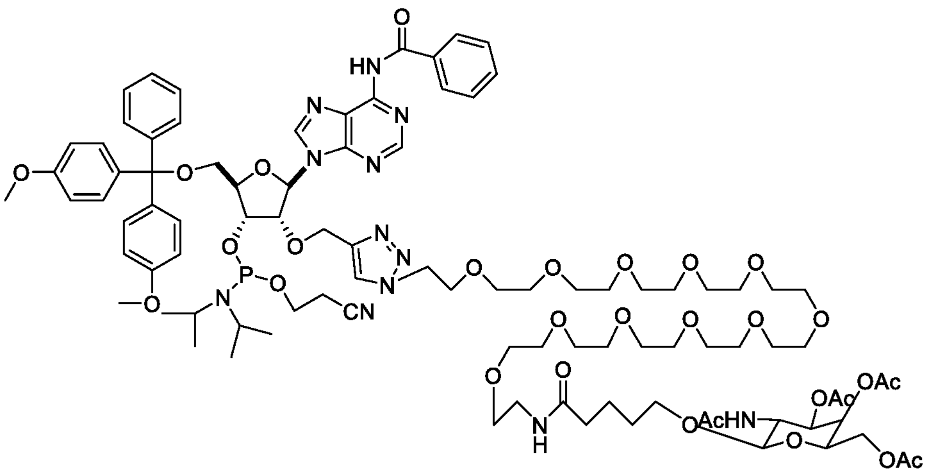

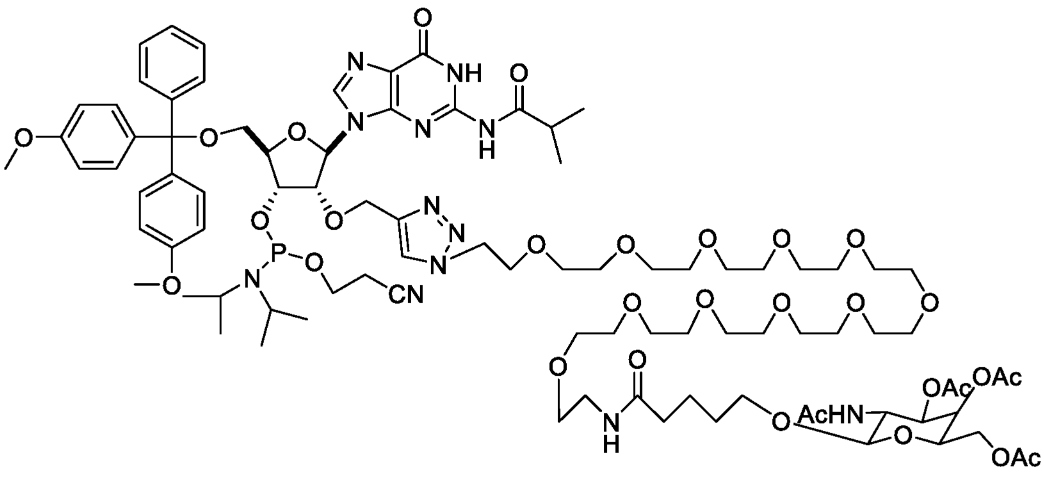

Scheme I

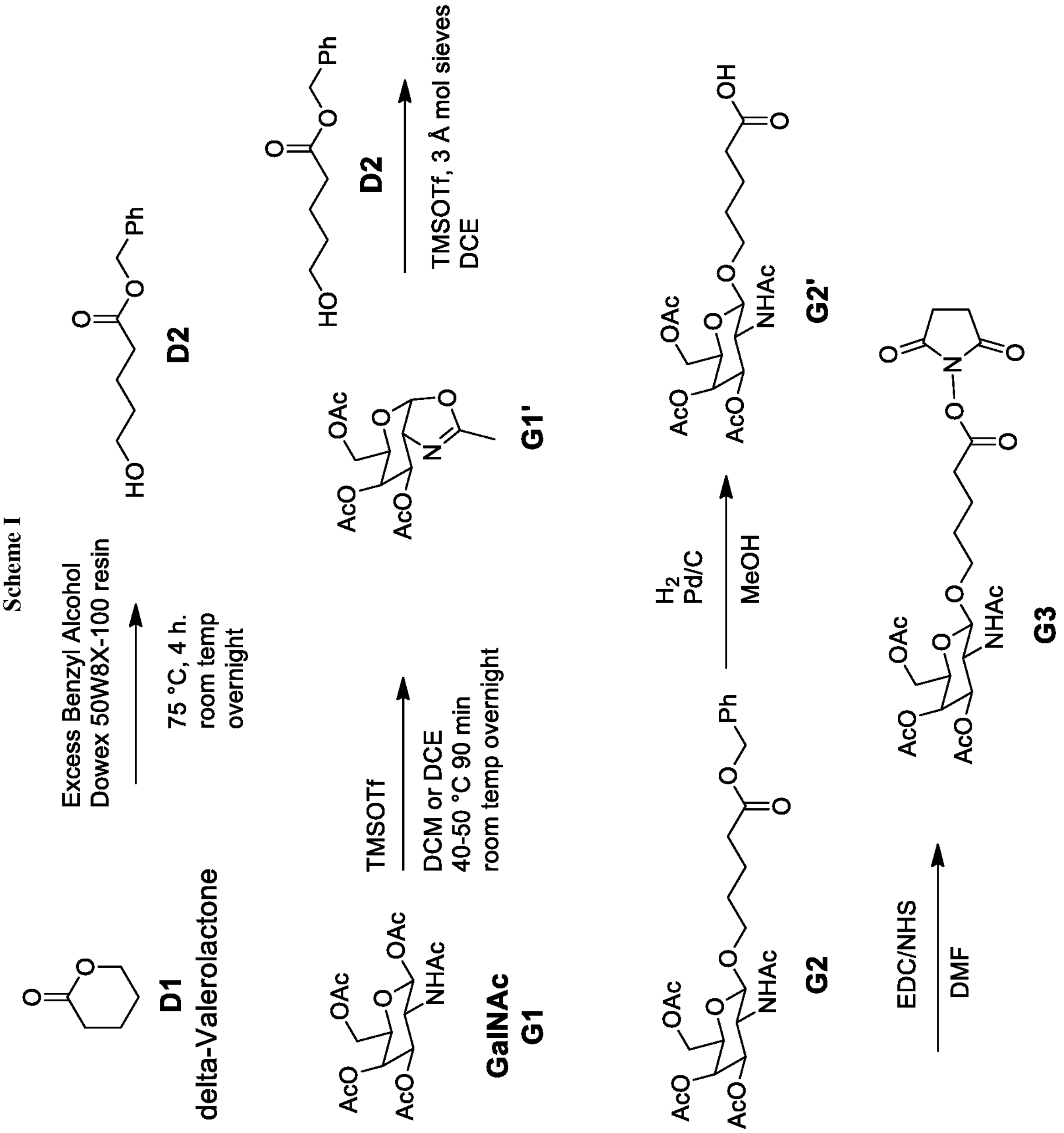


D1 delta-Valerolactone

D2

GalNAc G1

G1'

D2

G2

G2'

G3

Scheme II

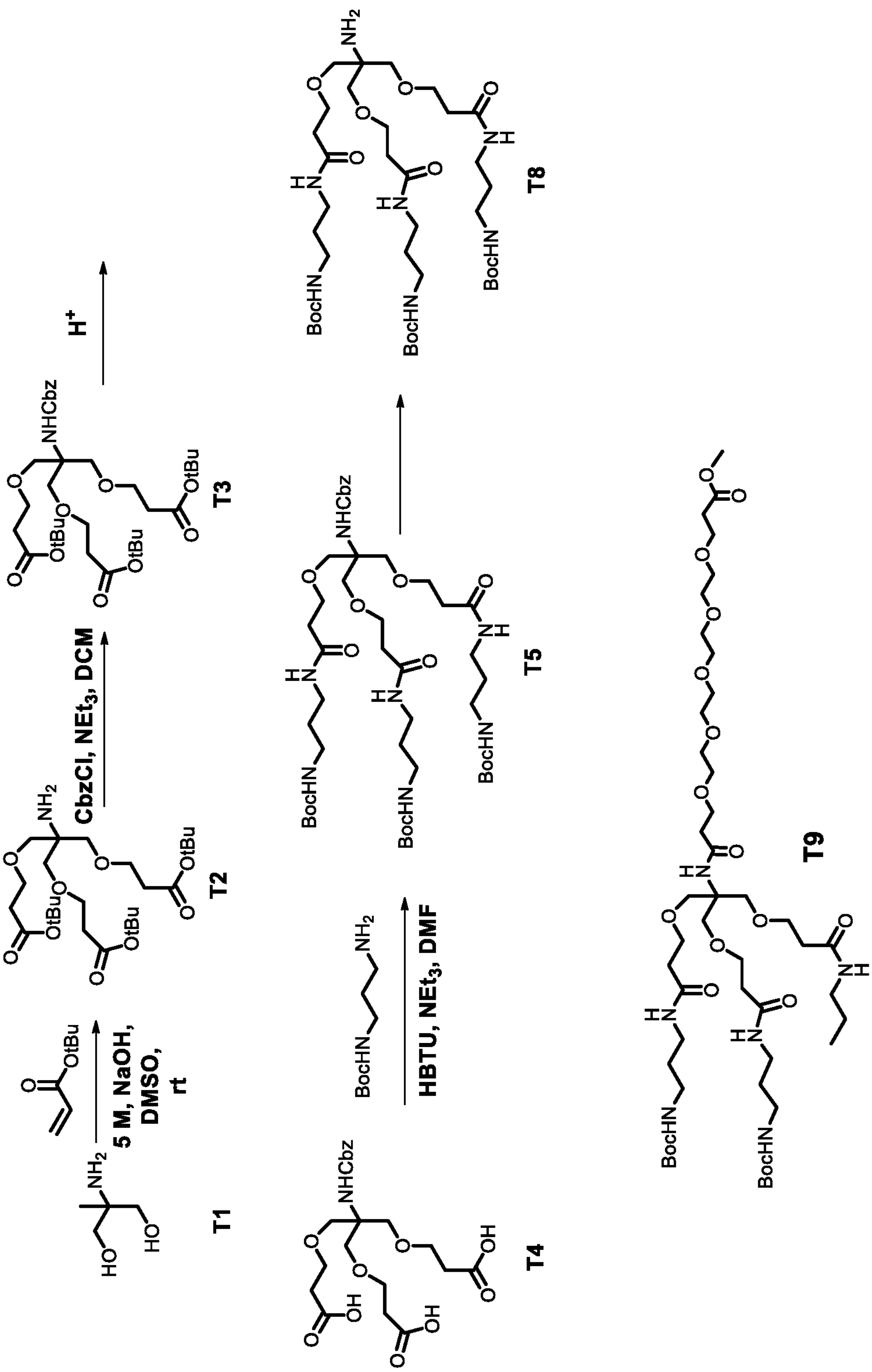


T1 T2 T3 T4 T5 T8 T9

Scheme II continued

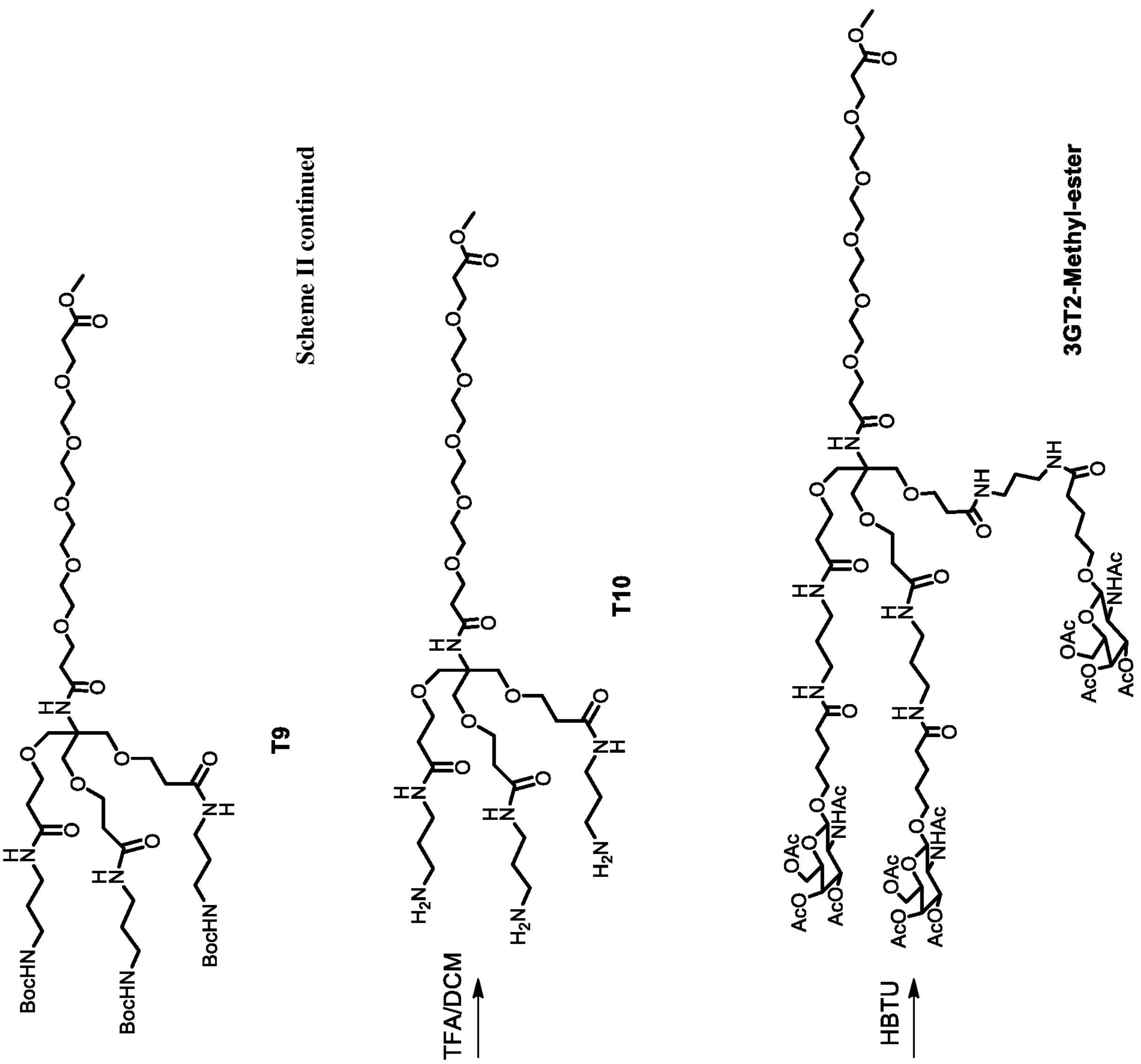


T9

T10

3GT2-Methyl-ester

Scheme II continued

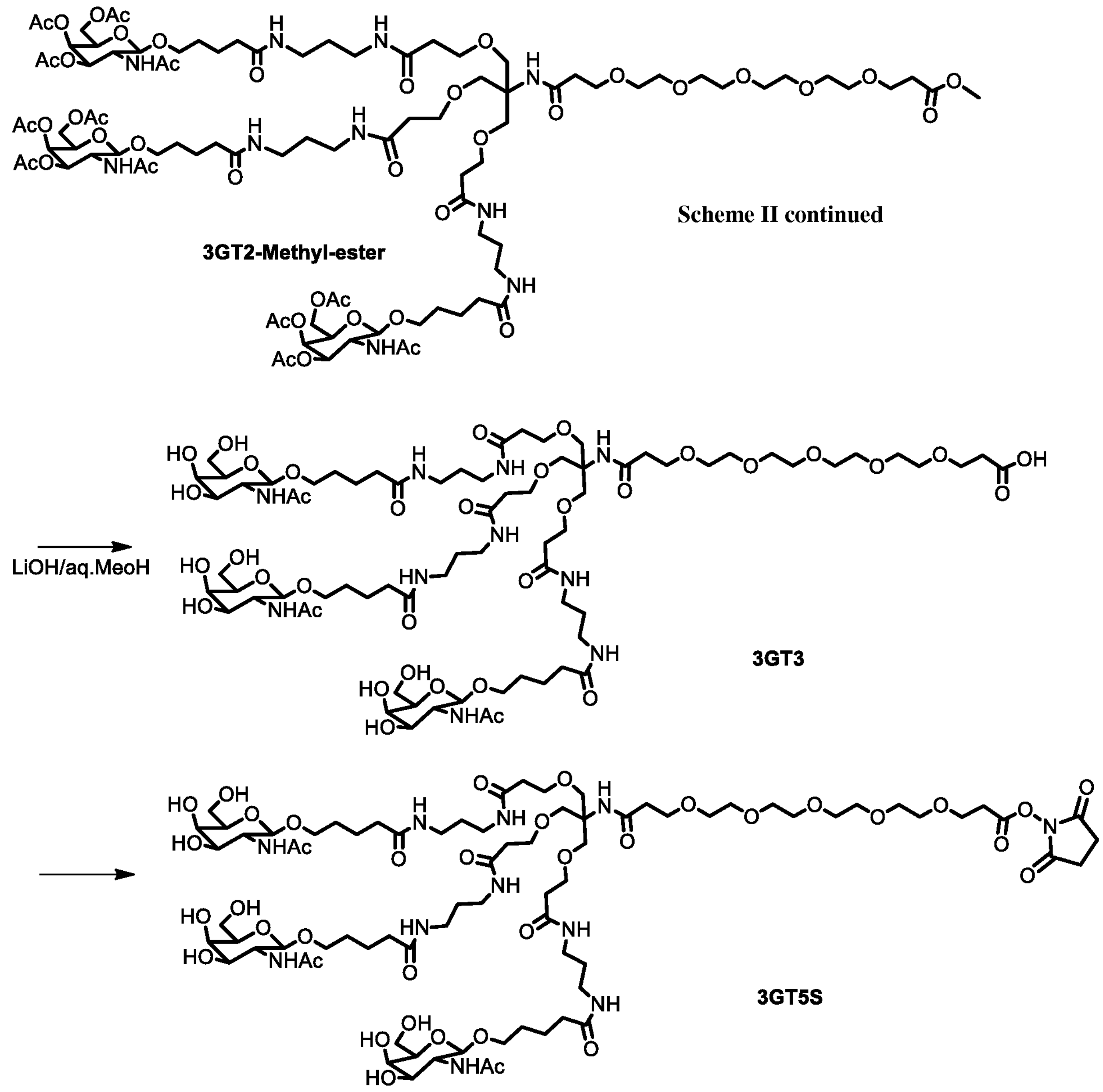


3GT2-Methyl-ester

3GT3

3GT5S

Scheme III

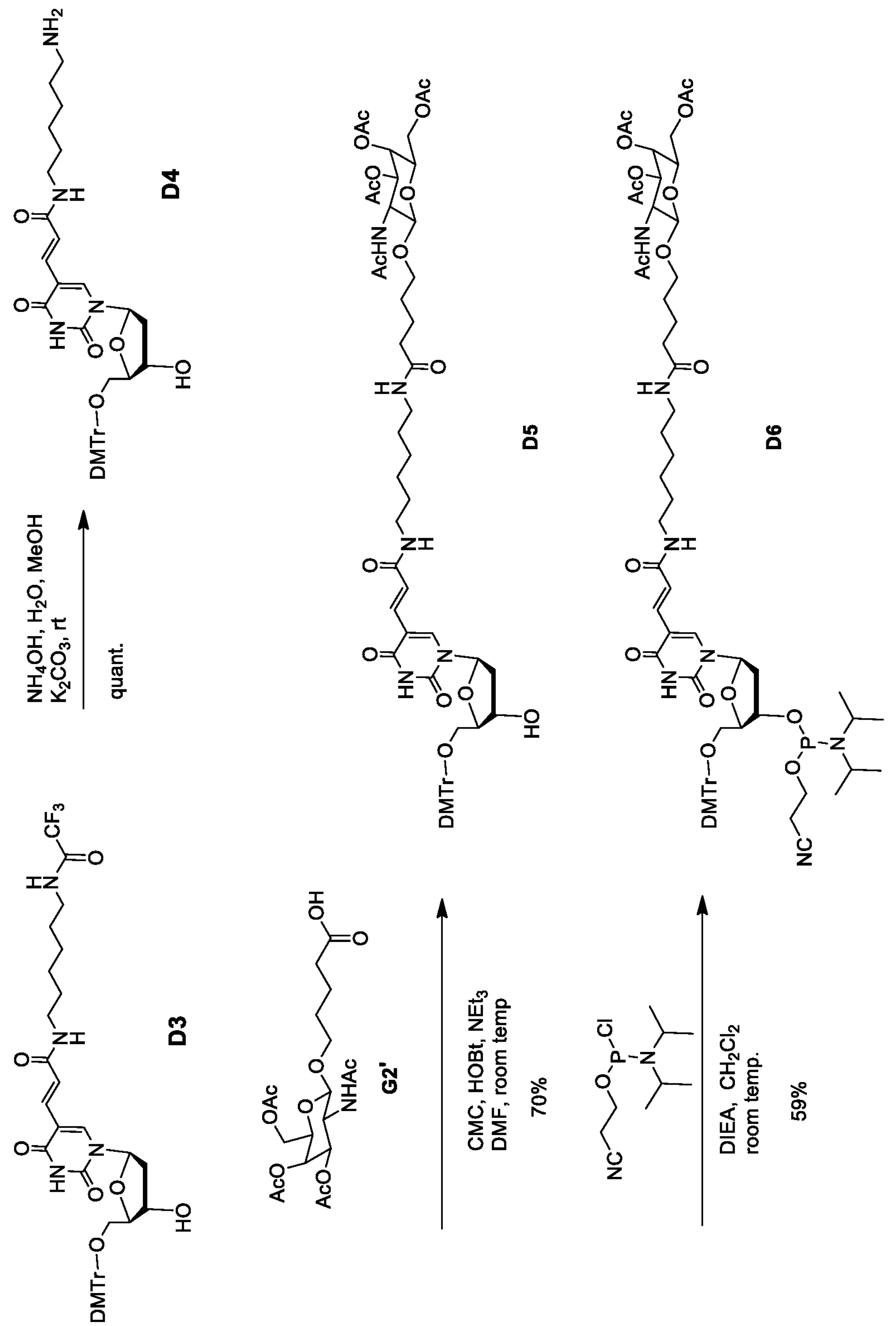


D3

D4

G2'

D5

D6

dTC6-amine-N-acetylgalactosamine-amidite

## Scheme IV

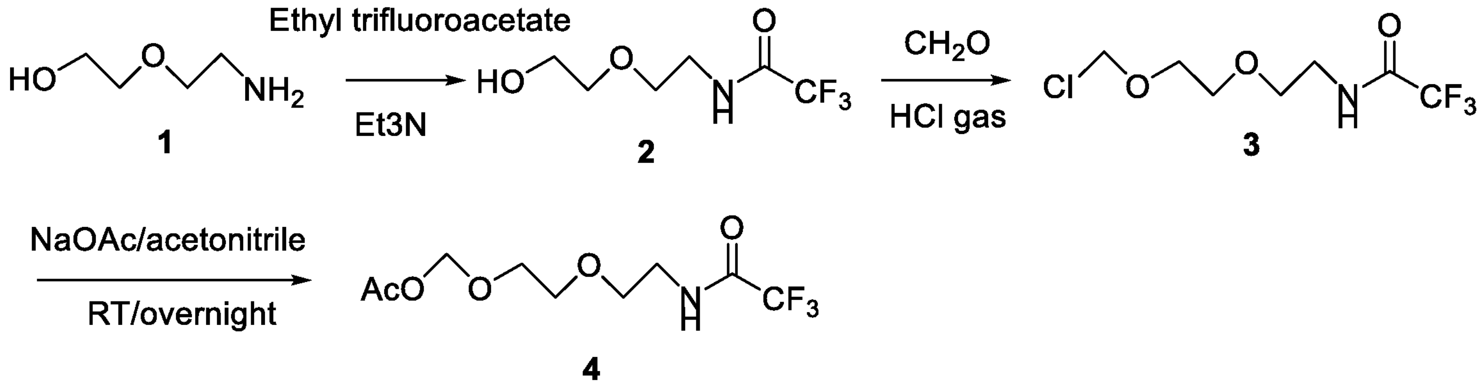


1 2 3 4

## Scheme VI

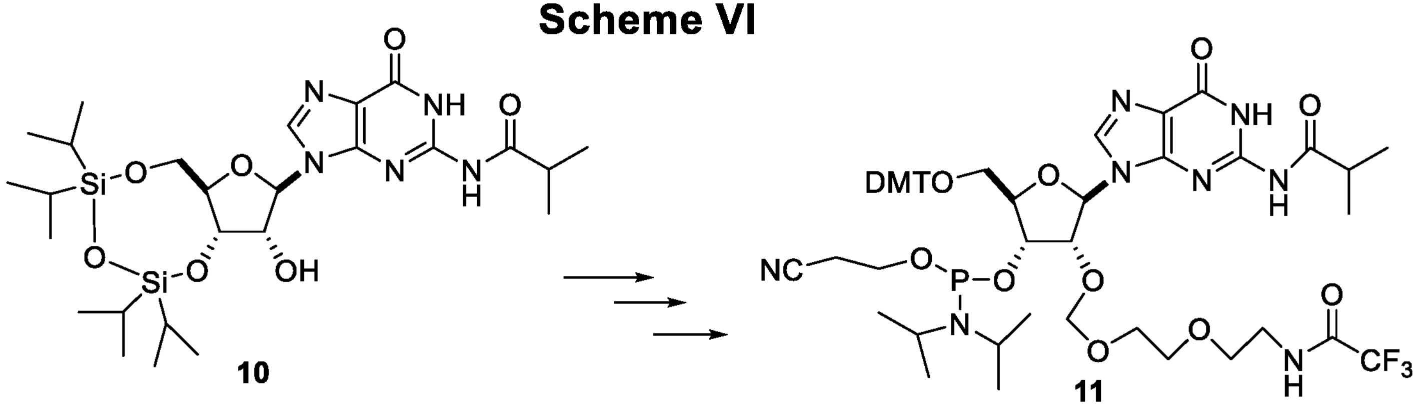

10 11

**Scheme V**

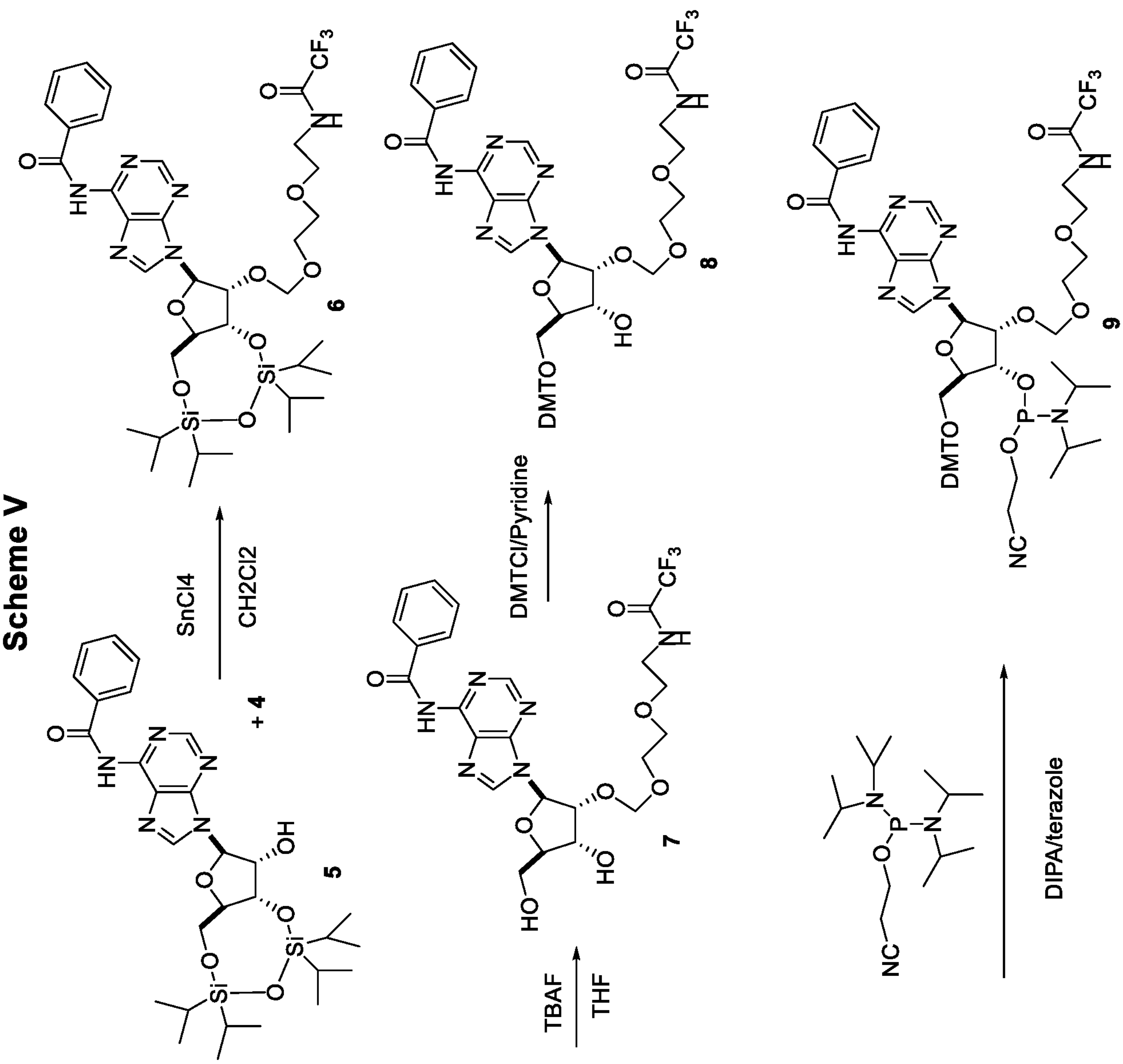


5 6 7 8 9

Scheme VI

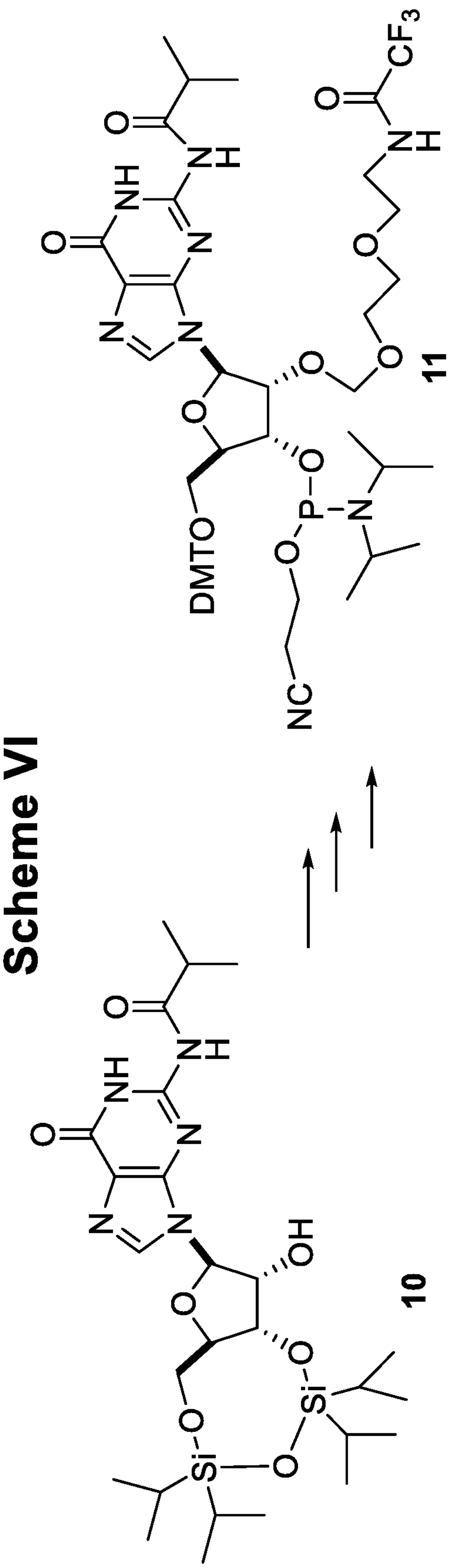

10

11

**Scheme VII**

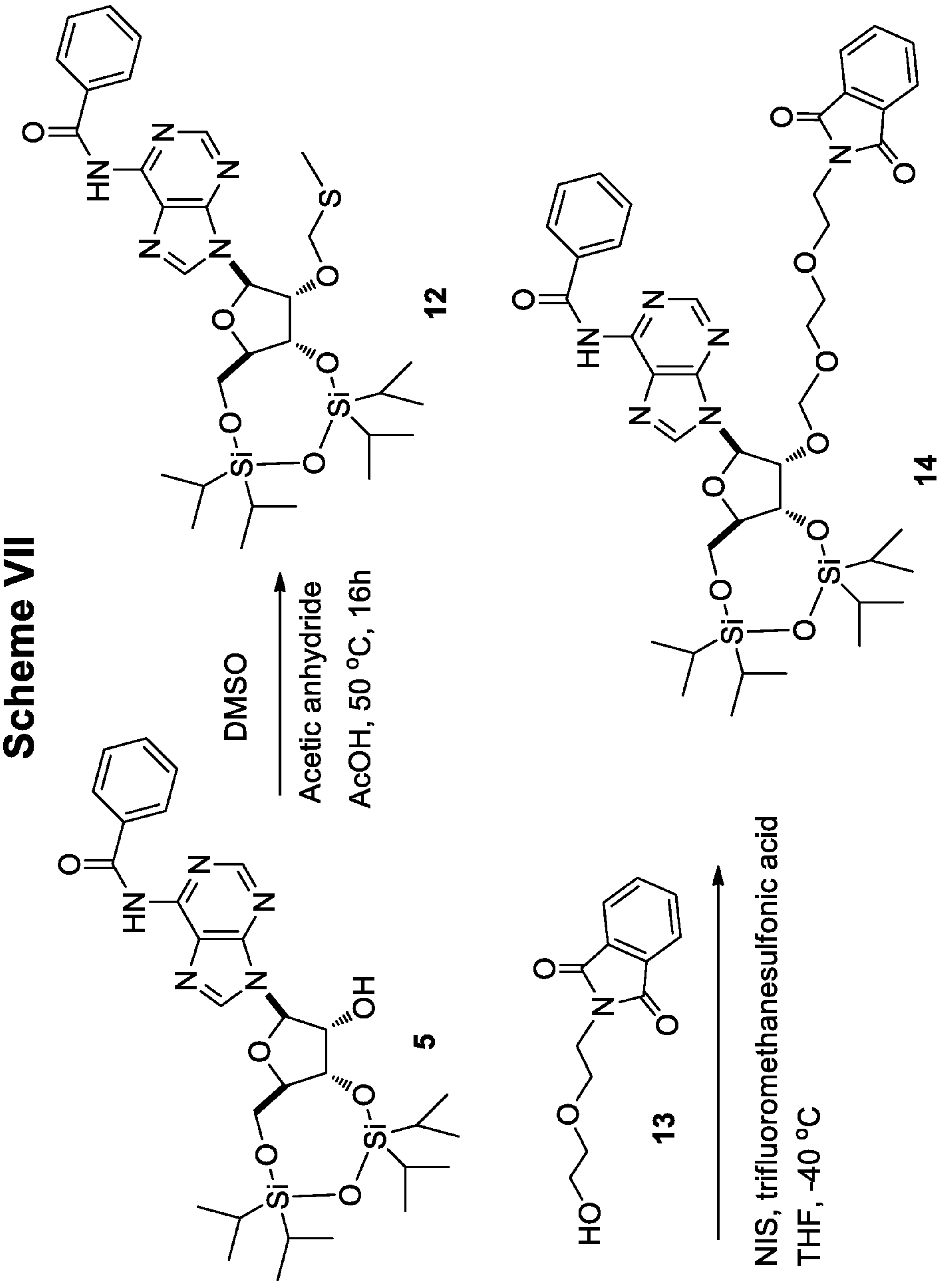


5

12

13

14

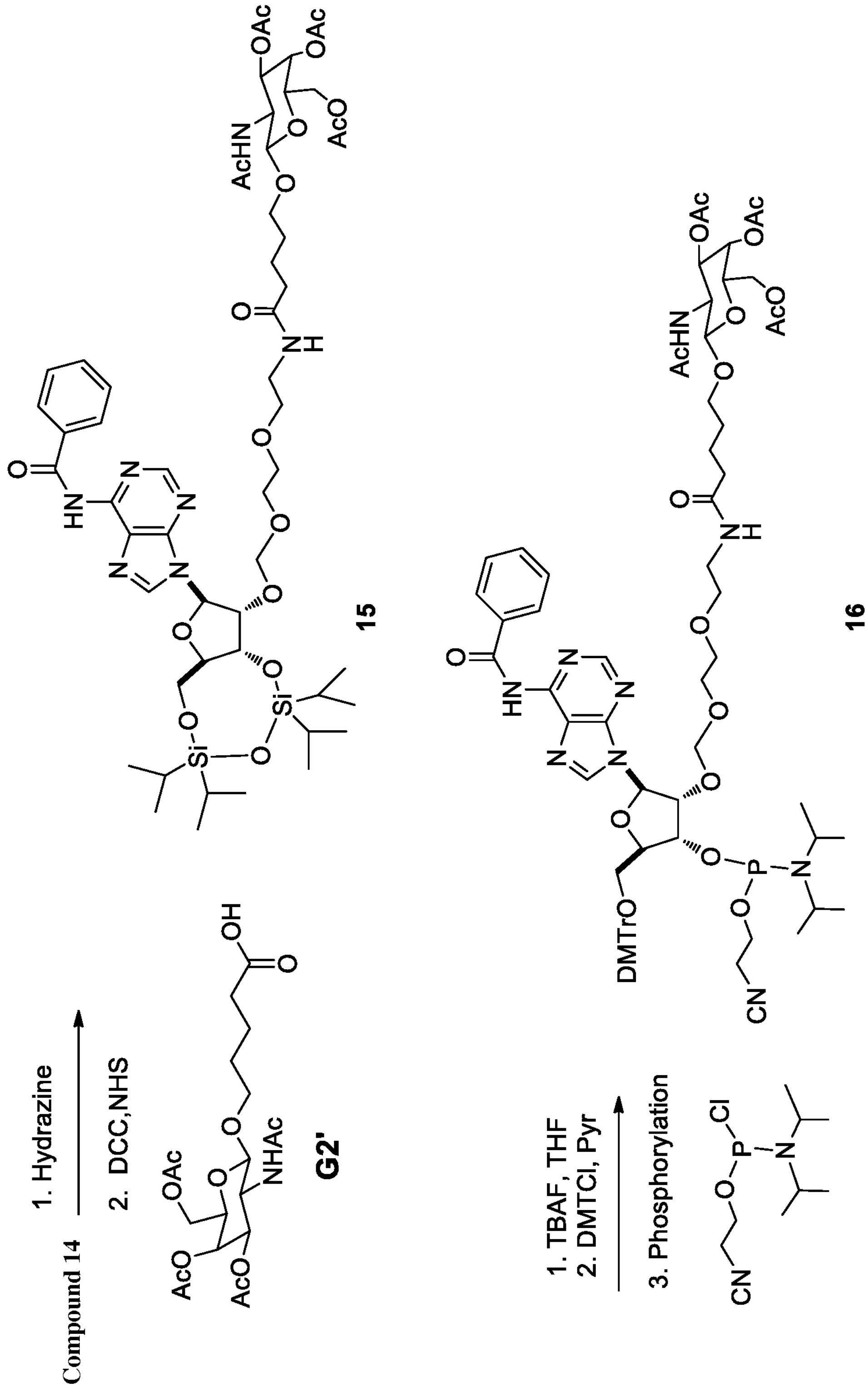
Compound 14
1. Hydrazine
2. DCC,NHS
1. TBAF, THF
2. DMTCl, Pyr
3. Phosphorylation

G2'

15

16

## Scheme VIII

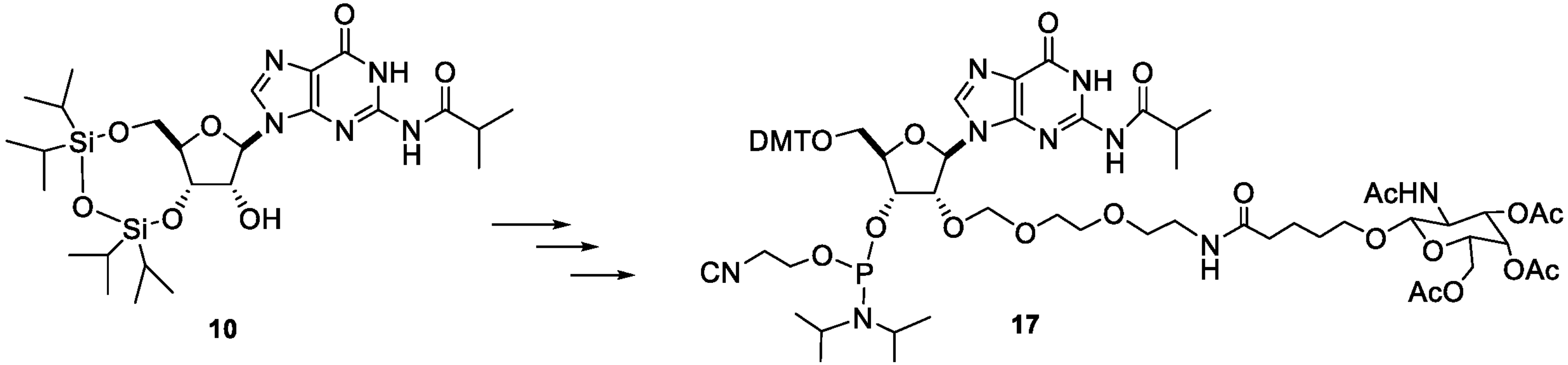

10

17

Compound 17 was made in the same way

**Scheme IX**

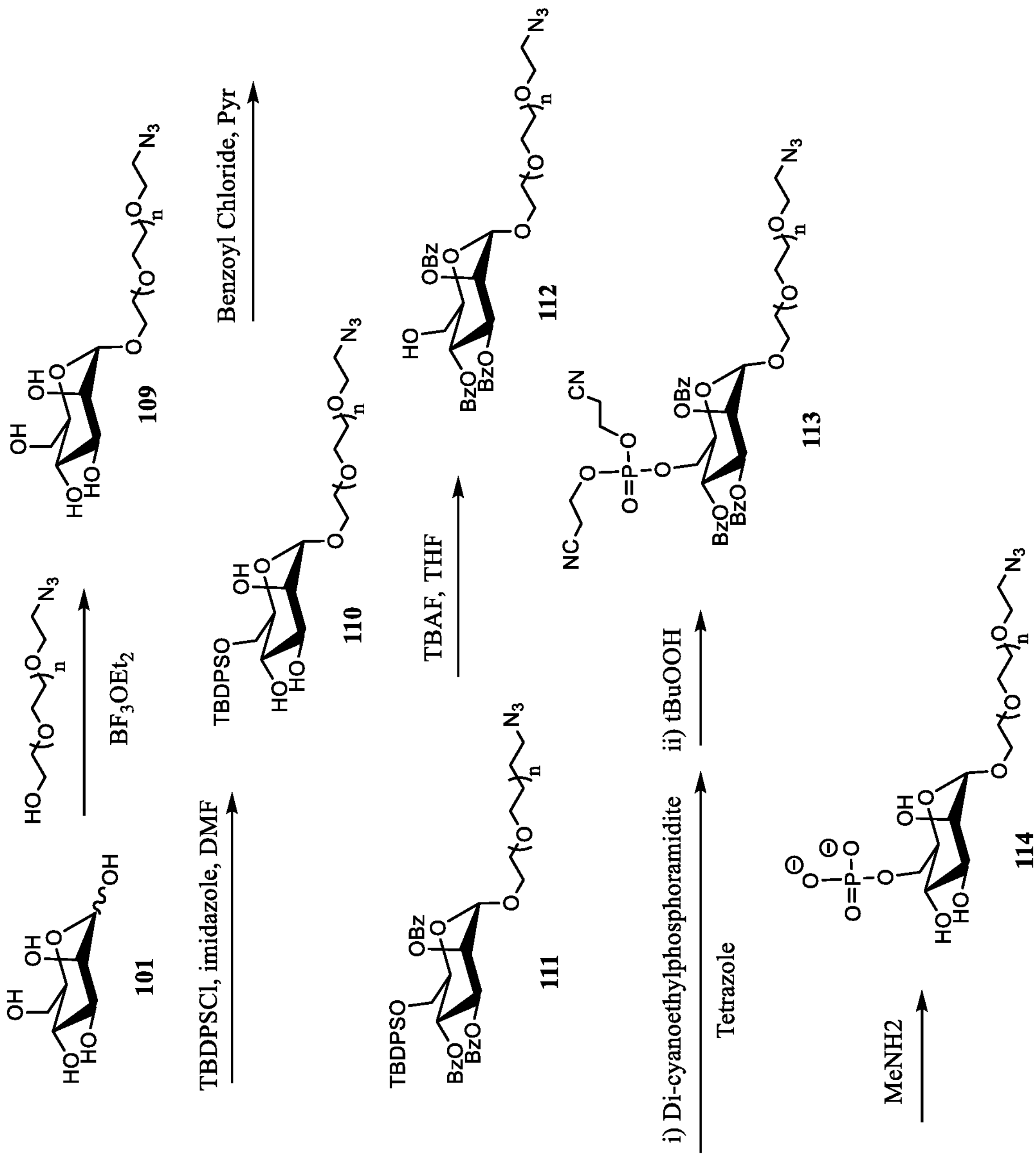


101 109 110 111 112 113 114

## Scheme X

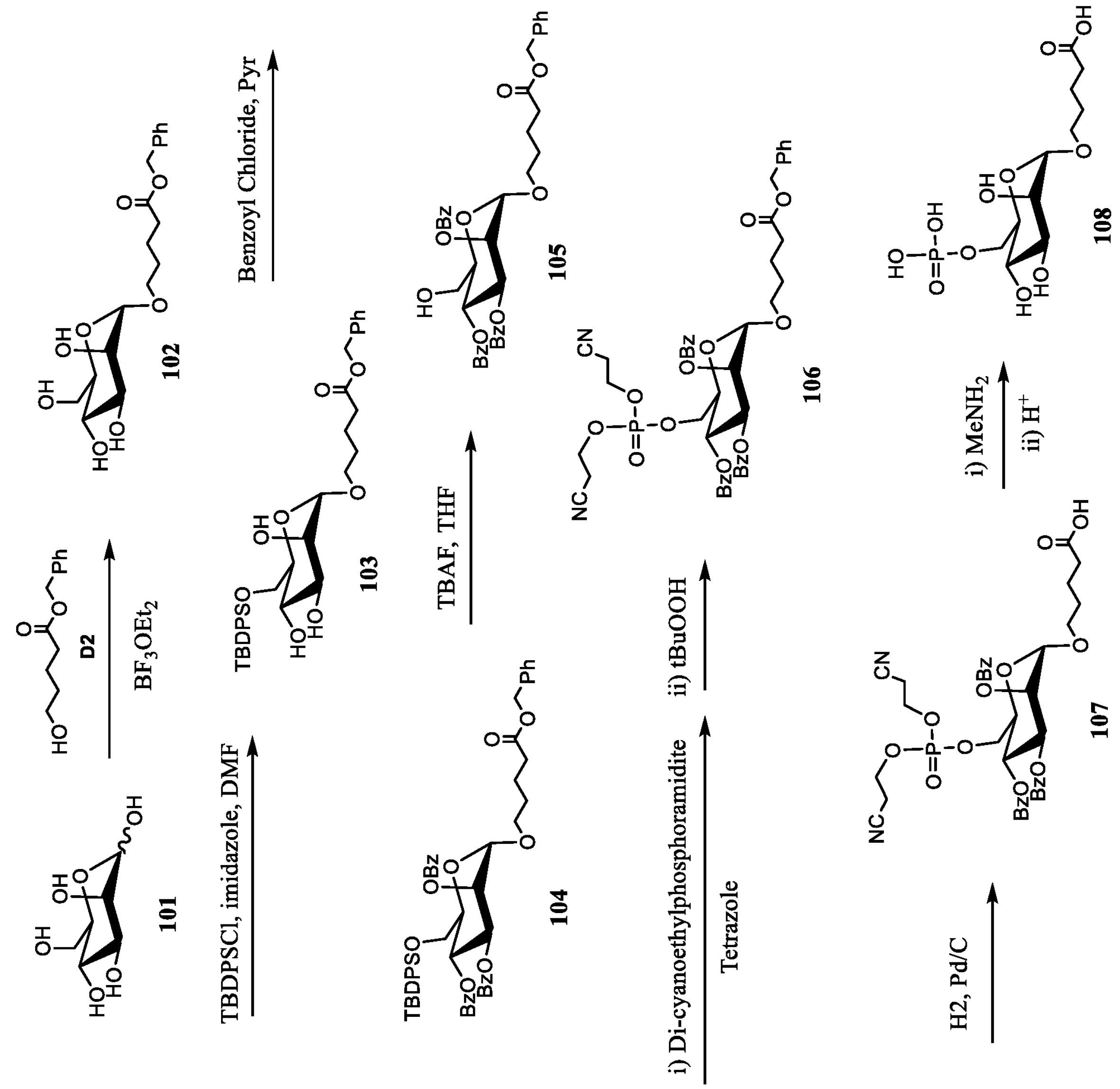
101
D2
$BF_3OEt_2$
102
TBDPSCl, imidazole, DMF
103
Benzoyl Chloride, Pyr
104
TBAF, THF
105
i) Di-cyanoethylphosphoramidite
Tetrazole
ii) tBuOOH
106
H2, Pd/C
107
i) $MeNH_2$
ii) $H^+$
108

# Scheme X (continued)

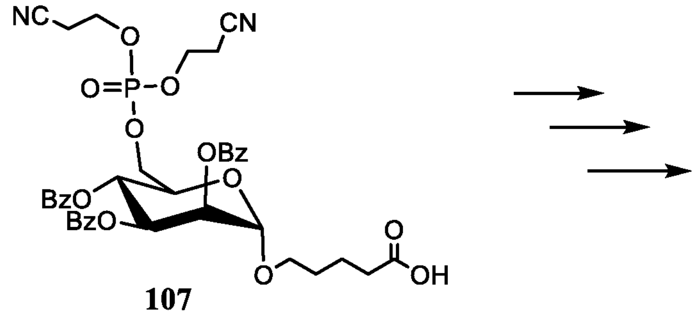

**107**

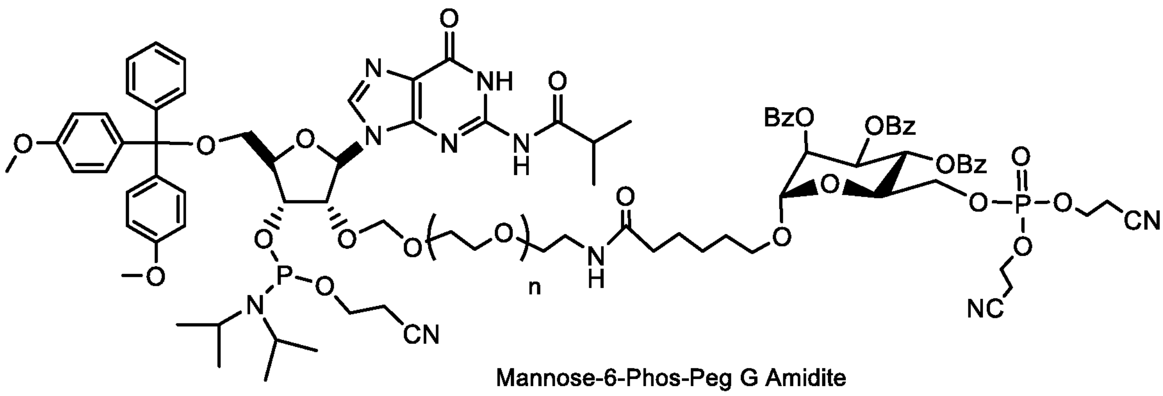

Mannose-6-Phos-Peg G Amidite

n = 1 or 8

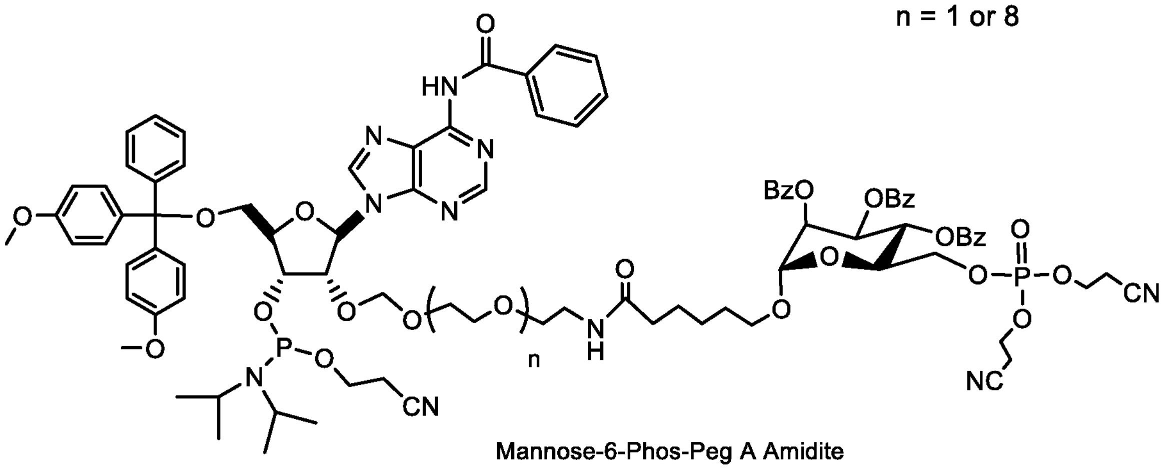

Mannose-6-Phos-Peg A Amidite

**Scheme XI**

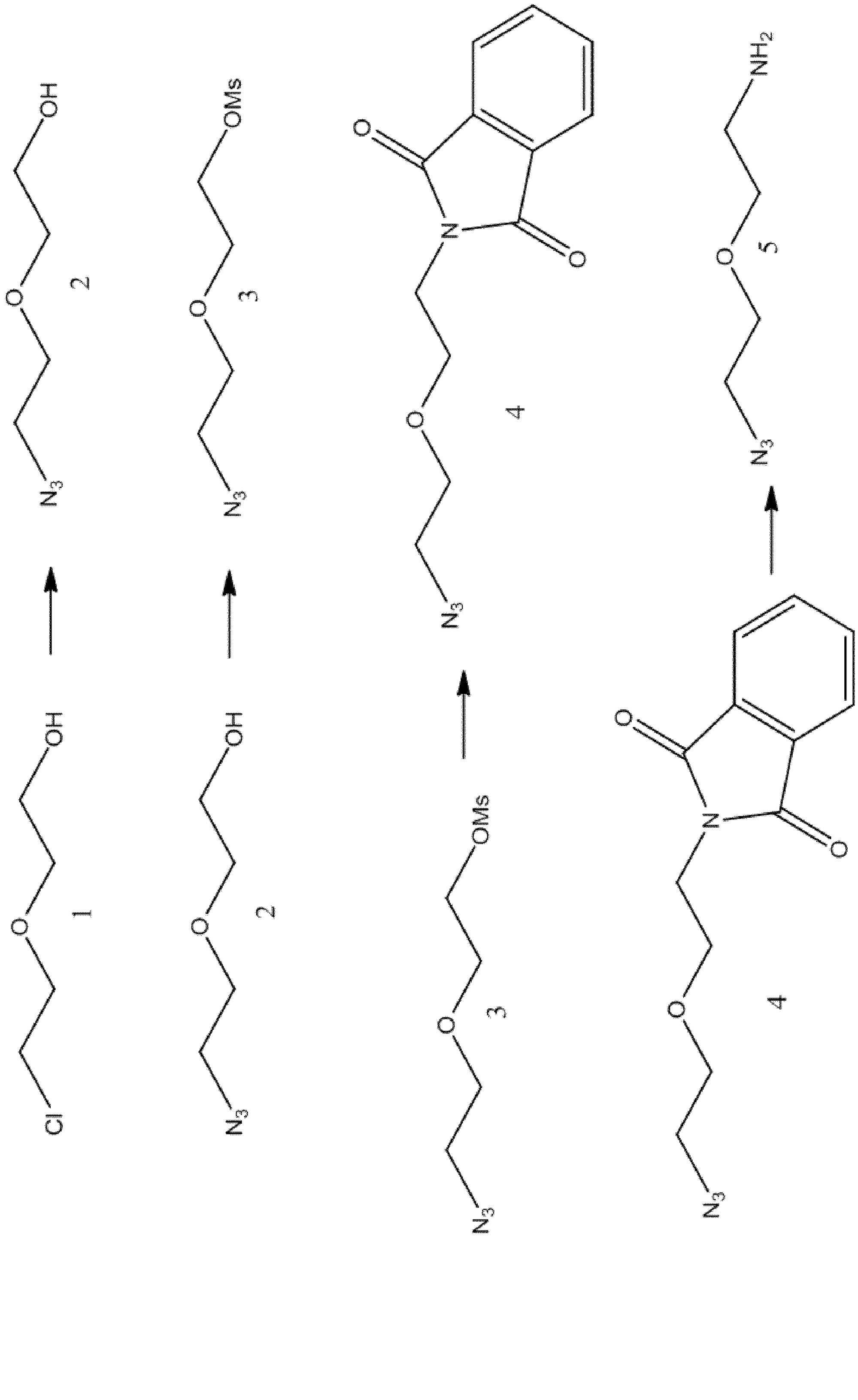

1 2 2 3 3 4 4 5

**SCHEME A: Synthesis of GalNAc Nicked Tetraloop dsNA Conjugate with Multiple GalNAc on the loop Post Synthetic Conjugation Using "Click" Chemsitry**

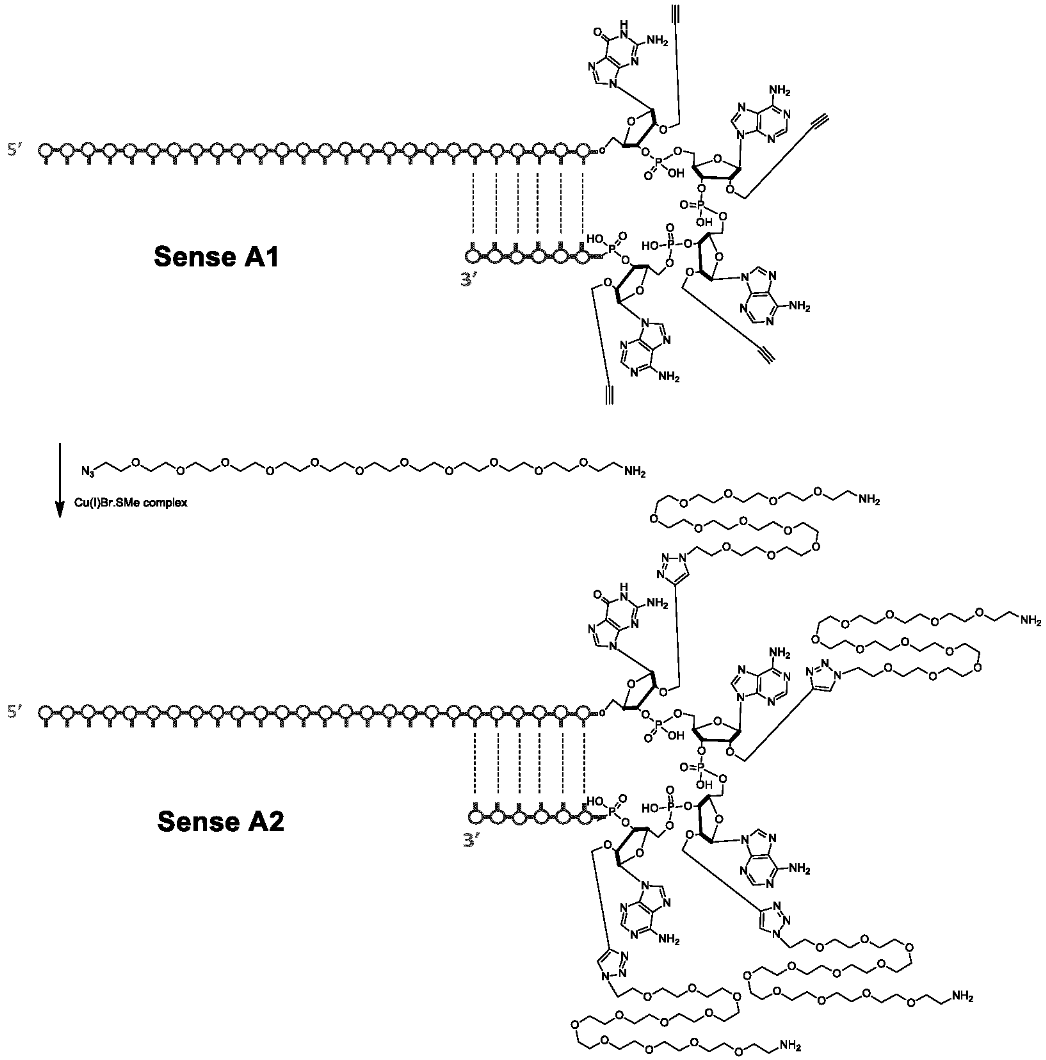


Sense A1

Sense A2

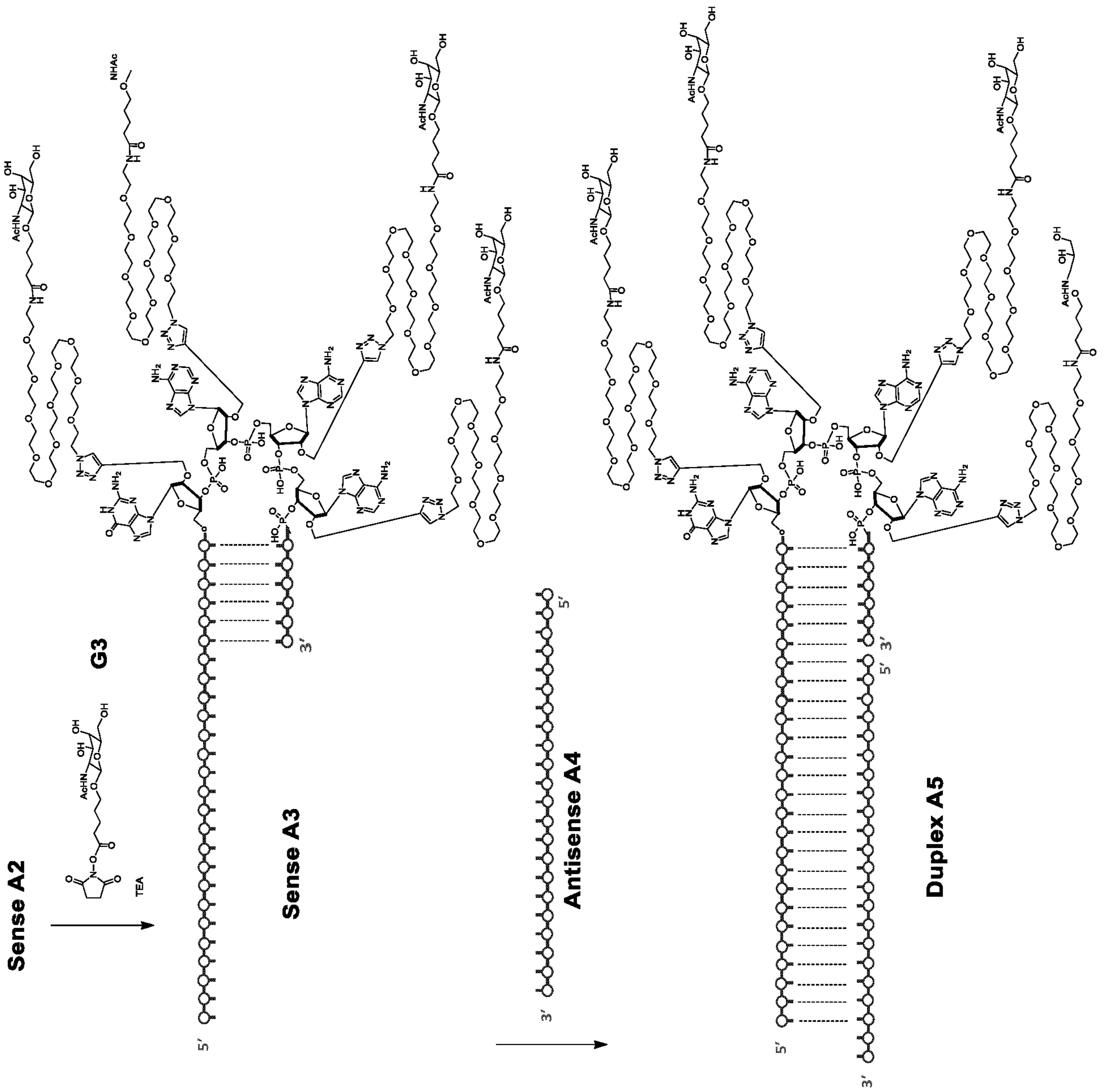


G3

Sense A2

Sense A3

Antisense A4

Duplex A5

**SCHEME A1: Synthesis of GalNAc Nicked Tetraloop dsNA Conjugate with Multiple GalNAc on the loop Post Synthetic Conjugation Using Amide Formation**

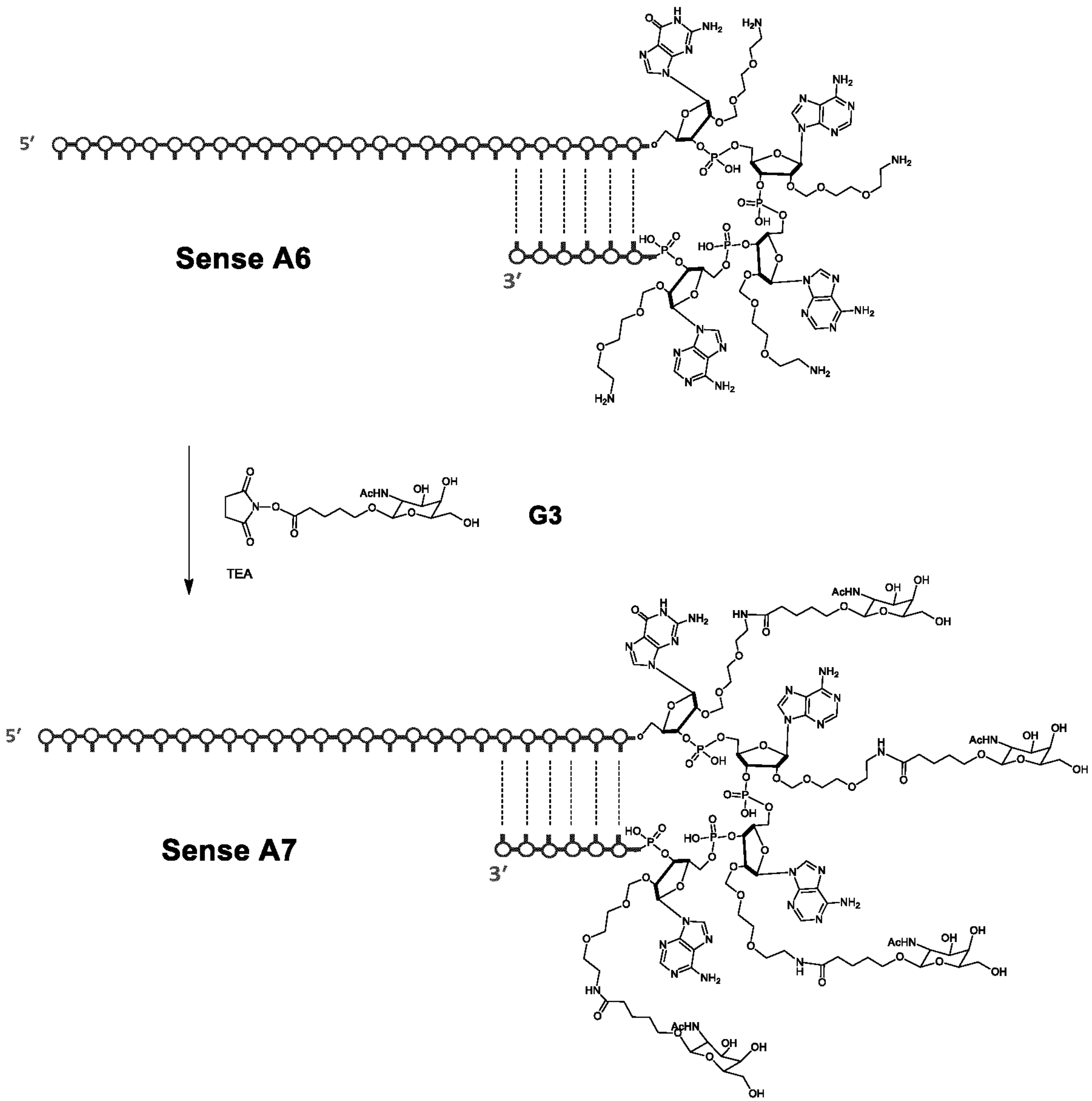

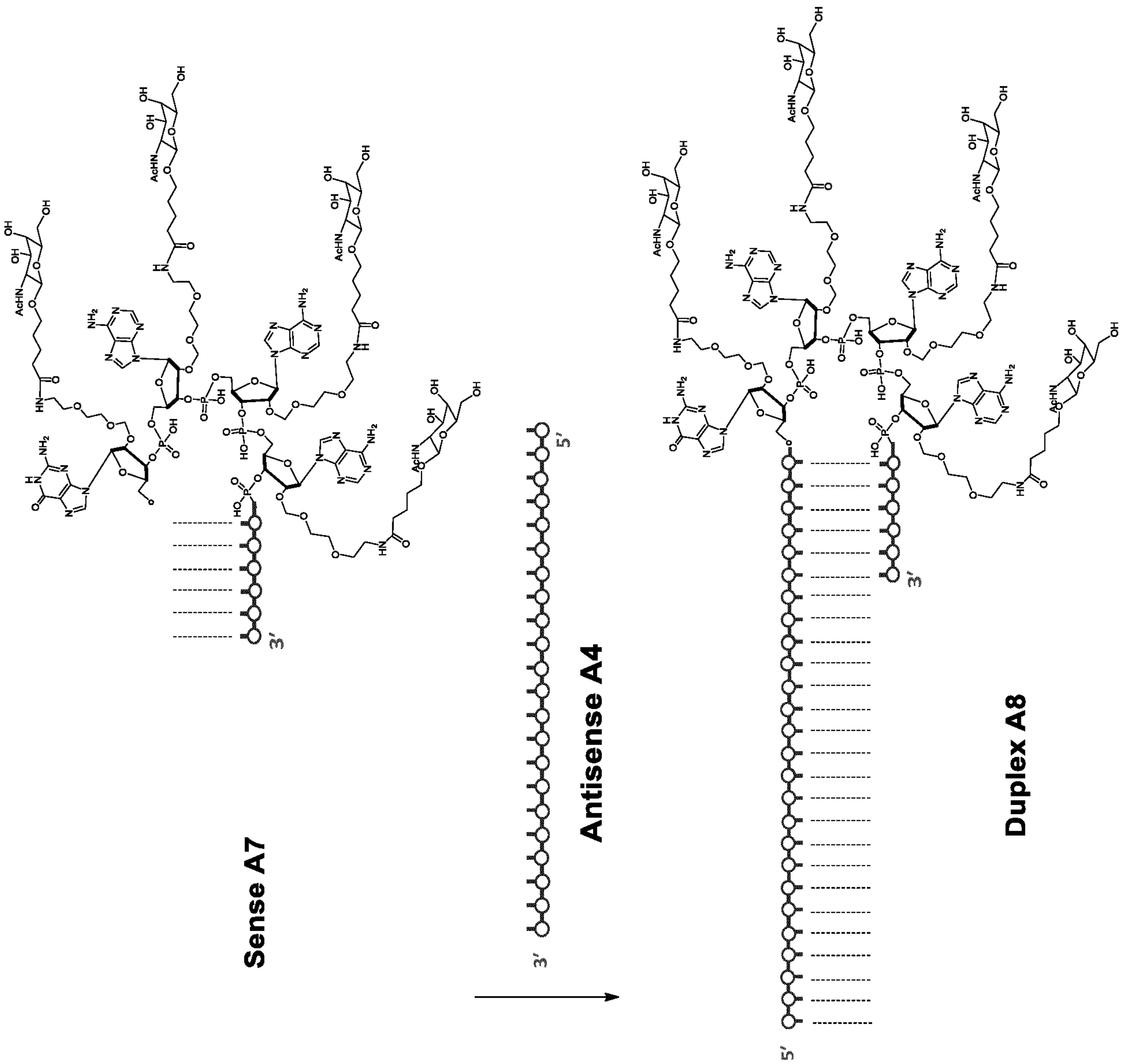

Sense A7

Antisense A4

Duplex A8

**SCHEME A2: Solid Phase Synthesis of GalNAc Nicked Tetraloop dsNA Conjugate Using 2'- Triazole Linked GalNAc Nucleoside Phosphoroamidites**

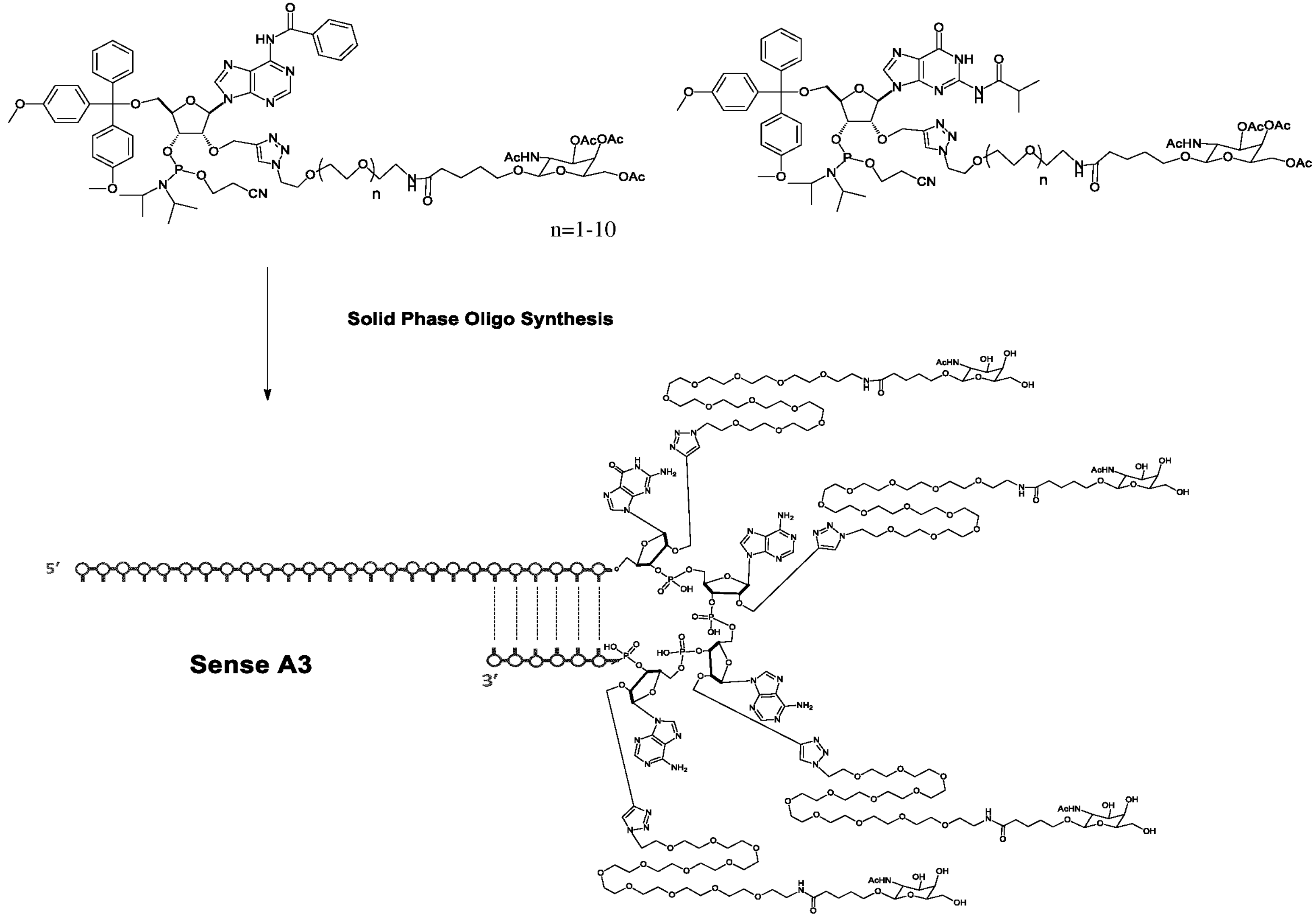


n=1-10

Sense A3

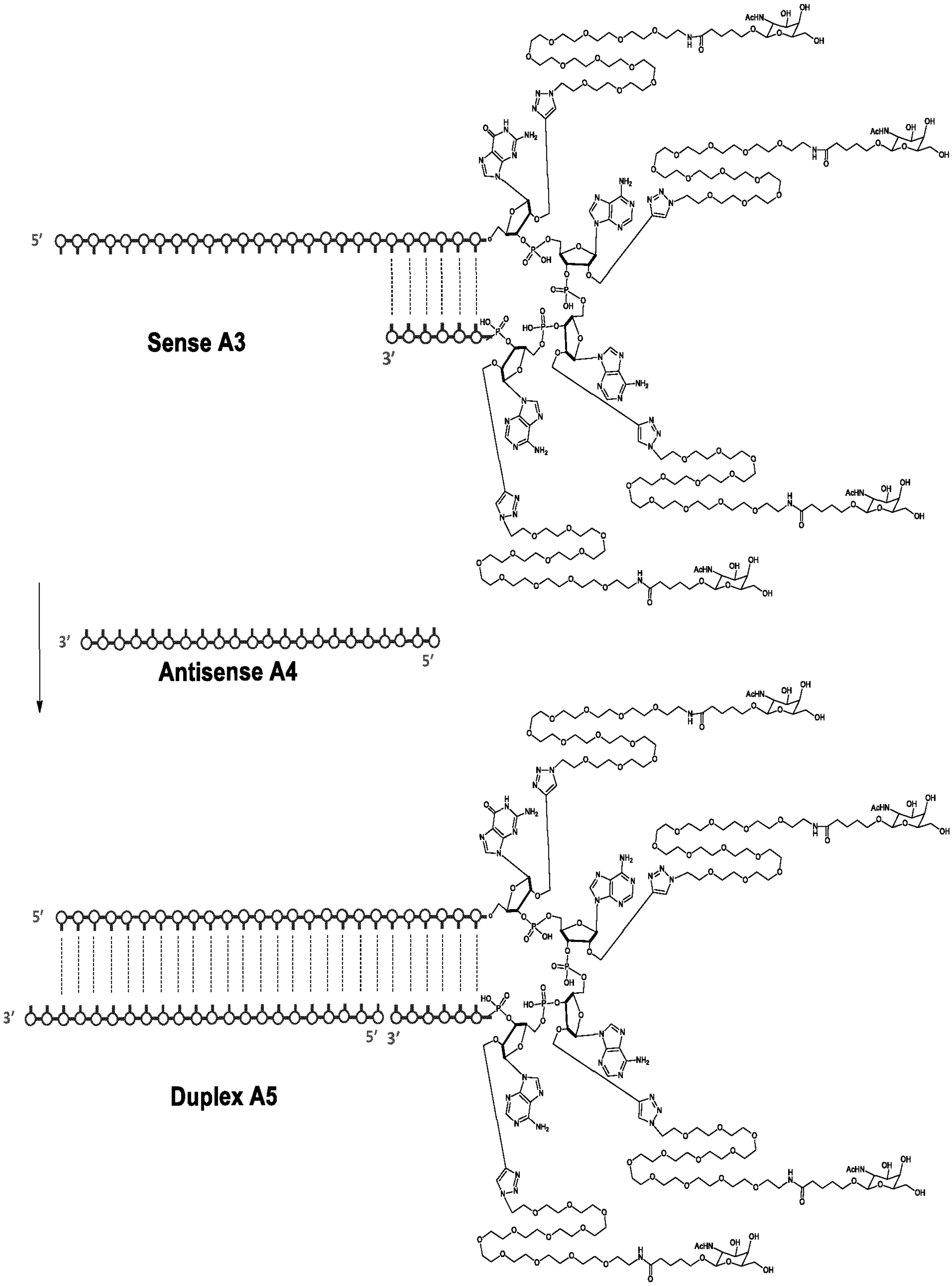

Sense A3

Antisense A4

Duplex A5

**SCHEME A3: Solid Phase Synthesis of GalNAc Nicked Tetraloop dsNA Conjugate Using 2'- Acetal Linked GalNAc Nucleoside Phosphoroamidites**

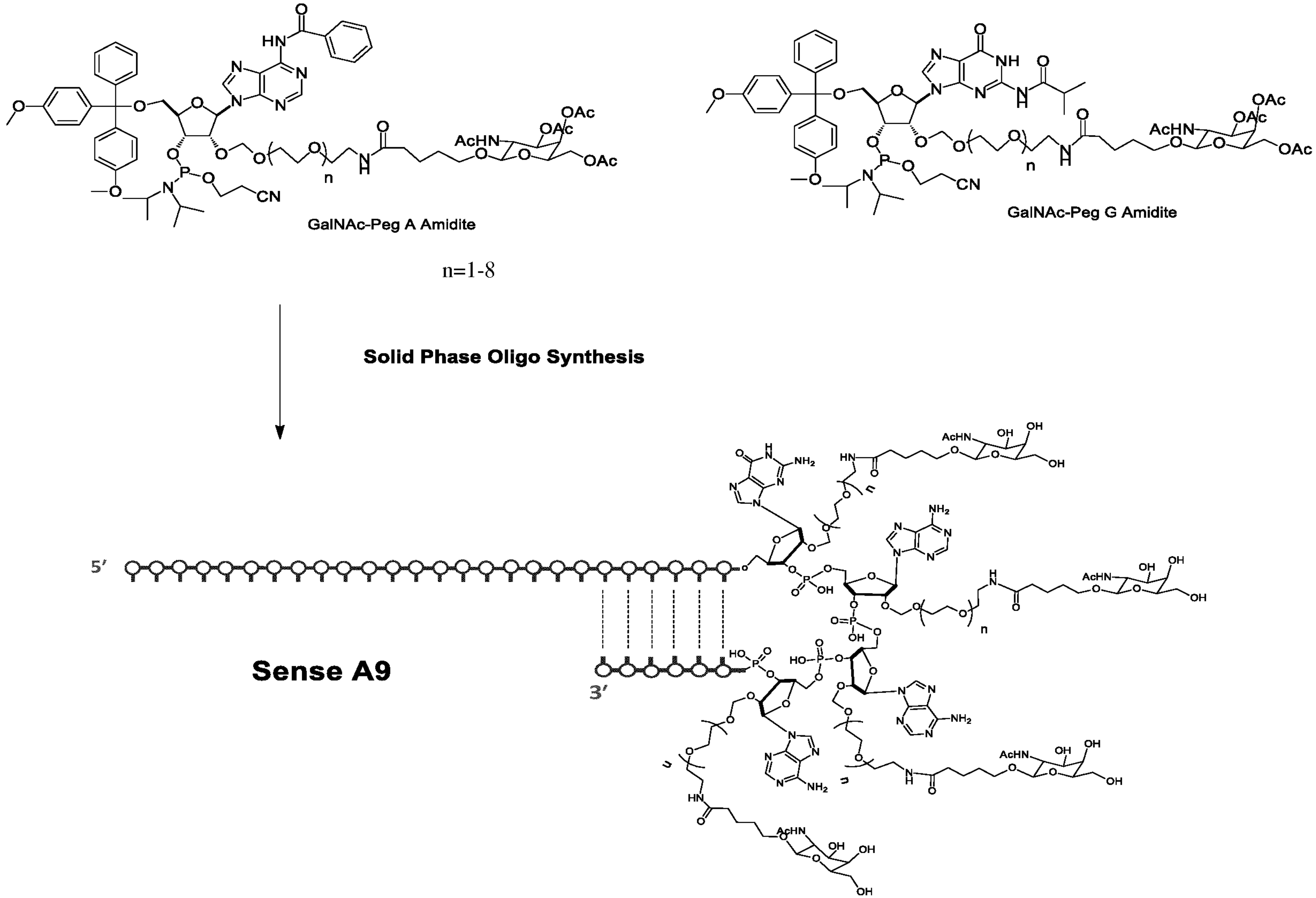


GalNAc-Peg A Amidite

GalNAc-Peg G Amidite

n=1-8

**Sense A9**

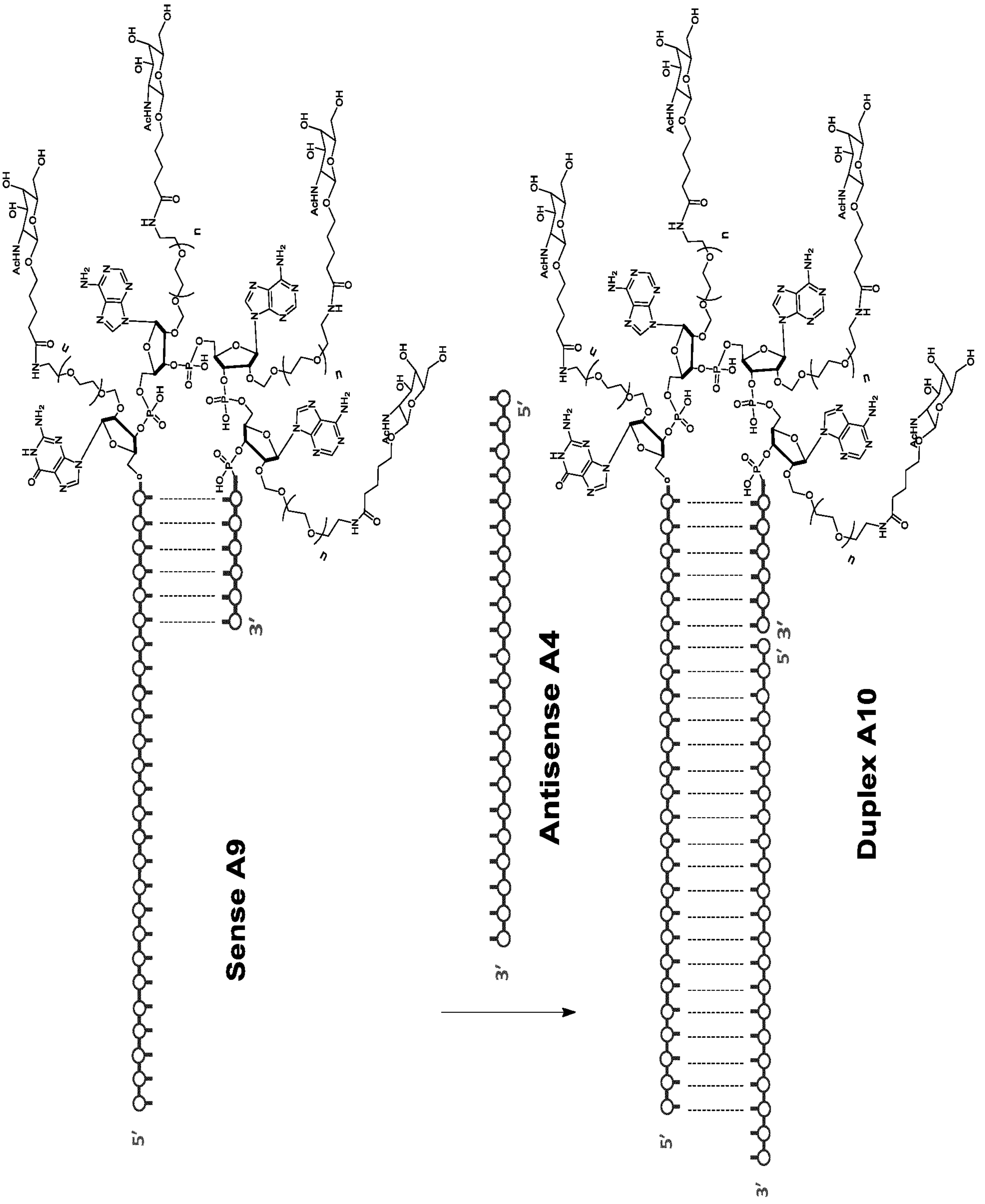
Sense A9
Antisense A4
Duplex A10

**SCHEME B: Synthesis of GalNAc Nicked Tetraloop dsNA Conjugate with Multiple GalNAc on the Loop Post Synthetic Conjugation Using "Click" Chemistry**

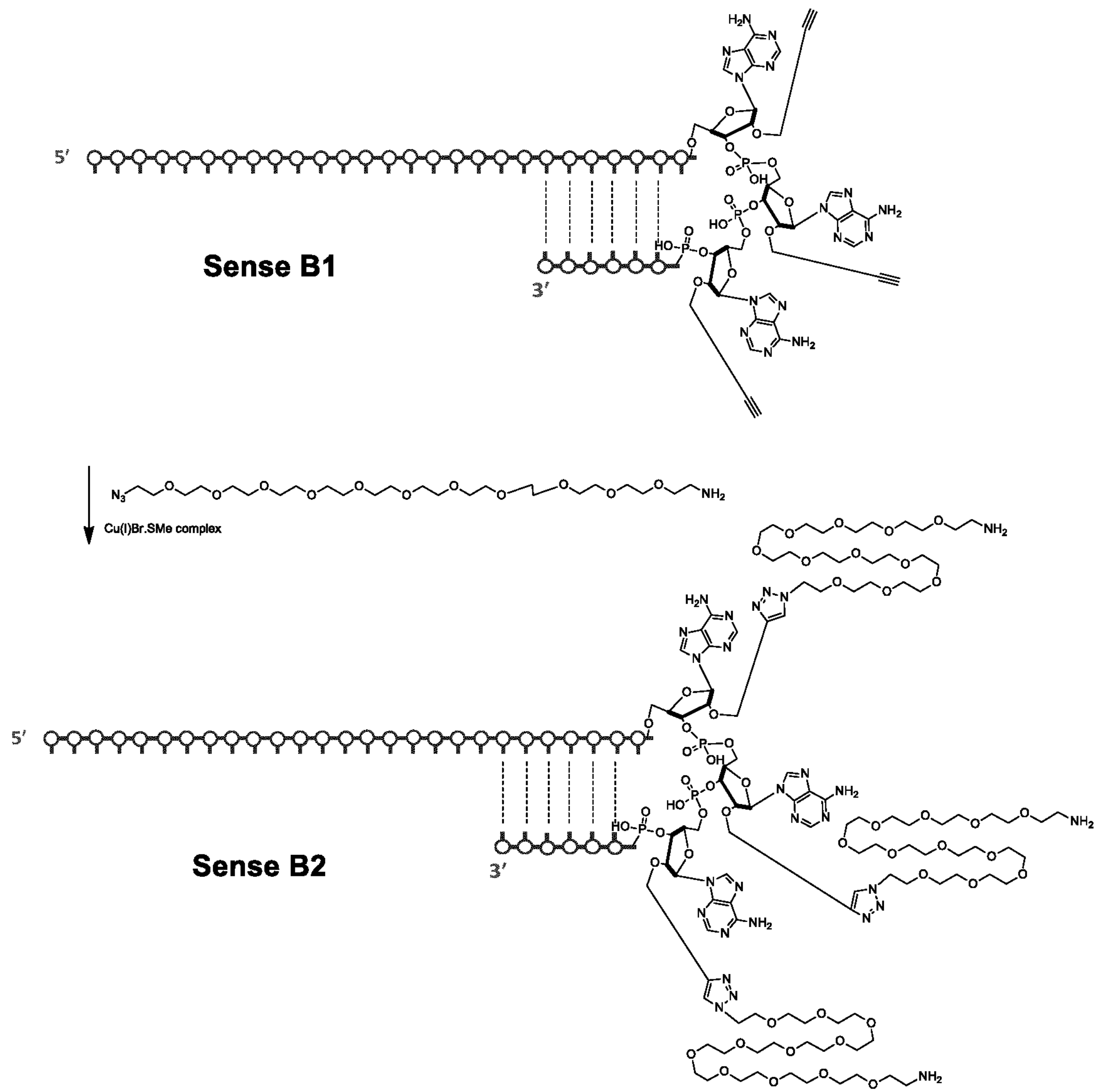


Sense B1

Sense B2

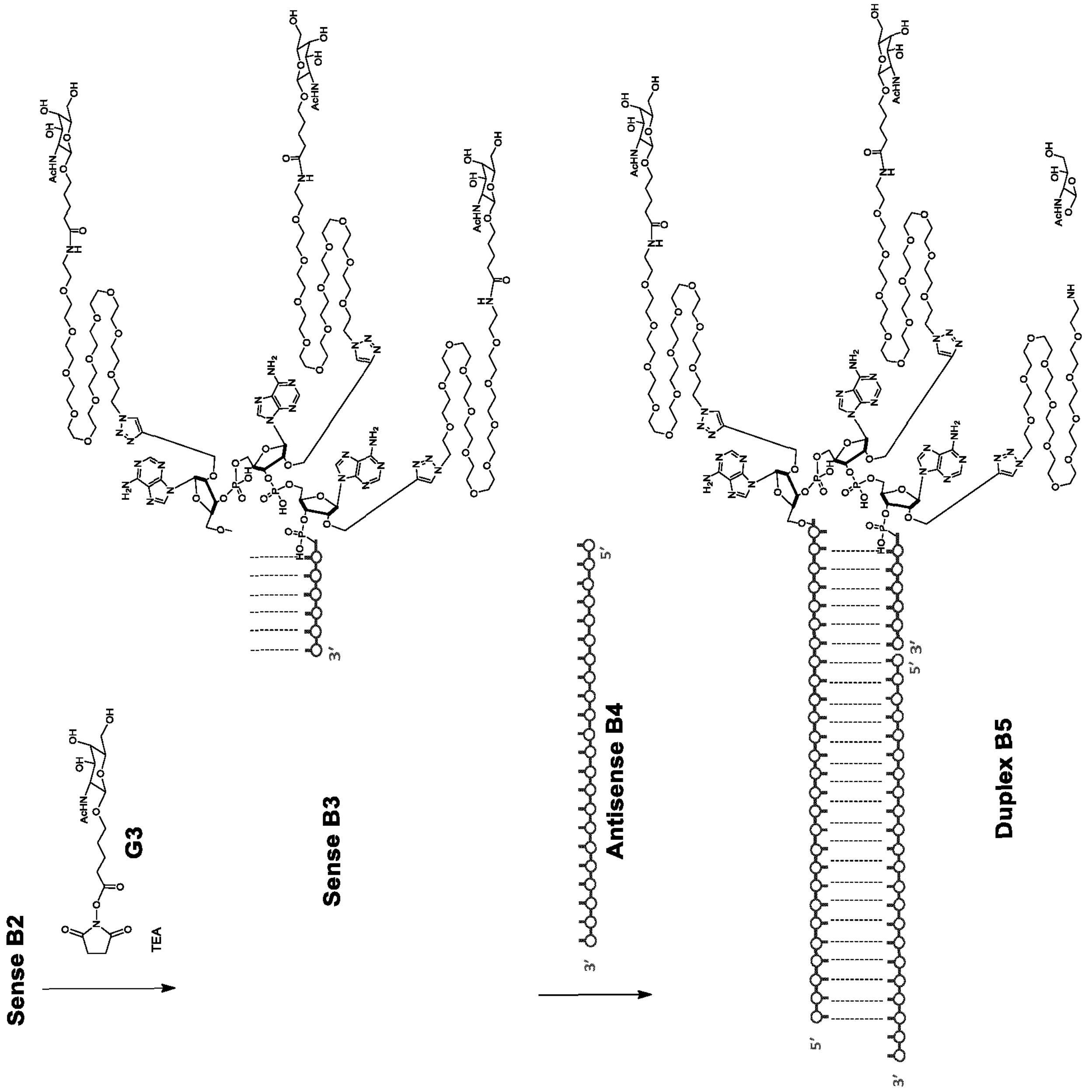


Sense B2

G3

Sense B3

Antisense B4

Duplex B5

**SCHEME C:Synthesis of GalNAc Nicked Tetraloop dsNA Conjugate with Branched Linker Based Tri- GalNAc on the Loop Post Synthetic Conjugation Using "Click" Chemistry**

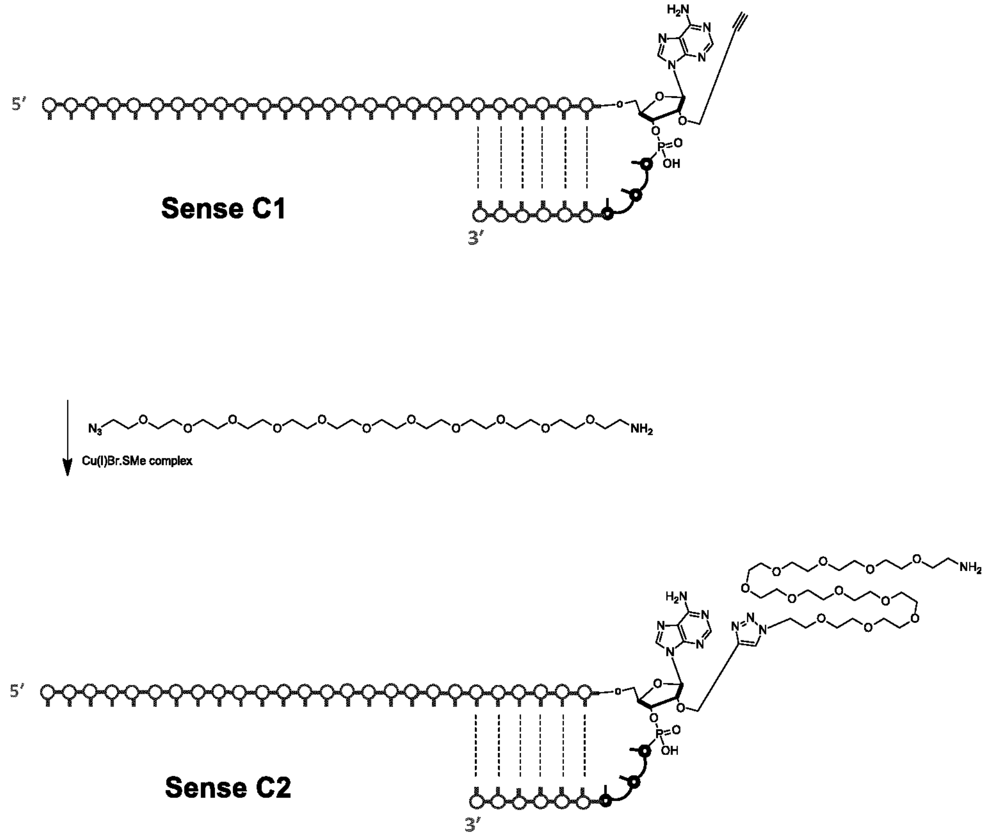


Sense C1

Sense C2

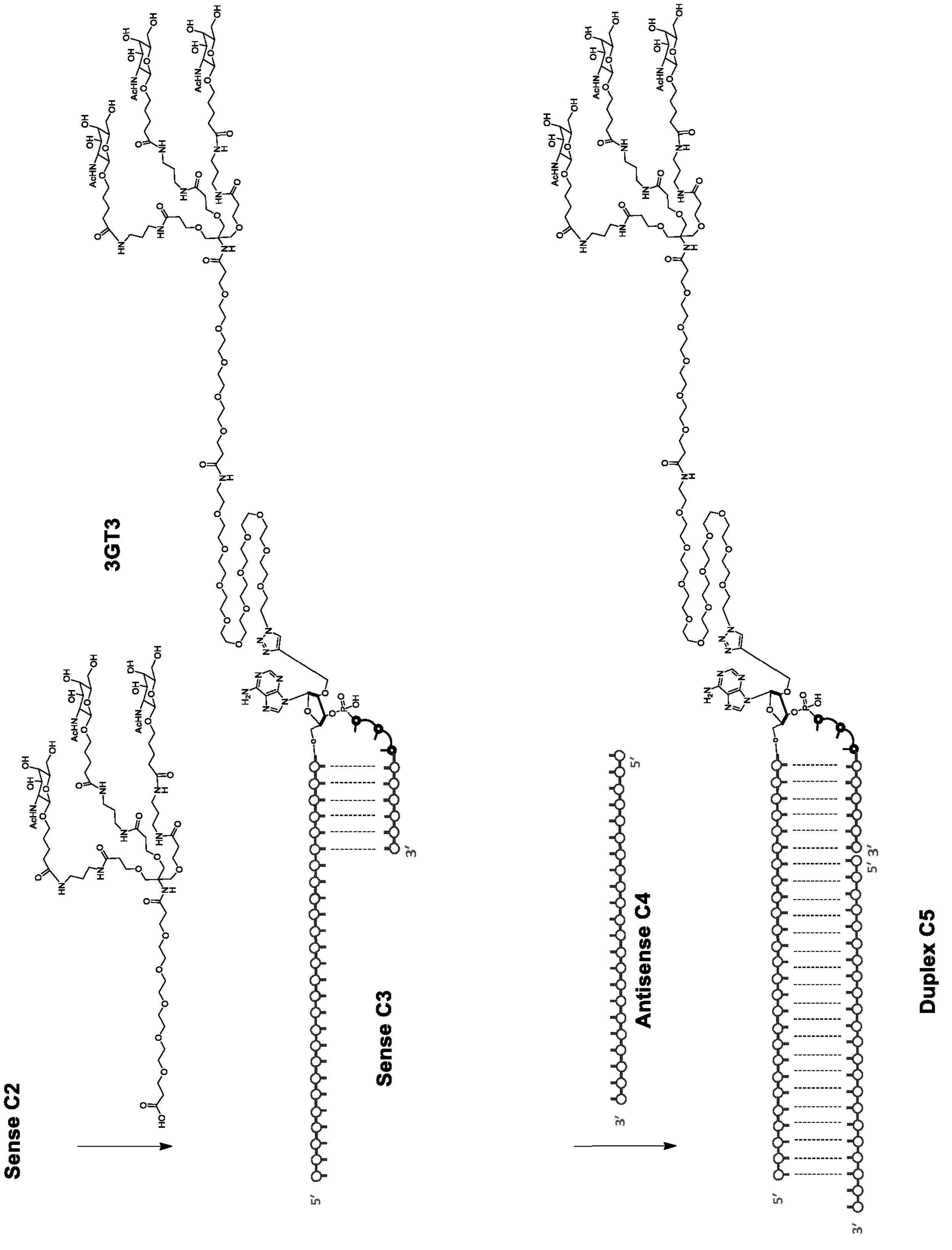

Sense C2

3GT3

Sense C3

Antisense C4

Duplex C5

## SCHEME D: Synthesis of GalNAc Nicked Tetraloop dsNA Conjugate with Multiple GalNAc on the Stem Post Synthetic Conjugation Using "Click" Chemsitry

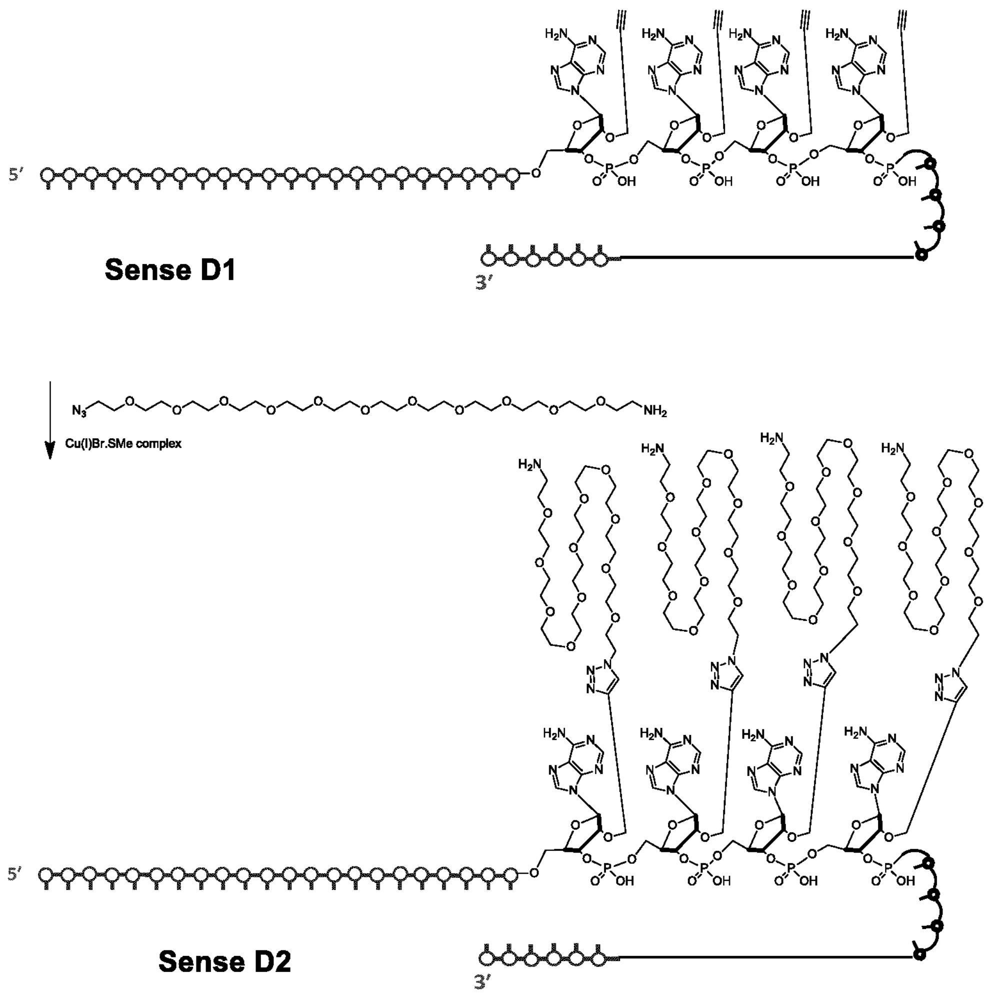


Sense D1

Sense D2

Sense D2

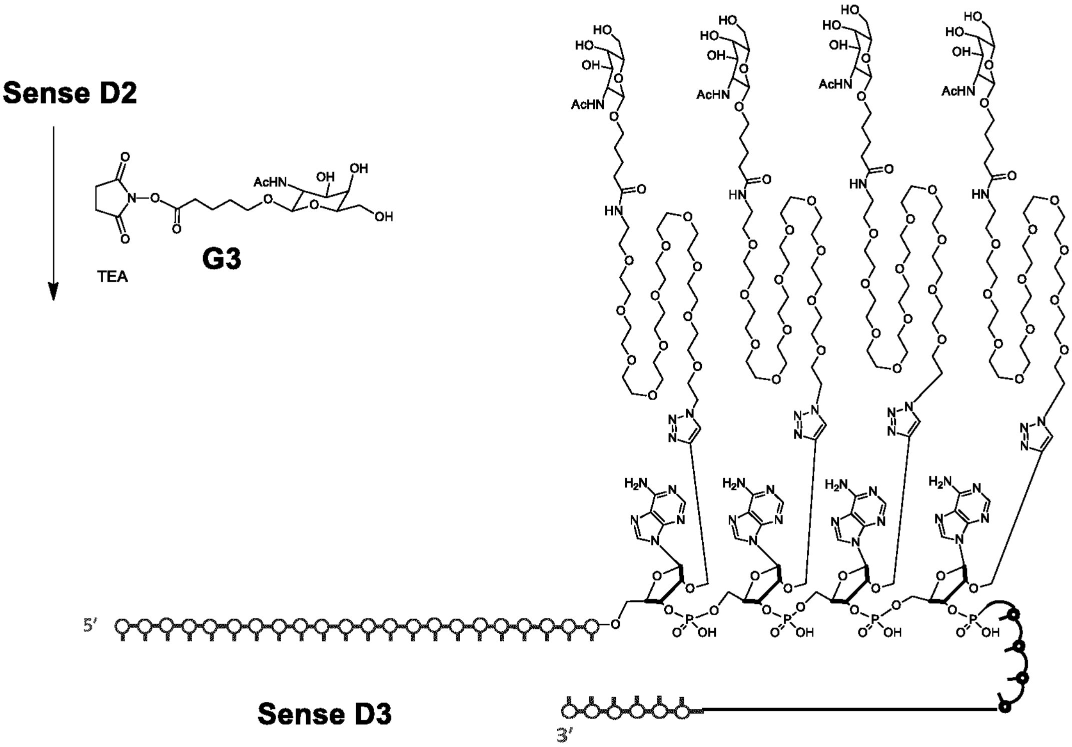


G3

Sense D3

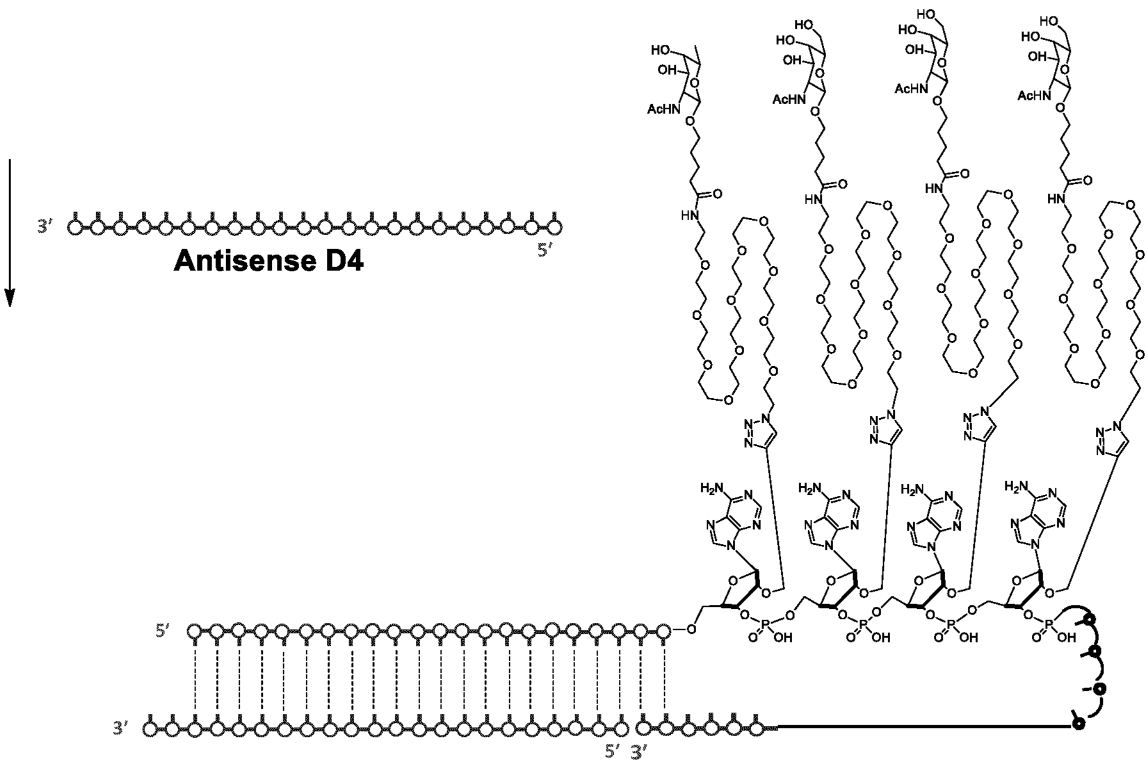


Antisense D4

Duplex D5

**SCHEME E: Synthesis of GalNAc dsNA Conjugate with Multiple GalNAc on the 5'-Extension of Antisense via Post Synthetic Conjugation**

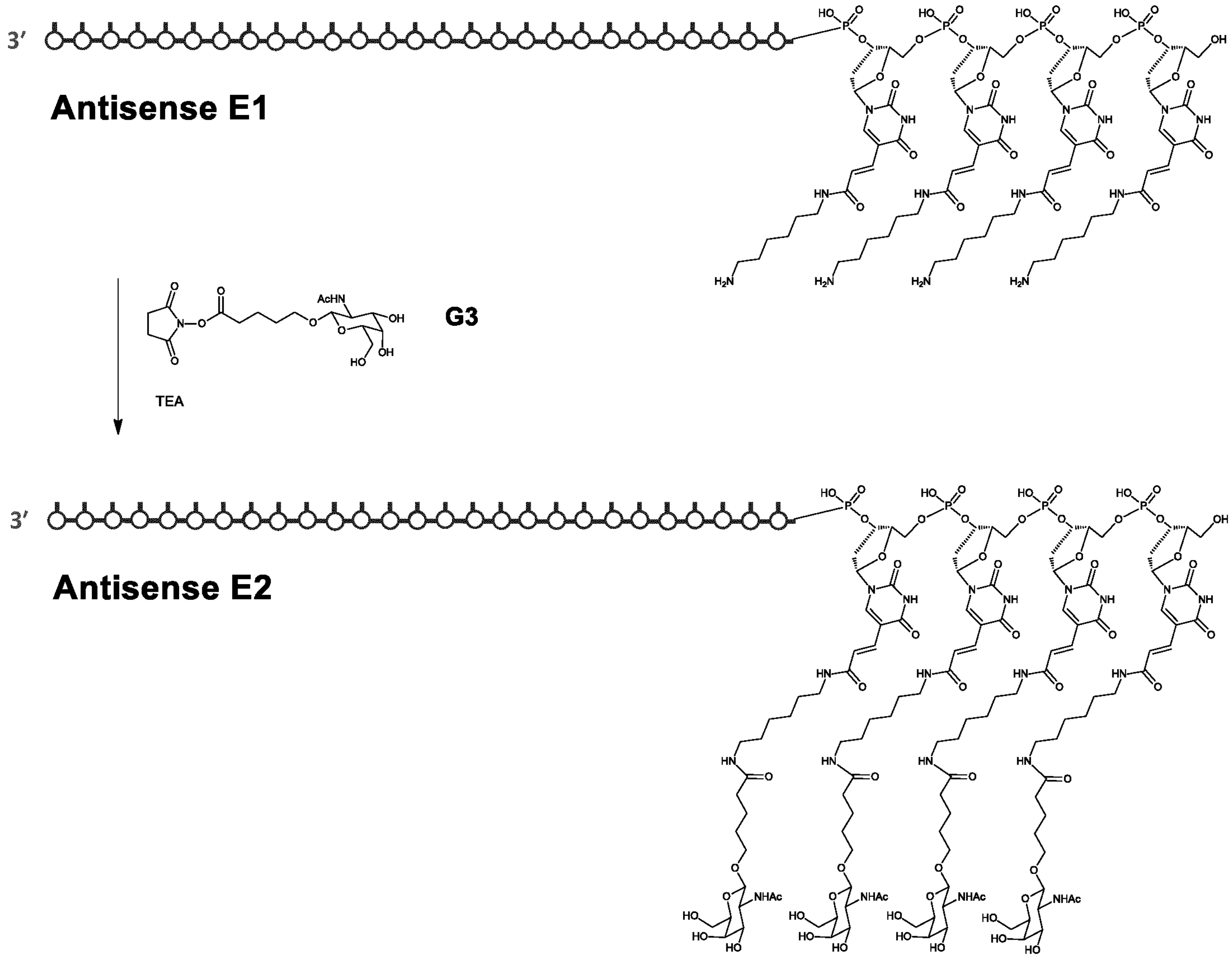


Antisense E1

G3

Antisense E2

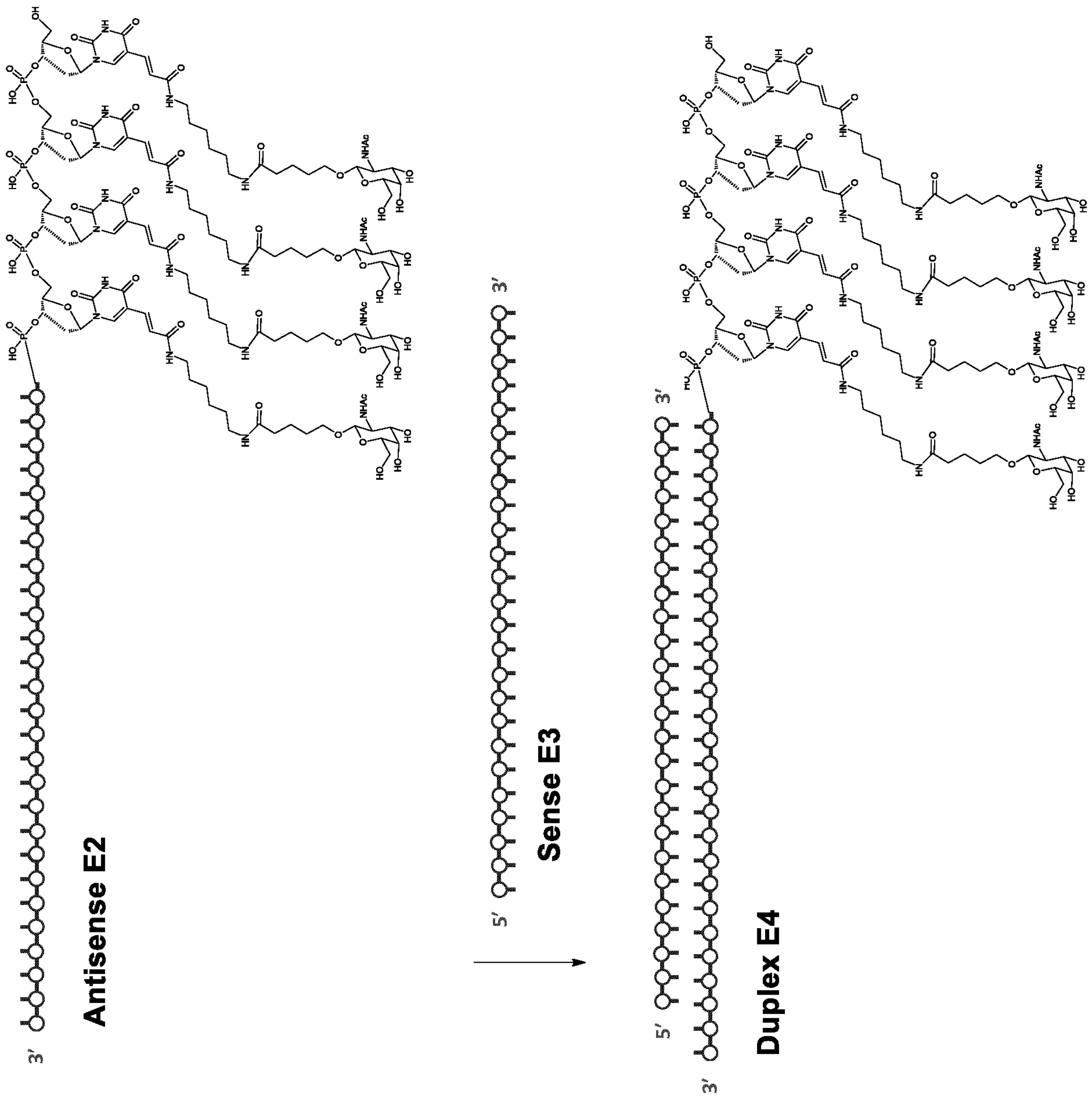
Antisense E2
3'
Sense E3
5'
3'
Duplex E4
5'
3'
3'

## SCHEME F: Synthesis of GalNAc dsNA Conjugate with Multiple GalNAc on the 5'-Extension of Sense via Post Synthetic Conjugation

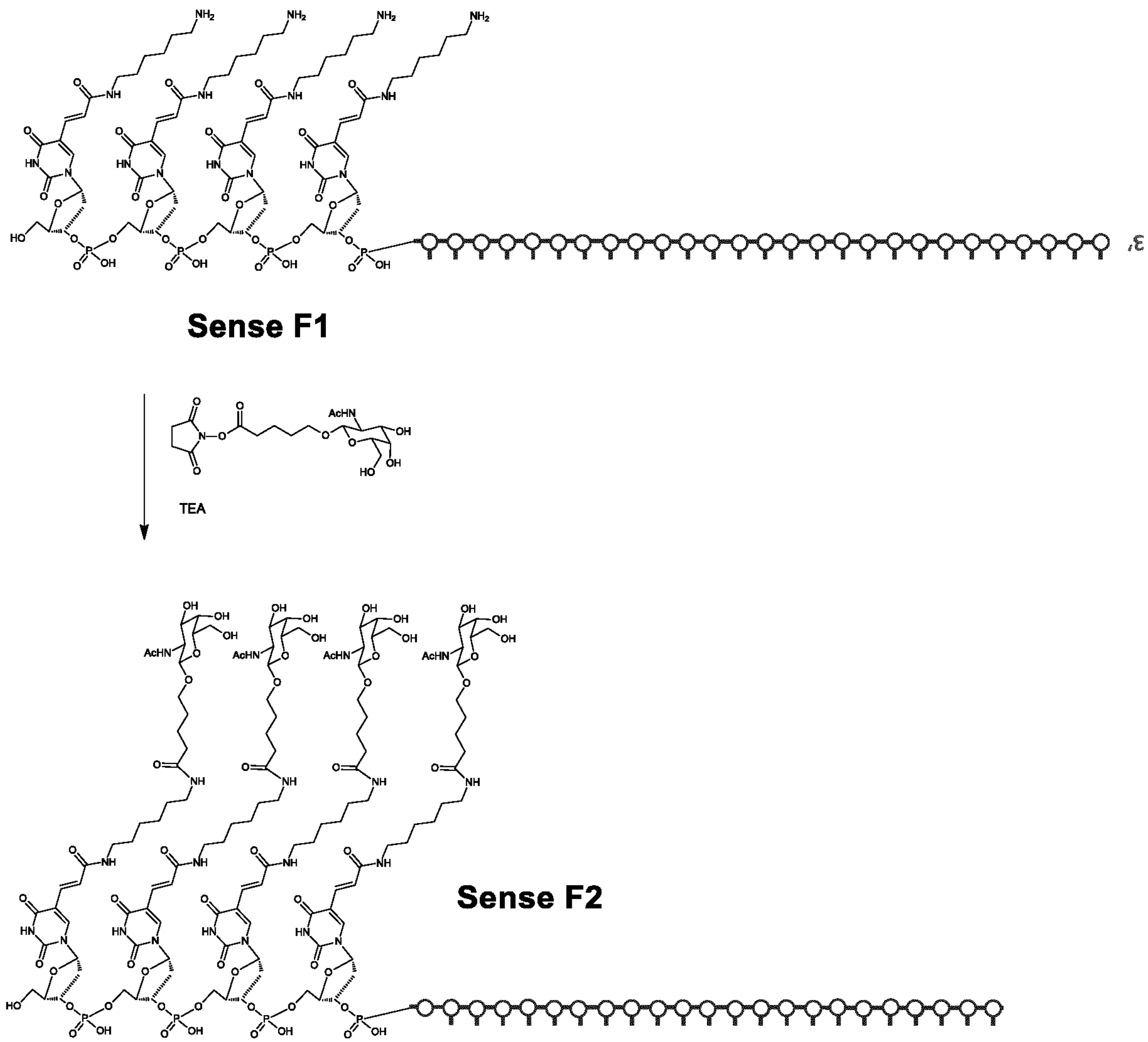


Sense F1

Sense F2

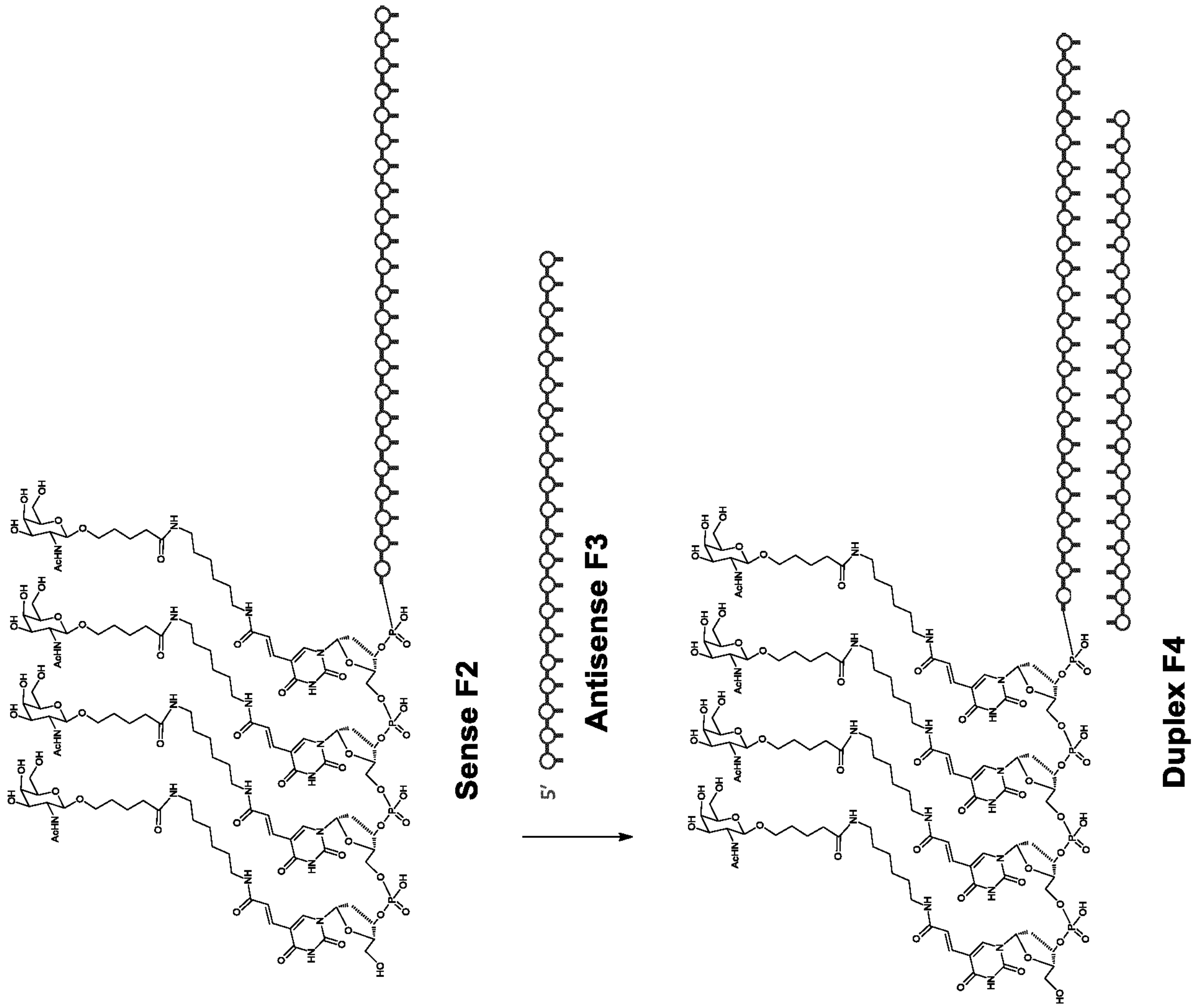
Sense F2
5'
Antisense F3
Duplex F4

**SCHEME G: Synthesis of GalNAc dsNA Conjugate with GalNAc Separated By One Spacer On The 5'-Extension of Sense via Post Synthetic Conjugation**

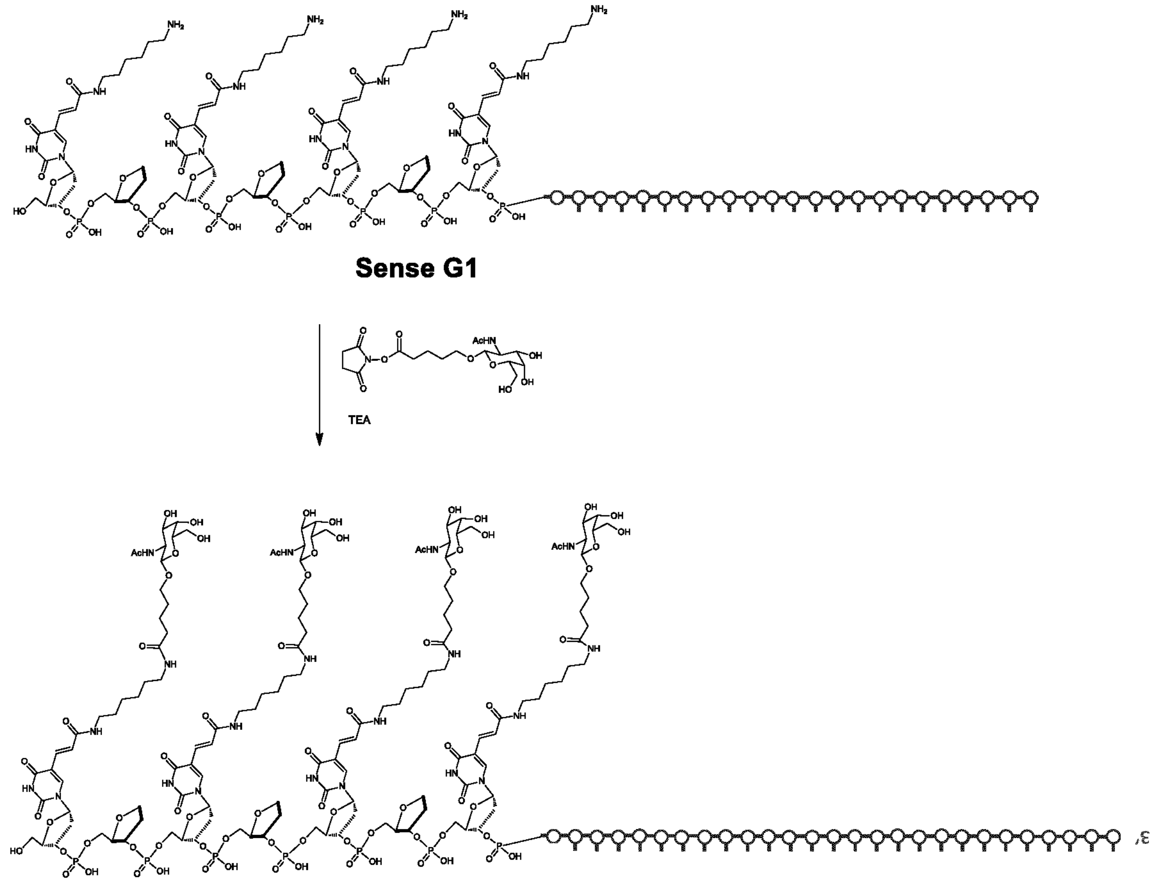


Sense G1

Sense G2

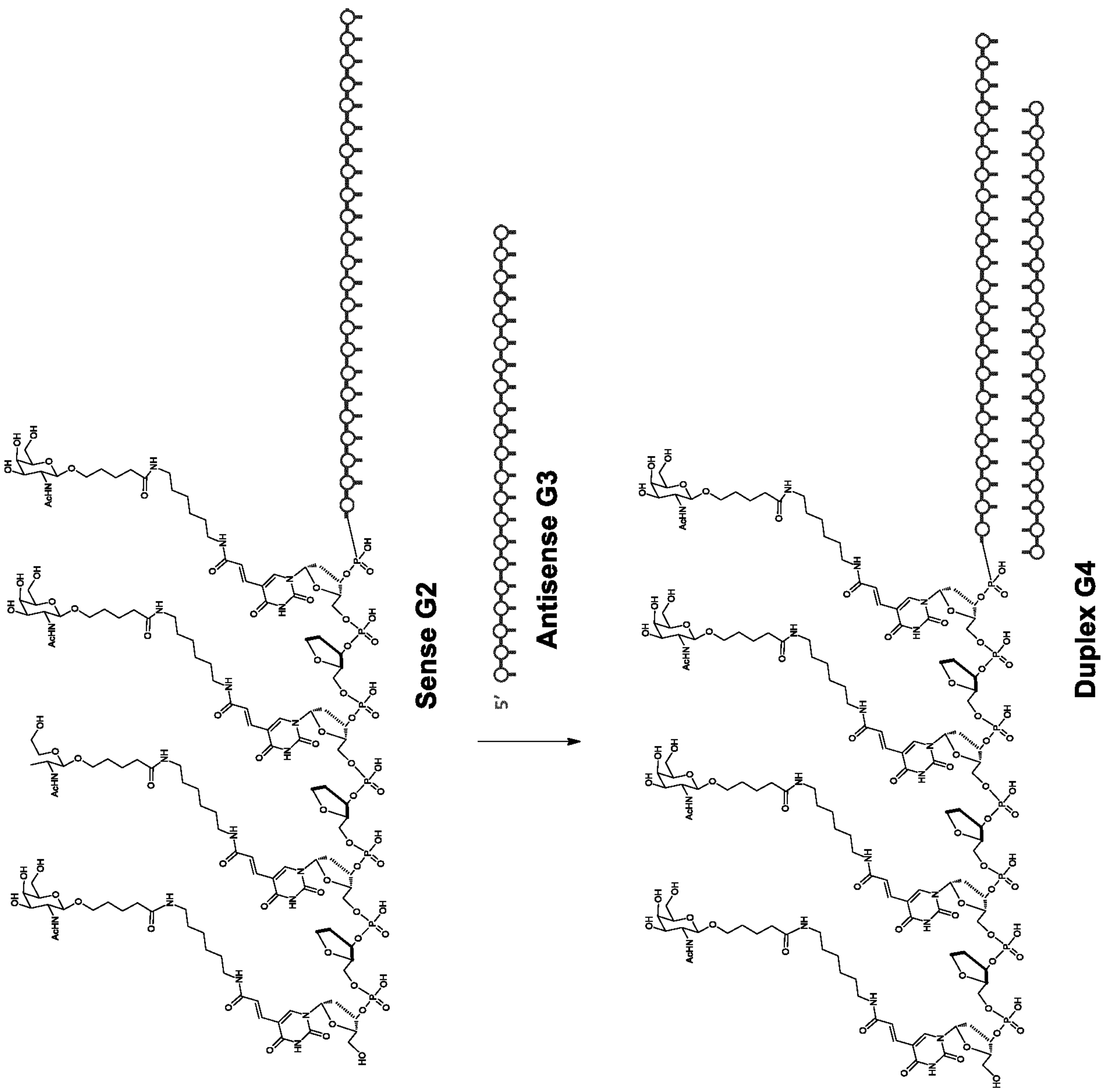
Sense G2
5'
Antisense G3
Duplex G4

**SCHEME H: Synthesis of GalNAc dsNA Conjugate with GalNAc Separated By Two Spacers On The 5'-Extension of Sense via Post Synthetic Conjugation**

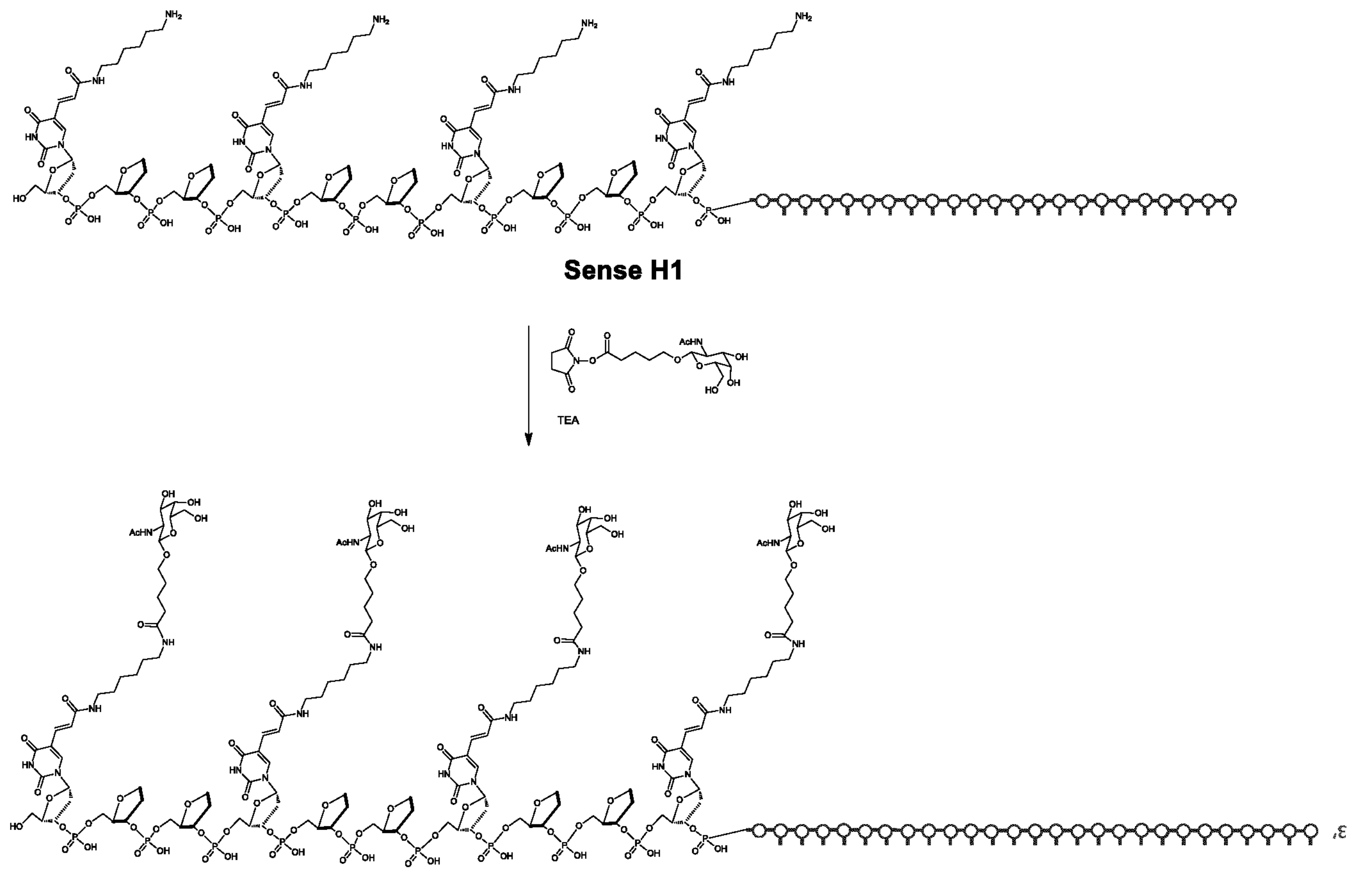


**Sense H1**

**Sense H2**

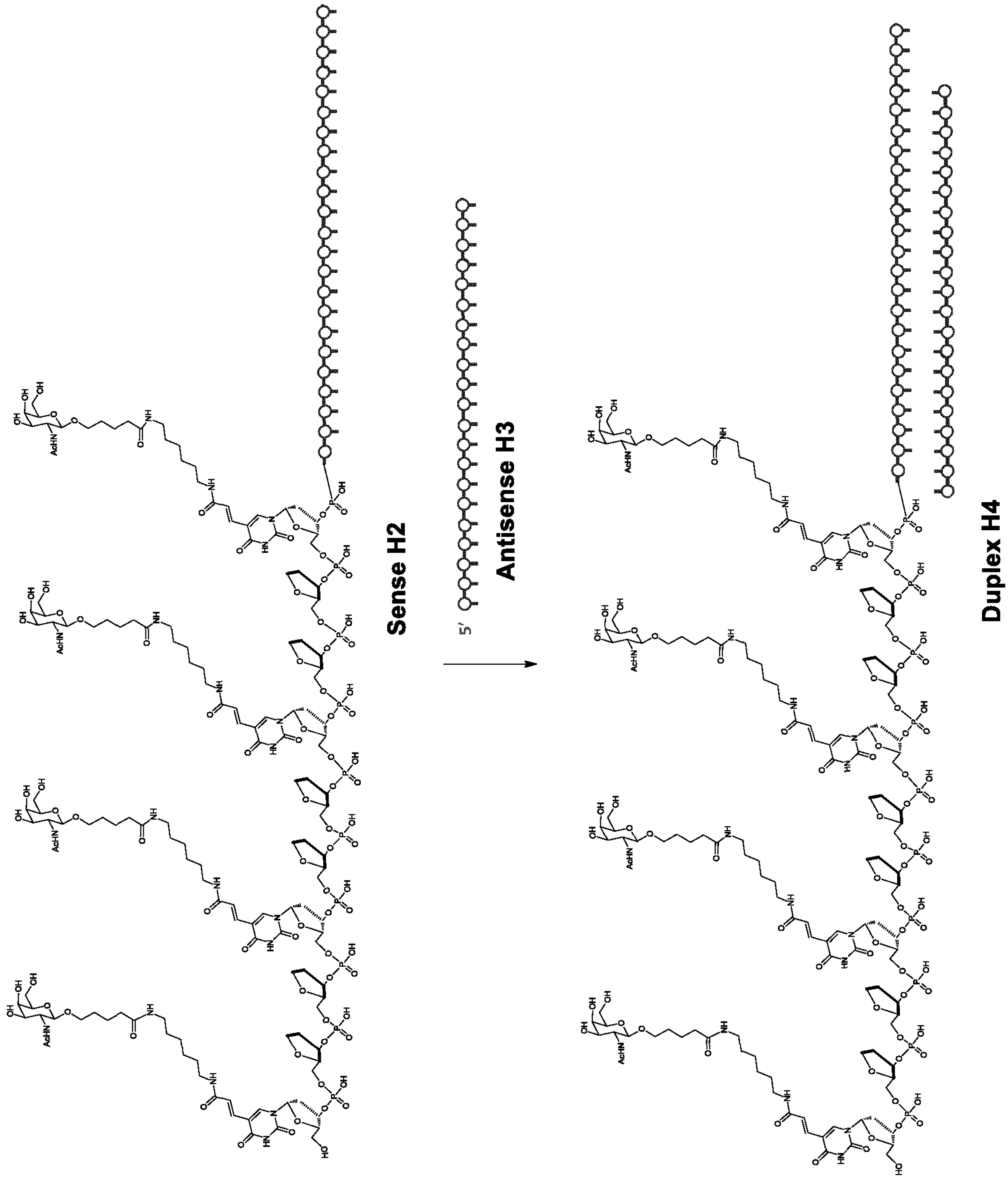

Sense H2

5'

Antisense H3

Duplex H4

## SCHEME I: Synthesis of GalNAc dsNA Conjugate with GalNAc Block Separated By Multiple Spacers On The 5'-Extension of Sense via Post Synthetic Conjugation

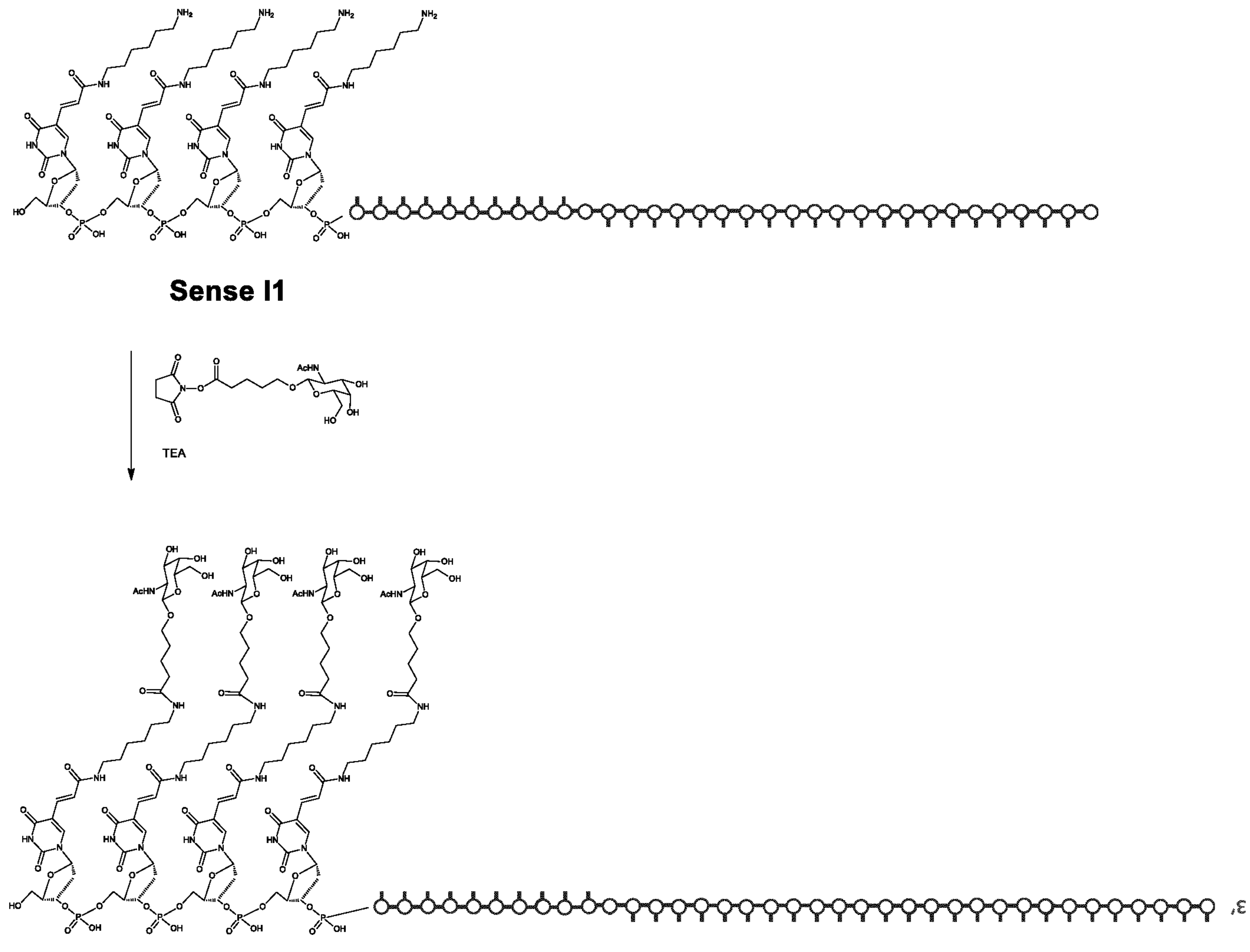


Sense I1

Sense I2

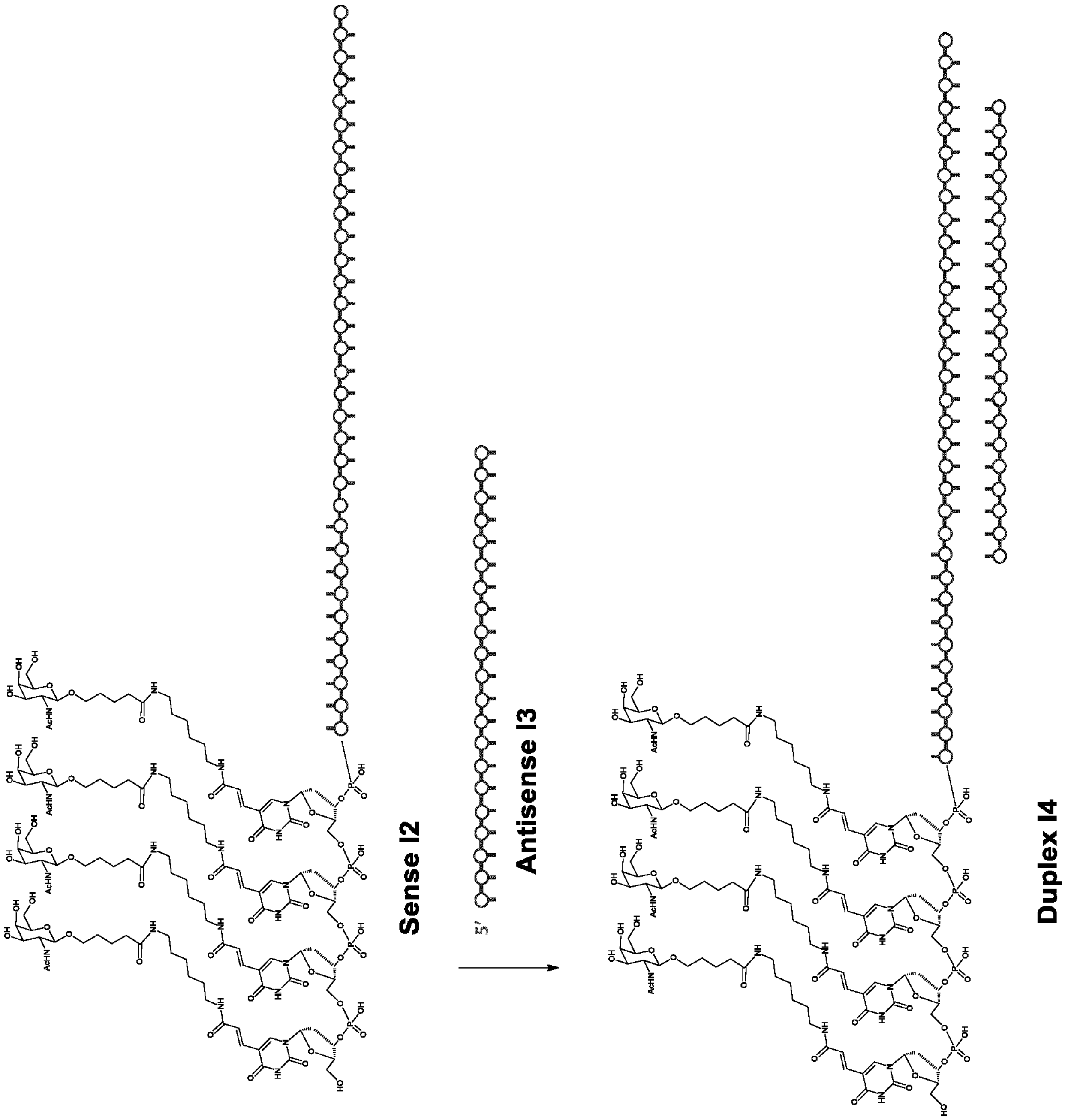

Sense I2

Antisense I3

Duplex I4

**SCHEME J: Synthesis of GalNAc dsNA Conjugate with GalNAc Separated By Two Spacers On The 5'-Extension of Antisense via Post Synthetic Conjugation**

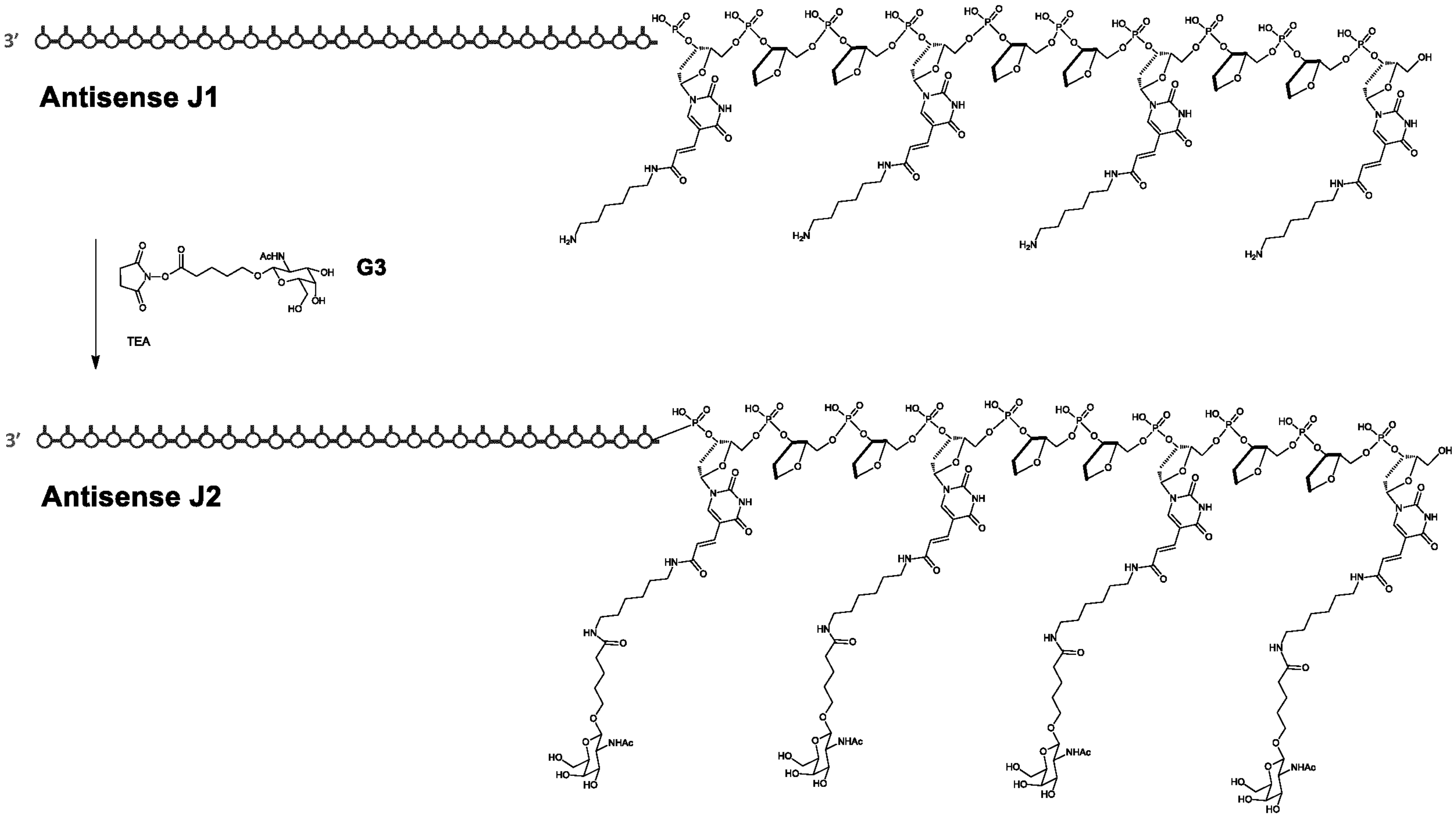


3' Antisense J1

G3

3' Antisense J2

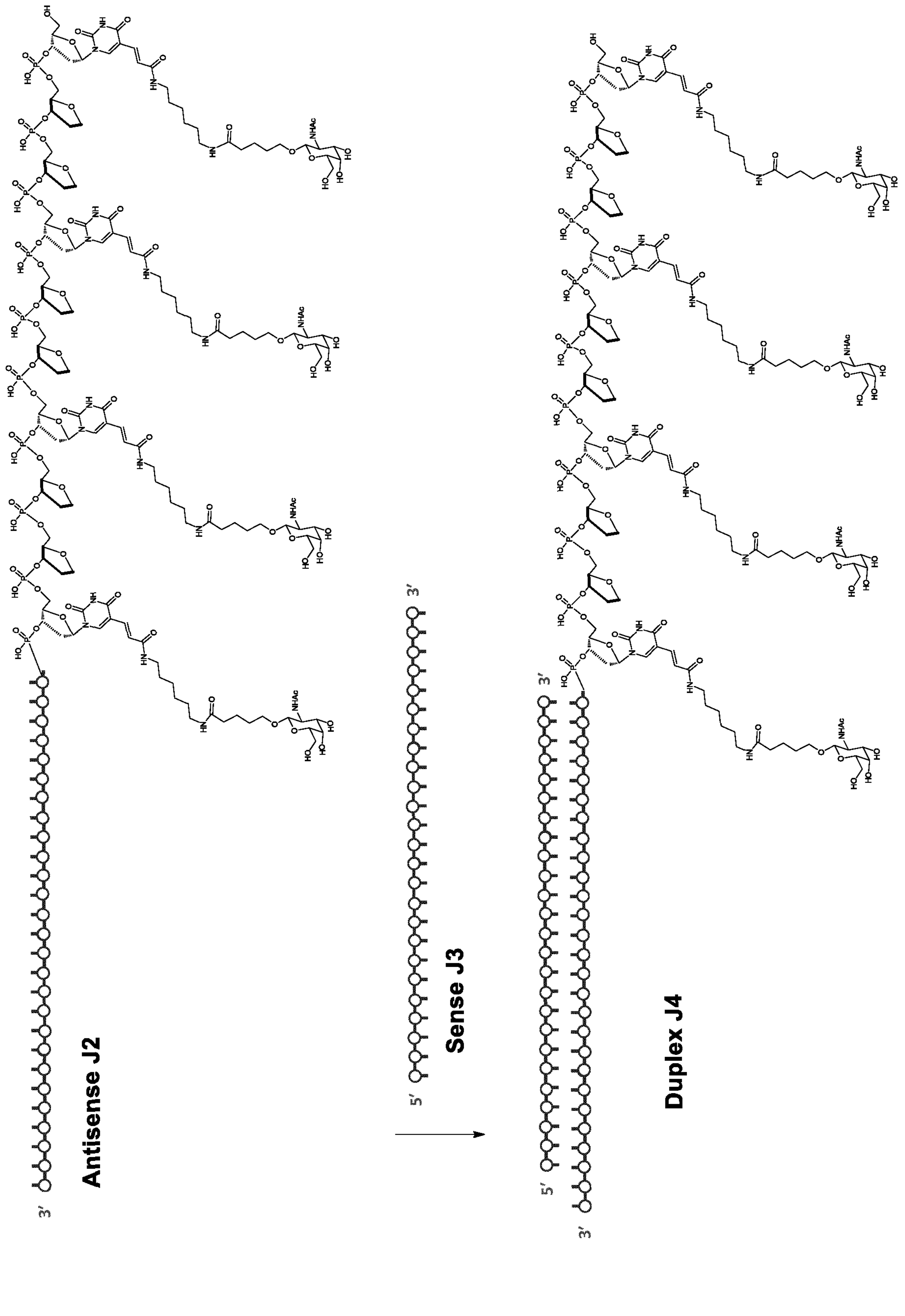

Antisense J2

Sense J3

Duplex J4

**SCHEME Ka: Synthesis of GalNAc dsNA Conjugate with GalNAc Block Separated By Multiple Spacers On The 5'-Extension of Antisense via Post Synthetic Conjugation**

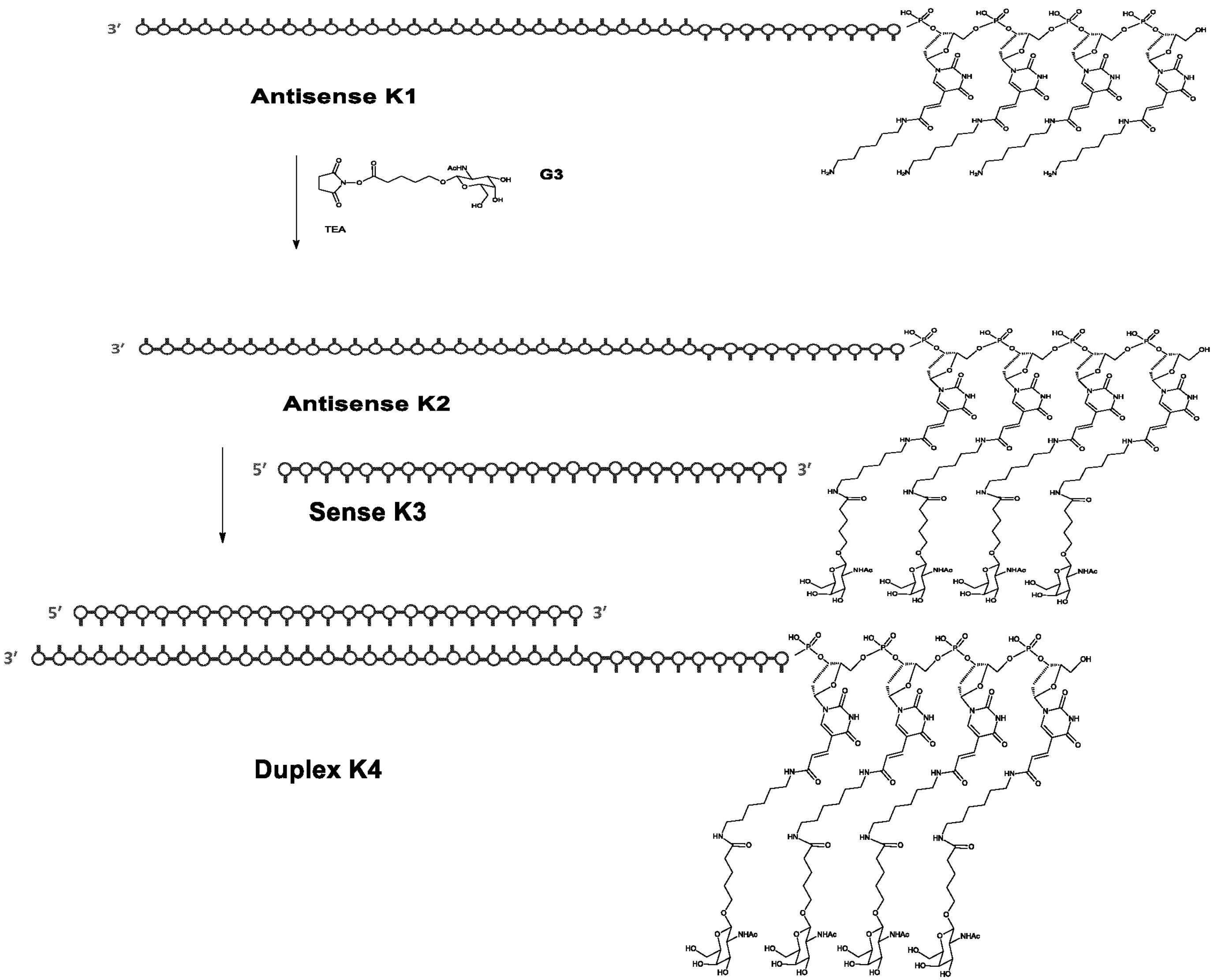

**Scheme K1: Solid phase synthesis of 5'Extended dsNA GalNAc conjugate using Mannose-6-phosphate-amidite with Mannose -6-phopshate attached to the C5 of pyrimidine**

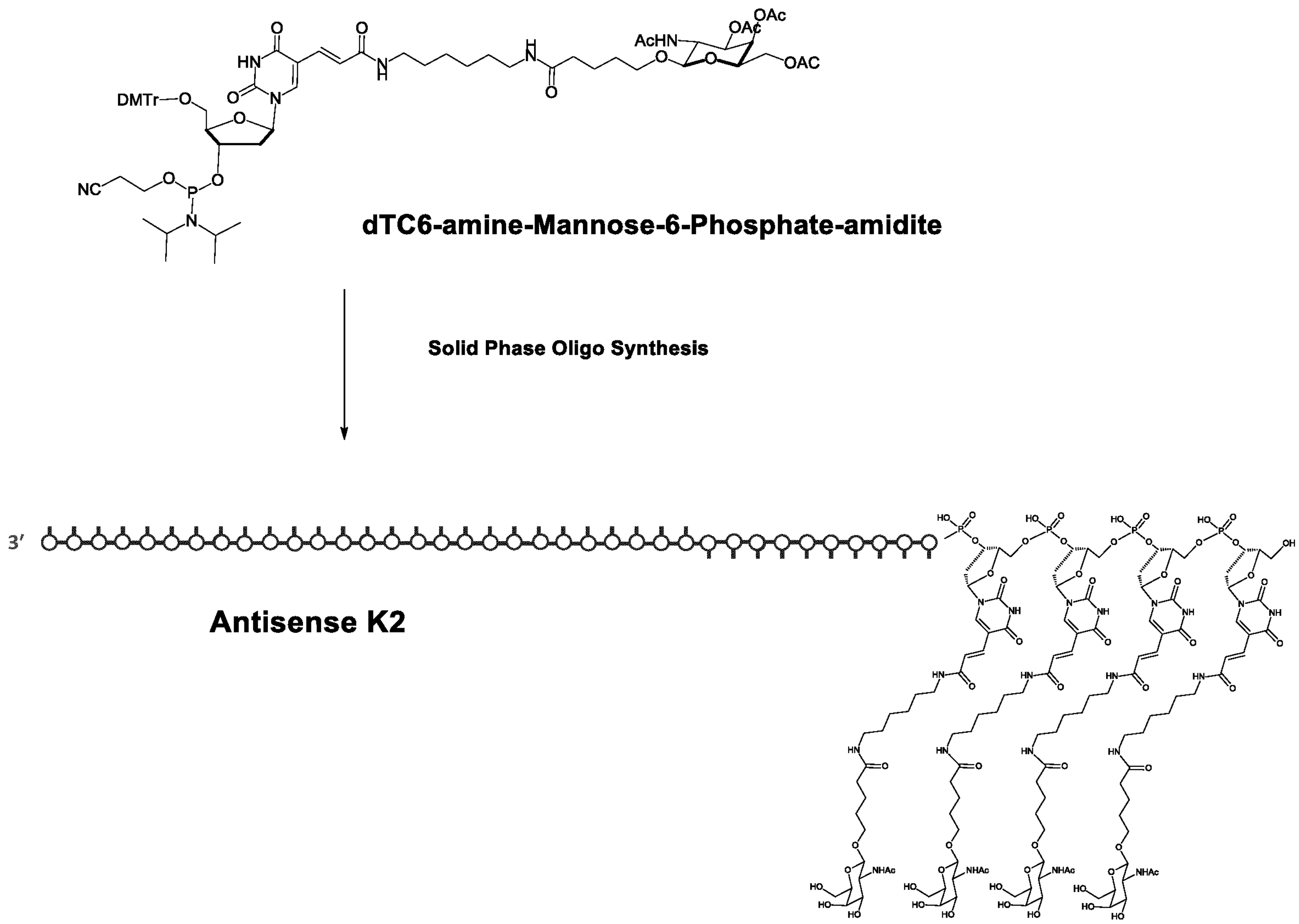


dTC6-amine-Mannose-6-Phosphate-amidite

Antisense K2

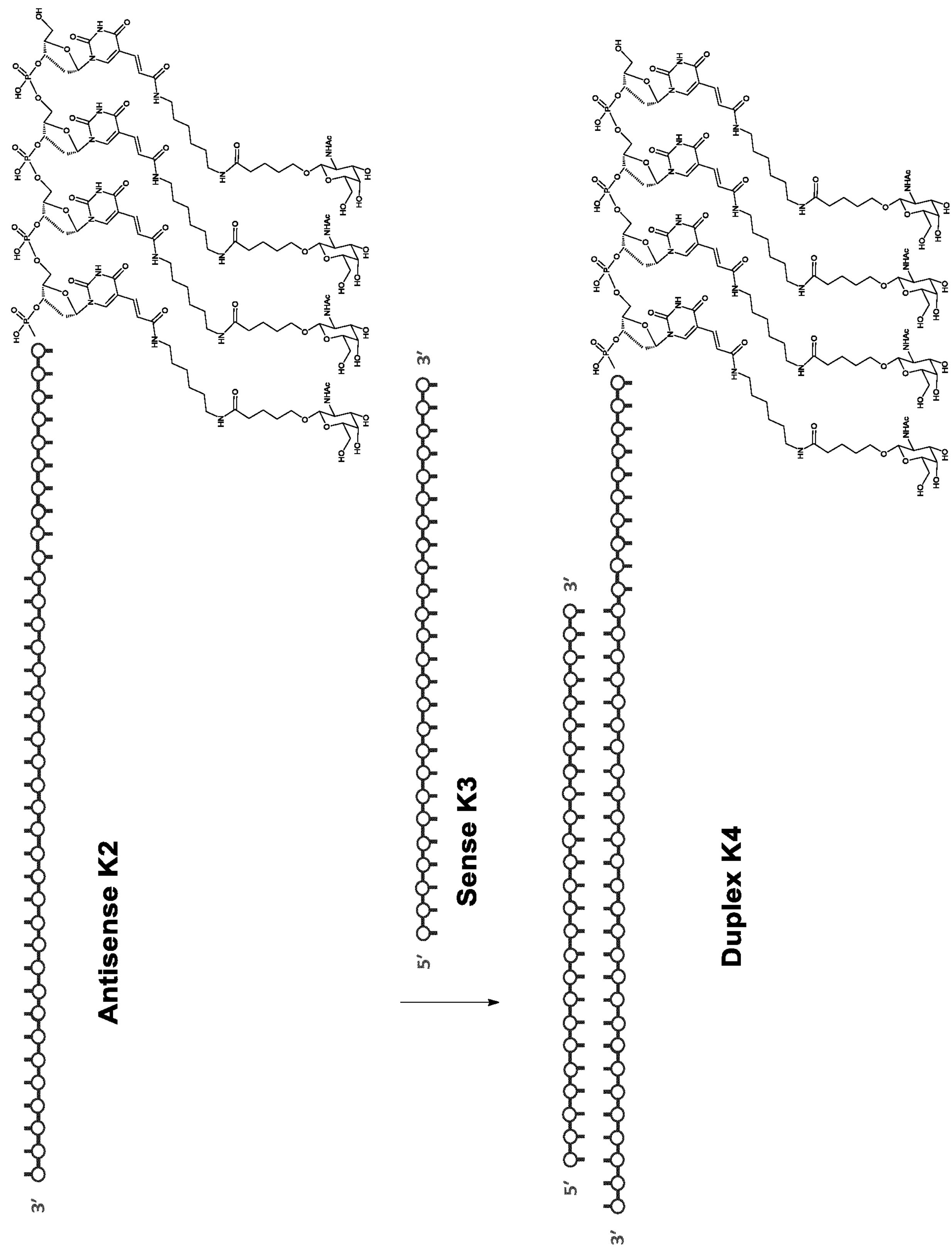
Antisense K2

Sense K3

Duplex K4

## SCHEME Kb

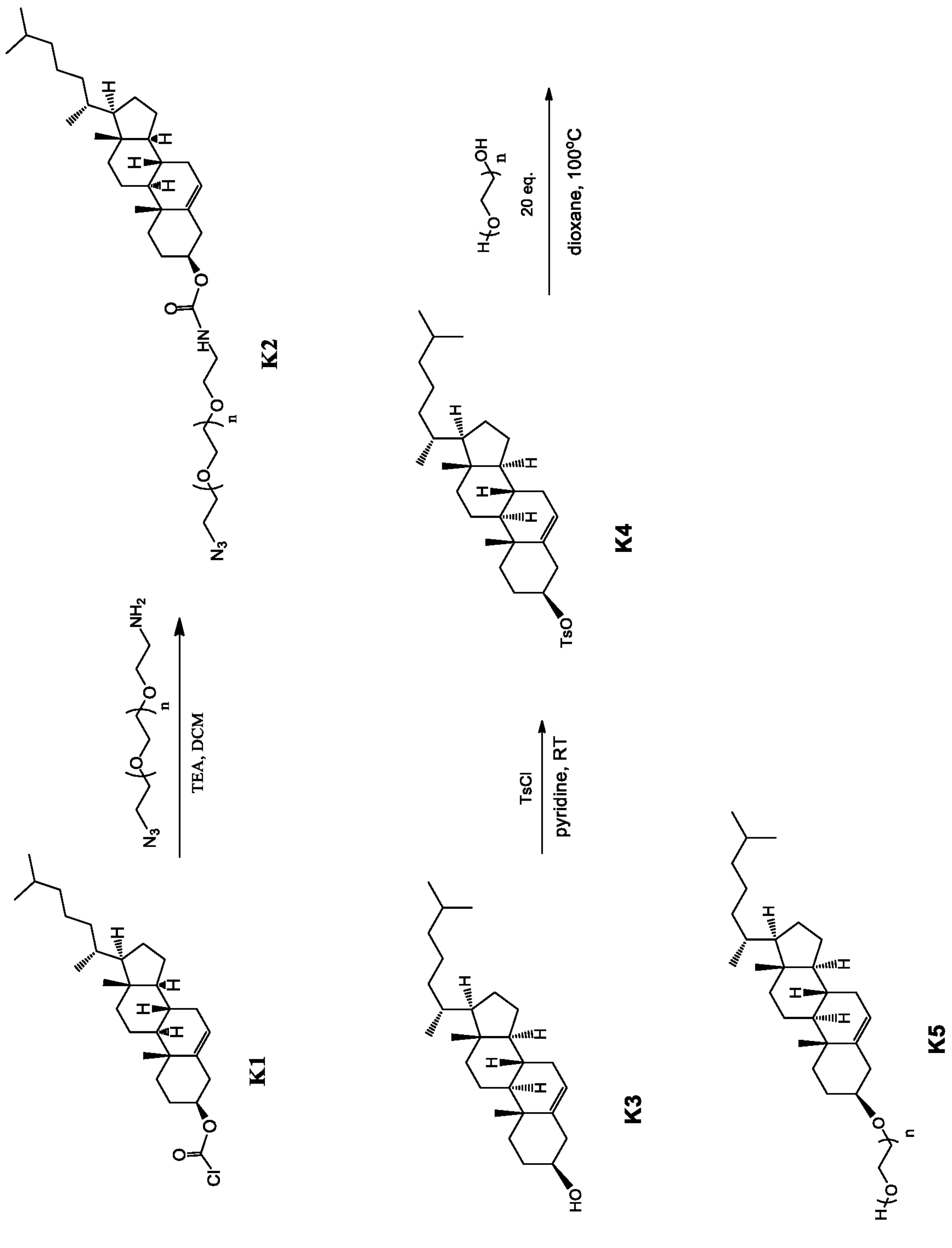


K1 K2 K3 K4 K5

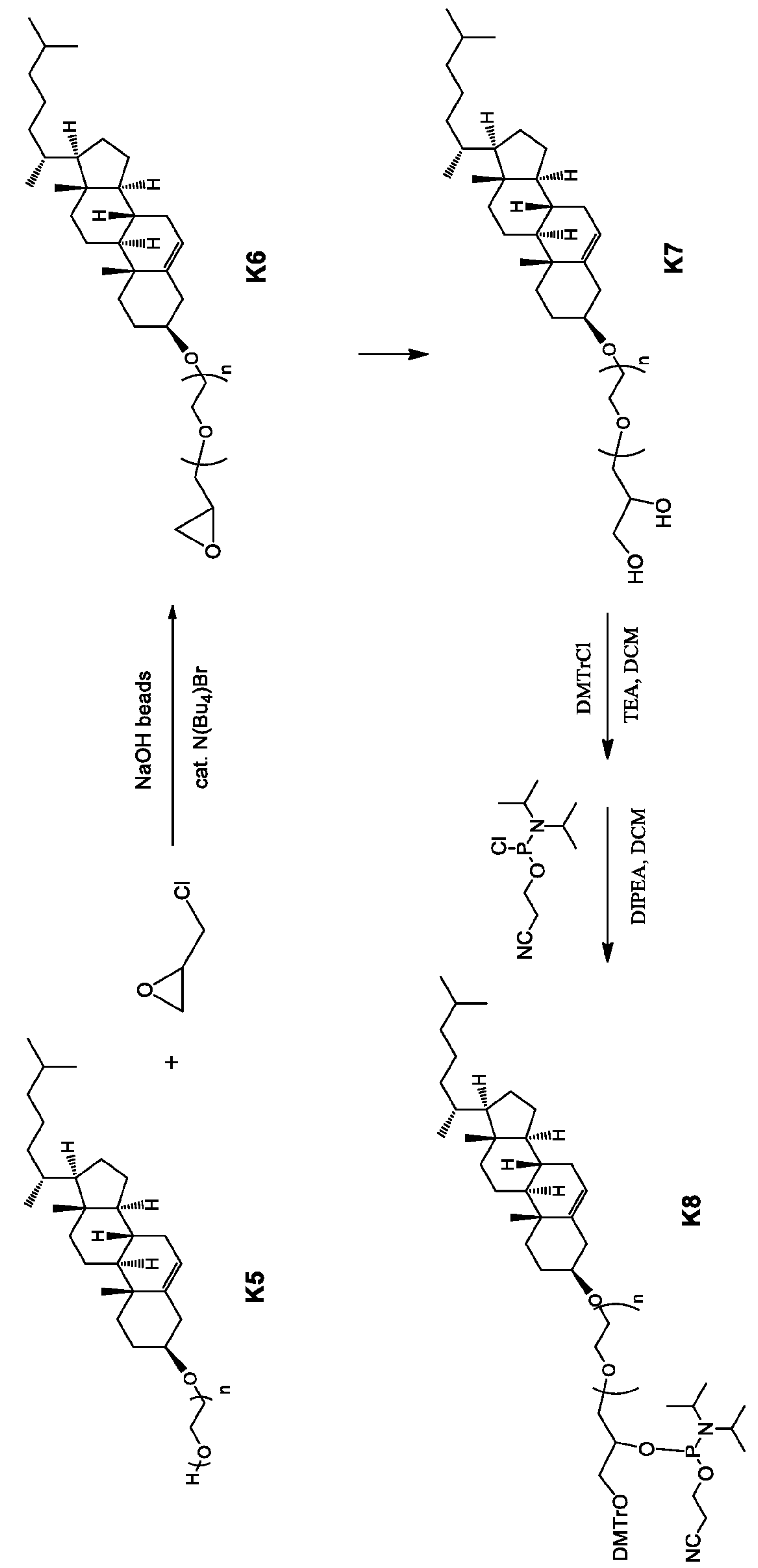


K5

K6

K7

K8

**SCHEME L: Synthesis of Cholesterol Nicked Tetraloop dsNA Conjugate with One Cholesterol On The Loop - Post Solid Phase Conjugation via "Click" Chemsitry**

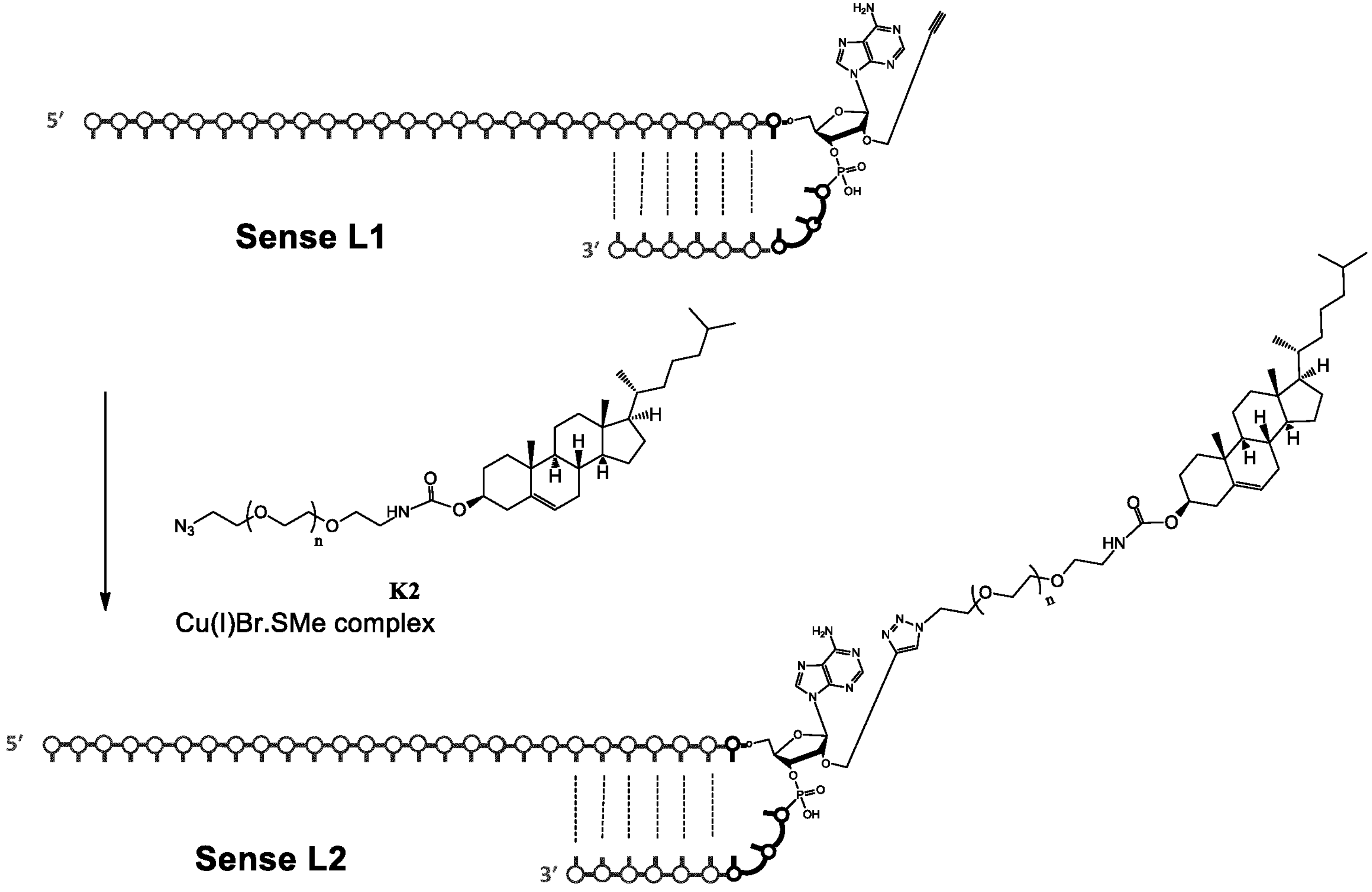

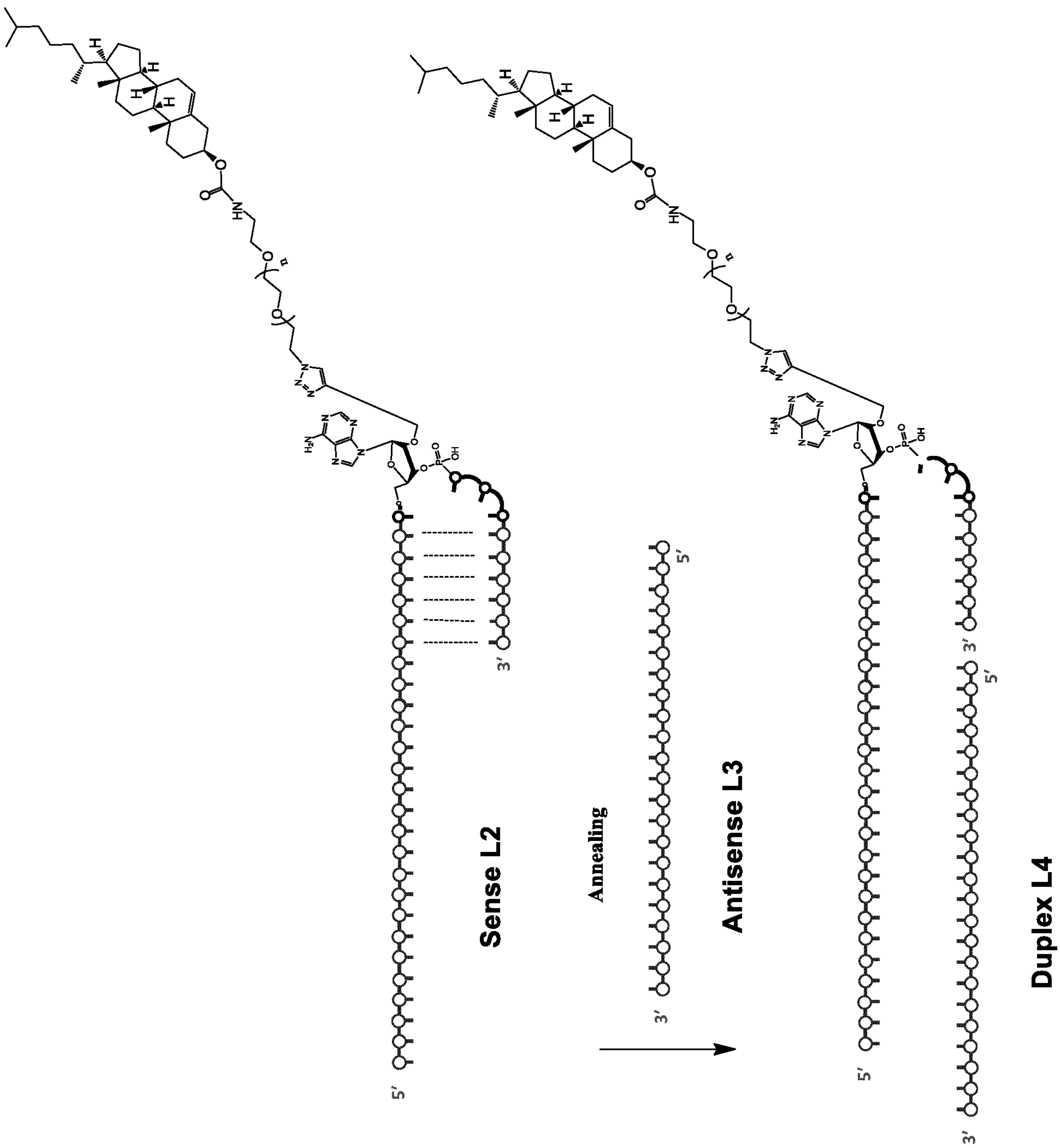


Sense L2

Antisense L3

Duplex L4

**SCHEME L1: Synthesis of Cholesterol Nicked Tetraloop dsNA Conjugate with One Cholesterol On The Loop - Post Solid Phase Conjugation via Amide Chemsitry**

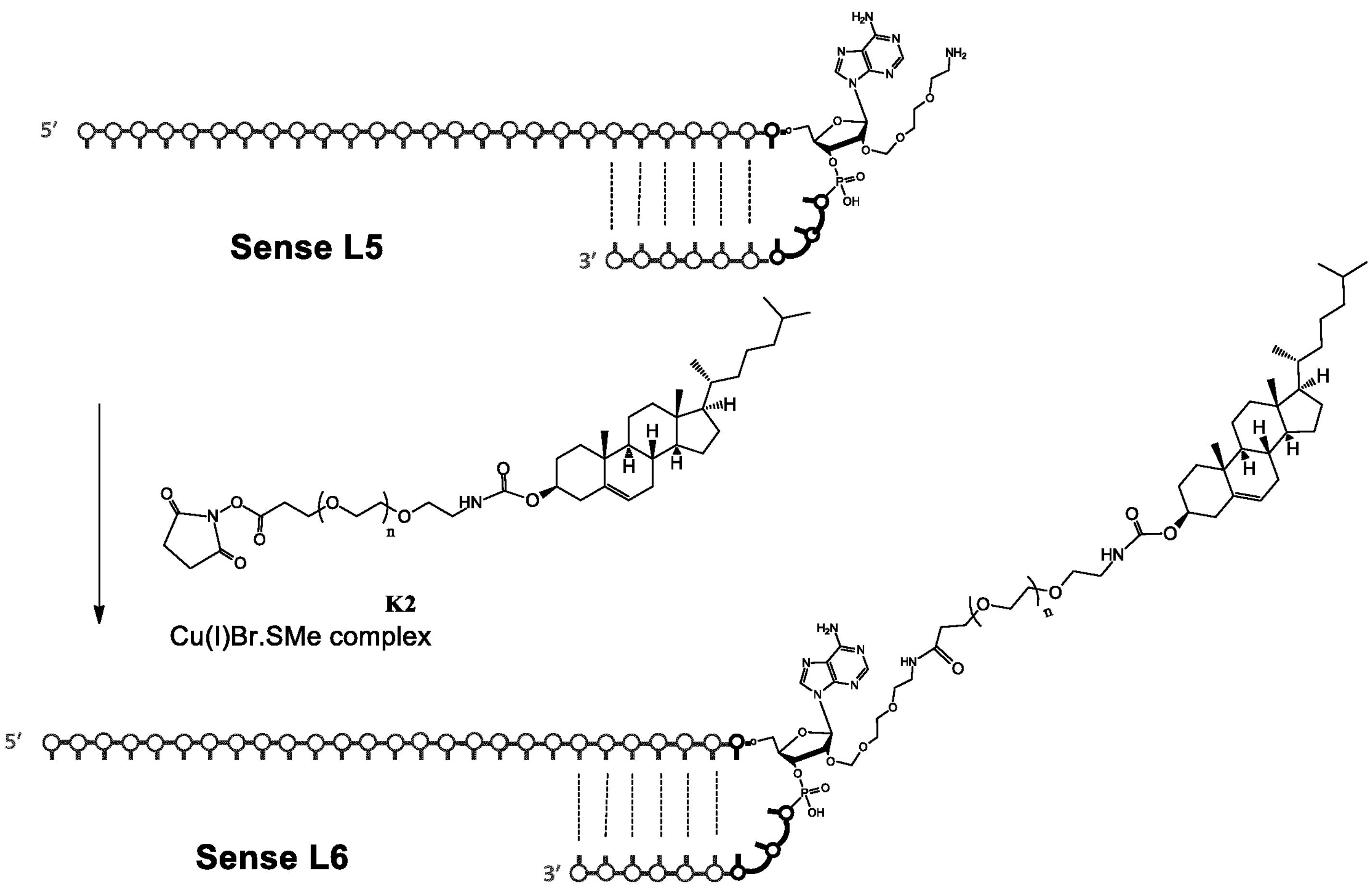


Sense L5

K2

Sense L6

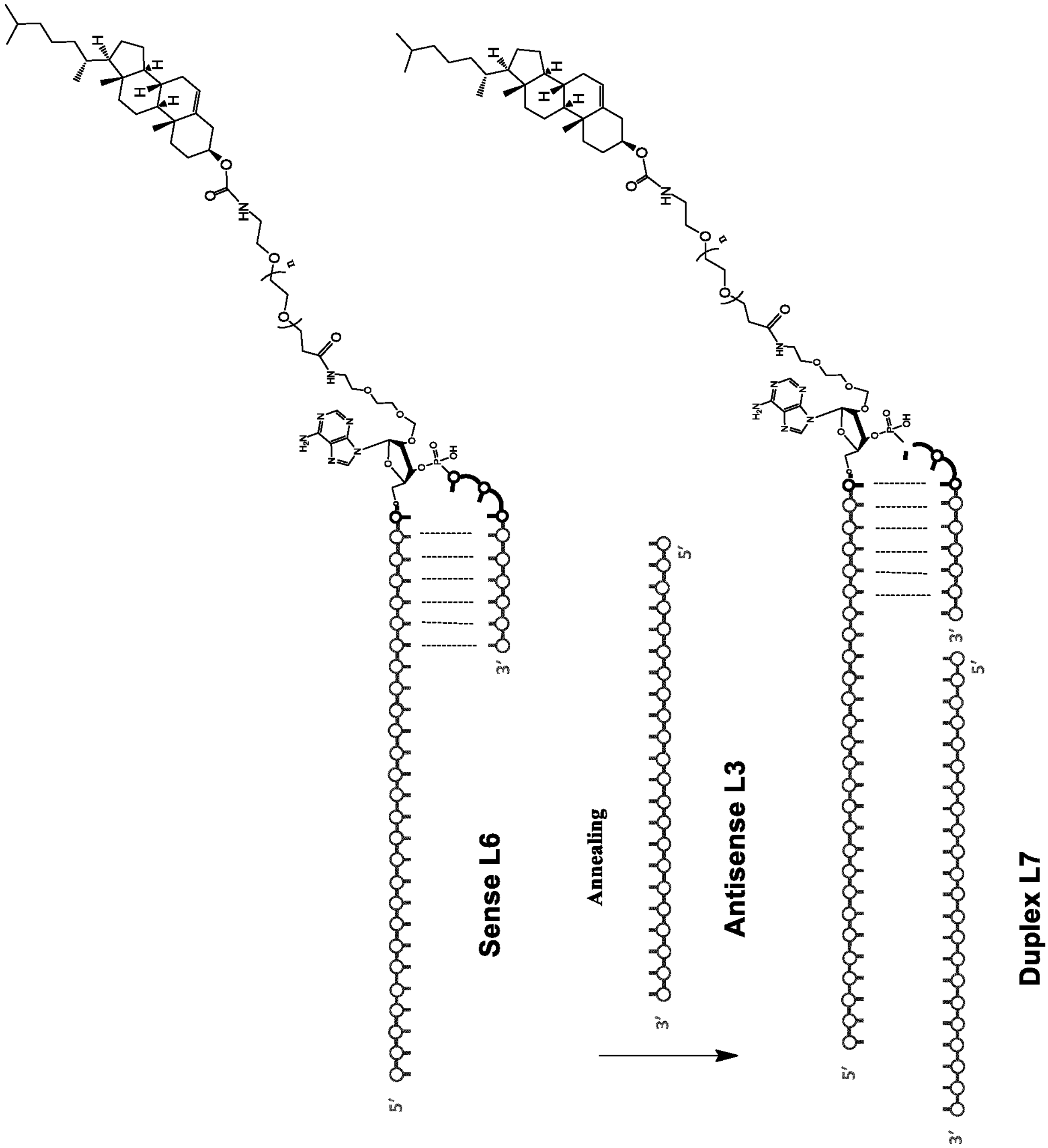
5'
3'
Sense L6
Annealing
5'
3'
Antisense L3
3'
5'
5'
3'
Duplex L7

**SCHEME M: Synthesis of Cholesterol Nicked Tetraloop dsNA Conjugate with Multiple Cholesterol On The Loop - Post Solid Phase Conjugation via "Click" Chemsitry**

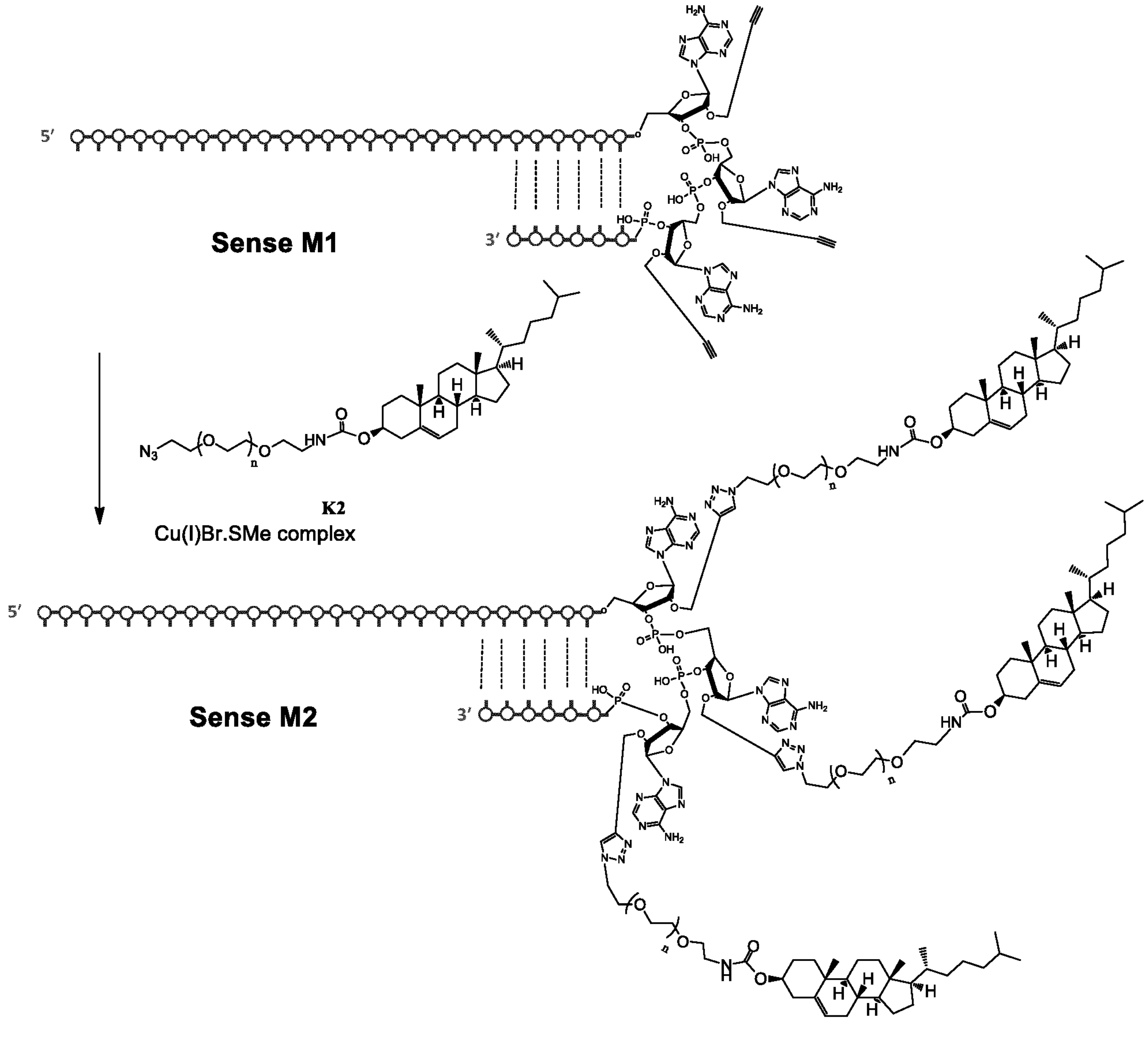


Sense M1

Sense M2

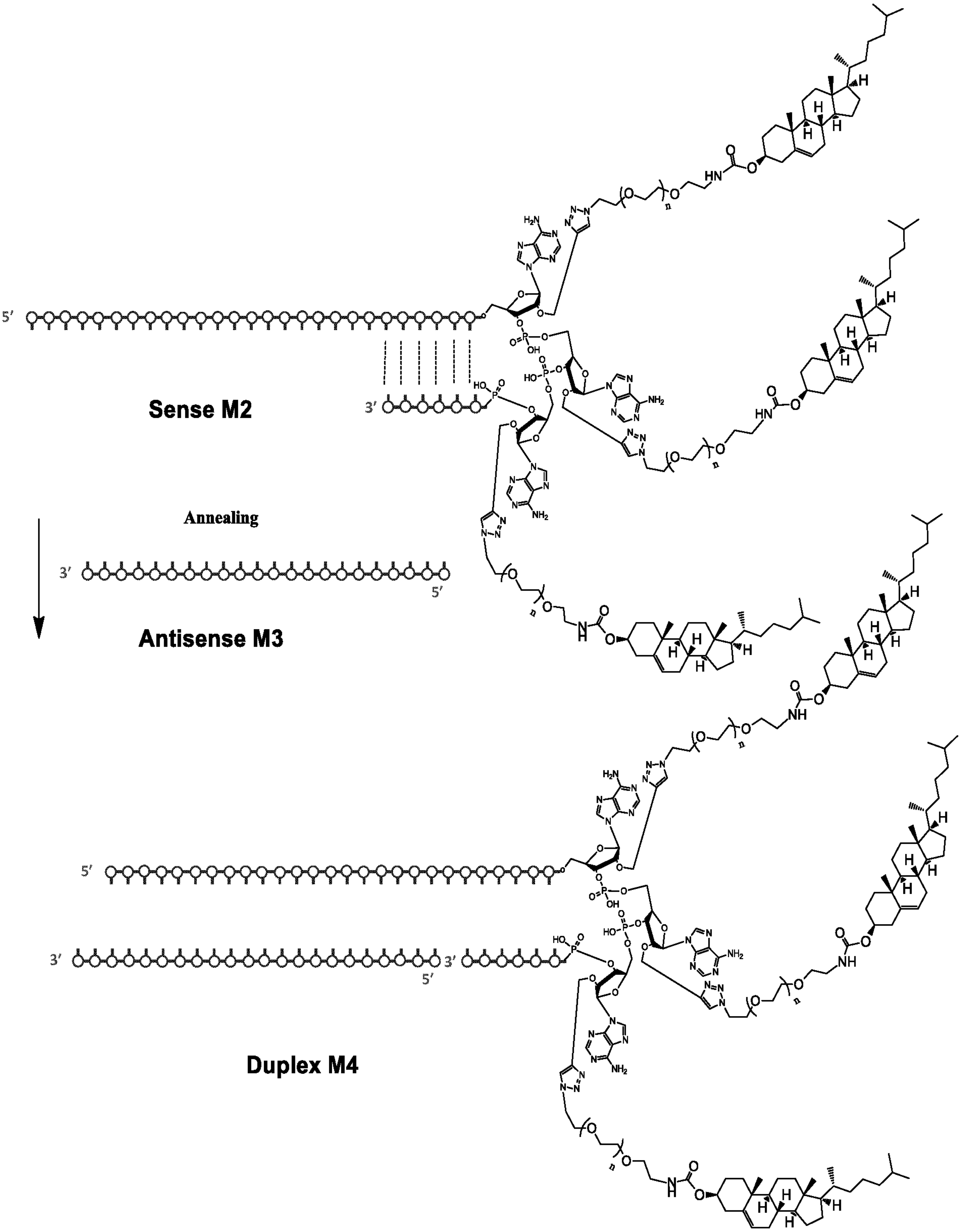


Sense M2

Antisense M3

Duplex M4

**SCHEME N: Synthesis of Cholesterol Nicked Tetraloop dsNA Conjugate with one Cholesterol On The Stem - Post Solid Phase Conjugation via "Click" Chemsitry**

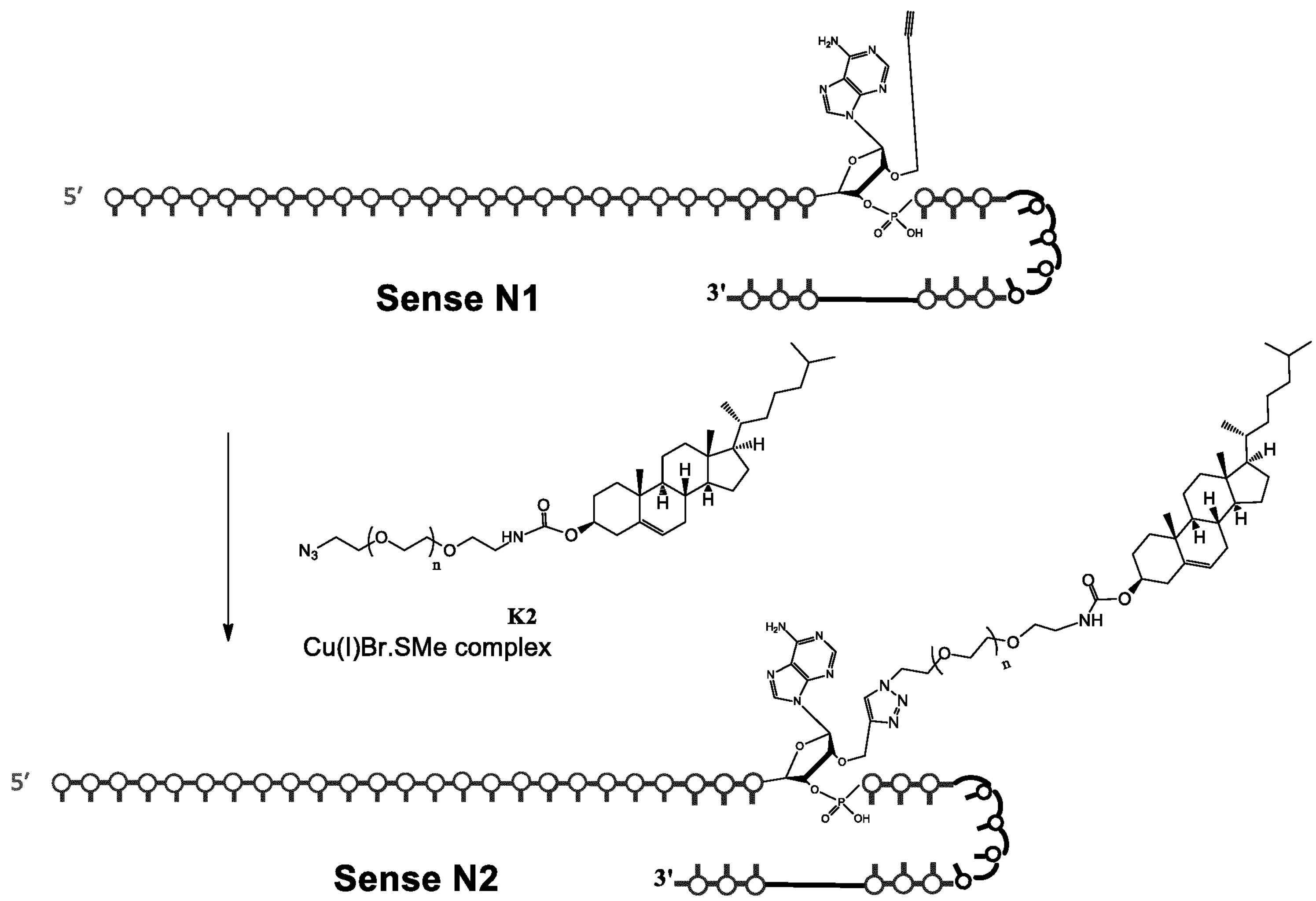


Sense N1

K2

Sense N2

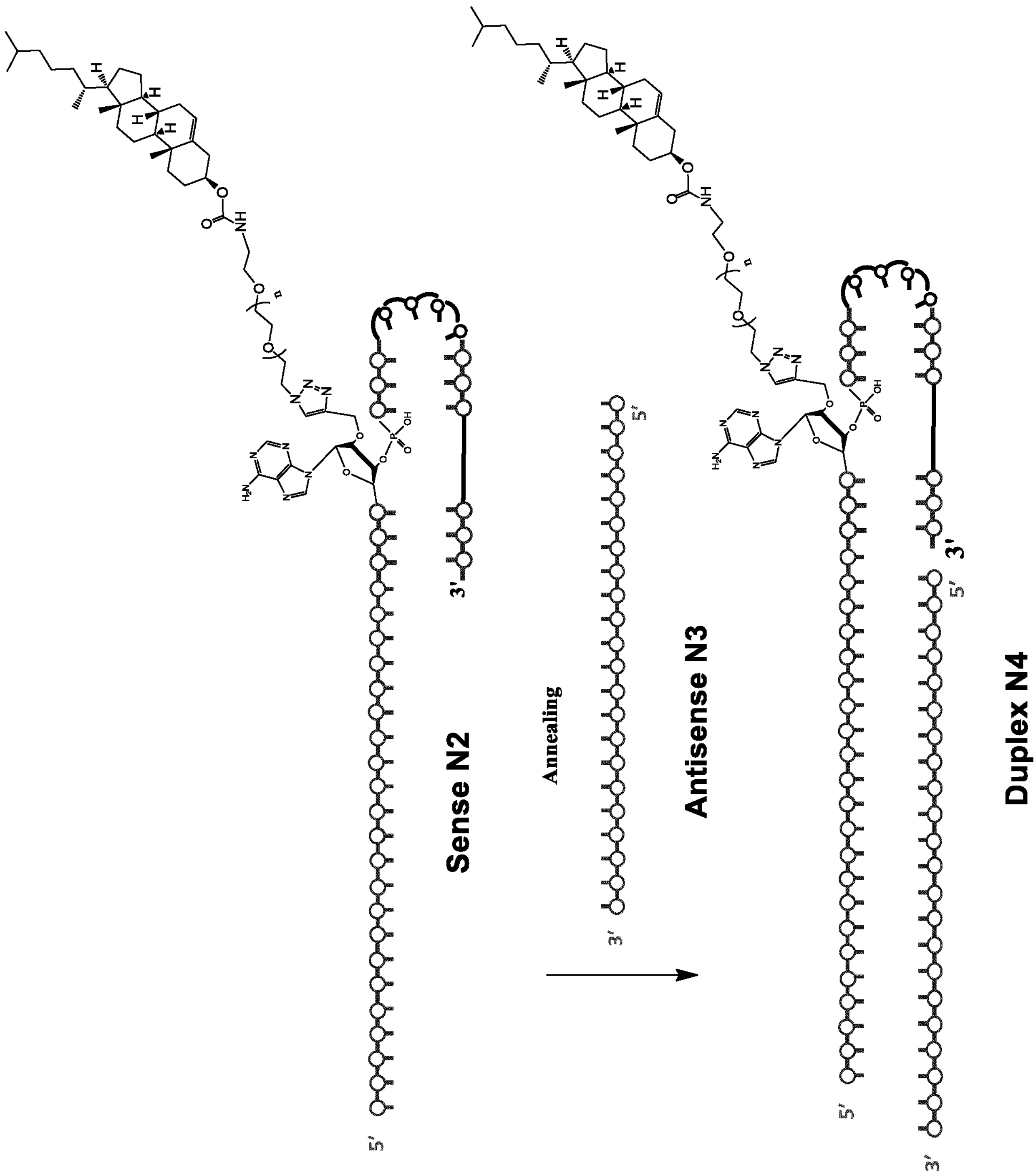
Sense N2
Annealing
Antisense N3
Duplex N4
5'
3'

**SCHEME O: Synthesis of Cholesterol Nicked Tetraloop dsNA Conjugate with Multiple Cholesterol On The Stem - Post Solid Phase Conjugation via "Click" Chemsitry**

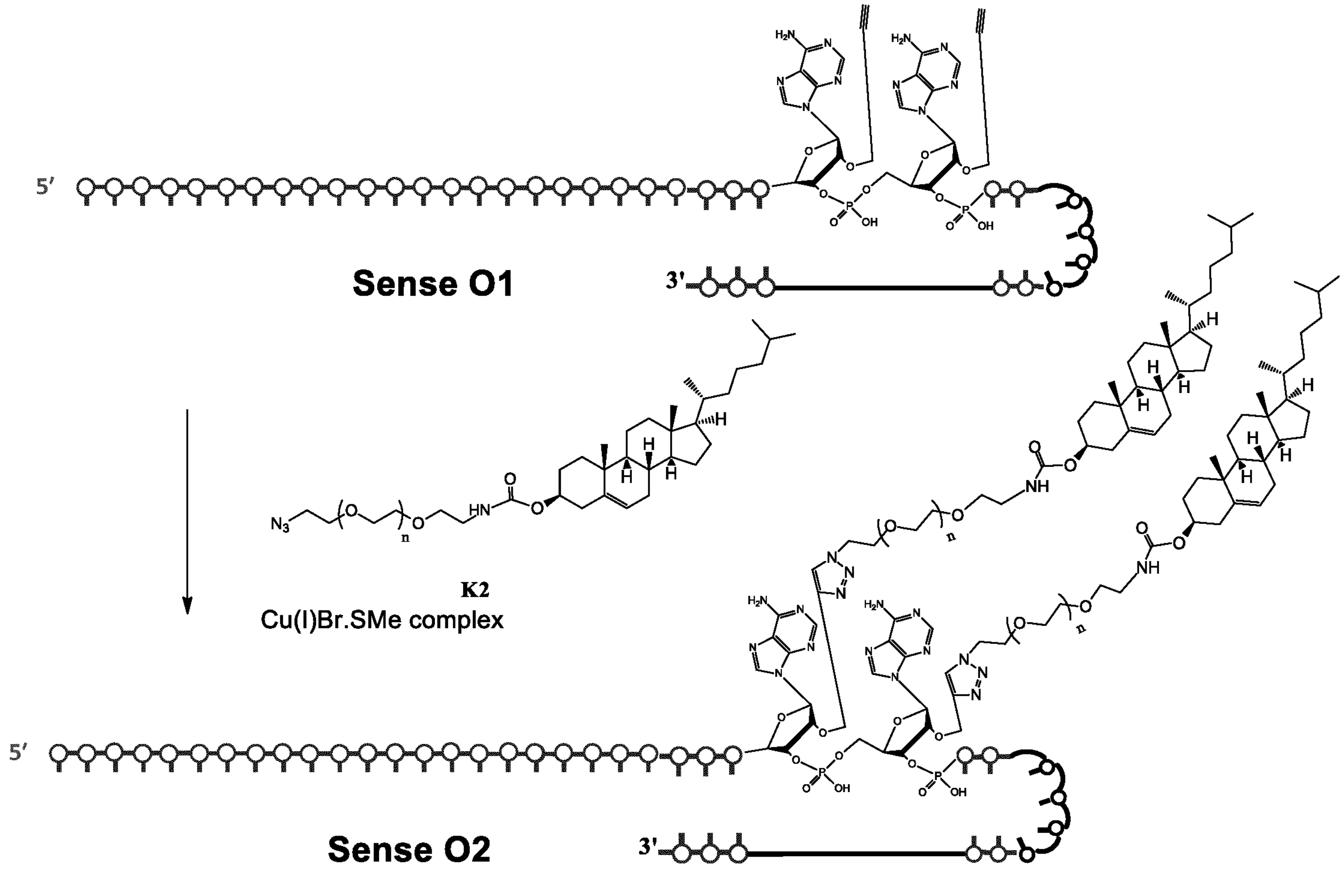

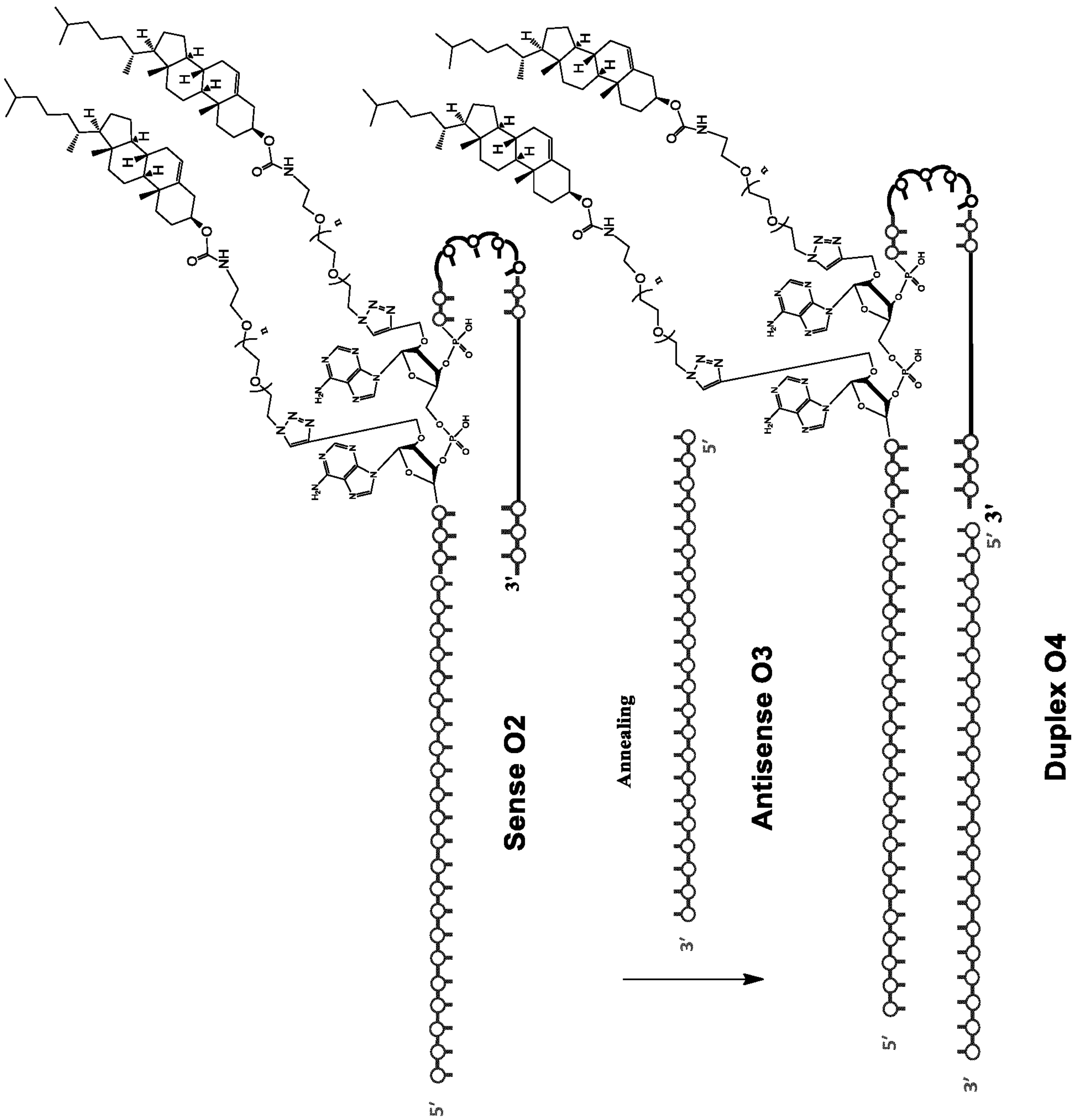


Sense O2

Antisense O3

Duplex O4

**SCHEME P: Synthesis of Cholesterol dsNA Conjugate with One Cholesterol On The 5'-Extension of Antisense - Post Solid Phase Conjugation via "Click" Chemsitry**

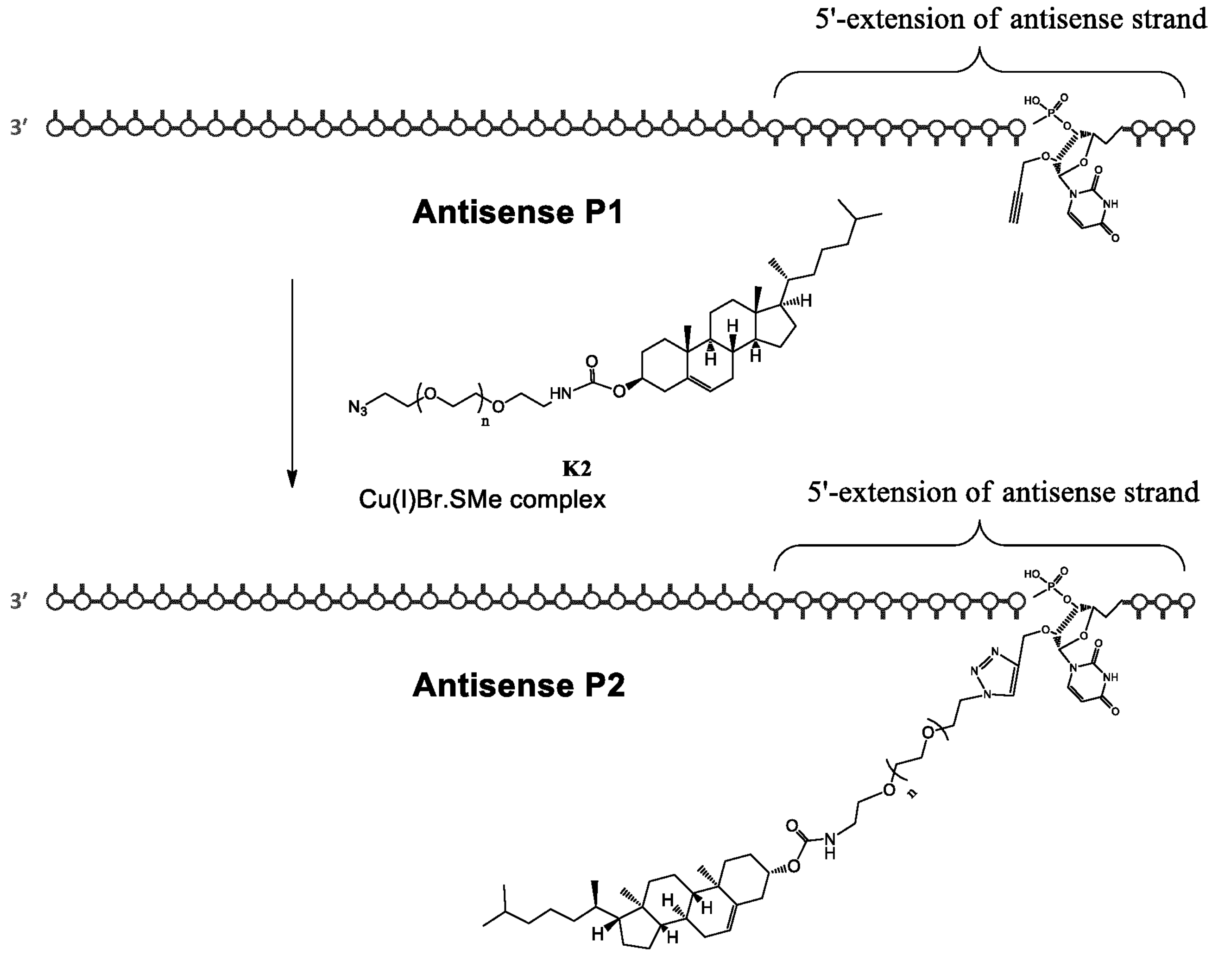


Antisense P1

Antisense P2

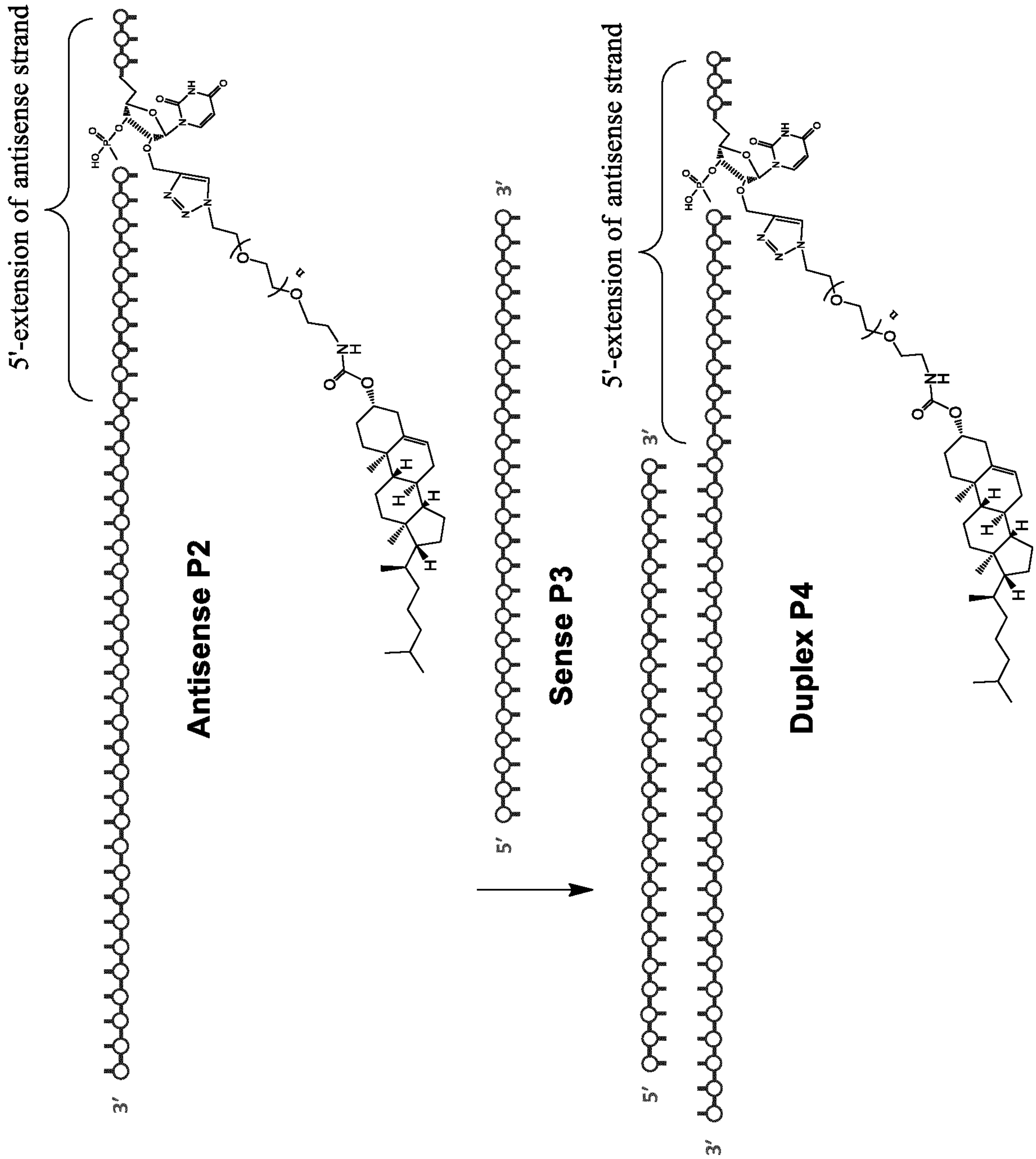


Antisense P2

Sense P3

Duplex P4

**SCHEME P1: Solid Phase Synthesis of Cholesterol dsNA Conjugate with One Cholesterol On The 5'-Extension of Antisense Using Cholesterol Phosphoroamidite**

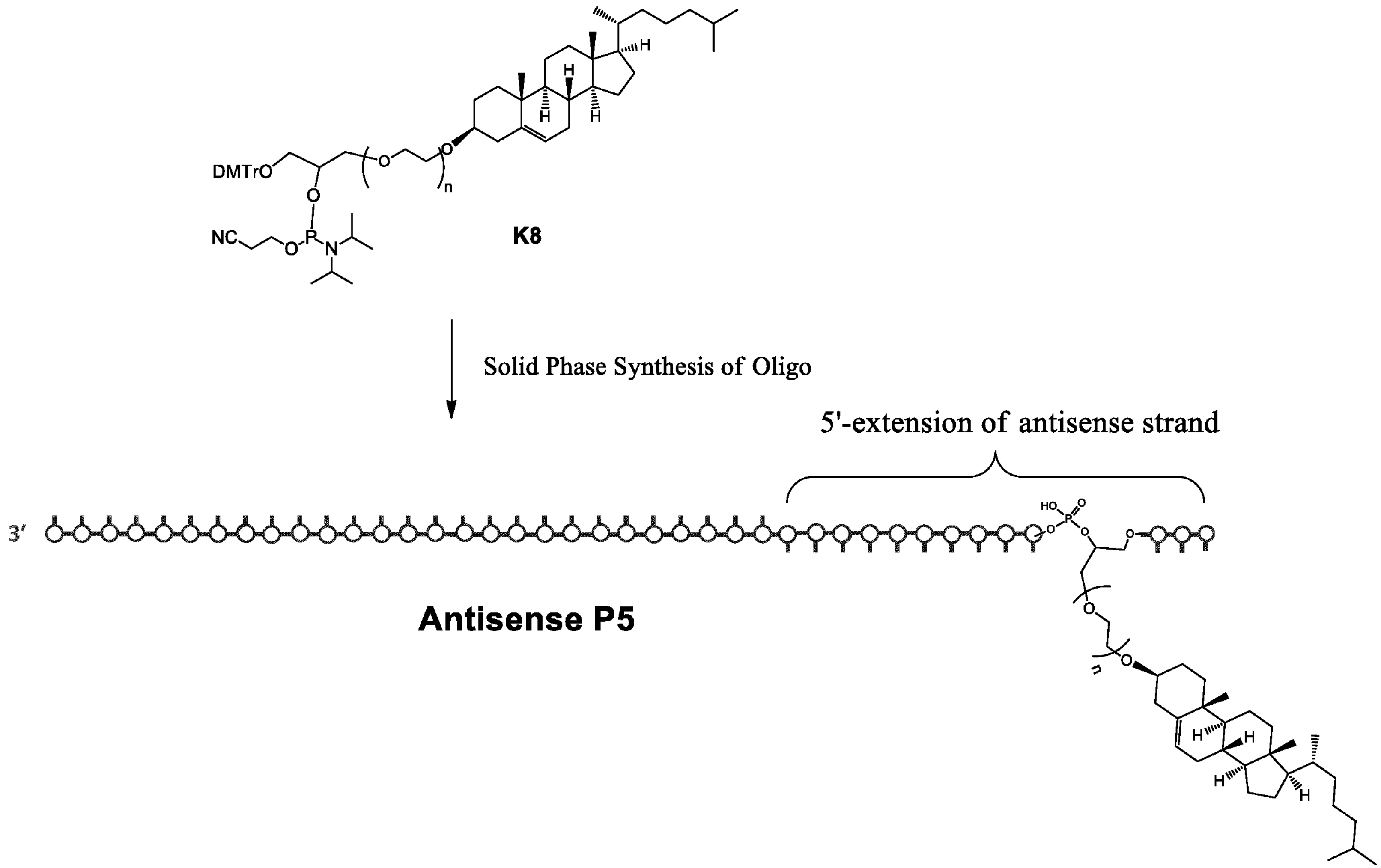


K8

Antisense P5

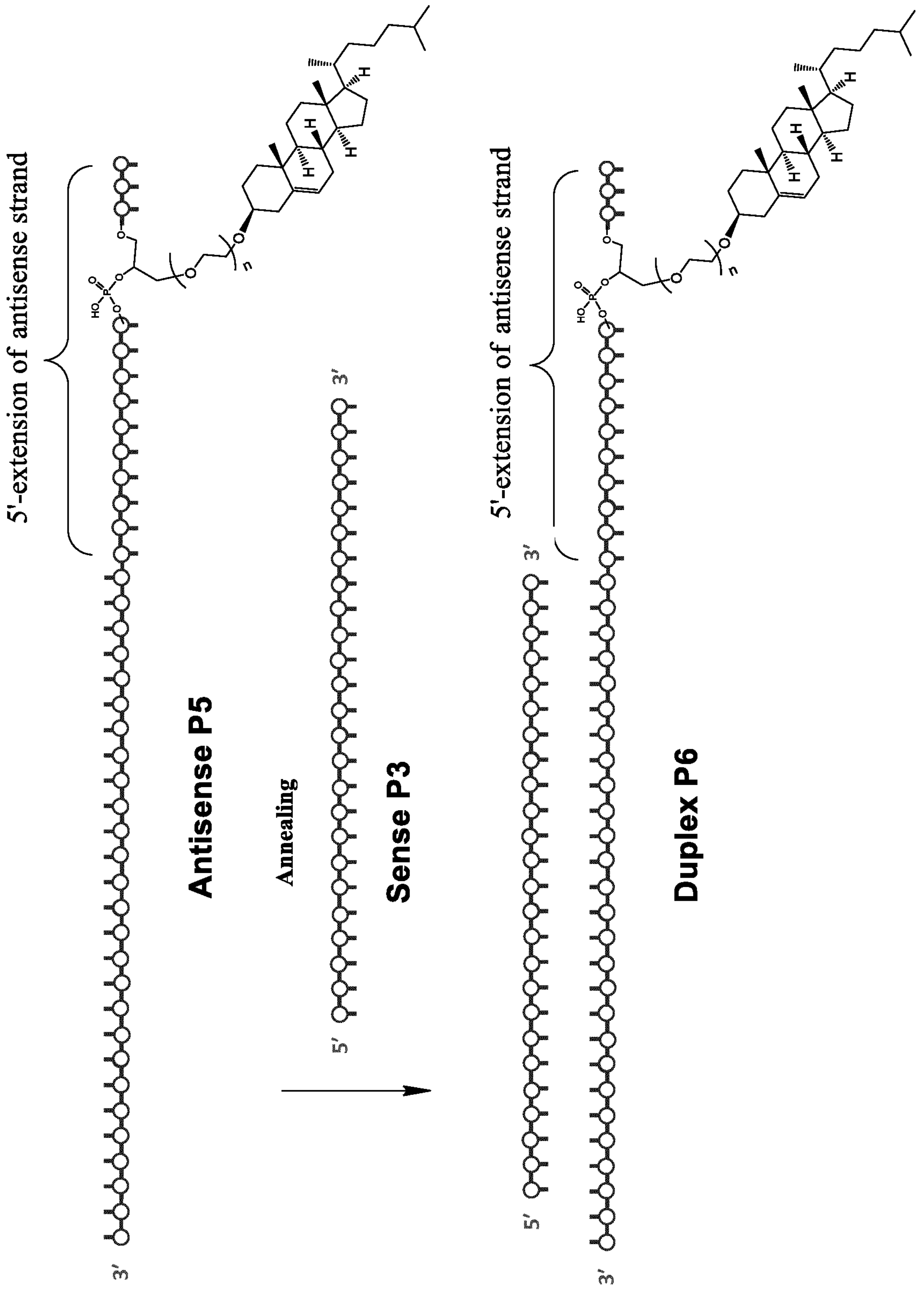
5'-extension of antisense strand
3'
Antisense P5
Annealing
5'
3'
Sense P3
5'-extension of antisense strand
3'
5'
3'
Duplex P6

**SCHEME Q: Synthesis of Cholesterol dsNA Conjugate with Multiple Cholesterols On The 5'-Extension of Antisense - Post Solid Phase Synthesis via "Click" Chemistry**

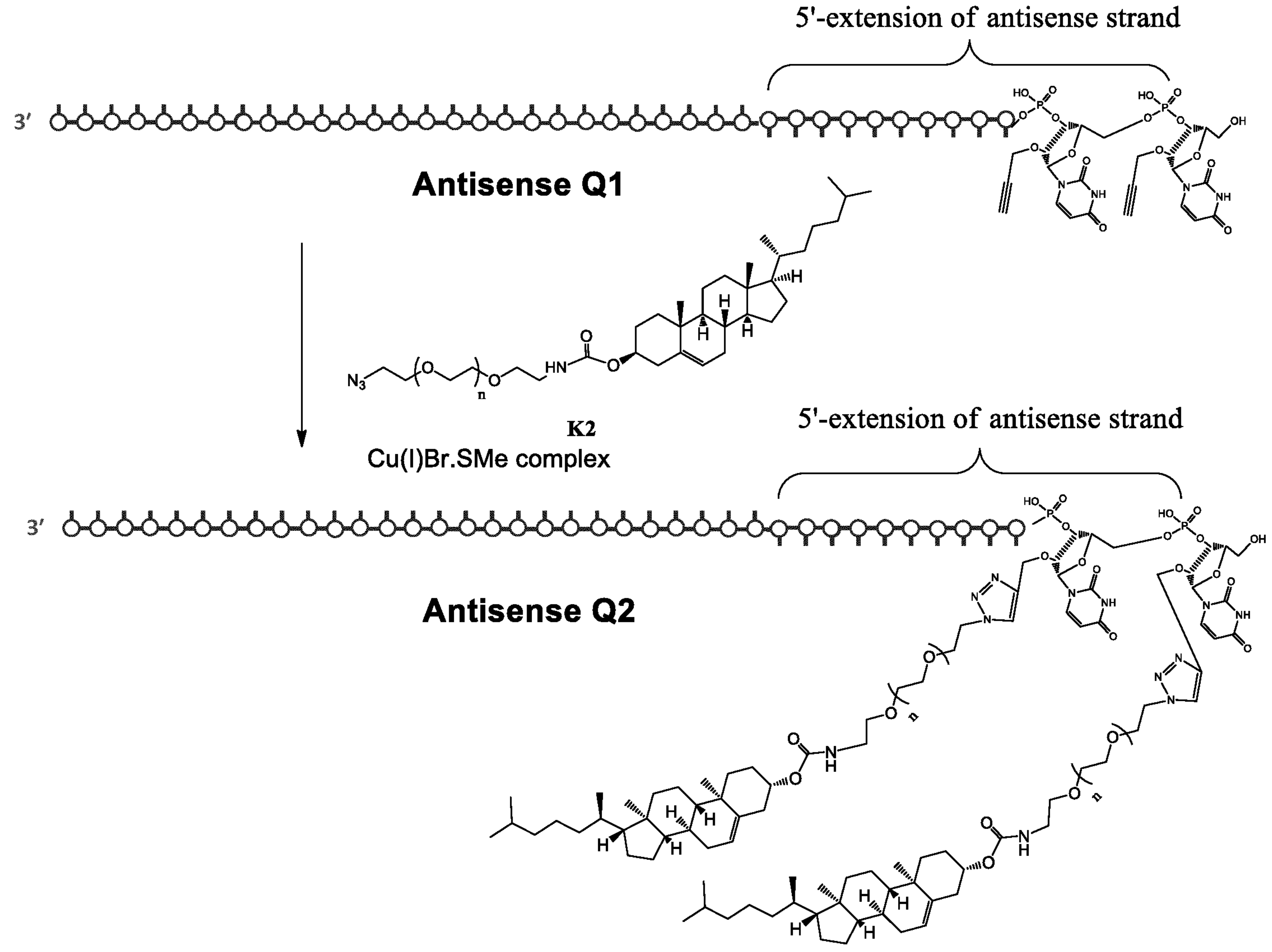

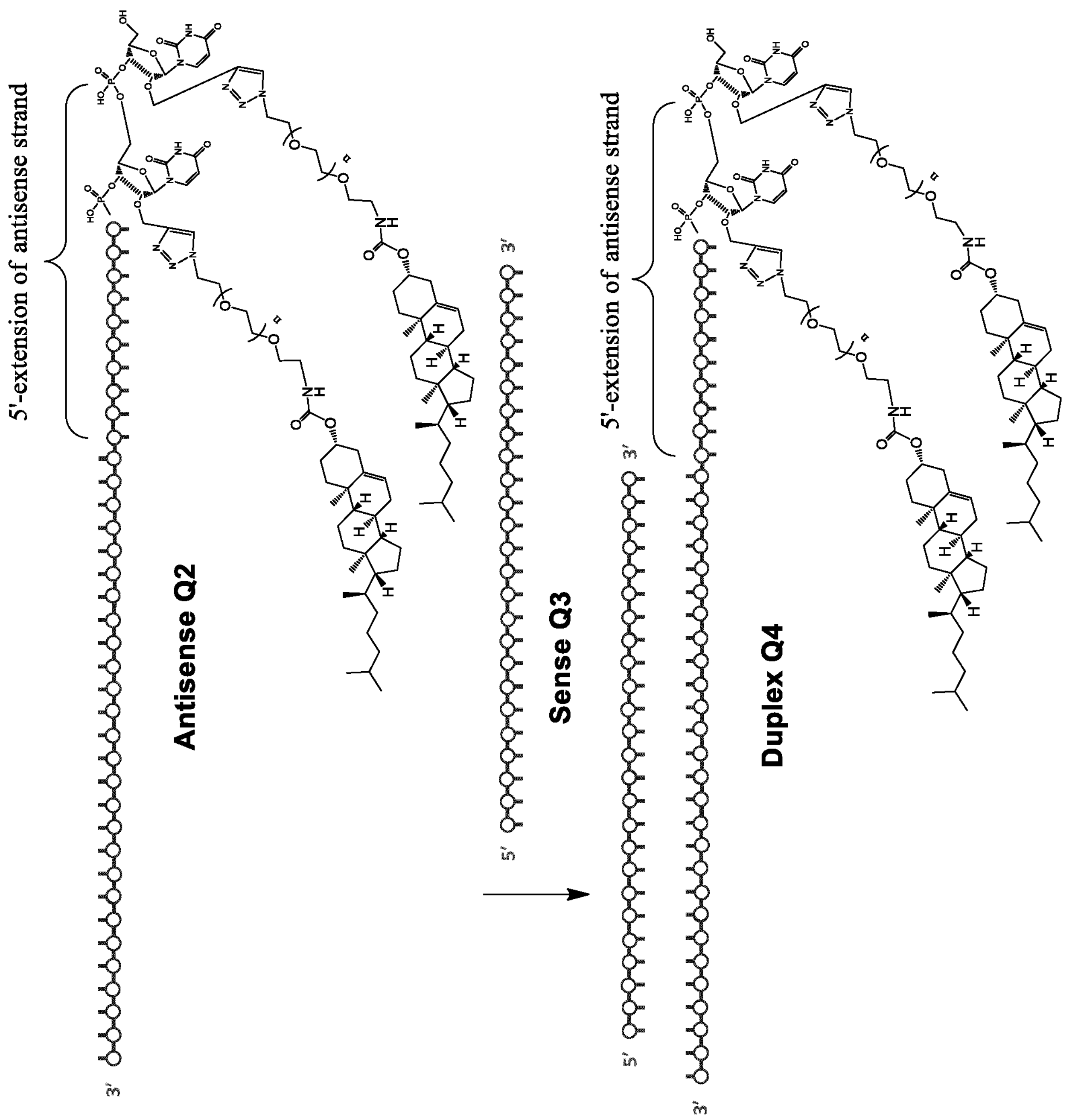


Antisense Q2

Sense Q3

Duplex Q4

**SCHEME Q1: Solid Phase Synthesis of Cholesterol dsNA Conjugate with Multiple Cholesterols On The 5'-Extension of Antisense Using Cholesterol Phosphoroamidite**

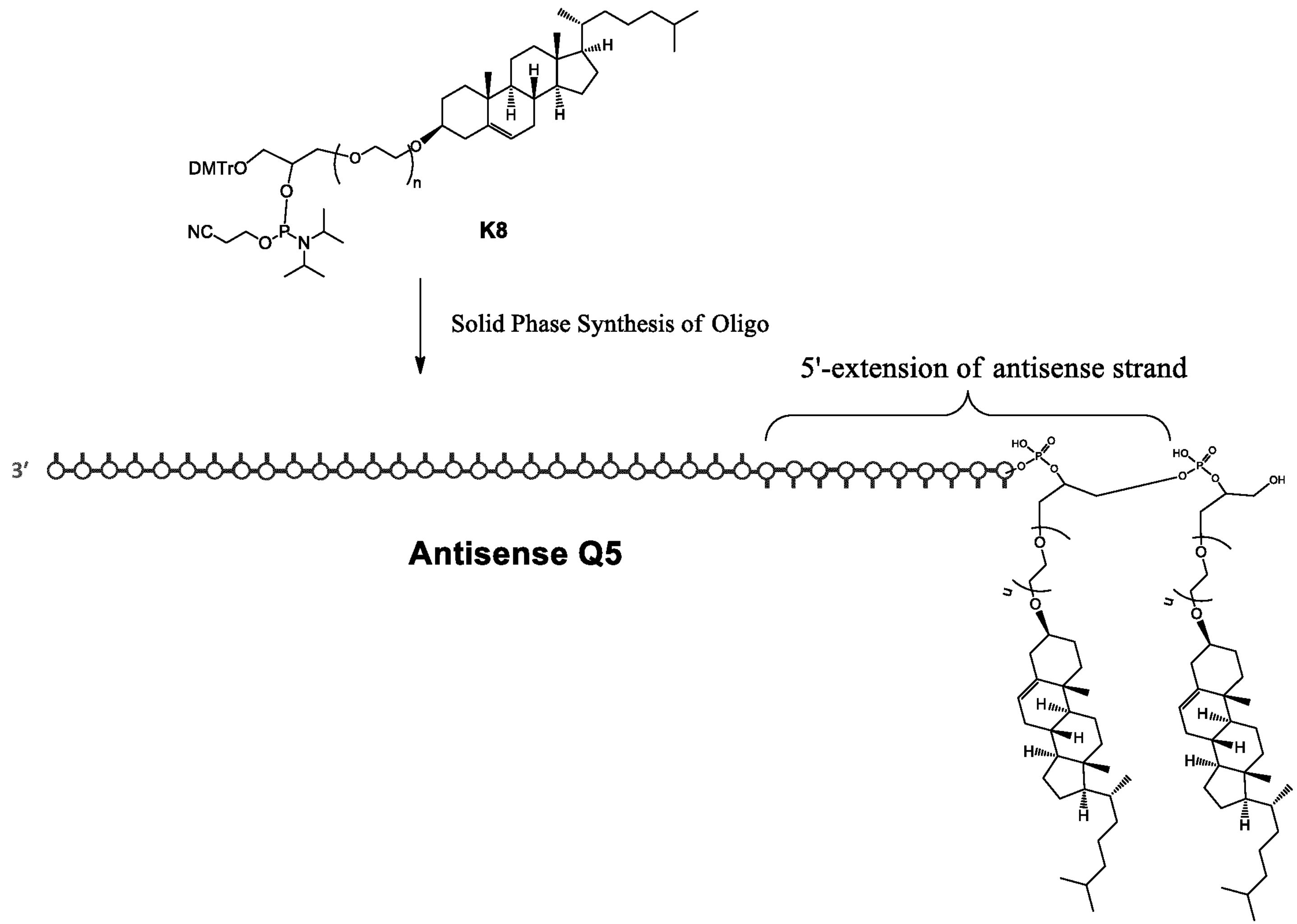


K8

Antisense Q5

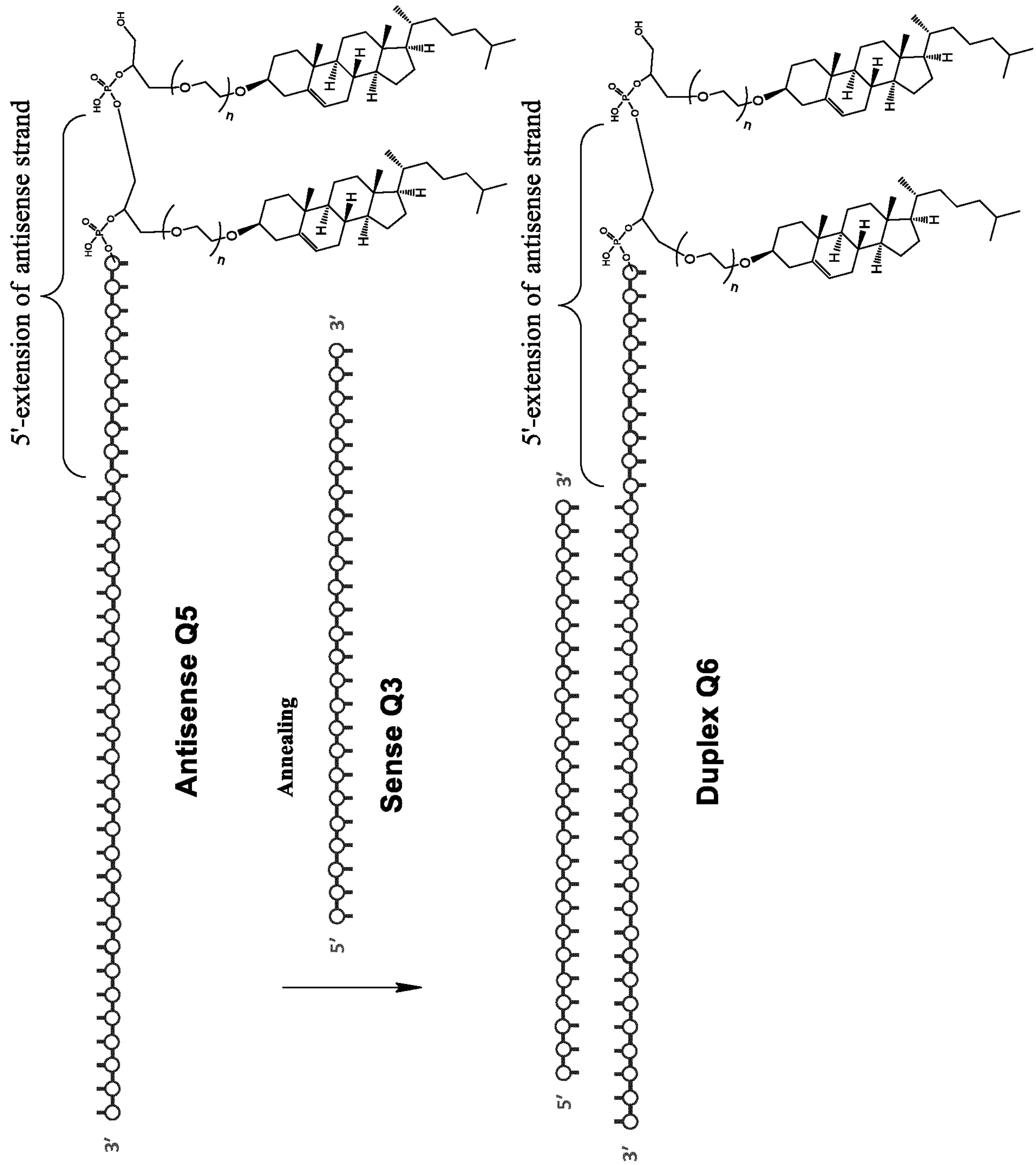
5'-extension of antisense strand
Antisense Q5
Annealing
Sense Q3
Duplex Q6
5'-extension of antisense strand
3'
5'
n

**SCHEME R: Synthesis of Cholesterol dsNA Conjugate with One Cholesterol On The 5'-Extension of Sense - Post Solid Phase Conjugation via "Click" Chemsitry**

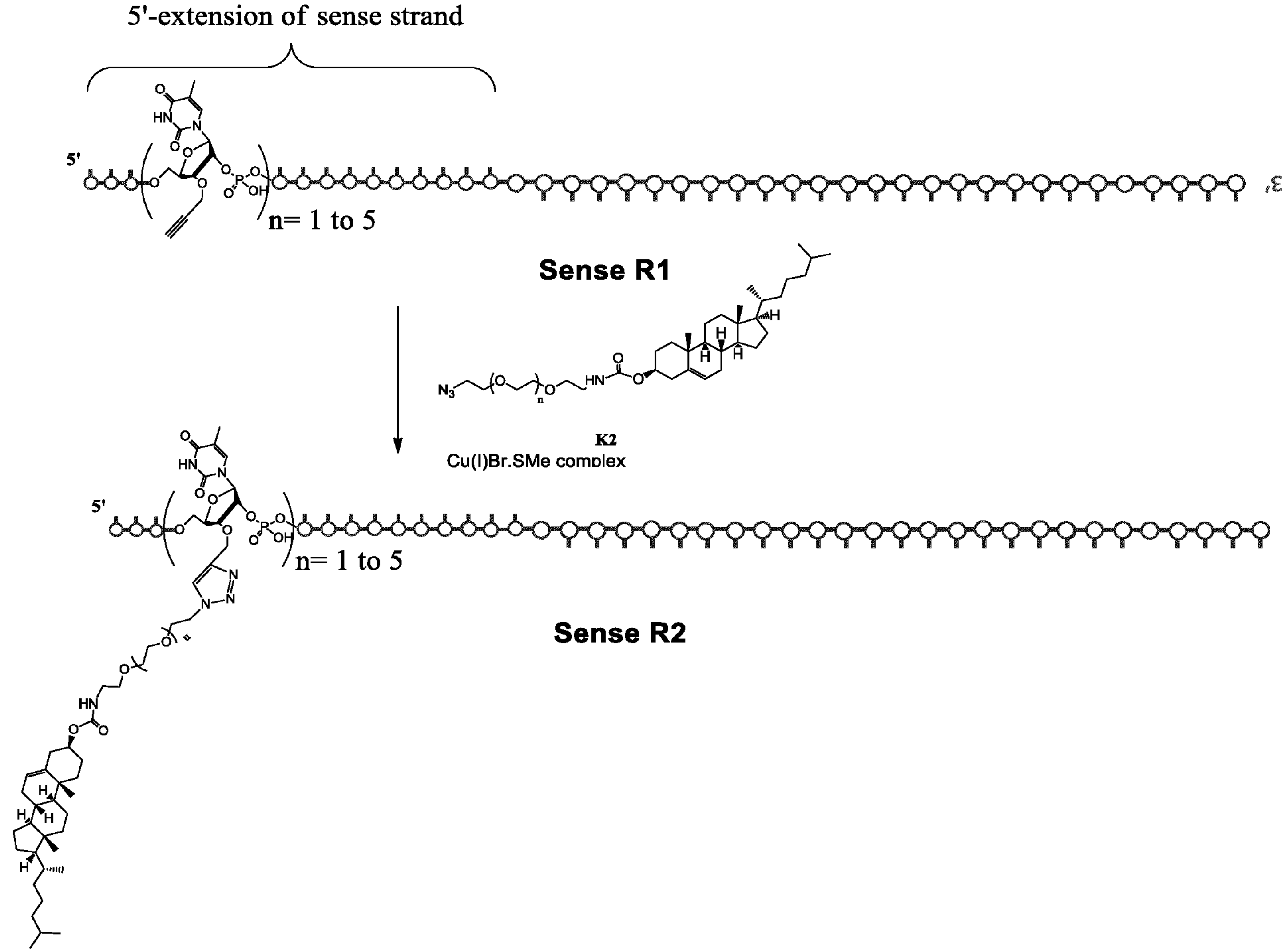

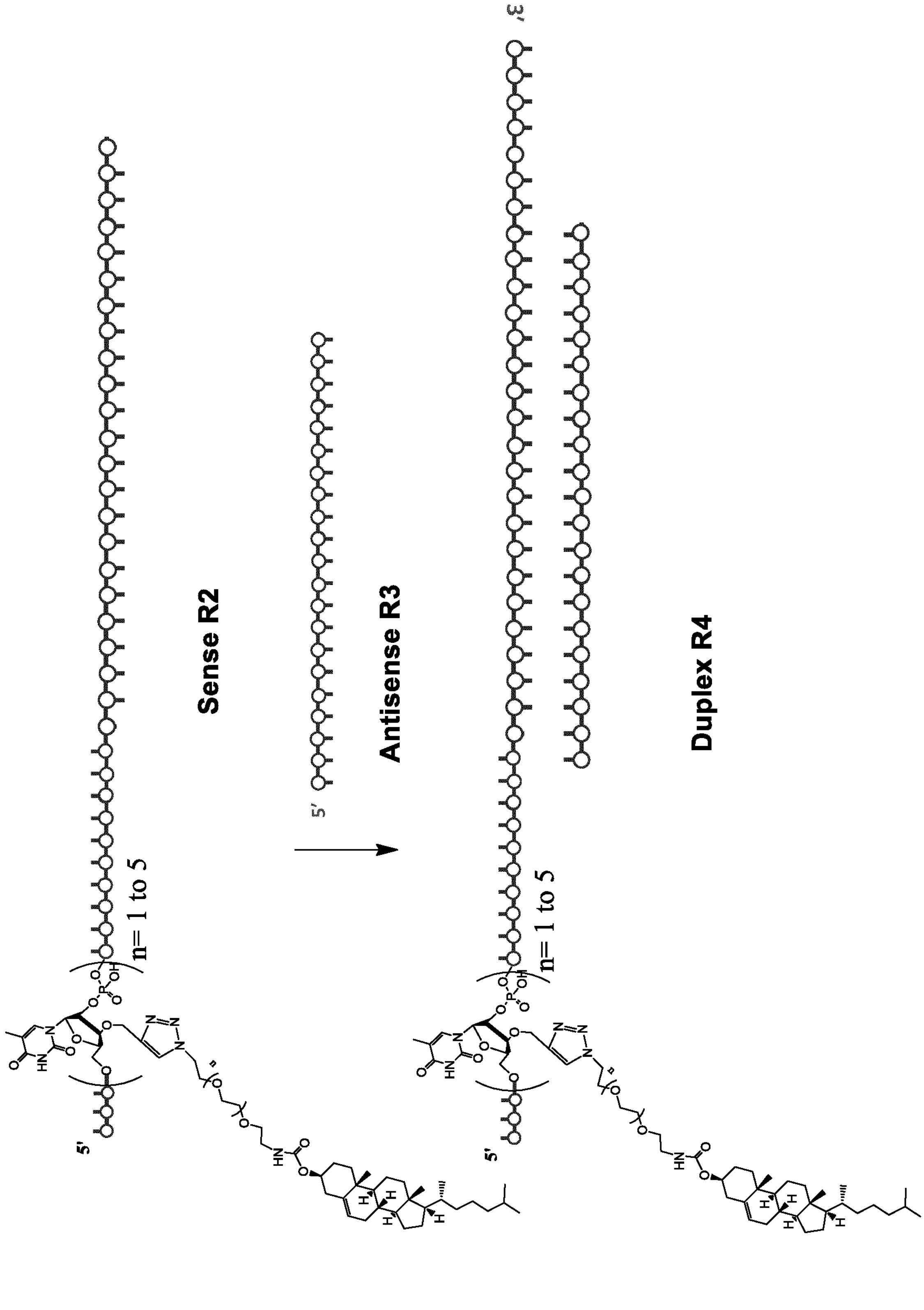

Sense R2

Antisense R3

Duplex R4

**SCHEME R1: Solid Phase Synthesis of Cholesterol dsNA Conjugate with One Cholesterol On The 5'-Extension of Sense Using Cholesterol Phosphoroamidite**

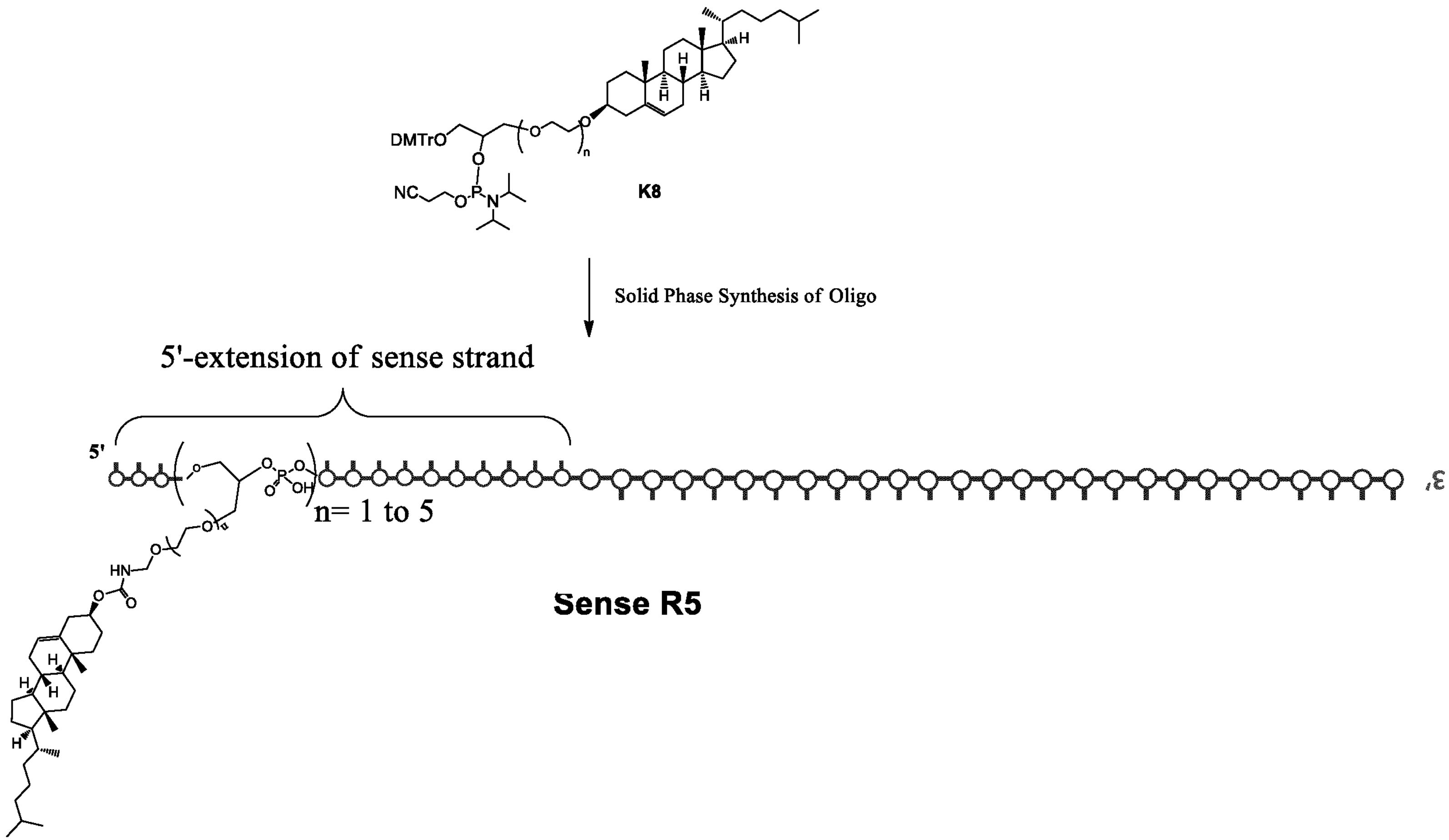


K8

Sense R5

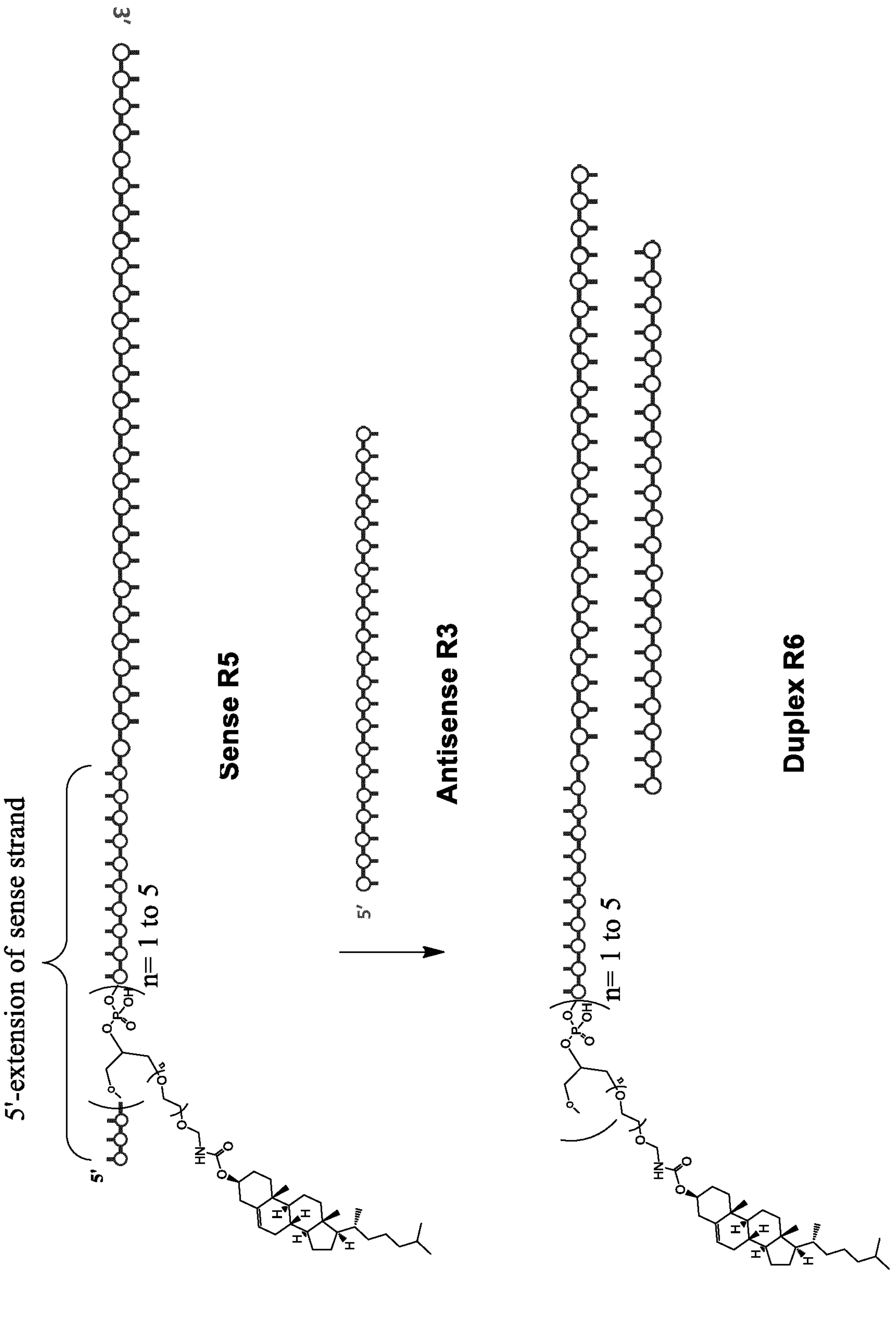

3'
5'
5'-extension of sense strand
n= 1 to 5
Sense R5
5'
Antisense R3
n= 1 to 5
Duplex R6

Scheme S

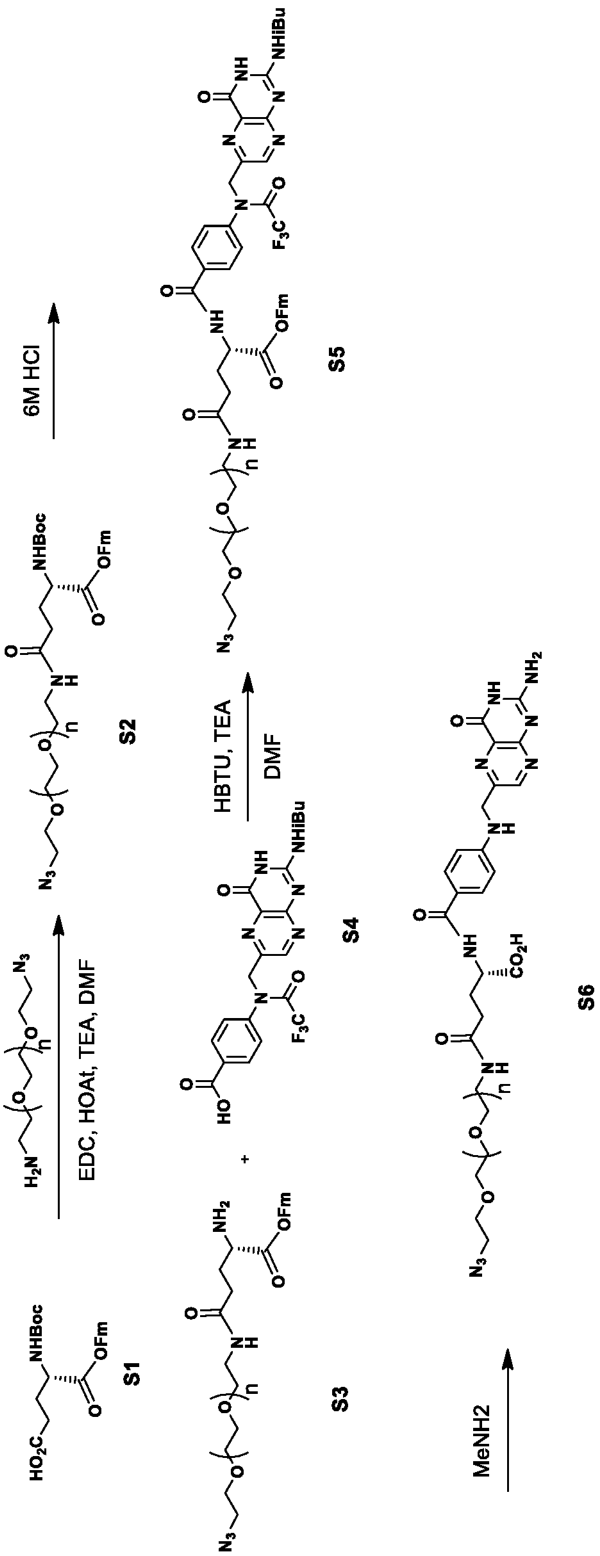

**Scheme S (Continued)**

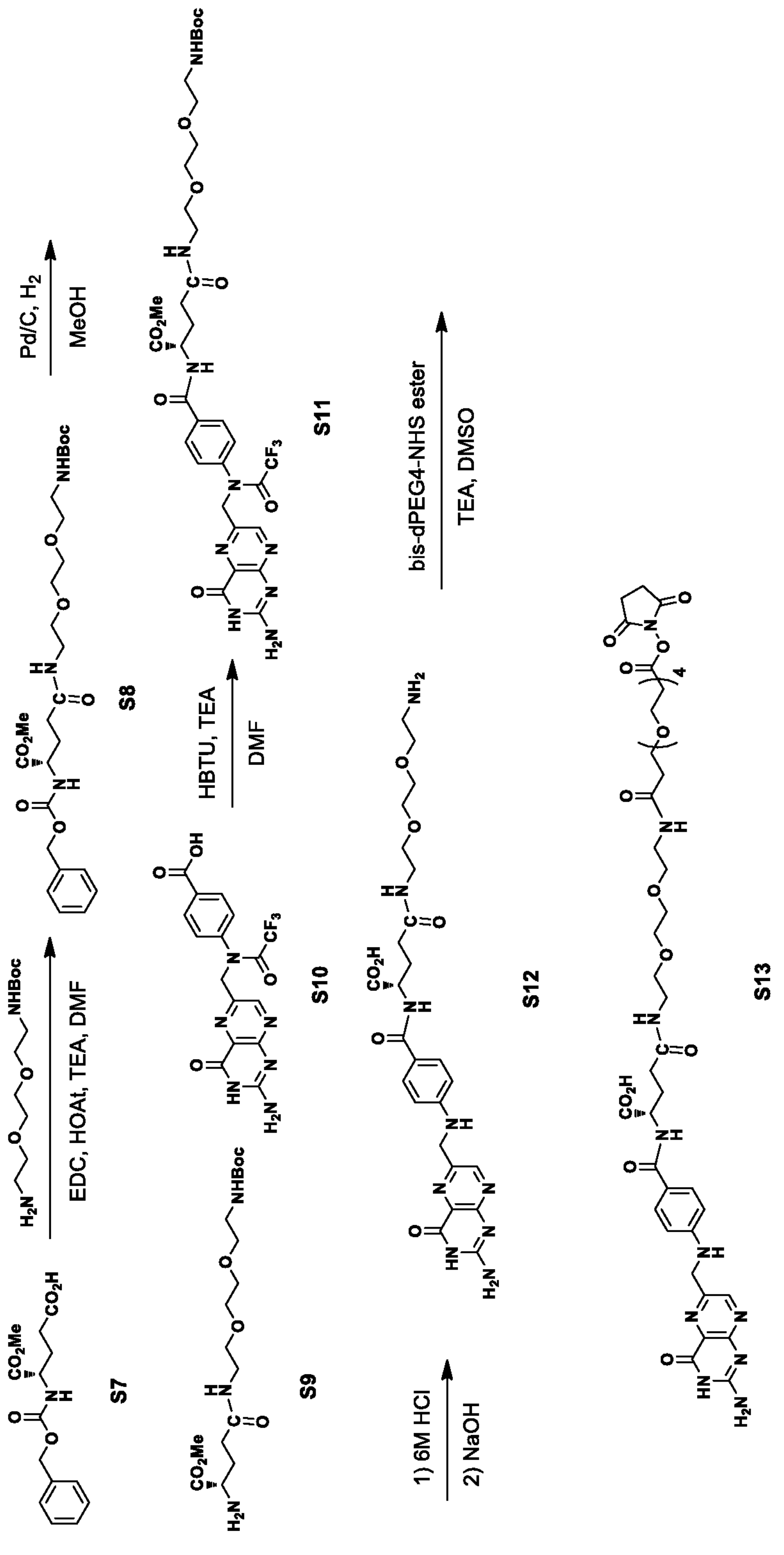


S7 S8 S9 S10 S11 S12 S13

Scheme T

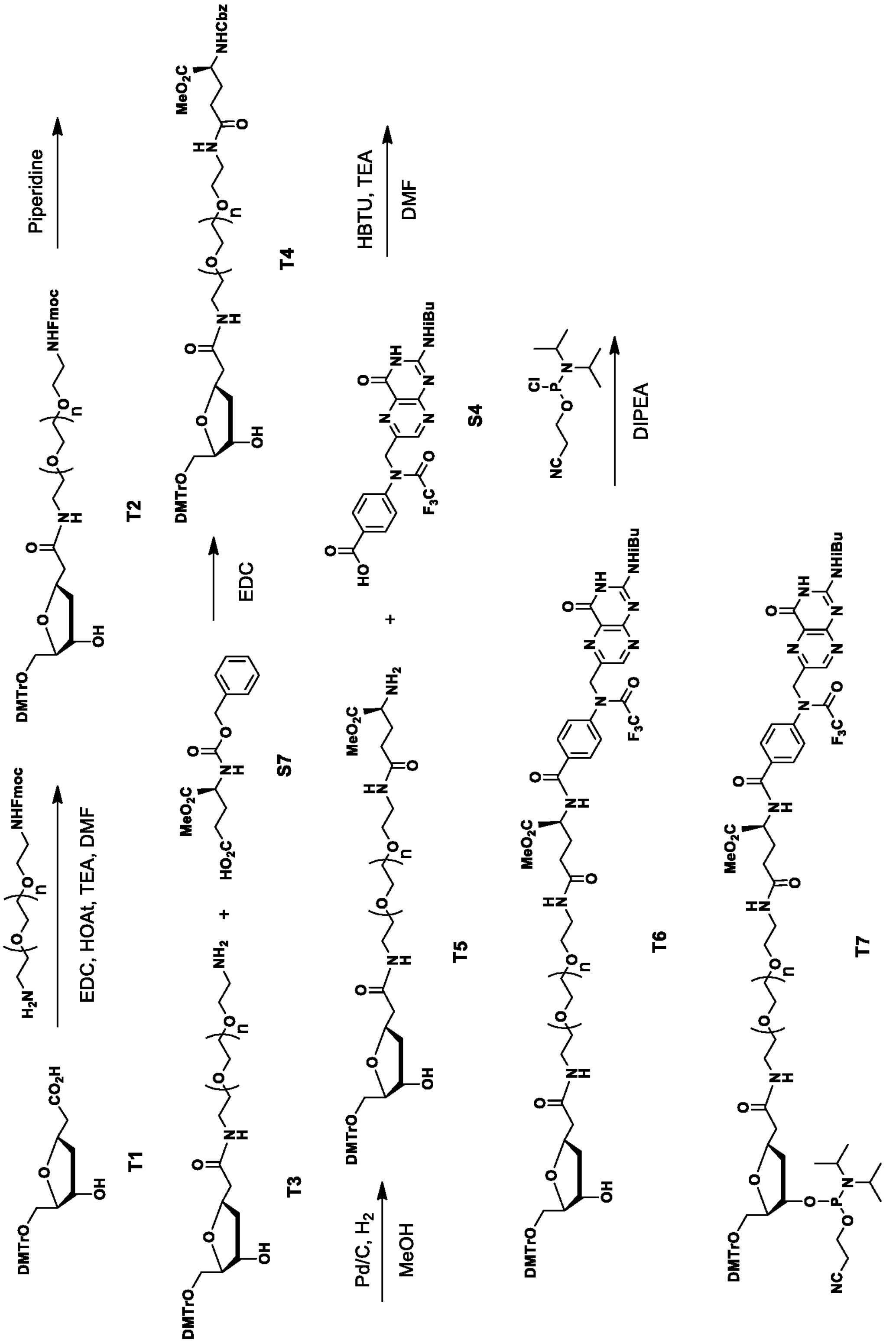


T1 T2 T3 S7 T4 S4 T5 T6 T7

**SCHEME U: Synthesis of Folate Nicked Tetraloop dsNA Conjugate with One Folate On The Loop - Post Solid Phase Conjugation via "Click" Chemsitry**

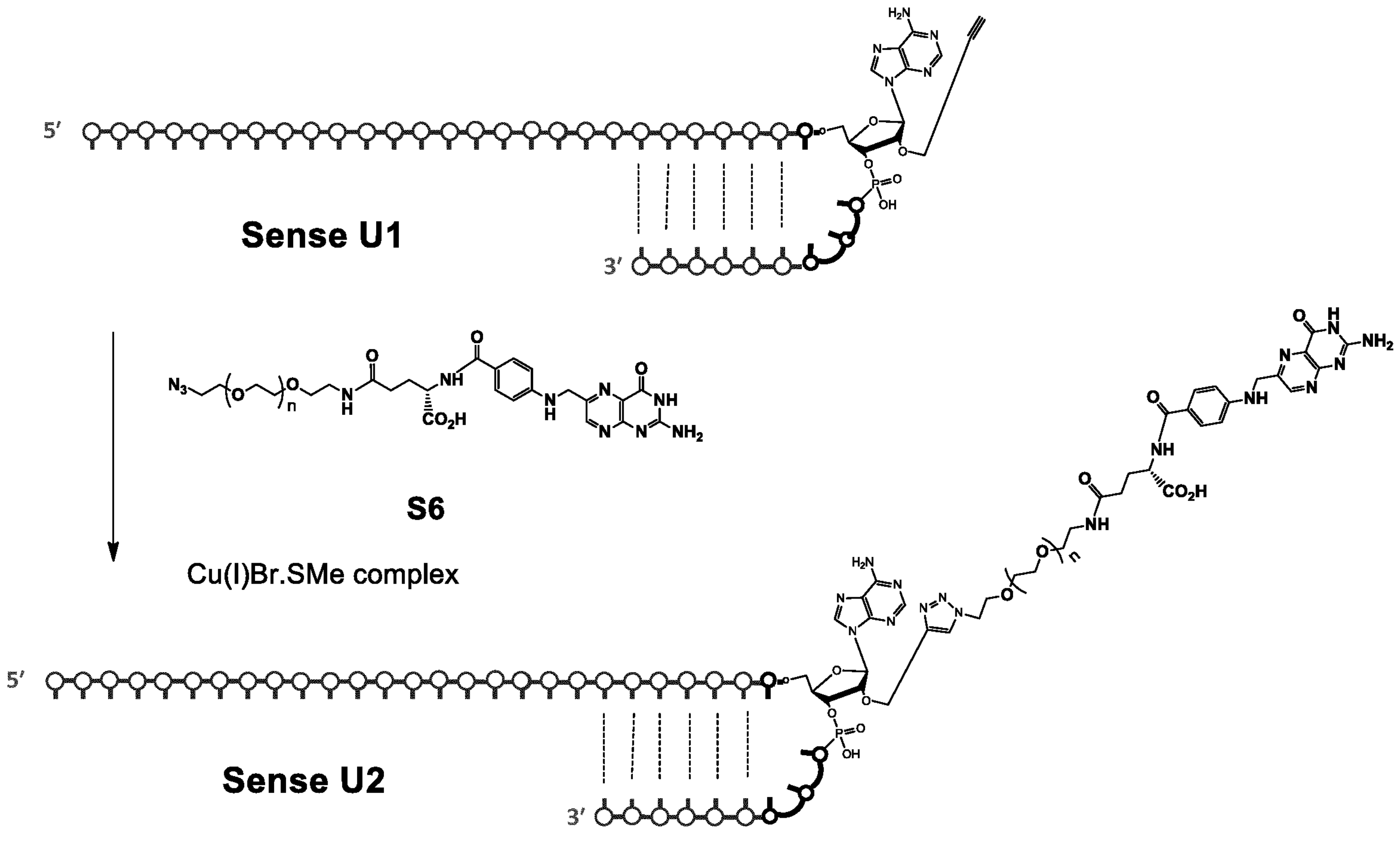


Sense U1

S6

Sense U2

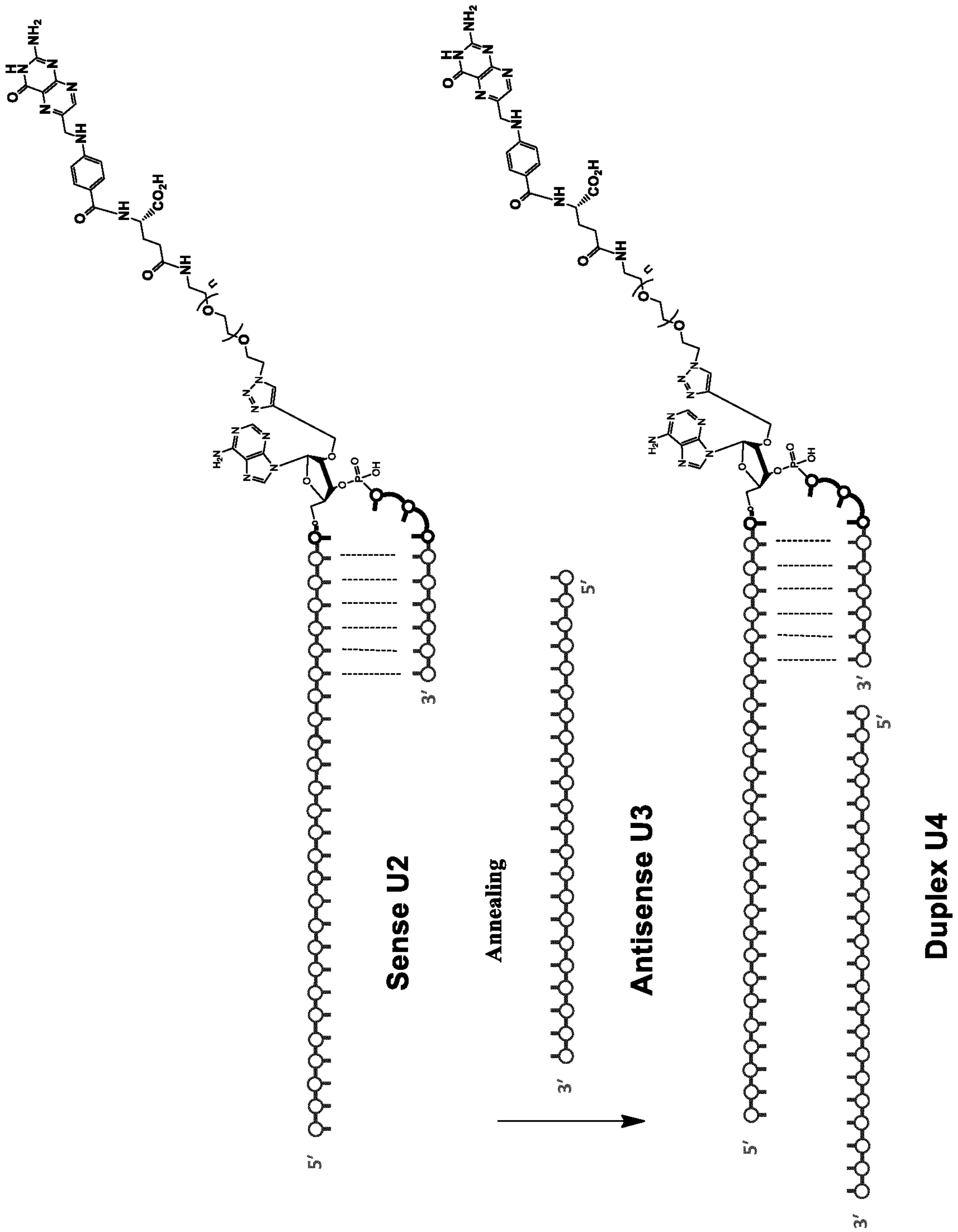
Sense U2
Annealing
Antisense U3
Duplex U4

**SCHEME U1: Synthesis of Folate Nicked Tetraloop dsNA Conjugate with One Folate On The Loop - Post Solid Phase Conjugation via Amide Chemsitry**

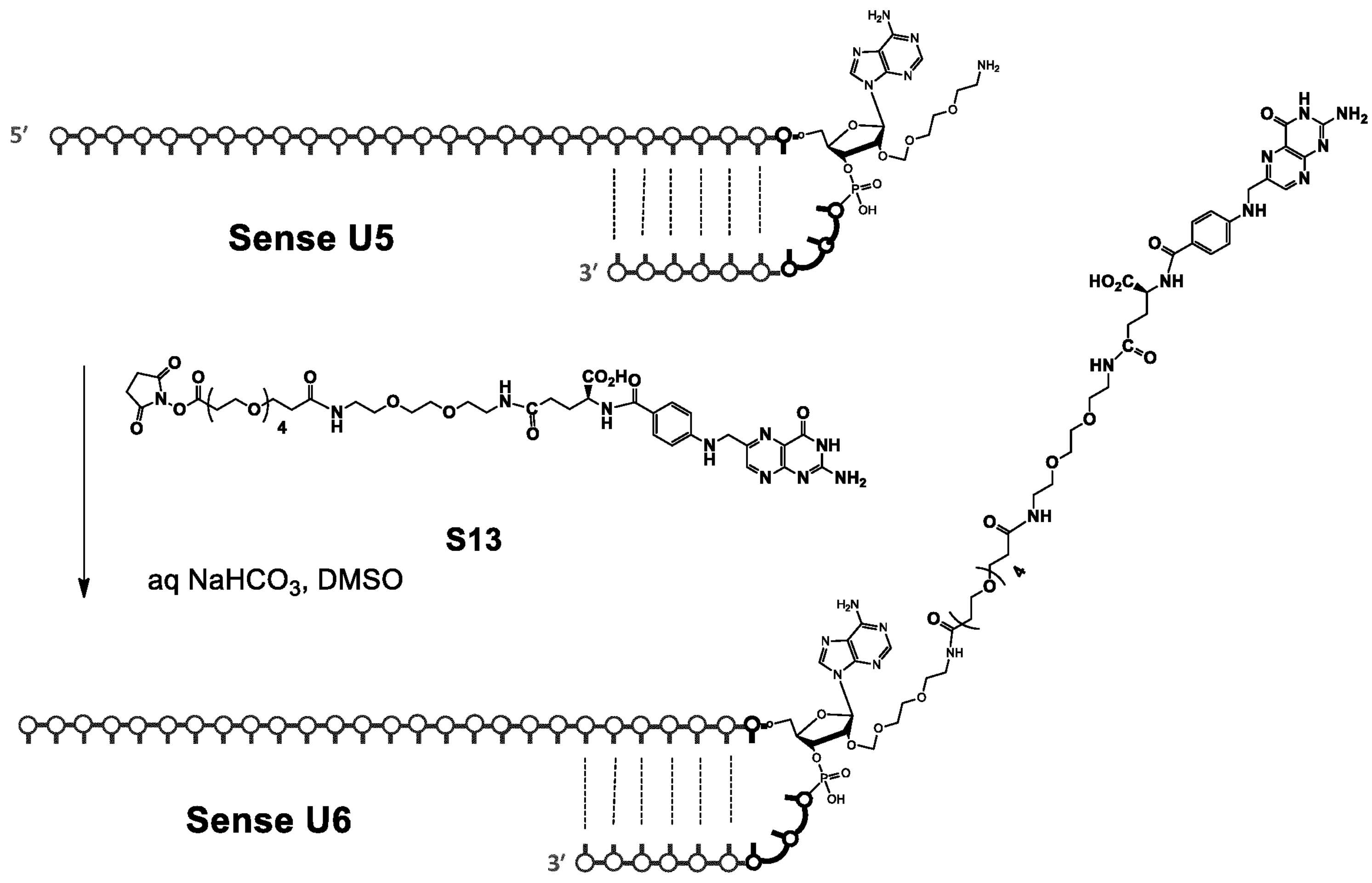


Sense U5

S13

Sense U6

**SCHEME V: Synthesis of Folate Nicked Tetraloop dsNA Conjugate with Multiple Folate On The Loop - Post Solid Phase Conjugation via "Click" Chemsitry**

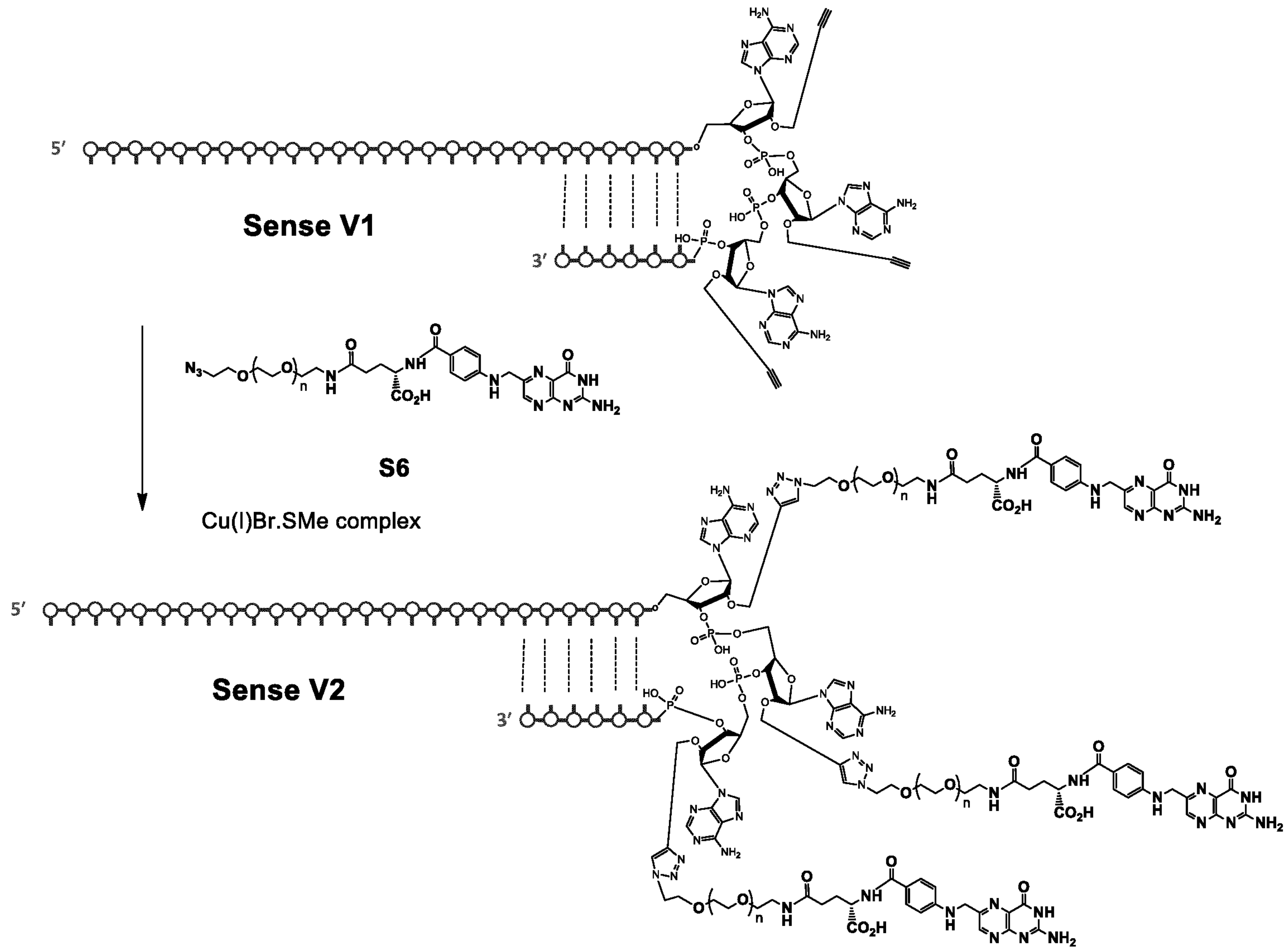


Sense V1

S6

Sense V2

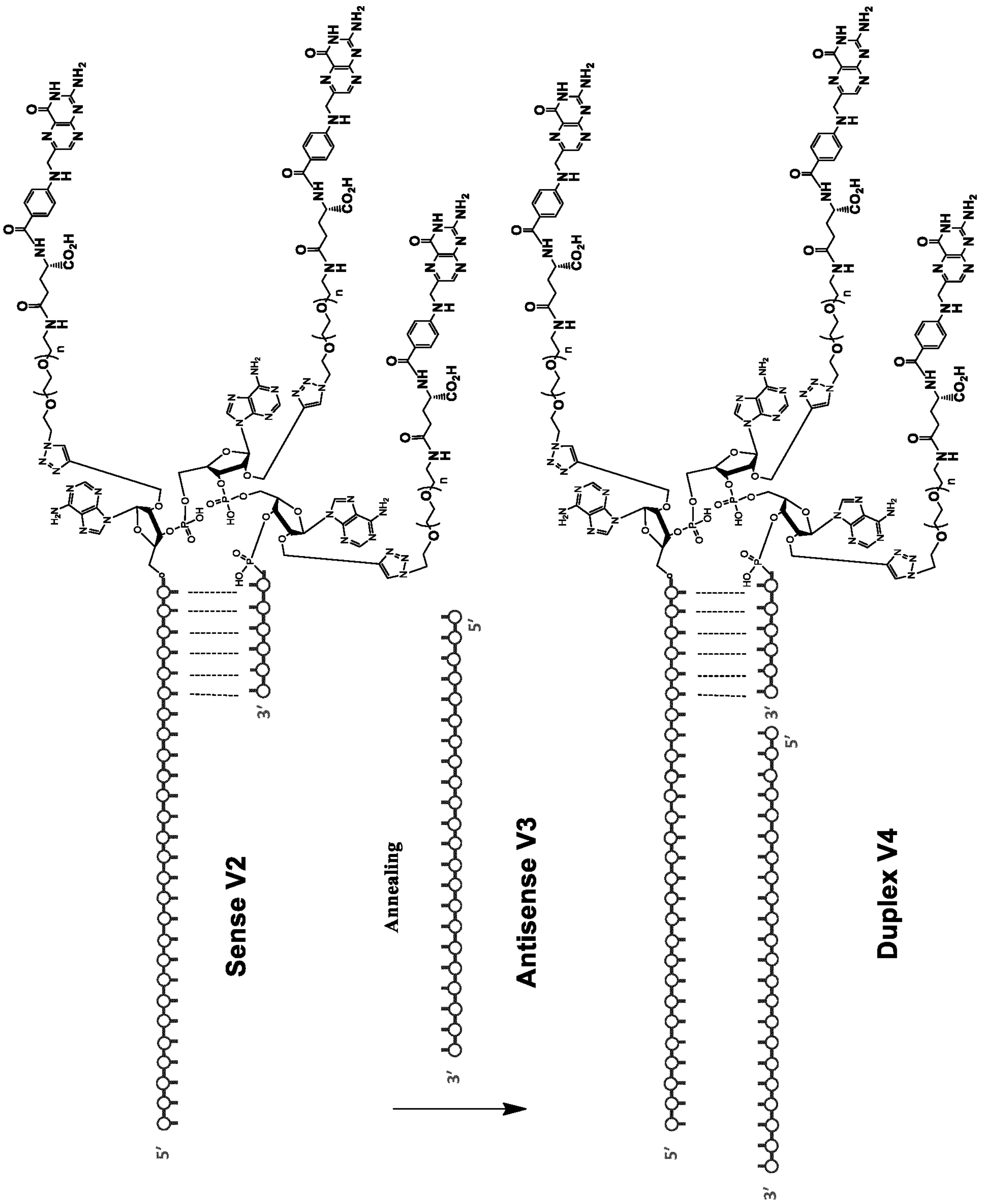


Sense V2

Antisense V3

Duplex V4

**SCHEME V1: Synthesis of Folate Nicked Tetraloop dsNA Conjugate with Multiple Folates On The Loop - Post Solid Phase Conjugation via Amide Chemsitry**

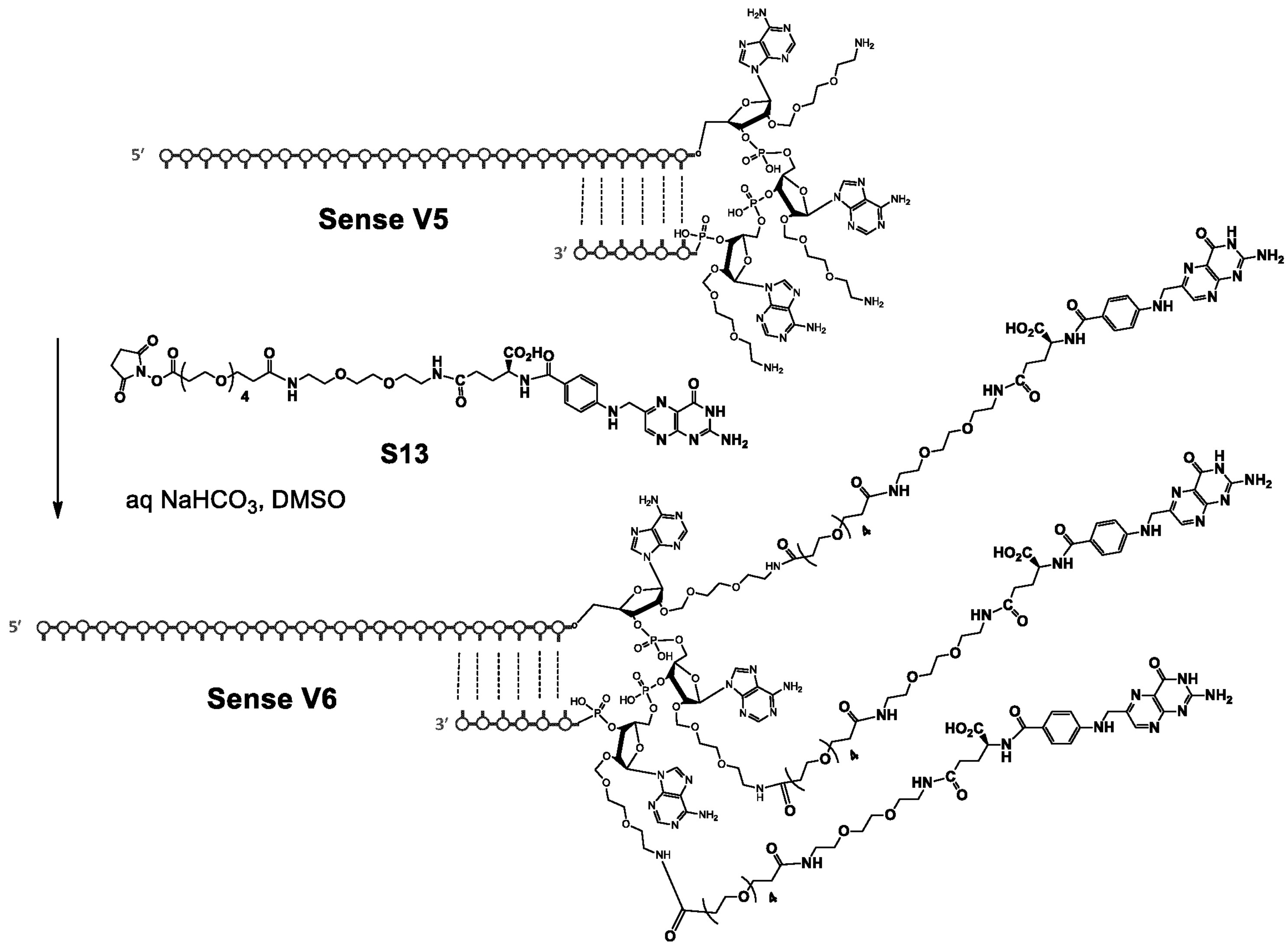


Sense V5

S13

Sense V6

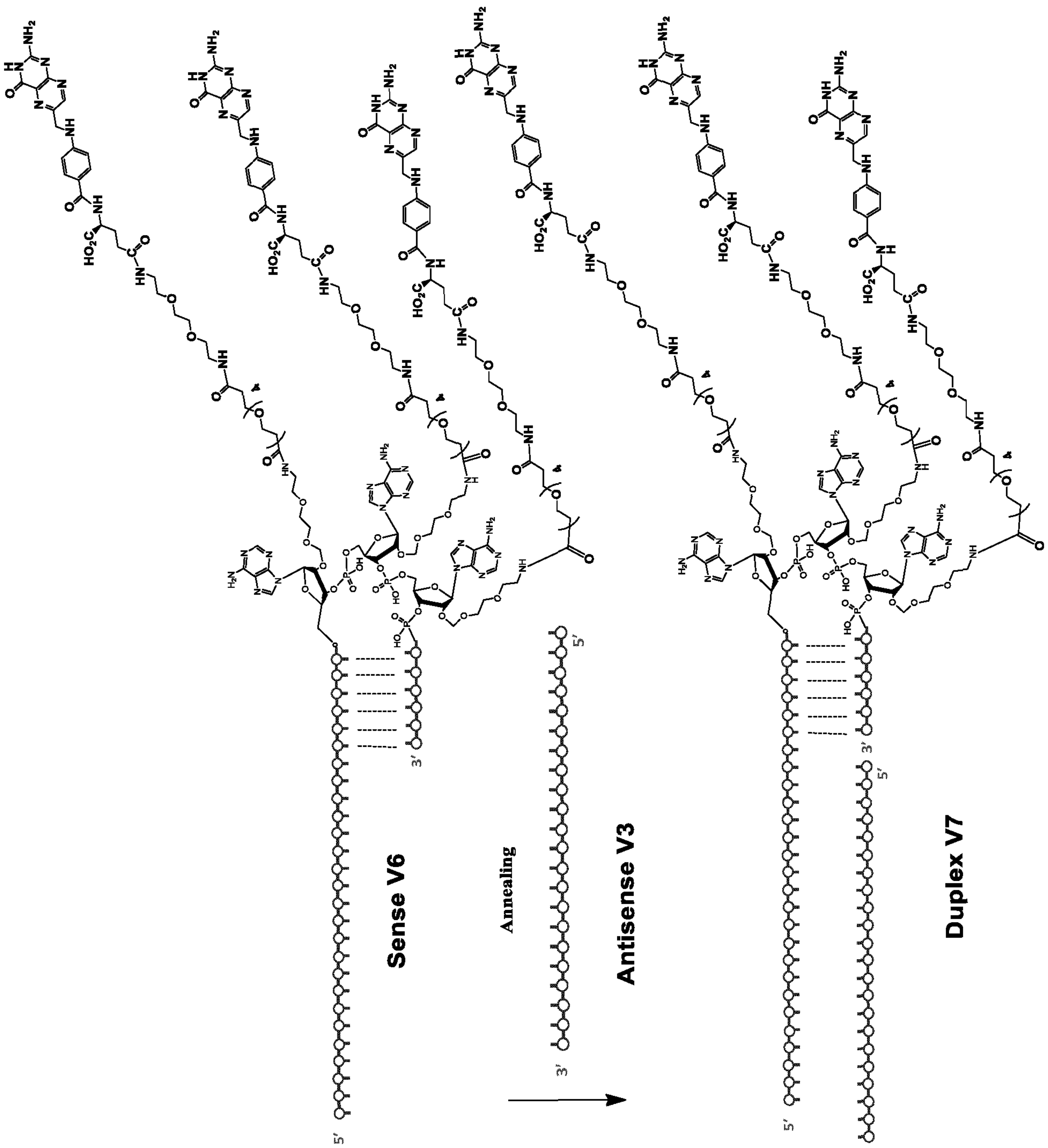
Sense V6
Annealing
Antisense V3
Duplex V7

**SCHEME W: Synthesis of Folate Nicked Tetraloop dsNA Conjugate with One Folate On The Stem - Post Solid Phase Conjugation via "Click" Chemsitry**

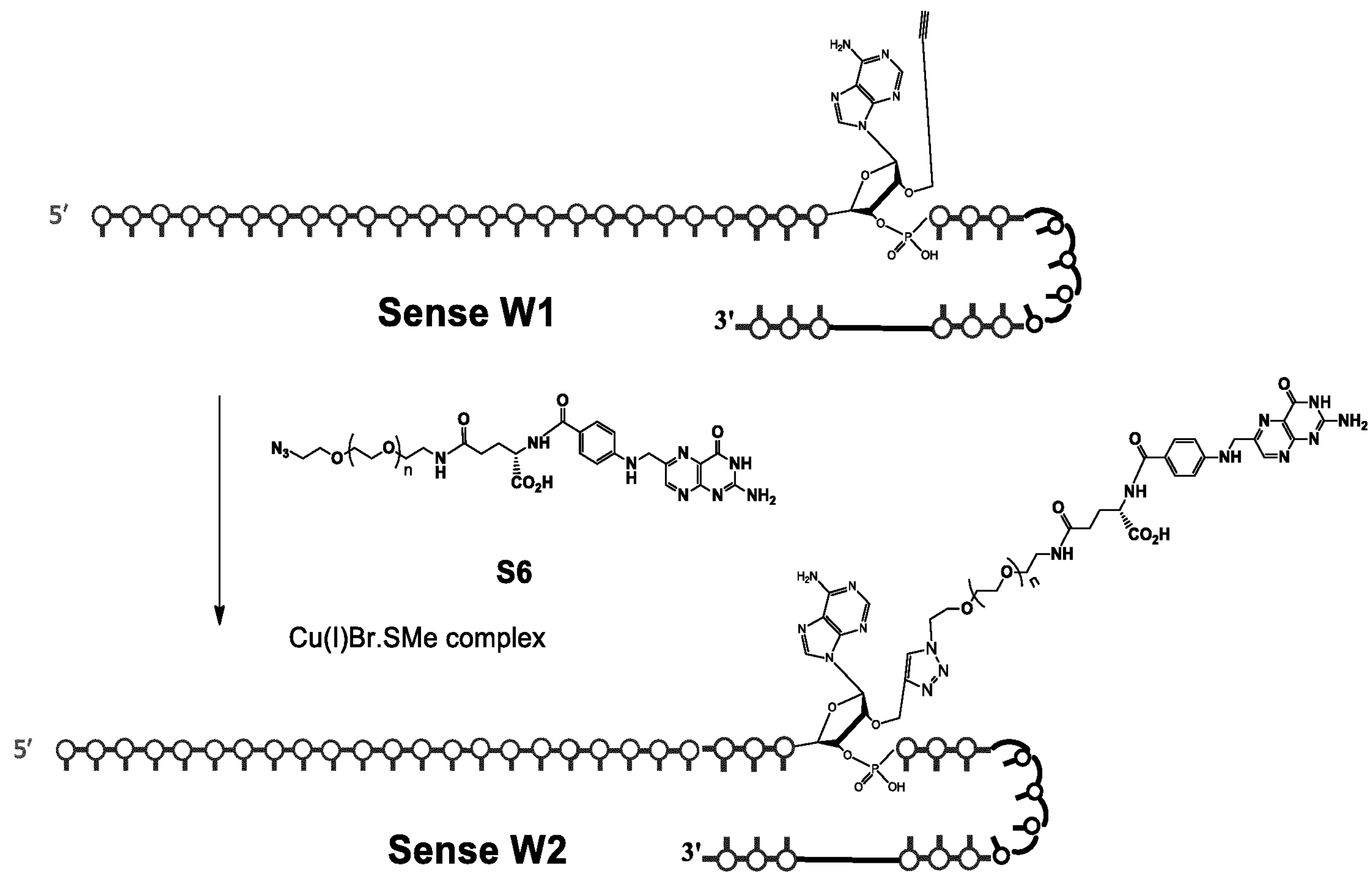


Sense W1

S6

Sense W2

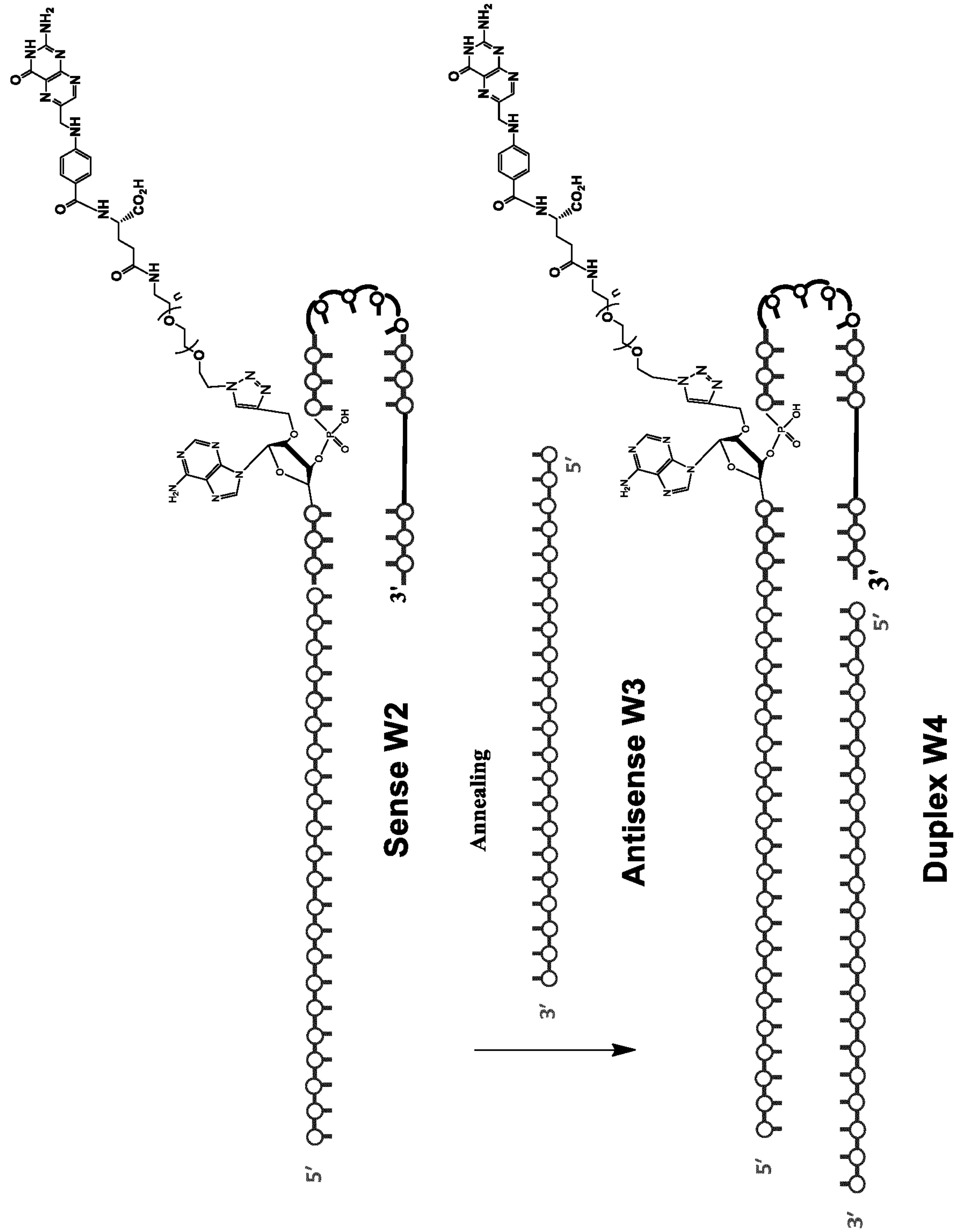
Sense W2
Annealing
Antisense W3
Duplex W4

**SCHEME W1: Synthesis of Folate Nicked Tetraloop dsNA Conjugate with One Folate On The Stem - Post Solid Phase Conjugation via Amide Chemsitry**

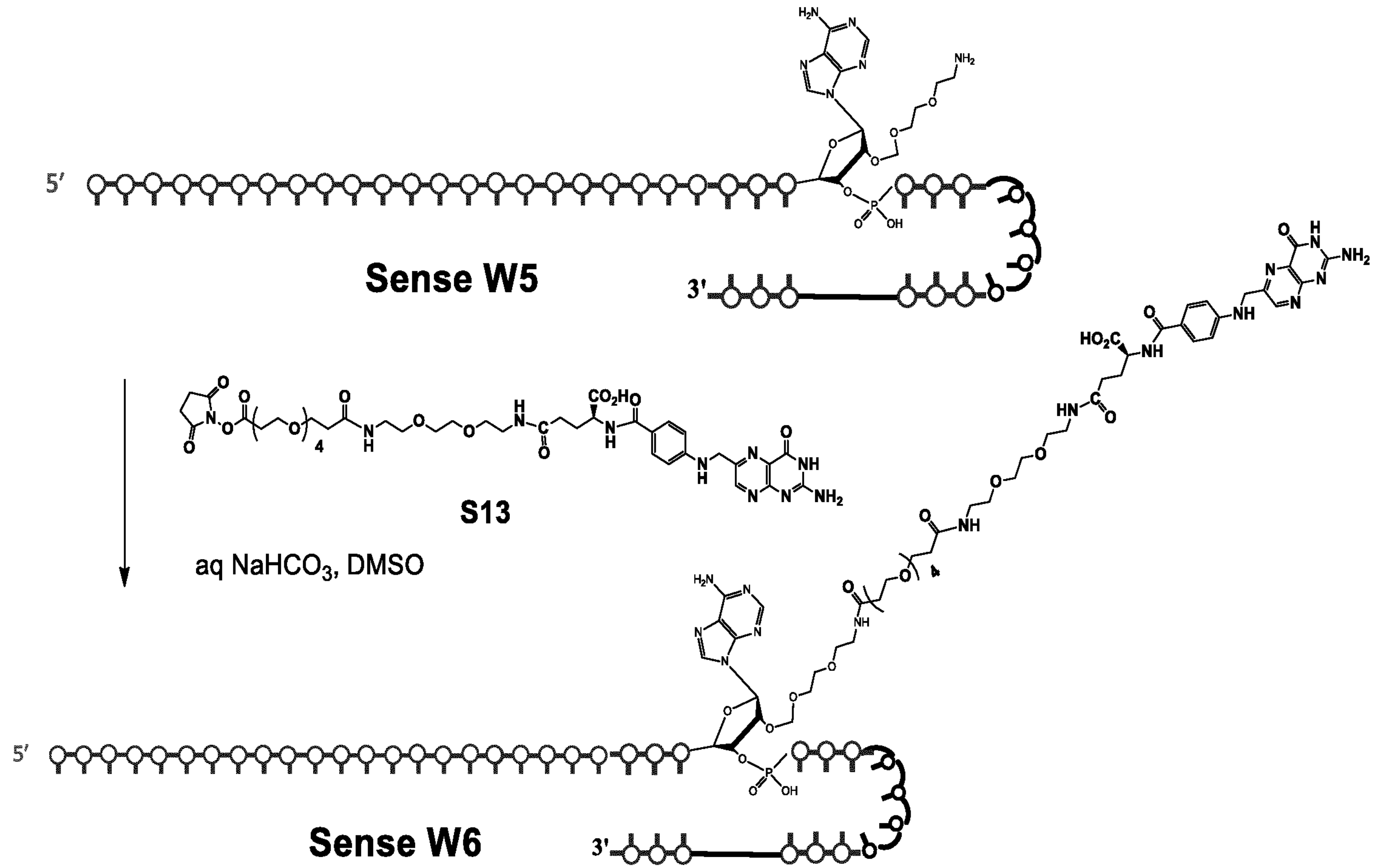


Sense W5

S13

Sense W6

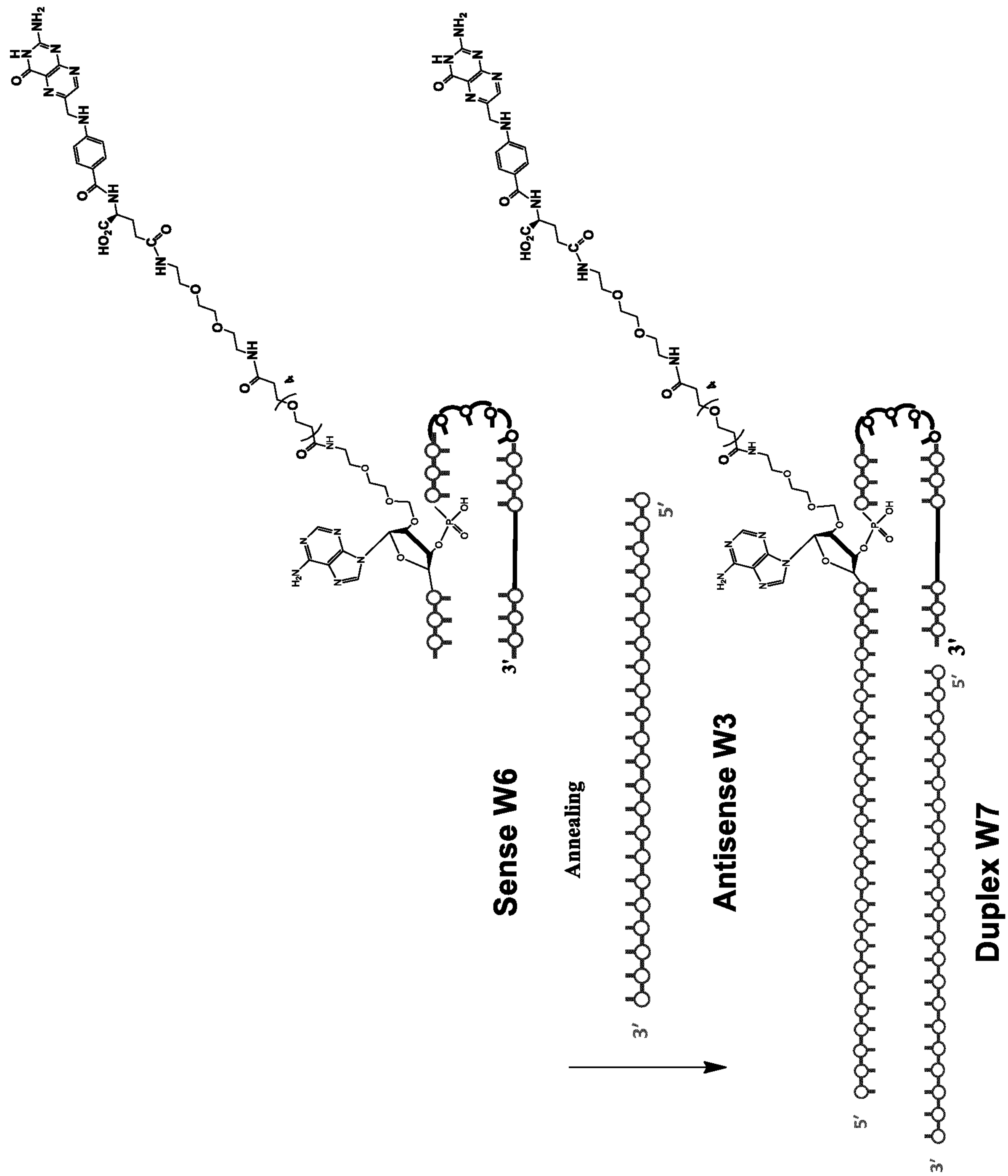
Sense W6
Annealing
Antisense W3
Duplex W7

**SCHEME X: Synthesis of Folate Nicked Tetraloop dsNA Conjugate with Multiple Folates On The Stem - Post Solid Phase Conjugation via "Click" Chemsitry**

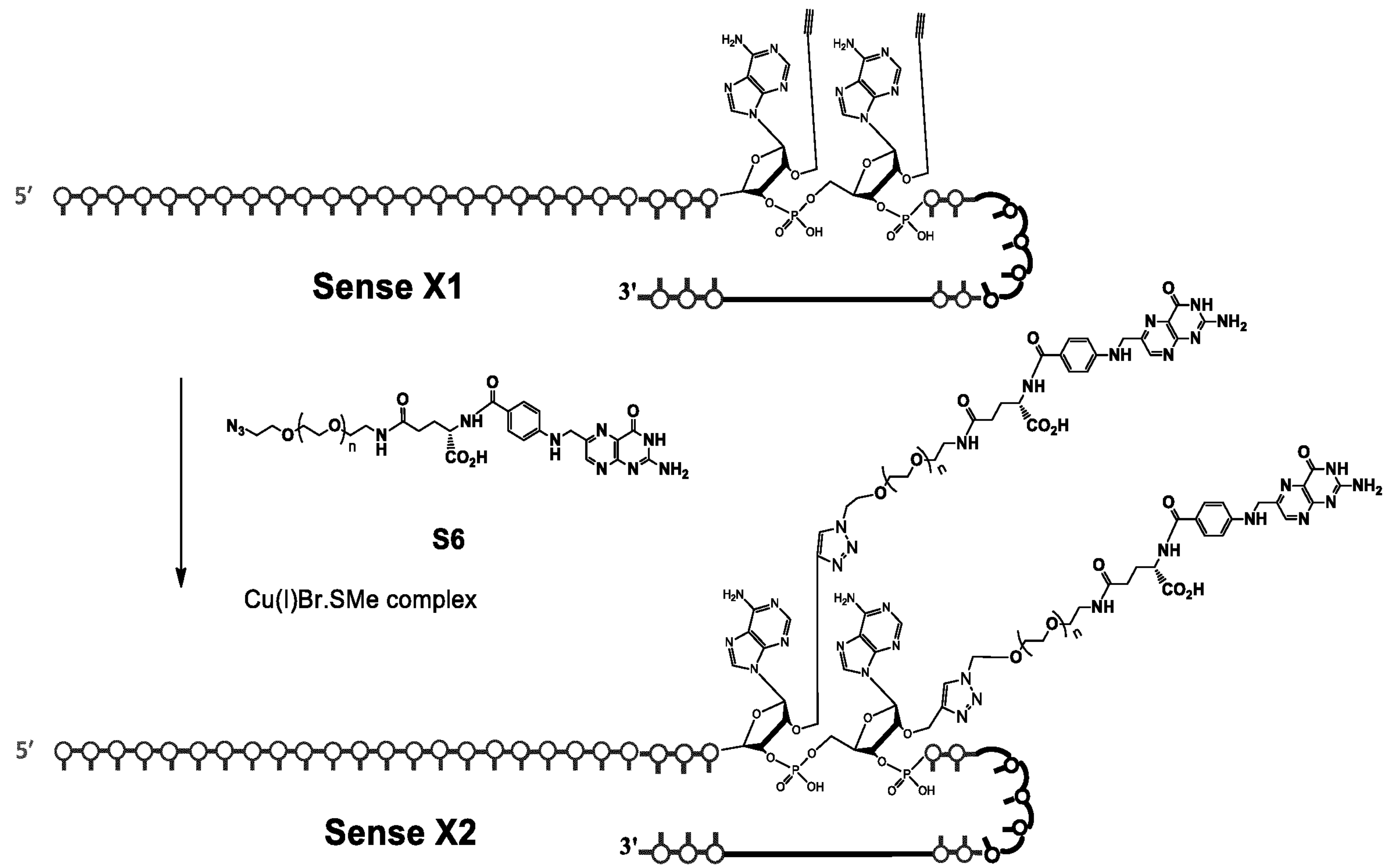


Sense X1

S6

Sense X2

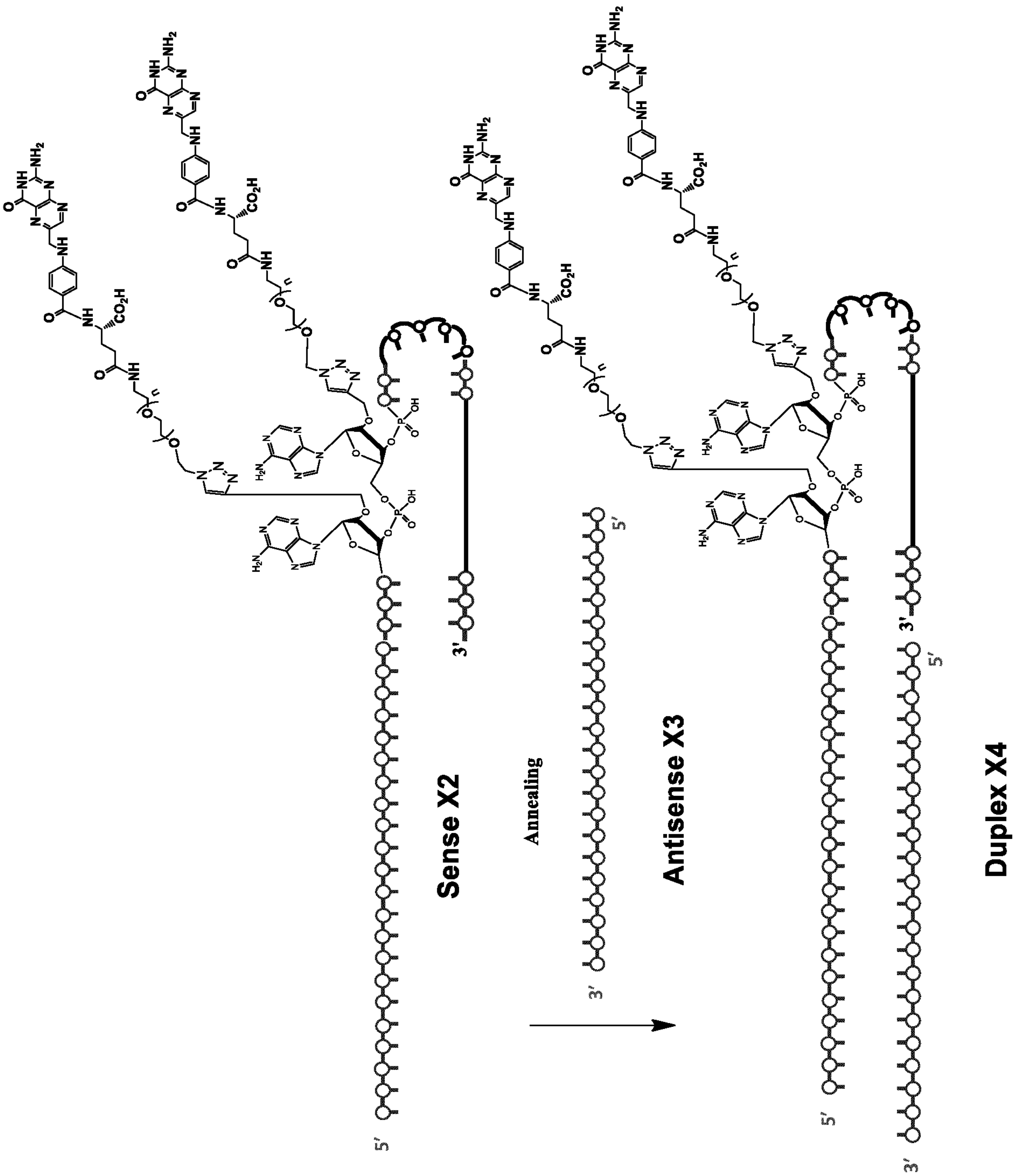
Sense X2
Annealing
Antisense X3
Duplex X4

**SCHEME X1: Synthesis of Folate Nicked Tetraloop dsNA Conjugate with Multiple Folates On The Stem - Post Solid Phase Conjugation via Amide Chemsitry**

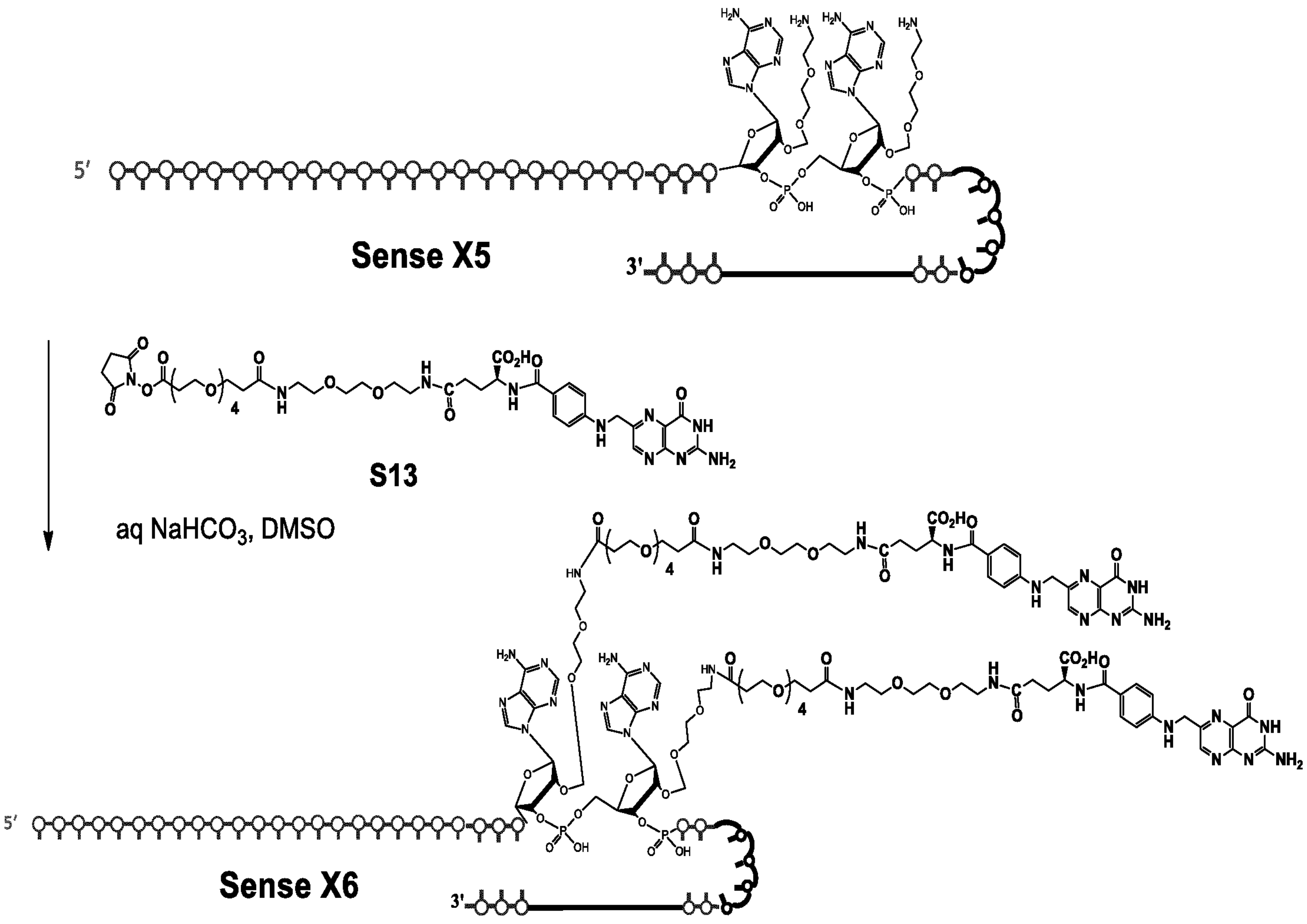


Sense X5

S13

Sense X6

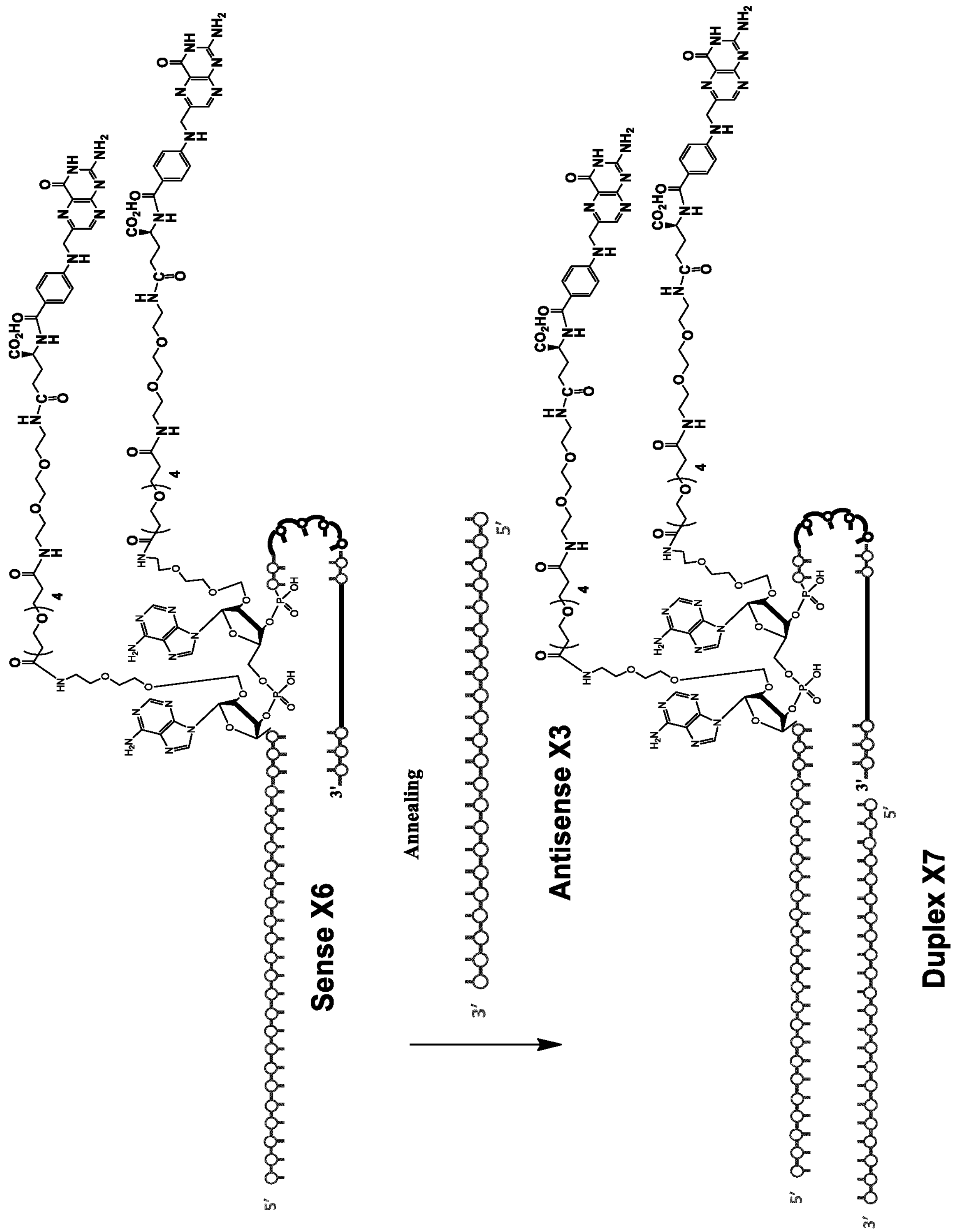


Sense X6

Antisense X3

Duplex X7

**SCHEME Y: Synthesis of Folate dsNA Conjugate with One Folate On The 5'-Extension of Antisense - Post Solid Phase Conjugation via "Click" Chemsitry**

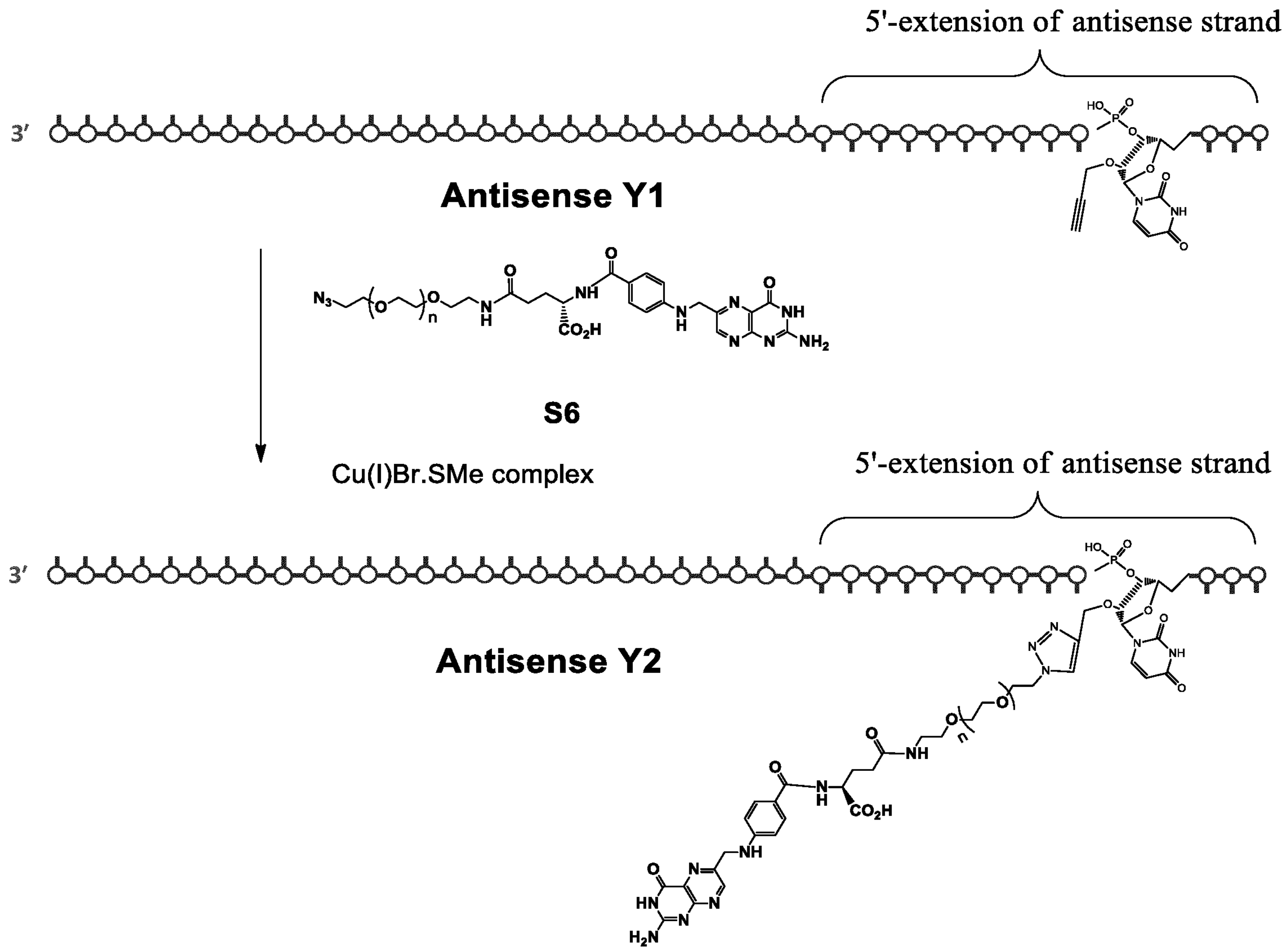

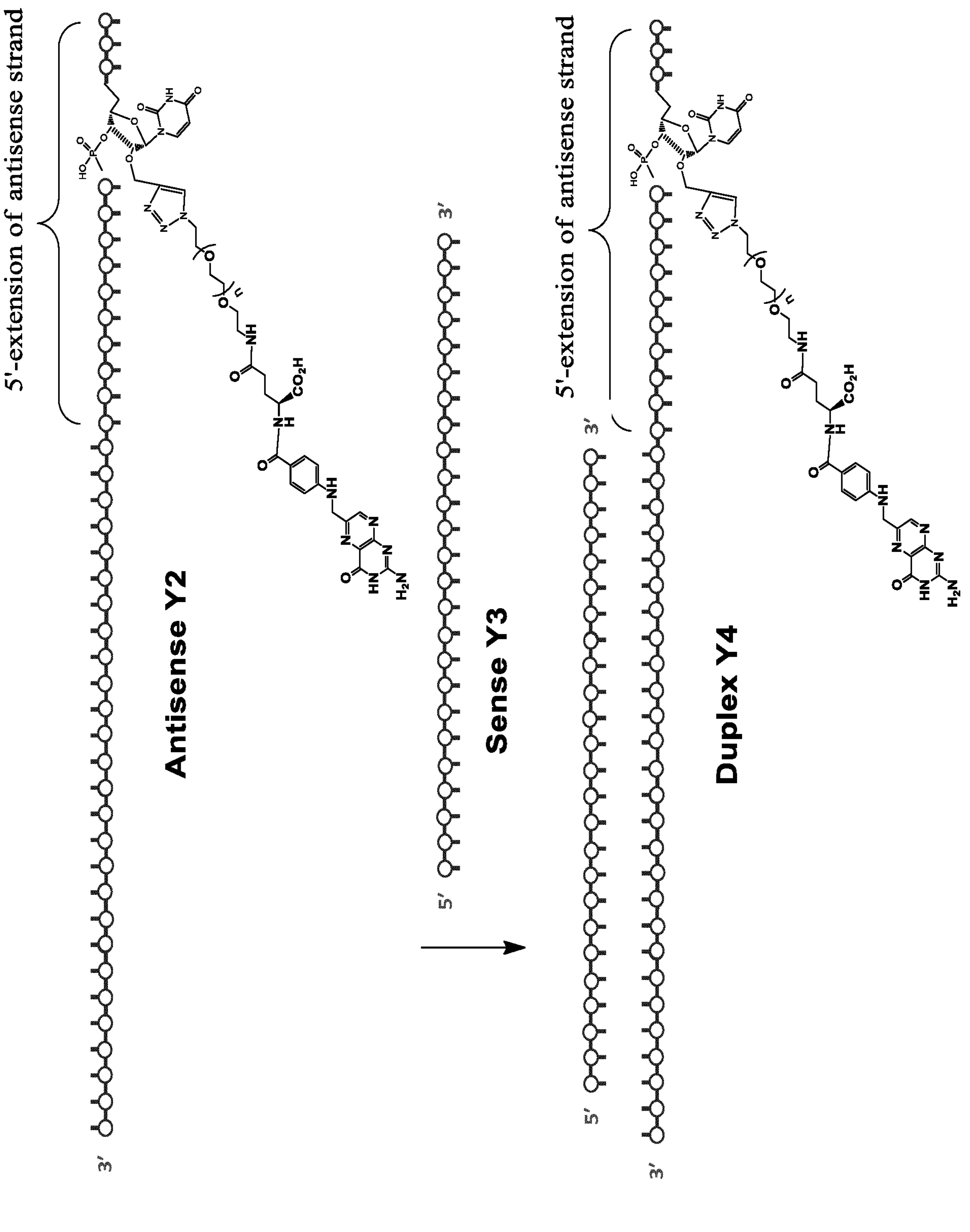
5'-extension of antisense strand
Antisense Y2
3'
Sense Y3
3'
5'
5'-extension of antisense strand
3'
5'
3'
Duplex Y4

**SCHEME Y1: Solid Phase Synthesis of Folate dsNA Conjugate with One Folate On The 5'-Extension of Antisense Using Folate Phosphoroamidite**

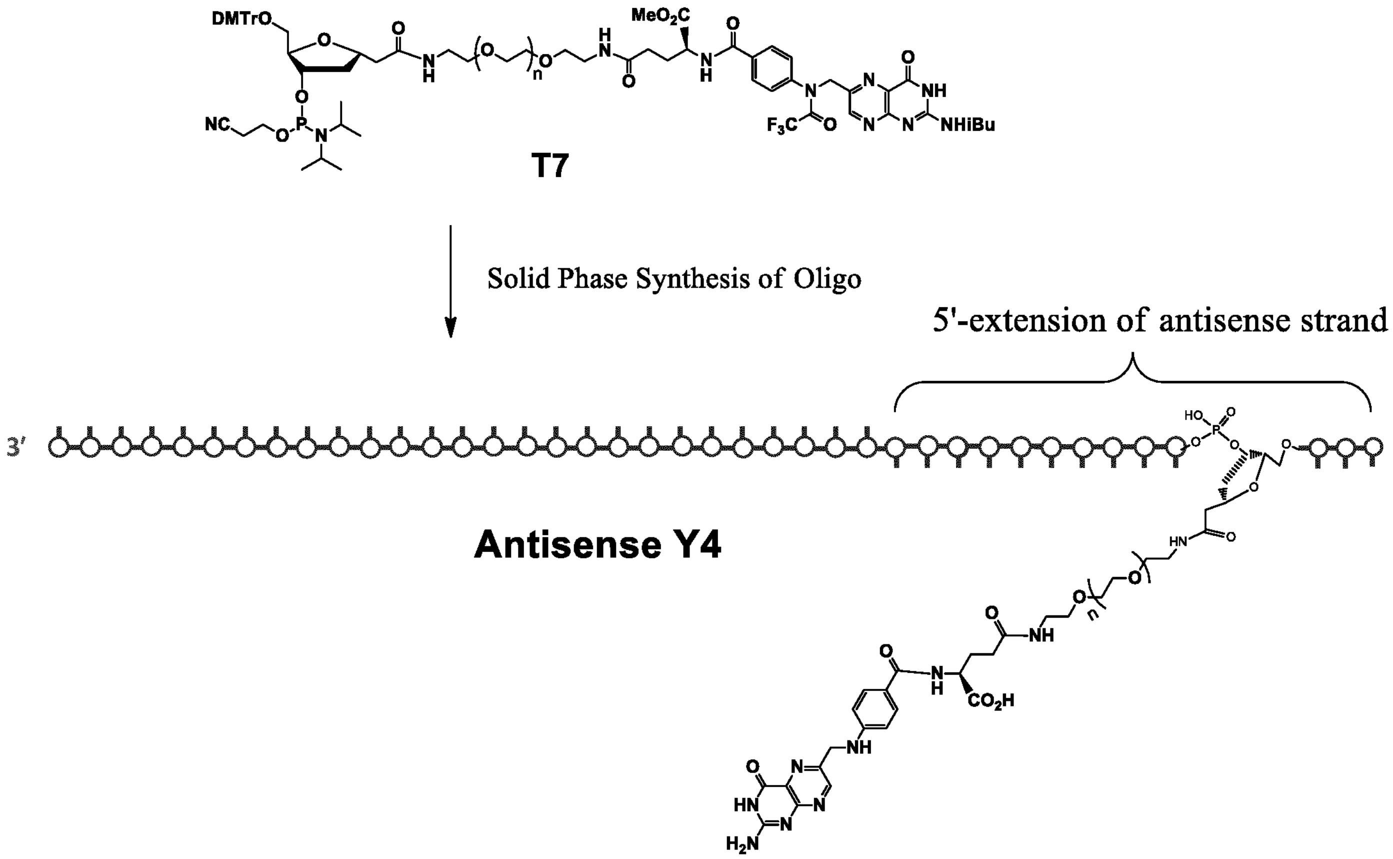


T7

Antisense Y4

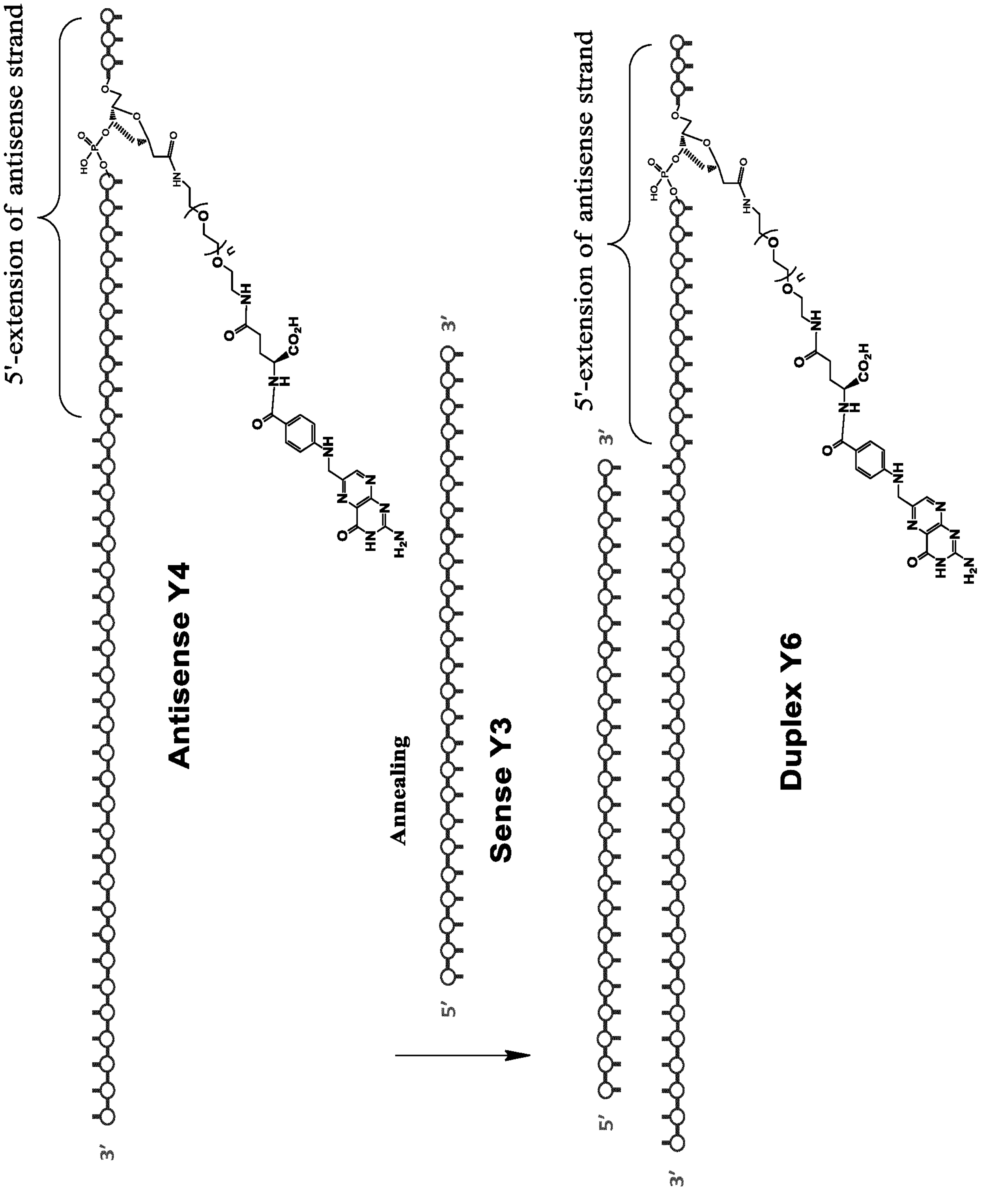
5'-extension of antisense strand
3'
Antisense Y4
Annealing
3'
5'
Sense Y3
5'-extension of antisense strand
3'
5'
3'
Duplex Y6

**SCHEME Z: Synthesis of Folate dsNA Conjugate with Multiple Folates On The 5'-Extension of Antisense - Post Solid Phase Conjugation via "Click" Chemsitry**

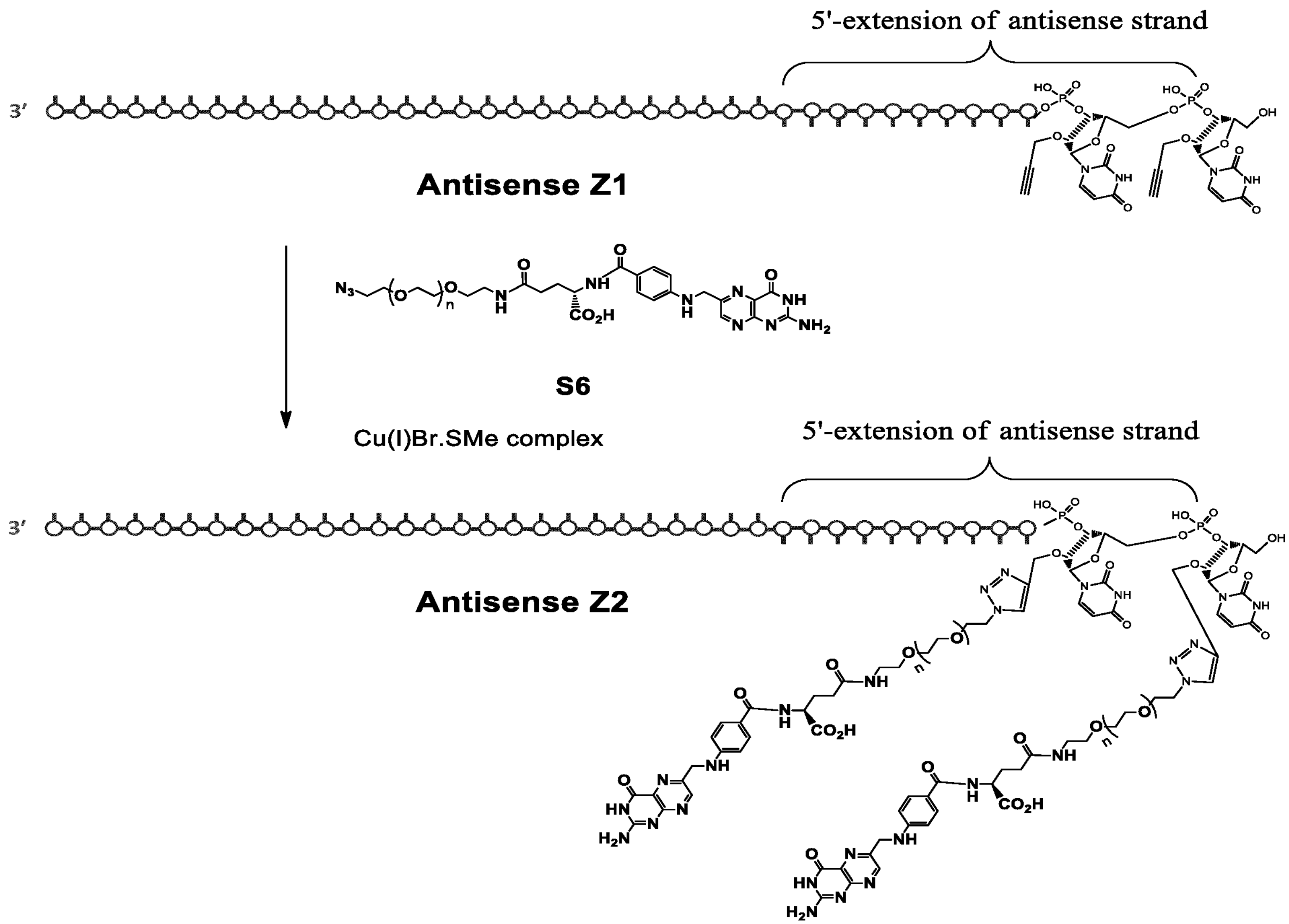


**Antisense Z1**

**S6**

**Antisense Z2**

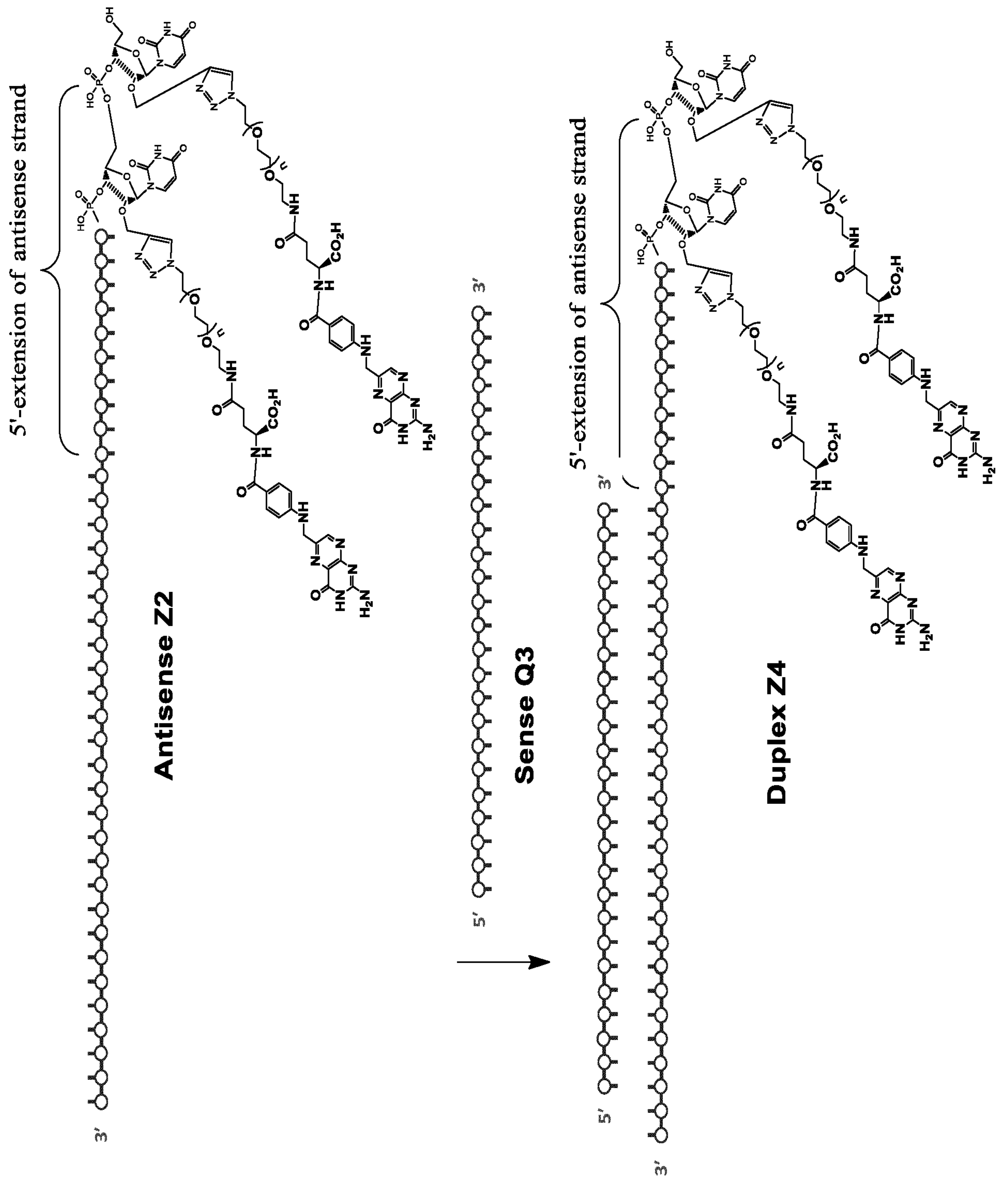
5'-extension of antisense strand
3'
Antisense Z2
3'
5'
Sense Q3
5'-extension of antisense strand
3'
5'
3'
Duplex Z4

**SCHEME Z1: Solid Phase Synthesis of Folate dsNA Conjugate with Multiple Folates On The 5'-Extension of Antisense Using Folate Phosphoroamidite**

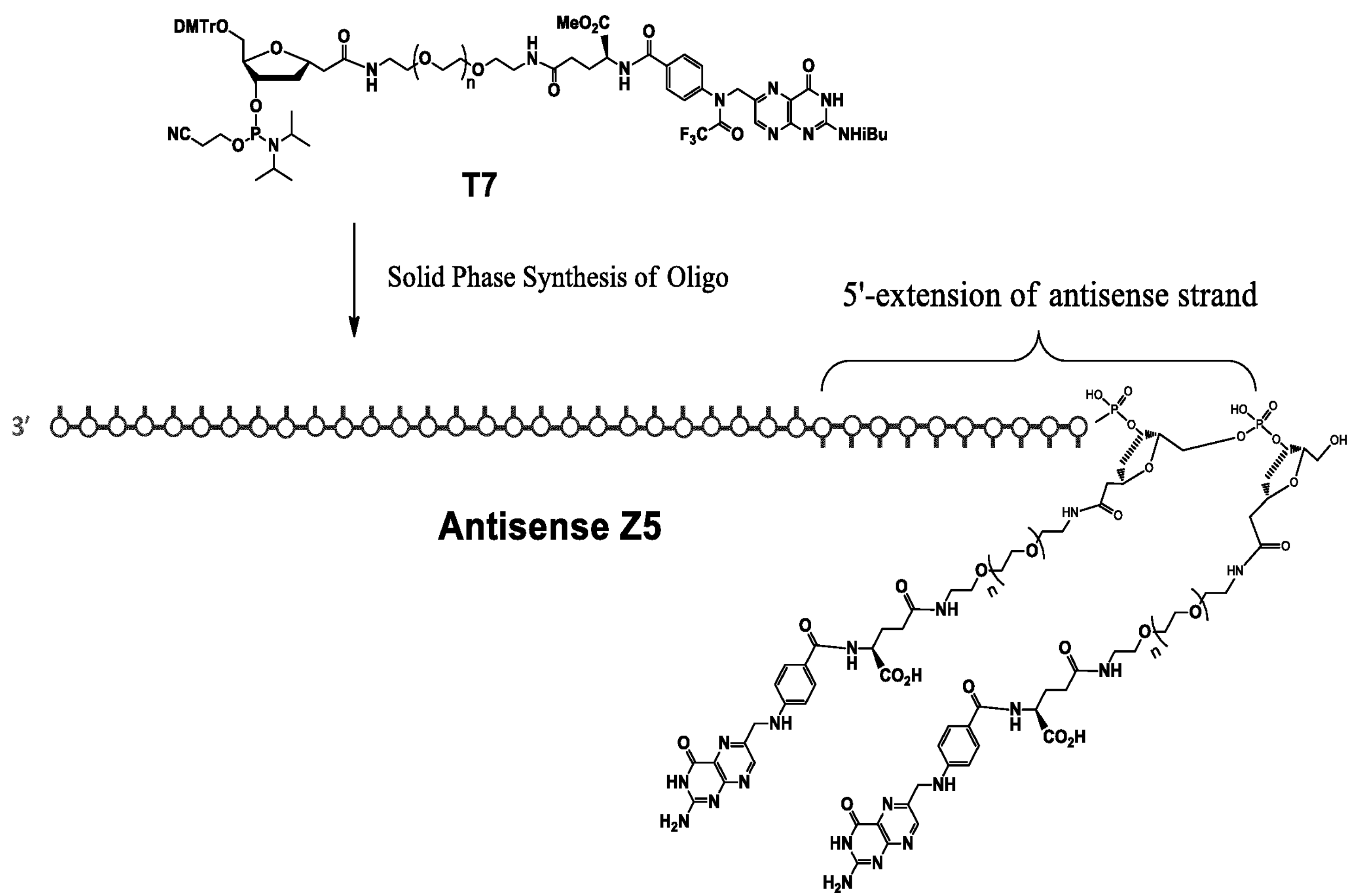


T7

Antisense Z5

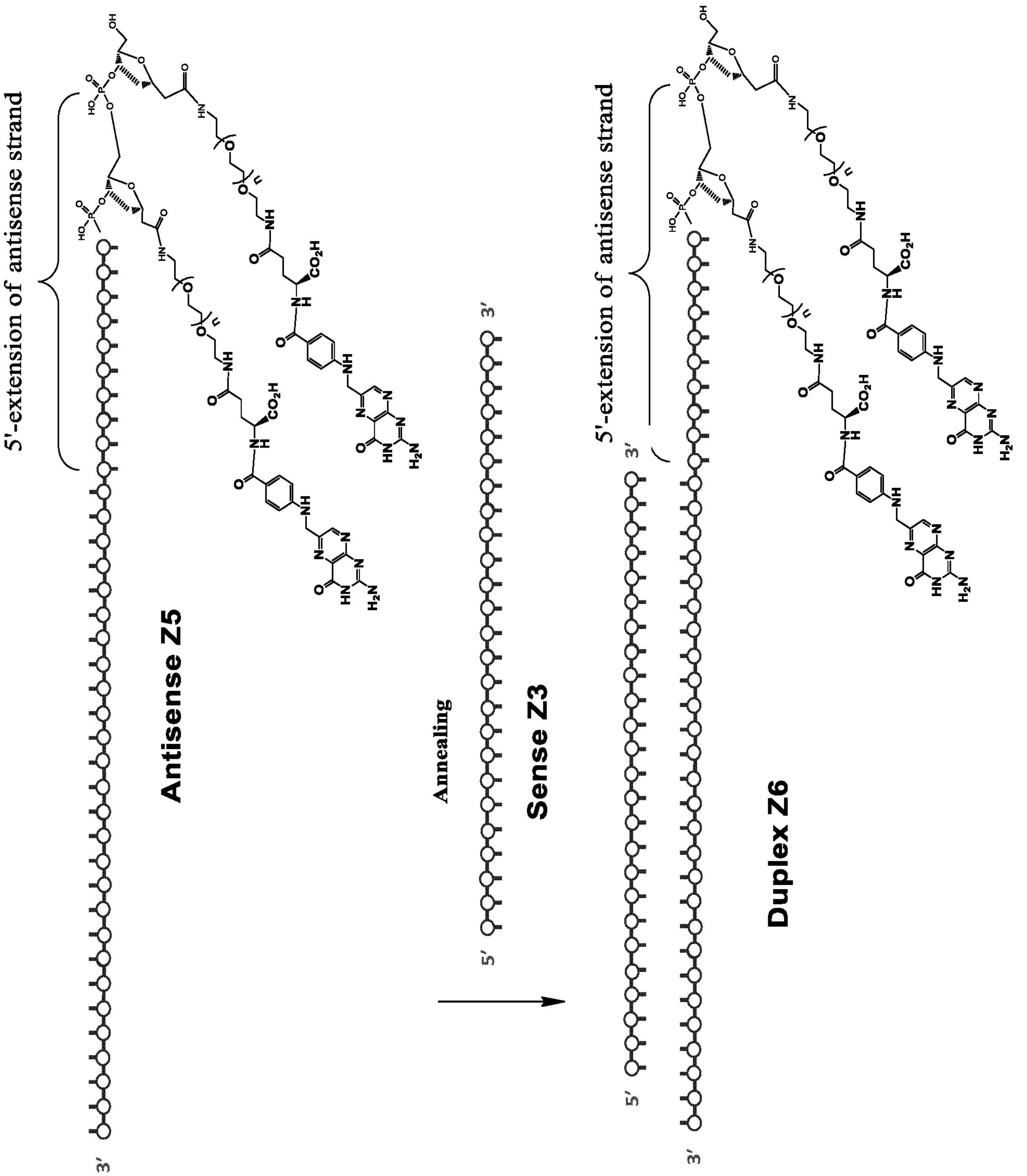
5'-extension of antisense strand
3'
Antisense Z5
Annealing
5'
3'
Sense Z3
5'-extension of antisense strand
3'
5'
3'
Duplex Z6

**SCHEME AA: Synthesis of Folate dsNA Conjugate with One Folates On The 5'-Extension of Sense - Post Solid Phase Conjugation via "Click" Chemsitry**

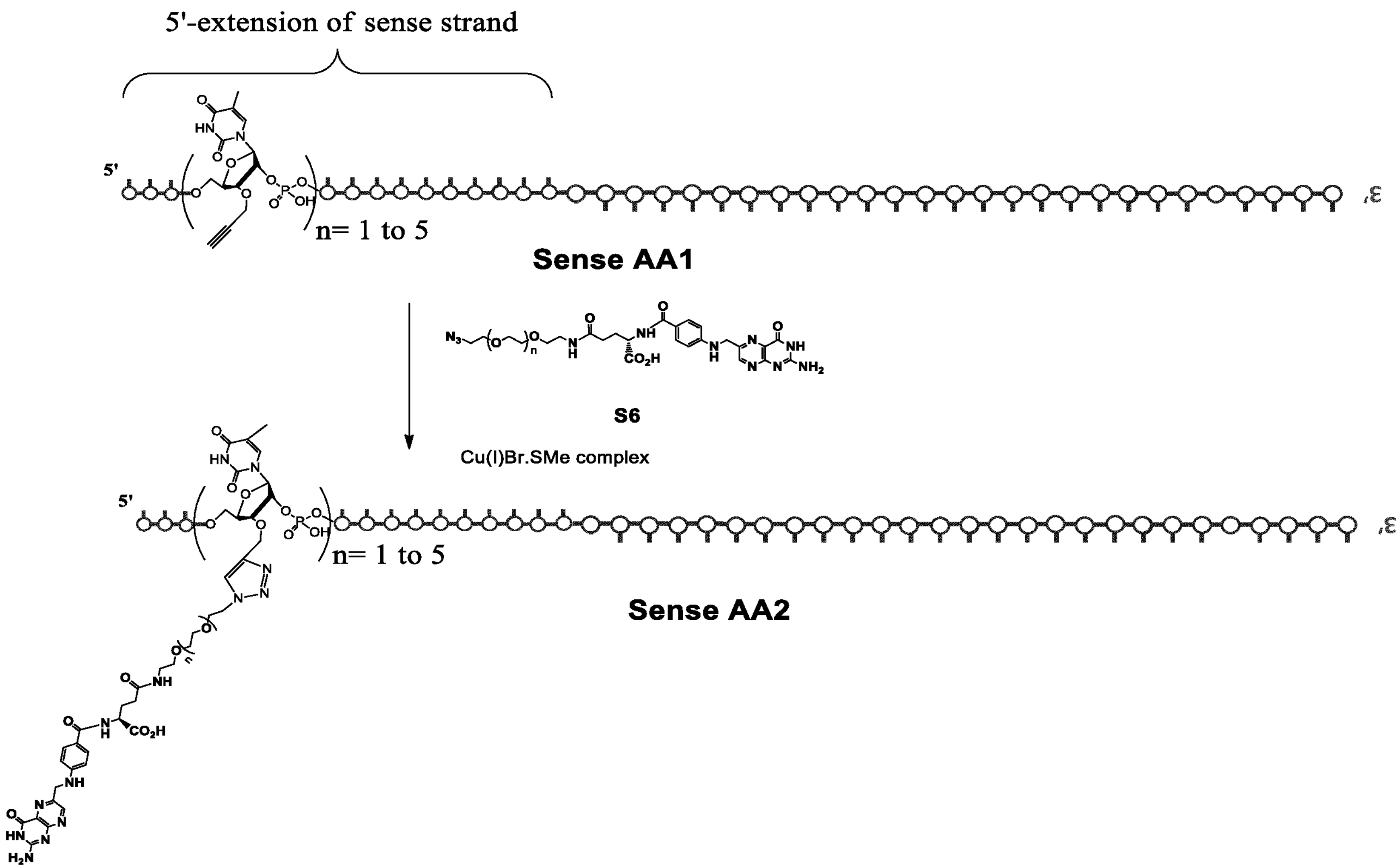


Sense AA1

S6

Sense AA2

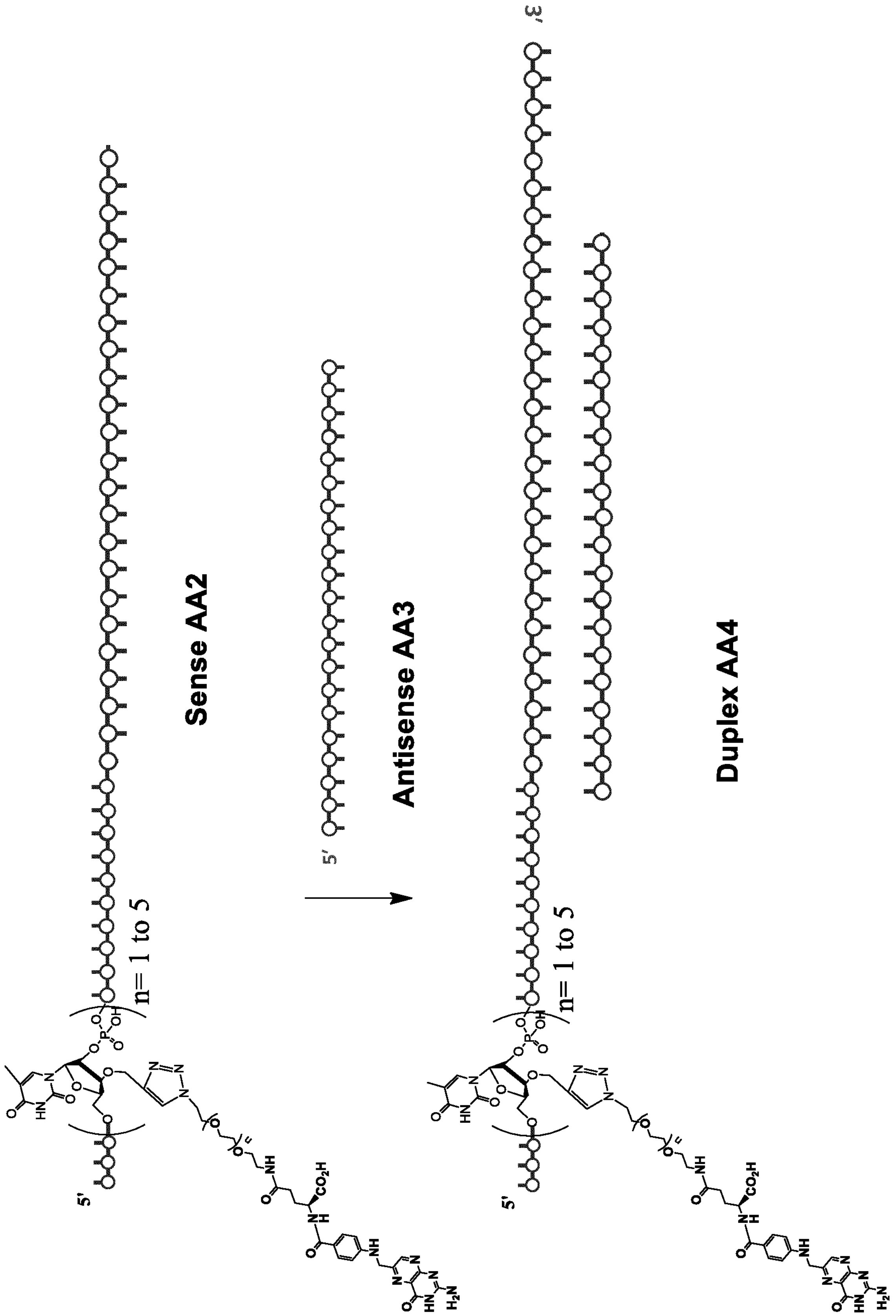
3'
5'
Sense AA2
n= 1 to 5
5'
Antisense AA3
Duplex AA4
n= 1 to 5
5'

**SCHEME AA1: Solid Phase Synthesis of Folate dsNA Conjugate with One Folate On The 5'-Extension of Sense Using Folate Phosphoroamidite**

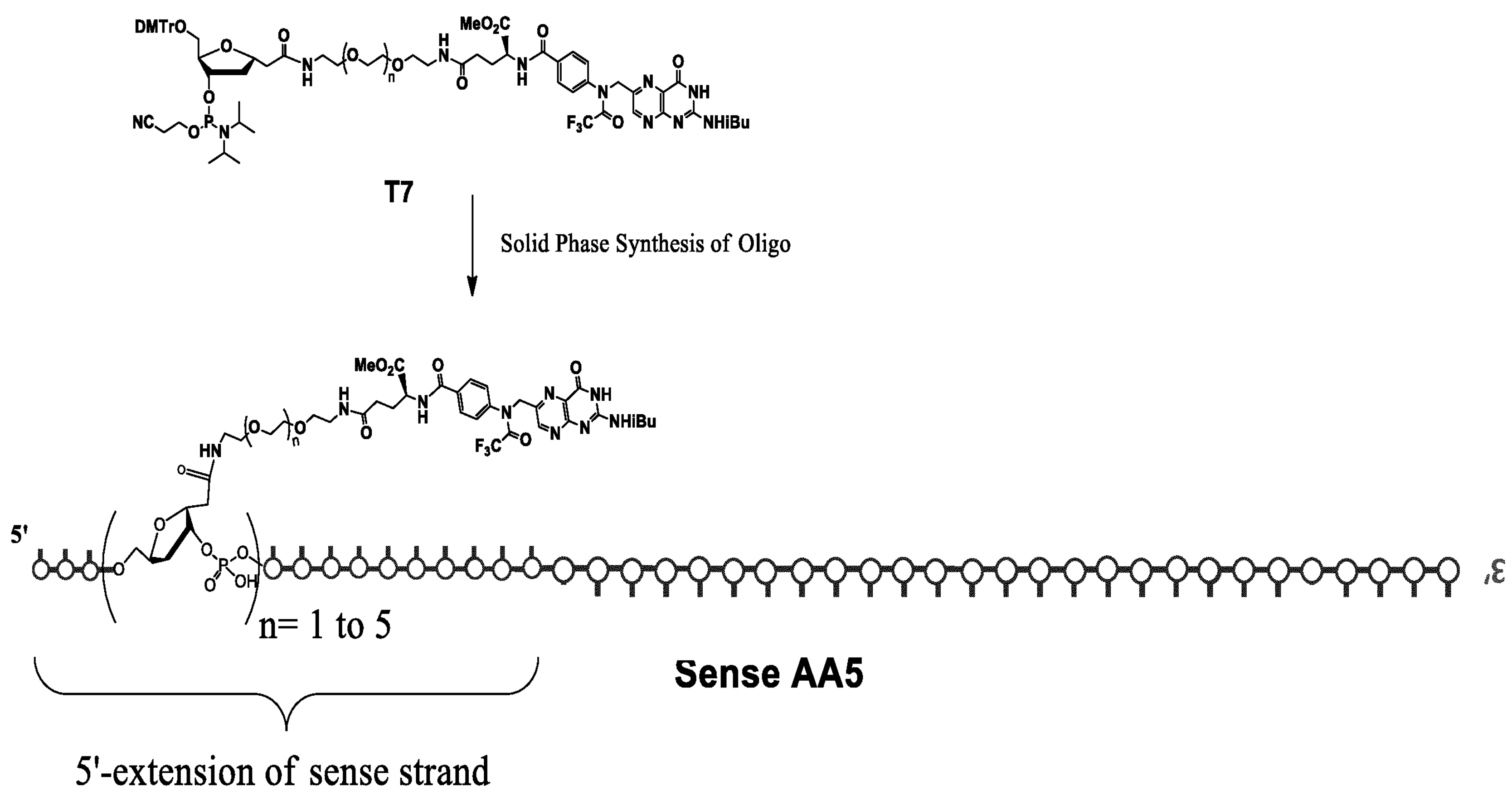


T7

Sense AA5

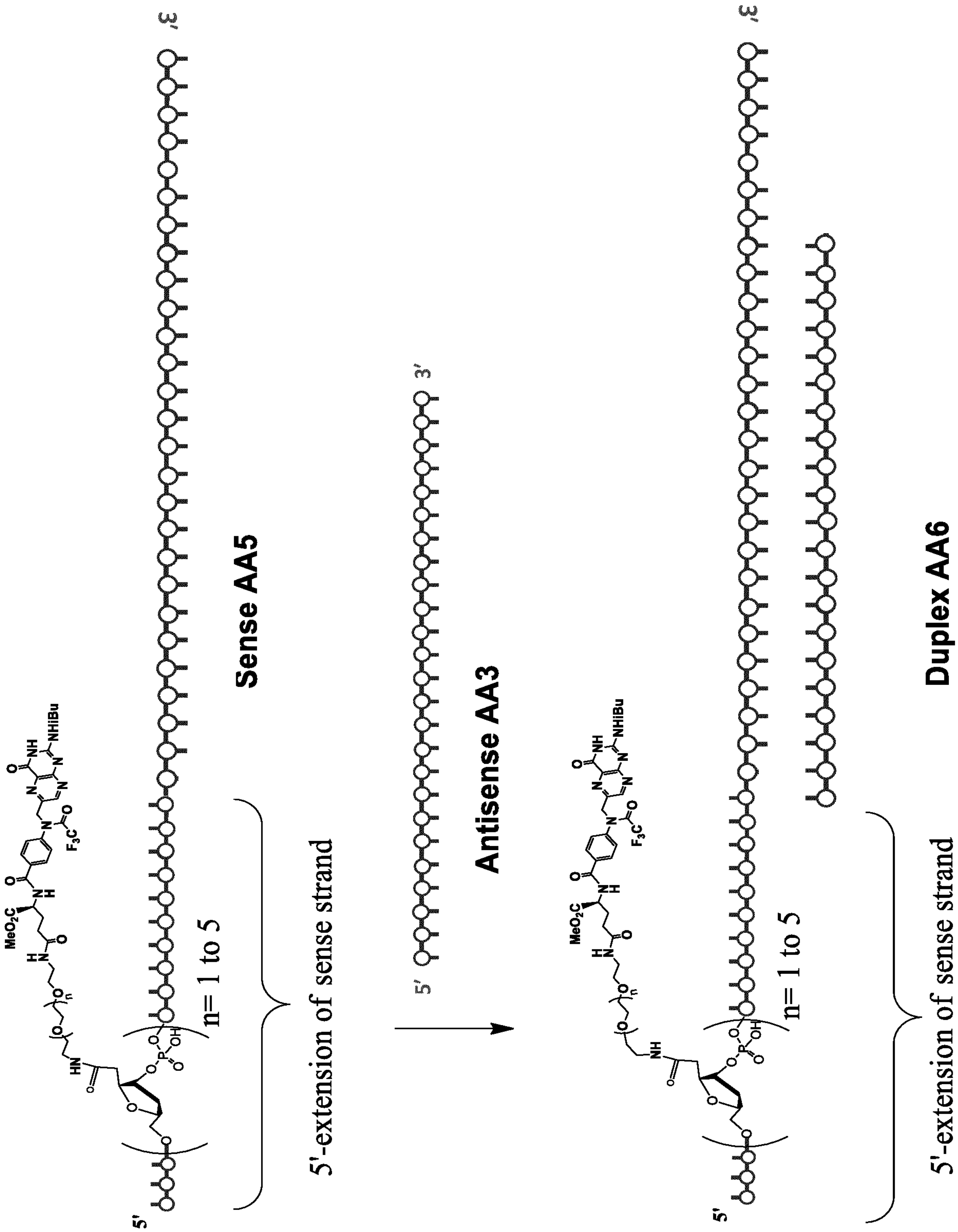


Sense AA5

Antisense AA3

Duplex AA6

**SCHEME AB: Synthesis of Nicked Tetraloop dsNA Conjugate with Multiple Mannose-6-Phosphate on the loop Post Synthetic Conjugation Using "Click" Chemsitry**

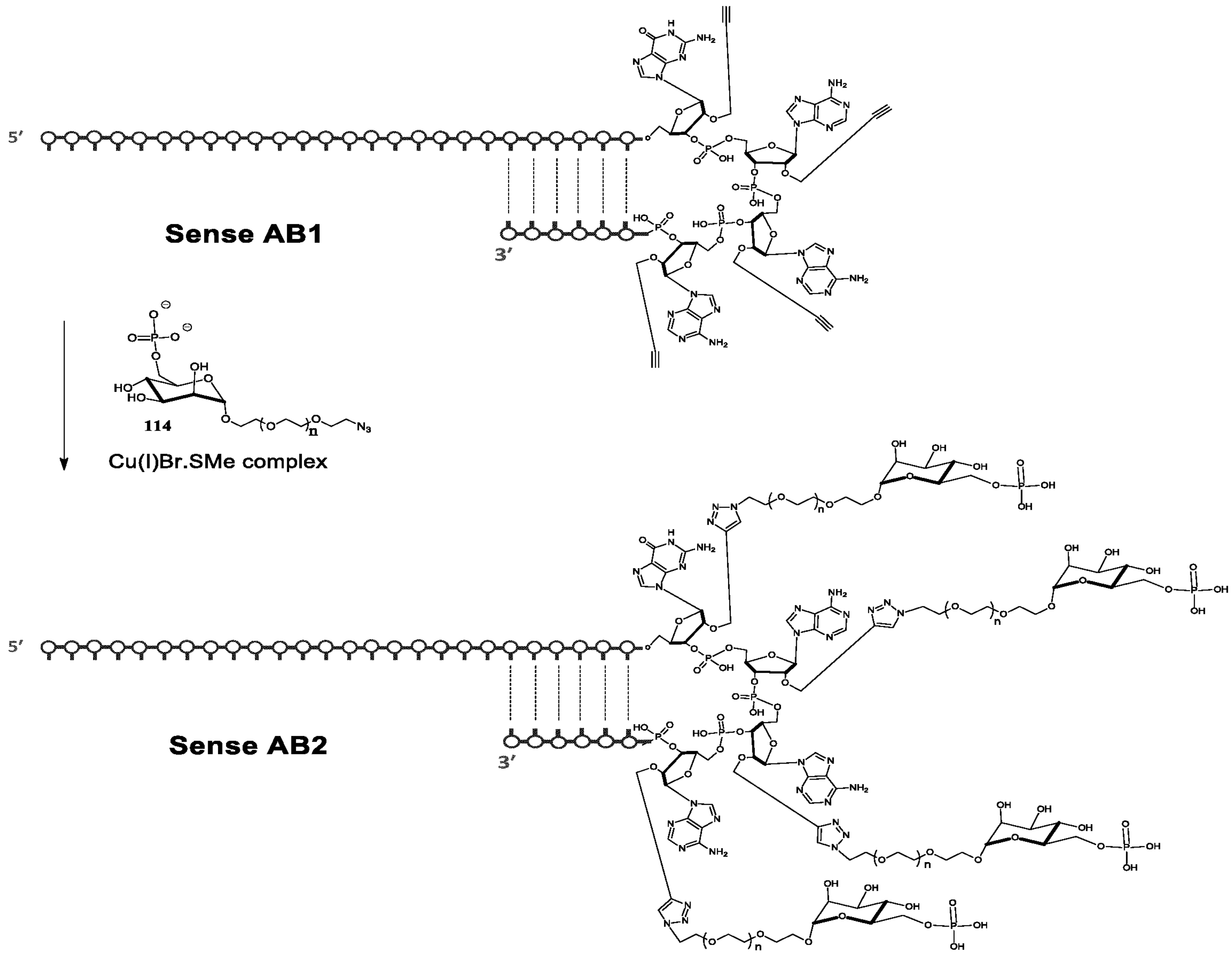


Sense AB1

114

Sense AB2

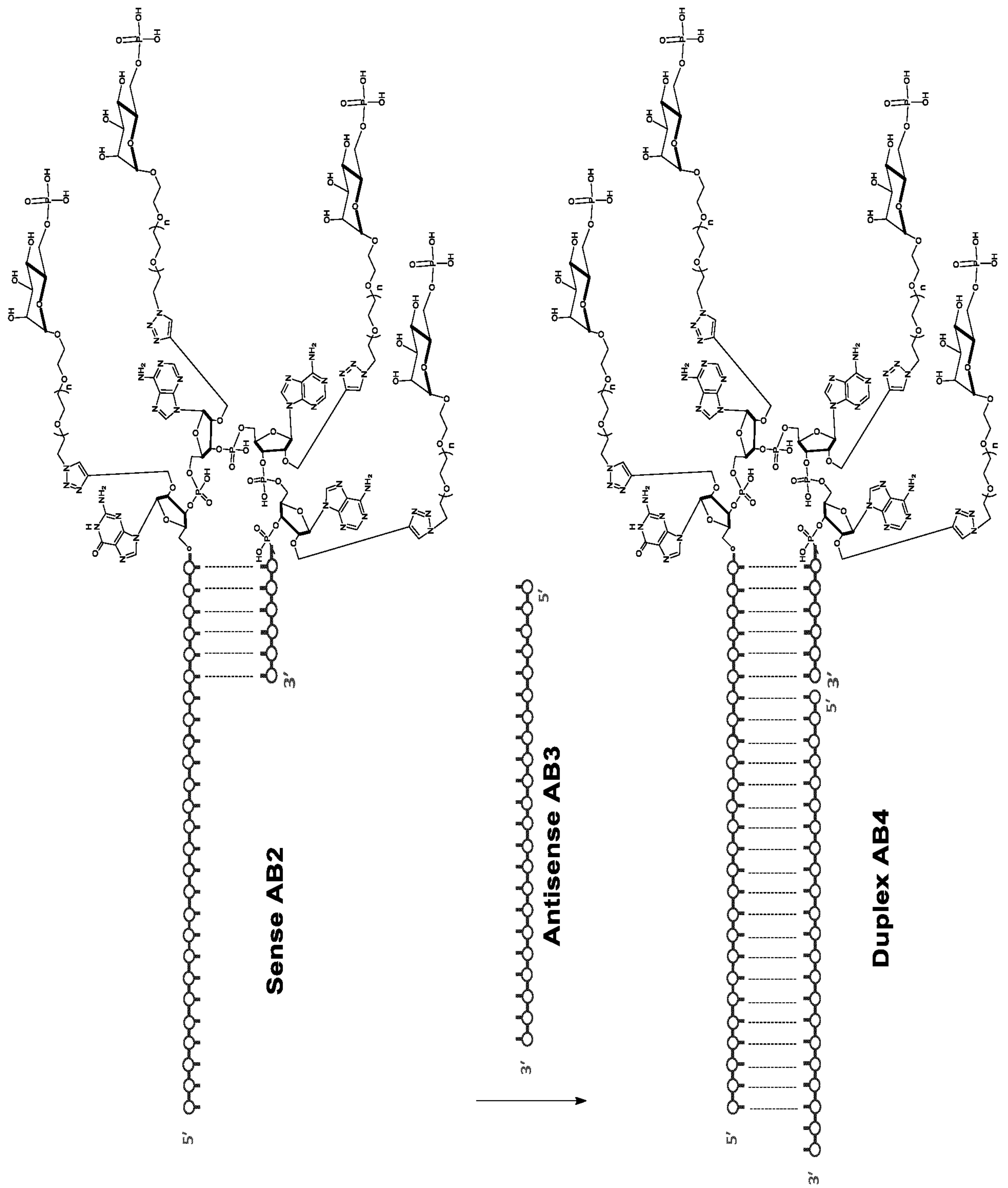

Sense AB2

Antisense AB3

Duplex AB4

**SCHEME AC: Synthesis of Nicked Tetraloop dsNA Conjugate with Multiple Mannose-6-Phosphate on the loop Post Synthetic Conjugation Using Amide Formation**

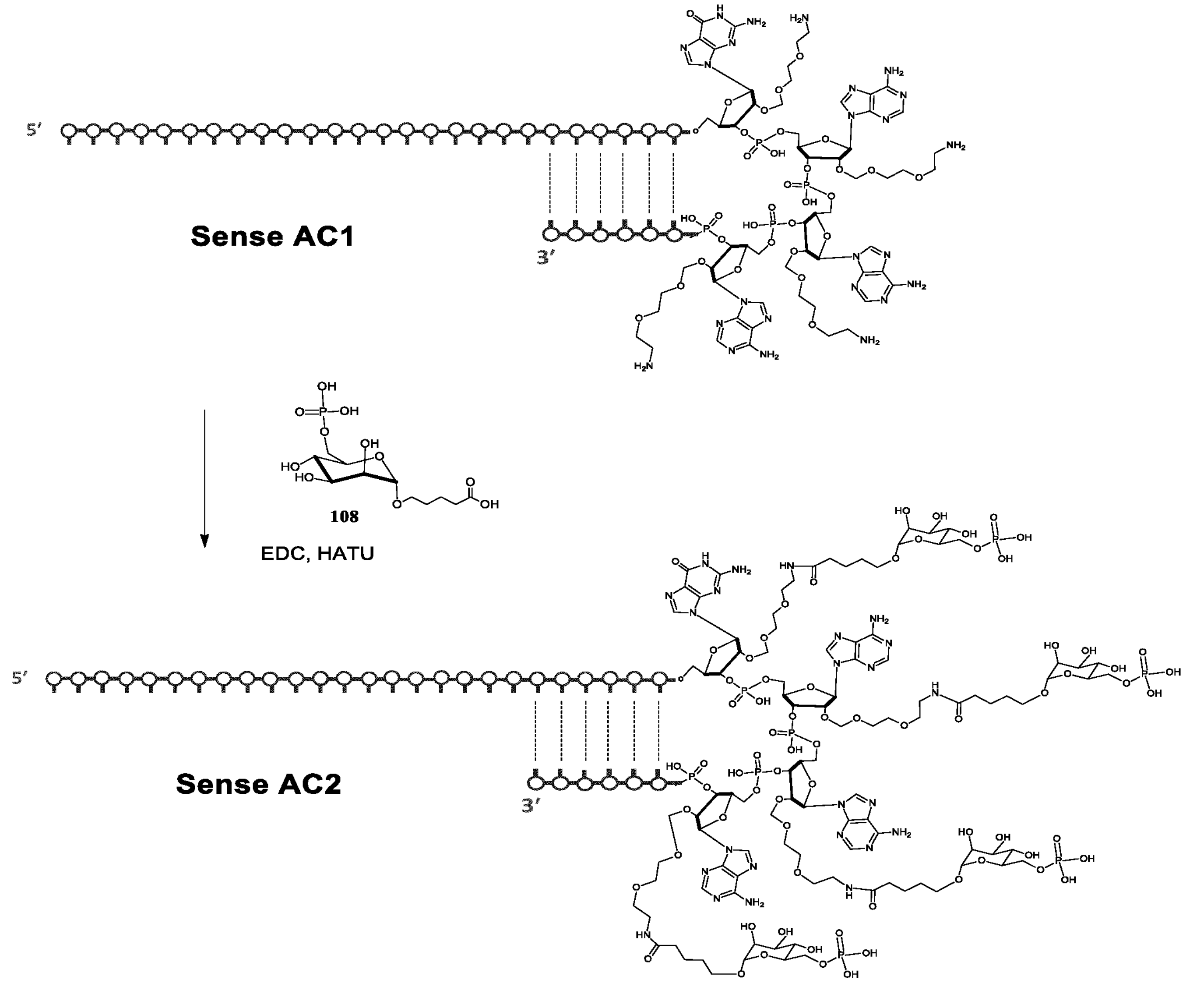

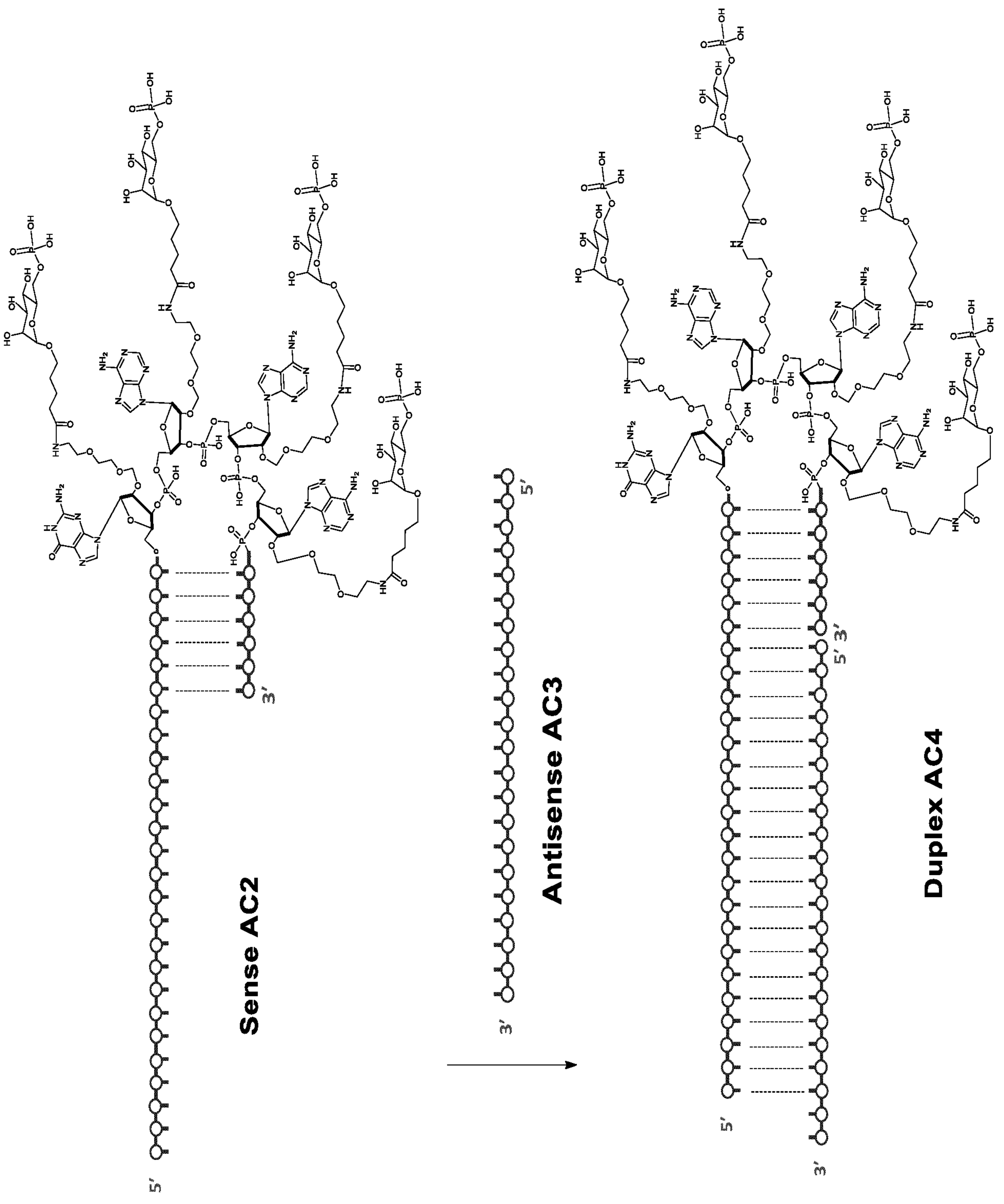


Sense AC2

Antisense AC3

Duplex AC4

**SCHEME AD: Solid Phase Synthesis of Mannose-6-Phosphate Nicked Tetraloop dsNA Conjugate Using 2'- Triazole Linked Mannose-6-Phosphate Nucleoside Phosphoroamidites**

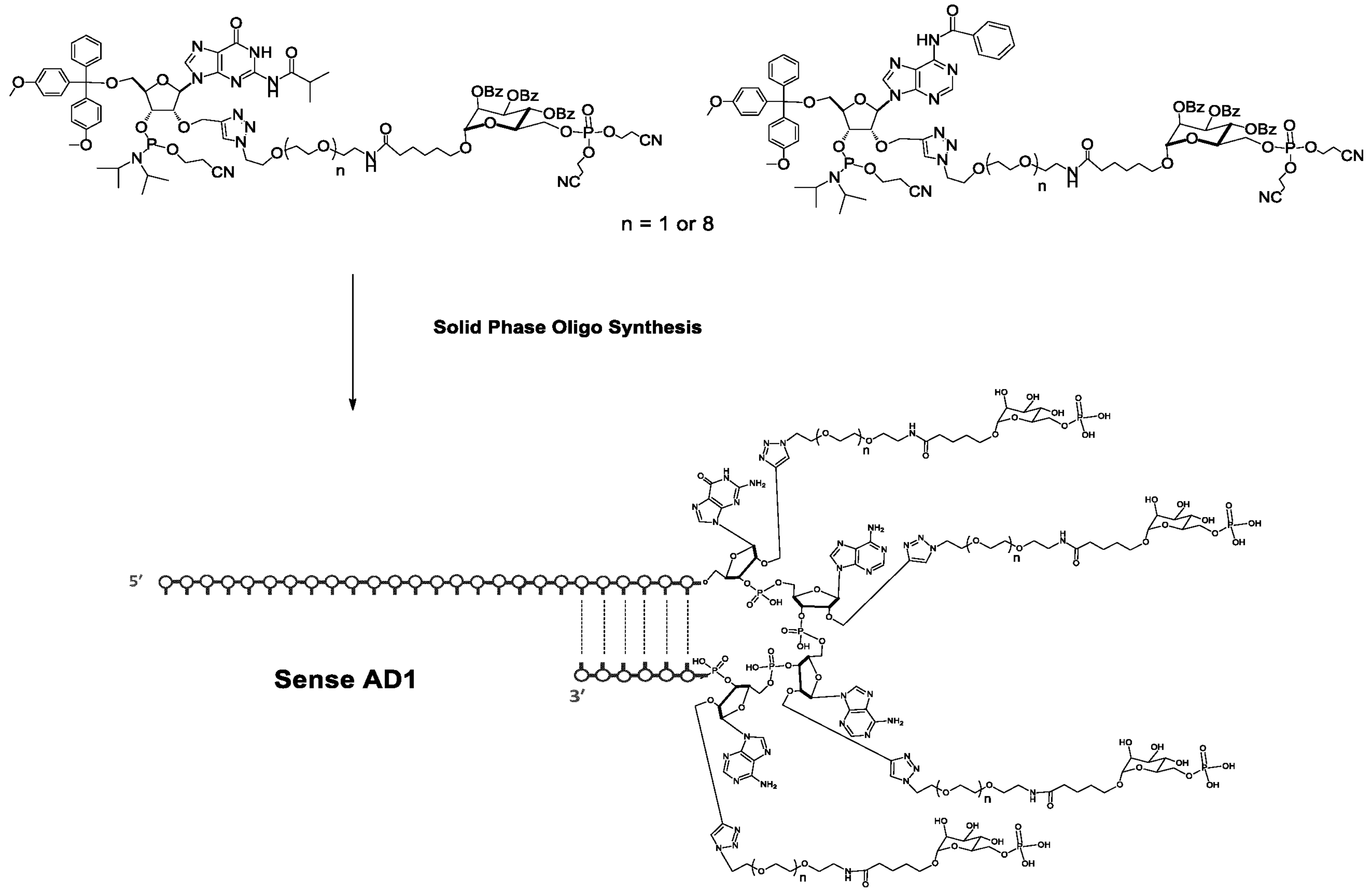

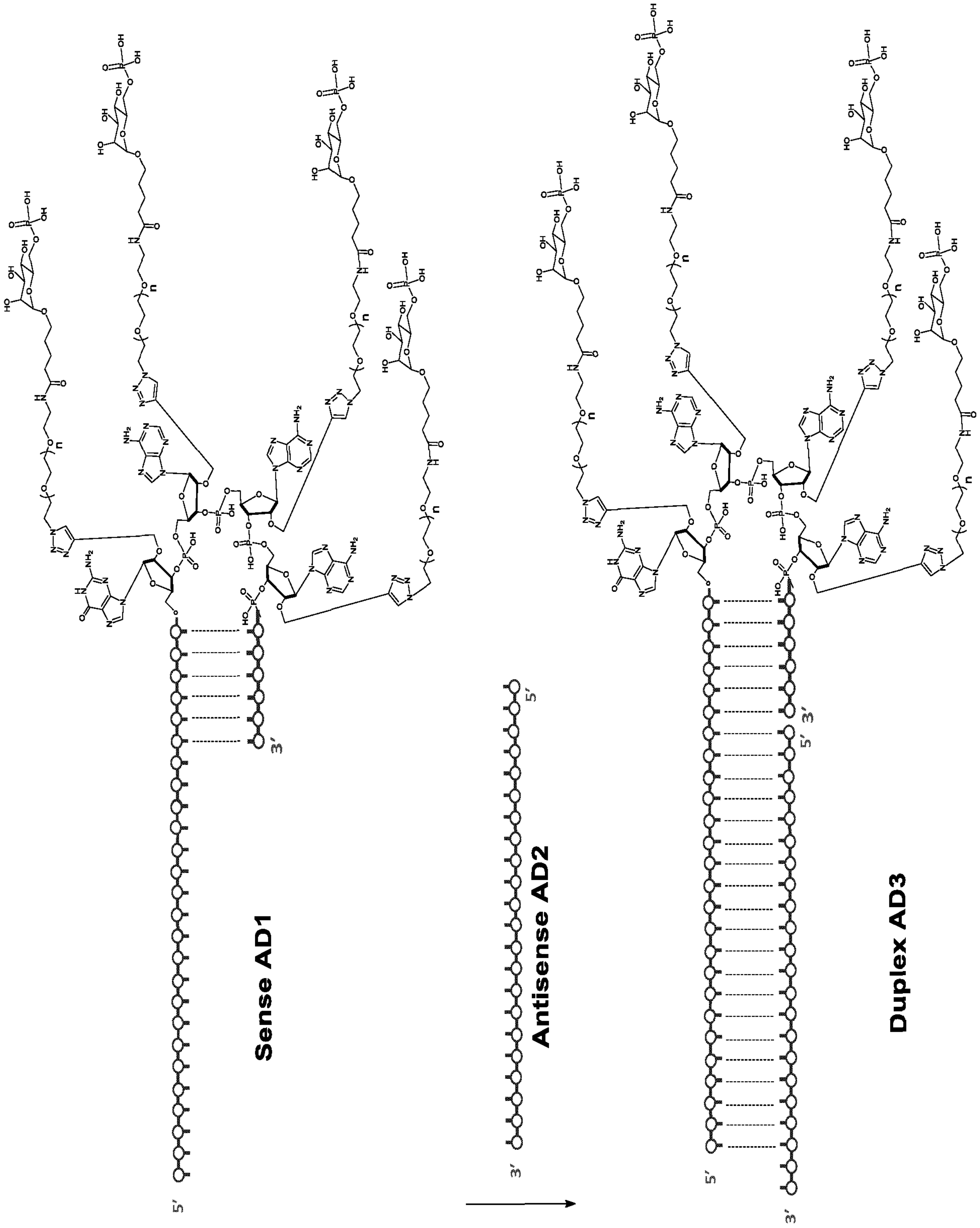
Sense AD1
Antisense AD2
Duplex AD3
5'
3'

**SCHEME AE: Solid Phase Synthesis of Mannose-6-Phosphate Nicked Tetraloop dsNA Conjugate Using 2'- Acetal Linked Mannose-6-Phosphate Nucleoside Phosphoroamidites**

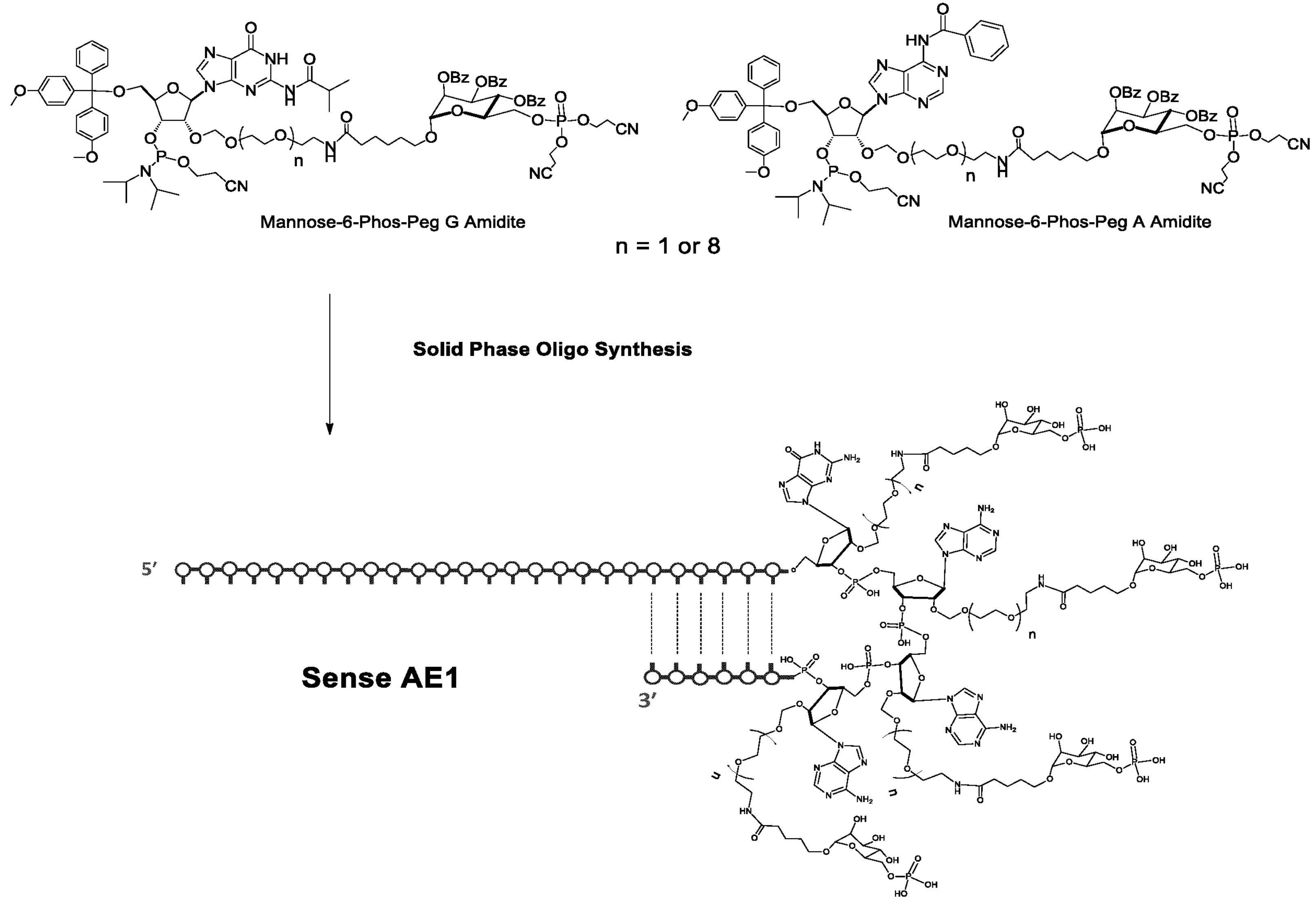

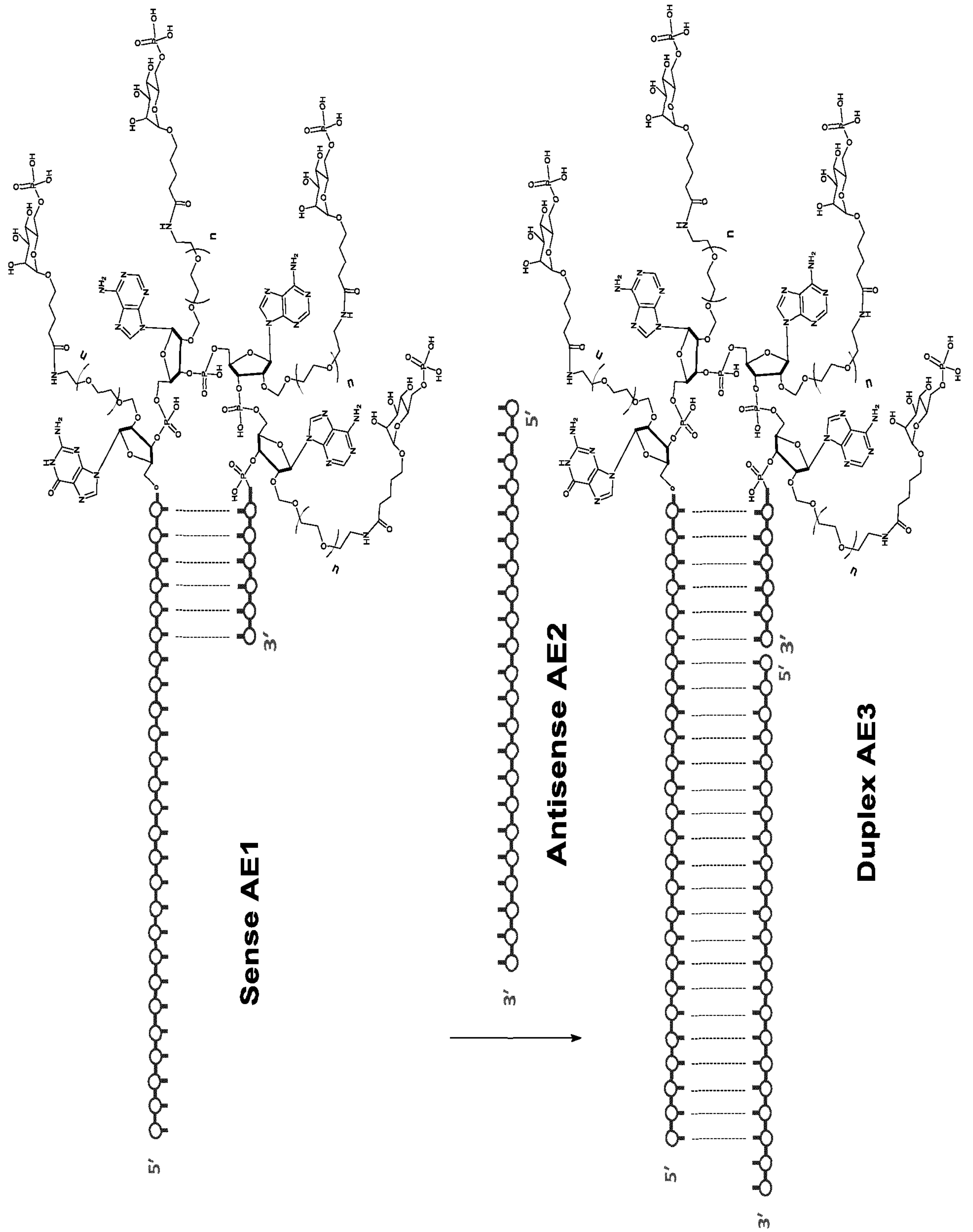

Sense AE1

Antisense AE2

Duplex AE3

**SCHEME AF: Synthesis of Nicked Tetraloop dsNA Conjugate with Multiple Mannose-6-Phosphate on the Stem Post Synthetic Conjugation Using "Click" Chemsitry**

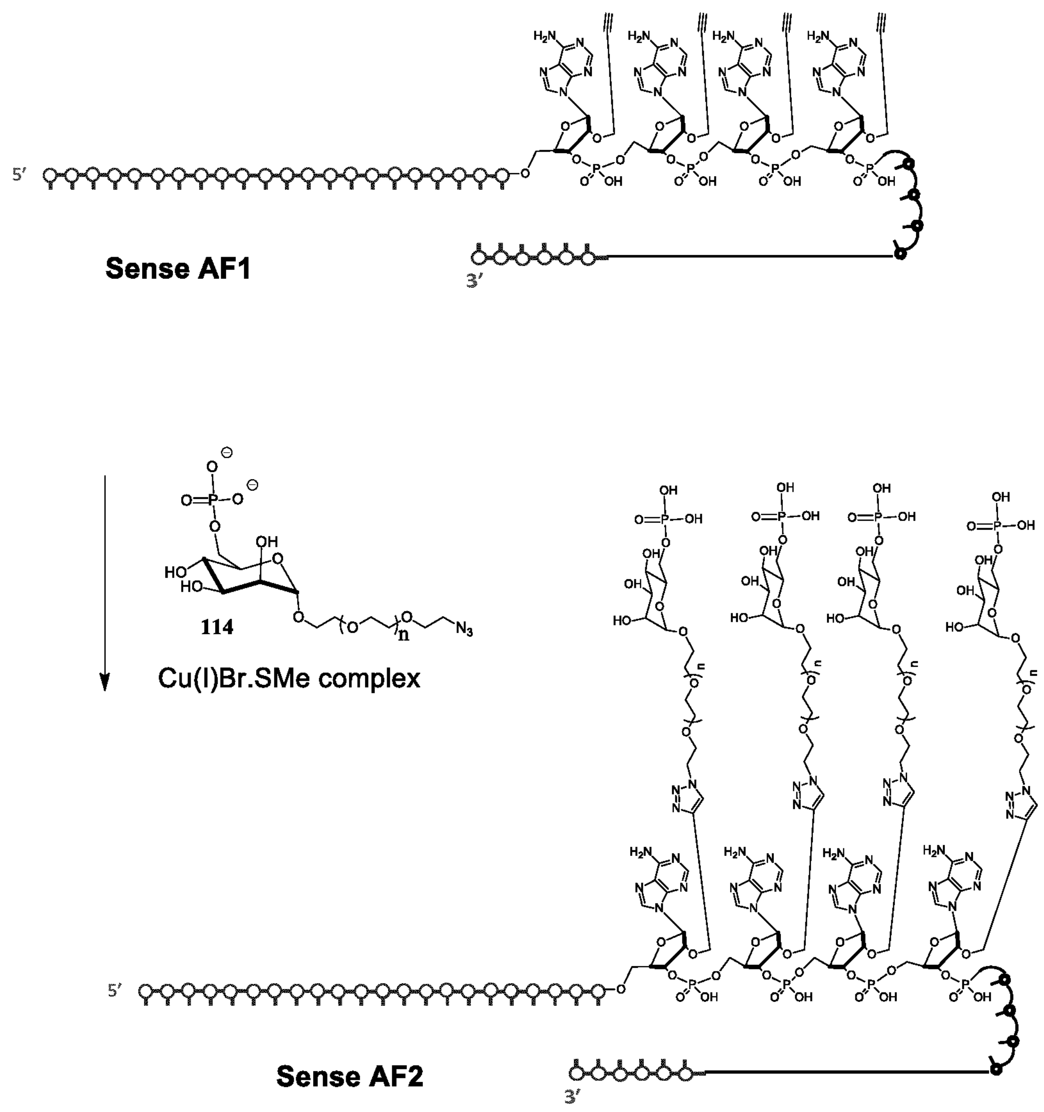

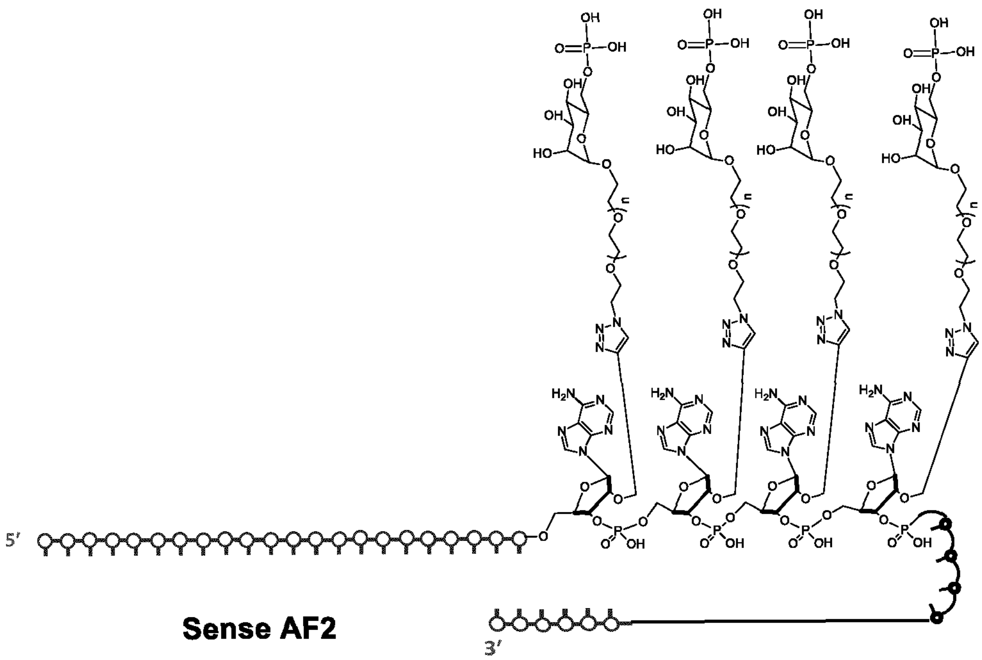

Sense AF2

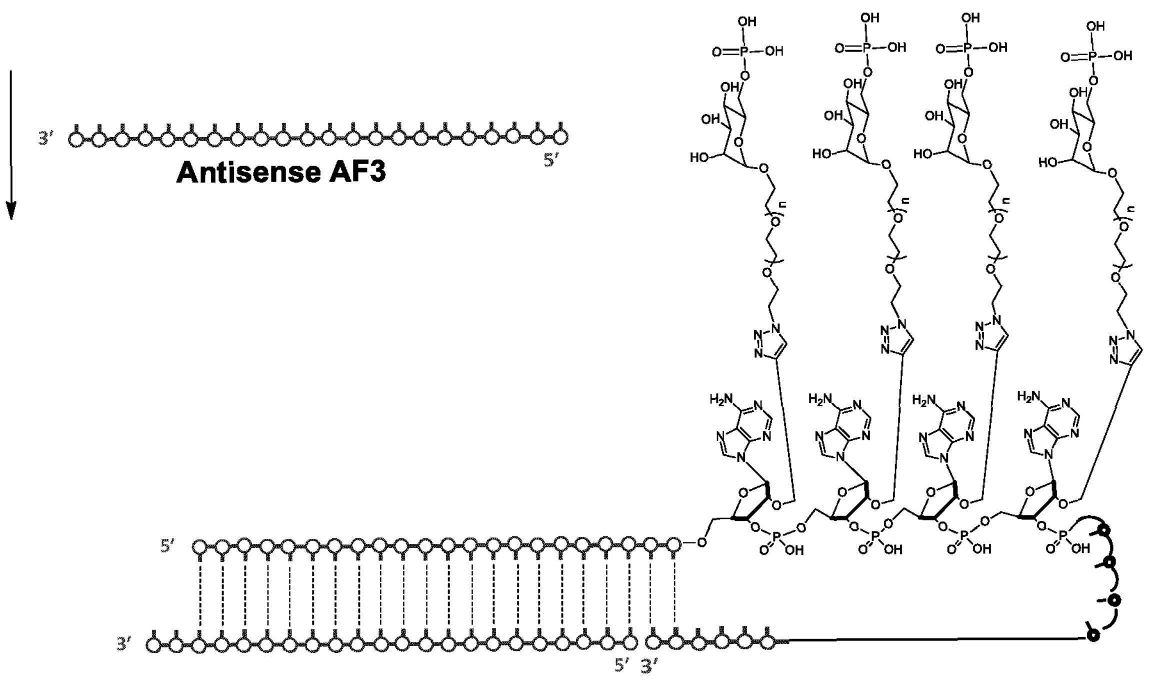

Antisense AF3

Duplex AF4

**SCHEME AG: Synthesis of dsNA Conjugate with Multiple Mannose-6-Phosphate on the 5'-Extension of Antisense via Post Synthetic Conjugation**

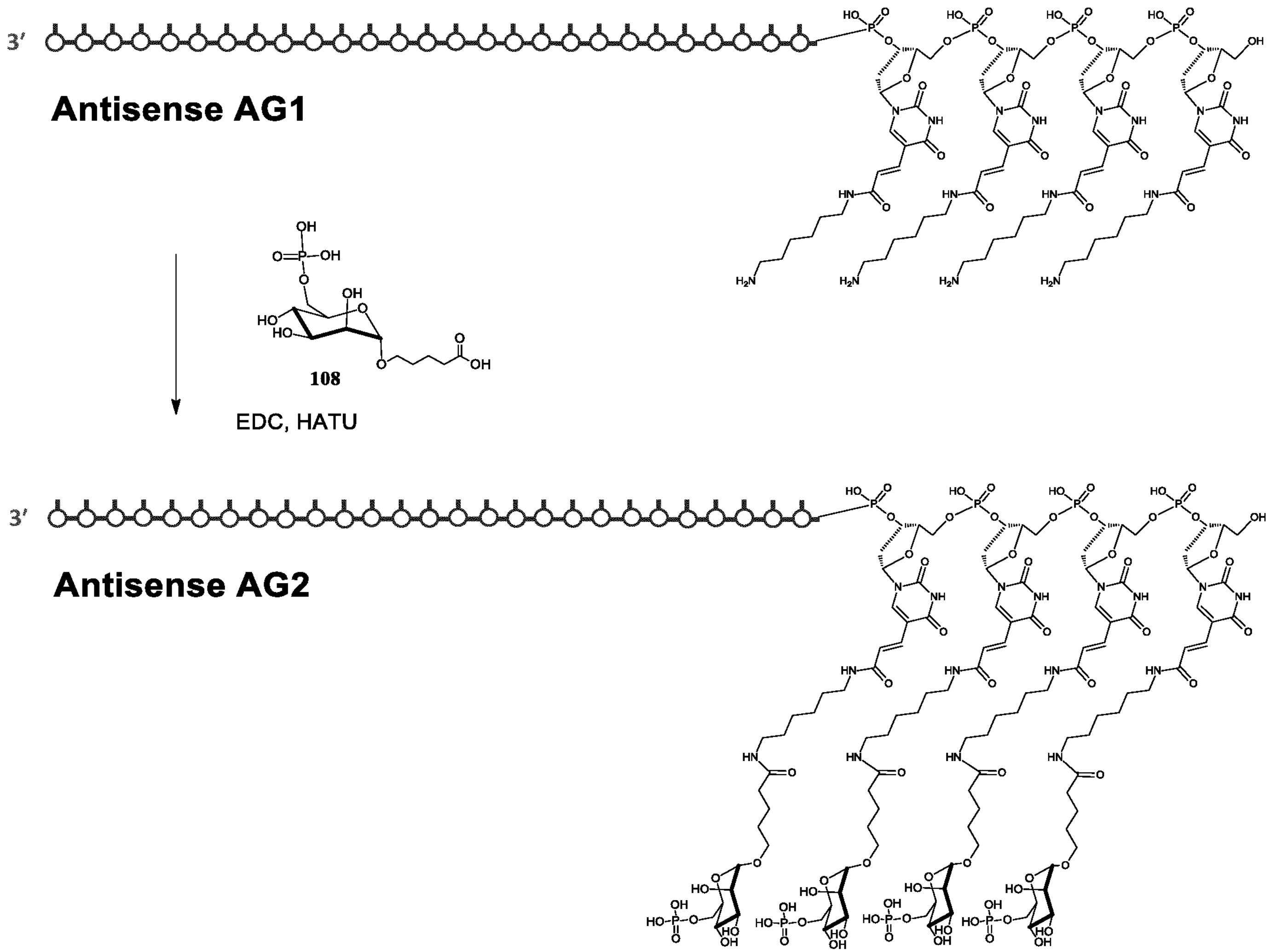


Antisense AG1

108

Antisense AG2

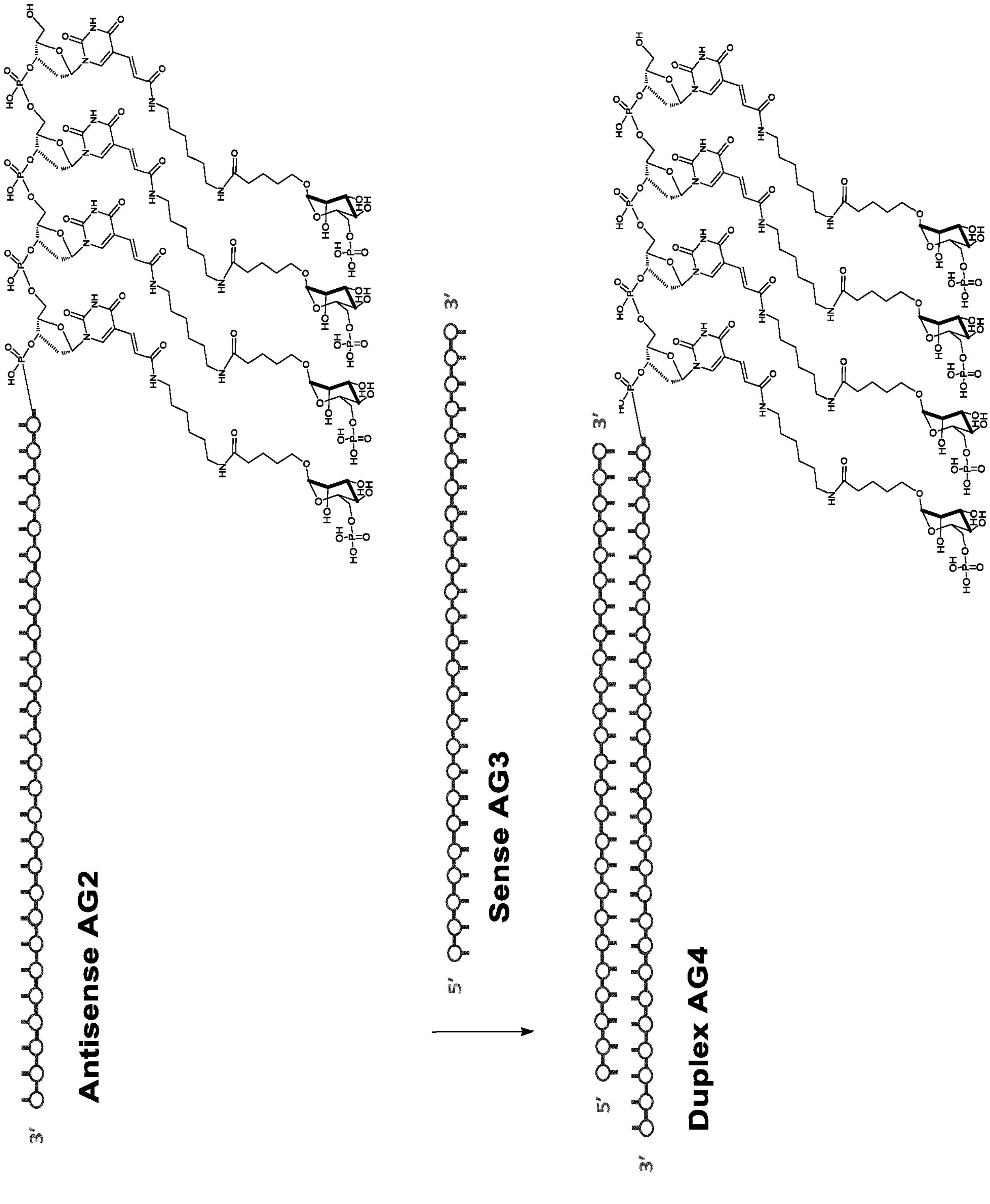

Antisense AG2

Sense AG3

Duplex AG4

## SCHEME AH: Synthesis of dsNA Conjugate with Multiple Mannose-6-Phosphate on the 5'-Extension of Sense via Post Synthetic Conjugation

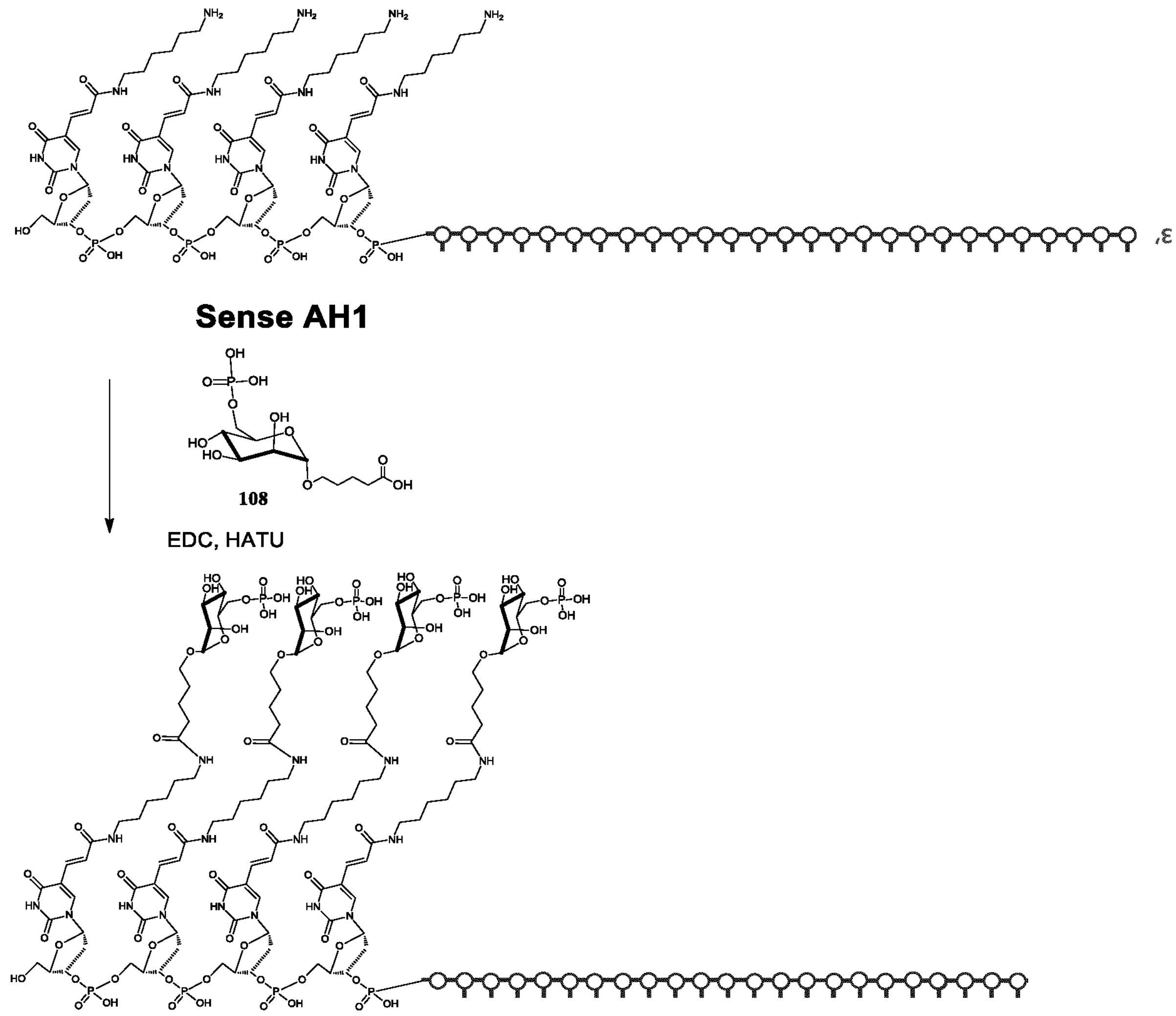


Sense AH1

108

Sense AH2

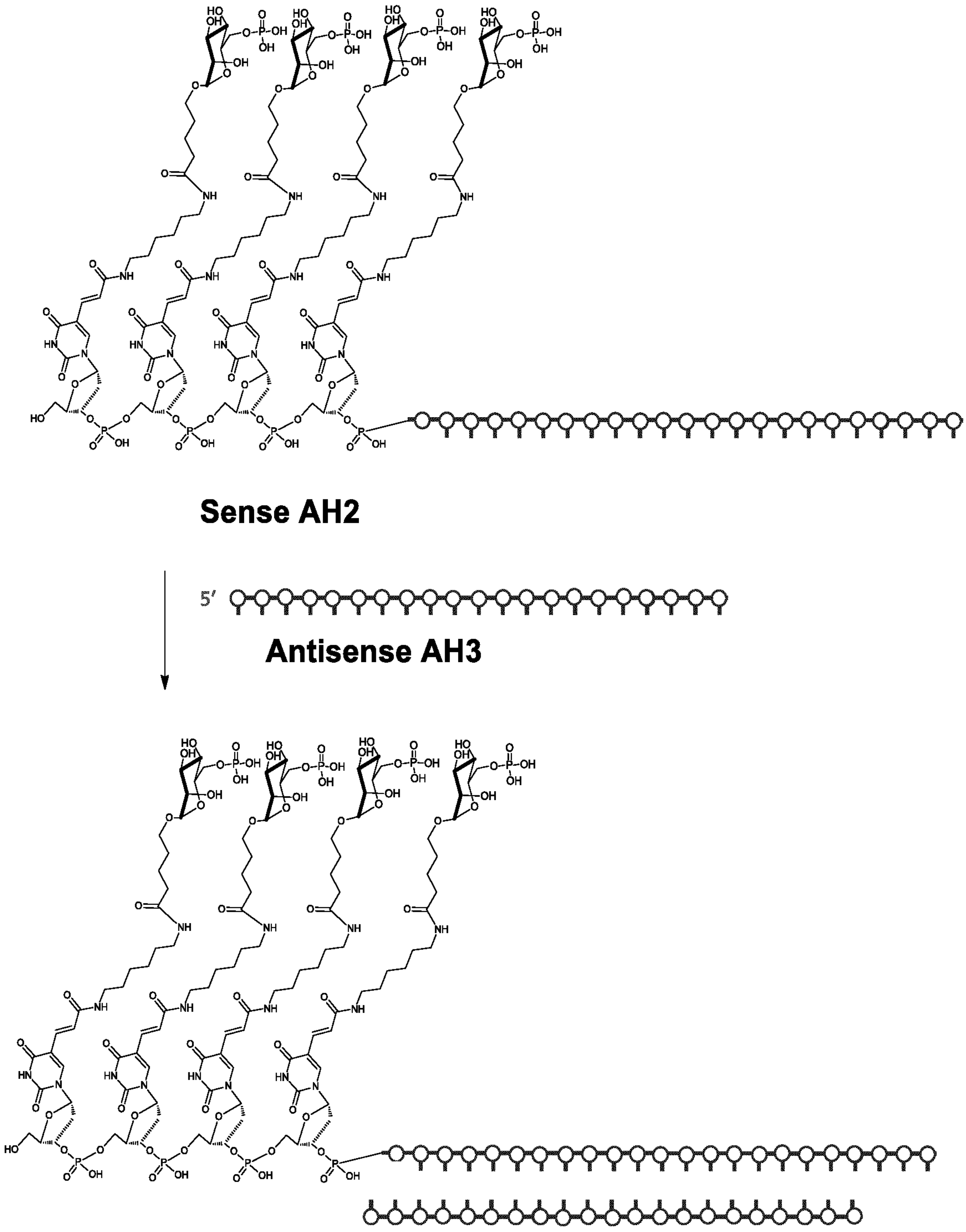

Sense AH2

5'

Antisense AH3

Duplex AH4

**SCHEME AI: Synthesis of dsNA Conjugate with Mannose-6-Phosphate Separated By One Spacer On The 5'-Extension of Sense via Post Synthetic Conjugation**

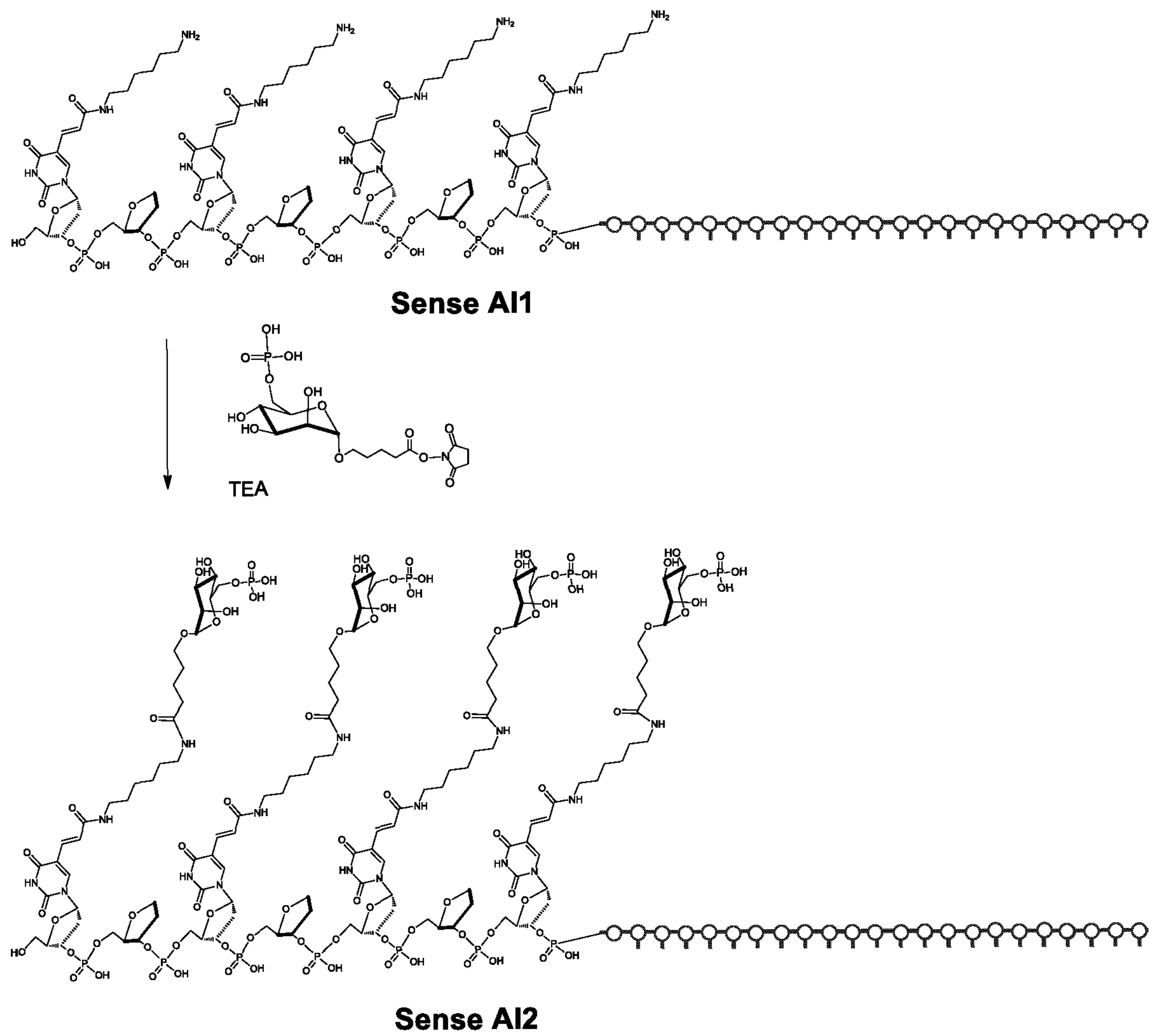


Sense AI1

Sense AI2

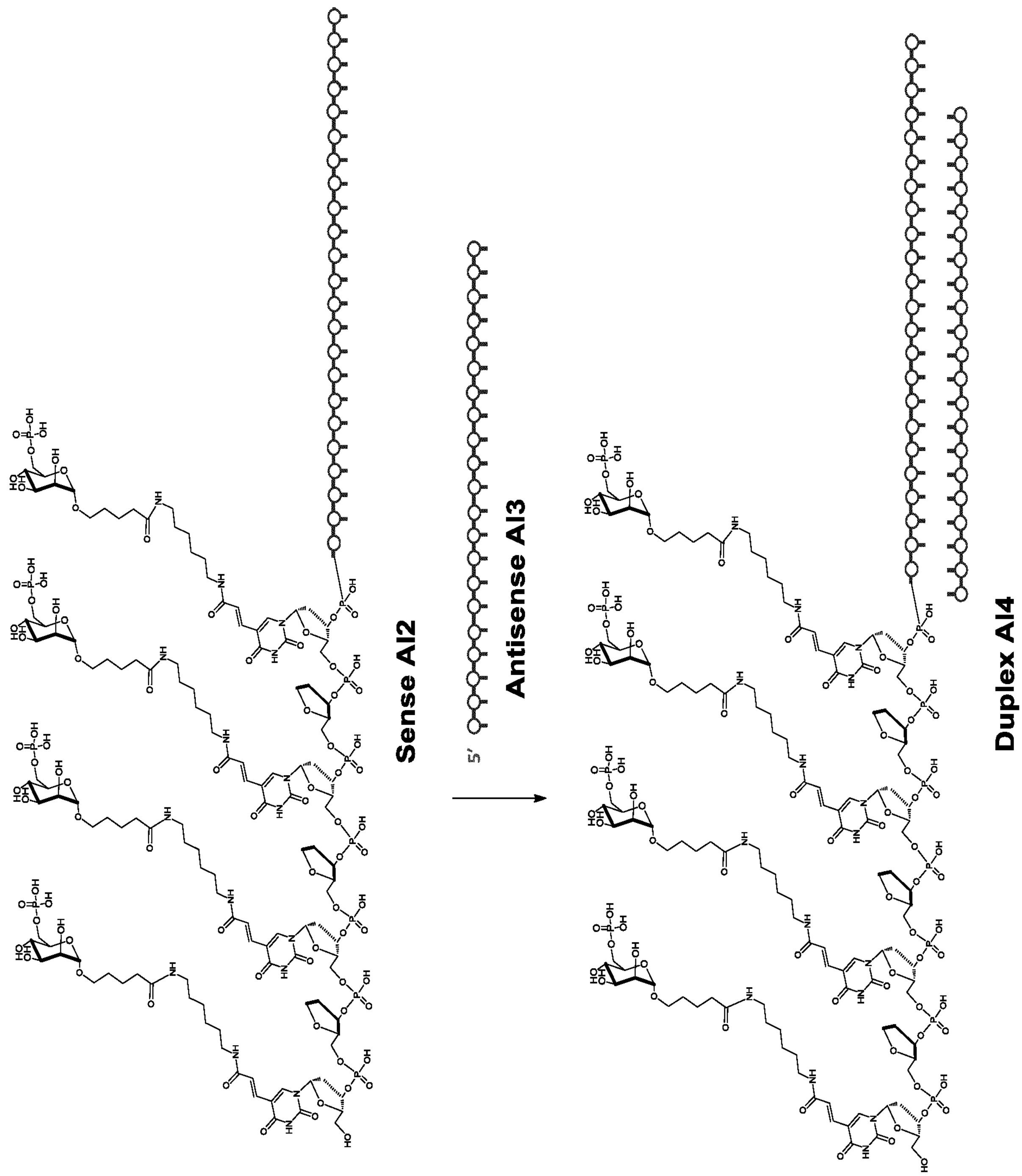

Sense AI2

5'

Antisense AI3

Duplex AI4

**SCHEME AJ: Synthesis of dsNA Conjugate with Mannose-6-Phosphate Separated By Two Spacers On The 5'-Extension of Sense via Post Synthetic Conjugation**

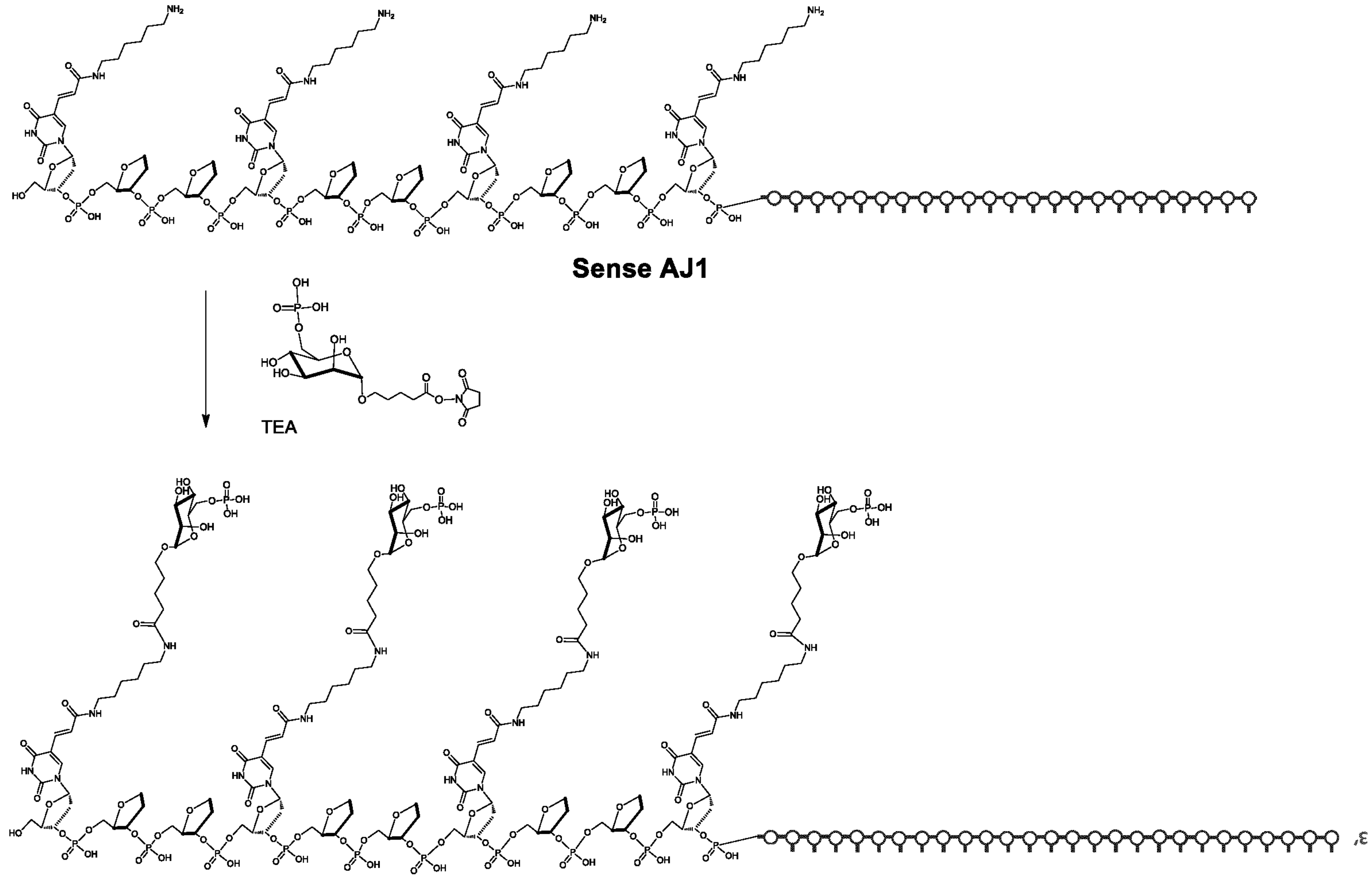


**Sense AJ1**

**Sense AJ2**

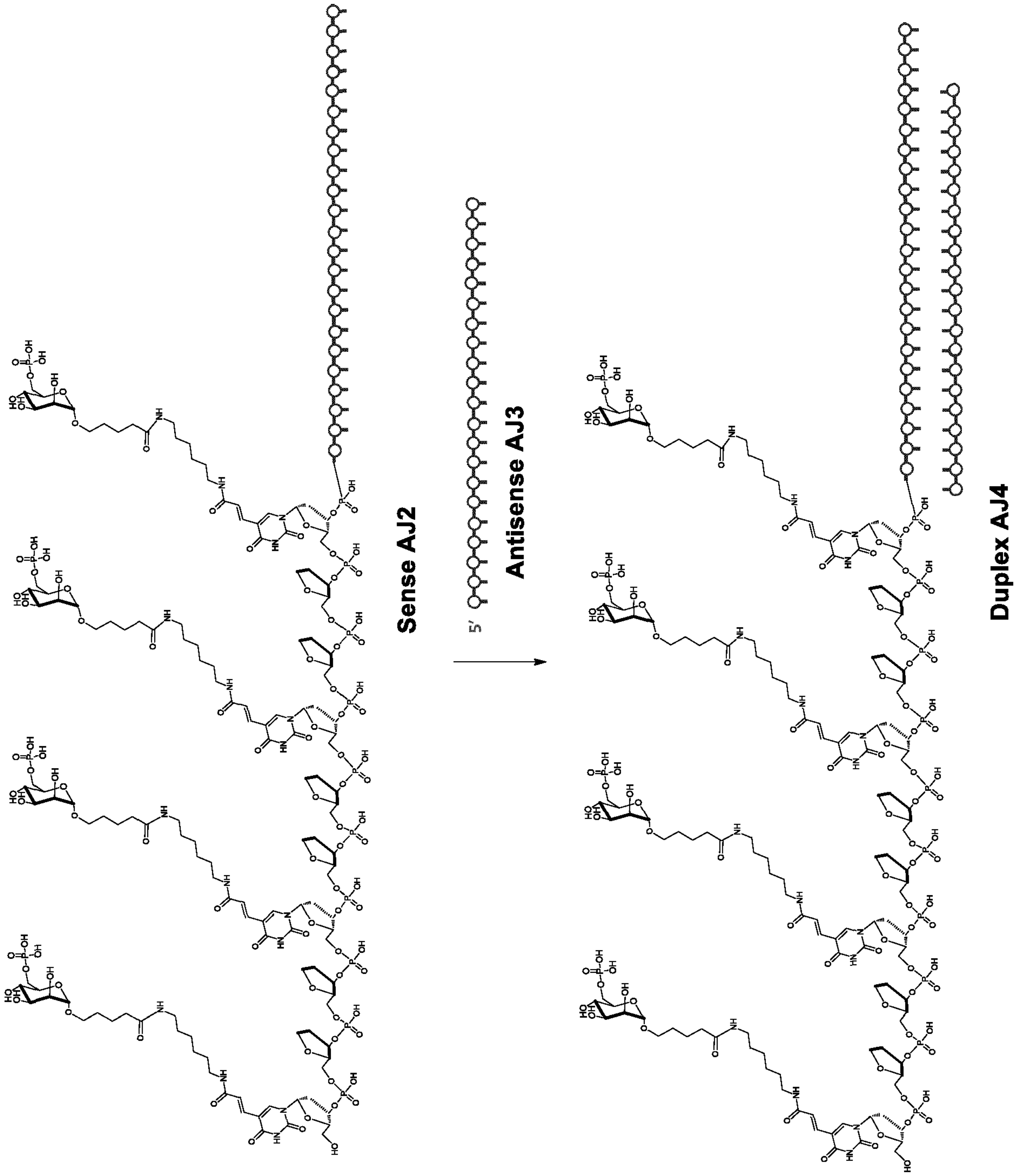

Sense AJ2

Antisense AJ3

Duplex AJ4

**SCHEME AK: Synthesis of dsNA Conjugate with Mannose-6-Phosphate Block Separated By Multiple Spacers On The 5'-Extension of Sense via Post Synthetic Conjugation**

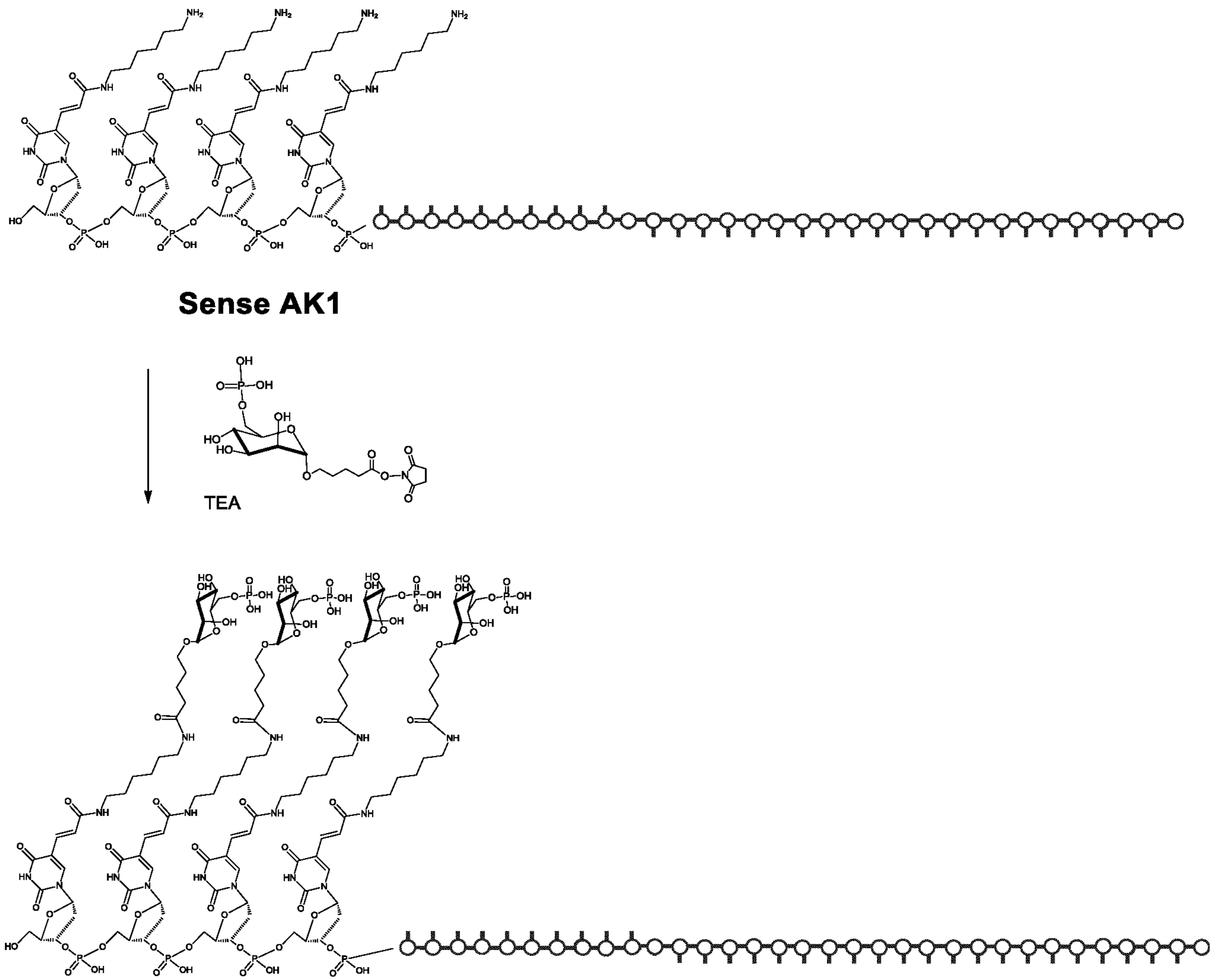


**Sense AK1**

**Sense AK2**

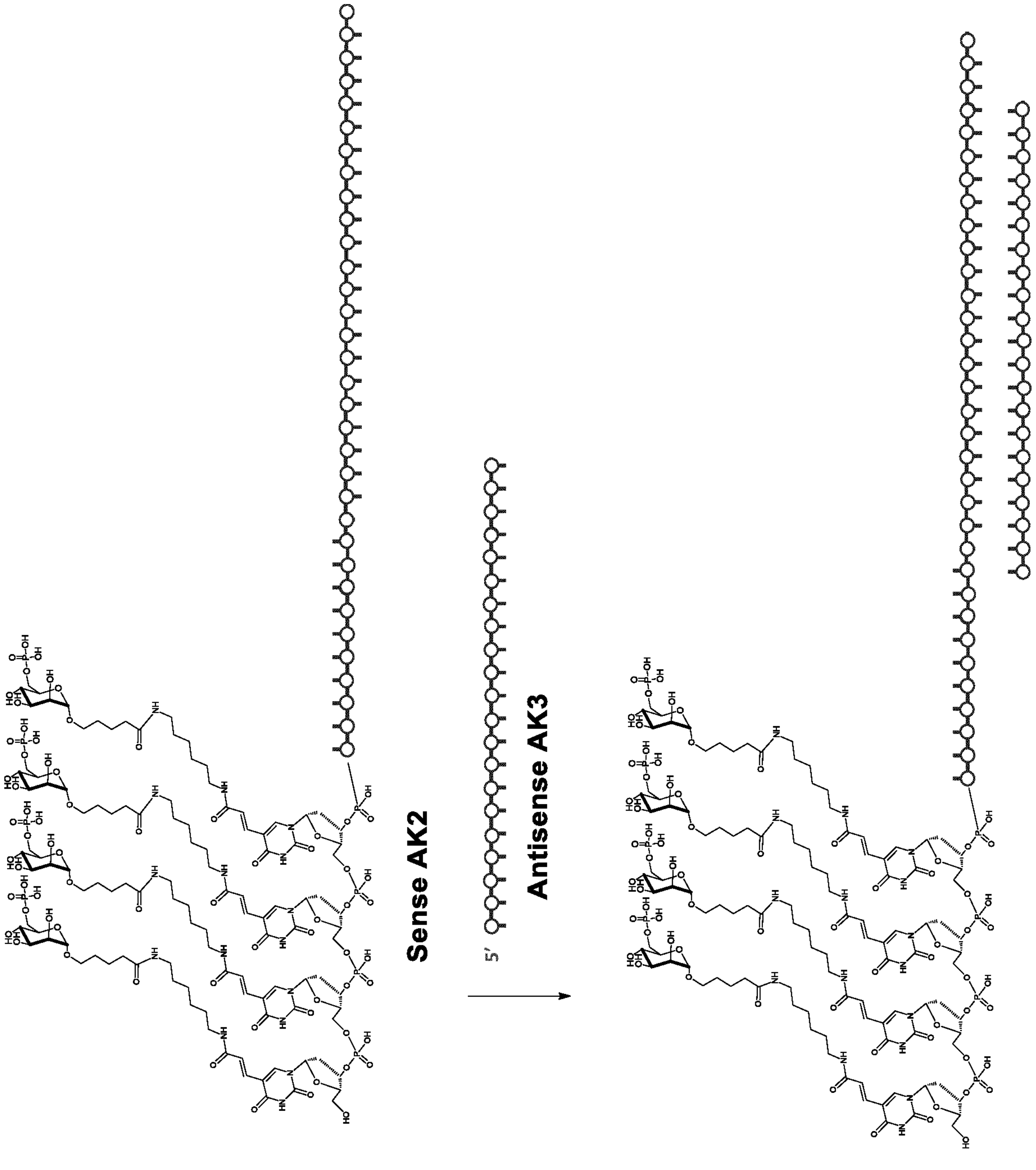

Sense AK2

5'

Antisense AK3

Duplex AK4

**SCHEME AL: Synthesis of dsNA Conjugate with Mannose-6-Phosphate Separated By Two Spacers On The 5'-Extension of Antisense via Post Synthetic Conjugation**

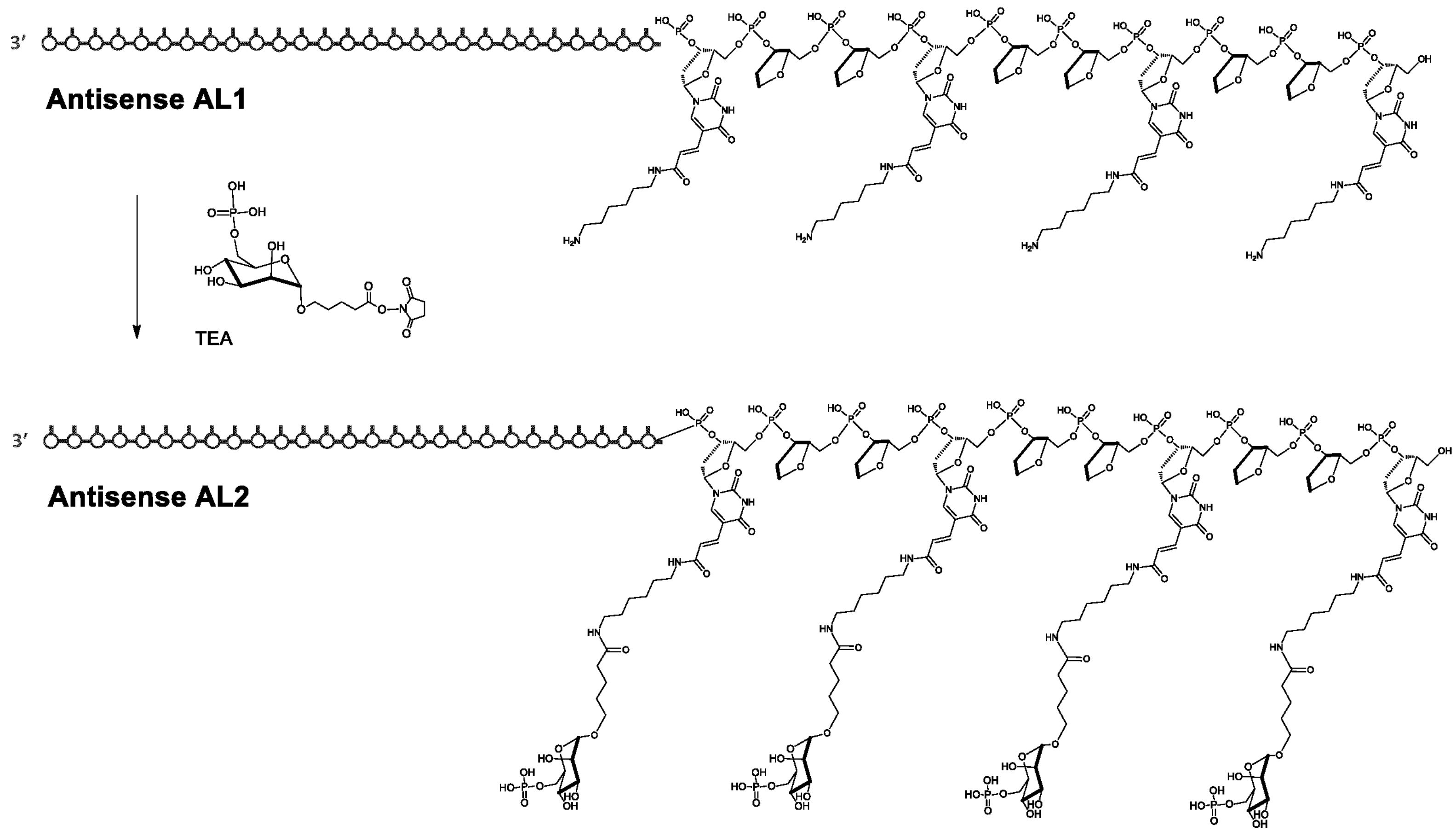

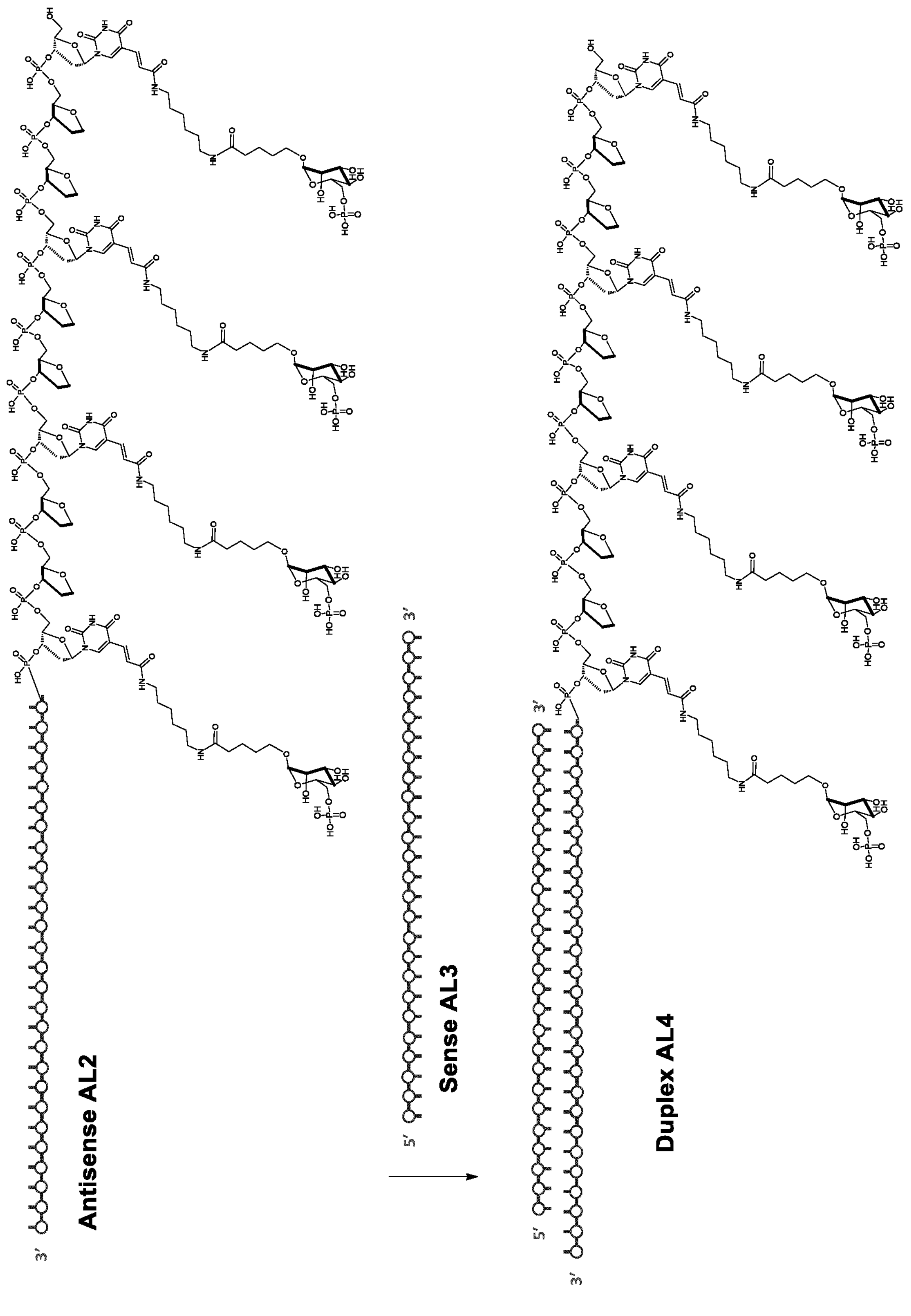
Antisense AL2
3'
Sense AL3
5'
3'
Duplex AL4
5'
3'
3'

**SCHEME AM: Synthesis of dsNA Conjugate with Mannose-6-Phosphate Block Separated By Multiple Spacers On The 5'-Extension of Antisense via Post Synthetic Conjugation**

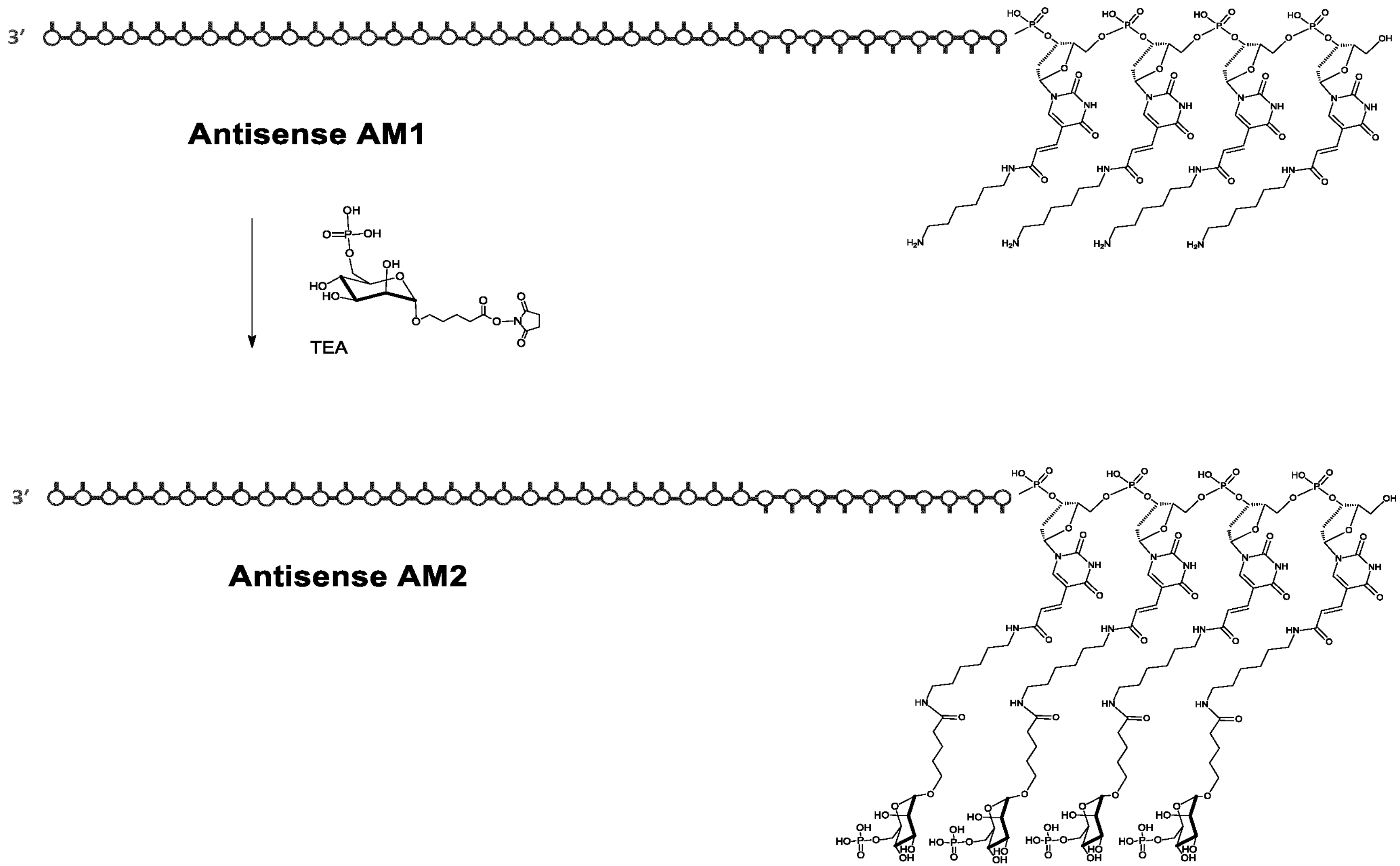


**Antisense AM1**

**Antisense AM2**

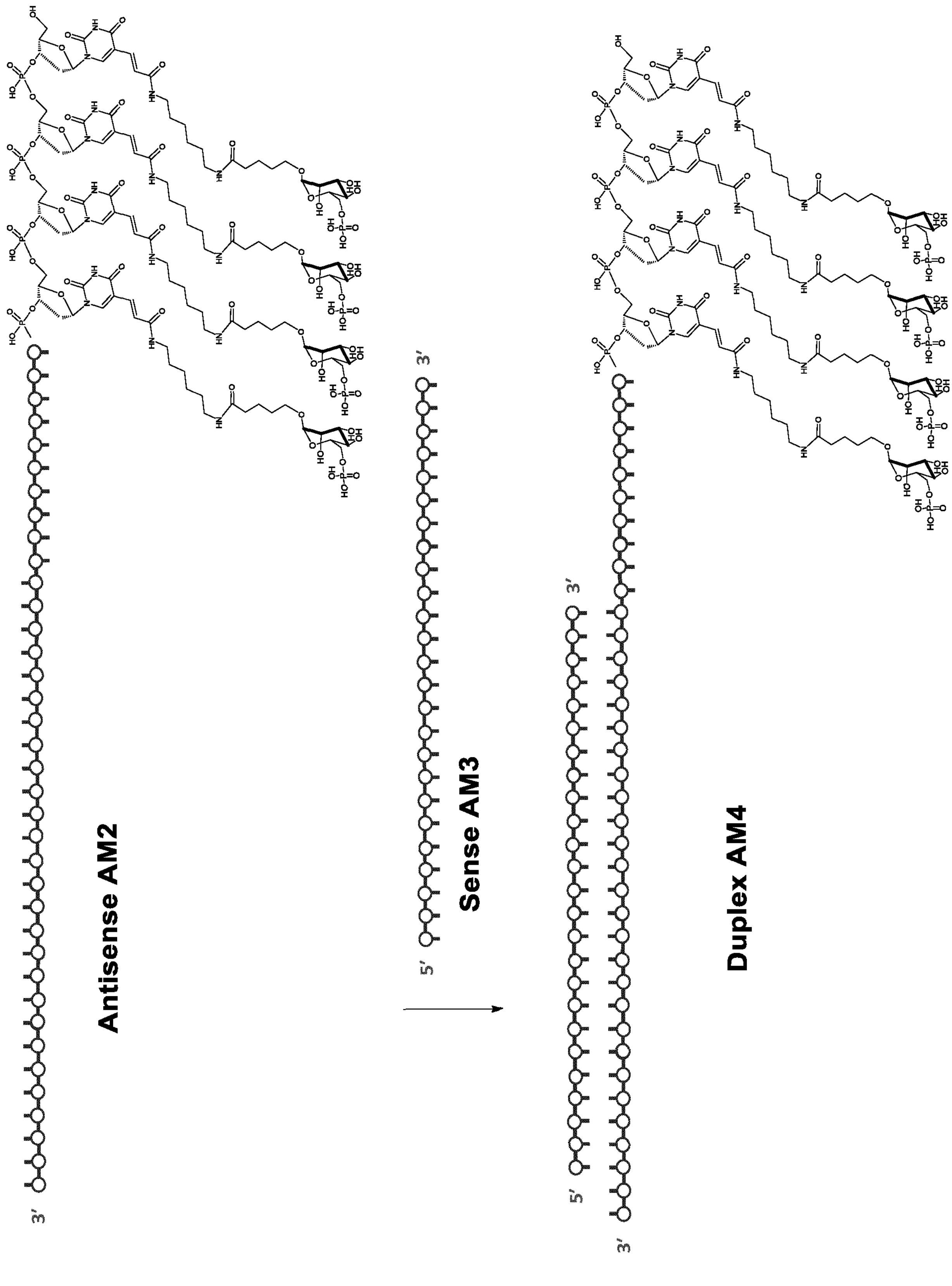
Antisense AM2
3'
Sense AM3
5'
3'
Duplex AM4
5'
3'
3'

**SCHEME AN: Solid Phase Synthesis of Mannose-6-Phosphate 5'-Extended dsNA Conjugate Using Mannose-6-Phosphate Bearing Nucleoside Phosphoroamidite**

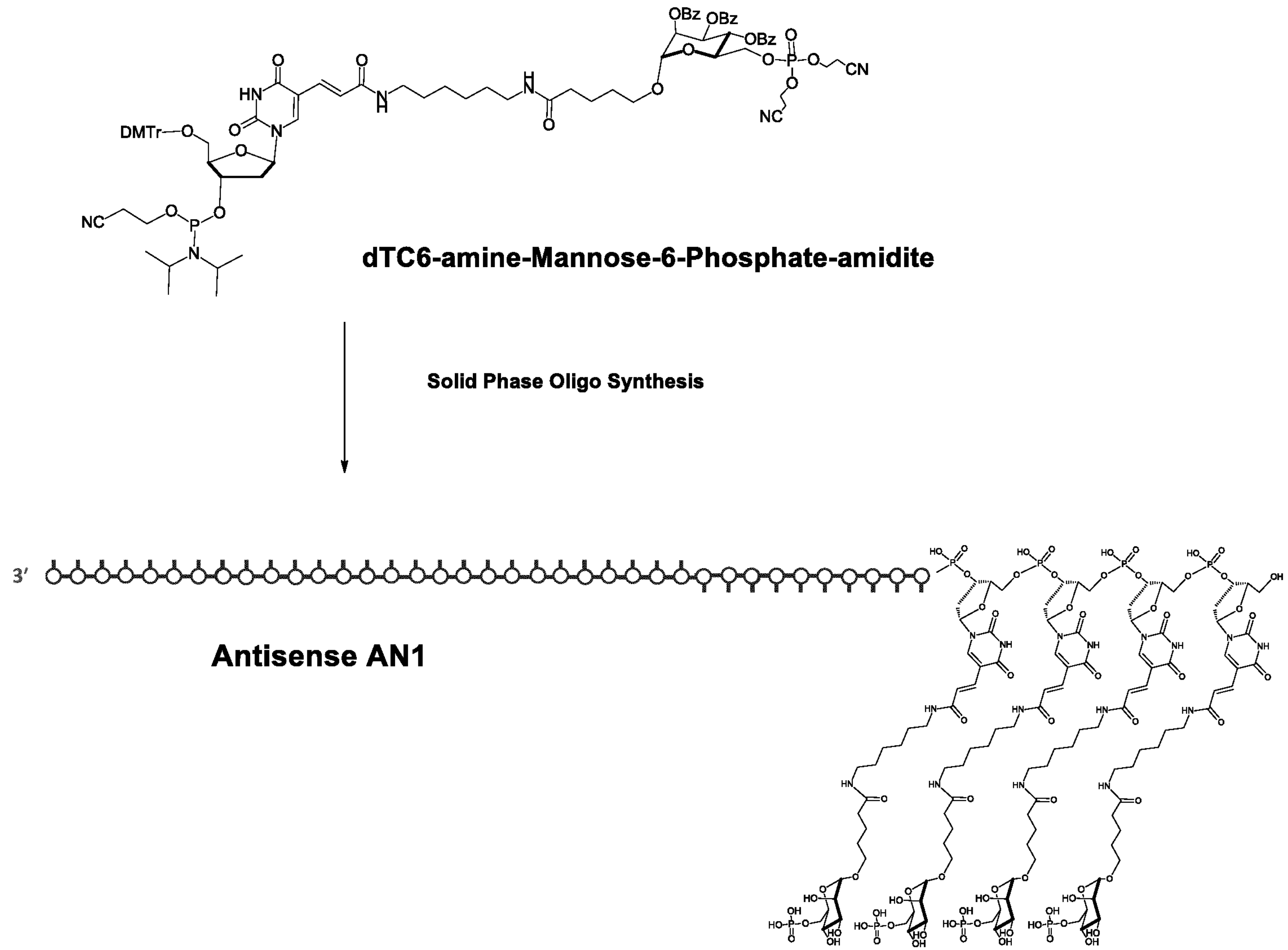


**dTC6-amine-Mannose-6-Phosphate-amidite**

**Antisense AN1**

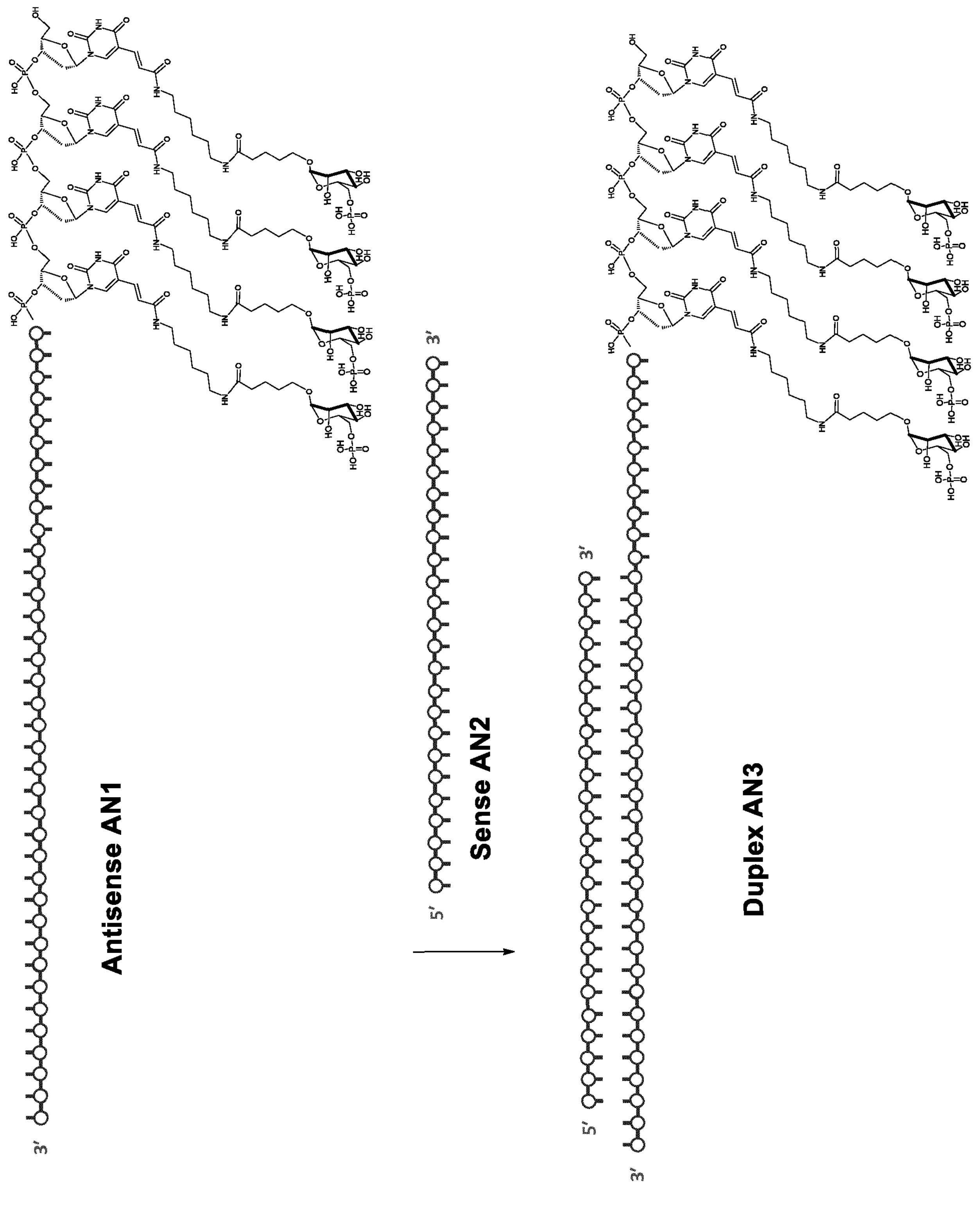

Antisense AN1

Sense AN2

Duplex AN3

**Table 3.**

Preparations of N-acetylgalactosamine ligands, N-acetylgalactosamine containing nucleotides, and N-acetylgalactosamine oligonucleotide conjugates are described above in the examples and synthetic schemes. Specific oligonucleotide sequences used for the compounds and/or conjugates described below are shown in Table 2.

**Table 4.**

Linkers useful according to the invention include but are not limited to the linkers presented in Table 3. A single ligand conjugated dsNA may comprise more than one type of linker.

**Table 5.**

Linkers useful according to the invention include but are not limited to the linkers presented in Table 4.

**TABLE 3**

| Duplex Code | Gene Target | Strand | ID | Sequence |
|---|---|---|---|---|
| DP2294P: DP2483G | CTNNB1 | sense | 1 | [rAs][mGs][rA][mA][mU][mA][rC][mA][rA][rA][rU][mG][rA][mU][rG][mU][mA][mG][rA][rA][rA][rC][rAs][dGs][dC] |
| | | antisense | 2 | [C6Tn-GalNAc][ab][ab][C6Tn-GalNAc][ab][ab][C6Tn-GalNAc][ab][ab][C6Tn-GalNAc][mGs][mC][mU][mG][rU][rU][rU] [rC][mU][rA][mC][rA][mU][rC][mA][rU][rU][rU][rG][rU][mA][rU][mU][rC][mU][mGs][mC] |
| DP2479P: DP2281G | CTNNB1 | sense | 3 | [C6Tn-GalNAc][ab][ab][C6Tn-GalNAc][ab][ab][C6Tn-GalNAc][ab][ab][C6Tn-GalNAc][iB][mC][mU][fG][mU][mU][fG] [fG][fA][mU][mU][fG][fA][mU][mU][mC][fG][fA][fA][fA][mUs] [mU][iB] |
| | | antisense | 4 | [mUs][fUs][mUs][fC][mG][fA][mA][fU][mC][fA][mA][fU][mC][fC][mA][fA][mC][fA][mG][mUs][mU] |

(continued)

| Duplex Code | Gene Target | Strand | ID | Sequence |
|---|---|---|---|---|
| DP2480P: DP2281G | CTNNB1 | sense | 5 | [C6Tn-GalNAc][ab][C6Tn-GalNAc][ab][C6Tn-GalNAc][ab][C6Tn-GalNAc][iB][mC][mU][fG][mU][mU][fG][fG][fA][mU][mU][fG][fA][mU][mU][mC][fG][fA][fA][fA][mUs][mU][iB] |
| | | antisense | 6 | [mUs][fUs][mUs][fC][mG][fA][mA][fU][mC][fA][mA][fU][mC][fC][mA][fA][mC][fA][mG][mUs][mU] |
| DP2481P: DP2281G | CTNNB1 | sense | 7 | [C6Tn-GalNAc][C6Tn-GalNAc][C6Tn-GalNAc][C6Tn-GalNAc][iB][mC][mU][fG][mU][mU][fG][fG][fA][mU][mU][fG][fA][mU][mU][mC][fG][fA][fA][fA][mUs][mU][iB] |
| | | antisense | 8 | [mUs][fUs][mUs][fC][mG][fA][mA][fU][mC][fA][mA][fU][mC][fC][mA][fA][mC][fA][mG][mUs][mU] |

(continued)

| Duplex Code | Gene Target | Strand | ID | Sequence |
|---|---|---|---|---|
| DP2292P: DP2483G | CTNNB1 | sense | 9 | [rAs][mG][rA][mA][mU][mA][rC][mA][rA][rA][rU][mG][rA][mU][rG][mU][mA][mG][rA][rA][rA][rC][rAs][dGs][dC] |
| | | antisense | 10 | [C6Tn-GalNAc][ab][ab][C6Tn-GalNAc][ab][ab][C6Tn-GalNAc] [ab][ab][C6Tn-GalNAc][mGs][mC][mU][mG][rU][rU][rU][rC][mU][rA][mC][rA][mU][rC][mA][rU][rU][rU][rG][rU][mA][rU] [mU][rC][mU][mGs][mC] |
| DP2421P: DP2439G | CTNNB1 | sense | 11 | [fAs][mG][fA][mA][mU][mA][fC][mA][fA][fA][fU][mG][fA][mU][fG][mU][mA][mG][fA][fA][rA][rC][fAs][dGs][dC] |
| | | antisense | 12 | [C6Tns-GalNAc][C6Tns-GalNAc][C6Tns-GalNAc][C6Tns-GalNAc][mUs][fAs][mGs][mCs][mUs][fAs][mUs][mCs][mGs][dTs][mGs][mC][mU][mG][rU][rU][rU][mC][mU][fA][mC][fA][mU][fC][mA][rU][rU][rU][fG][fU][mA][fU][mU][fC][mU][mGs][mC] |

(continued)

| Duplex Code | Gene Target | Strand | ID | Sequence |
|---|---|---|---|---|
| DP2421P: DP2436G | CTNNB1 | sense | 13 | [fAs][mG][fA][mA][mU][mA][fC][mA][fA][fA][fU][mG][fA][mU][fG][mU][mA][mG][fA][fA][rA][rC][fAs][dGs][dC] |
| | | antisense | 14 | [C6Tn-GalNAc][C6Tn-GalNAc][C6Tn-GalNAc][C6Tn-GalNAc][mUs][fAs][mGs][mCs][mUs][fAs][mUs][mCs][mGs][dTs][mGs][mC][mU][mG][rU][rU][rU][mC][mU][fA][mC][fA][mU][fC][mA][rU][rU][rU][fG][fU][mA][fU][mU][fC][mU][mGs][mC] |
| DP2555P: DP2501G | HAO1 | sense | 15 | [mAs][mU][fA][mU][mU][mU][mU][fC][fC][fC][fA][fU][fC][mU][mG][mU][fA][mU][mU][fA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 16 | [5Phos][fUs][fAs][mAs][mU][mA][mC][fA][mG][mA][mU][fG][fG][fG][fA][fA][fA][mA][mU][fA][mU][mUs][mG] |

(continued)

| Duplex Code | Gene Target | Strand | ID | Sequence |
|---|---|---|---|---|
| DP2554P: DP2505G | HAO1 | sense | 17 | [mAs][mU][fA][mU][fU][mU][fU][fC][fC][fC][fA][fU][fC][mU][fG][mU][fA][mU][fU][mA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 18 | [5Phos][fUs][mAs][fAs][mU][fA][mC][fA][mG][fA][mU][fG][mG][fG][fA][fA][mA][fA][mU][fA][mU][mUs][mU] |
| DP2554P: DP2509G | HAO1 | sense | 19 | [mAs][mU][fA][mU][fU][mU][fU][fC][fC][fC][fA][fU][fC][mU][fG][mU][fA][mU][fU][mA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 20 | [5Phos][fUs][fAs][mAs][fU][mA][fC][fA][fG][mA][fU][fG][fG][fG][fA][fA][fA][mA][fU][fA][fU][mUs][mG] |

(continued)

| Duplex Code | Gene Target | Strand | ID | Sequence |
|---|---|---|---|---|
| DP2556P: DP2501G | HAO1 | sense | 21 | [iB][mA][mU][fA][mU][fU][mU][fU][mC][fC][fC][fA][mU][fC][mU][fG][mU][fA][mU][fU][mA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 22 | [5Phos][fUs][fAs][mAs][mU][mA][mC][fA][mG][mA][mU][fG][fG][fG][fA][fA][fA][mA][mU][fA][mU][mUs][mG] |
| DP2557P: DP2501G | HAO1 | sense | 23 | [iB][mA][mU][fA][mU][mU][mU][mU][mC][mC][mC][fA][mU][mC][mU][fG][mU][fA][mU][mU][fA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 24 | [5Phos][fUs][fAs][mAs][mU][mA][mC][fA][mG][mA][mU][fG][fG][fG][fA][fA][fA][mA][mU][fA][mU][mUs][mG] |

(continued)

| Duplex Code | Gene Target | Strand | ID | Sequence |
|---|---|---|---|---|
| DP2553P: DP2507G | HAO1 | sense | 25 | [iB][mA][mU][fA][mU][mU][mU][mU][mC][mC][mC][fA][mU][mC][mU][fG][mU][fA][mU][mU][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 26 | Phos][mAs][fAs][mUs][fA][mC][fA][mG][fA][mU][fGnG][fG][mA][fA][mA][fA][mU][fA][mU][mUs][mU] |
| DP2463P: DP2362G | CTNNB1 | sense | 27 | [fA][mG][fA][mA][fU][mA][fC][mA][fA][fA][fU][mG][fA][mU][fG][mU][fA][mG][fA][mA][mG][mC][mA][mG][mC][rG][prgA-dPEG11-GalNAc][prgA-dPEG11-GalNAc][prgA-dPEG11-GalNAc][mG][mC][mU][mG][mC][mU] |
| | | antisense | 28 | [5Phos][mUs][dCs][fU][mA][fC][mA][fU][mC][fA][mU][fUs][dT][mG][fU][mAs][dT][fUs][dC][fUs][dGs][dC] |

(continued)

| Duplex Code | Gene Target | Strand | ID | Sequence |
|---|---|---|---|---|
| DP2464P: DP2362G | CTNNB1 | sense | 29 | [fA][mG][fA][mA][fU][mA][fC][mA][fA][fA][fU][mG][fA][mU][fG][mU][fA][mG][fA][mA][mG][mC][mA][mG][mC][prgG-dPEG11-GalNAc][prgA-dPEG11-GalNAc][prgA-dPEG11-GalNAc][prgA-dPEG11-GalNAc][mG][mC][mU][mG][mC][mU] |
| | | antisense | 30 | [5Phos][mUs][dCs][fU][mA][fC][mA][fU][mC][fA][mU][fUs][dT][mG][fU][mAs][dT][fUs][dC][fUs][dGs][dC] |
| DP2465P: DP2362G | CTNNB1 | sense | 31 | [fA][mG][fA][mA][fU][mA][fC][mA][fA][fA][fU][mG][fA][mU][fG][mU][fA][mG][fA][mA][mG][mC][mA][mG][mC][rG][prgA-dPEG11-TGN][rA][rA][mG][mC][mU][mG][mC][mU] |
| | | antisense | 32 | [5Phos][mUs][dCs][fU][mA][fC][mA][fU][mC][fA][mU][fUs][dT][mG][fU][mAs][dT][fUs][dC][fUs][dGs][dC] |

(continued)

| Duplex Code | Gene Target | Strand | ID | Sequence |
|---|---|---|---|---|
| DP2466P: DP2362G | CTNNB1 | sense | 33 | [fA][mG][fA][mA][fU][mA][fC][mA][fA][fA][fU][mG][fA][mU][fG][mU][fA][mG][fA][mA][mG][prgC-dPEG11-GalNAc][prgA-dPEG11-GalNAc][prgG-dPEG11-GalNAc][prgC-dPEG11-GalNAc][rG][rA][rA][rA][mG][mC][mU][mG][mC][mU] |
| | | antisense | 34 | [5Phos][mUs][dCs][fU][mA][fC][mA][fU][mC][fA][mU][fUs][dT][mG][fU][mAs][dT][fUs][dC][fUs][dGs][dC] |
| DP2467P: DP2362G | CTNNB1 | sense | 35 | [fA][mG][fA][mA][fU][mA][fC][mA][fA][fA][fU][mG][fA][mU][fG][mU][fA][mG][fA][mA][mG][mC][mA][mG][mC][rG][rA][rA][rA][mG][prgC-dPEG11-GalNAc][prgU-dPEG11-GalNAc][prgG-dPEG11-GalNAc][prgC-dPEG11-GalNAc][mU] |
| | | antisense | 36 | [5Phos][mUs][dCs][fU][mA][fC][mA][fU][mC][fA][mU][fUs][dT][mG][fU][mAs][dT][fUs][dC][fUs][dGs][dC] |

(continued)

| Duplex Code | Gene Target | Strand | ID | Sequence |
|---|---|---|---|---|
| DP2578P: DP2281G | CTNNB1 | sense | 37 | [C6Tn-GalNAc][C6Tn-GalNAc][C6Tn-GalNAc][C6Tn-GalNAc][iB][mC][mU][fG][mU][mU][fG][fG][fA][mU][mU][fG][fA][mU][mU][mC][fG][prgA][fA][fA][mUs][mU][iB] |
| | | antisense | 38 | [mUs][fUs][mUs][fC][mG][fA][mA][fU][mC][fA][mA][fU][mC][fC][mA][fA][mC][fA][mG][mUs][mU] |
| DP2579P: DP2281G | CTNNB1 | sense | 39 | [C6Tn-GalNAc][C6Tn-GalNAc][C6Tn-GalNAc][C6Tn-GalNAc][iB][mC][mU][fG][mU][mU][fG][fG][fA][mU][mU][fG][fA][mU][mU][mC][fG][prgA-2KPEG][fA][fA][mUs][mU][iB] |
| | | antisense | 40 | [mUs][fUs][mUs][fC][mG][fA][mA][fU][mC][fA][mA][fU][mC][fC][mA][fA][mC][fA][mG][mUs][mU] |
| DP2580P: DP2281G | CTNNB1 | sense | 41 | [C6Tn-GalNAc][C6Tn-GalNAc][C6Tn-GalNAc][C6Tn-GalNAc][iB][mC][mU][fG][mU][mU][fG][fG][fA][mU][mU][fG][fA][mU][mU][mC][fG][prgA-10KPEG][fA][fA][mUs][mU][iB] |
| | | antisense | 42 | [mUs][fUs][mUs][fC][mG][fA][mA][fU][mC][fA][mA][fU][mC][fC][mA][fA][mC][fA][mG][mUs][mU] |

(continued)

| Duplex Code | Gene Target | Strand | ID | Sequence |
|---|---|---|---|---|
| DP2525P: DP2362G | CTNNB1 | sense | 43 | [mAs][mG][fA][mA][fU][mA][fC][mA][fA][fA][fU][mG][fA][mU][fG][mU][fA][mG][fA][mA][mG][mC][mA][mG][mC][prgG-dPEG11-GalNAc][prgA-dPEG11-GalNAc][prgA-dPEG11-GalNAc][prgA-dPEG11-GalNAc][mG][mC][mU][mG][mC][mU] |
| | | antisense | 44 | [5Phos][mUs][dCs][fU][mA][fC][mA][fU][mC][fA][mU][fUs][dT][mG][fU][mAs][dT][fUs][dC][fUs][dGs][dC] |
| DP2525P: DP2469G | CTNNB1 | sense | 45 | [mAs][mG][fA][mA][fU][mA][fC][mA][fA][fA][fU][mG][fA][mU][fG][mU][fA][mG][fA][mA][mG][mC][mA][mG][mC][prgG-dPEG11-GalNAc][prgA-dPEG11-GalNAc][prgA-dPEG11-GalNAc][prgA-dPEG11-GalNAc][mG][mC][mU][mG][mC][mU] |
| | | antisense | 46 | [5Phos][mUs][fCs][mUs][fA][mC][fA][mU][fC][mA][fU][mU][fU][mG][fU][mA][fU][mU][fC][mUs][mGs][mC] |

(continued)

| Duplex Code | Gene Target | Strand | ID | Sequence |
|---|---|---|---|---|
| DP2525P: DP2470G | CTNNB1 | sense | 47 | [mAs][mG][fA][mA][fU][mA][fC][mA][fA][fA][fU][mG][fA][mU][fG][mU][fA][mG][fA][mA][mG][mC][mA][mG][mC][prgG-dPEG11-GalNAc][prgA-dPEG11-GalNAc][prgA-dPEG11-GalNAc][prgA-dPEG11-GalNAc][mG][mC][mU][mG][mC][mU] |
| | | antisense | 48 | [5Phos][mUs][fCs][mUs][fA][mC][fA][mU][fC][mA][fU][mU][fU][mG][fU][mA][fU][mU][fC][mUs][mUs][mU] |
| DP2526P: DP2470G | CTNNB1 | sense | 49 | [iB][mA][mG][fA][mA][fU][mA][fC][mA][fA][fA][fU][mG][fA][mU][fG][mU][fA][mG][fA][mA][mG][mC][mA][mG][mC][prgG-dPEG11-GalNAc][prgA-dPEG11-GalNAc][prgA-dPEG11-GalNAc][prgA-dPEG11-GalNAc][mG][mC][mU][mG][mC][mU] |
| | | antisense | 50 | [5Phos][mUs][fCs][mUs][fA][mC][fA][mU][fC][mA][fU][mU][fU][mG][fU][mA][fU][mU][fC][mUs][mUs][mU] |

(continued)

| Duplex Code | Gene Target | Strand | ID | Sequence |
|---|---|---|---|---|
| DP2526P: DP2472G | CTNNB1 | sense | 51 | [iB][mA][mG][fA][mA][fU][mA][fC][mA][fA][fA][fU][mG][fA][mU][fG][mU][fA][mG][fA][mA][mG][mC][mA][mG][mC][prgG-dPEG11-GalNAc][prgA-dPEG11-GalNAc][prgA-dPEG11-GalNAc][prgA-dPEG11-GalNAc][mG][mC][mU][mG][mC][mU] |
| | | antisense | 52 | [5Phos][mUs][fCs][mUs][fA][mC][fA][mU][fC][mA][fU][mU][fU][mG][fU][mA][fU][mU][fC][mU][mUs][mU] |
| DP2527P: DP2472G | CTNNB1 | sense | 53 | [iB][mA][mG][fA][fA][mU][fA][mC][fA][fA][fA][mU][fG][fA][mU][fG][mU][fA][fG][fA][mA][mG][mC][mA][mG][mC][prgG-dPEG11-GalNAc][prgA-dPEG11-GalNAc][prgA-dPEG11-GalNAc][prgA-dPEG11-GalNAc][mG][mC][mU][mG][mC][mU] |
| | | antisense | 54 | [5Phos][mUs][fCs][mUs][fA][mC][fA][mU][fC][mA][fU][mU][fU][mG][fU][mA][fU][mU][fC][mU][mUs][mU] |

(continued)

| Duplex Code | Gene Target | Strand | ID | Sequence |
|---|---|---|---|---|
| DP2600P: DP2281G | CTNNB1 | sense | 55 | [5TriGalNAc-PEG-C6amine][iB][mC][mU][fG][mU][mU][fG][fG][fA][mU][mU][fG][fA][mU][mU][mC][fG][fA][fA][fA][mUs][mU][iB] |
| | | antisense | 56 | [mUs][fUs][mUs][fC][mG][fA][mA][fU][mC][fA][mA][fU][mC][fC][mA][fA][mC][fA][mG][mUs][mU] |
| DP2421P: DP2460G | CTNNB1 | sense | 57 | [fAs][mG][fA][mA][mU][mA][fC][mA][fA][fA][fU][mG][fA][mU][fG][mU][mA][mG][fA][fA][rA][rC][fAs][dGs][dC] |
| | | antisense | 58 | [C6Tn-GalNAc][C6Tn-GalNAc][C6Tn-GalNAc][C6Tn-GalNAc][mGs][mC][mU][mG][rU][rU][rU][mC][mU][fA][mC][fA][mU][fC][mA][rU][rU][rU][fG][fU][mA][fU][mU][fC][mU][mGs][mC] |

(continued)

| Duplex Code | Gene Target | Strand | ID | Sequence |
|---|---|---|---|---|
| DP2421P: DP2461G | CTNNB1 | sense | 59 | [fAs][mG][fA][mA][mU][mA][fC][mA][fA][fA][fU][mG][fA][mU][fG][mU][mA][mG][fA][fA][rA][rC][fAs][dGs][dC] |
| | | antisense | 60 | [C6Tn-GalNAc][C6Tn-GalNAc][C6Tn-GalNAc][C6Tn-GalNAc][mU][fA][mG][mC][mU][fA][mU][mC][mG][dT][mGs][mC][mU][mG][rU][rU][rU][mC][mU][fA][mC][fA][mU][fC][mA][rU][rU][rU][fG][fU][mA][fU][mU][fC][mU][mGs][mC] |
| DP2422P: DP2462G | CTNNB1 | sense | 61 | [fAs][mG][fA][mA][mU][mA][fC][mA][fA][fA][fU][mG][fA][mU][fG][mU][mA][mG][fA][fA][fA][fC][fAs][dGs][dC] |
| | | antisense | 62 | [C6Tn-GalNAc][C6Tn-GalNAc][C6Tn-GalNAc][C6Tn-GalNAc] [mGs][mC][mU][mG][rU][rU][rU][mC][mU][fA][mC][fA][mU][fC][mA][rU][rU][rU][rG][fU][mA][fU][mU][fC][mU][mGs][mC] |
| DP2344P: DP2486G | CTNNB1 | sense | 63 | [iB][mC][mU][fG][mU][mU][fG][fG][fA][mU][mU][fG][fA][mU][mU][mC][fG][fA][fA][fA][mUs][mC][rU][rU][dGs][dC] |

(continued)

| Duplex Code | Gene Target | Strand | ID | Sequence |
|---|---|---|---|---|
| | | antisense | 64 | [C6Tn-GalNAc][C6Tn-GalNAc][C6Tn-GalNAc][C6Tn-GalNAc] [mGs][mC][mA][rA][rG][rA][mUs][fUs][mUs][fC][mG][fA][mA][fU][mC][fA][mA][fU][mC][fC][mA][fA][mC][fA][mG][mUs][mU] |
| DP2555P: DP2501G | HAO1 | sense | 65 | [mAs][mU][fA][mU][mU][mU][mU][fC][fC][fC][fA][fU][fC][mU][mG][mU][fA][mU][mU][fA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 66 | [5Phos][fUs][fAs][mAs][mU][mA][mC][fA][mG][mA][mU][fG][fG][fG][fA][fA][fA][mA][mU][fA][mU][mUs][mG] |

(continued)

| Duplex Code | Gene Target | Strand | ID | Sequence |
|---|---|---|---|---|
| DP2554P: DP2505G | HAO1 | sense | 67 | [mAs][mU][fA][mU][fU][mU][fU][fC][fC][fC][fA][fU][fC][mU][fG][mU][fA][mU][fU][mA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 68 | [5Phos][fUs][mAs][fAs][mU][fA][mC][fA][mG][fA][mU][fG][mG][fG][fA][fA][mA][fA][mU][fA][mU][mUs][mU] |
| DP2554P: DP2509G | HAO1 | sense | 69 | [mAs][mU][fA][mU][fU][mU][fU][fC][fC][fC][fA][fU][fC][mU][fG][mU][fA][mU][fU][mA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 70 | [5Phos][fUs][fAs][mAs][fU][mA][fC][fA][fG][mA][fU][fG][fG][fG][fA][fA][fA][mA][fU][fA][fU][mUs][mG] |

(continued)

| Duplex Code | Gene Target | Strand | ID | Sequence |
|---|---|---|---|---|
| DP2556P: DP2501G | HAO1 | sense | 71 | [iB][mA][mU][fA][mU][fU][mU][fU][mC][fC][fC][fA][mU][fC][mU][fG][mU][fA][mU][fU][mA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 72 | [5Phos][fUs][fAs][mAs][mU][mA][mC][fA][mG][mA][mU][fG][fG][fG][fA][fA][fA][mA][mU][fA][mU][mUs][mG] |
| DP2557P: DP2501G | HAO1 | sense | 73 | [iB][mA][mU][fA][mU][mU][mU][mU][mC][mC][mC][fA][mU][mC][mU][fG][mU][fA][mU][mU][fA][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 74 | [5Phos][fUs][fAs][mAs][mU][mA][mC][fA][mG][mA][mU][fG][fG][fG][fA][fA][fA][mA][mU][fA][mU][mUs][mG] |

(continued)

| Duplex Code | Gene Target | Strand | ID | Sequence |
|---|---|---|---|---|
| DP2553P: DP2507G | HAO1 | sense | 75 | [iB][mA][mU][fA][mU][mU][mU][mU][mC][mC][mC][fA][mU][mC][mU][fG][mU][fA][mU][mU][mG][mC][mA][mG][mC][mC][prgG-dPEG11-GalNAc][prgA-dPEG11-GalNAc][prgA-dPEG11-GalNAc][prgA-dPEG11-GalNAc][mG][mG][mC][mU][mG][mC] |
| | | antisense | 76 | [5Phos][mAs][fAs][mUs][fA][mC][fA][mG][fA][mU][fG][mG][fG][mA][fA][mA][fA][mU][fA][mU][mUs][mU] |
| DP2540P: DP2541G | HAO1 | sense | 77 | [C6Tn-GalNAc][C6Tn-GalNAc][C6Tn-GalNAc][C6Tn-GalNAc] [mUs][fAs][mGs][mCs][mUs][fAs][mUs][mCs][mGs][dTs][fAs][mU][fA][mU][mU][mU][fU][mC][fC][fC][fA][mU][fC][mU][fG][mU][mA][mU][fU][fA][mU][mU] |
| | | antisense | 78 | [5Phos][rU][rA][fA][mU][fA][mC][fA][mG][fA][mU][rG][rG][rG][fA][fA][mA][fA][mU][fA][mU][mU][mG] |

(continued)

| Duplex Code | Gene Target | Strand | ID | Sequence |
|---|---|---|---|---|
| DP2542P: DP2543G | HAO1 | sense | 79 | [fAs][mU][fA][mU][mU][mU][fU][mC][fC][fC][fA][mU][fC][mU][fG][mU][mA][mU][fU][fA][mU][mU][fU][fUs][dTs][dC] |
| | | antisense | 80 | [C6Tn-GalNAc][C6Tn-GalNAc][C6Tn-GalNAc][C6Tn-GalNAc][mUs][fAs][mGs][mCs][mUs][fAs][mUs][mCs][mGs][dTs][mG][mA][mA][mA][mA][rA][rU][rA][fA][mU][fA][mC][fA][mG][fA][mU][rG][rG][rG][fA][fA][mA][fA][mU][fA][mU][mUs][mG] |
| DP2544P: DP2545G | HAO1 | sense | 81 | [C6Tn-GalNAc][C6Tn-GalNAc][C6Tn-GalNAc][C6Tn-GalNAc][mUs][fAs][mGs][mCs][mUs][fAs][mUs][mCs][mGs][dTs][fAs][mU][fU][mG][mC][mU][fU][mU][fU][fG][fA][mC][fU][mU][fU][mU][mC][mA][fA][fUs][dGs][dG] |
| | | antisense | 82 | [5Phos][mA][mU][fU][mG][fA][mA][fA][mA][fG][mU][rC][rA][rA][fA][fA][mG][fC][mA][fA][mUs][dGs][dA] |

(continued)

| Duplex Code | Gene Target | Strand | ID | Sequence |
|---|---|---|---|---|
| DP2546P: DP2547G | HAO1 | sense | 83 | [C6Tn-GalNAc][C6Tn-GalNAc][C6Tn-GalNAc][C6Tn-GalNAc][mUs][fAs][mGs][mCs][mUs][fAs][mUs][mCs][mGs][dTs][fUs][mG][fG][mA][mA][mA][fU][mA][fU][fA][fU][mU][fA][mA][fC][mU][mG][mU][fU][fAs][dAs][dA] |
| | | antisense | 84 | [5Phos][mU][mA][fA][mC][fA][mG][fU][mU][fA][mA][rU][rA][rU][fA][fU][mU][fU][mC][fC][mAs][dGs][dG] |
| DP2546P: DP2548G | HAO1 | sense | 85 | [C6Tn-GalNAc][C6Tn-GalNAc][C6Tn-GalNAc][C6Tn-GalNAc][mUs][fAs][mGs][mCs][mUs][fAs][mUs][mCs][mGs][dTs][fUs][mG][fG][mA][mA][mA][fU][mA][fU][fA][fU][mU][fA][mA][fC][mU][mG][mU][fU][fAs][dAs][dA] |
| | | antisense | 86 | [5Phos][mUs][mAs][dAs][fC][mA][fG][mU][fU][mA][fA][mU][fAs][dT][fA][fU][mU][dT][fC][dC][fAs][dGs][dG] |

(continued)

| Duplex Code | Gene Target | Strand | ID | Sequence |
|---|---|---|---|---|
| DP2549P: DP2505G | HAO1 | sense | 87 | [C6Tn-GalNAc][C6Tn-GalNAc][C6Tn-GalNAc][C6Tn-GalNAc][mUs][fAs][mGs][mCs][mUs][fAs][mUs][mCs][mGs][dTs][iB][mA][mU][fA][mU][mU][mU][mU][mC][mC][mC][fA][mU][mC][mU][fG][mU][fA][mU][mU][fA][mUs][mU][iB] |
| | | antisense | 88 | [5Phos][fUs][mAs][fAs][mU][fA][mC][fA][mG][fA][mU][fG][mG][fG][fA][fA][mA][fA][mU][fA][mU][mUs][mU] |
| DP2482P: DP2281G | CTNNB1 | sense | 89 | [C6Tn-GalNAc][iB][C6Tn-GalNAc][iB][C6Tn-GalNAc][iB][C6Tn-GalNAc][iB][mC][mU][fG][mU][mU][fG][fG][fA][mU][mU][fG][fA][mU][mU][mC][fG][fA][fA][fA][mUs][mU][iB] |
| | | antisense | 90 | [mUs][fUs][mUs][fC][mG][fA][mA][fU][mC][fA][mA][fU][mC][fC][mA][fA][mC][fA][mG][mUs][mU] |

As used herein, m = 2'-O-methyl; f = 2'-fluoro; r = 2'-ribo; d = 2'-deoxy; "s" subscript = phosphorothioate; iB = inverted abasic; prg = 2'-propargyl.

| V | W | R,R2 | SP |
|---|---|---|---|
| O | O | H,H | 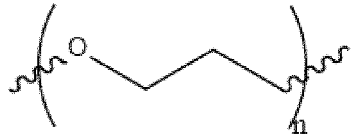 (n=3-12) |
| O | O | H,Me | 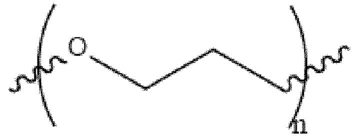 (n=3-12) |
| O | O | Me,Me | 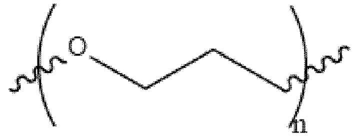 (n=3-12) |
| O | O | 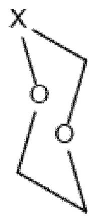 (X=O,C,NH,S) | 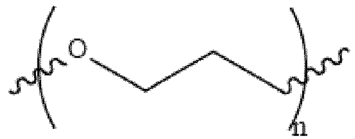 (n=3-12) |

(continued)

| V | W | R,R2 | SP |
|---|---|---|---|
| O | o | 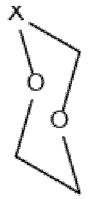 (X=O,C,NH,S) | 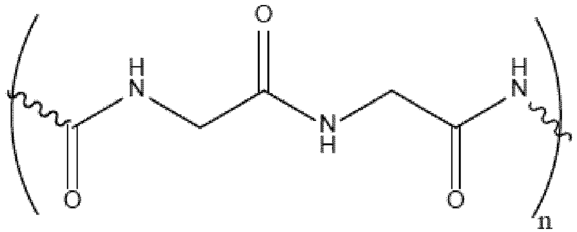 |
| O | N(R) | H,Me | 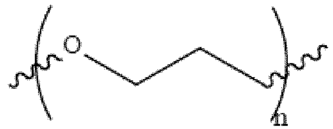 (n=3-12) |
| O | N(R) (R=H,Me) | 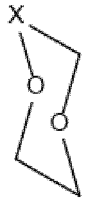 (X=O,C,NH,S) | 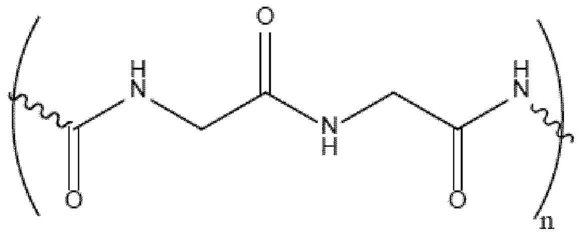 |
| O | O | 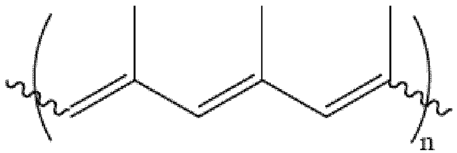 (n=1-3) | 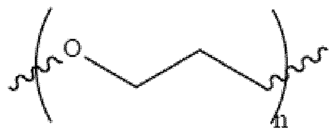 (n=3-12) |

(continued)

| V | W | R,R2 | SP |
|---|---|---|---|
| O | O | 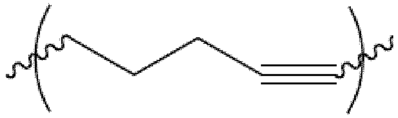 | 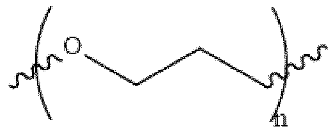 (n=3-12) |
| O | N(R) (R=H,Me) | 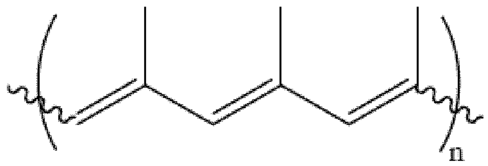 (n=1-3) | 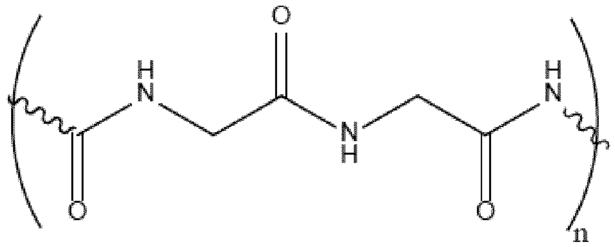 |
| O | 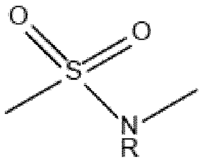 | H,H | 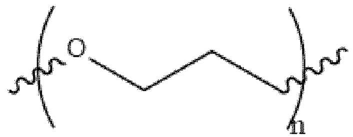 (n=3-12) |
| O | 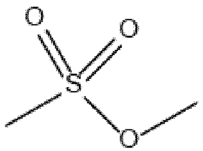 | H,H | 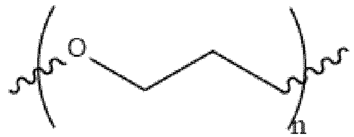 (n=3-12) |
| O | 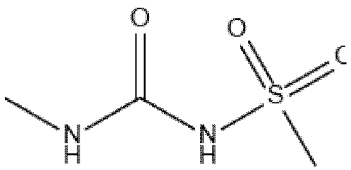 | H,H | 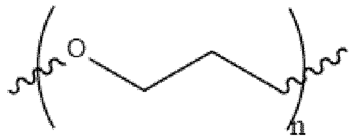 (n=3-12) |

(continued)

| V | W | R,R2 | SP |
|---|---|---|---|
| O | 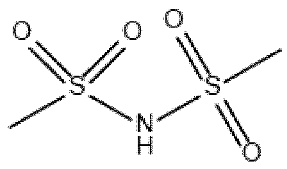 | H,H | 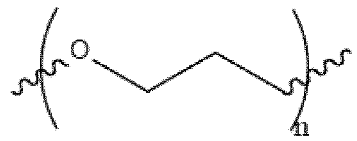 (n=3-12) |

| | | | |
|---|---|---|---|
| O | 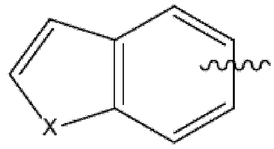 (X=C,N,O,S) | H,H | 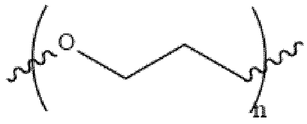 (n=3-12) |
| O | 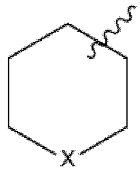 (X=C,N,O,S) | H,H | 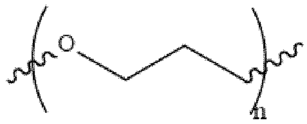 (n=3-12) |

Table 4 depicts potential linker group combinations of various components shown in the formula below

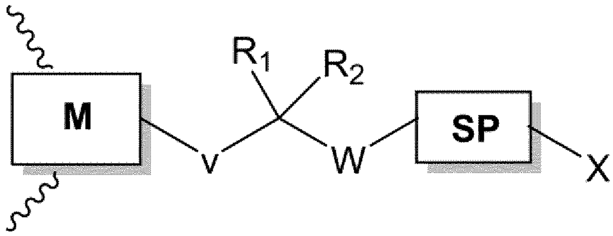

| | | | | | | | | |
|---|---|---|---|---|---|---|---|---|
| 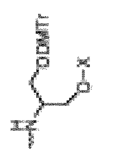 | 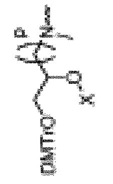 | 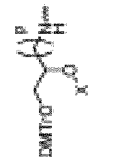 | 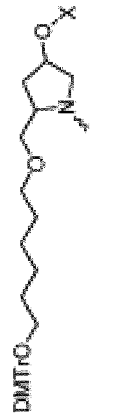 | 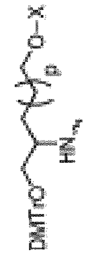 | 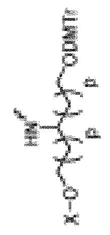 | 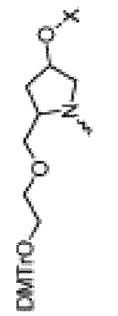 | 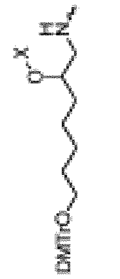 | 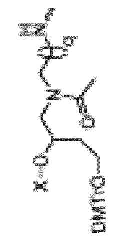 |
| 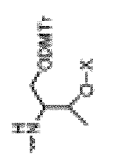 | 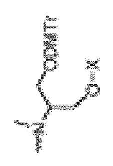 | 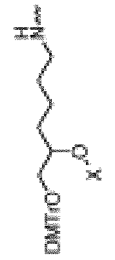 | 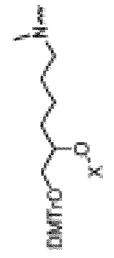 | 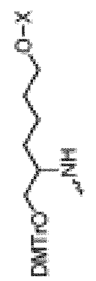 | 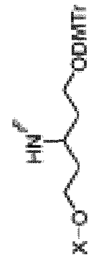 | 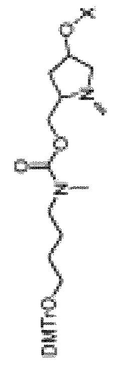 | 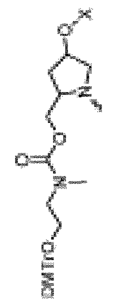 | 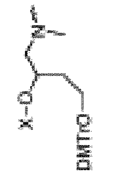 |
| 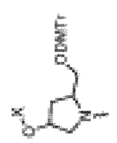 | 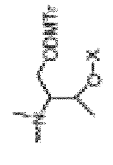 | 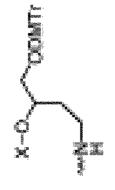 | 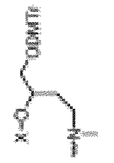 | 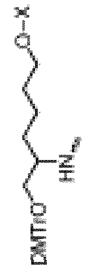 | 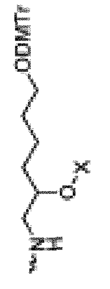 | 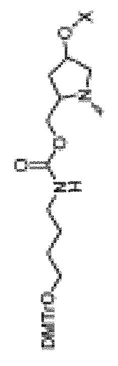 | 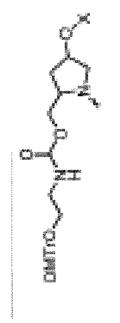 | 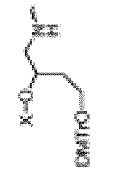 |

(continued)

| | | |
|---|---|---|
| 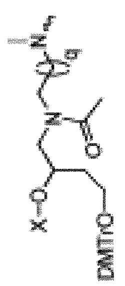 | 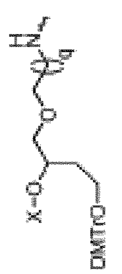 | 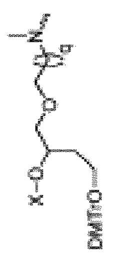 |
| 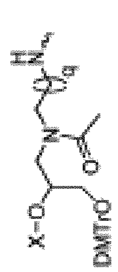 | 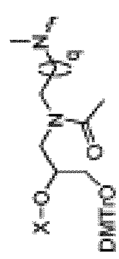 | 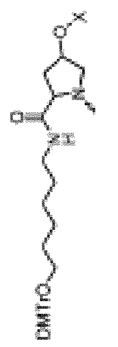 |
| 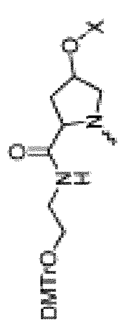 | 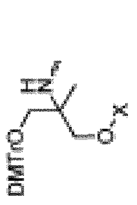 | 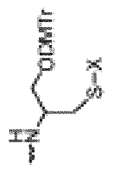 |
| 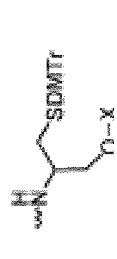 | 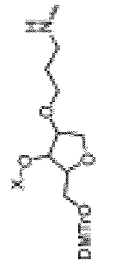 | 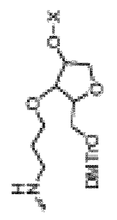 |
| 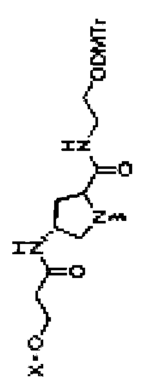 | 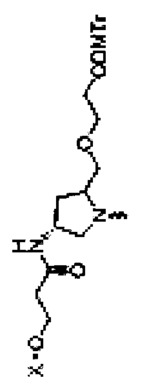 | 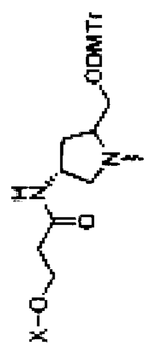 |
| 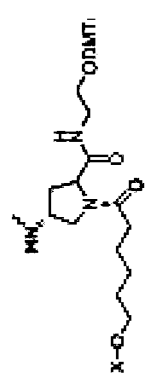 | 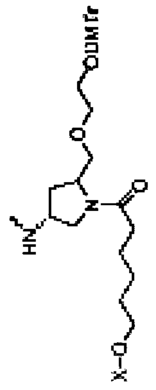 | 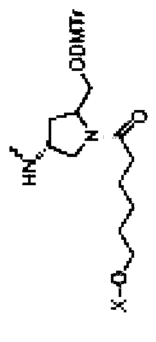 |
| 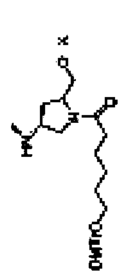 | 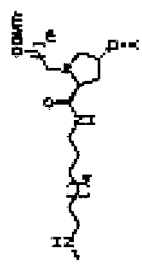 | 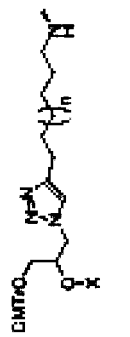 |

(continued)

| | | |
|---|---|---|
| 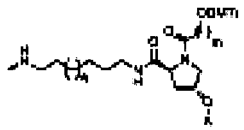 | 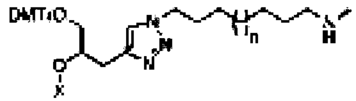 | 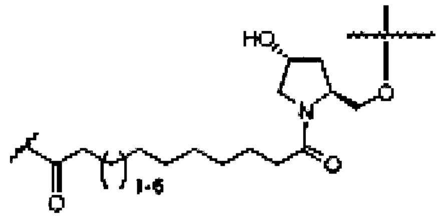<br>The top right squiggly line is the point of attachement to the oligonucleotide or other biologically active agent. The bottom left squiggly link is the point of attachment for the Ligand. |
| | 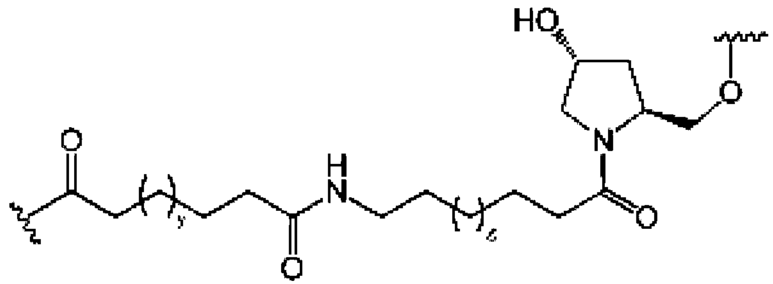<br>where y is 1-20 and z is 1-20 | |

[a] indicates the site of attachment of the Ligand.

[b] Each structure represents chirally pure or racemic isomers when one or more asymmetric centers are present.

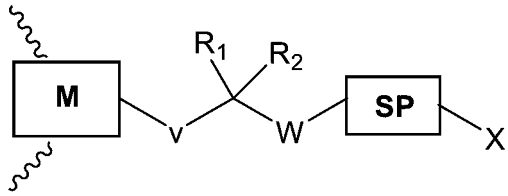

Table 4 depicts potential linker group combinations of various components

## Claims

1. A double stranded nucleic acid (dsNA) comprising:

   a sense strand comprising 21 to 83 nucleotides;
   an antisense strand comprising 15 to 39 nucleotides;
   a duplex formed by said sense and antisense strand, having a length of 15 to 35 base pairs;
   wherein the dsNA comprises a discontinuity between the 5' terminus of the sense strand and the 3' terminus of the antisense strand or between the 3' terminus of the sense strand and the 5' terminus of the antisense strand;
   wherein the sense strand comprises a tetraloop and the tetraloop comprises at least one ligand conjugated nucleotide, wherein the ligand is GalNAc; and
   wherein said antisense strand is complementary to a target mRNA along at least 15 nucleotides of said antisense strand and said double stranded nucleic acid reduces target gene expression when said double stranded nucleic acid is introduced into a mammal or a mammalian cell, and preferably wherein the tetraloop comprises one, two, three or four ligand conjugated nucleotide and each of the ligands is GalNAc.

2. The dsNA of claim 1 or claim 2, wherein at least one of the ligands is conjugated to a nucleotide of the tetraloop through the 2' hydroxyl on the ribose of the nucleotide; preferably wherein each ligand is conjugated to a nucleotide of the tetraloop through the 2' hydroxyl on the ribose of the nucleotide.

3. The dsNA of any preceding claim, wherein the tetraloop comprises three ligand conjugated nucleotides and each of the ligands is GalNAc.

4. The dsNA of any of claims 1 to 3, wherein the tetraloop comprises four ligand conjugated nucleotides and each of the ligands is GalNAc.

5. The dsNA of any preceding claim, wherein said antisense strand has a length range of: 15-30 nucleotides, 18-25 nucleotides or 19-24 nucleotides.

6. The dsNA of claim 1, wherein said duplex has a length range of: 15-22 base pairs or 15-30 base pairs.

7. The dsNA of any preceding claim, wherein said dsNA comprises at least one modified nucleotide; preferably wherein the modified nucleotide comprises a sugar modification selected from the group consisting of: 2'-*O*-methyl, 2'-methoxyethoxy, 2'-fluoro, 2'-allyl, 2'-*O*-[2-(methylamino)-2-oxoethyl], 4'-thio, 4'-$CH_2$-*O*-2'- bridge, 4'-$(CH_2)_2$-*O*-2'-bridge, LNA, 2'-amino, and 2'-*O*-(N-methylcarbamate) modification.

8. The dsNA of claim 1, comprising a backbone modification selected from the group consisting of: phosphonate, phosphorothioate, phosphotriester, methylphosphonate, morpholino, SATE (S-acyl-2-thioethyl) modified phosphate, and BMEG (Isobutyryl Mercapto Ethyl Glycol) modified phosphate.

9. The dsNA of claim 1, comprising a region containing at least one phosphorothioate linkage; and/or wherein both sense and antisense strands consist of up to 100% chemically modified nucleotides.

10. The dsNA of any preceding claim, wherein the sense strand has a length between 35 and 55 nucleotides.

11. The dsNA of any preceding claim, wherein the antisense strand has a length range of 19-24 nucleotides.

**12.** The dsNA of any preceding claim, wherein the duplex formed by the sense and antisense strand has a length range of 15-22 base pairs.

**13.** The dsNA of any preceding claim, wherein the 3' end of the antisense strand overhangs the 5' end of the sense strand and comprises a 3' extension, wherein the 3' extension is 1-4 nucleotides, optionally 2 nucleotides.

**14.** The dsNA of any preceding claim, wherein the discontinuity is between the 3' terminus of the sense strand and the 5' terminus of the antisense strand.

**15.** The dsNA of any preceding claim, wherein each GalNAc ligand is conjugated to the sugar of a nucleotide of the tetraloop via a linker as set forth in Formula VII:

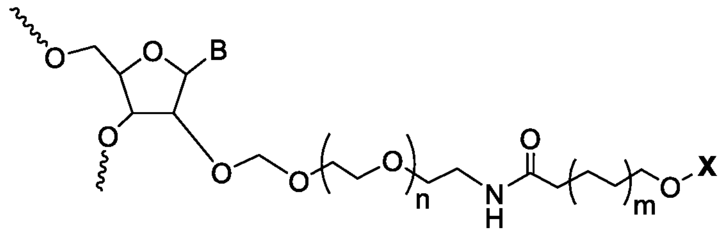

Formula VII

wherein:

B is a nucleobase;
m and n are each independently 1 - 20; and
X has the structure

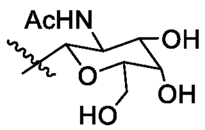

;

or
wherein each GalNAc ligand is conjugated to a nucleotide of the tetraloop via a linker selected from the group consisting of:

a)

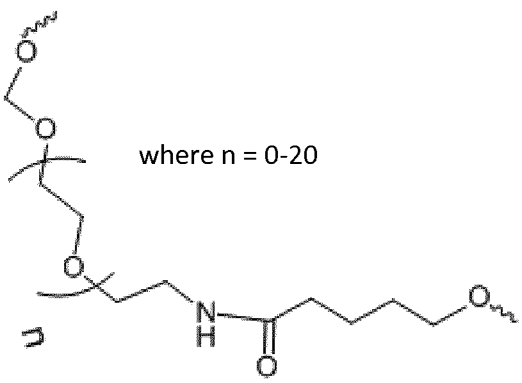

where n = 0-20

b)

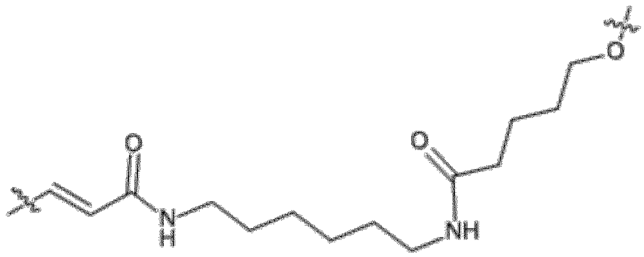

;

c)

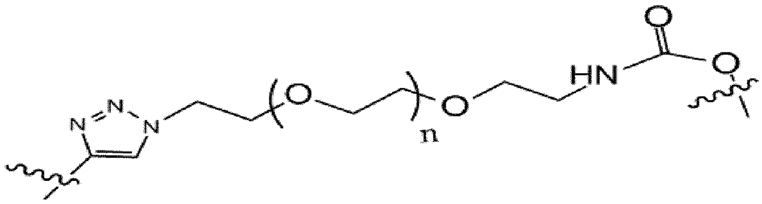

where n = 0-20

;

and

d)

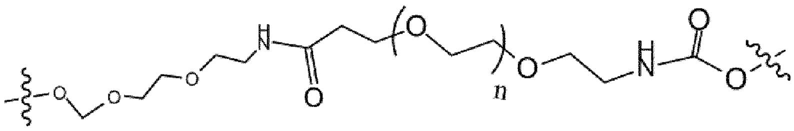

where n = 0-20

wherein said ligand is conjugated to the sugar or base of said nucleotide; and wherein said ligand-conjugated nucleotide is used for synthesizing the dsNA of any preceding claim.

**16.** The dsNA of any one of claims 1-15, wherein each GalNAc ligand is conjugated to a nucleotide of the tetraloop via an acetal linker.

**17.** The dsNA of claim 1, wherein:

the sense strand consists of 36 nucleotides,
the antisense consists of 22 nucleotides, wherein the 3' end has a single stranded overhang consisting of two nucleotides,
the duplex formed by the sense and antisense strand consists of 20 base pairs,
the discontinuity is between the 3' terminus of the sense strand and the 5' terminus of the antisense strand, and the tetraloop consists of the nucleotide sequence GAAA, wherein the 2' position of the sugar of each nucleotide of the tetraloop is conjugated to GalNAc via a linker, wherein the linker has the structure

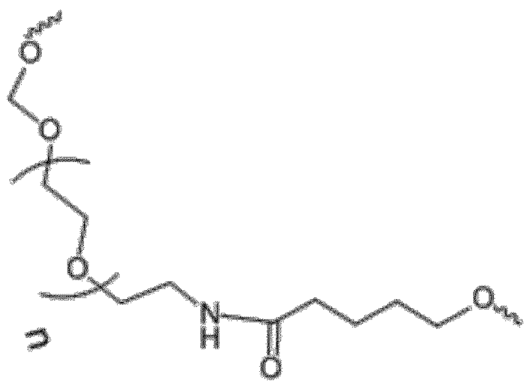

,

wherein n is 1 to 8.

**Patentansprüche**

1. Doppelsträngige Nukleinsäure (dsNA), die folgendes umfasst:

   einen Sense-Strang, der 21 bis 83 Nukleotide umfasst;
   einen Antisense-Strang, der 15 bis 39 Nukleotide umfasst;
   einen Duplex, der aus dem Sense- und Antisense-Strang gebildet wird, mit einer Länge von 15 bis 35 Basenpaaren;
   wobei die dsNA eine Unterbrechung zwischen dem 5'-Ende des Sense-Strangs und dem 3'-Ende des Antisense-Strangs oder zwischen dem 3'-Ende des Sense-Strangs und dem 5'-Ende des Antisense-Strangs umfasst;
   wobei der Sense-Strang einen Tetraloop umfasst, und wobei der Tetraloop mindestens ein mit Ligand konjugiertes Nukleotid umfasst, wobei der Ligand GalNAc ist; und
   wobei der Antisense-Strang komplementär ist zu einer Ziel-mRNA entlang mindestens 15 Nukleotiden des Antisense-Strangs, und wobei die doppelsträngige Nukleinsäure die Zielgenexpression reduziert, wenn die doppelsträngige Nukleinsäure in ein Säugetier oder eine Säugetierzelle eingeführt wird, und wobei der Tetraloop vorzugsweise ein mit ein, zwei, drei oder vier Liganden konjugiertes Nukleotid umfasst, und wobei jeder der Liganden GalNAc ist.

2. dsNA nach Anspruch 1 oder 2, wobei mindestens einer der Liganden durch das 2'-Hydroxyl an der Ribose des Nukleotids an ein Nukleotid des Tetraloop konjugiert ist;
   wobei vorzugsweise jeder Ligand durch das 2'-Hydroxyl an der Ribose des Nukleotids an ein Nukleotid des Tetraloop konjugiert ist.

3. dsNA nach einem der vorstehenden Ansprüche, wobei der Tetraloop drei mit Ligand konjugierte Nukleotide umfasst, und wobei jeder der Liganden GalNAc ist.

4. dsNA nach einem der Ansprüche 1 bis 3, wobei der Tetraloop vier mit Ligand konjugierte Nukleotide umfasst, und wobei jeder der Liganden GalNAc ist.

5. dsNA nach einem der vorstehenden Ansprüche, wobei der Antisense-Strang einen Längenbereich von 15-30 Nukleotiden, 18-25 Nukleotiden oder 19-24 Nukleotiden aufweist.

6. dsNA nach Anspruch 1, wobei der Duplex einen Längenbereich von 15-22 Basenpaaren oder 15-30 Basenpaaren aufweist.

7. dsNA nach einem der vorstehenden Ansprüche, wobei die dsNA mindestens ein modifiziertes Nukleotid aufweist; wobei das modifizierte Nukleotid vorzugsweise eine Zuckermodifikation umfasst, die ausgewählt ist aus der Gruppe bestehend aus: 2'-*O*-Methyl, 2'-Methoxyethoxy, 2'-Fluor, 2'-Allyl, 2'-*O*[2-(Methylamino)-2-oxoethyl], 4'-Thio, 4'-$CH_2$,*O*-2'-Brücke, 4'-$(CH_2)_2$-*O*-2'-Brücke, LNA, 2'-Amino und 2'-*O*-(N-Methylcarbamat-Modifikation.

8. dsNA nach Anspruch 1, die eine Rückgratmodifikation umfasst, die ausgewählt ist aus der Gruppe bestehend aus:

Phosphonat, Phosphorthioat, Phosphotriester, Methylphosphonat, Morpholino, SAT (S-Acyl-2-thioethyl) modifiziertem Phosphat und BMEG (Isobutyryl Mercapto Ethylglykol) modifiziertem Phosphat.

**9.** dsNA nach Anspruch 1, welche eine Region umfasst, die mindestens eine Phosphorthiotatverknüpfung umfasst; und/oder wobei sowohl der Sense- als auch der Antisense-Strang aus bis zu 100% chemisch modifizierten Nukleotiden bestehen.

**10.** dsNA nach einem der vorstehenden Ansprüche, wobei der Sense-Strang eine Länge zwischen 35 und 55 Nukleotiden aufweist.

**11.** dsNA nach einem der vorstehenden Ansprüche, wobei der Antisense-Strang eine Länge zwischen 19 und 24 Nukleotiden aufweist.

**12.** dsNA nach einem der vorstehenden Ansprüche, wobei der durch den Sense-Strang und den Antisense-Strang gebildete Duplex einen Längenbereich zwischen 15 und 22 Basenpaaren aufweist.

**13.** dsNA nach einem der vorstehenden Ansprüche, wobei das 3'-Ende des Antisense-Strangs einen Überhang des 5'-Endes des Sense-Strangs bildet und eine 3'-Erweiterung umfasst, wobei die 3'-Erweiterung 1 bis 4 Nukleotiden entspricht, optional 2 Nukleotiden.

**14.** dsNA nach einem der vorstehenden Ansprüche, wobei sich die Unterbrechung zwischen dem 3'-Ende des Sense-Strangs und dem 5'-Ende des Antisense-Strangs befindet.

**15.** dsNA nach einem der vorstehenden Ansprüche, wobei jeder GalNAc-Ligand über einen Linker an den Zucker eines Nukleotids konjugiert ist, wie dies in Formel VII ausgeführt ist:

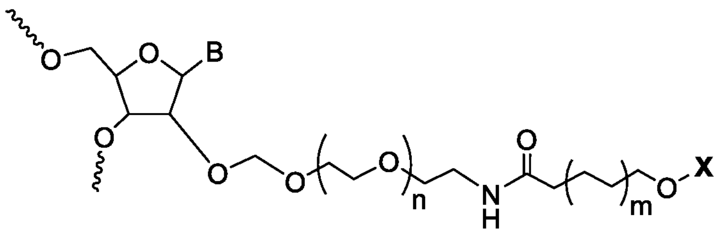

Formel VII

wobei:

B eine Nukleobase ist;
m und n jeweils unabhängig 1 bis 20 sind; und
X die folgende Struktur aufweist:

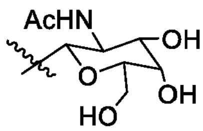

;

oder
wobei jeder GalNAc-Ligand an ein Nukleotid des Tetraloop konjugiert ist über einen Linker, ausgewählt aus der Gruppe bestehend aus:

a)

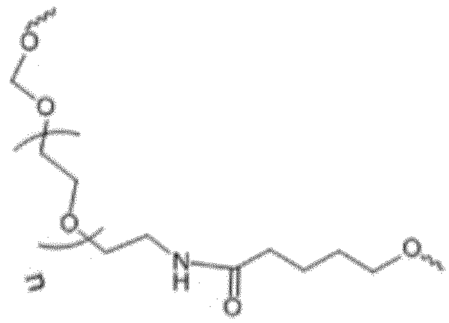

mit n = 0-20;
b)

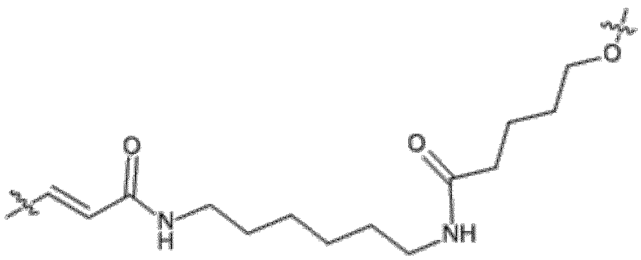

;

c)

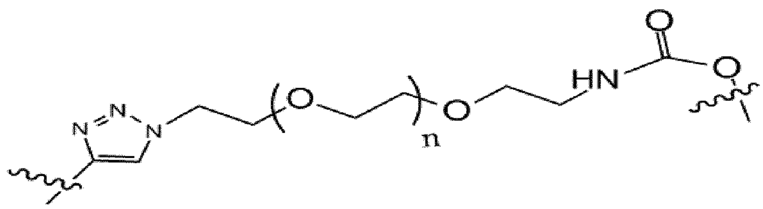

mit n = 0-20; und
d)

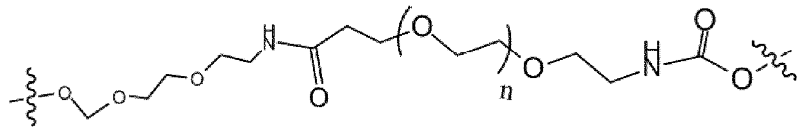

mit n = 0-20;

wobei der Ligand an den Zucker oder die Base des Nukleotids konjugiert ist; und
wobei das mit Ligand konjugierte Nukleotid verwendet wird zum Synthetisieren der dsNA nach einem der vorstehenden Ansprüche.

**16.** dsNA nach einem der Ansprüche 1 bis 15, wobei jeder GalNAc-Ligand über einen Acetal-Linker an ein Nukleotid des Tetraloop konjugiert ist.

**17.** dsNA nach Anspruch 1, wobei:

der Sense-Strang aus 36 Nukleotiden besteht;
der Antisense-Strang aus 22 Nukleotiden besteht, wobei das 3'-Ende eine einsträngigen Überhang aufweist,

der aus zwei Nukleotiden besteht;
der durch den Sense-Strang und den Antisense-Strang gebildete Duplex aus 20 Basenpaaren besteht;
sich die Unterbrechung zwischen dem 3'-Ende des Sense-Strangs und dem 5'-Ende des Antisense-Strangs befindet; und
der Tetraloop aus der Nukleotidsequenz GAAA besteht, wobei die 2'-Position des Zuckers jedes Nukleotides des Tetraloops über einen Linker an GalNAc konjugiert ist, wobei der Linker die folgende Struktur aufweist:

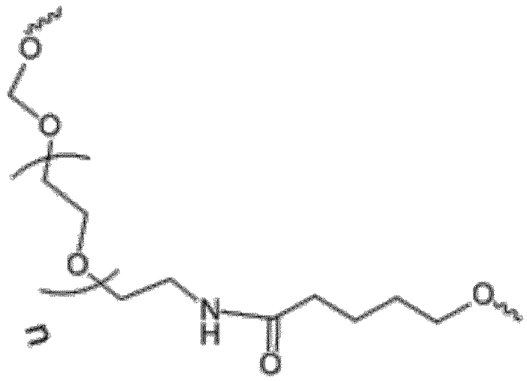
,

wobei n 1 bis 8 ist.

## Revendications

1. Acide nucléique double brin (ANdb) comprenant :

un brin sens comprenant de 21 à 83 nucléotides ;
un brin antisens comprenant 15 à 39 nucléotides ;
un duplex formé par lesdits brins sens et antisens, ayant une longueur de 15 à 35 paires de bases ;
l'ANdb comprenant une discontinuité entre l'extrémité 5' du brin sens et l'extrémité 3' du brin antisens ou entre l'extrémité 3' du brin sens et l'extrémité 5' du brin antisens ;
le brin sens comprenant un tétraloop et le tétraloop comprenant au moins un nucléotide conjugué à un ligand, le ligand étant le GalNAc ; et
ledit brin antisens étant complémentaire d'un ARNm cible le long d'au moins 15 nucléotides dudit brin antisens et ledit acide nucléique double brin réduisant l'expression génique cible lorsque ledit acide nucléique double brin est introduit dans un mammifère ou une cellule de mammifère, et de préférence le tétraloop comprenant un, deux, trois ou quatre nucléotides conjugués à un ligand et chaque ligand étant GalNAc.

2. ANdb selon la revendication 1 ou 2, au moins l'un des ligands étant conjugué à un nucléotide du tétraloop par l'intermédiaire de l'hydroxyle 2' sur le ribose du nucléotide, de préférence chaque ligand étant conjugué à un nucléotide du tétraloop par l'intermédiaire de l'hydroxyle 2' sur le ribose du nucléotide.

3. ANdb selon l'une quelconque des revendications précédentes, le tétraloop comprenant trois nucléotides conjugués à un ligand et chacun des ligands étant GalNAc.

4. ANdb selon l'une quelconque des revendications 1 à 3, le tétraloop comprenant quatre nucléotides conjugués à un ligand et chacun des ligands étant GalNAc.

5. ANdb selon l'une quelconque des revendications précédentes, ledit brin antisens ayant une plage de longueur de : 15 à 30 nucléotides, 18 à 25 nucléotides ou 19 à 24 nucléotides.

6. ANdb selon la revendication 1, ledit duplex ayant une plage de longueur de : 15 à 22 paires de bases ou 15 à 30 paires de bases.

7. ANdb selon l'une quelconque des revendications précédentes, ledit ANdb comprenant au moins un nucléotide

modifié ; de préférence le nucléotide modifié comprenant une modification de sucre choisie dans le groupe constitué par : modification 2'-*O*-méthyl, 2'-méthoxyéthoxy, 2'-fluoro, 2'-allyle, 2'-*O*-[2-(méthylamino)-2-oxoéthyl], 4'-thio, pont 4'-$CH_2$-*O*-2'-, pont 4'-$(CH_2)_2$-*O*-2'-, LNA, 2'-amino et 2'-*O*-(N-methylcarbamate).

**8.** ANdb selon la revendication 1, comprenant une modification du squelette choisie dans le groupe constitué par : phosphonate, phosphorothioate, phosphotriester, méthylphosphonate, morpholino, phosphate modifié SATE (S-acyl-2-thioéthyl) et phosphate modifié BMEG (Isobutyryl Mercapto Ethyl Glycol).

**9.** ANdb selon la revendication 1, comprenant une région contenant au moins une liaison phosphorothioate ; et/ou les brins sense et antisens comprenant jusqu'à 100 % de nucléotides modifiés chimiquement.

**10.** ANdb selon l'une quelconque des revendications précédentes, le brin sens ayant une longueur comprise entre 35 et 55 nucléotides.

**11.** ANdb selon l'une quelconque des revendications précédentes, le brin antisens ayant une longueur comprise entre 19 et 24 nucléotides.

**12.** ANdb selon l'une quelconque des revendications précédentes, le duplex formé par le brin sens et le brin antisens ayant une longueur comprise entre 15 et 22 paires de bases.

**13.** ANdb selon l'une quelconque des revendications précédentes, l'extrémité 3' du brin antisens surplombant l'extrémité 5' du brin sens et comprenant une extension 3', l'extension 3' étant constituée de 1 à 4 nucléotides, éventuellement de 2 nucléotides.

**14.** ANdb selon l'une quelconque des revendications précédentes, la discontinuité étant entre l'extrémité 3' du brin sens et l'extrémité 5' du brin antisens.

**15.** ANdb selon l'une quelconque des revendications précédentes, chaque ligand GalNAc étant conjugué au sucre d'un nucléotide du tétraloop par l'intermédiaire d'un agent de liaison comme indiqué dans la formule VII :

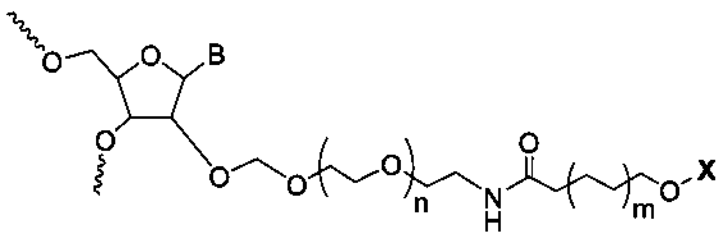

Formule VII

B étant une nucléobase ;
m et n éant chacun indépendamment 1 à 20 ; et
X ayant la structure

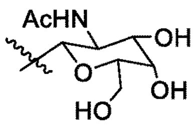

;

ou
chaque ligand GalNAc étant conjugué à un nucléotide du tétraloop par l'intermédiaire d'un lieur choisi dans le groupe constitué par :

a) ;

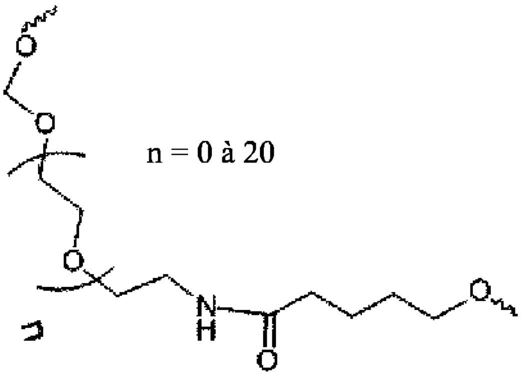

n = 0 à 20

b)

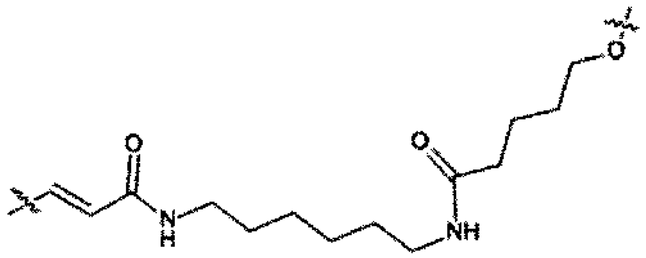

;

c)

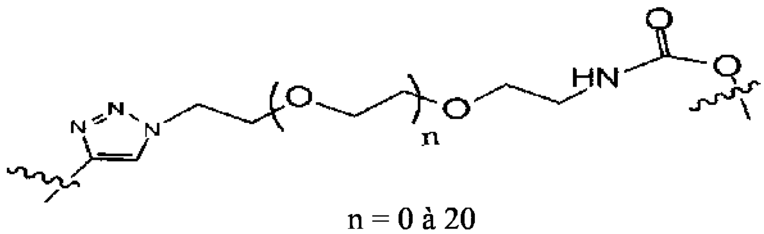

n = 0 à 20

d)

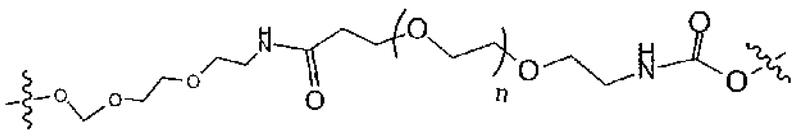

n = 0 à 20

ledit ligand étant conjugué au sucre ou à la base dudit nucléotide ; et
ledit nucléotide conjugué au ligand étant utilisé pour synthétiser l'ANdb selon l'une quelconque des revendications précédentes.

**16.** ANdb selon l'une quelconque des revendications 1 à 15, chaque ligand GalNAc étant conjugué à un nucléotide du tétraloop par l'intermédiaire d'un lieur acétal.

**17.** ANdb selon la revendication 1,
le brin sense étant composé de 36 nucléotides,
l'antisens étant constitué de 22 nucléotides, l'extrémité 3' ayant un surplomb simple brin constitué de deux nucléotides,
le duplex formé par les brins sens et antisens étant constitué de 20 paires de bases,
la discontinuité étant entre l'extrémité 3' du brin sens et l'extrémité 5' du brin antisens, et
le tétraloop étant constitué par la séquence nucléotidique GAAA, la position 2' du sucre de chaque nucléotide du tétraloop étant conjuguée à GalNAc par l'intermédiaire d'un lieur, le lieur ayant la structure suivante

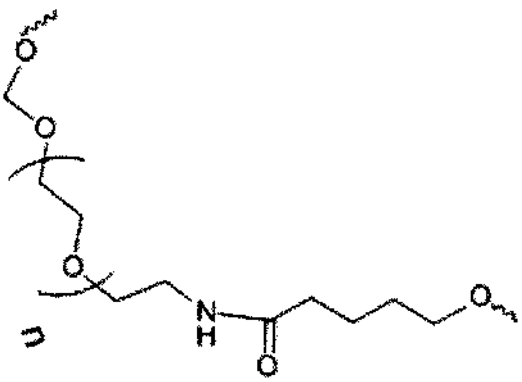

,

n étant compris entre 1 et 8.

Figure 1A. DsiRNA with ssDNA/RNA strand extensions

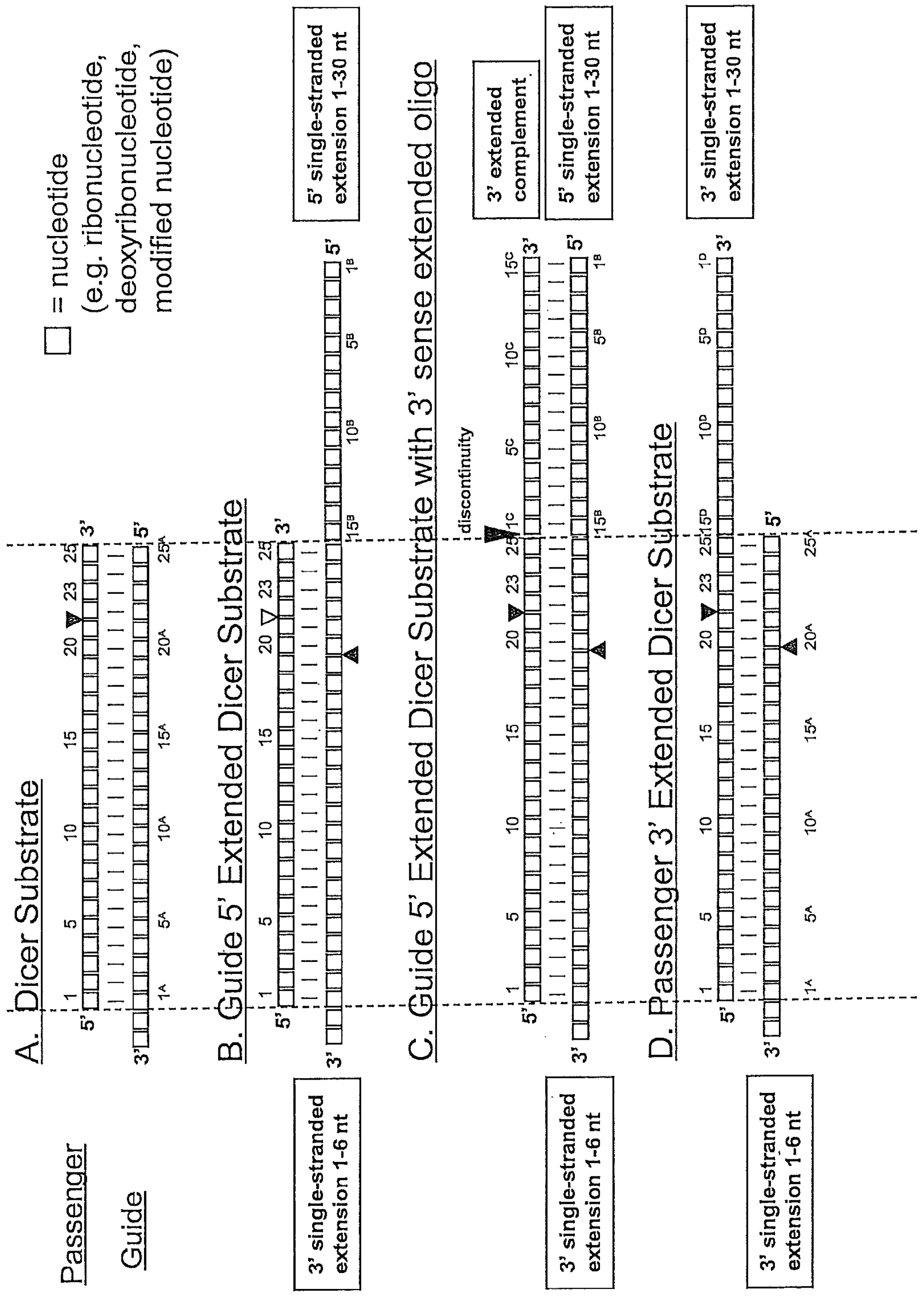

Figure 1A. (continued) DsiRNA with ssDNA/RNA strand extensions

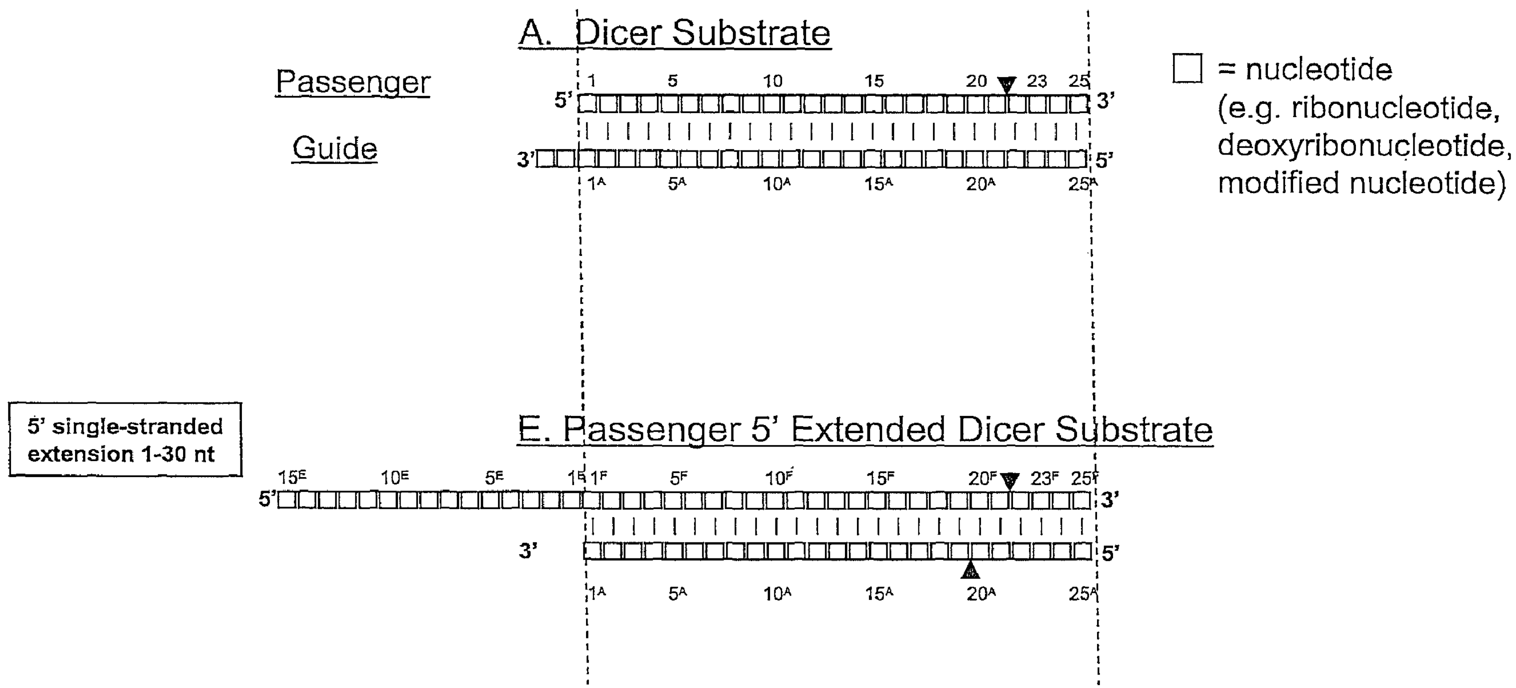

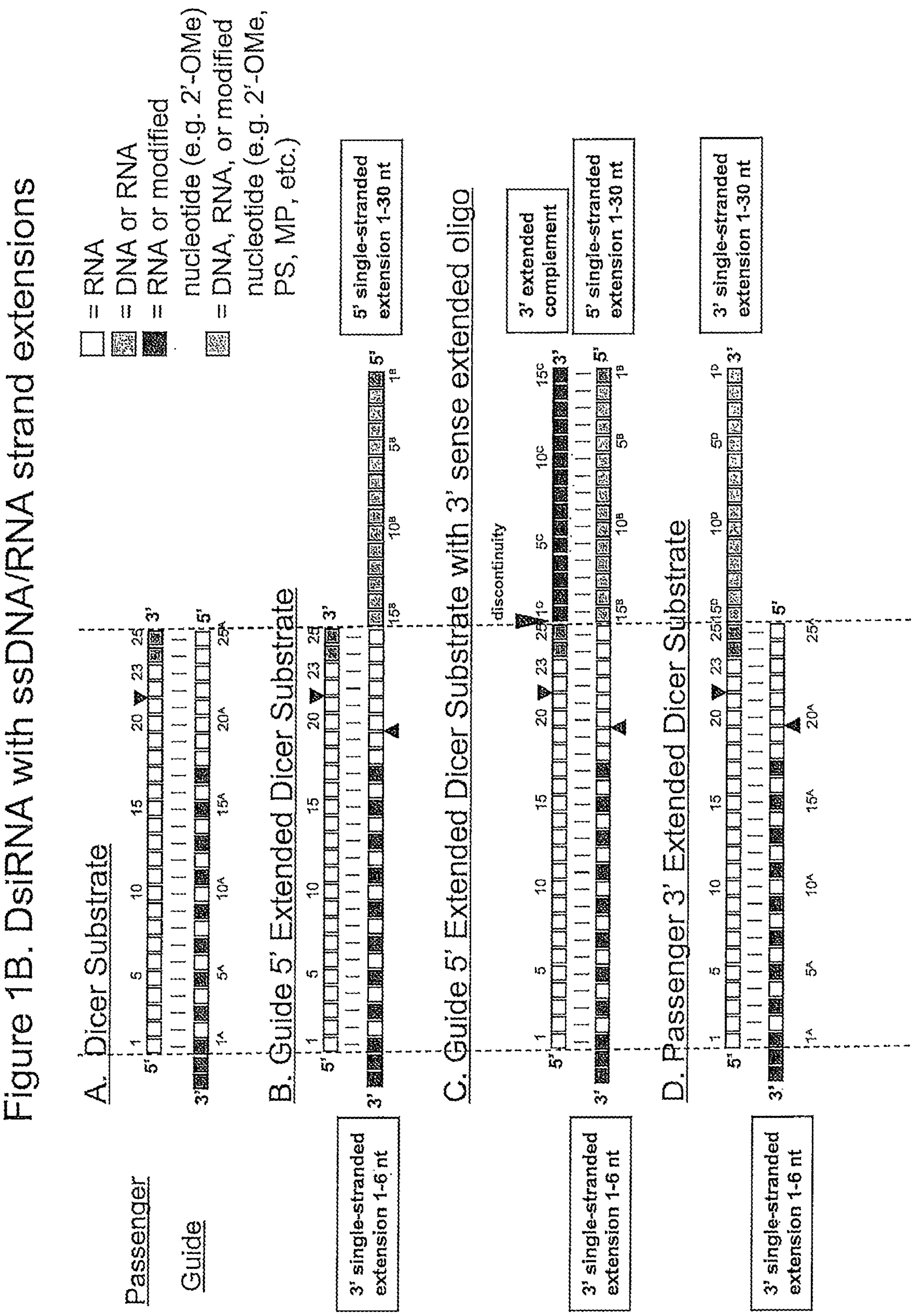
Figure 1B. DsiRNA with ssDNA/RNA strand extensions
Passenger
Guide
A. Dicer Substrate
B. Guide 5' Extended Dicer Substrate
C. Guide 5' Extended Dicer Substrate with 3' sense extended oligo
D. Passenger 3' Extended Dicer Substrate
= RNA
= DNA or RNA
= RNA or modified nucleotide (e.g. 2'-OMe)
= DNA, RNA, or modified nucleotide (e.g. 2'-OMe, PS, MP, etc.)
3' single-stranded extension 1-6 nt
5' single-stranded extension 1-30 nt
3' extended complement
3' single-stranded extension 1-30 nt
discontinuity

Figure 2. DsiRNA with ssDNA/RNA guide strand extensions

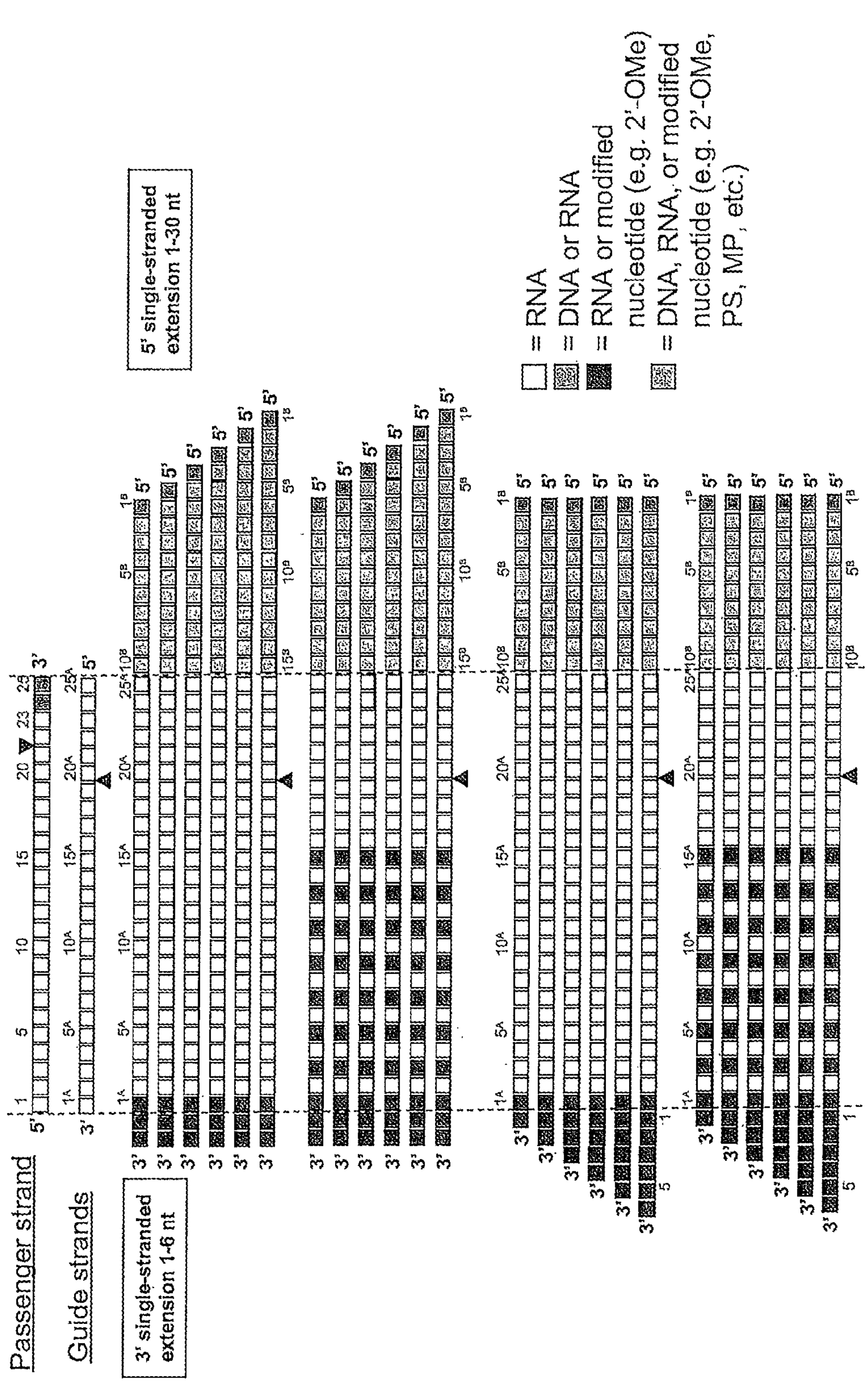

# Figure 3. DsiRNA with ssDNA/RNA passenger strand extensions

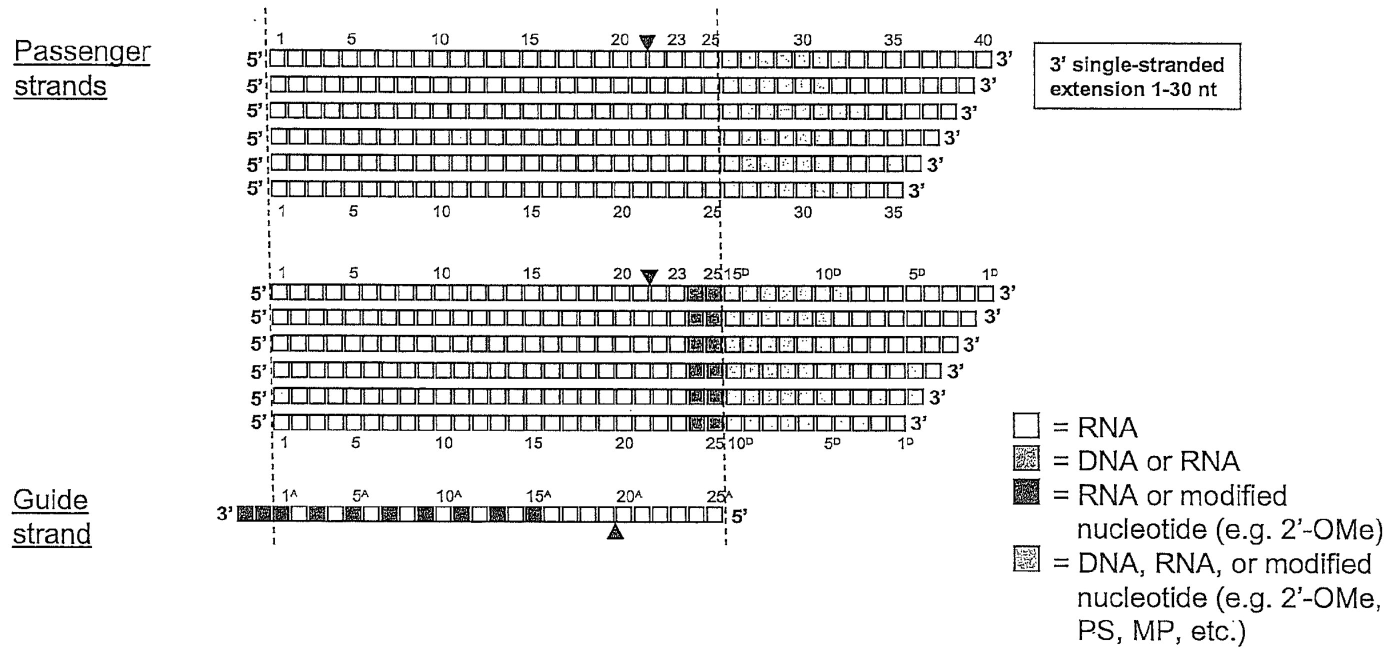

Figure 4. DsiRNA with ssDNA/RNA guide extensions

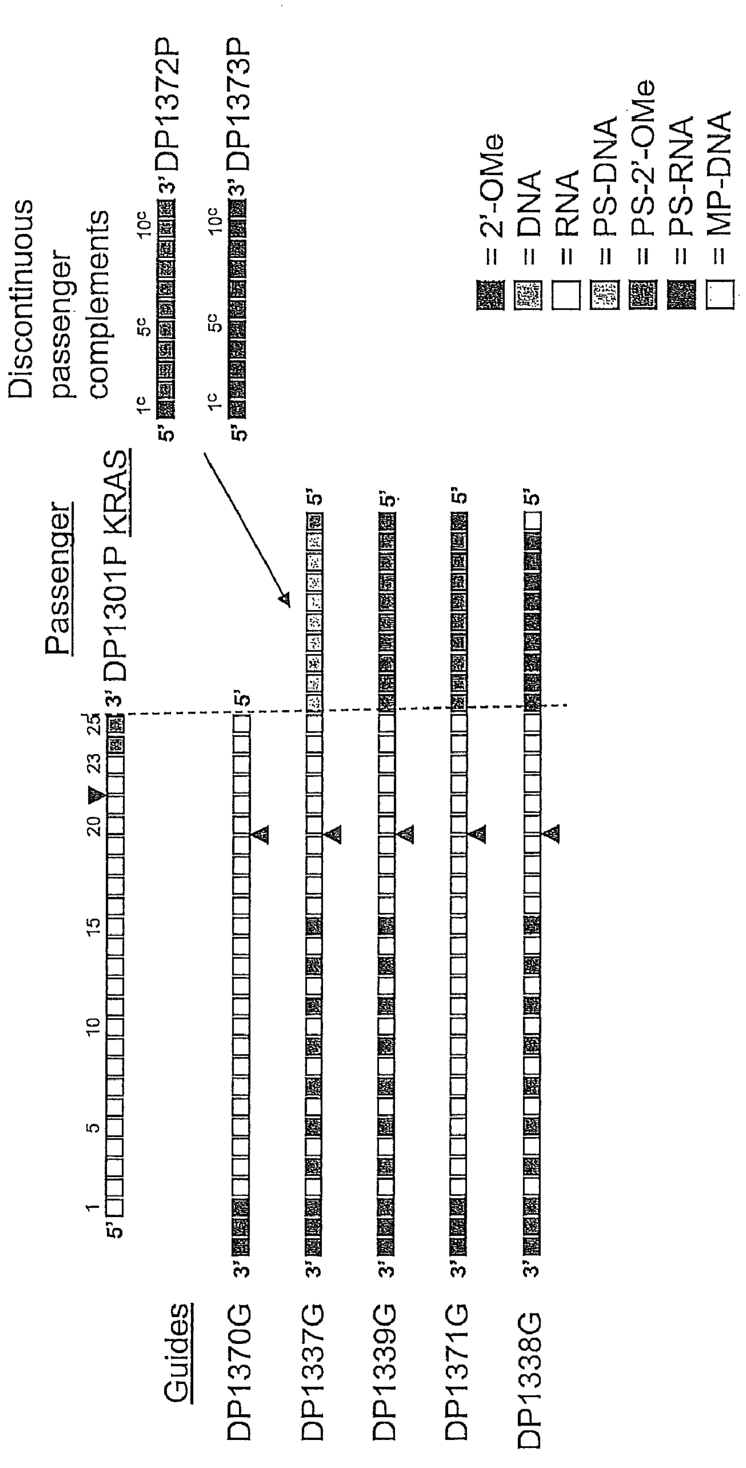

Figure 5. DsiRNA with ssDNA/RNA passenger strand extensions

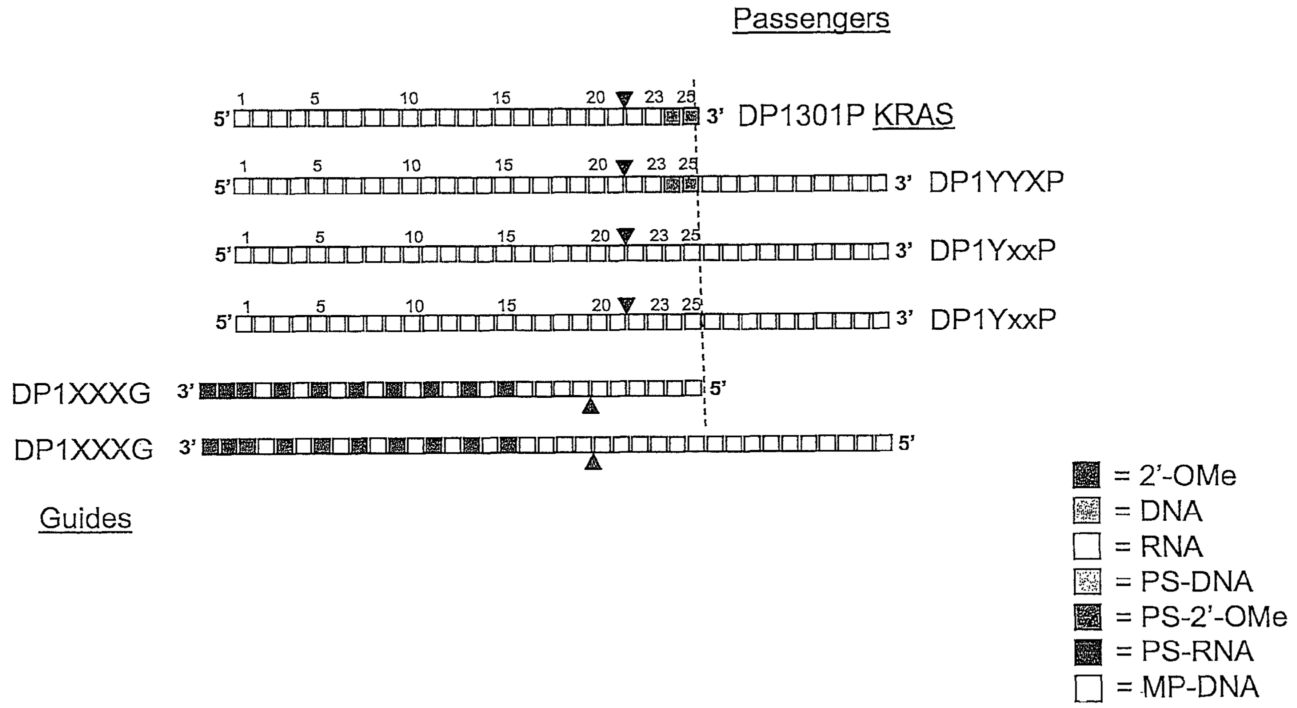

Figure 6. Different length ssDNA/RNA extensions KRAS-249M

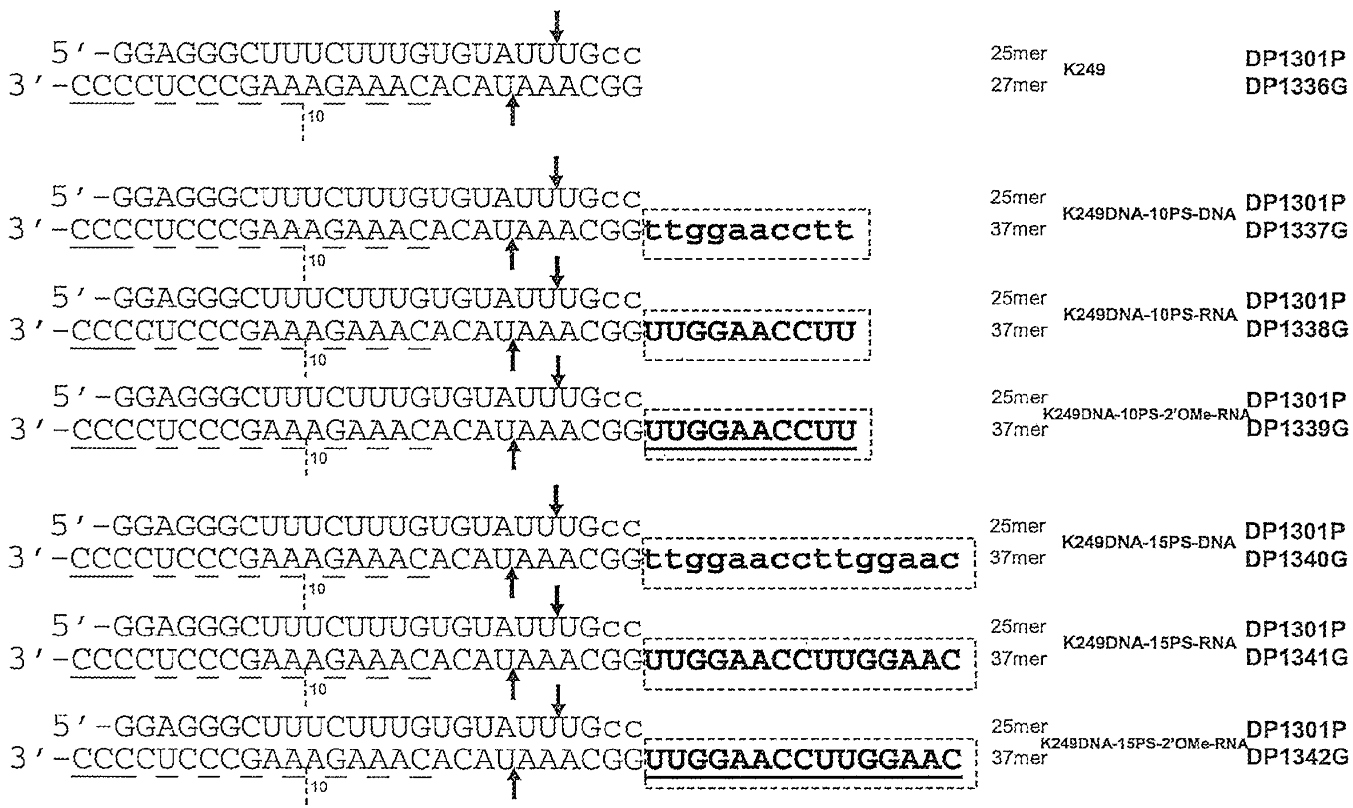

Figure 7. Different length ssDNA/RNA extensions KRAS-249

EXPERIMENT 06-30-09

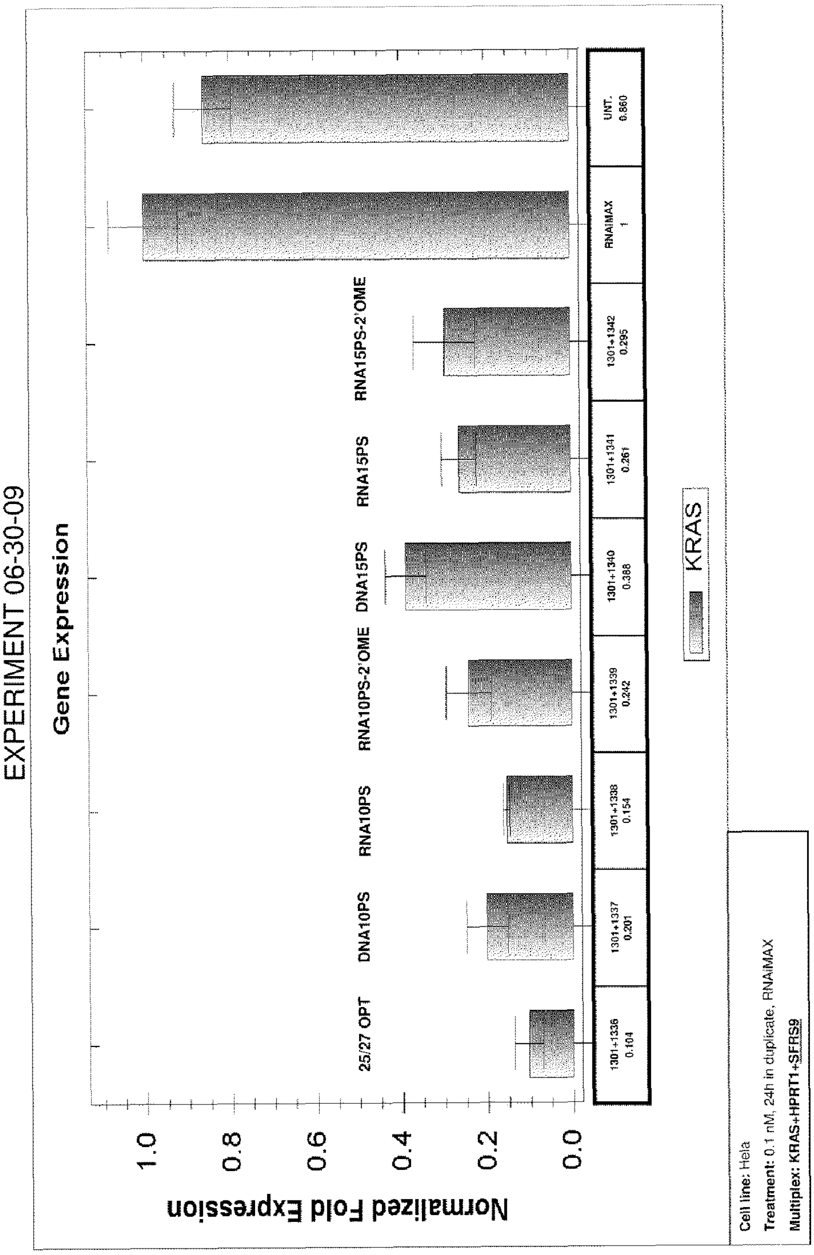

Figure 8. Different length ssDNA/RNA extensions HPRT1

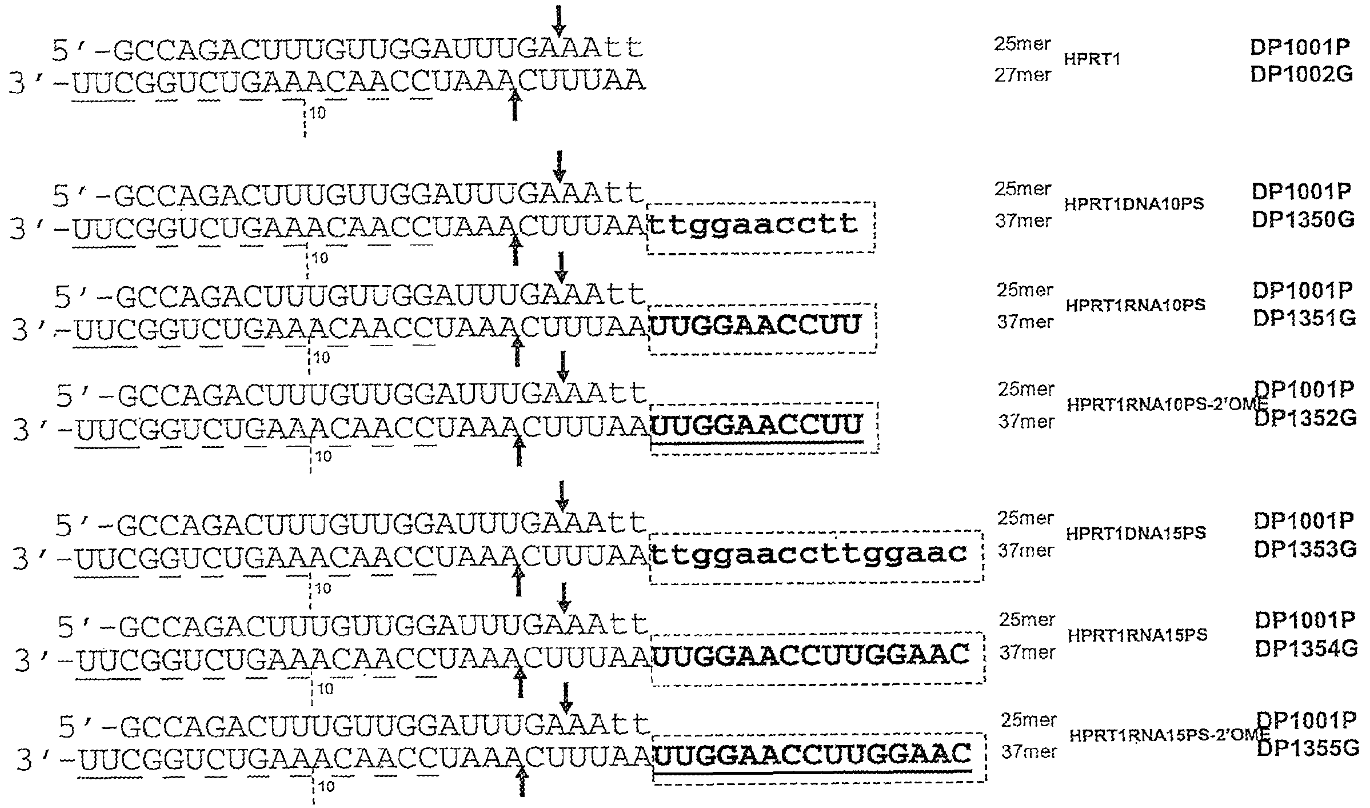

Figure 9. Different length ssDNA/RNA extensions HPRT-1

EXPERIMENT 06-30-09

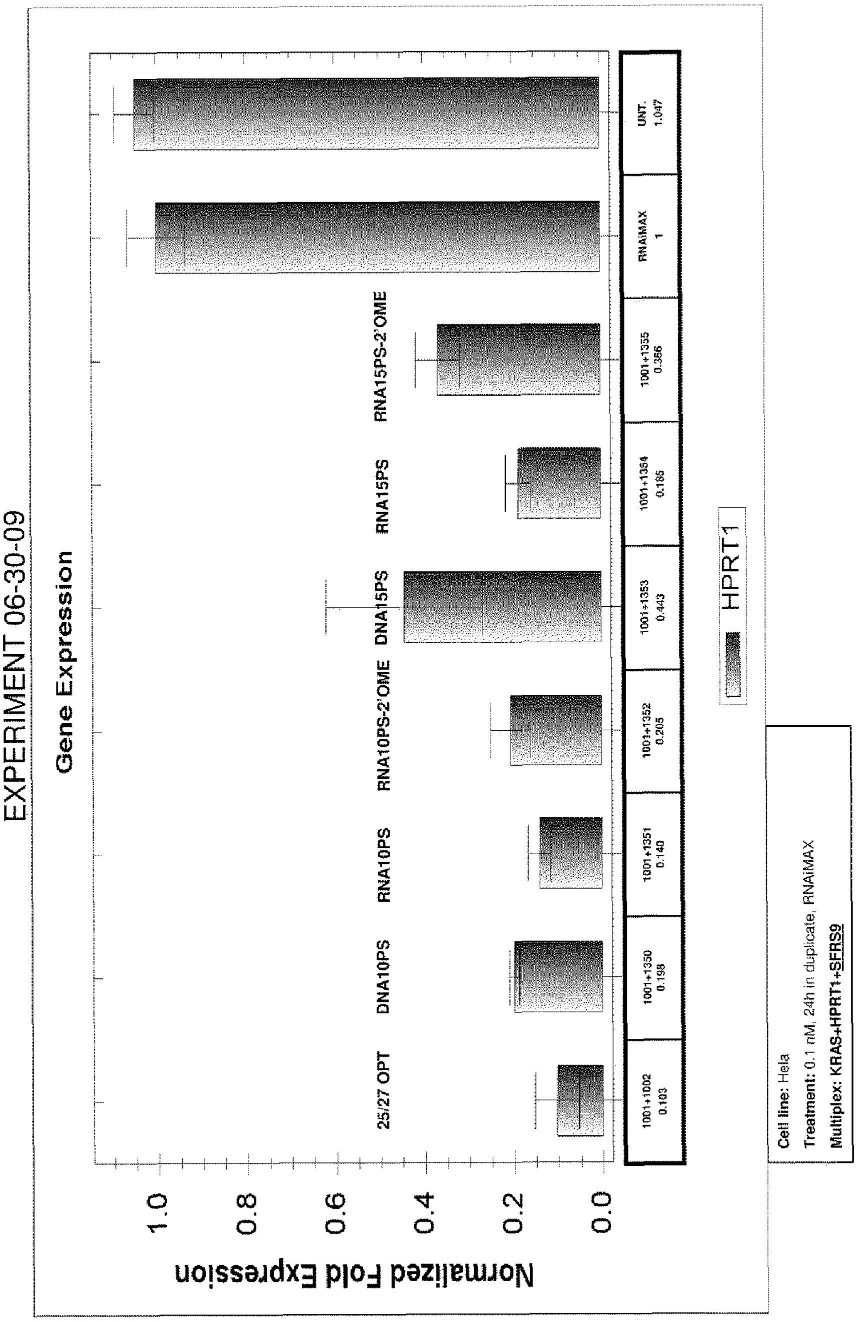

Figure 10. Different length ssDNA/RNA extensions DICING ASSAY

EXPERIMENT 07-07-09

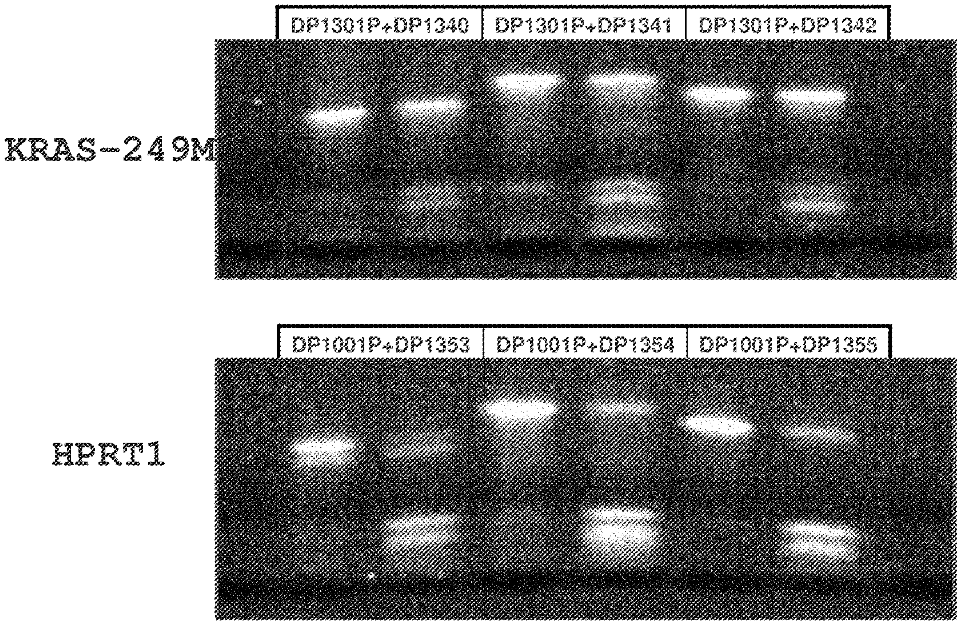


**Treatment:** 2h @ 37C Turbo Dicer (1U/reaction)

**Gel:** 18% Tris 90' @ 10W

**Loading:** (1ul 50uM+50ul Buffer and load 10ul) or (5ul reaction+20ul Buffer and load 10ul)

Figure 11. Short oligos that complement the ssExtensions

| Duplex | Name | ID |
|---|---|---|
| 5'-AACCUUGGAACCUUG-3'<br>3'-UUGGAACCUUGGAAC-5' | RNA15 | DP1365P |
| 5'-**AACCUUGGAACCUUG**-3'<br>3'-UUGGAACCUUGGAAC-5' | PS-RNA15 | DP1366P |
| 5'-**aaccttggaaccttg**-3'<br>3'-UUGGAACCUUGGAAC-5' | PS-DNA15 | DP1367P |
| 5'-**AACCUUGGAACCUUG**-3'<br>3'-UUGGAACCUUGGAAC-5' | PS-2'OMe-RNA15 | DP1368P |
| 5'-AACCUUGGAACCUUG-3'<br>3'-UUGGAACCUUGGAAC-5' | 2'OMe-RNA15 | DP1369P |

Figure 12. 15bp length ssDNA/RNA extensions + 15bp oligos that complement the extensions KRAS-249M

EXPERIMENT 07-27-09

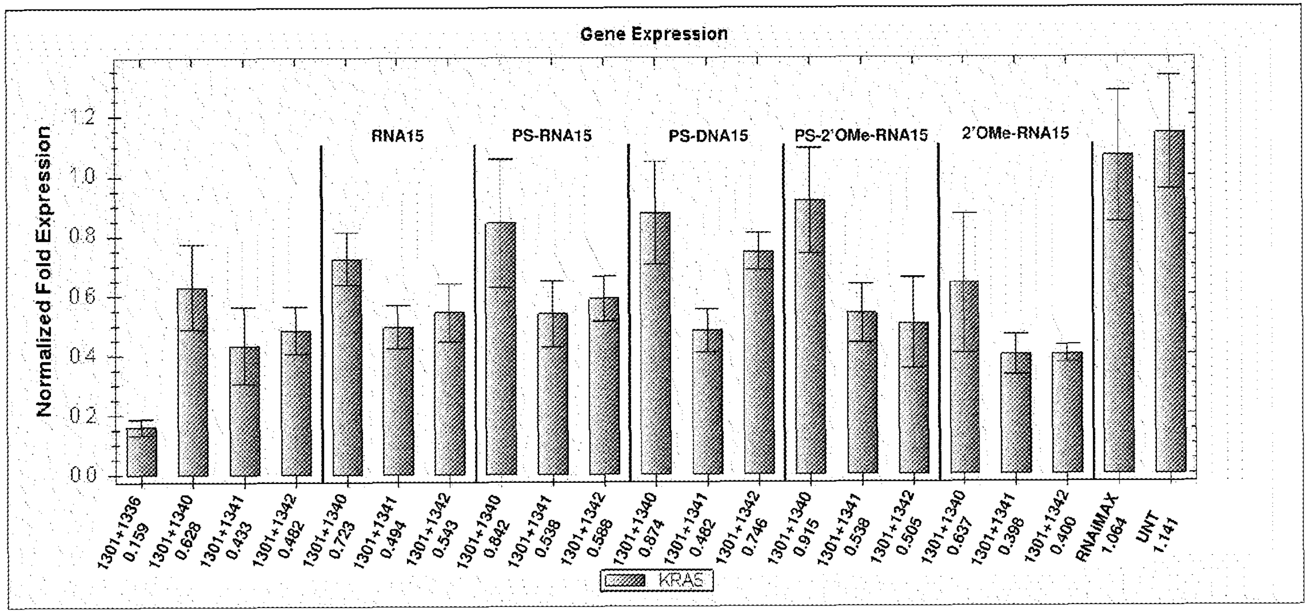


**Cell line:** Hela
**Treatment:** 0.1 nM, 24h in duplicate, RNAiMAX
**Multiplex:** KRAS+HPRT1+SFRS9

Figure 13. 15bp length ssDNA/RNA extensions + 15bp oligos that complement the extensions HPRT1

EXPERIMENT 07-27-09

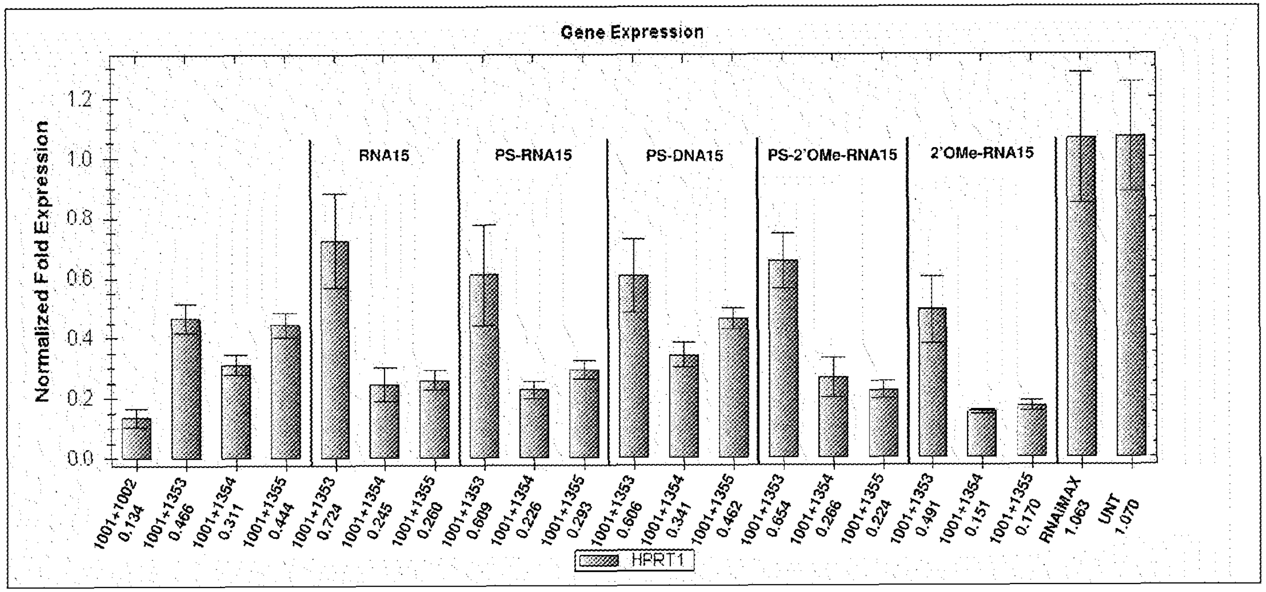


**Cell line:** Hela
**Treatment:** 0.1 nM, 24h in duplicate, RNAiMAX
**Multiplex:** KRAS+HPRT1+SFRS9

Figure 14. PS-2'OMe DsiRNA in vivo experiment
(09-RES-019, ver 12.1, Liver)

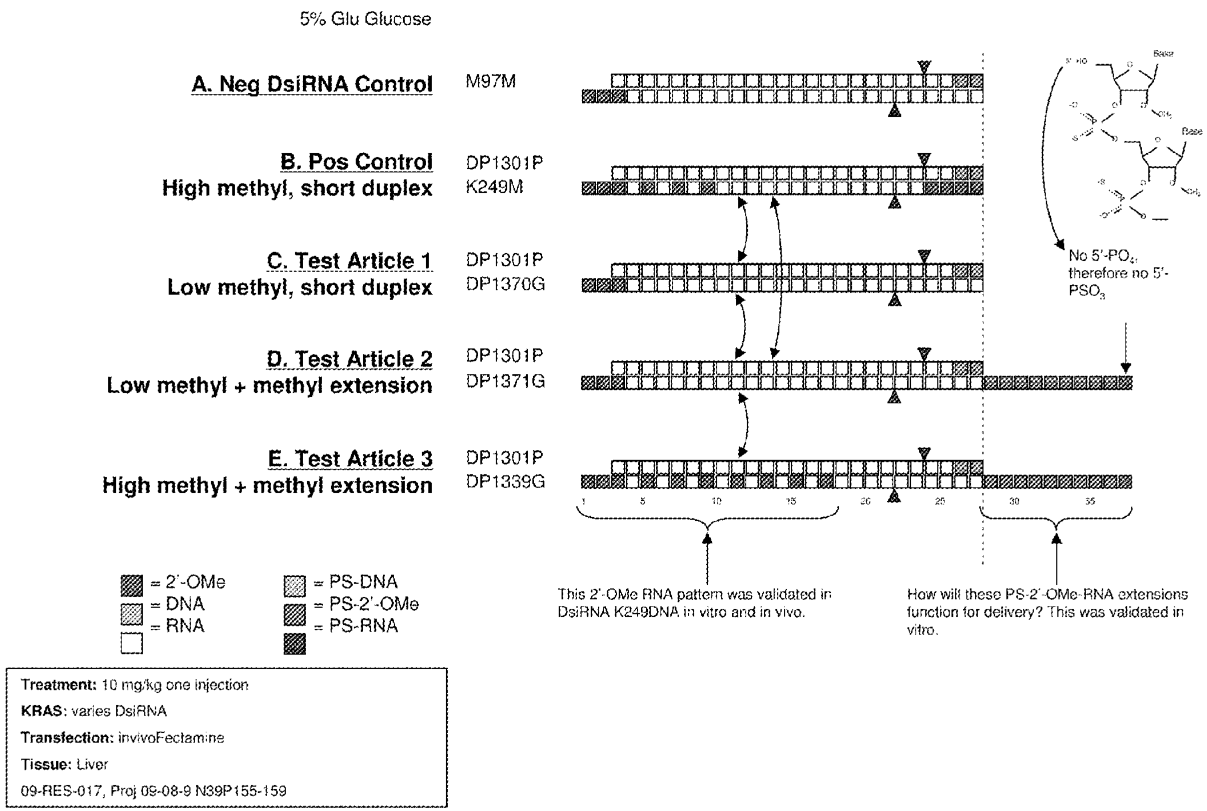

Figure 15. DsiRNA with ssDNA/RNA strand extensions 10bp length

5'-GGAGGGCUUUCUUUGUGUAUUUGcc 100mg 25mer K249 DP1301P
3'-CCCCUCCCGAAAGAAACACAUAAACGG 25mg 27mer DP1370G
10

5'-GGAGGGCUUUCUUUGUGUAUUUGcc 25mer K249DNA-10PS-DNA DP1301P
3'-CCCCUCCCGAAAGAAACACAUAAACGGttggaacctt 25mg 37mer DP1337G
10

5'-GGAGGGCUUUCUUUGUGUAUUUGcc 25mer K249DNA-10PS-RNA DP1301P
3'-CCCCUCCCGAAAGAAACACAUAAACGGUUGGAACCUU 25mg 37mer DP1338G
10

5'-GGAGGGCUUUCUUUGUGUAUUUGcc 25mer K249DNA10PS-2'OME-RNA DP1301P
3'-CCCCUCCCGAAAGAAACACAUAAACGGUUGGAACCUU 25mg 37mer DP1339G
10

5'-GGAGGGCUUUCUUUGUGUAUUUGcc 25mer K249-10PS-2'OME-RNA DP1301P
3'-CCCCUCCCGAAAGAAACACAUAAACGGUUGGAACCUU 25mg 37mer DP1371G
10

5'-AACCUUGGAA-3' 25mg 10PS-2'OMe-RNA DP1372P
3'-UUGGAACCUU-5'

5'-AACCUUGGAA-3' 25mg 10-2'OMe-RNA DP1373P
3'-UUGGAACCUU-5'

5'-GCCAGACUUUGUUGGAUUUGAAAtt 25mg 25mer HPRT1RNA10PS-2'OMe DP1001P
3'-UUCGGUCUGAAACAACCUAAACUUUAAUUGGAACCUU 25mg 37mer DP1352G
10

Figure 16. PS-2'OMe DsiRNA in vivo experiment
(09-RES-019, ver 12.1, Liver)

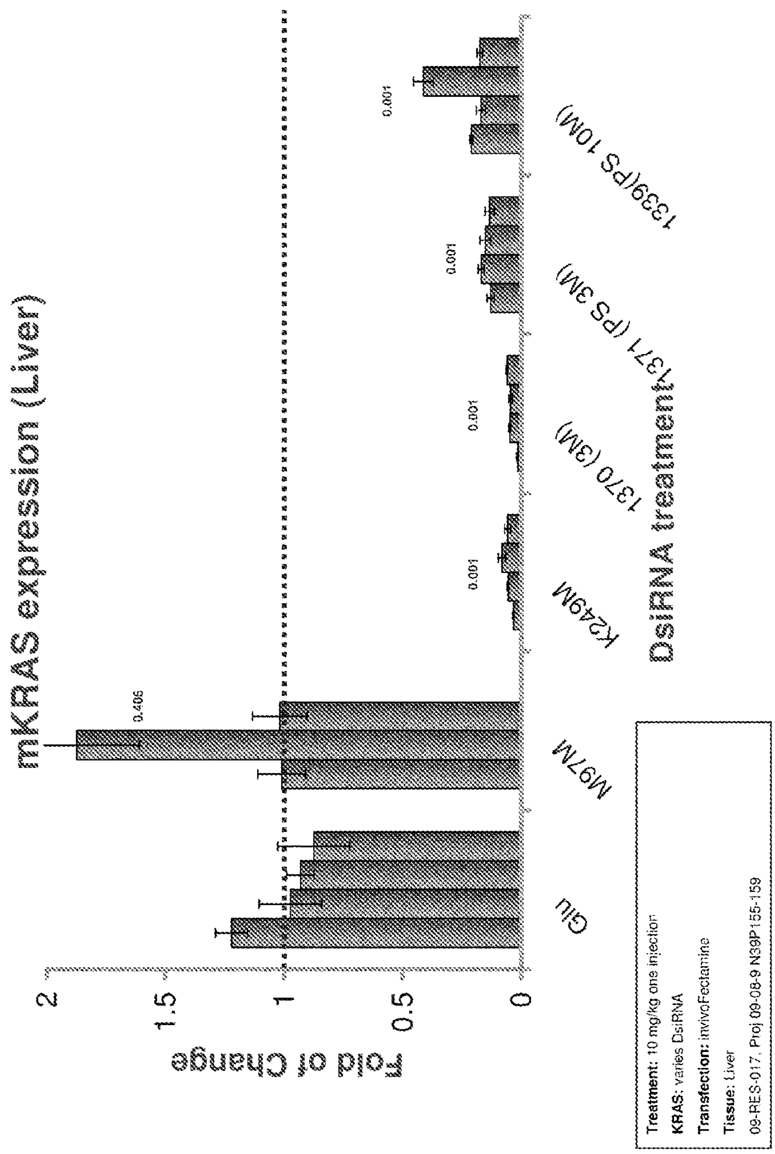

Figure 17. PS-2'OMe DsiRNA in vivo experiment
(09-RES-019, ver 12.1, Liver)

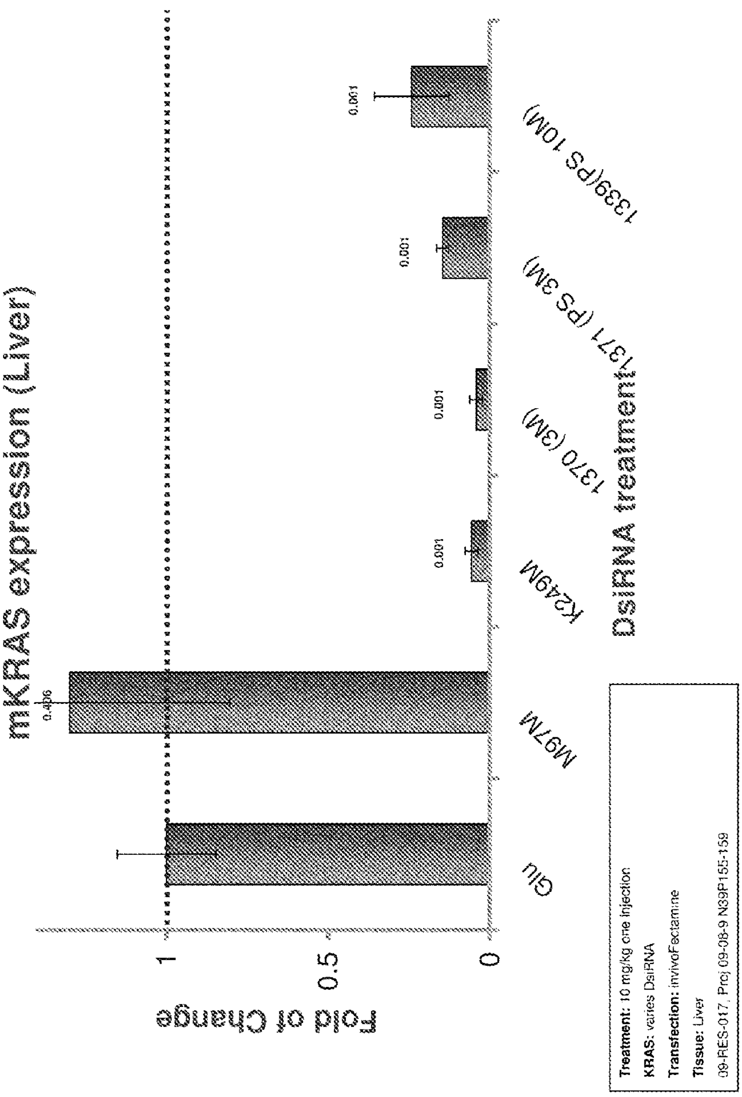

Figure 18. PS-2'OMe DsiRNA in vivo experiment
(09-RES-019, ver 12.1, Liver)

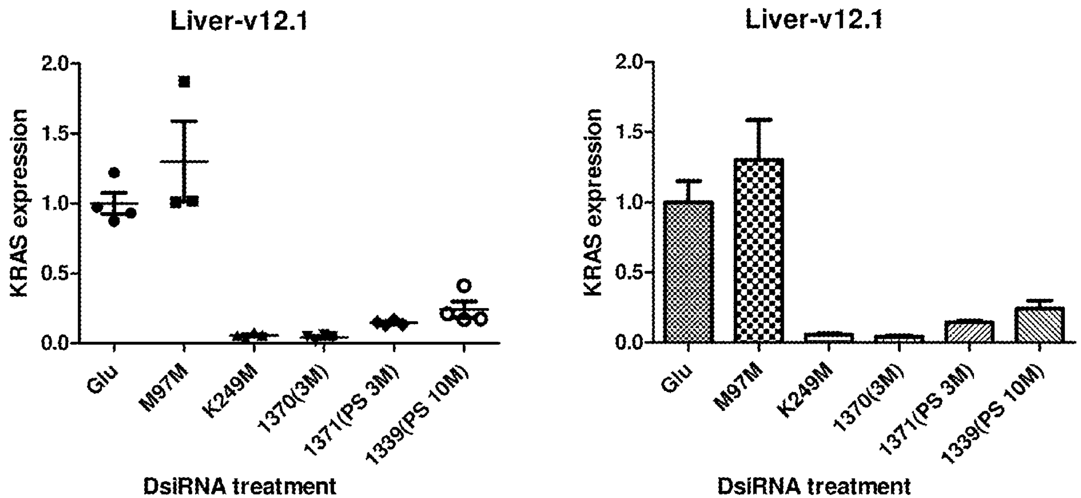


**Treatment:** 10 mg/kg one injection
**KRAS:** varies DsiRNA
**Transfection:** invivoFectamine
**Tissue:** Liver
09-RES-017, Proj 09-08-9 N39P155-159

Figure 19. PS-2'OMe DsiRNA in vivo experiment
(09-RES-019, ver 12.2, Spleen)

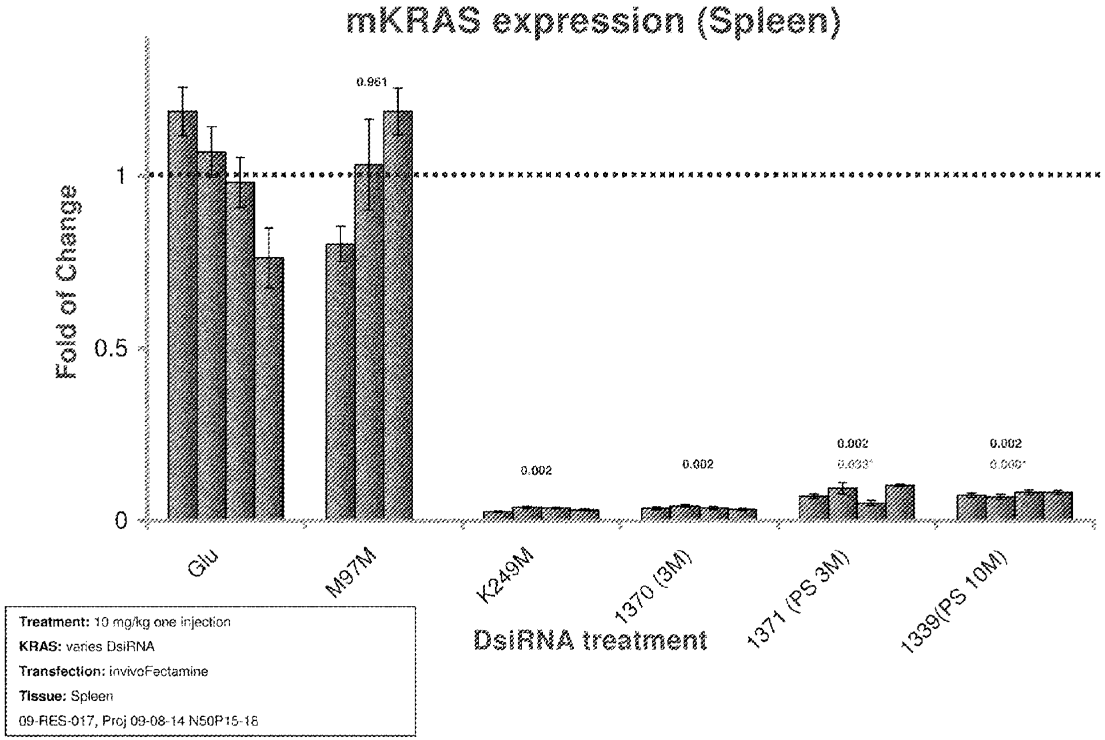

Figure 20. PS-2'OMe DsiRNA in vivo experiment
(09-RES-019, ver 12.2, Spleen)

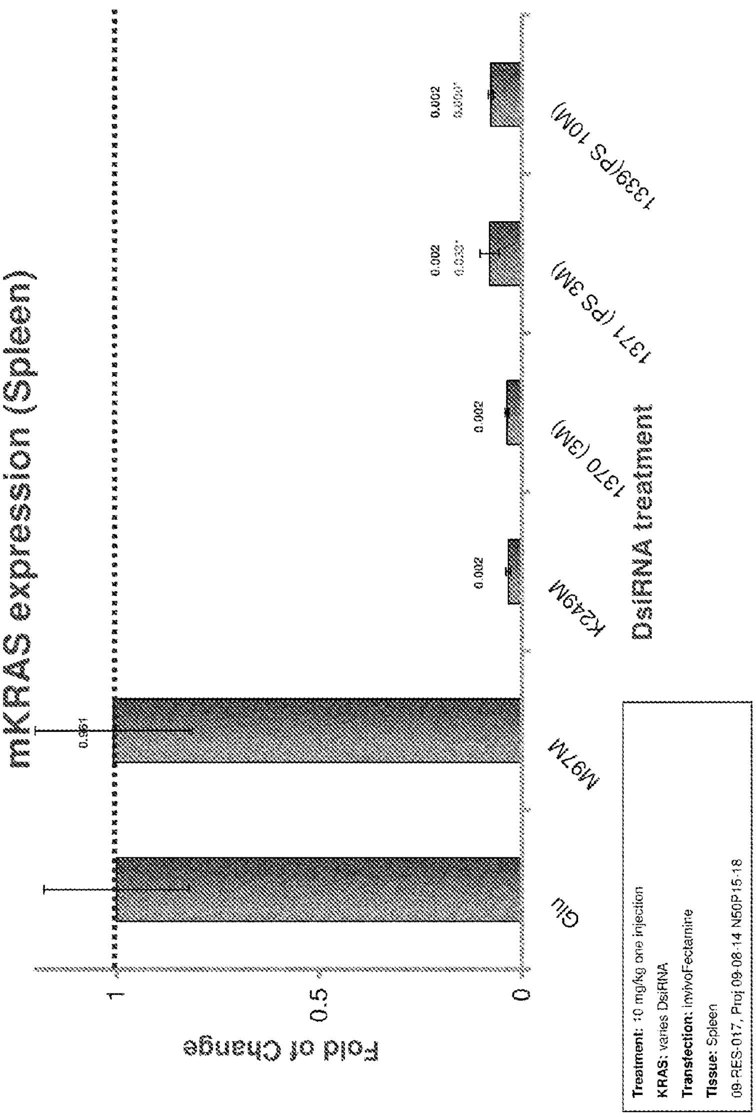

Figure 21. PS-2'OMe DsiRNA in vivo experiment
(09-RES-019, ver 12.2, Spleen)

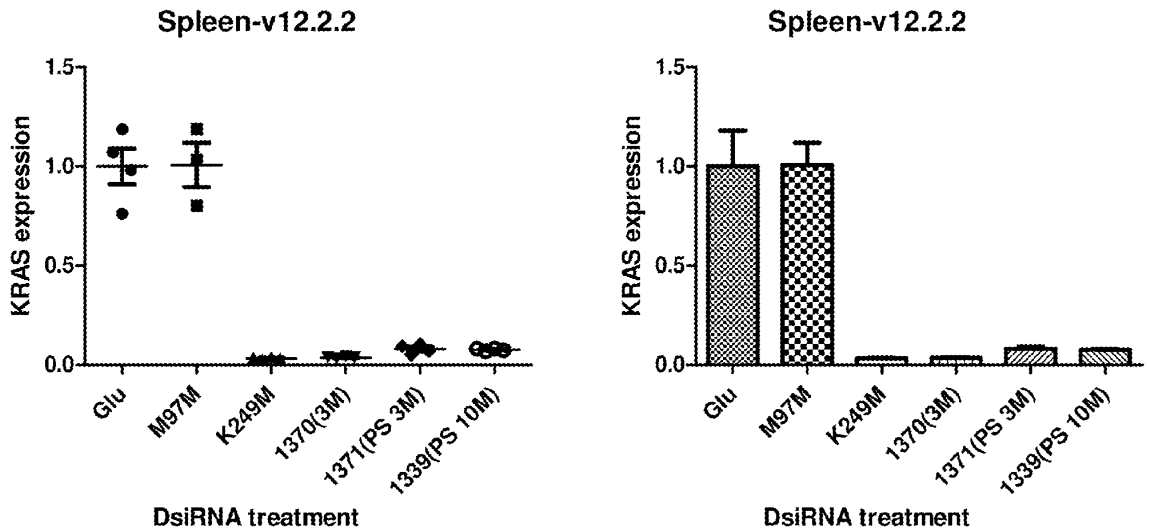


**Treatment:** 10 mg/kg one injection
**KRAS:** varies DsiRNA
**Transfection:** invivoFectamine
**Tissue:** Spleen
09-RES-017, Proj 09-06-14 N50P15-18

Figure 22. PS-2'OMe DsiRNA in vivo experiment
(09-RES-019, ver 12.3, Kidney)

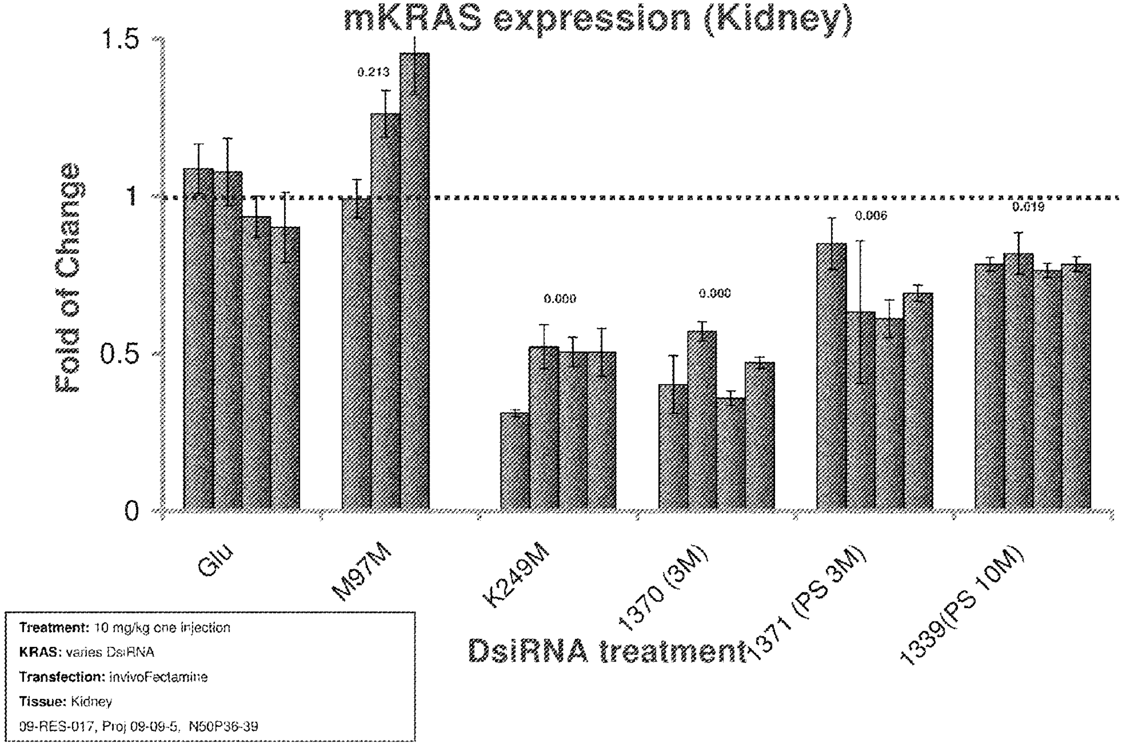

Figure 23. PS-2'OMe DsiRNA in vivo experiment
(09-RES-019, ver 12.3, Kidney)

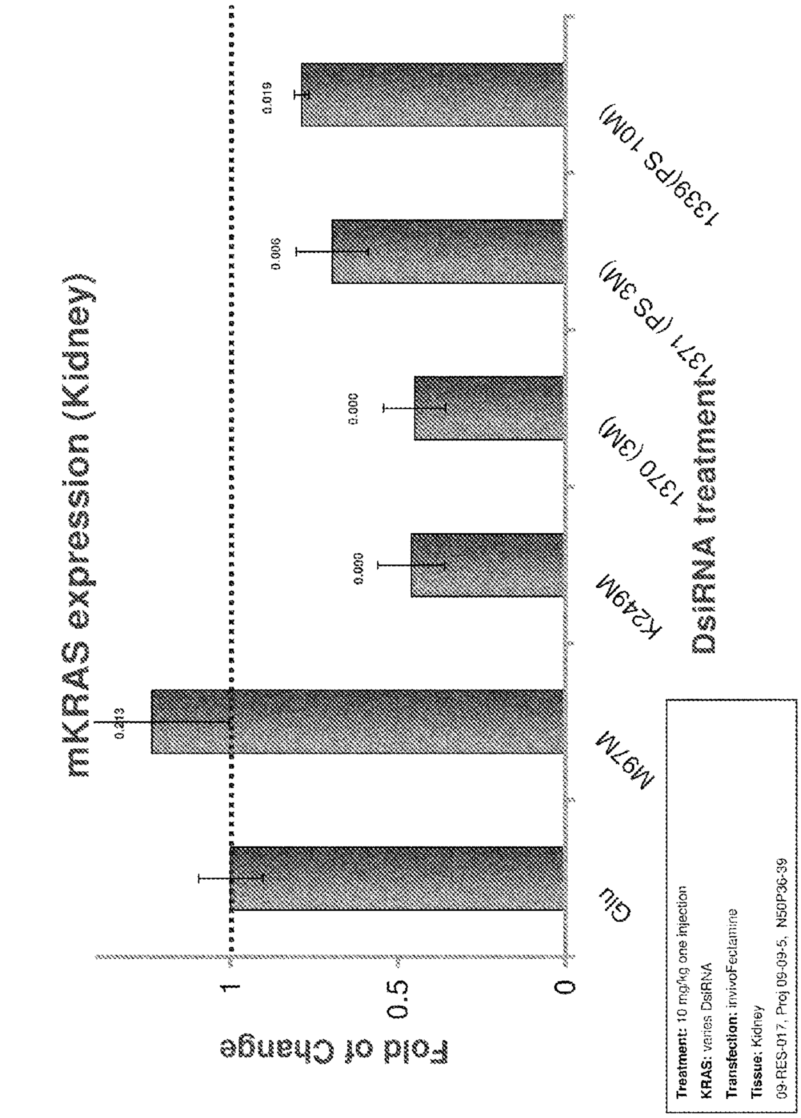

Figure 24. PS-2'OMe DsiRNA in vivo experiment
(09-RES-019, ver 12.3, Kidney)

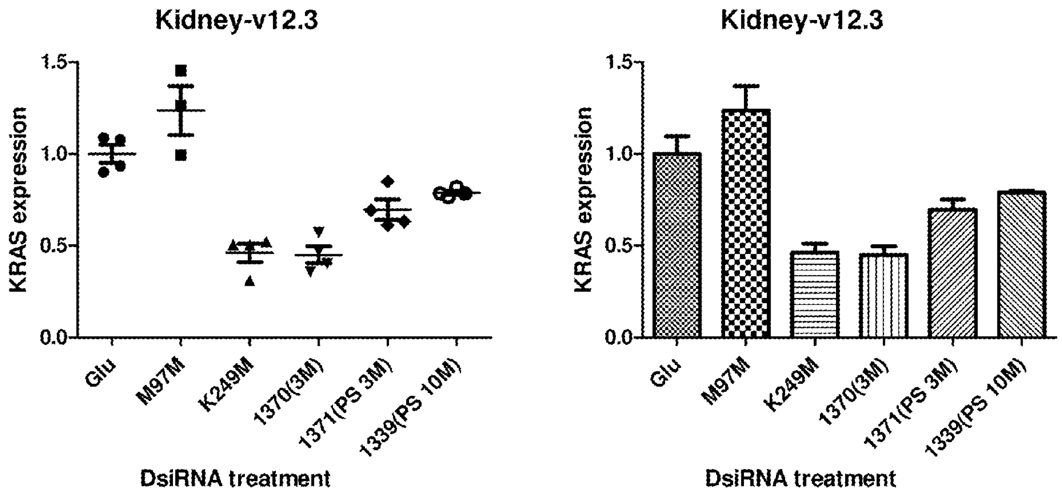


**Treatment:** 10 mg/kg one injection
**KRAS:** varies DsiRNA
**Transfection:** invivoFectamine
**Tissue:** Kidney
09-RES-017, Proj 09-09-5, N50P36-39

# Figure 25 A Self-delivery activity in primary mouse hepatocytes in culture

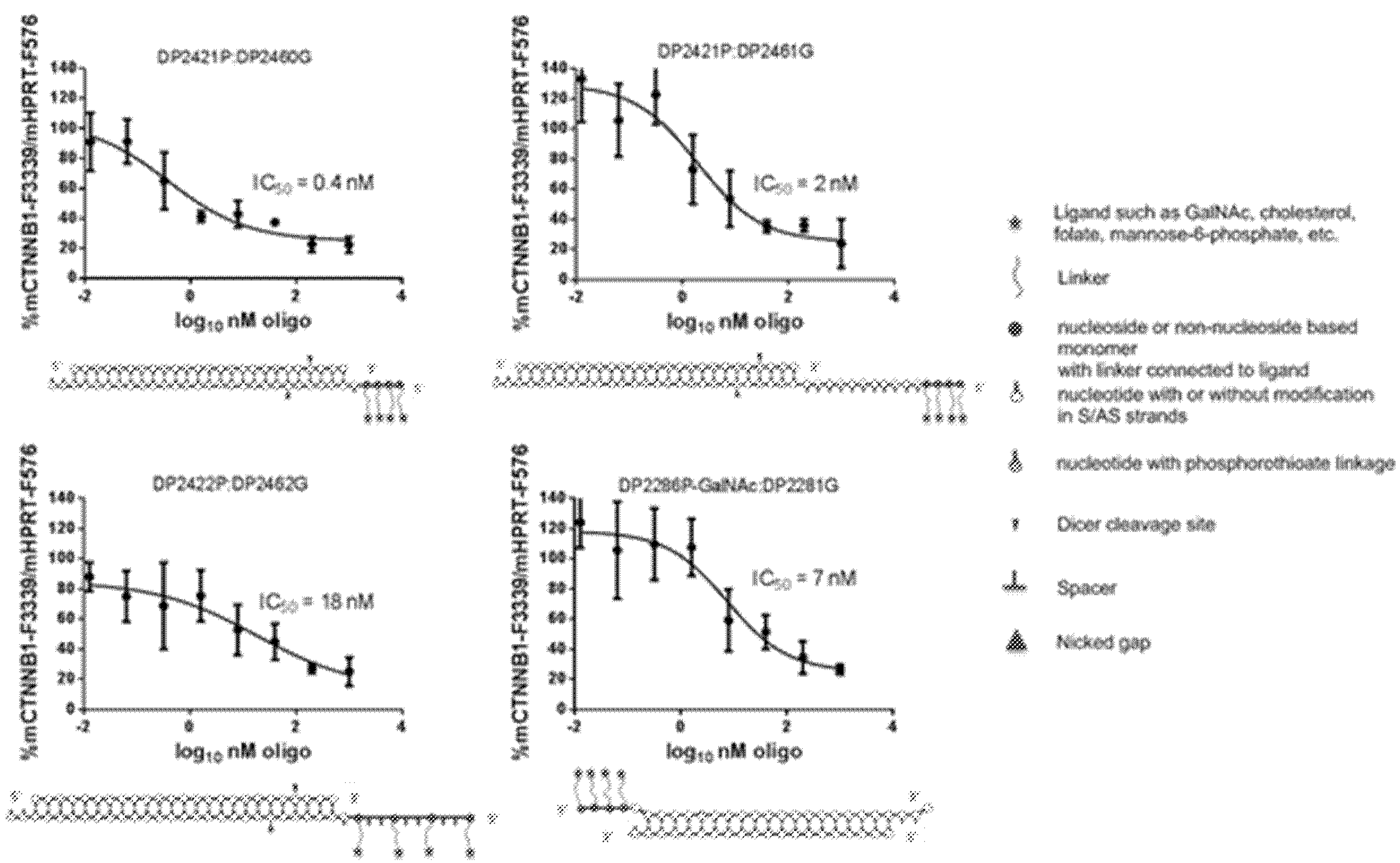

# Figure 25 B Self-delivery activity in primary mouse hepatocytes in culture

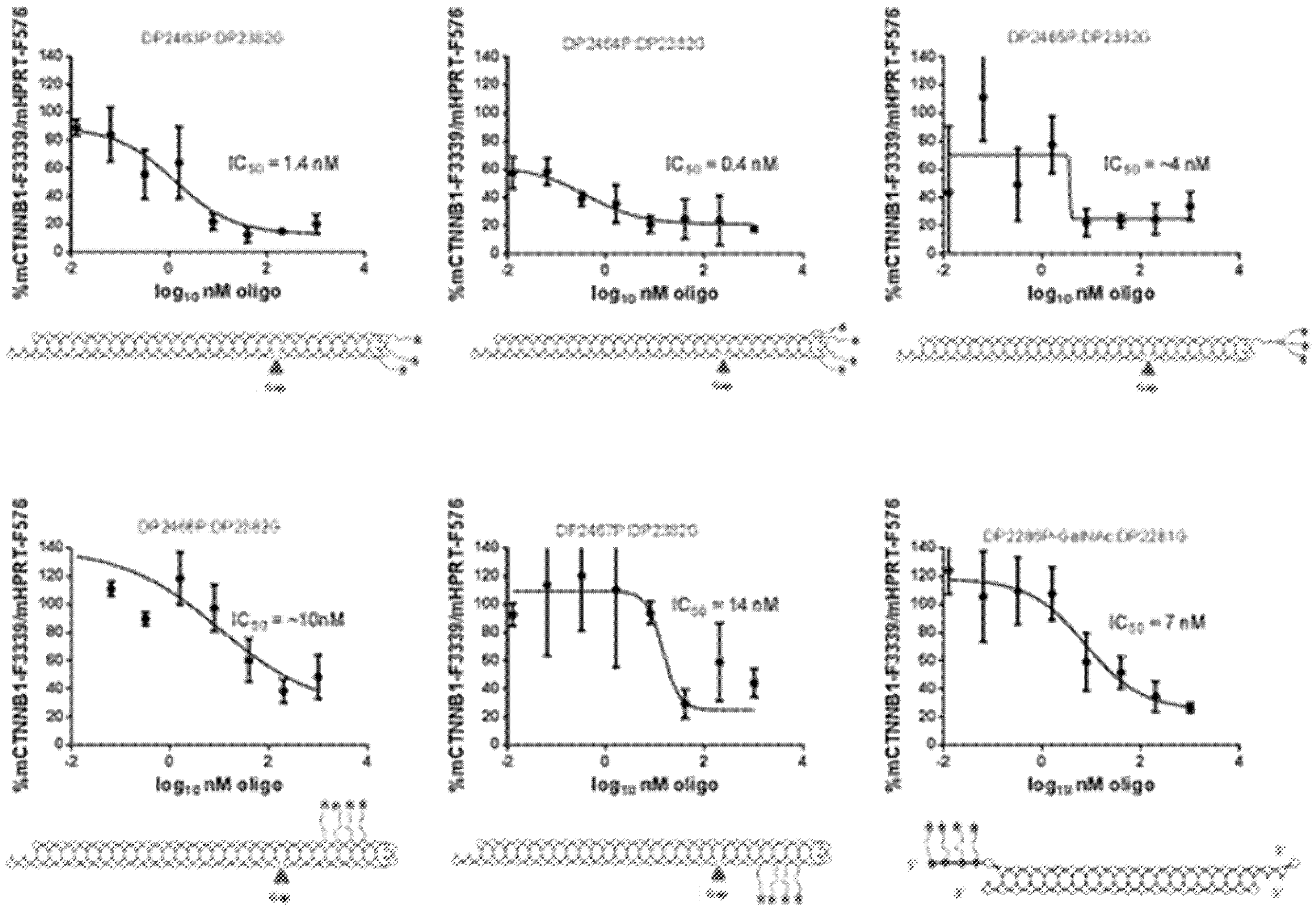

# Figure 26 ASGPr binding using Fluorescence polarization

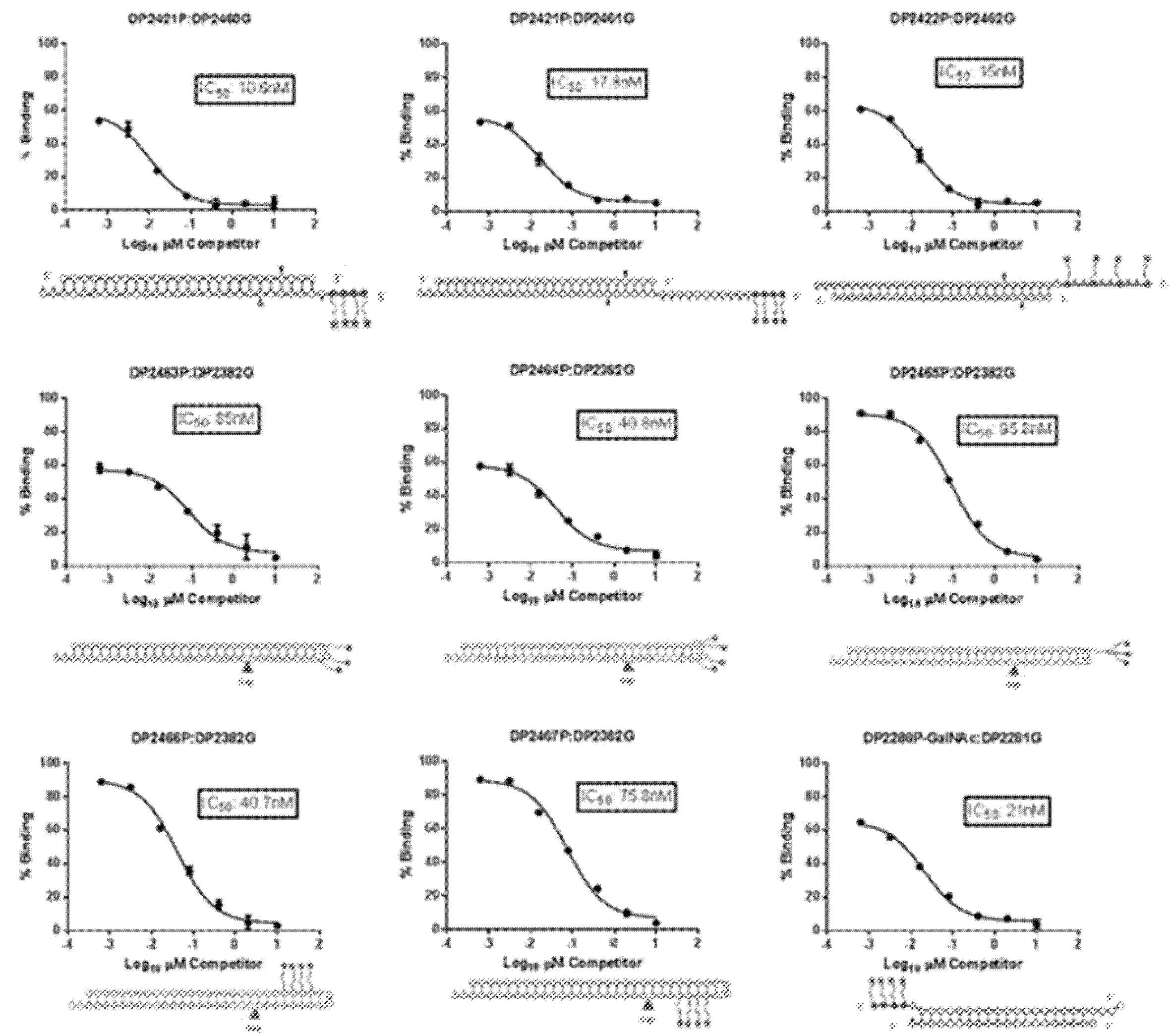

**Fig. 27. CTNNB1 mRNA knockdown activity in mice with N-acetylgalactosamine DsiRNA conjugates**

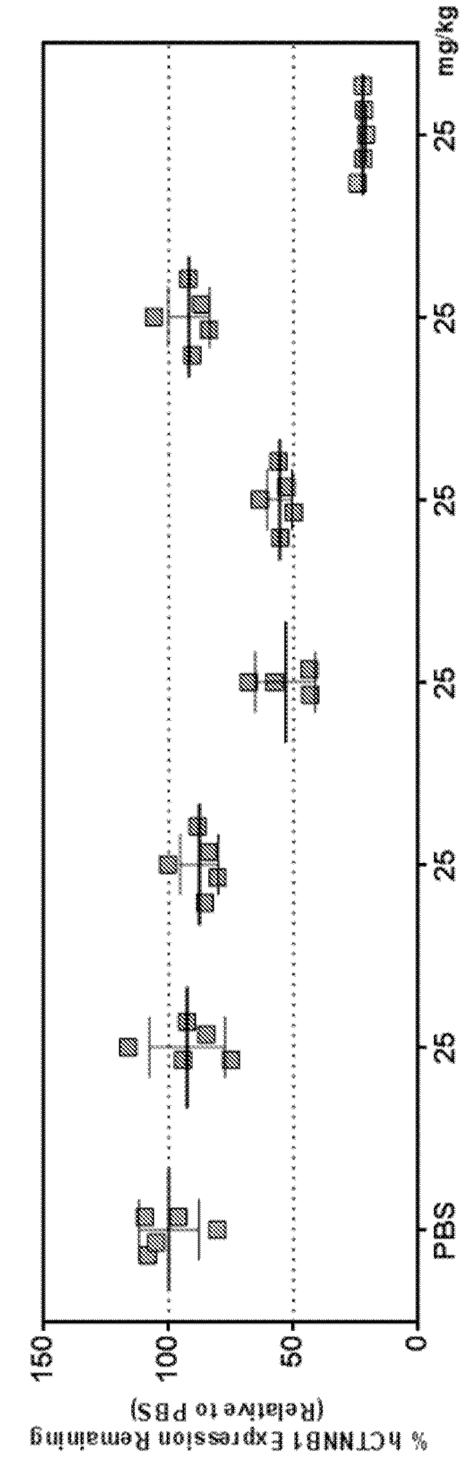

Figure 28. CTNNB1 mRNA knockdown activity in mice with N-acetylgalactosamine DsiRNA conjugates

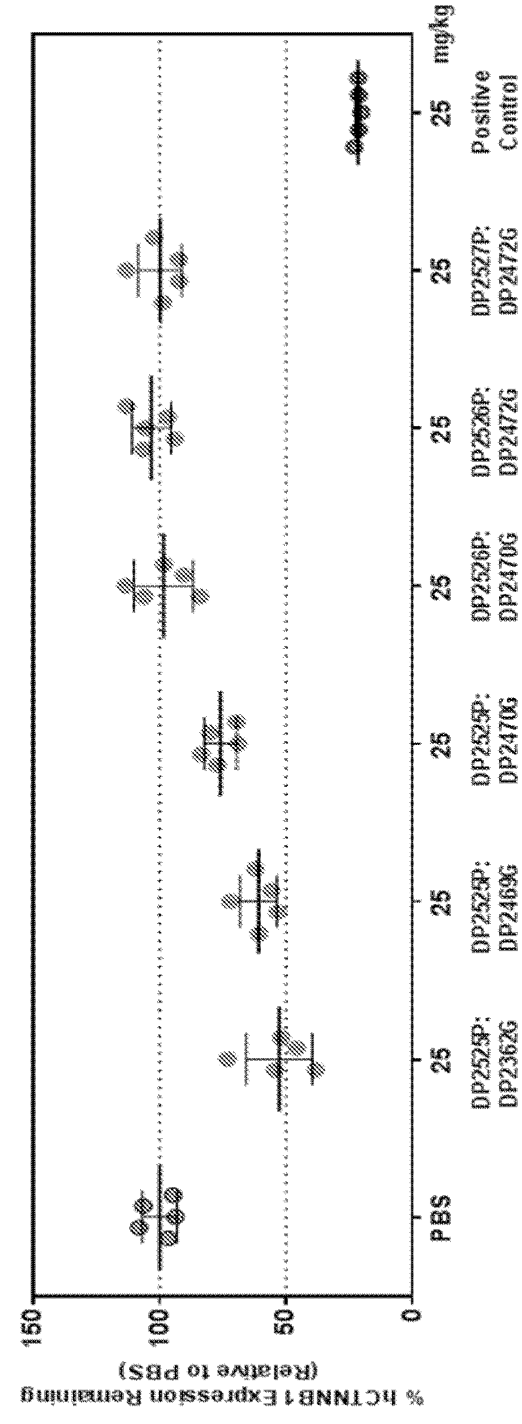

# Figure 29. HAO1 mRNA knockdown activity at 25mpk single dose in mice with N-acetylgalactosamine DsiRNA

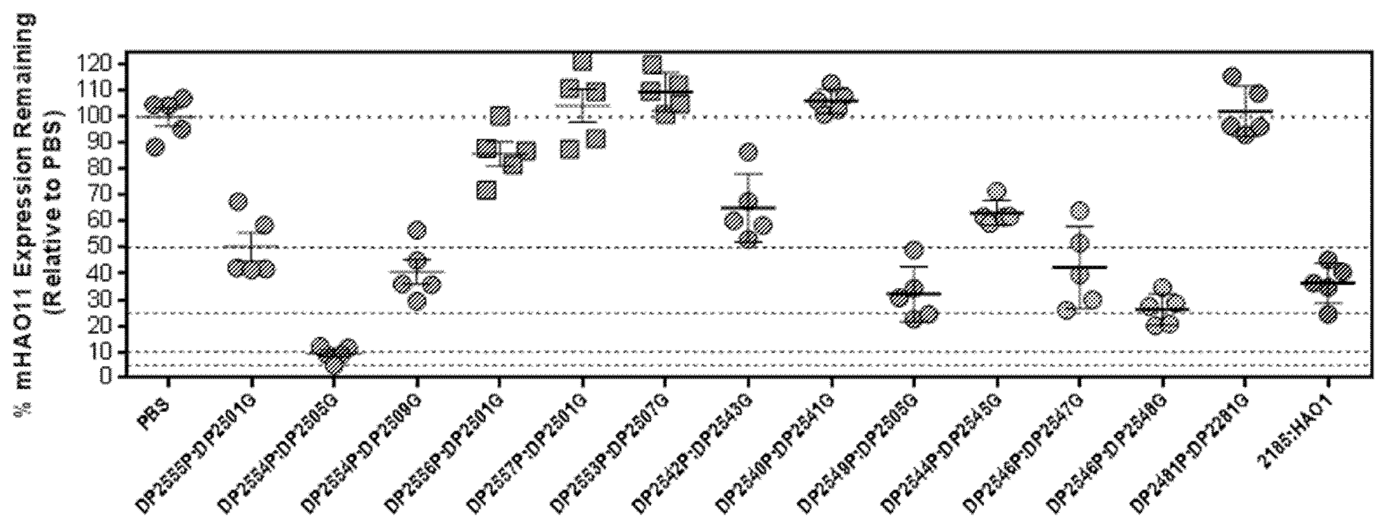

**Figure 30 Dose response of HAO1 DsiRNA N-acetylgalactosamine Conjugate in mice**

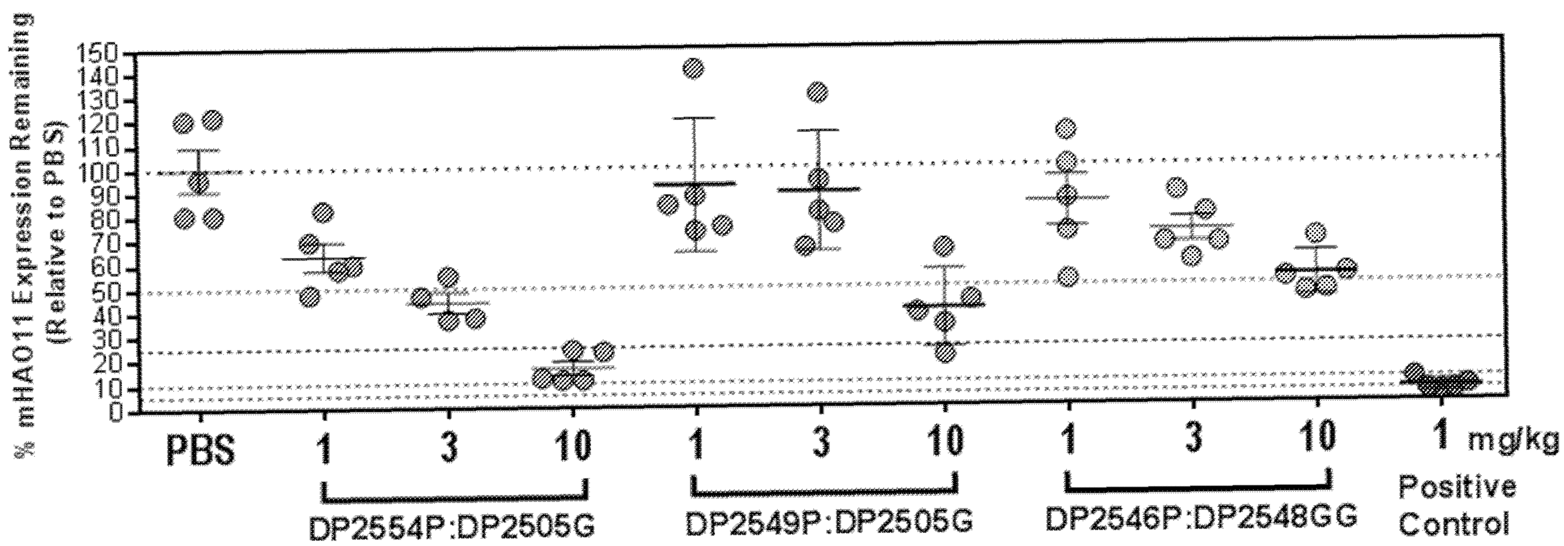

Fig. 31

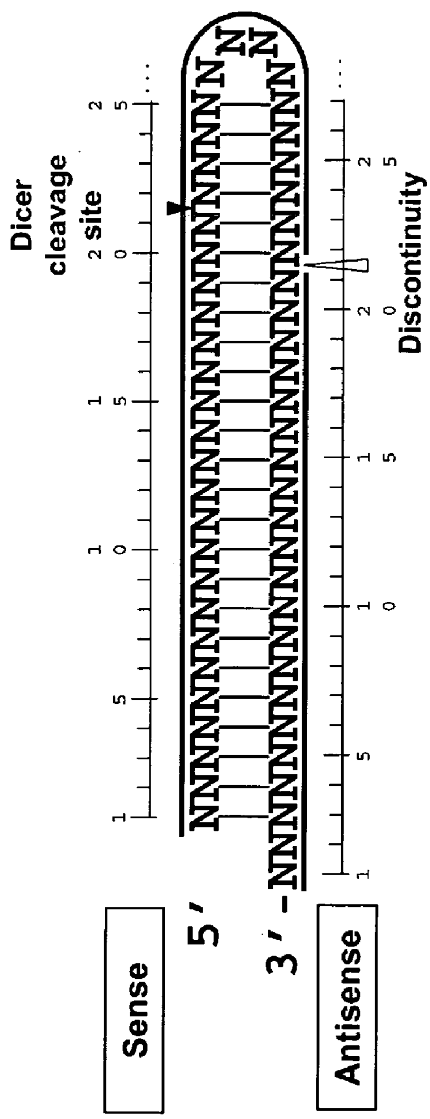

Fig 31A

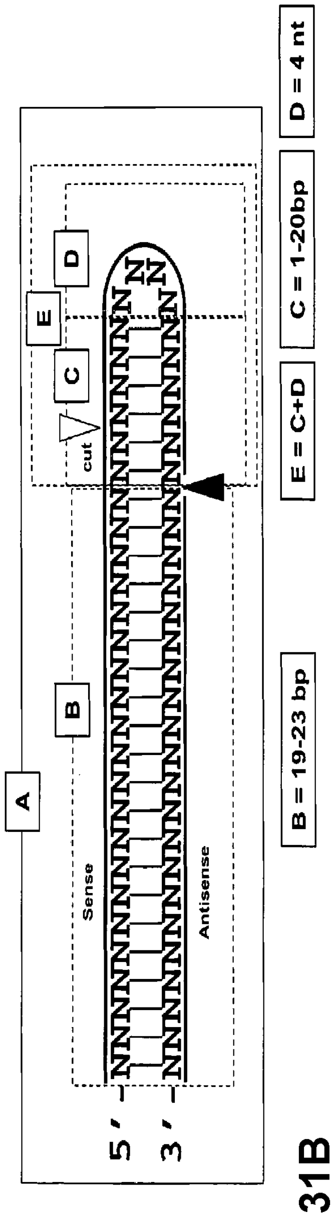


Fig 31B

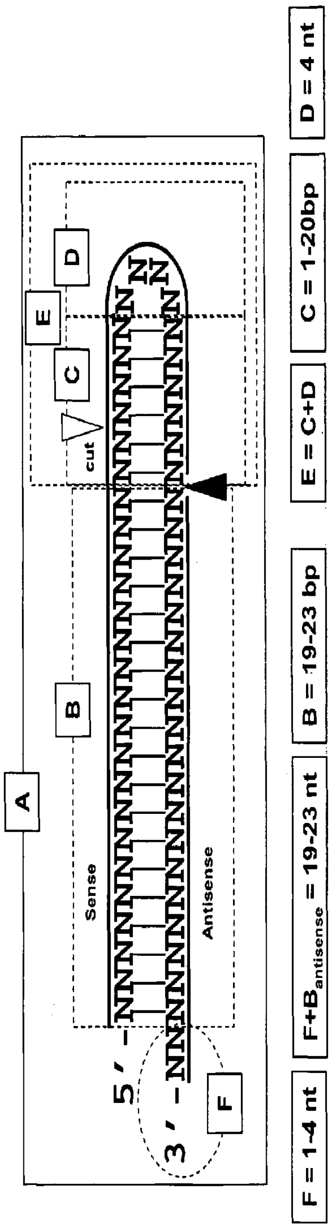

Fig 31C

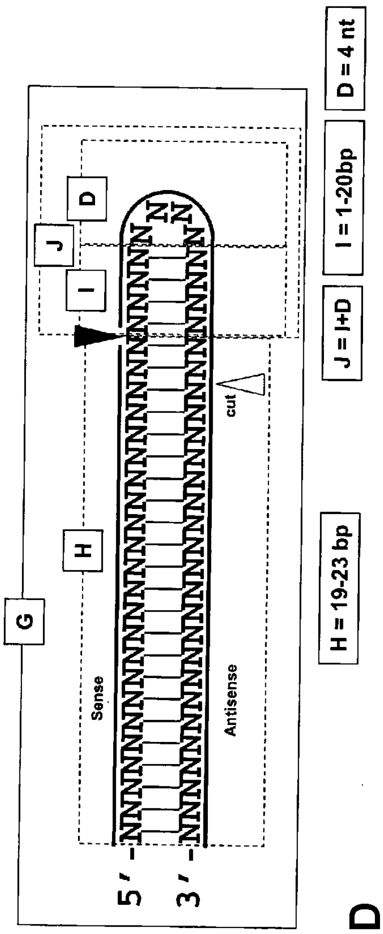


Fig 31D

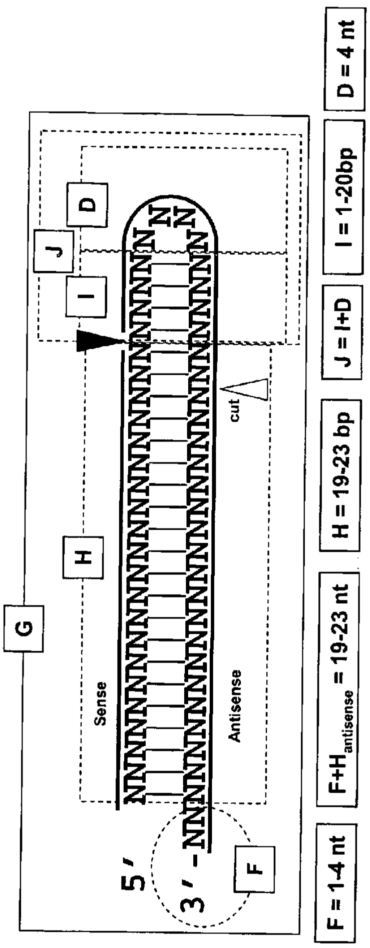

Fig 32A

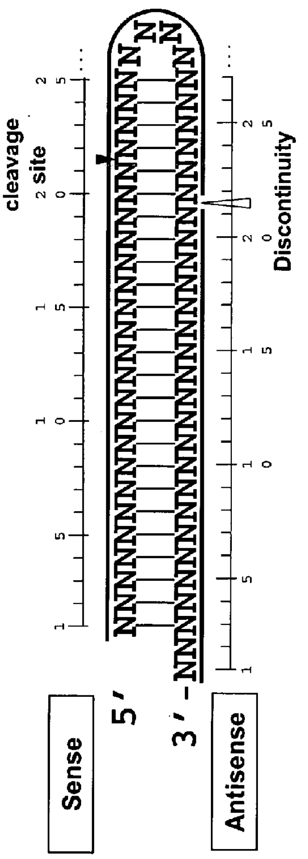


Fig 32B

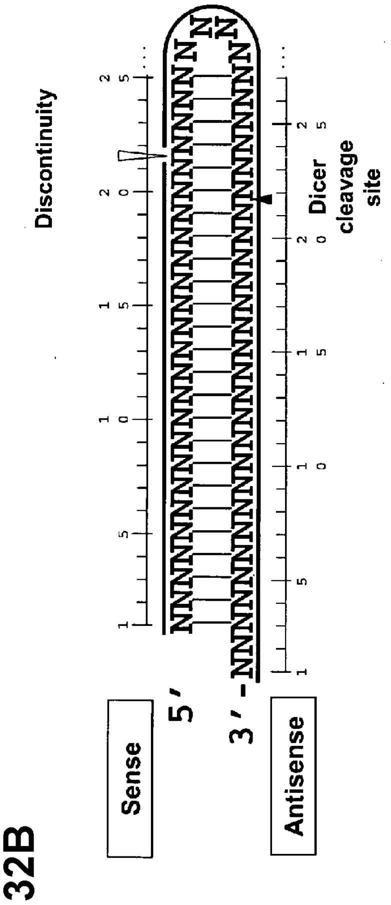

Fig 33A

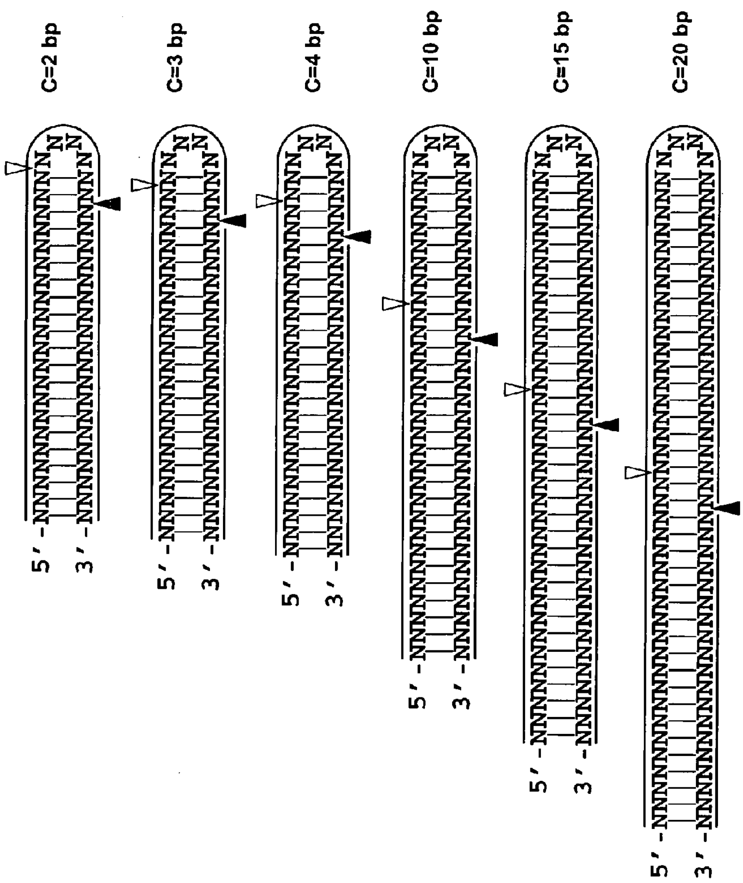

Fig 33B

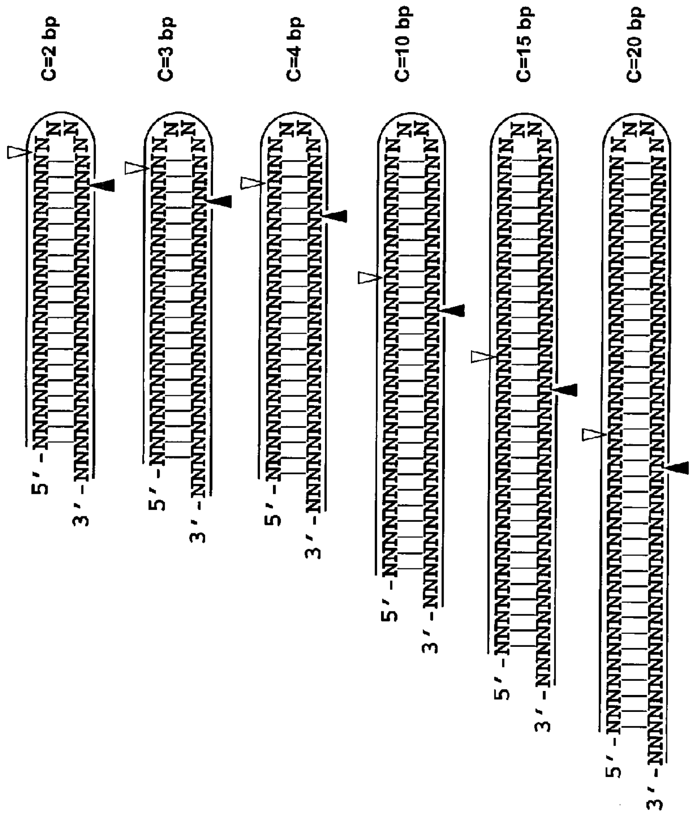

Fig 33C

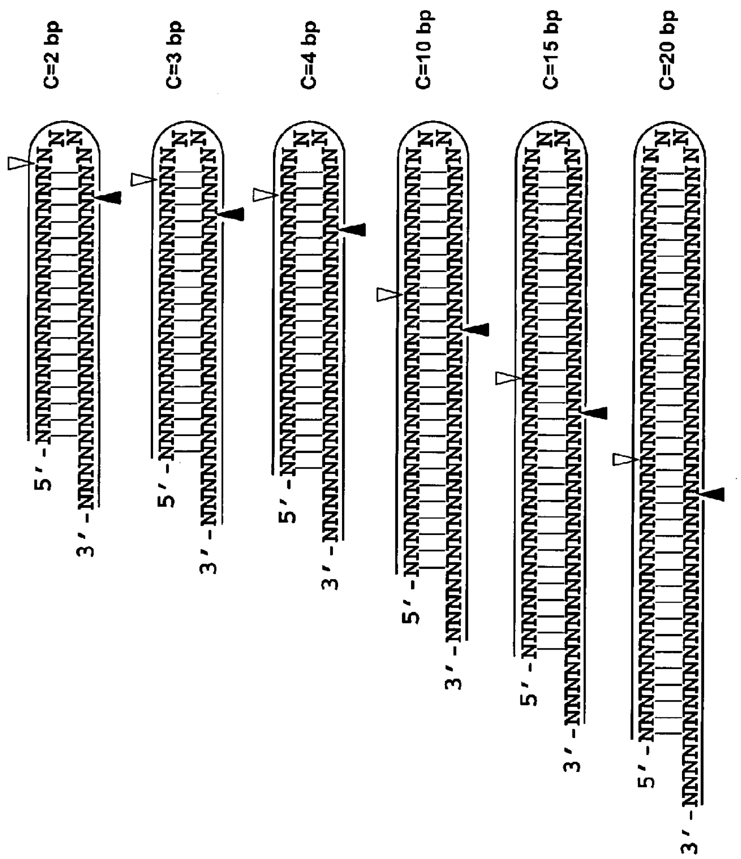

Fig 34A

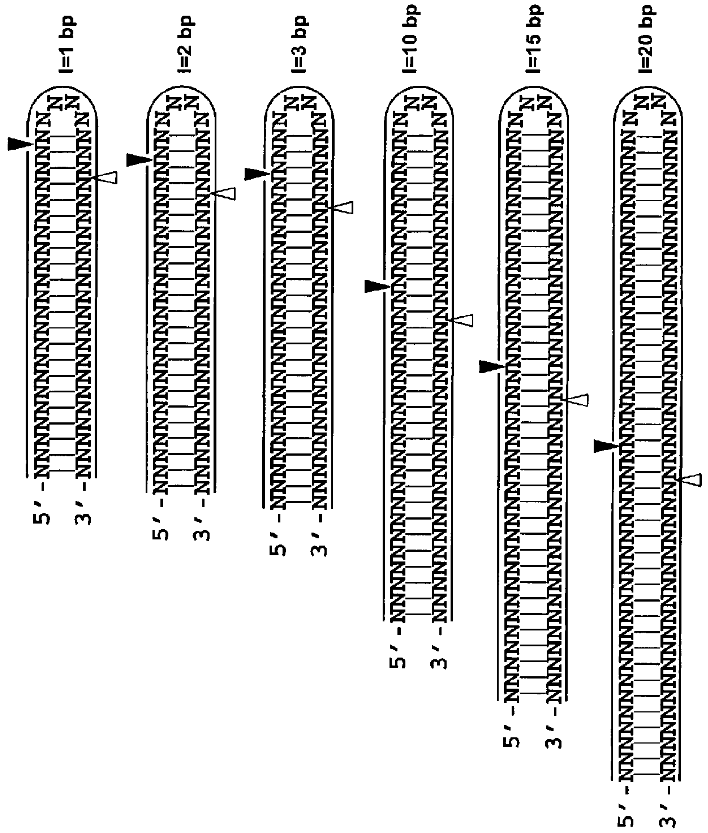

Fig 34B

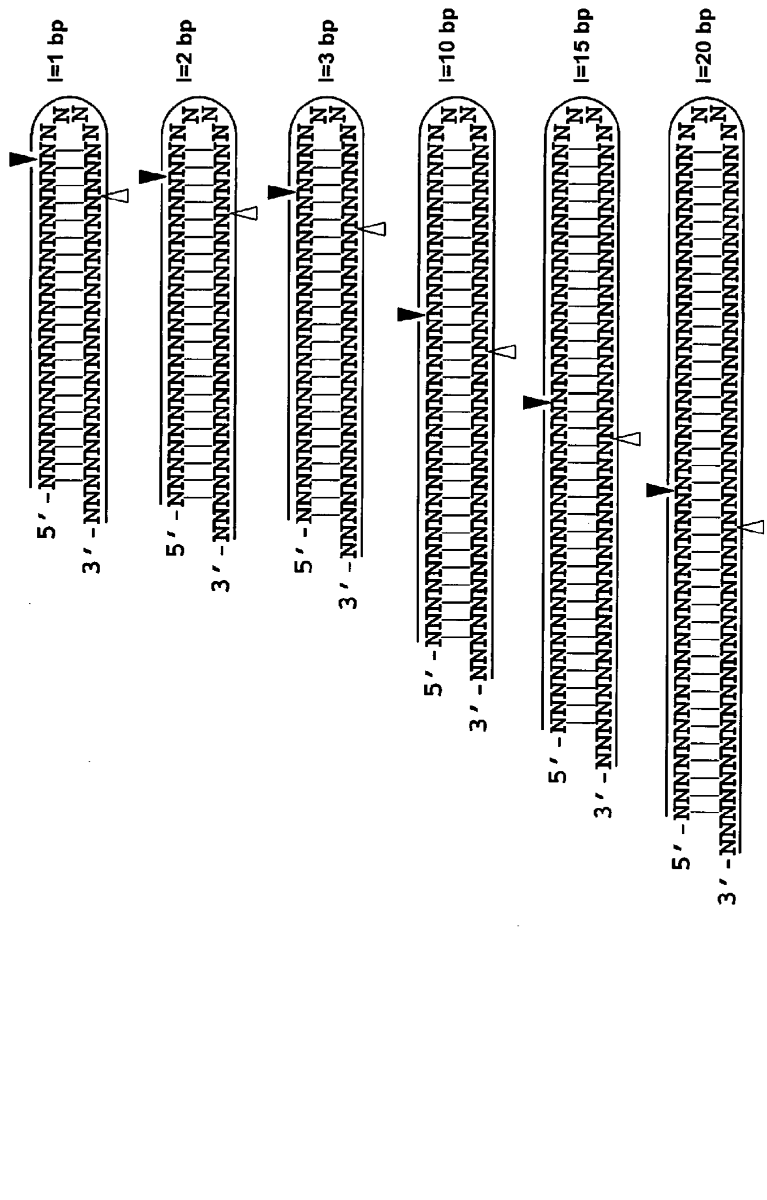

Fig 34C

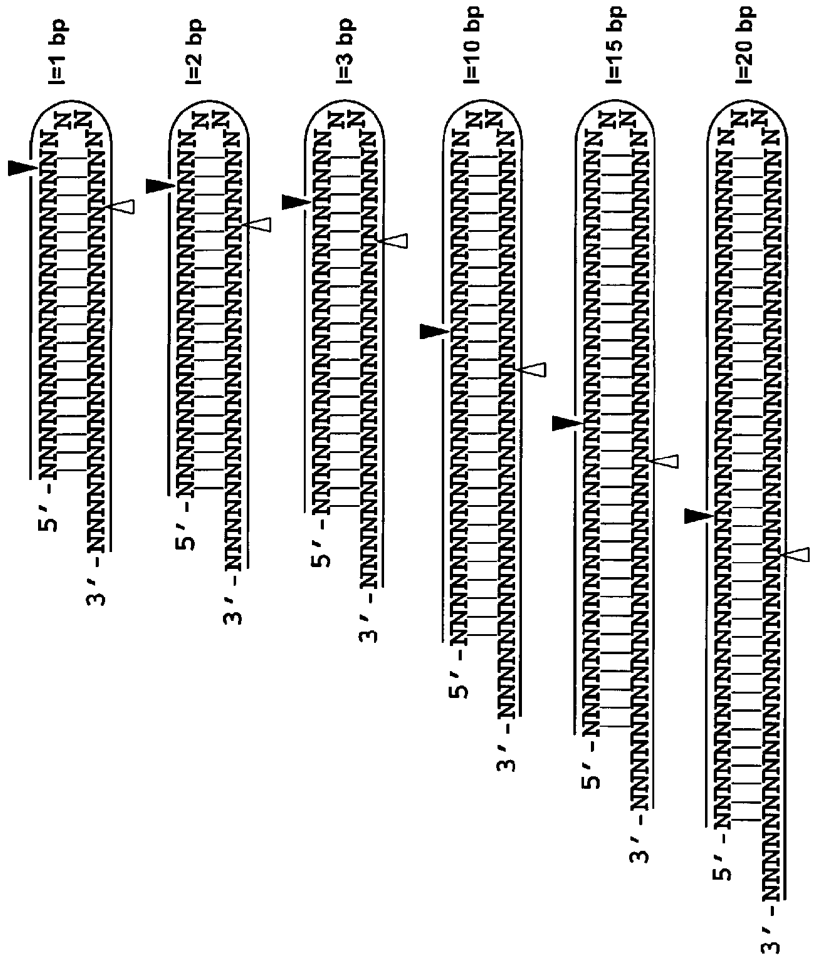

Fig 35A

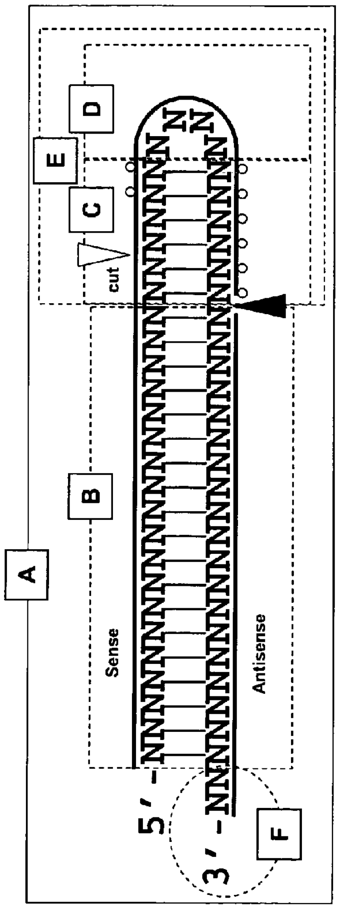


Fig 35B

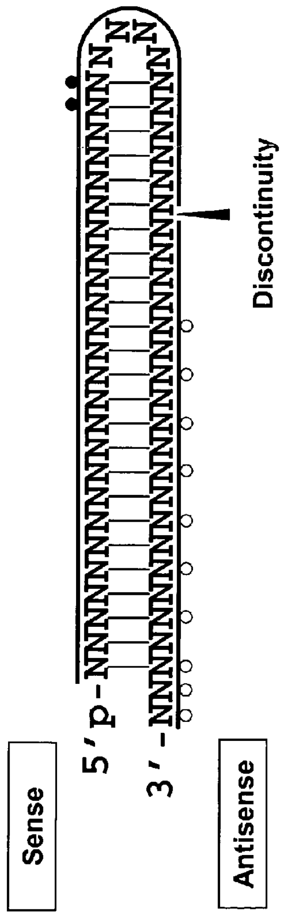

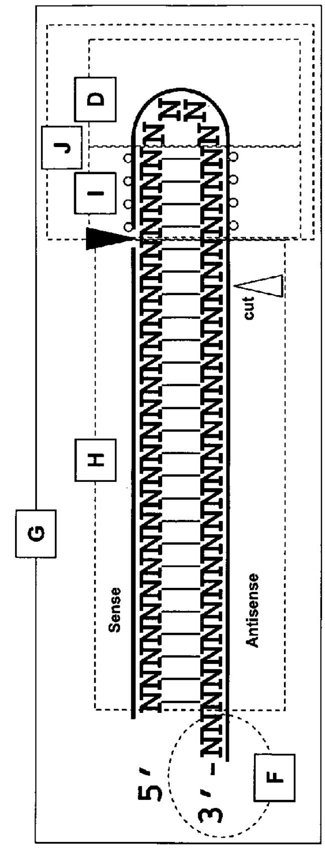


Fig 36A

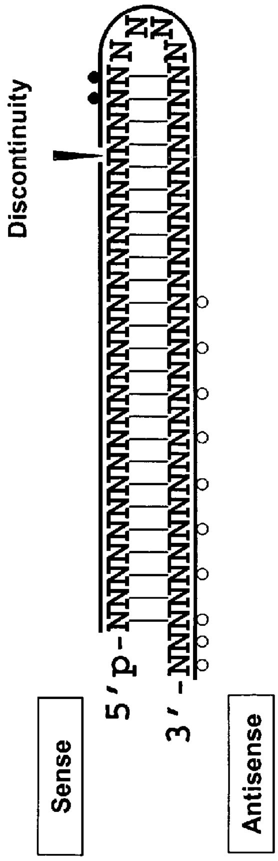


Fig 36B

Fig 37

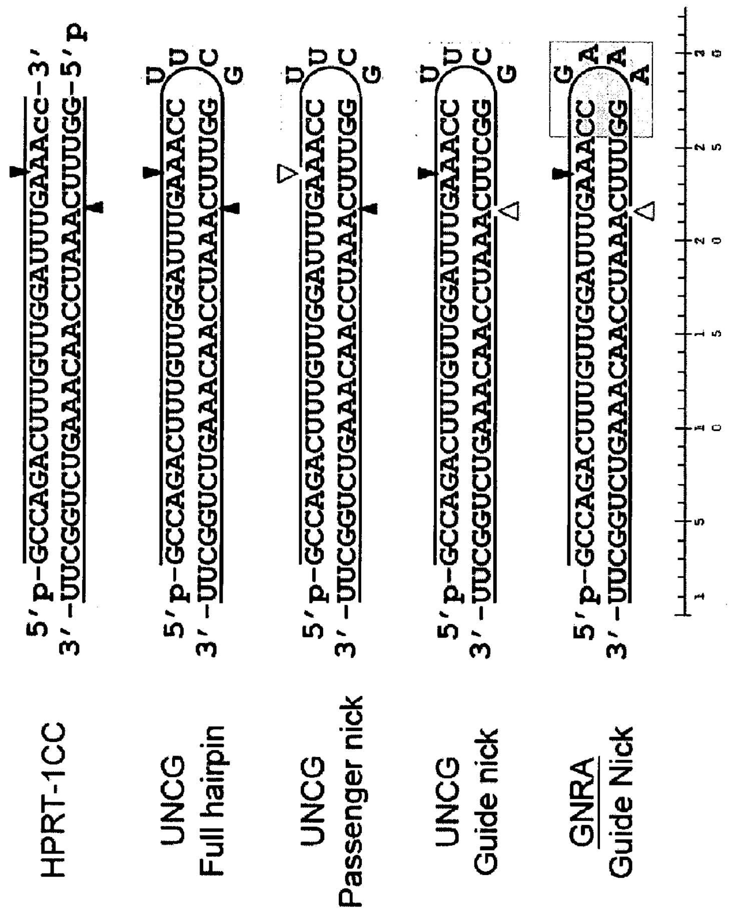
HPRT-1CC
5′p-GCCAGACUUUGUUGGAUUUGAAAcc-3′
3′-UUCGGUCUGAAACAACCUAAACUUUGG-5′p
UNCG
Full hairpin
5′p-GCCAGACUUUGUUGGAUUUGAAACC
3′-UUCGGUCUGAAACAACCUAAACUUUGG
UUCG
UNCG
Passenger nick
5′p-GCCAGACUUUGUUGGAUUUGAAACC
3′-UUCGGUCUGAAACAACCUAAACUUUGG
UUCG
UNCG
Guide nick
5′p-GCCAGACUUUGUUGGAUUUGAAACC
3′-UUCGGUCUGAAACAACCUAAACUUCGG
UUCG
GNRA
Guide Nick
5′p-GCCAGACUUUGUUGGAUUUGAAACC
3′-UUCGGUCUGAAACAACCUAAACUUUGG
GAAA

Fig 38

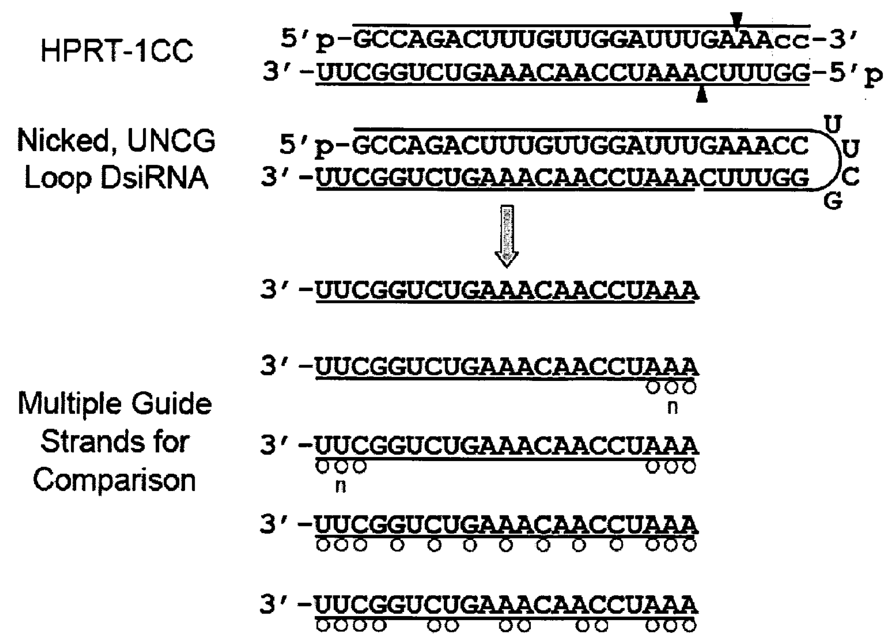
HPRT-1CC
5′p-GCCAGACUUUGUUGGAUUUGAAAcc-3′
3′-UUCGGUCUGAAACAACCUAAACUUUGG-5′p
Nicked, UNCG Loop DsiRNA
5′p-GCCAGACUUUGUUGGAUUUGAAACC
3′-UUCGGUCUGAAACAACCUAAACUUUGG
U
U
C
G
3′-UUCGGUCUGAAACAACCUAAA
Multiple Guide Strands for Comparison
3′-UUCGGUCUGAAACAACCUAAA
n
3′-UUCGGUCUGAAACAACCUAAA
n
3′-UUCGGUCUGAAACAACCUAAA
3′-UUCGGUCUGAAACAACCUAAA

Fig 39

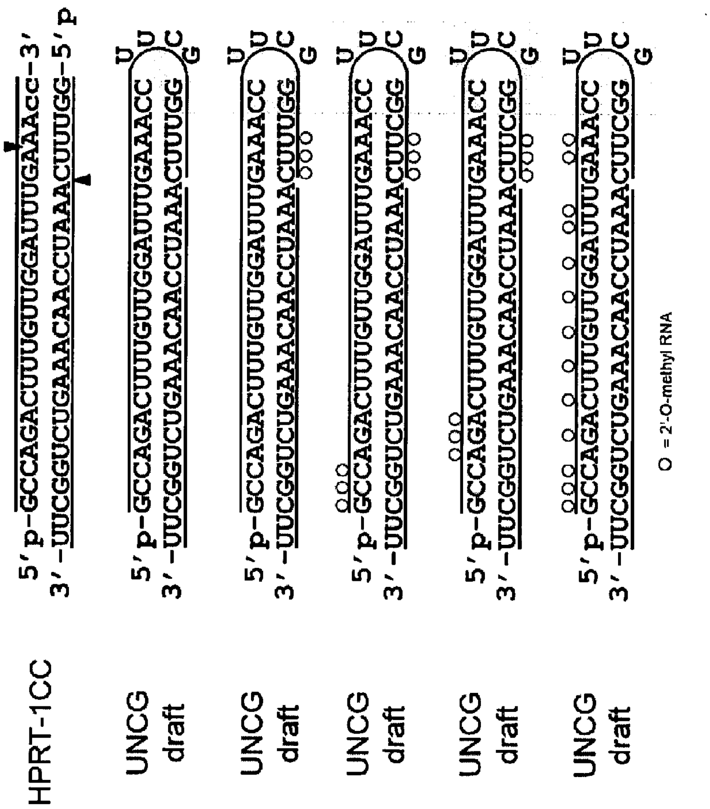
HPRT-1CC
5′p-GCCAGACUUUGUUGGAUUUGAAAcc-3′
3′-UUCGGUCUGAAACAACCUAAACUUUGG-5′p
UNCG draft
5′p-GCCAGACUUUGUUGGAUUUGAAACC
3′-UUCGGUCUGAAACAACCUAAACUUUGG
UUCG
UNCG draft
5′p-GCCAGACUUUGUUGGAUUUGAAACC
3′-UUCGGUCUGAAACAACCUAAACUUUGG
UUCG
UNCG draft
5′p-GCCAGACUUUGUUGGAUUUGAAACC
3′-UUCGGUCUGAAACAACCUAAACUUCGG
UUCG
UNCG draft
5′p-GCCAGACUUUGUUGGAUUUGAAACC
3′-UUCGGUCUGAAACAACCUAAACUUCGG
UUCG
UNCG draft
5′p-GCCAGACUUUGUUGGAUUUGAAACC
3′-UUCGGUCUGAAACAACCUAAACUUCGG
UUCG
O = 2′-O-methyl RNA

Fig 40

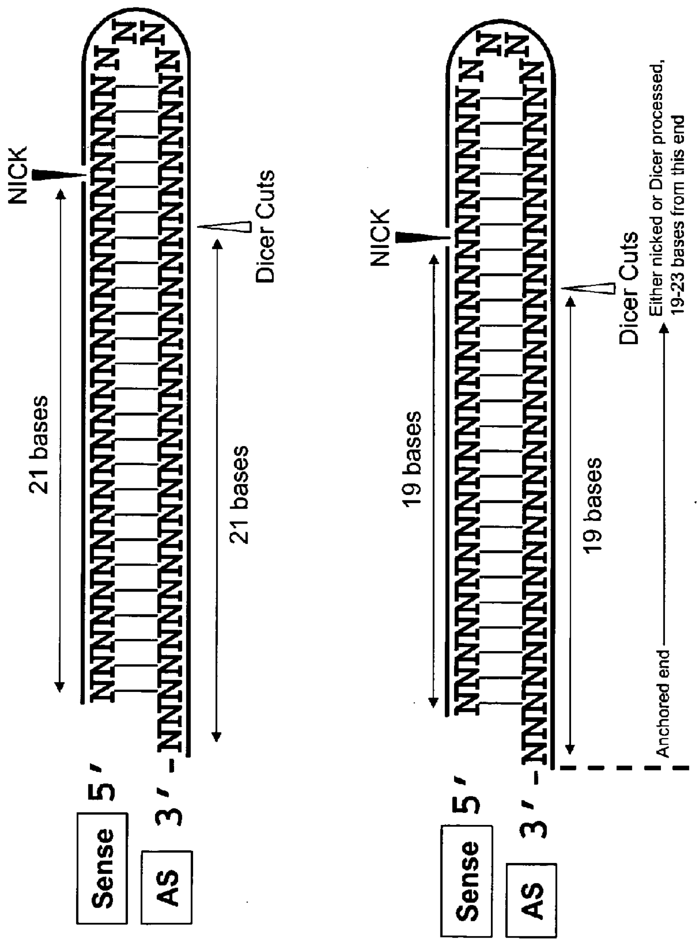
Sense
AS
5'
3'
NICK
21 bases
21 bases
Dicer Cuts
Sense
AS
5'
3'
NICK
19 bases
19 bases
Dicer Cuts
Anchored end
Either nicked or Dicer processed,
19-23 bases from this end

Fig 41

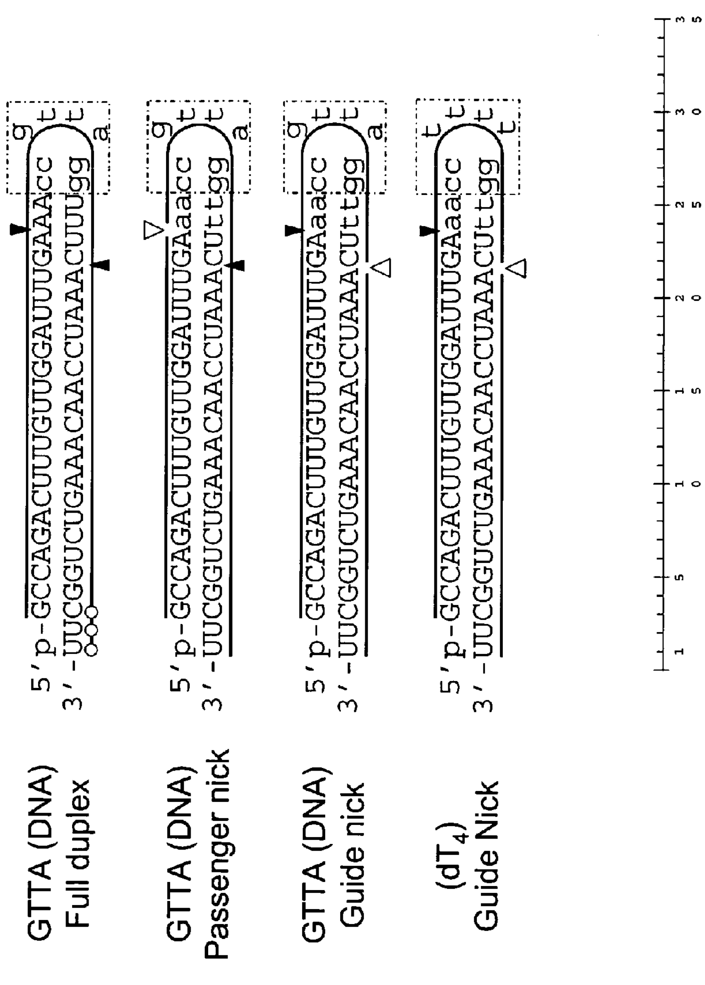
GTTA (DNA)
Full duplex
GTTA (DNA)
Passenger nick
GTTA (DNA)
Guide nick
$(dT_4)$
Guide Nick

## Fig 42 dsNA Conjugates with Nicked Tetraloop

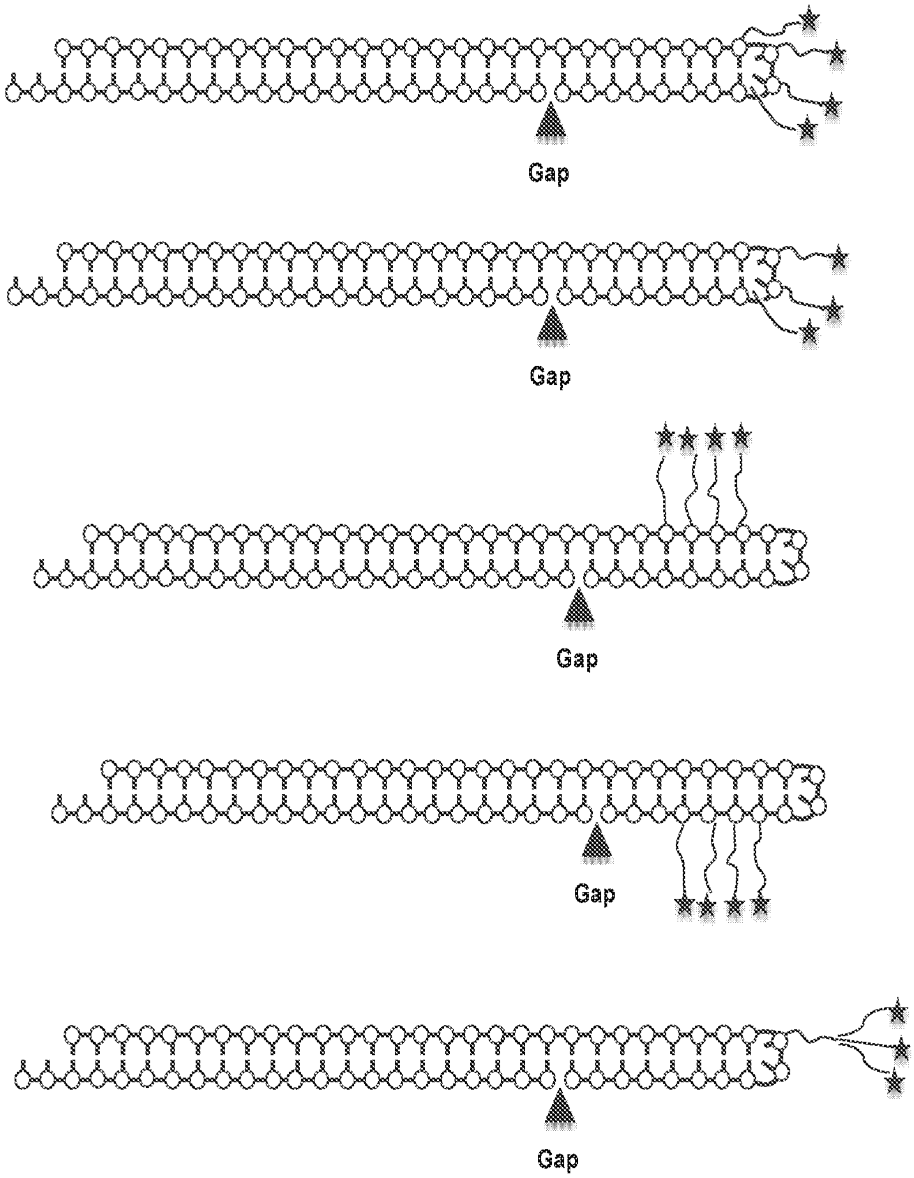


Ligand such as GalNAc, cholesterol, folate, mannose-6-phosphate, etc.

Linker

nucleotide with phosphorothioate linkage

nucleoside or non-nucleoside based monomer with linker connected to ligand

nucleotide with or without modification in S/AS strands

Nicked gap

# Fig 43 dsNA Conjugates with 5'-Extension of Guide Strand

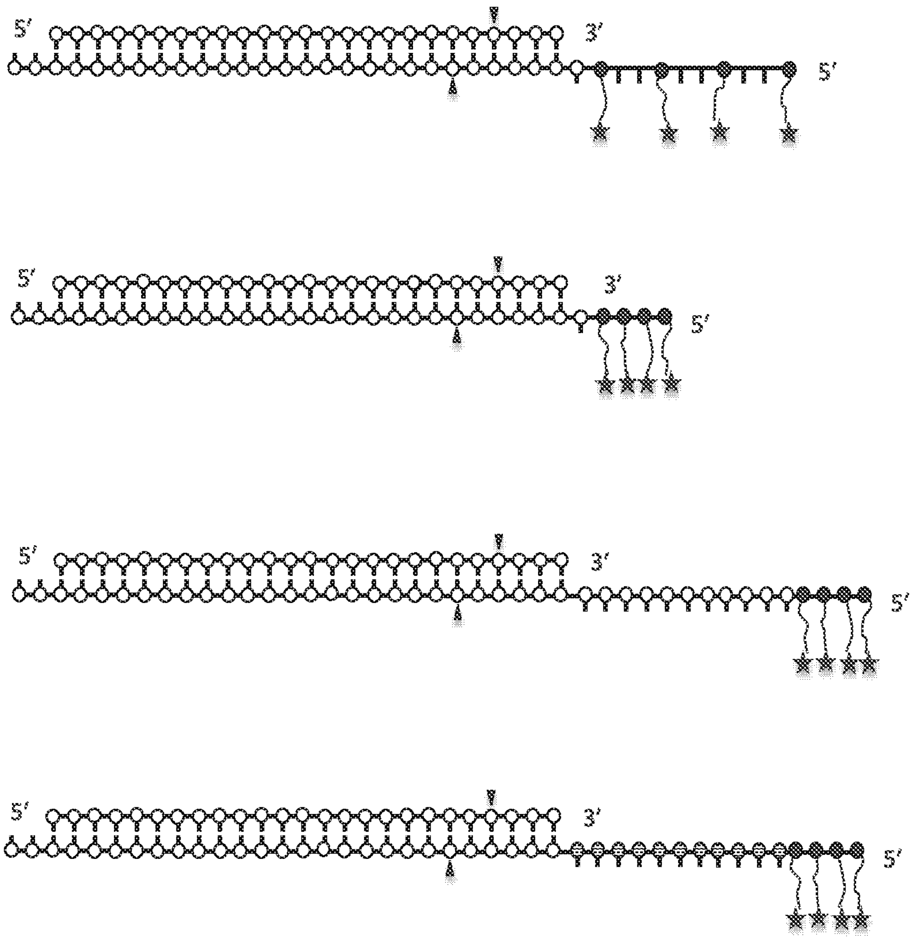


Ligand such as GalNAc, cholesterol,folate, mannose-6-phosphate, etc.

Linker

nucleoside or non-nucleoside based monomer with linker connected to ligand

nucleotide with or without modification in S/AS strands

nucleotide with phosphorothioate linkage

Dicer cleavage site

Spacer

# Fig 44 dsNA Conjugates with 5'-Extension of Passenger Strand

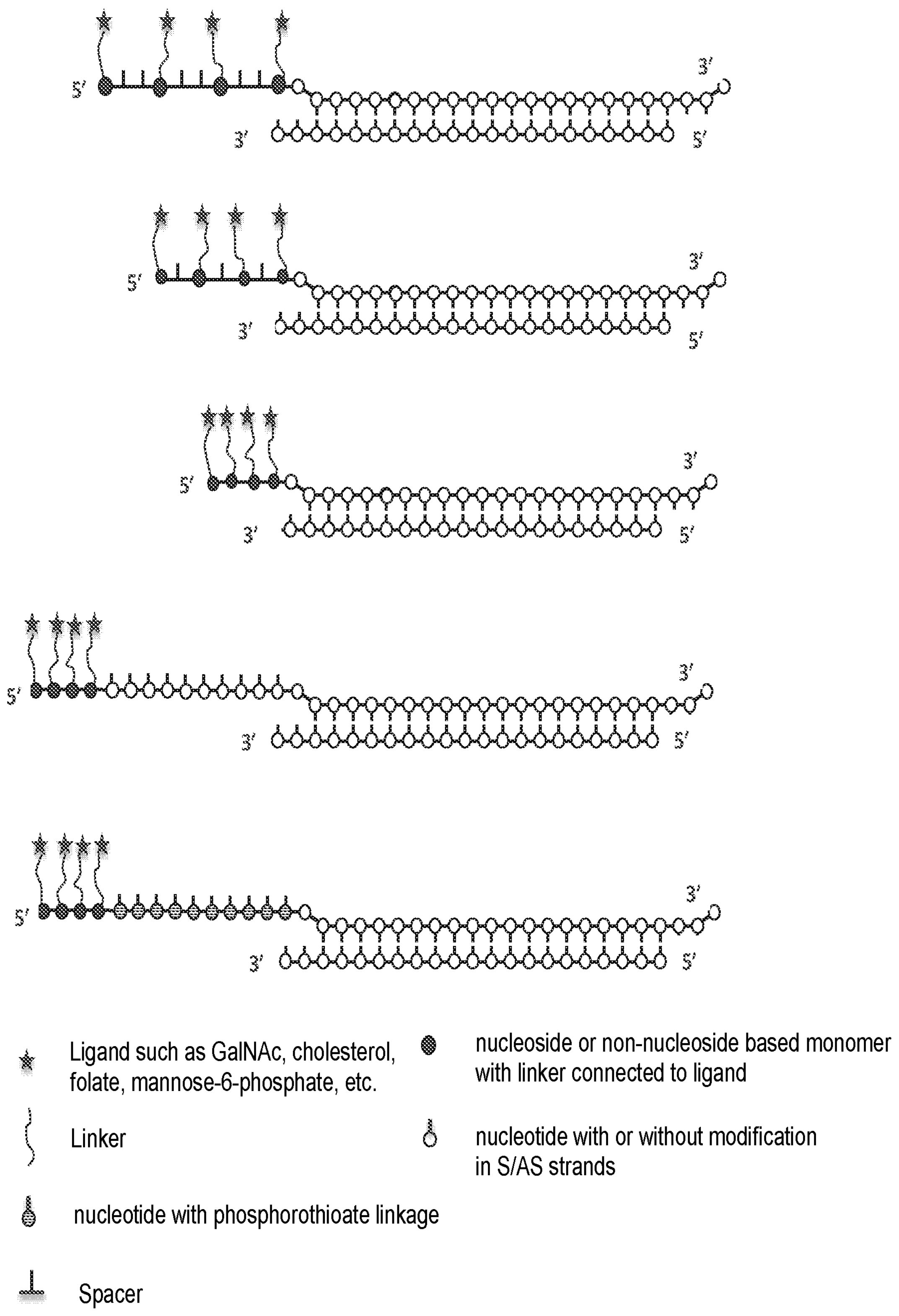

**Fig 45** . Schematics of dsNA agents with extensions

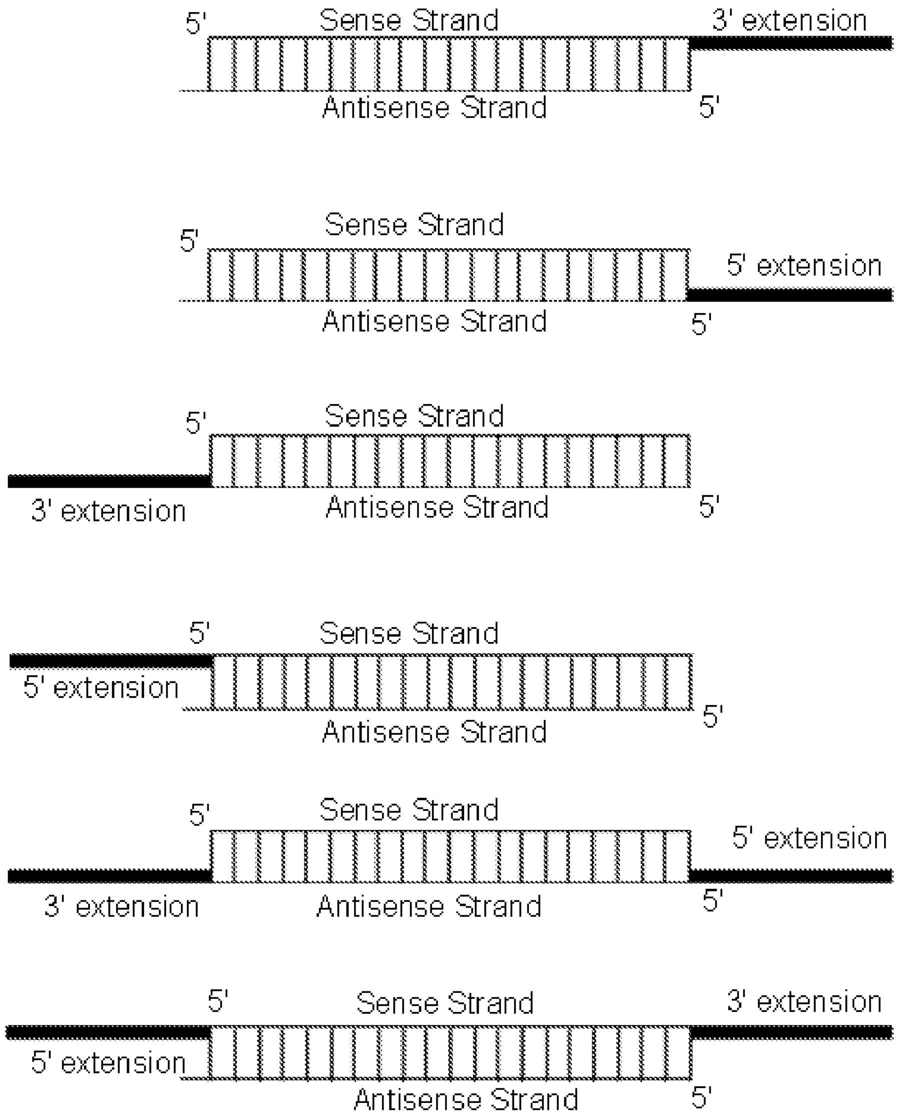

**Fig 46** . Schematics of dsNA agents with extensions

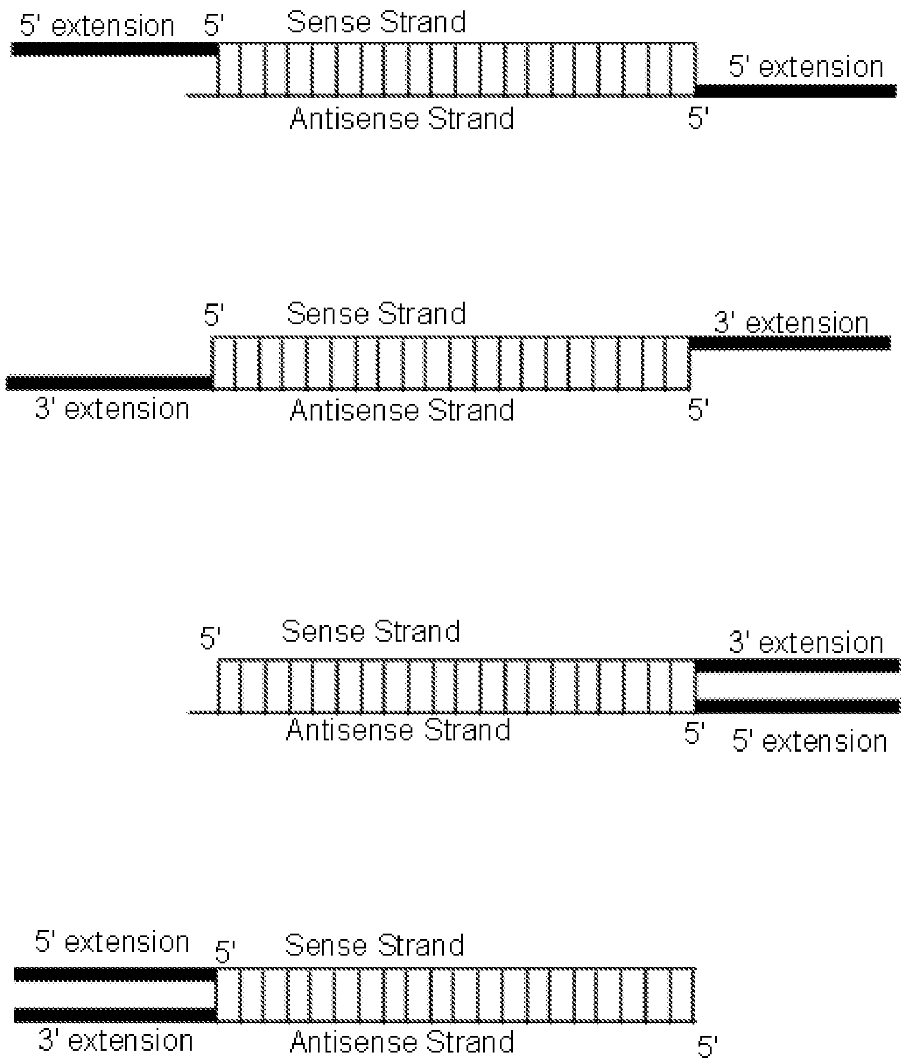

**Fig 47** '. Schematics of dsNA agents with tetraloop

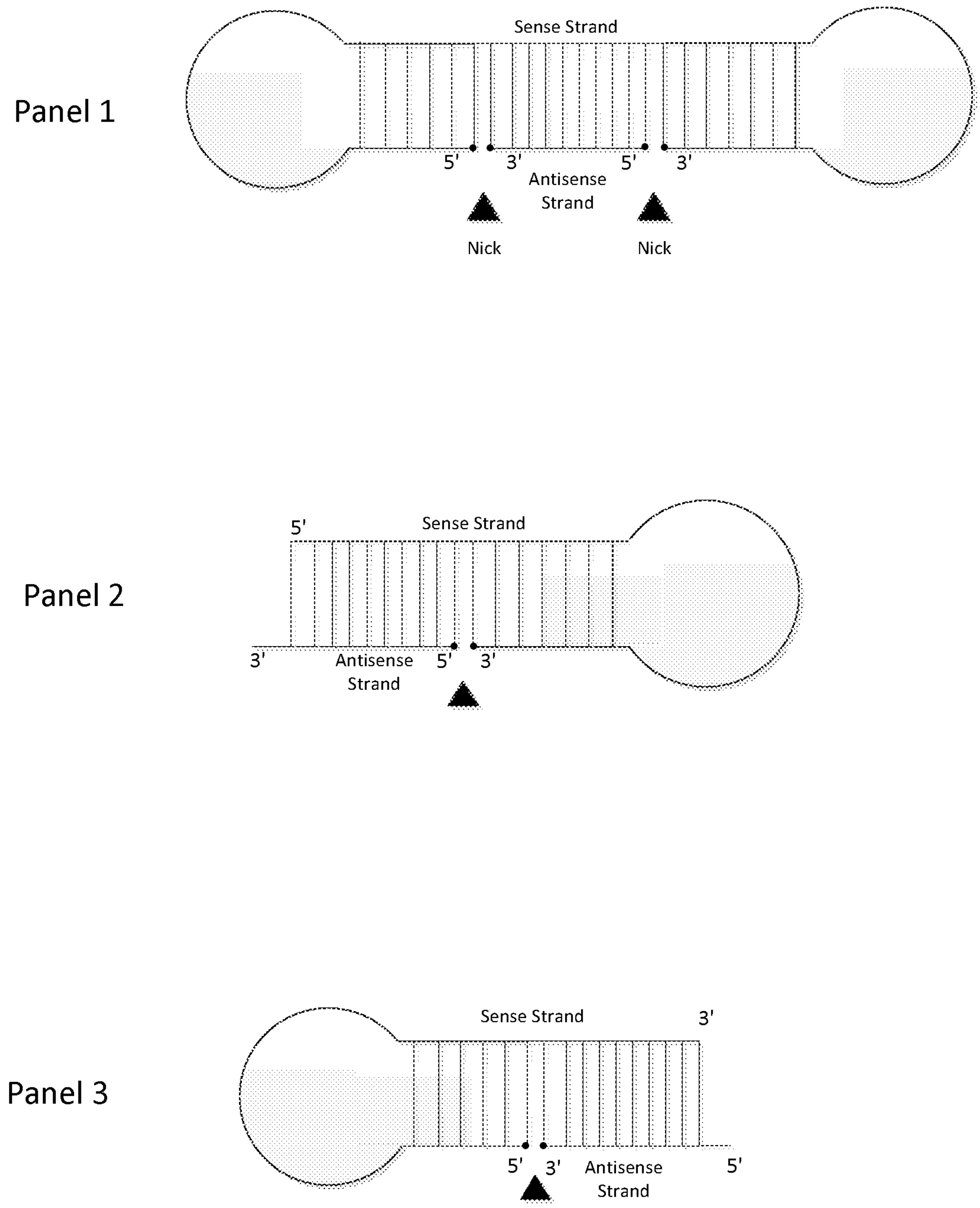

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050244858 A, Rossi
- US 20050277610 A
- US 20070265220 A, Rossi
- US 20140350074 A1
- WO 2015006740 A
- WO 9207065 A, Eckstein
- WO 9315187 A, Usman
- US 1975 A
- US 5672695 A, Eckstein
- US 6248878 B, Matulic-Adamic
- US 5432272 A
- US 6001983 A, Benner
- US 20080213891 A, Manoharan
- US 6218108 B
- US 20070254362 A, Quay
- US 20070223427 A
- US 5684143 A
- US 5858988 A
- US 6291438 B
- US 20040203145 A1
- US 20050042641 A1
- US 6506559 B
- US 20050054598 A1
- US 5705188 A
- WO 9730731 A
- US 6468798 B
- US 6194389 B
- US 6168587 B
- US 6472375 B
- US 6471996 B
- US 4522811 A


### Non-patent literature cited in the description


- **SINGLETON et al.** Dictionary of Microbiology and Molecular Biology. 1994
- The Cambridge Dictionary of Science and Technology. 1988
- The Glossary of Genetics. Springer Verlag, 1991
- **HALE ; MARHAM.** The Harper Collins Dictionary of Biology. 1991
- **LIMBACH et al.** *Nucleic Acids Res.,* 1994, vol. 22, 2183
- **BURGIN et al.** *Biochemistry,* 1996, vol. 35, 14090
- **TURNER et al.** *CSH Symp. Quant. Biol.,* 1987, vol. LII, 123-133
- **FRIER et al.** *Proc. Nat. Acad. Sci. USA,* 1986, vol. 83, 9373-9377
- **TURNER et al.** *J. Am. Chem. Soc.,* 1987, vol. 109, 3783-3785
- **AMBROS.** *Nature,* 2004, vol. 431, 350-355
- **BARTEL.** *Cell,* 2004, vol. 116, 281-297
- **CULLEN.** *Virus Research.,* 2004, vol. 102, 3-9
- **HE et al.** *Nat. Rev. Genet.,* 2004, vol. 5, 522-531
- **YING et al.** *Gene,* 2004, vol. 342, 25-28
- **WAHL, G. M. ; S. L. BERGER.** *Methods Enzymol.,* 1987, vol. 152, 399
- **KIMMEL, A. R.** *Methods Enzymol.,* 1987, vol. 152, 507
- **BENTON ; DAVIS.** *Science,* 1977, vol. 196, 180
- **GRUNSTEIN ; HOGNESS.** *Proc. Natl. Acad. Sci., USA,* vol. 72, 3961
- **AUSUBEL et al.** Current Protocols in Molecular Biology. Wiley Interscience, 2001
- **BERGER ; KIMMEL.** Antisense to Molecular Cloning Techniques. Academic Press, 1987
- **SAMBROOK et al.** Molecular Cloning: A Laboratory Manual. Cold Spring Harbor Laboratory Press
- **COLLINGWOOD et al.** *Oligonucleotides,* 2008, vol. 18, 187-200
- **MACRAE I et al.** Structural basis for double-stranded RNA processing by Dicer. *Science,* 2006, vol. 311 (5758), 195-8
- **SCHWEITZER et al.** *J. Org. Chem.,* 1994, vol. 59, 7238-7242
- **BERGER et al.** *Nucleic Acids Research,* 2000, vol. 28 (15), 2911-2914
- **MORAN et al.** *J. Am. Chem. Soc.,* 1997, vol. 119, 2056-2057
- **MORALES et al.** *J. Am. Chem. Soc.,* 1999, vol. 121, 2323-2324
- **GUCKIAN et al.** *J. Am. Chem. Soc.,* 1996, vol. 118, 8182-8183
- **MORALES et al.** *J. Am. Chem. Soc.,* 2000, vol. 122 (6), 1001-1007
- **MCMINN et al.** *J. Am. Chem. Soc.,* 1999, vol. 121, 11585-11586

- **GUCKIAN et al.** *J. Org. Chem.,* 1998, vol. 63, 9652-9656
- **MORAN et al.** *Proc. Natl. Acad. Sci.,* 1997, vol. 94, 10506-10511
- **DAS et al.** *J. Chem. Soc., Perkin Trans.,* 2002, vol. 1, 197-206
- **SHIBATA et al.** *J. Chem. Soc., Perkin Trans.,* 2001, vol. 1, 1605-1611
- **WU et al.** *J. Am. Chem. Soc.,* 2000, vol. 122 (32), 7621-7632
- **O'NEILL et al.** *J. Org. Chem.,* 2002, vol. 67, 5869-5875
- **CHAUDHURI et al.** *J. Am. Chem. Soc.,* 1995, vol. 117, 10434-10442
- **VAN AERSCHOT et al.** An acyclic 5-nitroindazole nucleoside analogue as ambiguous nucleoside. *Nucleic Acids Res.,* 11 November 1995, vol. 23 (21), 4363-70
- **LOAKES et al.** 3-Nitropyrrole and 5-nitroindole as universal bases in primers for DNA sequencing and PCR. *Nucleic Acids Res.,* 11 July 1995, vol. 23 (13), 2361-6
- **LOAKES ; BROWN.** 5-Nitroindole as a universal base analogue. *Nucleic Acids Res.,* 11 October 1994, vol. 22 (20), 4039-43
- **CHEONG et al.** *Nature,* 16 August 1990, vol. 346 (6285), 680-2
- **HEUS ; PARDI.** *Science,* 12 July 1991, vol. 253 (5016), 191-4
- **WOESE et al.** *Proc Natl Acad Sci USA.,* November 1990, vol. 87 (21), 8467-71
- **ANTAO et al.** *Nucleic Acids Res.,* 11 November 1991, vol. 19 (21), 5901-5
- **NAKANO et al.** *Biochemistry,* 2002, vol. 41 (48), 14281-14292
- **SHINJI et al.** *Nippon Kagakkai Koen Yokoshu,* 2000, vol. 78th (2), 731
- **ELBASHIR et al.** *Genes and Dev.,* 2001, vol. 15, 188-200
- **NYKANEN et al.** *Cell,* 2001, vol. 107, 309-321
- **MARTINEZ et al.** *Cell,* 2002, vol. 110, 563-574
- **LIU et al.** *Science,* vol. 305, 1437-41
- **RODRIGUEZ et al.** *Genome Res.,* 2004, vol. 14 (10A), 1902-1910
- **FARH et al.** *Science,* 2005, vol. 310 (5755), 1817-1821
- **BORCHERT et al.** *Nat. Struct. Mol. Biol.,* 2006, vol. 13 (12), 1097-1101
- **PFEFFER et al.** *Nat. Methods,* 2005, vol. 2 (4), 269-276
- **ANDERSSON et al.** *J. Virol.,* 2005, vol. 79 (15), 9556-9565
- **SAMOLS et al.** *J. Virol.,* 2005, vol. 79 (14), 9301-9305
- **CULLEN.** *Mol. Cell.,* 2004, vol. 16 (6), 861-865
- **CAI et al.** *RNA,* 2004, vol. 10 (12), 1957-1966
- **LEE et al.** *Nature,* 2003, vol. 425 (6956), 415-419
- **KIM.** *Nat. Rev. Mol. Cell. Biol.,* 2005, vol. 6 (5), 376-385
- **YI et al.** *Genes. Dev.,* 2003, vol. 17 (24), 3011-3016
- **BOHNSACK et al.** *RNA,* 2004, vol. 10 (2), 185-191
- **BASYUK et al.** *Nucl. Acids Res.,* 2003, vol. 31 (22), 6593-6597
- **ZAMORE.** *Mol. Cell.,* 2001, vol. 8 (6), 1158-1160
- **CHENDRIMATA et al.** *Nature,* 2005, vol. 436 (7051), 740-744
- **LEE et al.** *EMBO J.,* 2006, vol. 25 (3), 522-532
- **MANIATAKI et al.** *Genes. Dev.,* 2005, vol. 19 (24), 2979-2990
- **HAMMOND et al.** *Nature,* 2000, vol. 404 (6775), 293-296
- **MEISTER et al.** *Mol. Cell.,* 2004, vol. 15 (2), 185-197
- **ZENG et al.** *Proc. Natl. Acad. Sci. USA,* 2003, vol. 100 (17), 9779-9784
- **HUTVAGNER et al.** *Science,* 2002, vol. 297 (55 89), 2056-2060
- **YEKTA et al.** *Science,* 2004, vol. 304 (5670), 594-596
- **OLSEN et al.** *Dev. Biol.,* 1999, vol. 216 (2), 671-680
- **LEWIS et al.** *Cell,* 2005, vol. 120 (1), 15-20
- **BRENNECKE et al.** *PLoS Biol.,* 2005, vol. 3 (3), e85
- **LYTLE et al.** *Proc. Natl. Acad. Sci. USA,* 2007, vol. 104 (23), 9667-9672
- **ZENG et al.** *Molecular Cell,* vol. 9, 1-20
- **CARE et al.** *Nat. Med.,* vol. 13, 613-618
- **PAPPAS et al.** *Expert Opin Ther Targets.,* vol. 12, 115-27
- **TUSCHL et al.** *Genes and Development,* 1999, vol. 13, 3191-3197
- **ZAMORE et al.** *Cell,* 2000, vol. 101, 25-33
- **TS'O et al.** Cell-Type Specific and Ligand Specific Enhancement of Cellular Uptake of Oligodeoxynucleoside-Methylphosphonates Covalently Linked with a Neoglycopeptide, YEE(ah-GalNAc)s. *Bioconjugate Chem.,* 1995, vol. 6, 695-701
- **CHIU MH ; TAMURA T ; WADHWA MS ; RICE KG.** In vivo targeting function of N-linked oligosaccharides with terminating galactose and N-acetylgalactosamine residues. *J Biol Chem.,* 10 June 1994, vol. 269 (23), 16195-202
- **RIEGELMAN ; COLLIER.** *J.Pharmacokinet. BioPharm.,* 1980, vol. 8, 509-534
- **BENET ; GALEAZZI.** *J.Pharm.Sci,* 1979, vol. 68, 1071-1074
- **SETH et al.** Targeted delivery of antisense oligonucleotides to hepatocytes using triantennary N-acetyl galactosamine improves potency 10-fold in mice. *Nucleic Acids Res.,* July 2014, vol. 42 (13), 8796-807

- **ATSMA D.E. ; KEMPEN H.J. ; NIEUWENHUIZEN W. ; VAN 'T HOOFT, F.M. ; PAUWELS E.K.** Partial characterization of low density lipoprotein preparations isolated from fresh and frozen plasma after radiolabeling by seven different methods. *J. Lipid Res.,* 1991, vol. 32, 173-181
- **MCCAFFREY et al.** *Nature,* 2002, vol. 418 (6893), 38-9
- **XIA et al.** *Nature Biotechnol.,* 2002, vol. 20 (10), 1006-10
- **PUTNAM.** *Am. J. Health Syst. Pharm.,* 1996, vol. 53 (2), 151-160
- *Am. J. Health Syst. Pharm.,* 1996, vol. 53 (3), 325
- **HAMAJIMA et al.** *Clin. Immunol. Immunopathol.,* 1998, vol. 88 (2), 205-10
- **MANIATIS et al.** Molecular Cloning. Cold Spring Harbor Laboratory Press, 1982
- **SAMBROOK et al.** Molecular Cloning. Cold Spring Harbor Laboratory Press, 1989
- **SAMBROOK ; RUSSELL.** Molecular Cloning. Cold Spring Harbor Laboratory Press, 2001
- **AUSUBEL et al.** Current Protocols in Molecular Biology. John Wiley and Sons, 1992
- **GLOVER.** DNA Cloning. IRL Press, 1985
- **HARLOW ; LANE.** Antibodies. Cold Spring Harbor Laboratory Press, 1988
- Nucleic Acid Hybridization. 1984
- Transcription And Translation. 1984
- **R. I. FRESHNEY.** Culture Of Animal Cells. Alan R. Liss, Inc, 1987
- Immobilized Cells And Enzymes. IRL Press, 1986
- **B. PERBAL.** *A Practical Guide To Molecular Cloning,* 1984
- Methods In Enzymology. Academic Press, Inc,
- Gene Transfer Vectors For Mammalian Cells. Cold Spring Harbor Laboratory, 1987
- Methods In Enzymology. vol. 154, 155
- Immunochemical Methods In Cell And Molecular Biology. Academic Press, 1987
- Handbook Of Experimental Immunology. 1986, vol. I-IV
- **RIOTT.** Essential Immunology. Blackwell Scientific Publications, 1988
- **HOGAN et al.** Manipulating the Mouse Embryo. Cold Spring Harbor Laboratory Press, 1986
- **WESTERFIELD, M.** The zebrafish book. A guide for the laboratory use of zebrafish (Danio rerio). Univ. of Oregon Press, 2000